# EUROPEAN PATENT APPLICATION

(11) **EP 4 840 470 A1**
(43) Date of publication of application: **18.11.2026**
(21) Application number: 25738399.2
(22) Date of filing: 07.01.2025
(51) Int. Cl.: A61K 47/68, C07D 487/04, C07D 409/14, C07D 413/14, A61K 31/551, A61K 31/517, A61P 35/00

(54) **ANTIBODY-DRUG CONJUGATE AND USE THEREOF**

(30) Priority: 08.01.2024 CN 202410026865; 24.12.2024 CN 202411922793
(71) Applicant: Tyligand Bioscience (Shanghai) Limited, Shanghai 201203 (CN)
(72) Inventor: ZHONG, Boyu, Irving, TX 75038 (US); DENG, Zuyong, Shanghai 201318 (CN); CHEN, Guangming, Shanghai 201202 (CN); ZHANG, Tony Yantao, Fishers, IN 46037 (US); DONG, Chunlan, Shanghai 201203 (CN); WANG, Xiaowen, Shanghai 201299 (CN); SUN, Jinming, Shanghai 201314 (CN); WU, Yangyang, Shanghai 201399 (CN); WANG, Zhenglong, Shanghai 200137 (CN); WANG, Jifa, Shanghai 201210 (CN); CAO, Manli, Shanghai 201299 (CN); SHANG, Erchang, Shanghai 201112 (CN)
(74) Representative: Eisenführ Speiser
(86) International application number: PCT/CN2025/071020
(87) International publication number: WO 2025/148868

(57) **Abstract**

The present invention relates to an antibody-drug conjugate, and in particular, to an antibody-drug conjugate (ADC) carrying a Ras mutation inhibitor, a composition comprising the ADC molecule, and therapeutic use thereof.

## Description

### Technical Field

The present disclosure relates to antibody-drug conjugates (ADCs) based on Ras mutation inhibitors, pharmaceutical compositions comprising the same, and methods for their use in treating or preventing diseases associated with Ras mutations. Specifically, the present disclosure relates to compounds, uses, and methods for treating or preventing related diseases such as tumors or cancers by targeting oncogenic mutants of Ras.

### Background Technology

Ras belongs to the GTPase protein family. Under normal physiological conditions, Ras is activated by growth factors and various other extracellular signals and is responsible for regulating functions such as cell growth, survival, migration, and differentiation. These regulatory functions of Ras are carried out through the transition between an inactive GDP-bound state and an active GTP-bound state, i.e., a "molecular switch". GDP-bound Ras is an inactive form that is activated when exposed to several pro-growth stimuli, such as guanine nucleotide exchange factor (GEF), releasing GDP and binding to GTP, thereby converting to an active GTP-bound state, which recruits and activates various downstream effectors for signal transmission, thereby controlling numerous critical cellular processes such as differentiation, survival, and proliferation.

Ras has GTPase activity; it can cleave the terminal phosphate of GTP to convert it to GDP, thereby converting itself to an inactive state. However, the intrinsic GTPase activity of Ras is very low. Converting GTP-Ras to GDP-Ras requires exogenous protein GAP (GTPase-activating protein). GAP interacts with Ras and promotes the conversion of GTP to GDP. Therefore, any Ras gene mutation that affects the interaction between Ras and GAP or the conversion of GTP to GDP will lead to Ras being in an over-activated state, thereby continuously transmitting growth and division signals to cells, stimulating continuous cell proliferation, and ultimately leading to tumor formation and development. Indeed, dysregulation of the Ras signaling pathway is almost always associated with disease. Hyperactivating somatic mutations in Ras are among the most common lesions in human cancers.

Although mutations in any of the three Ras isoforms (K-Ras, N-Ras, or H-Ras) have been shown to result in oncogenic transformation, KRas mutations are by far the most common mutations in human cancers, which are common in pancreatic cancer, lung adenocarcinoma, colorectal cancer, gallbladder cancer, thyroid cancer, and cholangiocarcinoma, and can also be found in 25% of patients with non-small cell lung cancer. Most KRas mutations occur at codons G12, G13 and Q61, and about 80% of KRas mutations occur at glycine at codon 12, such as G12C mutation, G12D mutation, G12V mutation, G12A mutation, G12R mutation, G12S mutation and G13D mutation, of which G12D mutation is one of the most common mutations.

In view of this, Ras mutant proteins, such as KRas mutant proteins, have become very attractive anti-cancer drug targets in the pharmaceutical field, and the development of their inhibitors is also regarded as a very promising R & D direction in anti-cancer/oncology drug development. However, drug research and development for K-Ras mutations in the past decades has shown that existing small molecule inhibitors of K-Ras mutations have many insurmountable defects, such as inability to effectively deliver to some lesions such as the colon, breast and pancreas, severe drug resistance, poor membrane permeability, poor hydrophilicity, poor oral pharmacokinetics, and high intravenous toxicity, so KRas has even long been considered as an "incurable" target.

Therefore, the inventor has been dedicated to the development of small molecule inhibitors of Ras, e.g. KRas mutations with improved structural patterns that have been shown to have enhanced Ras, e.g. KRas mutation inhibitory activity and inhibitory activity against related tumors compared to existing technologies Ras, e.g. KRas mutation inhibitors, due to specifically designed structural fragments, while having good oral pharmacokinetic properties, resulting in good druggability, reduced toxic side effects, improved drug resistance and safety, and reduced risk of drug interactions.

Nevertheless, novel therapies that can deliver Ras, e.g. KRas mutant inhibitors site-directed into the tumor environment, can be administered in a more friendly manner, and prolong efficacy, reduce toxic side effects, and drug resistance are urgently needed in the field of cancer treatment. This present disclosure satisfies the above requirements.

Targeted conjugated drugs are a new drug delivery technology with high efficiency and low toxicity, which shows strong advantages in anti-cancer drug research and development. Cytotoxic molecules are linked to guide molecules such as antibodies and peptides through suitable linkers, which can highly selectively deliver conjugated molecules to tumor tissues with highly expressed guide receptors, selectively bring anti-cancer drugs into cancer cells through internalization effects, and then excise and release active anti-cancer molecules using differential functional molecules enriched in tumor tissues and cancer cells. Since the active anti-cancer molecule can only be released and enriched inside the tumor tissues with high expression of the guiding receptor and specific "cutters", this dual-selective drug delivery method greatly reduces the toxicity of the anti-cancer drug and improves its efficacy.

The inventor uses a targeted conjugation strategy to further link the developed set of Ras, e.g. KRas mutant inhibitor compounds to guide molecules, such as antibodies and peptides, through suitable linkers to prepare a set of ADC compounds based on targeted Ras, e.g. KRas mutant inhibitors. The developed ADC compounds, which are dually selective due to the use of targeted Ras, e.g., K-Ras enzyme inhibitors as ADC loadings, can significantly reduce toxicity; have increased coupling efficiency and product purity due to specific modifications to linker units, inhibit ADC macromolecule aggregation, and improve efficacy; and these ADC macromolecules delay the metabolism of loaded Ras, e.g., KRas mutant inhibitors, thus further improving the system stability and PK properties of the drug, obtaining a long half-life, long-lasting target inhibition, and prolonged efficacy in vivo, thereby delaying the development of drug resistance.

### Summary of Invention

To meet the above needs in the art, the present inventors conducted in-depth research and obtained antibody-drug conjugates as shown in the Examples by conjugating a group of Ras, e.g., KRas mutant, inhibitor compounds with specific structures to guiding molecules through selected linker units.

Ras inhibitors currently under development, such as KRas inhibitors, have shown many defects in the development and use process, including inability to effectively deliver to certain lesions, severe drug resistance, poor membrane permeability, poor hydrophilicity, poor oral PK properties, and high intravenous toxicity; while the disclosed antibody drug conjugate, through innovative structural modification of the components of the linker unit of ADC and the first use of this linker unit for conjugation Ras small molecule inhibitors and antibody guiding units, can be rapidly and effectively endocytosed by tumor cells, and shows significant tumor growth inhibition activity and good tolerability compared with antibodies alone and Ras inhibitors alone in animal models, which well overcomes the above defects. It is expected that more mature and friendly administration methods (e.g., oral and parenteral) can be used to provide enhanced and prolonged efficacy, good pharmacokinetic properties, reduced toxic side effects and drug resistance, as well as expanded indications and beneficiary populations.

Therefore, in the first aspect, the present disclosure provides an antibody-drug conjugate (ADC) of formula (X) or a pharmaceutically acceptable salt or solvate thereof:

[P-L]_{q}-Ab (X)

wherein,
P represents a Ras inhibitor, e.g., a Ras inhibitor as defined in the section on the Drug P unit of the present disclosure (e.g., a compound of formula (I) and its sub-formulas as defined herein);
L represents a linker unit connecting P to Ab;
q represents the number of [P-L] linking groups connected to Ab, e.g., q = an integer or non-integer from 1 to 20, such as 1-10, 1-8, 2-8, 3-8, 4-8, or 6-8;
Ab represents an antibody or antigen-binding fragment.

In the second aspect, the present disclosure provides a pharmaceutical composition comprising an ADC of the present disclosure or a stereoisomer, tautomer, stable isotopic variant, pharmaceutically acceptable salt, or solvate thereof, optionally at least one other therapeutic agent, and optionally one or more pharmaceutically acceptable excipients.

In the third aspect, the present disclosure provides an ADC of the present disclosure or a stereoisomer, tautomer, stable isotopic variant, pharmaceutically acceptable salt, or solvate thereof, or a pharmaceutical composition comprising the same, for use as a therapeutic agent for treating or preventing diseases mediated by Ras mutant proteins (such as, but not limited to, G12C mutant, G12D mutant, G12V mutant, G12A mutant, G12R mutant, G12S mutant, and G13D mutant proteins), more specifically for use in treating or preventing hyperproliferative diseases, especially as an anti-tumor therapeutic agent. In some specific embodiments, the Ras mutant protein is a KRas mutant protein.

In the fourth aspect, the present disclosure provides use of an ADC of the present disclosure or a stereoisomer, tautomer, stable isotopic variant, pharmaceutically acceptable salt, or solvate thereof, or a pharmaceutical composition comprising the same, in the prevention or treatment of diseases mediated by Ras mutant proteins (such as, but not limited to, G12C mutant, G12D mutant, G12V mutant, G12A mutant, G12R mutant, G12S mutant, and G13D mutant proteins), more specifically in the treatment or prevention of hyperproliferative diseases, especially tumors.

In the fifth aspect, the present disclosure provides a method for treating or preventing a disease mediated by a Ras mutant protein (such as, but not limited to, G12C mutant, G12D mutant, G12V mutant, G12A mutant, G12R mutant, G12S mutant, and G13D mutant proteins) in a subject, comprising administering to a human or animal an ADC of the present disclosure or a stereoisomer, tautomer, stable isotopic variant, pharmaceutically acceptable salt, or solvate thereof, or a pharmaceutical composition comprising the same; specifically, the present disclosure provides a method for treating or preventing a hyperproliferative disease, especially a tumor, in a subject, comprising administering to a human or animal an ADC of the present disclosure or a stereoisomer, tautomer, stable isotopic variant, pharmaceutically acceptable salt, or solvate thereof, or a pharmaceutical composition comprising the same.

In the sixth aspect, the present disclosure provides use of an ADC of the present disclosure or a stereoisomer, tautomer, stable isotopic variant, pharmaceutically acceptable salt, or solvate thereof, or a pharmaceutical composition comprising the same, in the manufacture of a medicament for the prevention or treatment of diseases mediated by Ras mutant proteins (such as, but not limited to, G12C mutant, G12D mutant, G12V mutant, G12A mutant, G12R mutant, G12S mutant, and G13D mutant proteins); specifically, the present disclosure provides use of an ADC of the present disclosure or a stereoisomer, tautomer, stable isotopic variant, pharmaceutically acceptable salt, or solvate thereof, or a pharmaceutical composition comprising the same, in the manufacture of a medicament for the treatment or prevention of hyperproliferative diseases, especially tumors.

In the seventh aspect, the present disclosure provides a pharmaceutical combination comprising an ADC of the present disclosure or a stereoisomer, tautomer, stable isotopic variant, pharmaceutically acceptable salt, or solvate thereof, and at least one other therapeutic agent; the combination is for use in the prevention or treatment of diseases mediated by Ras mutant proteins (such as, but not limited to, G12C mutant, G12D mutant, G12V mutant, G12A mutant, G12R mutant, G12S mutant, and G13D mutant proteins), more specifically in the treatment or prevention of hyperproliferative diseases, especially tumors; and a method for treating or preventing a disease mediated by a Ras mutant protein (such as, but not limited to, G12C mutant, G12D mutant, G12V mutant, G12A mutant, G12R mutant, G12S mutant, and G13D mutant proteins), more specifically a hyperproliferative disease, especially a tumor, in a subject, comprising administering to a human or animal the pharmaceutical combination of the present disclosure.

In the eighth aspect, the present disclosure also provides a method for preparing an ADC of the present disclosure or a stereoisomer, tautomer, stable isotopic variant, pharmaceutically acceptable salt, or solvate thereof by conjugating a Ras inhibitor as defined herein to an antibody or antigen-binding fragment via a linker unit. The Ras inhibitor compounds defined herein can be conjugated to the antibody or antigen-binding fragment via a cleavable or non-cleavable linker unit. In specific embodiments, upon cleavage of the linker unit, the Ras inhibitor is released into the tumor cell, cancer-associated immune cell, or tumor microenvironment.

In some embodiments of various aspects of the present disclosure described above, the Ras mutant protein is a KRas mutant protein, specifically a KRas G12D mutant protein, and correspondingly the P inhibitor compound is a KRas inhibitor, specifically a KRas G12D inhibitor; in other embodiments, the Ras mutant protein is not limited to a specific isoform and/or mutation site, i.e., it is pan-Ras, and correspondingly the P inhibitor compound is a pan-Ras inhibitor.

The present disclosure is further illustrated in the following drawings and specific embodiments. However, these drawings and specific embodiments should not be considered as limiting the scope of the present disclosure, and modifications apparent to those skilled in the art are intended to be included within the spirit of the disclosure and the protection scope of the appended claims.

### Description of Drawings

Figure 1 shows the anti-tumor effect and body weight changes of representative ADC compounds of the present disclosure in a subcutaneous xenograft BALB/c Nude mouse model of KRAS-G12D mutant human colon cancer cell GP2D (dose: 20 mg/kg, D0/7 days dosing, 21 days).
Figure 2 shows the anti-tumor effect and body weight changes of representative ADC compounds of the present disclosure in a subcutaneous xenograft BALB/c Nude mouse model of KRAS-G12D mutant human colon cancer cell GP2D in a separate experiment batch (dose: 10 mg/kg, D0 dosing, 21 days).
Figure 3 shows the anti-tumor effect of representative ADC compounds of the present disclosure in a subcutaneous xenograft BALB/c Nude mouse model of KRAS-G12D mutant human colon cancer cell GP2D in a separate experiment batch (dose: 10 mg/kg, D0 dosing, 20 days).
Figures 4A, 4B, 4C show results from the same separate experiment batch, i.e., the anti-tumor effect of representative ADC compounds of the present disclosure in a subcutaneous xenograft BALB/c Nude mouse model of KRAS-G12D mutant human colon cancer cell GP2D (dose: 10 mg/kg, D0 dosing, 20 days). For clarity of presentation, the example compounds in this batch experiment are shown separately in Figures 4A, 4B, and 4C.
Figures 5 and 6 respectively show the anti-tumor effect of representative ADC compounds of the present disclosure, drug P unit, and antibody in a subcutaneous xenograft BALB/c Nude mouse model of KRAS-G12D mutant human colon cancer cell GP2D in separate experiment batches (dose: 10 mg/kg, D0 and/or 7 and/or 14 days dosing, 21 days).

### Detailed Description of Invention

### Definitions

Unless otherwise defined, all technical and scientific terms used herein have the same meaning as commonly understood by one of ordinary skill in the art. For the purposes of the present disclosure, the following terms are defined below.

When a trade name is used herein, unless the context indicates otherwise, the trade name includes the product formulation, the generic drug, and the active pharmaceutical ingredient(s) of the trade name product.

As used herein, the term "substantially" means the majority, i.e., > about 50% of a population, mixture, sample, content, or any other numerical value, preferably greater than about 50%, 55%, 60%, 65%, 70%, 75%, 80%, 85%, 90%, 91%, 92%, 93%, 94%, 95%, 96%, 97%, 98%, or 99%.

As used herein, the term "and/or" should be understood to mean any one of the options or any combination of two or more of the options.

As used herein, the term "ADC" or "conjugate" refers to an antibody-drug conjugate.

As used herein, the term "drug" refers to a substance that produces a beneficial preventive or therapeutic effect on a disease mediated by a Ras mutant protein, e.g., a KRas mutant protein (such as, but not limited to, G12C mutant, G12D mutant, G12V mutant, G12A mutant, G12R mutant, G12S mutant, and G13D mutant proteins).

As used herein, the term "Ras mutation" or "Ras mutant protein" refers to a protein encoded and expressed by a Ras gene in which one or more codons have undergone mutation, typically including but not limited to Ras proteins with mutations at glycine codon 12, glycine codon 13, or glutamine codon 61 of Ras, such as mutated HRas, NRas, or KRas. These residues are located in the active site of Ras, and their mutations can impair the intrinsic or GAP-catalyzed GTPase activity of Ras, leading to the persistent presence of GTP-bound Ras.

For the purposes of the present disclosure, "Ras mutation" or "Ras mutant protein" and "Ras" when describing inhibitory activity are used interchangeably, for example, referring to mutated KRas, such as, but not limited to, KRas-G12C (glycine to cysteine mutation at codon G12), KRas-G12D (glycine to aspartic acid mutation at codon G12), HRas-G12D, NRas-G12D, KRas-G12V (glycine to valine mutation at codon G12), KRas-G13D (glycine to aspartic acid mutation at codon G13); in some embodiments refers to a KRas mutant protein, more particularly to a KRas-G12C mutant protein, KRas-G12D mutant protein, KRas-G12V mutant protein, G12A mutant protein, G12R mutant protein, G12S mutant protein, KRas-G13D mutant protein, most particularly to KRas-G12D; in other embodiments refers to a pan-RAS mutant protein, i.e., not limited to a specific isoform and mutation site.

As used herein, the term "Ras mutation-mediated disease" refers to a disease where Ras mutation contributes to the onset and progression of the disease, or where inhibiting Ras mutation will reduce the incidence, or reduce or eliminate the symptoms of the disease. For the present disclosure, "Ras mutation-mediated disease" in some embodiments refers to a KRas mutation-mediated disease, most preferably KRas-G12D; in other embodiments refers to a pan-Ras-mediated disease, such as a hyperproliferative disease like cancer or tumor.

As used herein, the term "cancer" or "tumor" refers to abnormal cell growth and proliferation, including solid tumors and hematological tumors, whether malignant or benign, and all precancerous and cancer cells and tissues. For various aspects of the present disclosure, the cancer or tumor includes, but is not limited to, lung adenocarcinoma, lung cancer (including lung squamous cell carcinoma and non-small cell lung cancer, small cell lung cancer (SCLC)), bone cancer, pancreatic cancer, pancreatic ductal adenocarcinoma, skin cancer, head and neck cancer (including head and neck squamous cell carcinoma), melanoma (including cutaneous or intraocular melanoma), squamous cell carcinoma, anal region cancer, testicular cancer, urethral cancer, penile cancer, prostate cancer (including hormone-refractory prostate cancer), bladder cancer, uterine cancer, ovarian cancer, ovarian epithelial cancer, fallopian tube cancer, endometrial cancer, cervical cancer, vaginal cancer, vulvar cancer, gastric cancer, gastric adenocarcinoma, colon cancer, rectal cancer, colorectal cancer, liver cancer, breast cancer (including metastatic breast cancer, triple-negative breast cancer (TNBC)), esophageal cancer, small intestine cancer, lip cancer, laryngeal cancer, nasopharyngeal cancer, oral cancer, salivary gland cancer, peritoneal cancer, gastrointestinal stromal tumor, gastroesophageal junction (GEJ) cancer, mesothelioma, biliary tract cancer, hepatocellular carcinoma, seminoma, soft tissue sarcoma, osteosarcoma, urothelial carcinoma, sweat gland carcinoma, endocrine system cancer, thyroid cancer, medullary thyroid cancer, follicular thyroid cancer, papillary thyroid cancer, parathyroid cancer, kidney cancer, renal parenchymal cancer, renal cell carcinoma, renal pelvic cancer, adrenal cancer, brain cancer such as glioblastoma, astrocytoma, meningioma, medulloblastoma, peripheral neuroectodermal tumor, glioblastoma (including glioblastoma multiforme), neuroblastoma; chronic or acute leukemia, Hodgkin's disease, lymphoma (including lymphocytic lymphoma, Hodgkin lymphoma, non-Hodgkin lymphoma, Burkitt lymphoma, adult T-cell lymphoma, diffuse large B-cell lymphoma (DLBCL), primary CNS lymphoma), acute lymphoblastic leukemia (ALL), chronic lymphocytic leukemia (CLL), chronic myeloid leukemia (CLL) and lymphocytic carcinoma, acute myeloid leukemia (AML), myeloid leukemia (chronic myeloid leukemia (CML)), central nervous system (CNS) tumors, spinal tumors, brainstem glioma, or pituitary adenoma.

For various aspects of the present disclosure, preferably, the cancer or tumor is associated with a Ras mutation, e.g., a KRas mutation, including but not limited to the above tumor types and their preferred ranges. Particularly preferred tumors of the present invention include lung cancer, lung adenocarcinoma, colon cancer, rectal cancer, pancreatic cancer, endometrial cancer, cholangiocarcinoma, leukemia, and ovarian cancer.

As used herein, the term "anti-tumor effect" refers to a biological effect that can be characterized in various forms, including, for example, reduction in tumor volume, reduction in the number of tumor cells, reduction in tumor cell proliferation, or reduction in tumor cell survival.

As used herein, the term "inhibit" and "reduce" or any variation of these terms refers to the ability of a biologically active agent to reduce the signaling activity of a target of interest by interacting directly or indirectly with the target, and refers to any measurable reduction or complete inhibition of the target activity. For example, compared to normal conditions, the activity (e.g., Ras activity, e.g., KRas activity) can be reduced by about, up to about, or at least about 5%, 10%, 20%, 30%, 40%, 50%, 60%, 70%, 80%, 90%, 95%, 99%, or more, or any range derivable therein.

As used herein, the term "selectively inhibit" refers to the ability of a biologically active agent to preferentially reduce the signaling activity of a target of interest compared to the signaling activity of an off-target, by interacting directly or indirectly with the target. For the Ras inhibitors and their ADCs of the present disclosure, relative to various mutations occurring at one or more codons of the Ras protein, they have the ability to selectively inhibit G12 or G13 mutations of the KRas protein, such as G12C mutation, G12D mutation, G12V mutation, G12A mutation, G12R mutation, G12S mutation, and G13D mutation, preferably the ability to selectively inhibit the G12D mutation of the KRas protein. For example, compared to another specific Ras mutation, the inhibitory activity of the Ras inhibitors and their ADCs of the present disclosure against a specific mutation such as KRas-G12D is at least 10%, 15%, 20%, 25%, 30%, 35%, 40%, 45%, 50%, 55%, 60%, 65%, 70%, 75%, 80%, 85%, 90%, 95%, 99%, or more, or any range derivable therein, or compared to the activity against another specific Ras mutation, the inhibitory activity against a specific mutation such as KRas-G12D is at least 0.1-, 0.5-, 1-, 2-, 3-, 4-, 5-, 10-, 25-, 50-, 100-, 250-, or 500-fold or higher.

As used herein, the term "dual selective inhibition" refers to the ability of the conjugated drug to exert dual selective inhibitory effects by, on the one hand, targeting and inhibiting a specific Ras mutation via the small molecule targeted inhibitor, and on the other hand, utilizing the conjugated guiding antibody molecule to highly selectively deliver the loaded Ras targeted inhibitor to tumor tissues with high expression of the guiding receptor.

As used herein, the term "antigen" refers to an entity that specifically binds to an antibody.

As used herein, the term "antibody" refers to a polypeptide that comprises at least a light chain or heavy chain immunoglobulin variable region which specifically recognizes and binds an antigen. The term encompasses various antibody structures, including but not limited to monoclonal antibodies, single-chain or multi-chain antibodies, monospecific or multispecific antibodies (e.g., bispecific antibodies), chimeric or humanized antibodies, full-length antibodies, and antibody fragments, as long as they exhibit the desired antigen-binding activity. The antibody can be of any class (e.g., IgG, IgE, IgM, IgD, and IgA), type (e.g., IgG1, IgG2, IgG3, IgG4, IgA1, and IgA2), or subtype.

As used herein, the terms "antibody fragment" and "antigen-binding fragment" of an antibody are used interchangeably and refer to a molecule other than an intact antibody that comprises a portion of an intact antibody that binds the antigen to which the intact antibody binds. As understood by those skilled in the art, for antigen-binding purposes, an antibody fragment typically comprises amino acid residues from a "complementarity determining region" or "CDR". Antibody fragments can be prepared by recombinant DNA techniques or by enzymatic or chemical cleavage of intact antibodies. Antigen-binding fragments include, but are not limited to, Fab, scFab, Fab', F(ab')2, Fab'-SH, Fv, single-chain Fv, diabody, triabody, tetrabody, minibody, single-domain antibody (sdAb); and multispecific antibodies formed from antibody fragments.

As used herein, reference to an IgG antibody means that the antibody is a heterotetrameric protein having the structure of an IgG class immunoglobulin. In an IgG antibody, typically the VH-CH1 of the heavy chain pairs with the VL-CL of the light chain to form a Fab fragment that specifically binds antigen. Thus, an IgG antibody essentially consists of two Fab molecules linked by an immunoglobulin hinge region and two dimerized Fc regions. In some embodiments, the IgG antibody is, e.g., an IgG1, IgG2, IgG3, or IgG4 antibody. In other embodiments, the IgG antibody is an IgGκ or IgGλ antibody, e.g., an IgG1κ or IgG1λ antibody.

As used herein, the terms "complementarity determining region" or "CDR region" or "CDR" or "hypervariable region" are used interchangeably and refer to regions of an antibody variable domain that are hypervariable in sequence and form structurally defined loops ("hypervariable loops") and/or contain antigen-contacting residues ("antigen contact points"). CDRs are primarily responsible for binding to an epitope. As used herein, the CDRs of the antibody heavy and light chains are numbered sequentially from the N-terminus, commonly referred to as CDR1, CDR2, and CDR3. The CDRs located within the antibody heavy chain variable domain are also referred to as HCDR1, HCDR2, and HCDR3, while the CDRs located within the antibody light chain variable domain are referred to as LCDR1, LCDR2, and LCDR3. In a given light chain variable region or heavy chain variable region amino acid sequence, its CDR sequences can be determined using various well-known schemes in the art, including CDR sequences defined based on Kabat, AbM, Chothia, Contact, and IMGT. Furthermore, CDRs can also be determined based on having the same Kabat numbering position as a reference CDR sequence.

As used herein, "variable region" or "variable domain" is the domain of an antibody heavy or light chain that is involved in binding the antibody to its antigen. The heavy chain variable region (VH) and light chain variable region (VL) can be further subdivided into hypervariable regions (HVRs, also termed complementarity determining regions (CDRs)) interspersed with more conserved regions (i.e., framework regions (FRs)). Each VH and VL is composed of three CDRs and four FRs, arranged from amino-terminus to carboxy-terminus in the following order: FR1, CDR1, FR2, CDR2, FR3, CDR3, FR4. In some aspects, the antibody variable region can be modified by CDR grafting. Since CDR sequences are responsible for most antibody-antigen interactions, it is possible to construct recombinant antibody variants that mimic the properties of a known antibody. In such antibody variants, the CDR sequences from a known antibody are grafted onto the framework regions of a different antibody with different properties. The properties of the mutated and/or modified antibody or ADC conjugate comprising it, such as target antigen binding properties or other desired functional properties, e.g., ADC internalization, pharmacokinetics, and in vivo tumor killing activity, can be assessed in in vitro or in vivo assays.

As used herein, the term "isotype" refers to the antibody type determined by the heavy chain constant region. For example, the antibody portion of the ADC according to the present disclosure can be an IgA (e.g., IgA1 or IgA2), IgG1, IgG2 (e.g., IgG2a or IgG2b), IgG3, IgG4, IgE, IgM, and IgD antibody, and have the heavy chain constant region of said immunoglobulin type. Furthermore, the present disclosure contemplates not only antibodies using native sequence constant regions but also antibodies comprising variant sequence constant regions.

As used herein, the term "epitope" includes any protein determinant capable of specifically binding to an immunoglobulin or otherwise interacting with a molecule. Epitopic determinants usually consist of chemically active surface groupings of molecules such as amino acids or carbohydrate or sugar side chains and can have specific three-dimensional structural characteristics as well as specific charge characteristics. An epitope can be "linear" or "conformational". Conformational and linear epitopes differ by loss of binding to the former but not the latter in the presence of denaturing solvent.

As used herein, the term "receptor-mediated endocytosis" refers to a process triggered by the binding of a ligand to its corresponding receptor on the cell surface, whereby the ligand/receptor complex is internalized and delivered into the cytosol or transferred to an appropriate intracellular compartment. The receptor-mediated endocytosis activity of an antibody can be characterized by measuring the rate of internalization.

As used herein, "sequence identity" refers to the extent to which sequences are identical on a nucleotide-by-nucleotide or amino acid-by-amino acid basis over a window of comparison. The "percent sequence identity" can be calculated by: comparing two optimally aligned sequences over a comparison window, determining the number of positions at which the identical nucleic acid base (e.g., A, T, C, G, I) or identical amino acid residue (e.g., Ala, Pro, Ser, Thr, Gly, Val, Leu, Ile, Phe, Tyr, Trp, Lys, Arg, His, Asp, Glu, Asn, Gln, Cys, and Met) occurs in both sequences to yield the number of matched positions, dividing the number of matched positions by the total number of positions in the comparison window (i.e., the window size), and multiplying the result by 100 to yield the percent sequence identity. Optimal alignment for determining percent sequence identity can be achieved in various ways known in the art, for example, using publicly available computer software such as BLAST, BLAST-2, ALIGN, or Megalign (DNASTAR) software. One skilled in the art can determine appropriate parameters for aligning sequences, including any algorithms needed to achieve maximal alignment over the full length of the sequences being compared or over a target sequence region.

As used herein, the term "isolated" antibody is one that has been separated from a component of its natural environment. In some embodiments, an antibody is purified to greater than 90%, 95%, or 99% purity as monitored by, for example, electrophoresis (e.g., SDS-PAGE, isoelectric focusing (IEF), capillary electrophoresis) or chromatography (e.g., ion exchange or reverse phase HPLC).

As used herein, the term "affinity" or "binding affinity" refers to the intrinsic binding affinity that reflects the interaction between members of a binding pair, e.g., the strength of the interaction between an antibody and an antigen at a single antigenic site; the stronger the interaction, the higher the affinity. The affinity of a molecule for its partner can typically be represented by the equilibrium dissociation constant (K_{D}), which is the ratio of the dissociation rate constant to the association rate constant (k_{dis} and kₒₙ, respectively). Affinity can be measured by common methods known in the art, such as antigen protein- or cell-based ELISA assays, flow cytometry assays, Bio-Layer Interferometry (BLI) technology, etc.

As used herein, the term "K_{D}" (M) refers herein to the dissociation equilibrium constant of a particular antibody-antigen interaction. Affinity is inversely related to the K_{D} value, i.e., the higher the affinity, the lower the K_{D} value; conversely, the lower the affinity, the higher the K_{D} value. Generally, the K_{D} value depends on the dissociation rate constant (Kd or Kdis, sec⁻¹) and the association rate constant (Ka, M⁻¹ × sec⁻¹) between the interacting antibody-antigen pair.

As used herein, the term "binding" or "specifically binding" refers to the ability of a single antibody binding site to react with one antigenic determinant and not with a different antigenic determinant. In the context of an antibody binding to its cognate antigen, it refers to binding with a K_{D} value affinity of about 10⁻⁶ M or less, e.g., a K_{D} value of about 10⁻⁷ M or less, or about 10⁻⁸ M or less. The K_{D} value for binding of an antibody to its cognate antigen is preferably at least 100-fold lower, or for example at least 1000-fold lower, than the K_{D} value for binding to a non-specific antigen (e.g., an unrelated antigen such as BSA). Measurement of K_{D} values is known in the art, for example based on Bio-Layer Interferometry (BLI) technology, determined in an instrument such as the ForteBio Octet^{®} using the antibody as the ligand and the antigen as the analyte.

As used herein, the term "effector function" refers to those biological activities attributable to the Fc region of an antibody, which vary with the antibody class. It is known that IgG Fc regions can mediate several important effector functions, such as cytokine induction, ADCC, phagocytosis, complement-dependent cytotoxicity (CDC), and the half- life/clearance rate of antibodies and antigen-antibody complexes. In some cases, depending on the therapeutic purpose, these effector functions may be desirable for a therapeutic antibody, but may be unnecessary in other cases. Thus, in one embodiment, the present disclosure provides antibodies having an Fc region that elicits effector functions such as ADCC or CDC, thereby inducing tumor cell apoptosis, cell lysis, and/or inhibiting proliferation, dissemination, and/or metastasis of TF antigen-bearing tumor cells in TF antigen-bearing tumor cells. In other embodiments, the present disclosure also provides antibodies having an Fc region with altered effector function. Effector function can be altered by making sequence changes to the Fc region of the antibody. Alternatively, antibodies can be prepared with altered types of glycosylation in the Fc region. Altering the glycosylation pattern of the Fc region can be conveniently achieved by altering the amino acid sequence of the Fc region to create or remove one or more glycosylation sites.

As used herein, the term "linker unit" or "linker" refers to a bifunctional moiety that connects the drug to the antibody in an antibody-drug conjugate. The linker unit of the present disclosure has multiple components, such as a self-immolative linker, a cleavable linker, a property-modulating unit, and an antibody linker.

As used herein, the term "self-immolative linker" refers to a temporary extender, spacer, or placeholder unit that links two or more molecules together via chemical bonds that break under defined conditions to release the two molecules. Typically, the self-immolative linker unit can be linear or branched and can link two or more identical molecules together, or can link two or more different molecules together. A self-immolative unit can be defined as a bifunctional chemical group capable of covalently linking two spaced-apart chemical moieties together to form a generally stable molecule, releasing one of the spaced-apart chemical moieties from the molecule by enzymatic cleavage; and following said enzymatic cleavage, spontaneously cleaving from the remainder of the bifunctional chemical group to release the other of the spaced-apart chemical moieties. In some examples, a self-immolative unit refers to a heterocyclic self-immolative moiety. Typical self-immolative linker units include, but are not limited to, His-Ala, p-aminobenzyloxycarbonyl (PABC), p-hydroxybenzyloxycarbonyl, 2,4-bis(hydroxymethyl)aniline, -NH(CH₂)₄-C(O)- and -NH-(CH₂)₃-C(O)-, etc.

As used herein, the term "cleavable linker" refers to a moiety in the linker unit of an ADC that is labile in vivo. Preferably, a "cleavable linker" allows activation of a label or therapeutic agent by cleavage of the label or agent from the remainder of the conjugate. Operationally defined, a cleavable linker is preferably cleaved by the biological environment in vivo. Cleavage can be by any process without limitation, e.g., enzymatic, reductive, pH, etc. Preferably, the cleavable group is selected such that activation occurs at the desired site of action, which can be at or near a target cell (e.g., cancer cell) or tissue, e.g., the site of therapeutic action or label activity. Such cleavage can be enzymatic, and exemplary enzymatically cleavable groups include naturally occurring amino acids or peptide sequences ending with naturally occurring amino acids, and attached at their carboxyl terminus to the linker unit or self-immolative linker.

As used herein, the term "antibody linker" refers to any chemical group designed to facilitate attachment of the drug conjugate to the antibody.

As used herein, the term "property-modulating unit" refers to a functional moiety linked in series or in a branched manner within the linker unit of the ADC, aimed at modulating the properties of the ADC, such as stability in the bloodstream, improving hydrophilicity, etc. Commonly used property-modulating units for ADCs include, but are not limited to, polyethylene glycol (PEG), hydrophilic peptides, monosaccharides, oligosaccharides, polysaccharides, cyclodextrin units, polyamines, polyamides, dendrimers, and bifunctional hydrocarbon chains. In the ADC of the present disclosure, the property-modulating unit can be linked as a separate component in series within the linker unit, or as a branch off various components within the linker unit, for example, the property-modulating unit can be linked to the self-immolative linker, the cleavable linker, and/or the antibody linker.

As used herein, a specific example of the "property-modulating unit" includes a "solubilizing sugar unit", which refers to a sugar unit attached (directly or indirectly through other structural fragments) to a linker unit, e.g., the self-immolative linker part of the linker. This sugar unit, together with the self-immolative linker, is specifically hydrolyzed in vivo by enzymes such as β-glucuronidase or β-galactosidase, undergoing a self-immolative reaction to release the drug payload. In the antibody-drug conjugates of the present disclosure, introducing a sugar group into the linker unit helps improve the hydrophilicity of the conjugated chain, increases the payload loading rate to the theoretical maximum value, while improving the solubility and purity of the conjugate, reducing aggregation, improving the druggability of the conjugate, and ultimately enhancing the effect of inhibiting tumor cell proliferation.

As used herein, as an example of a property-modulating unit, "monosaccharide" refers to a polyhydroxy aldehyde (aldose) or polyhydroxy ketone (ketose) containing 3 or more carbon atoms and their derivatives. They are the basic structural units constituting sugars and their complexes, cannot be further hydrolyzed, and are not linked to other similar units via glycosidic bonds. Depending on the number of carbon atoms, monosaccharides can be divided into three-carbon sugar (triose), four-carbon sugar (tetrose), five-carbon sugar (pentose), six-carbon sugar (hexose), seven-carbon sugar (heptose), etc. The ADCs of the present disclosure preferably carry pentoses or hexoses. Derivatives of said monosaccharides include, for example, phosphate esters of monosaccharides, sugar alcohols, sugar acids, deoxy sugars, amino sugars, acylated amino sugars, and glycosides. Examples of monosaccharides or their derivatives include, but are not limited to, glyceraldehyde, glyceraldehyde phosphate, dihydroxyacetone phosphate, erythrose, erythrulose, threose, arabinose, ribose, ribulose, deoxyribose, ribitol, ribose phosphate, xylose, xylulose, xylitol, lyxose, glucose, glucosamine, acetylglucosamine, glucuronic acid, acetylglucosaminic acid, glucose phosphate, mannose, mannitol, aminomannitol, acetylmannosamine, fructose, fructose phosphate, galactose, galactitol, galactosamine, acetylgalactosamine, allose, deoxyallose, altrose, deoxyaltrose, quinovose, rhamnose, psicose, sorbose, sorbitol, tagatose, gulose, deoxygulose, idose, talose, fucose, deoxytalose, etc.

It should be noted that the monosaccharides and their derivatives described in the present disclosure include their D-configuration, L-configuration, racemates (DL), and meso forms, as well as any optically active forms ((+), (-), (±)). The monosaccharides and their derivatives described in the present disclosure include their chain isomers, cyclic isomers, or mixtures thereof, wherein the cyclic forms include α-anomeric forms and β-anomeric forms, and include, for example, pyranose or furanose forms.

As used herein, "a disaccharide" as a property-modulating unit refers to a compound formed by linking two monosaccharides via a glycosidic bond, and derivatives thereof. Depending on the linkage mode, there are two types: one is non-reducing sugars formed by dehydration of the hemiacetal (hemiketal) hydroxyl groups of two sugars to form a glycosyl glycoside; the other is reducing sugars formed by glycosidation of the hemiacetal (hemiketal) hydroxyl group of one sugar with the non-hemiacetal (non-hemiketal) hydroxyl group of another sugar, known as glycosyl sugar. The glycosidic bond can be an α-1,4 glycosidic bond, α-1,6 glycosidic bond, β-1,4 glycosidic bond, α-1-2β glycosidic bond, β-1,6 glycosidic bond, α-1,1 glycosidic bond, β-1,3 glycosidic bond, β-2,1 glycosidic bond. Specific examples of disaccharides include, but are not limited to, maltose, isomaltose, lactose, sucrose, chitobiose, rutinose, trehalose, xylobiose, gentiobiose, etc., wherein the monosaccharide units constituting the disaccharide can be the various monosaccharide derivative forms as described above.

As used herein, the term "oligosaccharide" refers to low-polymerization sugars composed of 3~9 monosaccharides linked via glycosidic bonds and derivatives thereof, wherein the monosaccharides, glycosidic bonds, and derivatives are as defined above, e.g., but not limited to, mannotriose, selaginotriose, gentianose, plantose, stachyose, raffinose tetrasaccharide, etc.

As used herein, the term "polysaccharide" refers to a polymer composed of 10 or more monosaccharide units linked via glycosidic bonds. The glycosidic bonds can be α-type, β-type, or α/β mixed. Structurally, they can be linear, branched, or cyclic. Polysaccharides can be homopolysaccharides composed of one type of monosaccharide, wherein the sugar units of said homopolysaccharides are selected from trioses, tetroses, pentoses, hexoses, heptoses, octoses, or deoxy sugar units, such as dextran, xylan, polysialic acid, etc.; or heteropolysaccharides composed of two or more types of monosaccharides, such as hyaluronic acid, heparin, etc. Examples of polysaccharides include, but are not limited to, dextran, levan, hyaluronic acid, cyclodextrins (α, β, γ, etc.), hydroxyethyl starch, xylan, water-soluble starch, water-soluble cellulose, carboxymethyl cellulose, galactosamine, polysialic acid, rhamnan, Ganoderma lucidum polysaccharide, lentinan, chitin, deacetylated chitosan, alginate, carrageenan, gellan gum, pullulan, scleroglucan, xanthan gum, xyloglucan, amylose, etc. Said polysaccharides can inherently bear or be structurally modified to bear one or more substituents selected from, for example, carboxylic acid group, carboxylate group, amino group, sulfonic acid group, sulfonate group, phosphoric acid group, phosphate group, hydroxyethyl group, hydroxypropyl group, methyl group, acyl group, carboxymethyl group, natural amino acid group, unnatural amino acid group, etc.

When describing sugar units, the term "derivative" as used herein primarily refers to phosphate esters of sugars (sugar compounds formed by esterification of one or more hydroxyl groups of a sugar with phosphoric acid), sugar alcohols (sugar compounds formed by reduction of the carbonyl group of a sugar to OH), sugar acids (aldonic acids formed by oxidation of the aldehyde group of an aldose to a carboxyl group, uronic acids formed by oxidation of the primary alcohol group of an aldose to a carboxyl group, and aldaric acids formed by oxidation of both the aldehyde and primary alcohol groups of an aldose to carboxyl groups), deoxy sugars (sugars where one or two hydroxyl groups in the sugar molecule are replaced by hydrogen atoms), amino sugars (sugar derivatives where one or more hydroxyl groups of a sugar are replaced by amino groups), acylated amino sugars (sugar derivatives where the amino group of an amino sugar is acylated), and glycoside forms (sugar-containing derivatives formed by condensation of the hemiacetal hydroxyl group of a sugar with the hydroxyl, amine, or thiol group of another molecule such as an alcohol, sugar, purine, or pyrimidine, i.e., a derivative composed of a sugar residue (sugar lacking the hemiacetal hydroxyl group) and an aglycone linked via a glycosidic bond). Preferred are sugar acids, amino sugars, or acylated amino sugars.

Sugar derivatives suitable for the ADCs of the present disclosure can also be derivatives formed by derivatizing a sugar molecule in more than one of the above ways, for example, acylated amino uronic acids, such as 2-acetamido-2-deoxy-D-galacturonic acid, 2-acetamido-2-deoxy-D-glucuronic acid, 2-acetamido-2-deoxy-D-mannuronic acid.

As used herein, a specific example of the "property-modulating unit" also includes a "PEG unit". The term "PEG unit" refers to an organic moiety containing repeating ethyleneoxy subunits (PEG or PEG subunits), which can be polydisperse, monodisperse, or discrete (i.e., having a discrete number of ethylene-oxy subunits). Polydisperse PEG is a non-uniform mixture of sizes and molecular weights, whereas monodisperse PEG is typically purified from the non-uniform mixture and thus has a single chain length and molecular weight. Preferred PEG units comprise discrete PEG, which are compounds synthesized in a stepwise manner rather than via a polymerization process. Discrete PEG provides a single molecule with a defined and specified chain length.

The PEG unit provided herein includes one or more polyethylene glycol chains, each polyethylene glycol chain consisting of one or more ethyleneoxy subunits covalently linked to each other. Polyethylene glycol chains can be linked together, for example, in a linear, branched, or star configuration. Typically, prior to incorporation into the ADC conjugate, at least one polyethylene glycol chain is derivatized at one end with an alkyl moiety substituted by an electrophilic group to covalently attach to the carbamate nitrogen of a methylene carbamate unit. Typically, the terminal ethyleneoxy group in each polyethylene glycol chain not involved in covalent attachment to the remainder of the linker unit is modified with a PEG capping unit, typically an optionally substituted alkyl group such as -CH₃, CH₂CH₃, or CH₂CH₂CO₂H. Preferred PEG units have a single polyethylene glycol chain with 2 to 24 -CH₂CH₂O- subunits covalently linked in series.

As used herein, a specific example of the "property-modulating unit" also includes a "hydrophilic peptide". The term "hydrophilic peptide" can be attached, for example, to the self-immolative linker of the linker unit, or to the antibody linker. It can be attached directly to the linker unit or via a suitable structural fragment (such as the moiety of formula (A) or (A1) as defined herein). Specifically, a "hydrophilic peptide" refers to an organic moiety comprising repeating subunits -CO-CR'R"-NR-, where one or more amino acids, which may be the same or different from each other, are linked via amide bonds in a linear, branched, or star configuration. Each hydrophilic peptide generally comprises 1-20 amino acids, preferably 4-14 amino acids, more preferably 6-12 amino acids.

As used herein, the amino acid monomers constituting the hydrophilic peptide can be natural amino acids, such as alanine (Ala), arginine (Arg), aspartic acid (Asp), asparagine (Asn), histidine (His), glycine (Gly), glutamic acid (Glu), glutamine (Gln), phenylalanine (Phe), lysine (Lys), leucine (Leu), serine (Ser), tyrosine (Tyr), threonine (Thr), isoleucine (Ile), proline (Pro), tryptophan (Trp), valine (Val), cysteine (Cys), methionine (Met). Correspondingly, in the repeating subunit -CO-CR'R"-NR-, R is H and one of R' and R" is H and the other corresponds to the respective group or moiety in the respective natural amino acid; preferred are polar natural amino acids, such as arginine, serine, threonine, tyrosine, cysteine, aspartic acid, asparagine, glutamic acid, glutamine, lysine, histidine, glycine, tryptophan.

As used herein, the amino acid monomers constituting the hydrophilic peptide can also be amino acids other than the twenty natural amino acids mentioned above, such as ornithine (Orn), β-alanine (β-Ala), citrulline (Cit), sarcosine (Sar), and those amino acids where R, R', R" in the repeating subunit -CO-CR'R"-NR- differ from the corresponding groups or fragments in natural amino acids. For example, R, R', R" are selected from alkyl, aryl, acyl, amido, ketone, azido, hydroxyl, mercapto, hydrazine, cyano, quaternary ammonium, halogen, hydrazide, alkenyl, alkynyl, ether, thiol, seleno, sulfonic acid, sulfate, borate, phosphono, phosphate, heterocycle, enone, imine, aldehyde, ester, thioacid, hydroxylamine, amino, etc., or any combination thereof, or groups containing alkyl, aryl, acyl, amido, ketone, azido, hydroxyl, mercapto, hydrazine, cyano, quaternary ammonium, halogen, hydrazide, alkenyl, alkynyl, ether, thiol, seleno, sulfonic acid, sulfate, borate, phosphono, phosphate, heterocycle, enone, imine, aldehyde, ester, thioacid, hydroxylamine, amino, etc. Preferred are those amino acids where R, R', and/or R" contain hydrophilic groups, such as R, R', R" each independently is carboxyl, sulfonic acid, sulfate, phosphate, amino, amido, quaternary ammonium, oxygen-containing group, ether group, mercapto, or hydroxyl, or is a group containing carboxyl, sulfonic acid, sulfate, phosphate, amino, amido, quaternary ammonium, oxygen-containing group, ether group, mercapto, and/or hydroxyl, such as alkyl, e.g., C₁₋₆ alkyl.

For the ADCs of the present disclosure, when the linker unit carries a hydrophilic peptide, at least 50% to 100% of the amino acids constituting the hydrophilic peptide are hydrophilic amino acids, e.g., 80%-100%, such as 60%, 70%, 80%, 90%, or 100%. Said amino acids are preferably arginine, serine, threonine, tyrosine, cysteine, aspartic acid, asparagine, glutamic acid, glutamine, lysine, histidine, glycine, tryptophan, ornithine, citrulline (Cit), sarcosine (Sar).

For the ADCs of the present disclosure, the hydrophilic peptide carried by the linker unit is preferably 4-14 units of polysarcosine, polyarginine, or polyglycine, more preferably 6-12 units of polysarcosine.

As used herein, the term "drug:antibody ratio" or "DAR" refers to the ratio of the number of drug moieties (D) conjugated to the Ab moiety described herein relative to the Ab moiety in the ADC conjugate. In some embodiments described herein, DAR can be determined by q in formula I, for example, DAR can be an integer or non-integer of at least 1, such as about 1 to 20, e.g., about 2-18, 4-16, 5-12, 6-10, 1-10, 1-8, 2-8, 3-8, 2-6, 4-6, 4-8, 6-8, 6-10, e.g., about 2, 3, 4, 5, 6, 7, 8, 9, 10, 11, 12, 13, 14, or 15. DAR can also be calculated as the average DAR of the product population, i.e., the overall ratio of small molecule drug moieties (D) conjugated to the Ab moiety described herein in the product as measured by detection methods (e.g., by conventional methods such as mass spectrometry, ELISA assay, electrophoresis, and/or HPLC). This DAR is referred to herein as the average DAR. In some embodiments, the average DAR value of the conjugates of the present disclosure is about 1 to 20, e.g., about 2-18, 4-16, 5-12, 1-10, 1-8, 6-10, 2-8, 3-8, 2-6, 4-6, 4-8, 6-8, 6-10, e.g., 1.0-8.0, 2.0-6.0, e.g., about 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8.0, 7.9, 8, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, or 10.0, or a range having two of these values as endpoints.

As used herein, the term "halogen" generally refers to fluorine, chlorine, bromine, iodine, for example, fluorine, chlorine.

As used herein, the term "alkyl" refers to a straight or branched chain aliphatic saturated hydrocarbon group having the specified number of carbon atoms. Specifically, alkyl can have 1 to 14, 1 to 12, 1 to 10, 1 to 8, 1 to 7, 1 to 6, 1 to 5, 1 to 4, 1 to 3, or 1 to 2 carbon atoms. Examples of suitable C₁₋₁₄ alkyl groups include, but are not limited to, methyl, ethyl, n-propyl, isopropyl, n-butyl, isobutyl, sec-butyl, tert-butyl, n-pentyl, isopentyl, neopentyl, n-hexyl, isohexyl, dimethylmethyl, dipropylmethyl, ethylbutylmethyl, diethylmethyl, methylethylmethyl, ethylpropylmethyl, diethylethyl, diethylpropyl, dipropylethyl, etc. Particular alkyl groups have 1 to 7 carbon atoms, e.g., 1 to 6 carbon atoms, 1 to 4 carbon atoms.

As used herein, the term "-O-alkyl" or "alkoxy" means an alkyl group as defined herein attached to the remainder of the molecule through an oxygen atom. Specifically, -O-alkyl has 1-10, e.g., 1 to 8, 1 to 6, 1 to 5, 1 to 4, 1 to 3, or 1 to 2 carbon atoms. For example, as used herein, the term "-O- C₁₋₆ alkyl" refers to a straight or branched chain saturated hydrocarbon group having 1 to 6 carbon atoms attached to the remainder of the molecule through an oxygen atom. Examples thereof include, e.g., - O-methyl, -O-ethyl, -O-propyl (including -O-n-propyl and -O-isopropyl), -O-butyl (including -O-n-butyl, -O-isobutyl, -O-sec-butyl, or -O-tert-butyl), -O-pentyl (including -O-n-pentyl, -O-isopentyl, - O-neopentyl), -O-n-hexyl, 2-methylpentyl-O-, etc.

As used herein, the term " C₁₋₆ alkyl optionally substituted by halogen" refers to the above-mentioned C₁₋₆ alkyl group, wherein one or more (e.g., 1, 2, 3, 4, or 5) hydrogen atoms are optionally replaced by halogen. Those skilled in the art will understand that when more than one halogen substituent is present, the halogens can be the same or different and can be located on the same or different carbon atoms. Examples of "halogen-substituted C₁₋₆ alkyl" include, e.g., -CH₂F, -CHF₂, - CF₃, -CCl₃, -C₂F₅, -C₂Cl₅, -CH₂CF₃, -CH₂Cl, -CH₂CH₂CF₃, or -CF(CF₃)₂, etc.

As used herein, the term "alkenyl" refers to a straight or branched chain unsaturated hydrocarbon group consisting of carbon and hydrogen atoms and containing at least one double bond. Specifically, alkenyl has 2-8, e.g., 2 to 6, 2 to 5, 2 to 4, or 2 to 3 carbon atoms. For example, as used herein, the term " C₂-C₆ alkenyl" refers to an alkenyl group having 2 to 6 carbon atoms, such as ethenyl, propenyl, allyl, 1-butenyl, 2-butenyl, 1,3-butadienyl, 1-pentenyl, 2-pentenyl, 3-pentenyl, 1,3-pentadienyl, 1,4-pentadienyl, 1-hexenyl, 2-hexenyl, 3-hexenyl, 1,4-hexadienyl, etc. The carbon atom of the alkenyl group attached to the remainder of the molecule can be saturated or can be an olefinic carbon atom.

As used herein, the term "alkynyl" refers to a straight or branched chain unsaturated hydrocarbon group consisting of carbon and hydrogen atoms and containing at least one triple bond. Specifically, alkynyl has 2-8, e.g., 2 to 6, 2 to 5, 2 to 4, or 2 to 3 carbon atoms. For example, as used herein, the term "C₂₋₆ alkynyl" refers to an alkynyl group having 2 to 6 carbon atoms, such as ethynyl, propynyl, propargyl, 1-butynyl, 2-butynyl, 1-pentynyl, 2-pentynyl, 3-pentynyl, 4-methyl-1-pentynyl, 1-hexynyl, 2-hexynyl, 3-hexynyl, 5-methyl-2-hexynyl, etc. The carbon atom of the alkynyl group attached to the remainder of the molecule can be saturated or can be an acetylenic carbon atom.

As used herein, the term "alkylene" refers to a divalent group obtained by removing two hydrogen atoms from the same or two different carbon atoms of a straight or branched chain saturated alkane. Specifically, alkylene has 1-10 carbon atoms, e.g., 1 to 6, 1 to 5, 1 to 4, 1 to 3, or 1 to 2 carbon atoms. For example, as used herein, the term " C₁₋₆ alkylene" refers to a straight or branched chain alkylene group having 1 to 6 carbon atoms, including but not limited to methylene, ethylene, propylene, butylene, etc.

As used herein, a specific type of "alkylene" refers to a divalent group obtained by removing two hydrogen atoms from the same carbon atom of a straight or branched chain saturated alkane, such as the =C(R^{d})₂ group defined in the drug P part of the present disclosure, as shown in the structural fragment

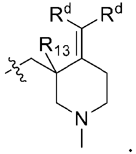

As used herein, the term "alkenylene" refers to a divalent group obtained by removing two hydrogen atoms from the same or two different carbon atoms of a straight or branched chain unsaturated alkene containing at least one double bond. Specifically, alkenylene has 2-8, e.g., 2 to 6, 2 to 5, 2 to 4, or 2 to 3 carbon atoms. For example, as used herein, the term "C₂₋₆ alkenylene" refers to a straight or branched chain alkenylene group having 2 to 6 carbon atoms, such as ethenylidene, propenylene, allylene, butenylene, pentenylene, and hexenylene.

As used herein, the term "alkynylene" refers to a divalent group obtained by removing two hydrogen atoms from the same or two different carbon atoms of a straight or branched chain unsaturated alkyne containing at least one triple bond. Specifically, alkynylene has 2-8, e.g., 2 to 6, 2 to 5, 2 to 4, or 2 to 3 carbon atoms. For example, as used herein, the term "C₂-C₆ alkynylene" refers to a straight or branched chain alkynylene group having 2 to 6 carbon atoms, such as ethynylene, propynylene, propargylene, butynylene, pentynylene, and hexynylene.

As used herein, the term "cycloalkyl" refers to a monocyclic, fused polycyclic, bridged polycyclic, or spirocyclic non-aromatic monovalent hydrocarbon ring structure having the specified number of ring atoms, which can be saturated or unsaturated, e.g., containing one or more double bonds. Cycloalkyl groups can contain 3 or more carbon atoms in the ring, e.g., 3-18, 3-10, or 3-8 carbon atoms, such as C₃₋₁₀ cycloalkyl, C₃₋₈ cycloalkyl, C₃₋₆ cycloalkyl, C₅₋₆ cycloalkyl. Examples of cycloalkyl groups include, but are not limited to, cyclopropyl, cyclobutyl, cyclopentyl, cyclohexyl, cycloheptyl.

As used herein, the term "heterocycle" or "heterocyclyl" refers to a 5-20 membered (e.g., 5-14 membered, 5-8 membered, 5-6 membered) aromatic or non-aromatic monocyclic, bicyclic, or polycyclic ring system having 1-4 ring heteroatoms independently selected from N, O, or S. One or more N, C, or S atoms in the heterocycle can be oxidized. Preferably, the heterocycle is a 5-10 membered ring system, monocyclic or fused bicyclic. Representative examples include, but are not limited to, pyrrolidine, azetidine, piperidine, morpholine, tetrahydrofuran, tetrahydropyran, benzofuran, benzothiophene, indole, benzopyrazole, pyrrole, thiophene, furan, thiazole, imidazole, pyrazole, pyrimidine, pyridine, pyrazine, pyridazine, isothiazole, and isoxazole.

As used herein, the term "heterocycloalkyl" means a monocyclic, fused polycyclic, spirocyclic, or bridged polycyclic non-aromatic saturated ring structure including one or more (e.g., 1, 2, 3, or 4) heteroatoms independently selected from O, N, and S, having the specified number of ring atoms, or an N-oxide thereof, or an S-oxide or S-dioxide thereof. Heterocycloalkyl can have 3 to 12 ring members (can be referred to as 3-12 membered heterocycloalkyl), e.g., 3 to 10 ring members, 3 to 8 ring members, 3 to 7 ring members, 4 to 7 ring members, 4 to 6 ring members, 5 to 6 ring members. Heterocycloalkyl typically contains up to 4 (e.g., 1, 2, 3, or 4) heteroatoms, e.g., a 4-7 membered heterocycloalkyl containing 1 to 3 heteroatoms selected from N, O, S. Examples of suitable heterocycloalkyl groups include, but are not limited to, azetidinyl, oxetanyl, thietanyl, pyrrolidinyl (e.g., 1-pyrrolidinyl, 2-pyrrolidinyl, and 3-pyrrolidinyl), tetrahydrofuranyl (e.g., 1-tetrahydrofuranyl, 2-tetrahydrofuranyl, and 3-tetrahydrofuranyl), tetrahydrothienyl (e.g., 1-tetrahydrothienyl, 2-tetrahydrothienyl, and 3-tetrahydrothienyl), piperidinyl (e.g., 1-piperidinyl, 2-piperidinyl, 3-piperidinyl, and 4-piperidinyl), tetrahydropyranyl (e.g., 4-tetrahydropyranyl), tetrahydrothiopyranyl (e.g., 4-tetrahydrothiopyranyl), morpholinyl (e.g., morpholino), thiomorpholinyl, dioxanyl, piperazinyl, or azepanyl, diazepanyl such as 1,4-diazepanyl, 3,6-diaza-bicyclo[3.1.1]heptyl, or 3-aza-bicyclo[3.2.1]octyl. The atom of the heterocycloalkyl group attached to the remainder of the compound can be a carbon atom or a heteroatom, as long as it is chemically feasible.

As used herein, the term "aryl" refers to a monocyclic or polycyclic aromatic hydrocarbon group having 6-20, e.g., 6-12, carbon atoms in the ring portion. Preferably, aryl is (C₆-C₁₀)aryl. Non-limiting examples include phenyl, biphenyl, naphthyl, or tetrahydronaphthyl, each of which can be optionally substituted by 1-4 substituents such as alkyl, trifluoromethyl, cycloalkyl, halogen, hydroxy, alkoxy, acyl, alkyl-C(O)-O-, aryl-O-, heteroaryl-O-, amino, mercapto, alkyl-S-, aryl-S-, nitro, cyano, carboxy, alkyl-O-C(O)-, carbamoyl, alkyl-S(O)-, sulfonyl, sulfonylamino, heterocyclyl, etc.

As used herein, the term "heteroaryl" refers to a 5-20 membered (e.g., 5-14 membered, 5-8 membered, 5-6 membered) aromatic monocyclic or polycyclic ring system containing 1-4 heteroatoms selected from N, O, or S, which can be substituted or unsubstituted. Preferably, heteroaryl is a 5-10 membered ring system, monocyclic or fused bicyclic. Representative heteroaryl groups include 2- or 3-thienyl, 2- or 3-furyl, 2- or 3-pyrrolyl, 2-, 4- or 5-imidazolyl, 3-, 4- or 5-pyrazolyl, 2-, 4- or 5-thiazolyl, 3-, 4- or 5-isothiazolyl, 2-, 4- or 5-oxazolyl, 3-, 4- or 5-isoxazolyl, 3- or 5-1,2,4-triazolyl, 4- or 5-1,2,3-triazolyl, tetrazolyl, 2-, 3- or 4-pyridyl, 3- or 4-pyridazinyl, 3-, 4- or 5-pyrazinyl, 2-pyrazinyl, 2-, 4- or 5-pyrimidinyl.

As used herein, the term "heteroalkyl" refers to a stable straight or branched chain hydrocarbon that is fully saturated or contains 1 to 3 degrees of unsaturation, consisting of the indicated number of carbon atoms and one to ten, preferably one to three, heteroatoms selected from O, N, Si, and S, wherein the nitrogen and sulfur atoms can be optionally oxidized and the nitrogen heteroatom can be optionally quaternized. The heteroatoms O, N, Si, and S can be placed at any internal position of the heteroalkyl group or at the position where the heteroalkyl group is attached to the remainder of the molecule. Representative examples of heteroalkyl include -CH₂-CH₂-O-CH₃, -CH₂-CH₂-NH-CH₃, - CH₂-CH₂-N(CH₃)-CH₃, -CH₂-S-CH₂-CH₃, --CH₂-CH₂-S(O)-CH₃, --NH-CH₂-CH₂-NH-C(O)-CH₂-CH₃, -CH₂-CH₂-S(O)₂-CH₃, -CH=CH-O-CH₃, -Si(CH₃)₃, -CH₂-CH=N-O-CH₃, and -CH=CH-N(CH₃)-CH₃. Up to two heteroatoms can be consecutive, such as -CH₂-NH-OCH₃ and -CH₂-O-Si(CH₃)₃. Typically, C1 to C4 heteroalkyl or heteroalkylene has 1 to 4 carbon atoms and 1 or 2 heteroatoms, C1 to C3 heteroalkyl or heteroalkylene has 1 to 3 carbon atoms and 1 or 2 heteroatoms. In some aspects, heteroalkyl and heteroalkylene are saturated.

Unless otherwise indicated, the term "substituted" when used to define various groups means that the corresponding group can be substituted by, for example but not limited to, the following groups as defined herein or conventional in the art: alkyl, alkenyl, alkynyl, cycloalkyl, aryl, heteroaryl, heterocyclyl, halogen, cyano, nitro, azido, carboxy, hydroxy, mercapto, amino, mono- or di-alkylamino, mono- or di-cycloalkylamino, mono- or di-arylamino, mono- or di-heterocyclylamino, mono- or di-heteroarylamino, alkyl- or cycloalkyl- or heterocyclyl- or heteroaryl- or aryl-oxy, alkyl- or cycloalkyl- or heterocyclyl- or heteroaryl- or aryl-thio, alkyl- or cycloalkyl- or heterocyclyl- or heteroaryl- or aryl-acyl, alkyl- or cycloalkyl- or heterocyclyl- or heteroaryl- or aryl-acylamino, alkyl- or cycloalkyl- or heterocyclyl- or heteroaryl- or aryl-acyloxy, alkyl- or cycloalkyl- or heterocyclyl- or heteroaryl- or aryl-sulfonyl, alkyl- or cycloalkyl- or heterocyclyl- or heteroaryl- or aryl-sulfonyloxy, alkyl- or cycloalkyl- or heterocyclyl- or heteroaryl- or aryl-sulfonylamino, or the above optionally substituted amino-carbamoyl, and groups thereof further substituted by the remaining optional substituents, wherein the various groups are as defined herein. Examples of substituents include, but are not limited to, one or more groups independently selected from: halogen, OH, SH, CN, NH₂, NHCH₃, N(CH₃)₂, NO₂, N₃, C(O)CH₃, COOH, C(O)-amino, OCOCH₃, methyl, ethyl, propyl, iso-propyl, butyl, isobutyl, sec-butyl, tert-butyl, cyclopropyl, methoxy, ethoxy, propoxy, oxo, trifluoromethyl, difluoromethyl, sulfonylamino, methylsulfonylamino, SO, SO₂, phenyl, piperidinyl, piperazinyl, and pyrimidinyl.

As used herein, the term "substitution" or "substituted" means that one or more (e.g., 1, 2, 3, or 4) hydrogens on the designated atom are replaced with the designated groups, provided that the normal valence of the designated atom is not exceeded under the current circumstances and a stable compound is formed. Combinations of substituents and variables are permissible only if such combinations result in stable compounds.

The term "optionally substituted" as used herein, unless otherwise indicated, means that a group can be unsubstituted or substituted by one or more (e.g., 1, 2, 3, 4, or 5 or more, or any range derivable therein) of the substituents listed for that group, wherein the substituents can be the same or different. In one embodiment, an optionally substituted group has 1 substituent. In another embodiment, an optionally substituted group has 2 identical or different substituents. In another embodiment, an optionally substituted group has 3 identical or different substituents. In another embodiment, an optionally substituted group has 4 identical or different substituents. In another embodiment, an optionally substituted group has 5 identical or different substituents.

Many of the groups defined herein are optionally substituted, and the lists of substituents provided in the specific group definitions are not intended to limit the substituents defined elsewhere in the specification and claims.

As used herein, the term "pharmaceutically acceptable salt" represents a salt that retains the biological effectiveness and properties of the ADC conjugate of the present disclosure and is not biologically or otherwise undesirable. The ADC conjugates of the present disclosure can exist in the form of their pharmaceutically acceptable salts, including acid addition salts and base addition salts. In the present disclosure, pharmaceutically acceptable non-toxic acid addition salts represent salts formed by the ADC conjugates of the present disclosure with organic or inorganic acids, including but not limited to hydrochloric acid, sulfuric acid, hydrobromic acid, hydroiodic acid, phosphoric acid, nitric acid, perchloric acid, acetic acid, oxalic acid, maleic acid, fumaric acid, tartaric acid, benzenesulfonic acid, methanesulfonic acid, salicylic acid, succinic acid, citric acid, lactic acid, propionic acid, benzoic acid, p-toluenesulfonic acid, malic acid, etc. Pharmaceutically acceptable non-toxic base addition salts represent salts formed by the ADC conjugates of the present disclosure with organic or inorganic bases, including but not limited to alkali metal salts, e.g., lithium, sodium, or potassium salts; alkaline earth metal salts, e.g., calcium or magnesium salts; organic base salts, e.g., ammonium salts formed with organic bases containing N groups.

As used herein, the term "solvate" represents an association formed by one or more solvent molecules with the ADC conjugate of the present disclosure. Solvents forming solvates include, but are not limited to, water, methanol, ethanol, isopropanol, ethyl acetate, tetrahydrofuran, N,N-dimethylformamide, dimethyl sulfoxide, etc. It should be understood that such solvates of the compounds of the present invention also include solvates of the pharmaceutically acceptable salts of the compounds of the present invention.

As used herein, the term "isotopic variant" refers to a compound in which one or more atoms constituting the compound are replaced with atoms having an atomic mass or mass number different from that normally found in nature. Examples of isotopes that can be incorporated into one or more atoms of the compounds of the present disclosure include, for example, ²H, ³H, ¹³C, ¹⁴C, ¹⁵N, ¹⁷O, ¹⁸O, ³¹P, ³²P, ³⁵S, and ¹⁸F, thereby forming isotopic variants of the compounds of the present disclosure, whether or not they are radioactive, and are intended to be encompassed within the scope of the present disclosure. In certain embodiments, the compounds of the present disclosure are provided in unlabeled form, while in other embodiments, they are provided in isotopically labeled form, such as the hydrogen isotope D-labeled form. In particular, one or more H atoms in the R⁹, R¹⁰, and R¹¹ groups defined in the drug P part herein can be replaced with the isotope D, for example, R⁹ and R¹⁰ can each independently be H or D, and R¹¹ can be substituted by one or more D atoms, particularly a -C₁₋₆ alkyl substituted by one or more D atoms.

As used herein, the term "isomer" refers to any stereoisomer, enantiomeric mixture, including racemates, diastereomeric mixtures, geometric isomers, atropisomers, and/or tautomers that may exist structurally for a compound. The methods for determining and separating the stereochemistry of such isomers are well known to those skilled in the art (S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994). The present disclosure encompasses all possible isomeric forms of the compounds defined herein, and their pharmaceutically acceptable salts or solvates, unless otherwise indicated. Furthermore, the compounds of the present disclosure can exist as mixtures of two or more different structures in rapid equilibrium (commonly referred to as tautomers). It is to be understood that the scope of the present application covers all such isomers or mixtures thereof in any ratio (e.g., 60%, 65%, 70%, 75%, 80%, 85%, 90%, 95%, 96%, 97%, 98%, 99%).

Certain compounds of the present disclosure contain at least one (e.g., 1, 2, 3, or 4) asymmetric center, and therefore can be prepared as (R)- or (*S*)-stereoisomers or as mixtures thereof.

The use of " 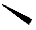 " or " 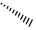 " in the structural formulas or structural fragments of the compounds of the present disclosure indicates the configuration of the stereocenter, i.e., the chiral center. Correspondingly, in the naming of the compounds or intermediates provided by the present invention, the configuration with respect to that chiral center is represented by R or S; " 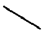 " attached to a chiral center indicates the racemic form in which both configurations at that chiral center are present simultaneously, such as 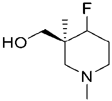 representing a mixture of 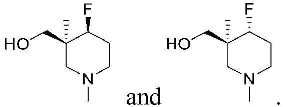 **In** some definitions of the compounds of the present disclosure, axial chirality can also be used to represent the configuration of the compound. The determination of these configurations uses the Cahn-Ingold-Prelog rules well known to those skilled in the art. The absolute configuration of axial chirality in the two exemplary structures below is described as follows: 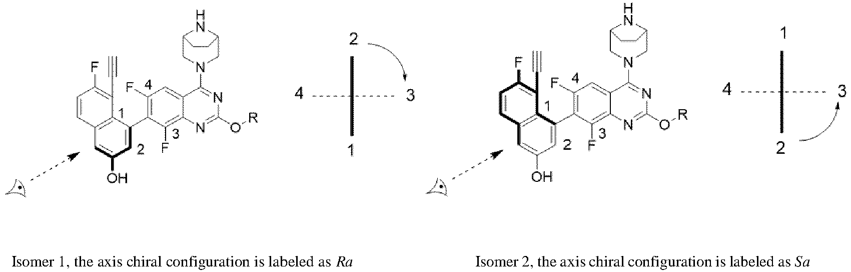 When the bond linking the axially chiral center is marked with "*", it indicates that the compound is of a single chiral configuration obtained by SFC resolution, but the absolute configuration is uncertain. For example, 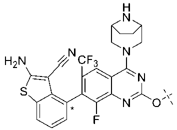 means

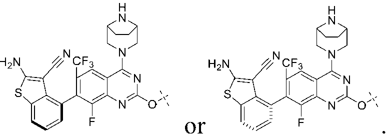

It should be understood that when a person skilled in the art can determine, based on the compound structures shown herein, that a compound exists as a pair of chiral isomers and that the isomers can be readily resolved based on conventional methods in the art, then the disclosure of the racemate of that compound herein (whether by structural formula or chemical name) should be deemed to have separately disclosed each individual isomer of that compound.

As used herein, in the definitions, structural formulas, or structural fragments of compounds, the number of groups attached to each atom depends on the chemical valence of that atom and need not be fully shown. Generally speaking, only non-hydrogen groups are shown in the group definitions, structural formulas, or structural fragments; unshown groups generally represent H. A person skilled in the art can readily determine whether unshown groups are present and their number.

The " 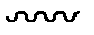 " used in the structural fragments involved herein indicates that the bond crossing it is the bond connecting that structural fragment to the remainder of the molecule.

Substituents shown across chemical bonds in cyclic structural fragments involved herein, such as -(R₁₂)ₘ in 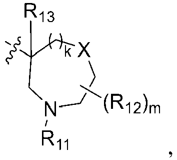 indicate that the m of R₁₂ substituents can be substituted at any chemically feasible substitution site(s) on the ring, including X when chemically feasible.

Unless otherwise specified, Cₙ₋ₙ₊ₘ or Cₙ-Cₘ in the definitions of the compounds of the present invention includes various cases of n to n+m carbons, for example, C₁₋₆ includes C₁, C₂, C₃, C₄, C₅, and C₆, and also includes any range within n to n+m, for example, C₀₋₆ includes C₁, C₂, C₃, C₄, C₅, C₆, C₀₋₁, C₀₋₂, C₀₋₃, C₀₋₄, C₀₋₅, C₁₋₂, C₁₋₃, C₁₋₄, C₂₋₃, etc.; C₁₋₆ includes C₁₋₂, C₁₋₃, C₁₋₄, C₂₋₆, C₃₋₆, etc.

As used herein, the term "about" used to modify a numerical value, numerical range, or parameter means that the value or parameter associated with it fluctuates by ±10%, such as ±5%, ±2%, or ±1%. By way of example, the expression "about 100" as used herein includes 90 and 110 and all values in between (e.g., 90.5, 95, 101, 105, 109.95, etc.). For ratios, the term "about" is used to modify each number of the given ratio, for example, a ratio of "about 1:1" means the ratio is (0.9-1.1):(0.9-1.1); and a range of "about n-m" or "about n to about m" means a range from 90% n-110% n to 90% m-110% m.

As used herein, "pharmaceutically acceptable" and "pharmaceutical" are used interchangeably as the context allows and without contradiction.

As used herein, the term "pharmaceutical composition" refers to a composition that is in a form effective for the biological activity of the active ingredients contained therein and contains no additional ingredients that are unacceptably toxic to a subject to whom the composition is administered.

As used herein, the terms "pharmaceutically acceptable excipient", "pharmaceutically acceptable carrier", and "therapeutically inert excipient" are used interchangeably and represent any pharmaceutically acceptable ingredient in a pharmaceutical composition that has no therapeutic activity and is non-toxic to the subject administered, such as disintegrants, binders, fillers, solvents, buffers, tonicity agents, stabilizers, antioxidants, surfactants, carriers, diluents, or lubricants used to formulate pharmaceutical products.

As used herein, the term "pharmaceutical combination" refers to a non-fixed combination product or a fixed combination product, including but not limited to kits, pharmaceutical compositions. The term "non-fixed combination" means that the active ingredients, e.g., (i) the ADC conjugate of the present disclosure and (ii) the other therapeutic agent, are administered to a patient as separate entities simultaneously, without specific time limits, or sequentially at the same or different time intervals, wherein such administration provides the patient with prophylactically or therapeutically effective levels of the two or more active agents. In some embodiments, the ADC conjugate of the present disclosure and the other therapeutic agent used in the pharmaceutical combination are administered at levels not exceeding those at which they are used alone. The term "fixed combination" means that two or more active agents are administered to a patient simultaneously as a single entity. The doses and/or time intervals for the two or more active agents are preferably selected so that their combined use produces an effect greater than that achievable with either component alone in treating a disease or condition. Each component can be in separate formulation forms, which can be the same or different.

As used herein, the term "combination therapy" refers to the administration of two or more therapeutic agents or treatment modalities, such as radiation therapy or surgery, to treat a disease described herein. Such administration includes co-administering these therapeutic agents in a substantially simultaneous manner, e.g., in a single capsule with a fixed ratio of active ingredients. Alternatively, such administration includes co-administration of each active ingredient in multiple or separate containers (e.g., tablets, capsules, powders, and liquids). Powders and/or liquids can be reconstituted or diluted to the desired dose prior to administration. Furthermore, such administration also includes sequential use of each type of therapeutic agent at substantially the same time or at different times. In either case, the treatment regimen will provide a beneficial effect of the drug combination in treating the condition or disease described herein.

As used herein, the terms "individual" or "subject" are used interchangeably and refer to a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In particular, the subject is a human.

As used herein, the term "treatment" refers to slowing, interrupting, arresting, alleviating, stopping, reducing, or reversing the progression or severity of an existing symptom, disorder, condition, or disease.

As used herein, the term "prevention" includes inhibition of the occurrence or development of a disease or condition or a symptom of a particular disease or condition. In some embodiments, a subject with a family history of cancer is a candidate for a prophylactic regimen. Generally, in the context of cancer, the term "prevention" refers to drug administration before the onset of signs or symptoms of cancer, particularly in subjects at risk for cancer.

As used herein, the term "effective amount" refers to such an amount or dose of the antibody-drug conjugate or composition or combination of the present disclosure that, when administered to a patient in a single or multiple doses, produces the desired effect in the patient in need of treatment or prevention.

As used herein, the term "therapeutically effective amount" refers to an amount effective to achieve the desired therapeutic result at the required doses and for the required period of time. A therapeutically effective amount is also one in which any toxic or harmful effects of the antibody-drug conjugate or composition or combination of the present disclosure are outweighed by the therapeutic benefits. Relative to an untreated individual, a "therapeutically effective amount" preferably achieves at least about 30%, even more preferably at least about 40%, 45%, 50%, 55%, 60%, 65%, 70%, 75%, 80%, 85%, 90%, or even 100% inhibition of a measurable parameter (e.g., tumor volume).

As used herein, the term "prophylactically effective amount" refers to an amount effective to achieve the desired prophylactic result at the required doses and for the required period of time. Typically, since a prophylactic dose is used in a subject prior to or at an earlier stage of the disease, the prophylactically effective amount will be less than the therapeutically effective amount.

As used in this specification and the appended claims, the term "comprise" and variations thereof such as "comprising" and "includes" mean "including but not limited to" and are not intended to exclude, for example, other additives, components, integers, or steps. When an element is described as comprising multiple components, steps, or conditions, it is to be understood that the element can also be described as comprising any combination of the multiple components, steps, or conditions, or as "consisting of" or "consisting essentially of" the multiple or combined components, steps, or conditions.

### I: Antibody-Drug Conjugate

In one aspect, the present disclosure provides an antibody-drug conjugate (ADC) of formula (X) or a pharmaceutically acceptable salt or solvate thereof:

[P-L]_{q}-Ab (X)

wherein,
P represents a Ras inhibitor, e.g., a KRas inhibitor;
L represents a linker unit connecting P to Ab;
q represents the number of [P-L] units connected to Ab, which is an integer or non-integer of at least 1, e.g., q = 1 to 20, such as about 1-10, 1-8, 2-8, 3-10, 3-8, 4-10, 4-8, 6-8, or 6-10;
Ab represents an antibody or antigen-binding fragment.

In some embodiments of formula X, q represents an integer selected from 1 to 20, including 1, 2, 3, 4, 5, 6, 7, 8, 9, 10, 11, 12, 13, 14, 15, 16, 17, 18, 19, or 20. In some cases, q ranges from 1 to 2, 1 to 3, 1 to 4, 1 to 5, 1 to 6, 1 to 7, 1 to 8, 1 to 9, 1 to 10, 1 to 11, 1 to 12, 1 to 13, 1 to 14, 1 to 15, 1 to 16, 1 to 17, 1 to 18, 1 to 19, or 1 to 20, or is a range formed by any two values between 1 and 20, e.g., 2 to 10, 2 to 8, 2 to 6, 2 to 4, 3 to 8, 3 to 10, 4 to 6, 4 to 8, 4 to 10, 6 to 8, or 6 to 10. In other embodiments, formula X describes an ADC in an ADC mixture, which exhibits a q value ranging from 1 to 2, 1 to 3, 1 to 4, 1 to 5, 1 to 6, 1 to 7, 1 to 8, 1 to 9, 1 to 10, 1 to 11, 1 to 12, 1 to 13, 1 to 14, 1 to 15, 1 to 16, 1 to 17, 1 to 18, 1 to 19, or 1 to 20, or a range formed by any two values between 1 and 20, e.g., 2 to 10, 2 to 8, 2 to 6, 2 to 4, 3 to 8, 3 to 10, 4 to 6, 4 to 8, 4 to 10, 6 to 8, or 6 to 10.

In certain embodiments, formula X describes an ADC in an ADC mixture such that more than 60%, 65%, 70%, 75%, 80%, 85%, 90%, or 95% of the ADCs in the mixture have a q value of about 1, 2, 3, 4, 5, 6, 7, 8, 9, 10, 11, 12, 13, 14, 15, 16, 17, 18, 19, or 20. In some embodiments, formula X describes an ADC in an ADC mixture such that more than 60%, 65%, 70%, 75%, 80%, 85%, 90%, or 95% of the ADCs in the mixture have a q value ranging from 1 to 2, 1 to 3, 1 to 4, 1 to 5, 1 to 6, 1 to 7, 1 to 8, 1 to 9, 1 to 10, 1 to 11, 1 to 12, 1 to 13, 1 to 14, 1 to 15, 1 to 16, 1 to 17, 1 to 18, 1 to 19, or 1 to 20, or a range formed by any two values between 1 and 20, e.g., 2 to 10, 2 to 8, 2 to 6, 2 to 4, 3 to 8, 3 to 10, 4 to 6, 4 to 8, 4 to 10, 6 to 8, or 6 to 10.

In other embodiments, formula X describes an ADC mixture, in which case q is "q_{avg}", which represents the average of the q values of the mixture, or the average DAR, i.e., the average number of linker units (L) connected to a given antibody (Ab) in the mixture. In such embodiments, q_{avg} or average DAR represents an integer or non-integer value from 1 to 20, e.g., about 1 to 2, 1 to 3, 1 to 4, 1 to 5, 1 to 6, 1 to 7, 1 to 8, 1 to 9, 1 to 10, 1 to 11, 1 to 12, 1 to 13, 1 to 14, 1 to 15, 1 to 16, 1 to 17, 1 to 18, 1 to 19, or 1 to 20, or an integer or decimal within a range formed by any two values between 1 and 20, e.g., about 2 to 10, 2 to 8, 2 to 6, 2 to 4, 3 to 8, 3 to 10, 4 to 6, 4 to 8, 4 to 10, 6 to 8, or 6 to 10.

In preferred embodiments, q in formula (X) of the present disclosure is an integer or non-integer from 1 to 10 or a range formed by any two values between 1 and 10, e.g., about 1, 2, 3, 4, 5, 6, 7, 8, 9, 10, about 1 to 2, 1 to 3, 1 to 4, 1 to 5, 1 to 6, 1 to 7, 1 to 8, 1 to 9, 1 to 10, 2 to 10, 2 to 8, 2 to 6, 2 to 4, 4 to 6, 3 to 8, 3 to 10, 4 to 8, 4-10, 6 to 8, or 6 to 10.

In some embodiments, q represents an average DAR of about 3. In some embodiments, q represents an average DAR of about 6. In some embodiments, q represents an average DAR of about 8.

In some embodiments, q represents an average DAR of about 1-10, 1-8, 2-8, 3-10, 3-8, 4-10, 4-8, 6-8, or 6-10.

Those skilled in the art will also understand that the various ADCs described herein can be in the form of salts or solvates, and in some specific embodiments, are pharmaceutically acceptable salts.

The various components of the ADC conjugates of the present disclosure and the ADC conjugates of the present disclosure composed thereof are described in detail below. Those skilled in the art will understand that the ADCs disclosed herein are essentially "modular" in nature, as each has the modular components Ab, L, and P described above. Throughout this disclosure, various specific non-limiting examples and illustrations of these modular components are described, and the present disclosure encompasses all specific combinations of all module-specific embodiments as if each specific combination were explicitly described individually.

### Ab-Antibody or Antigen-Binding Fragment

In the ADC of the present disclosure, the antibody is an antibody or antigen-binding fragment that specifically binds to a target antigen. Its function is to target and deliver the KRas mutant inhibitor compound to a specific target cell population. Due to the presence of the targeting component or molecule, the antibody or antigen-binding fragment interacts with the specific target cell population, and subsequently releases the free drug within the target cell (intracellular mode) or in the vicinity of the target cell (extracellular mode).

In one set of embodiments, the antibody or antigen-binding fragment is bonded to a linker unit that comprises a releasable peptide component. As noted above, other linking components may also be present in the conjugates described herein to provide additional space between the Ras, e.g., KRas mutant, inhibitor compound and the antibody unit, or to provide increased solubility properties to the composition. In some of these embodiments, the antibody or antigen-binding fragment is bonded to the linker unit via a heteroatom thereof. Heteroatoms that may be present on the antibody or antigen-binding fragment for such bonding include sulfur (in one embodiment, from a thiol group of the targeting ligand), oxygen (in one embodiment, from a carboxyl or hydroxyl group of the targeting ligand), and optionally substituted nitrogen (in one embodiment, from a primary or secondary amine functional group of the targeting ligand, or in another embodiment, from an optionally substituted amide nitrogen). These heteroatoms can be present on the antibody unit in the natural state of the ligand, e.g., in a naturally occurring antibody, or can be introduced into the antibody unit by chemical modification or bioengineering.

The conjugation site on the antibody affects properties such as ADC stability, pharmacokinetics, and pharmacodynamics. Excessively high drug loading can sometimes lead to rapid plasma clearance, while ADCs with low DAR (drug-to-antibody ratio) may exhibit weak activity. The drug loading on the antibody can be controlled by selecting the conjugation strategy and the conjugation site on the antibody, while maintaining the structural integrity and homogeneity of the antibody.

In some embodiments, the antibody or antigen-binding fragment has a thiol functional group such that it is bonded to the linker unit via the sulfur atom of the thiol functional group. In other embodiments, the thiol group is generated by reducing interchain disulfides of the antibody. Thus, in some embodiments, the linker unit is conjugated to a cysteine residue derived from a reduced interchain disulfide in the antibody. In other embodiments, the thiol group is introduced chemically into the antibody, e.g., by introducing a cysteine residue. Accordingly, in some embodiments, the linker unit is conjugated to the antibody or antigen-binding fragment via a cysteine residue introduced into the antibody or antigen-binding fragment.

In other embodiments, the antibody or antigen-binding fragment has one or more lysine residues that can react with an activated ester (including but not limited to N-hydroxysuccinimide, pentafluorophenyl, and p-nitrophenyl ester) in the linker unit, and thereby provide an amide bond consisting of the nitrogen atom from the antibody or antigen-binding fragment and the C=O of the linker unit.

In other embodiments, the antibody or antigen-binding fragment has one or more lysine residues that can be chemically modified to introduce one or more thiol groups. In these embodiments, the antibody or antigen-binding fragment is covalently attached to the linker unit via the sulfur atom of the thiol functional group. Reagents that can be used to modify lysine in this way include, but are not limited to, N-succinimidyl-S-acetylthioacetate (SATA) and 2-iminothiolane hydrochloride (Traut's reagent).

In other embodiments, the antibody or antigen-binding fragment has one or more carbohydrate groups that can be modified to provide one or more thiol functional groups. The chemically modified antibody or antigen-binding fragment in the ADC is bonded to the linker unit via the sulfur atom of the thiol functional group.

In other embodiments, the antibody or antigen-binding fragment has one or more carbohydrate groups that can be oxidized to provide aldehyde (-CHO) functional groups. In these embodiments, the corresponding aldehyde interacts with a reactive site on the linker unit to form a chemical bond between the linker unit and the antibody unit.

In other embodiments, artificial attachment sites are introduced into the antibody to achieve more site-specific conjugation.

Other protocols for modifying proteins for attachment to linker units or related substances can be found in Coligan et al., Current Protocols in Protein Science, Vol. 2, John Wiley & Sons (2002), which are incorporated herein by reference.

In some embodiments, the antibody or antigen-binding fragment is capable of forming a covalent bond between the linker unit and the antibody or antigen-binding fragment corresponding to the antibody unit by interacting with a reactive functional group on the linker unit. The functional group capable of interacting with the antibody unit will depend on the nature of the antibody or antigen-binding fragment. In some embodiments, the reactive group is maleimide. Covalent attachment of the antibody or antigen-binding fragment to the linker unit is achieved through interaction of a thiol functional group of the antibody or antigen-binding fragment with a maleimide functional group of the linker unit, forming a thio-substituted succinimide. The thiol functional group can be present in the natural state of the antibody or antigen-binding fragment, e.g., in naturally occurring residues, or can be introduced into the antibody or antigen-binding fragment by chemical modification or by bioengineering.

The antibody constituting the ADC of the present disclosure can be polyclonal, monoclonal, genetically engineered, and/or otherwise modified, suitable for administration to humans, e.g., humanized antibodies or fully human antibodies.

In some embodiments, the Ab unit of the ADC of the present disclosure is a monospecific antibody. In some embodiments, the Ab unit of the ADC of the present disclosure is a multispecific antibody. In some embodiments, upon binding to an antigen receptor expressed on the surface of tumor cells, the Ab unit triggers antigen receptor-mediated endocytosis, thereby effectively delivering the anti-tumor drug of the ADC into the tumor cells.

In some embodiments, the Ab unit of the ADC of the present disclosure can be a bispecific antibody, a dual variable domain antibody, a multi-chain or single-chain antibody, a single-domain antibody, a camelized antibody, a scFv-Fc antibody, a surrogate antibody, etc. In some embodiments where the Ab unit is a bispecific antibody, one specificity of the antibody can target a tumor-associated antigen to promote specific binding of the ADC to tumor cells, while the other specificity of the antibody can target a tumor cell surface receptor to further promote internalization and degradation of the ADC. Non-limiting examples of such bispecific target combinations include HER2 and PRLR dual targeting on breast cancer cells. In other embodiments, the two specificities of the antibody can target different tumor-associated antigens, respectively, to provide a mechanism against drug resistance. Non-limiting examples of such bispecific target combinations include EGFR and MET dual targeting on lung cancer cells. In other embodiments, the two specificities of the antibody can also target different epitopes of the same tumor-associated antigen to increase antibody selectivity for cancer cells and/or enhance internalization and transport to lysosomes by inducing clustering and cross-linking of the antigen on the tumor cell surface. Non-limiting examples of such tumor-associated antigens include HER2 on breast cancer cells.

The antibody portion constituting the ADC of the present disclosure can be in the form of a full-length antibody, which can have or be derived from any antibody isotype, including, for example, IgA, IgD, IgE, IgG, IgM, or IgY. In some embodiments, the antibody constituting the ADC is an IgG (e.g., IgG1, IgG2, IgG3, or IgG4). In some embodiments, the antibody constituting the ADC comprises all or part of the constant region of an IgG immunoglobulin.

The antibody portion constituting the ADC of the present disclosure can be a functionally active fragment, derivative, or analog of an antibody that immunospecifically binds to a target cell (e.g., cancer cell antigen, viral antigen, or microbial antigen). In this regard, "functionally active" means that the fragment, derivative, or analog is capable of immunospecifically binding to the target cell.

Useful antibody fragments include, for example but not limited to, F(ab')₂ fragments, Fab fragments, Fvs, single-chain antibodies, diabodies, triabodies, tetrabodies, scFv, scFv-FV, or any other molecule with the same specificity as the antibody. The fragments can be obtained by molecular engineering or by chemical or enzymatic treatment of intact antibodies or antibody chains or by recombinant means.

Useful modified antibody analogs and derivatives include, for example but not limited to, derivatives and analogs of antibodies obtained by glycosylation, acetylation, PEGylation, phosphorylation, amidation, derivatization by known protecting/blocking groups, proteolytic cleavage, linking to cellular antibody units or other proteins, etc. Any of a number of chemical modifications can be carried out by known techniques, including but not limited to specific chemical cleavage, acetylation, formylation, metabolic synthesis in the presence of tunicamycin, etc.

### Target Antigen and Antibody

The antibody portion of the ADC of the present disclosure can target any suitable target molecule present on the surface of the target cell, e.g., a polypeptide, protein, polysaccharide, or lipid molecule. The binding of the antibody to the target molecule should be highly specific to ensure that the ADC specifically binds to the target cell, reducing off-target toxicity. In some embodiments, the binding affinity of the antibody to the target molecule can be selected at the nanomolar or sub-nanomolar level.

Suitable target antigens can be selected by searching for cell surface proteins that are highly expressed in tumors but low or even nearly absent in non-malignant tissues. In some embodiments, such target molecules are membrane antigens expressed on the surface of target tumor cells, such as tumor-specific antigens or tumor-associated antigens, the tumor includes hematological tumors and solid tumors, including primary and metastatic tumors. In particularly preferred embodiments, the antibody or antigen-binding fragment in the ADC of the present disclosure specifically binds to one or more tumor-specific antigens or tumor-associated antigens, or immune cell-associated antigens.

In some embodiments, the tumor-specific antigen or tumor-associated antigen targeted by the antibody portion of the ADC of the present disclosure is selected from: HER2, Her3, HER1 (ErbB1), HER4 (ErbB4), TROP2, Nectin-4, tissue factor, PD-L1, PD-1, PD-L1/PD-L2, MET, CLDN18.2, KIT, CTLA-4, RPR1, ephrin A2 receptor (EphA2), folate receptor (FRa), mesothelin, endothelin receptor, GCPII, IL-13Ra, BCMA, GD2, CLL-1, CA-IX, MUC1, 5T4, AOC3, ALK, AXL, C242, CA-125, CCL11, CCR5, CD2, CD3, CD4, CDS, CD15, CA15-3, CD16, CD18, CD19, CD20, CD21, CD22, CD25, CD30, CD32, CD33, CD37, CD38, CD44, CD52, CD56, CD64, CD66e, CD70, CD72, CD74, CD79a, CD79b, CD123, CD138, CD142, CD174, CD276, CDH3, CCD79b, CLDN9/CLDN6, CA19-9, DPEP3, AGS-16, IGFIR, IGF2R, VEGFR1, VEGFR2, VEGFR3, PDGFR-α, PDGFR-β, EGFR, EGFRvIII, ENPP3, FcRH5, FRα, KAAG1, LIV-1, Mesothelin, cMet, ROR1, SLTRK6, TF, BMPR1B, E16, TOP1, STEAP1, 0772P, MUC16, Napi3b, Sema 5b, PSCA hIg, ETBR, RNF124, prostate cancer-associated gene 1, TrpM4, teratocarcinoma-derived growth factor 1, C3DR, FcRH2, NCA, MDP, IL20R-α, Brevican, EphB2R, ASLG659, prostate stem cell antigen precursor, GEDA, BAFF-R, CXCR5, HLA-DOB, P2X5, LY64, FcRH1, IRTA2, TENB2, integrin α5β6, integrin α4β7, FGF2, FGFR1, FGFR2, FGFR3, FGFR4, PSMA, Somatostatin receptor, RANK, SLAMF7, ITGB6, CEACAM5, CA9, EGFRvlll, IL2RA, AXL receptor tyrosine kinase, TGF-βR, TNFRSF8, cancer/testis-associated antigens, CLEC14A, GRP78, stem cell-specific antigens, ASG-5, PRR4, GUCY2C, SLC39A6, TPBG, tumor-associated antigen CA242, FOLR1, GPNMB, HAVCR1, prostate tumor target Mindin, VTCN1, PTK7 protein tyrosine kinase 7, macrophage stimulating 1 receptor, TACSTD2, CA6, DLL3, DLL4, EpCAM, FAP, DKK-1, Endoglin, VCAM1, GPC3, DR5, ASCT2, B7H1, B7H3, B7H4.

In certain embodiments, the tumor-associated antigen and immune cell antigen are T cell co-inhibitory molecules. In some embodiments, the antibody or antigen-binding fragment thereof specifically binds to a tumor-associated antigen selected from PD-L1, PD-L2, CD47, CD80, CD86, HVEM, UL144, CD155, CD112, CD113, galectin-1, galectin-3, galectin 9, CD48, LIGHT, BTLA, and CD160. In some embodiments, the tumor-associated antigen is a molecule that binds to a T cell molecule selected from BTLA, Tim-3, PD-1, CTLA-4, TIGIT, CD244, and CD223.

In some embodiments, the antibody is an anti-PD-L1 antibody, such as atezolizumab, durvalumab, avelumab, or an antigen-binding fragment thereof, or an antibody or antigen-binding fragment having an amino acid sequence equivalent thereto.

In some embodiments, the antibody is an anti-PD-1 antibody, such as nivolumab, pembrolizumab, cemiplimab, anti-mouse PD-1 antibody clone J43, anti-mouse PD-1 antibody clone RMP1-14, mouse anti-PD-1 antibody clone EH12, ANB011, MDX-1106, AMP-514, AMP-224, or pidilizumab. In some embodiments, the anti-PD-1 antibody is pembrolizumab or nivolumab.

In some embodiments, the antibody is an anti-CTLA-4 antibody, such as ipilimumab, clone 9H10, tremelimumab, or clone BNI3.

In some embodiments, the antibody is an anti-CD47 antibody, such as Hu5F9-G4, IBI188, CC-90002, ZL1201, TTI-621, AO-176, SGN-CD47M antibody, ALX148 antigen-binding domain, or an antigen-binding fragment thereof, or an antibody or antigen-binding fragment having an amino acid sequence equivalent thereto.

In other embodiments, the antibody or antigen-binding fragment thereof specifically binds to a tumor-associated antigen that is a growth factor receptor (GFR). In certain embodiments, the tumor-associated antigen is an EGFR/ErbB/HER family GFR. In some embodiments, the tumor-associated antigen is selected from EGFR/HER1 (ErbB1), HER2/c-Neu (ErbB2), Her3 (ErbB3), and Her4 (ErbB4) receptors. In certain embodiments, the tumor-associated antigen is an IGFR family GFR. In some embodiments, the cancer-associated tumor antigen is an IGF1R or IGF2R receptor. In certain embodiments, the tumor-associated antigen is a TGF-βR (TβR) family GFR. In some embodiments, the cancer-associated tumor antigen is a TβR I or TβR II receptor. In certain embodiments, the tumor-associated antigen is a VEGFR family GFR. In some embodiments, the cancer-associated tumor antigen is a VEGFR1, VEGFR2, or VEGFR3 receptor. In certain embodiments, the tumor-associated antigen is a PDGFR family GFR. In some embodiments, the cancer-associated tumor antigen is a PDGFR-α or PDGFR-β receptor. In certain embodiments, the tumor-associated antigen is a FGFR family GFR. In some embodiments, the cancer-associated tumor antigen is a FGFR1, FGFR2, FGFR3, or FGFR4 receptor.

In some embodiments, the antibody is an anti-EGFR/HER1 (ErbB1) antibody, such as cetuximab, panitumumab, necitumumab, or an antigen-binding fragment thereof, or an antibody or antigen-binding fragment having an amino acid sequence equivalent thereto. In some embodiments, the antibody is an anti-HER2 (ErbB2) antibody, such as trastuzumab, pertuzumab, or an antigen-binding fragment thereof, or an antibody or antigen-binding fragment having an amino acid sequence equivalent thereto. In some embodiments, the antibody is an anti-VEGFR2 antibody, such as ramucirumab, or an antigen-binding fragment thereof, or an antibody or antigen-binding fragment having an amino acid sequence equivalent thereto. In some embodiments, the antibody is an anti-PDGFR-α antibody, such as olaratumab, or an antigen-binding fragment thereof, or an antibody or antigen-binding fragment having an amino acid sequence equivalent thereto.

In other embodiments, the antibody or antigen-binding fragment thereof specifically binds to a lymphoma-associated antigen. In certain embodiments, the lymphoma-associated antigen is CD20, CD30, CD19/CD3, CD22, or CD33. In some embodiments, the antibody is an anti-CD20 antibody, such as rituximab, ibritumomab, ofatumumab, obinutuzumab, or an antigen-binding fragment thereof, or an antibody or antigen-binding fragment having an amino acid sequence equivalent thereto. In some embodiments, the antibody is an anti-CD30 antibody, such as brentuximab, or an antigen-binding fragment thereof, or an antibody or antigen-binding fragment having an amino acid sequence equivalent thereto. In some embodiments, the antibody is an anti-CD19/CD3 antibody, such as blinatumomab, or an antigen-binding fragment thereof, or an antibody or antigen-binding fragment having an amino acid sequence equivalent thereto. In some embodiments, the antibody is an anti-CD22 antibody, such as inotuzumab, or an antigen-binding fragment thereof, or an antibody or antigen-binding fragment having an amino acid sequence equivalent thereto. In some embodiments, the antibody is an anti-CD33 antibody, such as gemtuzumab, or an antigen-binding fragment thereof, or an antibody or antigen-binding fragment having an amino acid sequence equivalent thereto.

In other embodiments, the antibody or antigen-binding fragment thereof specifically binds to a myeloma-associated antigen. In certain embodiments, the myeloma-associated antigen is SLAMF7 or CD38. In some embodiments, the antibody is an anti-SLAMF7 antibody, such as elotuzumab, or an antigen-binding fragment thereof, or an antibody or antigen-binding fragment having an amino acid sequence equivalent thereto. In some embodiments, the antibody is an anti-CD38 antibody, such as daratumumab, or an antigen-binding fragment thereof, or an antibody or antigen-binding fragment having an amino acid sequence equivalent thereto.

In other embodiments, the antibody or antigen-binding fragment thereof specifically binds to a blastoma-associated antigen. In certain embodiments, the blastoma-associated antigen is GD2. In some embodiments, the antibody is an anti-GD2 antibody, such as dinutuximab, or an antigen-binding fragment thereof, or an antibody or antigen-binding fragment having an amino acid sequence equivalent thereto.

In other embodiments, the antibody or antigen-binding fragment thereof specifically binds to RANK ligand. In some embodiments, the antibody is an anti-RANK ligand antibody, such as denosumab, or an antigen-binding fragment thereof, or an antibody or antigen-binding fragment having an amino acid sequence equivalent thereto.

In other embodiments, the antibody or antigen-binding fragment thereof specifically binds to TROP2. In some embodiments, the antibody is an anti-TROP2 antibody, such as Sacituzumab and Datopotamab, or an antigen-binding fragment thereof, or an antibody or antigen-binding fragment having an amino acid sequence equivalent thereto.

In other embodiments, the antibody or antigen-binding fragment thereof specifically binds to Claudin18.2. In some embodiments, the antibody is an anti-Claudin18.2 antibody, such as Zolbetuximab, Osemitamab (TST001), CMG901, ASKB589, ZL-1211, or an antigen-binding fragment thereof, or an antibody or antigen-binding fragment having an amino acid sequence equivalent thereto.

In other embodiments, the antibody or antigen-binding fragment thereof specifically binds to Met (also known as c-Met, or hepatocyte growth factor receptor (HGFR)). The Met gene with the pathogenic mutation will encode abnormal Met receptors that transmit aberrant signals, leading to multifaceted effects including cell growth, survival, invasion, metastasis, angiogenesis, etc. Tumors involving Met include non-small cell lung cancer, colorectal cancer, gastric cancer, esophageal cancer, glioma, etc. In some embodiments, the antibody is an anti-Met antibody, such as Onartuzumab, or an antigen-binding fragment thereof, or an antibody or antigen-binding fragment having an amino acid sequence equivalent thereto.

In other embodiments, the antibody or antigen-binding fragment thereof specifically binds to the ephrin receptor Eph, especially the EphA2 receptor. EphA2 is not only a biomarker for malignant characteristics but also an active participant in malignant progression, and has been shown to play an important role in regulating cancer development and tumor progression. Human EphA2 is abundantly expressed in prostate cancer, lung cancer, esophageal cancer, colorectal cancer, cervical cancer, ovarian cancer, breast cancer, and skin cancer, which is associated with poor prognosis in tumor patients, increased metastatic potential, and reduced survival. In some embodiments, the antibody is an anti-EphA2 antibody or antigen-binding fragment thereof, or an antibody or antigen-binding fragment having an amino acid sequence equivalent thereto.

In other embodiments, the antibody or antigen-binding fragment thereof specifically binds to Nectin-4. In some embodiments, the antibody is an anti-Nectin-4 antibody, such as Enfortumab, or an antigen-binding fragment thereof, or an antibody or antigen-binding fragment having an amino acid sequence equivalent thereto.

In other embodiments, the antibody or antigen-binding fragment thereof specifically binds to both EGFR and Met (also known as c-Met). In some embodiments, the antibody is a bispecific anti-EGFR and Met antibody, such as Amivantamab, or an antigen-binding fragment thereof, or an antibody or antigen-binding fragment having an amino acid sequence equivalent thereto.

In some embodiments, the tumor-associated antigen specifically bound by the antibody or antigen-binding fragment thereof is selected from AXL, B7H1, B7H3, B7H4, BCMA, CD16, CD19, CD22, CD25, CD30, CD32, CD33, CD44, CD64, CD70, CD74, CD79, CD138, CD142, CD276, CDH3, CEACAM5, Claudin 18.2, CLDN9/CLDN6, DPEP3, EGFR, ENPP3, EphA, FcRH5, FOLR1, FRα, GCPII, HER2, HER3, KAAG1, KIT, LIV-1, Mesothelin, c-Met, MUC1, Nectin-4, PD-L1, PD-L1/PD-L2, PSMA, ROR1, RPR1, TF, TOP1, TROP2, etc. The corresponding antibodies are commercially available or can be prepared by techniques known in the art.

In preferred embodiments, the antibody is one that binds to an antigen that is preferentially expressed or overexpressed in cancer cells, such as HER2 (ErbB2), PD-1, PD-L1, EGFR, TROP2, Claudin 18.2, EphA-2, Nectin-4, and Met, more preferably HER2 (ErbB2), EGFR, TROP2, Claudin 18.2, Nectin-4, or a combination of EGFR and Met.

For the tumor-associated antigens mentioned above, the ADC compounds of the present disclosure can treat tumors associated with the expression of the aforementioned antigens. The specific list of tumors is known or can be determined by those skilled in the art based on the prior art.

Antibodies immunospecific for tumor-associated antigens can be obtained commercially or produced by any method known to those skilled in the art, e.g., recombinant expression techniques. Nucleotide sequences encoding antibodies immunospecific for cancer cell antigens can be obtained, for example, from GenBank databases or similar databases, literature publications, or by conventional cloning and sequencing.

### Ab Unit Targeting HER2

In a particularly preferred embodiment, the antibody conjugates provided herein include an antibody or antigen-binding fragment that specifically binds to human HER2 (anti-HER2 antibody), i.e., the antibody portion specifically targets the tumor-associated antigen HER2. Accordingly, in some aspects, the present disclosure provides an antibody-drug conjugate (ADC) comprising an antibody or antigen-binding fragment thereof that specifically binds HER2 as the Ab unit of the ADC.

HER2 is the receptor tyrosine protein kinase ErbB2, and overexpression or gene amplification of HER2 is present in approximately 20-30% of breast cancers. Increased HER2 activation triggers multiple downstream pathways, leading to aberrant cancer cell proliferation (Treish I, Schwartz R, Lindley C: Pharmacology and therapeutic use of trastuzumab in breast cancer. Am J Health Syst Pharm. 2000 Nov 15;57(22):2063-76; quiz 2077-9). HER2 is also overexpressed in many other types of cancer, such as gastric cancer, esophageal cancer, colon cancer, rectal cancer, breast cancer, ovarian cancer, cervical cancer, uterine cancer, endometrial cancer, bladder cancer, pancreatic cancer, lung cancer, prostate cancer, osteosarcoma, neuroblastoma, or head and neck cancer.

In some embodiments, the antibody or antibody fragment thereof (e.g., antigen-binding fragment) that specifically binds to human HER2 can be selected from trastuzumab, pertuzumab, margetuximab, or HT-19, or an antibody fragment or site-specific mutant thereof, or other anti-human HER2 antibody that recognizes the same epitope or competitively binds to human HER2.

Trastuzumab (trade names Herceptin or Herclon) is a humanized anti-human epidermal growth factor receptor 2 monoclonal antibody used for the treatment of HER2-positive breast cancer, gastrointestinal cancer, and gastric cancer. It binds to the juxtamembrane portion of the extracellular domain of the HER2 receptor, and its heavy and light chain variable region amino acid sequences are described in US Patent 5,821,337. It interacts with three loop regions formed by human HER2 residues 557-561, 570-573, and 593-603 (Cho et al., Nature 421: 756-760, 2003), possibly interfering with HER2 signaling by preventing HER2 receptor dimerization, promoting HER2 receptor endocytic destruction, and inhibiting shedding of the extracellular domain (Hudis CA, N Engl J Med. 2007;357(1):39-51). Another important mechanism of action for anti-HER2 antibodies is the mediation of antibody-dependent cellular cytotoxicity (ADCC). In ADCC, the anti-HER2 antibody binds to tumor cells and then recruits immune cells, e.g., macrophages, via Fcε receptor (FcεR) interaction. Trastuzumab received US FDA approval in September 1998 for the treatment of metastatic breast cancer in patients.

Pertuzumab (also known as 2C4, Omnitarg, Perjeta) is a humanized monoclonal antibody that binds to the extracellular domain of the HER2 receptor and inhibits HER2 dimerization with other HER receptors. Its heavy and light chain amino acid sequences are described in US Patent 7,560,111. Pertuzumab primarily interacts with residues within the 245-333 region of human HER2, particularly residues His 245, Val 286, Ser 288, Leu 295, His 296, or Lys 311 (Franklin et al., Cancer Cell 5: 317-328, 2004). Pertuzumab has been shown to be more effective than trastuzumab in disrupting HER1-HER2 and HER3-HER2 complex formation in breast and prostate cancer cell lines (Agus et al., J Clin Oncol. 2005;23(11):2534-43. Epub Feb 7, 2005). For efficacy, pertuzumab does not require antibody-dependent cellular cytotoxicity, as an intact Fc region is not required for its activity (Agus et al., J Clin Oncol. 2005;23(11):2534-43. Epub Feb 7, 2005). Pertuzumab was approved by the US FDA for use in combination with trastuzumab and docetaxel for the treatment of patients with HER2-positive metastatic breast cancer.

Margetuximab (also known as MGAH22) is another anti-HER2 monoclonal antibody (see http://www.macrogenics.com/products-margetuximab.html). The Fc region of margetuximab is optimized to increase binding to activating FcεRs. Margetuximab is currently in clinical trials for the treatment of patients with relapsed or refractory advanced breast cancer whose tumors show HER2 expression at the 2+ level by immunohistochemistry and lack evidence of HER2 gene amplification by FISH.

HT-19 is another anti-HER2 monoclonal antibody that binds to an epitope on human HER2 different from that of trastuzumab or pertuzumab and has been demonstrated to inhibit HER2 signaling comparably to trastuzumab and, in combination with trastuzumab and pertuzumab, promotes HER2 degradation (Bergstrom D. A. et al., Cancer Res. 2015;75:LB-231).

Exemplary HER2-targeting antibodies useful in the ADCs of the present disclosure can be antibodies or antigen-binding fragments comprising all 6 CDR sequences of an antibody selected from the group (preferably, comprising the heavy chain variable region and light chain variable region sequences of an antibody selected from the group): trastuzumab (Herceptin, Genentech, US 6,054,297); ATCC Accession Nos. PTA-10355, PTA-10356, PTA-10357, PTA 10358 (US20100119511); ATCC Accession No. CRL-10463 (Genentech); ATCC Accession Nos. HB-12215, HB-12216, CRL 10463, HB-12697; pertuzumab (Genentech, US20110117097); ATCC Accession Nos. HB-12215, HB-12216, CRL 10463, HB-12698 (US20090202546); ATCC Accession Nos. HB-12215, HB-12216 (US20060088523); ATCC Accession Nos. (7C2) HB-12215, (7F3) HB-12216, (4D5) CRL-10463, (2C4) HB-12697 (US20060018899); TrasGEX (Glycotope: http://www.glycotope.conn/pipeline). The present disclosure also contemplates anti-HER2 antibodies disclosed in the following documents: US20110177095, US20100119511, US20110117097, US20090285837, US20090202546, US20060088523, US20060018899, US2011/0159014, US20090187007, US20110217305.
> Trastuzumab light chain amino acid sequence (SEQ ID NO: 1) 
DIQMTQSPSSLSASVGDRVTITCRASQDVNTAVAWYQQKPGKAPKLLIYSASFLYSGVPS
RFSGSRSGTDFTLTISSLQPEDFATYYCQQHYTTPPTFGQGTKVEIKRTVAAPSVFIFPP
SDEQLKSGTASVVCLLNNFYPREAKVQWKVDNALQSGNSQESVTEQDSKDSTYSLSSTLT
LSKADYEKHKVYACEVTHQGLSSPVTKSFNRGEC
> Trastuzumab heavy chain amino acid sequence (SEQ ID NO: 2) 
EVQLVESGGGLVQPGGSLRLSCAASGFNIKDTYIHWVRQAPGKGLEWVARIYPTNGYTRY
ADSVKGRFTISADTSKNTAYLQMNSLRAEDTAVYYCSRWGGDGFYAMDYWGQGTLVTV
SS
ASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPELLGG
PSVFLFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRW
QQGNVFSCSVMHEALHNHYTQKSLSLSPGK.
> Trastuzumab light chain LCDR1 sequence (SEQ ID NO: 3)
   RASQDVNTAVA
> Trastuzumab light chain LCDR2 sequence (SEQ ID NO: 4)
   SASFLYS
> Trastuzumab light chain LCDR3 sequence (SEQ ID NO: 5)
   QQHYTTPPT
> Trastuzumab heavy chain HCDR1 sequence (SEQ ID NO: 6)
   DTYIH
> Trastuzumab heavy chain HCDR2 sequence (SEQ ID NO: 7)
   RIYPTNGYTRYADSVKG
> Trastuzumab heavy chain HCDR3 sequence (SEQ ID NO: 8)
   WGGDGFYAMDY
> Trastuzumab light chain variable region VL (SEQ ID NO: 9)
> Trastuzumab heavy chain variable region VH (SEQ ID NO: 10)

In one embodiment, the antibody portion used in the ADC of the present disclosure comprises all 6 CDR sequences of trastuzumab. In another embodiment, the antibody portion used in the ADC of the present disclosure comprises the heavy chain variable region sequence and the light chain variable region sequence of trastuzumab. In yet another embodiment, the antibody portion used in the ADC of the present disclosure comprises the heavy chain sequence and the light chain sequence of trastuzumab.

In some embodiments, the Ab unit of the ADC of the present disclosure comprises the three CDRs of the heavy chain variable region (VH) sequence of SEQ ID NO: 10 and the three CDRs of the light chain variable region (VL) sequence of SEQ ID NO: 9, and preferably, wherein the CDRs are defined according to Kabat or IMGT or a combination thereof.

In some embodiments, the Ab unit of the ADC of the present disclosure comprises 3 heavy chain complementarity determining regions (HCDR) and 3 light chain complementarity determining regions (LCDR), wherein:
According to Kabat definition, HCDR1 comprises or consists of the amino acid sequence of SEQ ID NO: 6, HCDR2 comprises or consists of the amino acid sequence of SEQ ID NO: 7, HCDR3 comprises or consists of the amino acid sequence of SEQ ID NO: 8, LCDR1 comprises or consists of the amino acid sequence of SEQ ID NO: 3, LCDR2 comprises or consists of the amino acid sequence of SEQ ID NO: 4, and LCDR3 comprises or consists of the amino acid sequence of SEQ ID NO: 5.

In one embodiment, the Ab unit of the ADC of the present disclosure comprises a heavy chain variable region, wherein the heavy chain variable region comprises the amino acid sequence shown in SEQ ID NO: 10, or an amino acid sequence having at least 80%, 85%, 90%, 95%, 96%, 97%, 98%, or 99% sequence identity thereto.

In one embodiment, the Ab unit of the ADC of the present disclosure comprises a light chain variable region, wherein the light chain variable region comprises the amino acid sequence shown in SEQ ID NO: 9, or an amino acid sequence having at least 80%, 85%, 90%, 95%, 96%, 97%, 98%, or 99% sequence identity thereto.

In some preferred embodiments, the Ab unit of the ADC of the present disclosure comprises a heavy chain variable region and a light chain variable region, wherein the heavy chain variable region comprises the amino acid sequence shown in SEQ ID NO: 10, and wherein the light chain variable region comprises the amino acid sequence shown in SEQ ID NO: 9.

In some embodiments, the Ab unit of the ADC of the present disclosure preferably further comprises an antibody heavy chain constant region and/or light chain constant region. Preferably, the heavy chain constant region is a heavy chain constant region derived from a human immunoglobulin. Preferably, the light chain constant region is a light chain constant region derived from a human immunoglobulin. In some aspects, the heavy chain constant region comprised in the Ab unit can be of any isotype or subtype, e.g., an IgG1, IgG2, IgG3, or IgG4 isotype heavy chain constant region, and preferably an IgG1, IgG2, or IgG4 heavy chain constant region, especially a human IgG1 heavy chain constant region. In further aspects, the light chain constant region comprised in the Ab unit can be a κ light chain constant region or a λ light chain constant region, especially a human κ light chain constant region.

In some embodiments, the Ab unit of the ADC of the present disclosure comprises a human IgG1 heavy chain constant region. Preferably, the heavy chain constant region comprises the amino acid sequence of SEQ ID NO: 11, or an amino acid sequence comprising at least one, two, or three, but not more than 20, 10, or 5 amino acid changes relative to the amino acid sequence of SEQ ID NO: 11, or a sequence having at least 95-99% identity to the amino acid sequence of SEQ ID NO: 11.

An exemplary amino acid sequence of a human IgG1 heavy chain constant region (SEQ ID NO: 11)

ASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPELLGG
PSVFLFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRW
QQGNVFSCSVMHEALHNHYTQKSLSLSPGK

In some embodiments, the Ab unit of the ADC of the present disclosure comprises a human κ light chain constant region. Preferably, the light chain constant region comprises the amino acid sequence of SEQ ID NO: 12, or an amino acid sequence comprising at least one, two, or three, but not more than 20, 10, or 5 amino acid changes relative to the amino acid sequence of SEQ ID NO: 12, or a sequence having at least 95-99% identity to the amino acid sequence of SEQ ID NO: 12.

An exemplary amino acid sequence of a human κ light chain constant region (SEQ ID NO: 12)

In some embodiments, the Ab unit of the ADC of the present disclosure is a full-length antibody comprising a heavy chain constant region and a light chain constant region. In some embodiments, the Ab unit is a tetrameric structure formed by two light chains and two heavy chains. In further embodiments, the Ab unit is an IgG antibody, especially an IgG1 antibody.

In some preferred embodiments, the Ab unit of the ADC of the present disclosure comprises a heavy chain and a light chain, wherein: the heavy chain comprises the amino acid sequence shown in SEQ ID NO: 2, or an amino acid sequence having at least 80%, 85%, 90%, 95%, 96%, 97%, 98%, or 99% sequence identity thereto, or consists thereof. In other preferred embodiments, the Ab unit of the ADC of the present disclosure comprises a heavy chain and a light chain, wherein: the light chain comprises the amino acid sequence shown in SEQ ID NO: 1, or an amino acid sequence having at least 80%, 85%, 90%, 95%, 96%, 97%, 98%, or 99% sequence identity thereto.

In some more preferred embodiments, the Ab unit of the ADC of the present disclosure comprises:
(a) a heavy chain comprising the amino acid sequence of SEQ ID NO: 2, and
(b) a light chain comprising the amino acid sequence of SEQ ID NO: 1.

### Ab Unit Targeting TROP2

In a particularly preferred embodiment, the antibody conjugates provided herein include an antibody or antigen-binding fragment that specifically binds to human TROP2 (anti-TROP2 antibody), i.e., the antibody portion specifically targets the tumor-associated antigen TROP2. Accordingly, in some aspects, the present disclosure provides an antibody-drug conjugate (ADC) comprising an antibody or antigen-binding fragment thereof that specifically binds TROP2 as the Ab unit of the ADC.

Trophoblast cell surface antigen 2 (TROP2), also known as tumor-associated calcium signal transducer 2 (TACSTD2), is a type I transmembrane cell surface glycoprotein. The sequence of human TROP2 can be obtained from UniProtKB Accession No. P09758. It has been established that TROP2 is overexpressed in many solid tumors, including but not limited to various human epithelial cancers, e.g., cervical cancer, endometrial cancer, breast cancer, urothelial cancer, lung cancer, gastric cancer, prostate cancer, colorectal cancer, and pancreatic cancer. Moreover, TROP2 can play a role in tumor cell proliferation, invasion, migration, apoptosis, and therapeutic resistance by binding to or interacting with various molecules. These characteristics make TROP2 an attractive pan-cancer target for cancer therapy. Ying Wen et al., A literature review of the promising future of TROP2: a potential drug therapy target, Ann Transl Med. 2022 Dec; 10(24): 1403, doi: 10.21037/atm-22-5976.

Antibodies targeting human TROP2 useful in the ADCs of the present disclosure can be prepared using antibody preparation techniques known in the art. For example, anti-TROP2 antibodies can be obtained by immunizing animals with human TROP2 (UniProtKB Accession No. P09758) or a polypeptide comprising the amino acid sequence of the extracellular domain of TROP2, harvesting antibodies from the immunized animals, and purifying and preferably humanizing them. Furthermore, fully human sequence anti-human TROP2 antibodies can be obtained using yeast display libraries expressing human immunoglobulin sequences, transgenic animals, or the like.

In some embodiments, the antibody or antibody fragment thereof (e.g., antigen-binding fragment) that specifically binds to human TROP2 can be selected from Sacituzumab, Datopotamab, or an antibody fragment thereof, or other anti-human TROP2 antibody that recognizes the same epitope or competitively binds to human TROP2.

Sacituzumab, also known as sacituzumab, is a humanized form of the murine monoclonal antibody RS7 developed by Immunomedics, and is a humanized IgG1κ monoclonal antibody targeting TROP2. This antibody can directly bind to TROP2-expressing cancer cells and trigger antibody internalization. The sequence of Sacituzumab can be found in US10179171B2.

Datopotamab is an IgG1-type anti-TROP2 antibody. This antibody was generated by humanizing a mouse mAb that specifically binds to human TROP2. The sequence of Datopotamab can be found in Daisuke Okajima et al., Datopotamab Deruxtecan, a Novel TROP2-directed Antibody--drug Conjugate, Demonstrates Potent Antitumor Activity by Efficient Drug Delivery to Tumor Cells, Mol Cancer Ther (2021) 20 (12): 2329-2340.

Exemplary antibodies targeting human TROP2 useful in the ADCs of the present disclosure can be antibodies or antigen-binding fragments comprising all 6 CDR sequences of an antibody selected from the group (preferably, comprising the heavy chain variable region and light chain variable region sequences of an antibody selected from the group): Sacituzumab and Datopotamab. The present disclosure also contemplates anti-human TROP2 antibodies disclosed in the following documents: WO2010089782A1; US 2021/0393792 A1; WO2008/144891, WO2011/145744, WO2011/155579, WO2013/077458, WO2003/074566, WO2011/068845, WO2013/068946, US 2023/0270870 A1.
>Sacituzumab light chain amino acid sequence (SEQ ID NO: 13) 
DIQLTQSPSSLSASVGDRVSITCKASQDVSIAVAWYQQKPGKAPKLLIYSASYRYTGVPDRFS
GSGSGTDFTLTISSLQPEDFAVYYCQQHYITPLTFGAGTKVEIK
RTVAAPSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC
>Sacituzumab heavy chain amino acid sequence (SEQ ID NO: 14) 
QVQLQQSGSELKKPGASVKVSCKASGYTFTNYGMNWVKQAPGQGLKWMGWINTYTGEP
TYTDDFKGRFAFSLDTSVSTAYLQISSLKADDTAVYFCARGGFGSSYWYFDVWGQGSLVTV
SS
ASTKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKRVEPKSCDKTHTCPPCPAPELLGGPSVF
LFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSREEMTKNQ
VSLTCLVKGFYPSDIAVEWESNGQPENNYKTTPPVLDSDGSFFLYSKLTVDKSRWQQGNVF
 SCSVMHEALHNHYTQKSLSLSPGK
>Sacituzumab light chain LCDR1 sequence (SEQ ID NO: 15)
   KASQDVSIAVA
>Sacituzumab light chain LCDR2 sequence (SEQ ID NO: 16)
   SASYRYT
>Sacituzumab light chain LCDR3 sequence (SEQ ID NO: 17)
   QQHYITPLT
>Sacituzumab heavy chain HCDR1 sequence (SEQ ID NO: 18)
   NYGMN
>Sacituzumab heavy chain HCDR2 sequence (SEQ ID NO: 19)
   WINTYTGEPTYTDDFKG
>Sacituzumab heavy chain HCDR3 sequence (SEQ ID NO: 20)
   GGFGSSYWYFDV
>Sacituzumab light chain variable region VL (SEQ ID NO: 21) 
DIQLTQSPSSLSASVGDRVSITCKASQDVSIAVAWYQQKPGKAPKLLIYSASYRYTGVPDRFS
GSGSGTDFTLTISSLQPEDFAVYYCQQHYITPLTFGAGTKVEIK
>Sacituzumab heavy chain variable region VH (SEQ ID NO: 22)

QVQLQQSGSELKKPGASVKVSCKASGYTFTNYGMNWVKQAPGQGLKWMGWINTYTGEP
TYTDDFKGRFAFSLDTSVSTAYLQISSLKADDTAVYFCARGGFGSSYWYFDVWGQGSLVTV
SS

In one embodiment, the antibody portion used in the ADC of the present disclosure comprises all 6 CDR sequences of Sacituzumab or Datopotamab. In another embodiment, the antibody portion used in the ADC of the present disclosure comprises the heavy chain variable region sequence and the light chain variable region sequence of Sacituzumab or Datopotamab. In yet another embodiment, the antibody portion used in the ADC of the present disclosure comprises the heavy chain sequence and the light chain sequence of Sacituzumab or Datopotamab.

In some embodiments, the Ab unit of the ADC of the present disclosure comprises the three CDRs of the heavy chain variable region (VH) sequence of SEQ ID NO: 22 and the three CDRs of the light chain variable region (VL) sequence of SEQ ID NO: 21, and preferably, wherein the CDRs are defined according to Kabat or IMGT or a combination thereof.

In some embodiments, the Ab unit of the ADC of the present disclosure comprises 3 heavy chain complementarity determining regions (HCDR) and 3 light chain complementarity determining regions (LCDR), wherein:
According to Kabat definition, HCDR1 comprises or consists of the amino acid sequence of SEQ ID NO: 18, HCDR2 comprises or consists of the amino acid sequence of SEQ ID NO: 19, HCDR3 comprises or consists of the amino acid sequence of SEQ ID NO: 20, LCDR1 comprises or consists of the amino acid sequence of SEQ ID NO: 15, LCDR2 comprises or consists of the amino acid sequence of SEQ ID NO: 16, and LCDR3 comprises or consists of the amino acid sequence of SEQ ID NO: 17.

In one embodiment, the Ab unit of the ADC of the present disclosure comprises a heavy chain variable region, wherein the heavy chain variable region comprises the amino acid sequence shown in SEQ ID NO: 22, or an amino acid sequence having at least 80%, 85%, 90%, 95%, 96%, 97%, 98%, or 99% sequence identity thereto.

In one embodiment, the Ab unit of the ADC of the present disclosure comprises a light chain variable region, wherein the light chain variable region comprises the amino acid sequence shown in SEQ ID NO: 21, or an amino acid sequence having at least 80%, 85%, 90%, 95%, 96%, 97%, 98%, or 99% sequence identity thereto.

In some preferred embodiments, the Ab unit of the ADC of the present disclosure comprises a heavy chain variable region and a light chain variable region, wherein the heavy chain variable region comprises the amino acid sequence described in SEQ ID NO: 22, and wherein the light chain variable region comprises the amino acid sequence shown in SEQ ID NO: 21.

In some embodiments, the Ab unit of the ADC of the present disclosure preferably further comprises an antibody heavy chain constant region and/or light chain constant region. Preferably, the heavy chain constant region is a heavy chain constant region derived from a human immunoglobulin. Preferably, the light chain constant region is a light chain constant region derived from a human immunoglobulin. In some aspects, the heavy chain constant region comprised in the Ab unit can be of any isotype or subtype, e.g., an IgG1, IgG2, IgG3, or IgG4 isotype heavy chain constant region, and preferably an IgG1, IgG2, or IgG4 heavy chain constant region, especially a human IgG1 heavy chain constant region. In further aspects, the light chain constant region comprised in the Ab unit can be a κ light chain constant region or a λ light chain constant region, especially a human κ light chain constant region.

In some embodiments, the Ab unit of the ADC of the present disclosure comprises a human IgG1 heavy chain constant region. Preferably, the heavy chain constant region comprises the amino acid sequence of SEQ ID NO: 23, or an amino acid sequence comprising at least one, two, or three, but not more than 20, 10, or 5 amino acid changes relative to the amino acid sequence of SEQ ID NO: 23, or a sequence having at least 95-99% identity to the amino acid sequence of SEQ ID NO: 23.

An exemplary amino acid sequence of a human IgG1 heavy chain constant region (SEQ ID NO: 23)

ASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKRVEPKSCDKTHTCPPCPAPELLGG
PSVFLFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSREEM
TKNQVSLTCLVKGFYPSDIAVEWESNGQPENNYKTTPPVLDSDGSFFLYSKLTVDKSRWQ
QGNVFSCSVMHEALHNHYTQKSLSLSPGK

In some embodiments, the Ab unit of the ADC of the present disclosure comprises a human κ light chain constant region. Preferably, the light chain constant region comprises the amino acid sequence of SEQ ID NO: 12, or an amino acid sequence comprising at least one, two, or three, but not more than 20, 10, or 5 amino acid changes relative to the amino acid sequence of SEQ ID NO: 12, or a sequence having at least 95-99% identity to the amino acid sequence of SEQ ID NO: 12.

In some embodiments, the Ab unit of the ADC of the present disclosure is a full-length antibody comprising a heavy chain constant region and a light chain constant region. In some embodiments, the Ab unit is a tetrameric structure formed by two light chains and two heavy chains. In further embodiments, the Ab unit is an IgG antibody, especially an IgG1 antibody.

In some preferred embodiments, the Ab unit of the ADC of the present disclosure comprises a heavy chain and a light chain, wherein: the heavy chain comprises the amino acid sequence shown in SEQ ID NO: 14, or an amino acid sequence having at least 80%, 85%, 90%, 95%, 96%, 97%, 98%, or 99% sequence identity thereto, or consists thereof. In other preferred embodiments, the Ab unit of the ADC of the present disclosure comprises a heavy chain and a light chain, wherein: the light chain comprises the amino acid sequence shown in SEQ ID NO: 13, or an amino acid sequence having at least 80%, 85%, 90%, 95%, 96%, 97%, 98%, or 99% sequence identity thereto.

In some more preferred embodiments, the Ab unit of the ADC of the present disclosure comprises:
(a) a heavy chain comprising the amino acid sequence of SEQ ID NO: 14, and
(b) a light chain comprising the amino acid sequence of SEQ ID NO: 13.

Cancers that can be treated with the TROP2-targeting ADCs of the present disclosure include, but are not limited to, adenocarcinoma, squamous cell carcinoma, non-small cell lung cancer (NSCLC), small cell lung cancer (SCLC), colorectal cancer, gastric adenocarcinoma, esophageal cancer, hepatocellular carcinoma, ovarian epithelial cancer, breast cancer, metastatic breast cancer, triple-negative breast cancer (TNBC), prostate cancer, hormone-refractory prostate cancer, pancreatic ductal adenocarcinoma, head and neck cancer, renal cell carcinoma, bladder tumor, cervical cancer, endometrial cancer, uterine cancer, follicular thyroid cancer, glioblastoma multiforme.

### Ab Unit Targeting Claudin18.2

In a particularly preferred embodiment, the antibody conjugates provided herein include an antibody or antigen-binding fragment that specifically binds to human Claudin18.2 (anti-Claudin18.2 antibody), i.e., the antibody portion specifically targets the tumor-associated antigen Claudin18.2. Accordingly, in some aspects, the present disclosure provides an antibody-drug conjugate (ADC) comprising an antibody or antigen-binding fragment thereof that specifically binds Claudin18.2 as the Ab unit of the ADC.

Claudin18.2 (also abbreviated as CLDN18.2) belongs to the Claudin family of tight junction membrane proteins. Sequences of human and various mammalian Claudin18.2 can be found in UniProtKB. For example, the human Claudin18.2 sequence can be found under UniProtKB Accession No. P56856-2. The expression of this protein in healthy tissues is mainly restricted to differentiated gastric mucosal epithelial cells, but it exhibits aberrant overexpression in a range of malignancies, particularly digestive system malignancies. Therefore, Claudin18.2 has been proposed as a promising target for developing antibody-based ADC cancer therapeutics. Daisuke Kyuno et al., Claudin-18.2 as a therapeutic target in cancers: cumulative findings from basic research and clinical trials, Tissue Barriers. 2022; 10(1): 1967080. doi: 10.1080/21688370.2021.1967080; Jinxia Chen, Targeting CLDN18.2 in cancers of the gastrointestinal tract: New drugs and new indications, Front Oncol. 2023; 13: 1132319, doi: 10.3389/fonc.2023.1132319.

Antibodies targeting human Claudin18.2 useful in the ADCs of the present disclosure can be prepared using antibody preparation techniques known in the art. For example, anti-Claudin18.2 antibodies can be obtained by immunizing animals with human Claudin18.2 (UniProtKB Accession No. P56856-2) or a polypeptide comprising the amino acid sequence of the extracellular domain of Claudin18.2, harvesting antibodies from the immunized animals, and purifying and preferably humanizing them. Furthermore, fully human sequence anti-human Claudin18.2 antibodies can be obtained using yeast display libraries expressing human immunoglobulin sequences, transgenic animals, or the like.

In some embodiments, the antibody or antibody fragment thereof (e.g., antigen-binding fragment) that specifically binds to human Claudin18.2 can be selected from Zolbetuximab, Osemitamab (TST001), CMG901, ASKB589, ZL-1211, or an antibody fragment thereof, or other anti-human Claudin18.2 antibody that recognizes the same epitope or competitively binds to human Claudin18.2.

Zolbetuximab (also known as GC-182, IMAB-362, IMAB362, claudiximab) is an IgG1 antibody derived from a murine monoclonal antibody and has been chimerized to display a human IgG1 constant region for clinical use. This antibody can directly bind to CLDN18.2-expressing cancer cells and trigger antibody internalization. The sequence of Zolbetuximab can be found in WO2007059997 and WO2016/165762.

Osemitamab (TST001) is a high-affinity humanized anti-Claudin18.2 antibody. This antibody has enhanced antibody-dependent cellular cytotoxicity (ADCC) and complement-dependent cytotoxicity (CDC), exhibiting potent anti-tumor activity in tumor xenograft models. The sequence of this antibody can be found in Inxight Drugs and INN (code 11927).

CMG901, ASKB589, ZL-1211 are exemplary anti-human CLDN18.2 antibodies currently in clinical trials. See DOI: 10.1200/JCO.2023.41.4_suppl.352; DOI: 10.1200/JCO.2023.41.4_suppl.397; DOI: 10.1200/JC0.2023.41.16_suppl.2537.

Exemplary antibodies targeting human CLDN18.2 useful in the ADCs of the present disclosure can be antibodies or antigen-binding fragments comprising all 6 CDR sequences of an antibody selected from the group (preferably, comprising the heavy chain variable region and light chain variable region sequences of an antibody selected from the group): Zolbetuximab, Osemitamab (TST001), and CMG901.

The present disclosure also contemplates anti-human Claudin18.2 antibodies disclosed in the following documents: WO2007059997A1, CN107667118A, WO2016/166122, US11555070B2, WO2020/135674, WO2018/006882, CN109762067, WO2019/242505, WO2020/038404, WO2020/043044, WO2020/063988, WO2020/082209, WO2020/018852, WO2020/023679, WO2020/135674, WO2020/135201, WO2020/139956, WO2020/025792, WO2020160560, CN111808194, and WO2020200196.
> Zolbetuximab light chain amino acid sequence (SEQ ID NO: 24) 
DIVMTQSPSSLTVTAGEKVTMSCKSSQSLLNSGNQKNYLTWYQQKPGQPPKLLIYWASTRE
SGVPDRFTGSGSGTDFTLTISSVQAEDLAVYYCQNDYSYPFTFGSGTKLEIK
RTVAAPSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC
> Zolbetuximab heavy chain amino acid sequence (SEQ ID NO: 25) 
QVQLQQPGAELVRPGASVKLSCKASGYTFTSYWINWVKQRPGQGLEWIGNIYPSDSYTNY
NQKFKDKATLTVDKSSSTAYMQLSSPTSEDSAVYYCTRSWRGNSFDYWGQGTTLTVSS
ASTKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKRVEPKSCDKTHTCPPCPAPELLGGPSVF
LFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSREEMTKNQ
VSLTCLVKGFYPSDIAVEWESNGQPENNYKTTPPVLDSDGSFFLYSKLTVDKSRWQQGNVF
SCSVMHEALHNHYTQKSLSLSPGK
> Zolbetuximab light chain LCDR1 sequence (SEQ ID NO: 26)
   KSSQSLLNSGNQKNYLT
> Zolbetuximab light chain LCDR2 sequence (SEQ ID NO: 27)
   WASTRES
> Zolbetuximab light chain LCDR3 sequence (SEQ ID NO: 28)
   QNDYSYPFT
> Zolbetuximab heavy chain HCDR1 sequence (SEQ ID NO: 29)
   SYWIN
> Zolbetuximab heavy chain HCDR2 sequence (SEQ ID NO: 30)
   NIYPSDSYTNYNQKFKD
> Zolbetuximab heavy chain HCDR3 sequence (SEQ ID NO: 31)
   SWRGNSFDY
> Zolbetuximab light chain variable region VL (SEQ ID NO: 32) 
DIVMTQSPSSLTVTAGEKVTMSCKSSQSLLNSGNQKNYLTWYQQKPGQPPKLLIYWASTRE
SGVPDRFTGSGSGTDFTLTISSVQAEDLAVYYCQNDYSYPFTFGSGTKLEIK
> Zolbetuximab heavy chain variable region VH (SEQ ID NO: 33)

QVQLQQPGAELVRPGASVKLSCKASGYTFTSYWINWVKQRPGQGLEWIGNIYPSDSYTNY
NQKFKDKATLTVDKSSSTAYMQLSSPTSEDSAVYYCTRSWRGNSFDYWGQGTTLTVSS

In one embodiment, the antibody portion used in the ADC of the present disclosure comprises all 6 CDR sequences of Zolbetuximab. In another embodiment, the antibody portion used in the ADC of the present disclosure comprises the heavy chain variable region sequence and the light chain variable region sequence of Zolbetuximab. In yet another embodiment, the antibody portion used in the ADC of the present disclosure comprises the heavy chain sequence and the light chain sequence of Zolbetuximab.

In some embodiments, the Ab unit of the ADC of the present disclosure comprises the three CDRs of the heavy chain variable region (VH) sequence of SEQ ID NO: 33 and the three CDRs of the light chain variable region (VL) sequence of SEQ ID NO: 32, and preferably, wherein the CDRs are defined according to Kabat or IMGT or a combination thereof.

In some embodiments, the Ab unit of the ADC of the present disclosure comprises 3 heavy chain complementarity determining regions (HCDR) and 3 light chain complementarity determining regions (LCDR), wherein:
According to Kabat definition, HCDR1 comprises or consists of the amino acid sequence of SEQ ID NO: 29, HCDR2 comprises or consists of the amino acid sequence of SEQ ID NO: 30, HCDR3 comprises or consists of the amino acid sequence of SEQ ID NO: 31, LCDR1 comprises or consists of the amino acid sequence of SEQ ID NO: 26, LCDR2 comprises or consists of the amino acid sequence of SEQ ID NO: 27, and LCDR3 comprises or consists of the amino acid sequence of SEQ ID NO: 28.

In one embodiment, the Ab unit of the ADC of the present disclosure comprises a heavy chain variable region, wherein the heavy chain variable region comprises the amino acid sequence shown in SEQ ID NO: 33, or an amino acid sequence having at least 80%, 85%, 90%, 95%, 96%, 97%, 98%, or 99% sequence identity thereto.

In one embodiment, the Ab unit of the ADC of the present disclosure comprises a light chain variable region, wherein the light chain variable region comprises the amino acid sequence shown in SEQ ID NO: 32, or an amino acid sequence having at least 80%, 85%, 90%, 95%, 96%, 97%, 98%, or 99% sequence identity thereto.

In some preferred embodiments, the Ab unit of the ADC of the present disclosure comprises a heavy chain variable region and a light chain variable region, wherein the heavy chain variable region comprises the amino acid sequence described in SEQ ID NO: 33, and wherein the light chain variable region comprises the amino acid sequence shown in SEQ ID NO: 32.

In some embodiments, the Ab unit of the ADC of the present disclosure preferably further comprises an antibody heavy chain constant region and/or light chain constant region. Preferably, the heavy chain constant region is a heavy chain constant region derived from a human immunoglobulin. Preferably, the light chain constant region is a light chain constant region derived from a human immunoglobulin. In some aspects, the heavy chain constant region comprised in the Ab unit can be of any isotype or subtype, e.g., an IgG1, IgG2, IgG3, or IgG4 isotype heavy chain constant region, and preferably an IgG1, IgG2, or IgG4 heavy chain constant region, especially a human IgG1 heavy chain constant region. In further aspects, the light chain constant region comprised in the Ab unit can be a κ light chain constant region or a λ light chain constant region, especially a human κ light chain constant region.

In some embodiments, the Ab unit of the ADC of the present disclosure comprises a human IgG1 heavy chain constant region. Preferably, the heavy chain constant region comprises the amino acid sequence of SEQ ID NO: 34, or an amino acid sequence comprising at least one, two, or three, but not more than 20, 10, or 5 amino acid changes relative to the amino acid sequence of SEQ ID NO: 34, or a sequence having at least 95-99% identity to the amino acid sequence of SEQ ID NO: 34.

An exemplary amino acid sequence of a human IgG1 heavy chain constant region (SEQ ID NO: 34)

ASTKGPSVFPLAPSSKSTSGGTAALGCLVKDYFPEPVTVSWNSGALTSGVHTFPAVLQS
SGLYSLSSVVTVPSSSLGTQTYICNVNHKPSNTKVDKRVEPKSCDKTHTCPPCPAPELLGGPS
VFLFPPKPKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPPSREEMTK
NQVSLTCLVKGFYPSDIAVEWESNGQPENNYKTTPPVLDSDGSFFLYSKLTVDKSRWQQGN
VFSCSVMHEALHNHYTQKSLSLSPGK

In some embodiments, the Ab unit of the ADC of the present disclosure comprises a human κ light chain constant region. Preferably, the light chain constant region comprises the amino acid sequence of SEQ ID NO: 12, or an amino acid sequence comprising at least one, two, or three, but not more than 20, 10, or 5 amino acid changes relative to the amino acid sequence of SEQ ID NO: 12, or a sequence having at least 95-99% identity to the amino acid sequence of SEQ ID NO: 12.

In some embodiments, the Ab unit of the ADC of the present disclosure is a full-length antibody comprising a heavy chain constant region and a light chain constant region. In some embodiments, the Ab unit is a tetrameric structure formed by two light chains and two heavy chains. In further embodiments, the Ab unit is an IgG antibody, especially an IgG1 antibody.

In some preferred embodiments, the Ab unit of the ADC of the present disclosure comprises a heavy chain and a light chain, wherein: the heavy chain comprises the amino acid sequence shown in SEQ ID NO: 25, or an amino acid sequence having at least 80%, 85%, 90%, 95%, 96%, 97%, 98%, or 99% sequence identity thereto, or consists thereof. In other preferred embodiments, the Ab unit of the ADC of the present disclosure comprises a heavy chain and a light chain, wherein: the light chain comprises the amino acid sequence shown in SEQ ID NO: 24, or an amino acid sequence having at least 80%, 85%, 90%, 95%, 96%, 97%, 98%, or 99% sequence identity thereto.

In some more preferred embodiments, the Ab unit of the ADC of the present disclosure comprises:
(a) a heavy chain comprising the amino acid sequence of SEQ ID NO: 25, and
(b) a light chain comprising the amino acid sequence of SEQ ID NO: 24.

Cancers that can be treated with the Claudin18.2-targeting ADCs of the present disclosure include, but are not limited to, various digestive tract cancers, such as gastric cancer (GC), gastroesophageal junction (GEJ) cancer, esophageal cancer, and pancreatic cancer.

### Ab Unit Targeting EGFR

In a particularly preferred embodiment, the antibody conjugates provided herein include an antibody or antigen-binding fragment that specifically binds to human EGFR (anti-EGFR antibody), i.e., the antibody portion specifically targets the tumor-associated antigen EGFR. Accordingly, in some aspects, the present invention provides an antibody-drug conjugate (ADC) comprising an antibody or antigen-binding fragment thereof that specifically binds EGFR as the Ab unit of the ADC.

Epidermal growth factor receptor, also abbreviated herein as EGFR, is a member of the epidermal growth factor receptor (HER) family, encoded by the c-erbB proto-oncogene (also known as HER-1 or Erb-B1). Sequences of human and various mammalian EGFR can be found in UniProtKB. For example, the human EGFR sequence can be found under UniProtKB Accession No. P00533. EGFR is overexpressed in many solid tumors, including lung cancer, head and neck cancer, breast cancer, kidney cancer, gastric cancer, colon cancer, pancreatic cancer, ovarian cancer, prostate cancer, and bladder cancer; and EGFR can induce tumor proliferation through homodimerization.

Currently, EGFR has been proposed as a promising target for antibody-based cancer therapeutics. To date, five EGFR-targeting monoclonal antibodies have been approved for clinical cancer treatment, namely cetuximab (Erbitux^{®}), panitumumab (Vectibix^{®}), nimotuzumab (BIOMAB-EGFR^{®}), necitumumab (Portrazza^{®}), and amivantamab (amivantamab-vmjw; Rybrevant^{®}). In addition, three EGFR-based ADC drugs are in clinical trials. However, no EGFR-based ADC has yet been approved for therapy. See Jinfeng Yu et al., Antibody-Drug Conjugates Targeting the Human Epidermal Growth Factor Receptor Family in Cancers, Front Mol Biosci. 2022; 9: 847835, doi: 10.3389/fmolb.2022.847835.

Various anti-EGFR monoclonal antibodies have been developed for binding to the extracellular domain of this receptor to block receptor-ligand interaction or its dimerization. These antibodies are all suitable for use in the present invention. Furthermore, antibodies targeting human EGFR useful in the ADCs of the present invention can also be prepared using antibody preparation techniques known in the art. For example, anti-EGFR antibodies can be obtained by immunizing animals with human EGFR (UniProtKB Accession No. P00533) or a polypeptide comprising the amino acid sequence of the extracellular domain of EGFR, harvesting antibodies from the immunized animals, and purifying and preferably humanizing them. Furthermore, fully human sequence anti-human EGFR antibodies can be obtained using yeast display libraries expressing human immunoglobulin sequences, transgenic animals, or the like.

The antibody targeting human EGFR used in the ADCs of the present invention can be a monospecific antibody that binds EGFR. In some cases, the antibody targeting human EGFR used in the ADCs of the present invention can also be a multispecific antibody, especially a bispecific antibody, such as one targeting EGFR and MET; or one targeting MUC1 and EGFR.

In some embodiments, the anti-human EGFR antibody or antibody fragment thereof (e.g., antigen-binding fragment) used in the ADCs of the present invention can be selected from cetuximab (Erbitux^{®}), panitumumab (Vectibix^{®}), nimotuzumab (BIOMAB-EGFR^{®}), necitumumab (Portrazza^{®}), depatuxizumab (ABT-806), NECITUMUMAB, IZALONTAMAB, BAFISONTAMAB, Petosemtamab, PIMURUTAMAB, FUTUXIMAB, MODOTUXIMAB (Zatuximab), or an antibody fragment thereof, or other anti-human EGFR antibody that recognizes the same epitope or competitively binds to human EGFR. The sequences of the aforementioned antibodies can be found in Inxight Drugs and International Nonproprietary Names (INN) codes 7906, 8499, 8545, 9083, 11030, 10263, 9083, 12022, 11851, 11136, 11309, 9612, and 9613.

Cetuximab (Erbitux^{®}) is a recombinant chimeric human/murine IgG1 monoclonal antibody. This antibody binds to the extracellular domain of inactive EGFR with an affinity far greater than that of endogenous ligands, competitively blocking ligand-receptor binding, blocking the agonistic effect of ligands on the receptor, and can trigger EGFR internalization, downregulating EGFR expression levels on the cell membrane. Furthermore, this antibody can activate antibody-dependent cell-mediated cytotoxicity (ADCC), producing further cell-killing effects. The sequence of cetuximab can be found in WO2007092453 and INN code 7906.

Panitumumab (Vectibix^{®}) is a recombinant humanized IgG2 monoclonal antibody. Panitumumab specifically binds to EGFR on tumor cells and competitively inhibits the binding of EGFR ligands. Preclinical studies have shown that the binding of panitumumab to EGFR prevents ligand-induced receptor autophosphorylation and receptor-associated kinase activation, thereby inhibiting cell growth, inducing apoptosis, reducing the production of pro-inflammatory cytokines and vascular growth factors, and can induce EGFR internalization. The sequence of panitumumab can be found in INN code 8499.

Exemplary antibodies targeting human EGFR useful in the ADCs of the present invention can be antibodies or antigen-binding fragments comprising all 6 CDR sequences of an antibody selected from the group (preferably, comprising the heavy chain variable region and light chain variable region sequences of an antibody selected from the group): cetuximab, panitumumab, nimotuzumab, necitumumab, depatuxizumab, NECITUMUMAB, IZALONTAMAB, BAFISONTAMAB, Petosemtamab, PIMURUTAMAB, FUTUXIMAB, and MODOTUXIMAB.

The present invention also contemplates anti-human EGFR antibodies disclosed in the following documents: WO2023040941A1, WO2022271722A1, WO2022159576A1, WO2022128716A1, WO2022105878A1, WO2021247798A1, WO2022104697A1, WO2021066869A1, WO2020233534A1, WO2020130125A1, WO2019046858A1, WO2019046859A1, WO2019035630A2, WO2019035630A3, WO2018098035A1, WO2017214233A1, WO2017214282A1, WO2017214301A1, WO2017161206A1, WO2017139623A1, WO2017136581A1, WO2017076492A1, WO2017060322A3, WO2017025458A1, WO2017008169A1, WO2016065456A1, WO2015143382A1, WO2014143765A1, WO2014143765A8, WO2014152199A1, WO2014094355A1, WO2012143495A2, and WO2012143495A3.
> Cetuximab light chain amino acid sequence (SEQ ID NO: 35) 
DILLTQSPVILSVSPGERVSFSCRASQSIGTNIHWYQQRTNGSPRLLIKYASESISGIPSRFSGSG
SGTDFTLSINSVESEDIADYYCQQNNNWPTTFGAGTKLELK
RTVAAPSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC
> Cetuximab heavy chain amino acid sequence (SEQ ID NO: 36) 
QVQLKQSGPGLVQPSQSLSITCTVSGFSLTNYGVHWVRQSPGKGLEWLGVIWSGGNTDYN
TPFTSRLSINKDNSKSQVFFKMNSLQSNDTAIYYCARALTYYDYEFAYWGQGTLVTVSA
ASTKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKRVEPKSCDKTHTCPPCPAPELLGGPSVF
LFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSREEMTKNQ
VSLTCLVKGFYPSDIAVEWESNGQPENNYKTTPPVLDSDGSFFLYSKLTVDKSRWQQGNVF
SCSVMHEALHNHYTQKSLSLSPGK
> Cetuximab light chain LCDR1 sequence (SEQ ID NO: 37)
   RASQSIGTNIH
> Cetuximab light chain LCDR2 sequence (SEQ ID NO: 38)
   YASESIS
> Cetuximab light chain LCDR3 sequence (SEQ ID NO: 39)
   QQNNNWPTT
> Cetuximab heavy chain HCDR1 sequence (SEQ ID NO: 40)
   NYGVH
> Cetuximab heavy chain HCDR2 sequence (SEQ ID NO: 41)
   VIWSGGNTDYNTPFTS
> Cetuximab heavy chain HCDR3 sequence (SEQ ID NO: 42)
   ALTYYDYEFAY
> Cetuximab light chain variable region VL (SEQ ID NO: 43) 
DILLTQSPVILSVSPGERVSFSCRASQSIGTNIHWYQQRTNGSPRLLIKYASESISGIPSRFSGSG
SGTDFTLSINSVESEDIADYYCQQNNNWPTTFGAGTKLELK
> Cetuximab heavy chain variable region VH (SEQ ID NO: 44)

QVQLKQSGPGLVQPSQSLSITCTVSGFSLTNYGVHWVRQSPGKGLEWLGVIWSGGNTDYN
TPFTSRLSINKDNSKSQVFFKMNSLQSNDTAIYYCARALTYYDYEFAYWGQGTLVTVSA

In one embodiment, the antibody portion used in the ADC of the present invention comprises all 6 CDR sequences of Cetuximab. In another embodiment, the antibody portion used in the ADC of the present invention comprises the heavy chain variable region sequence and the light chain variable region sequence of Cetuximab. In yet another embodiment, the antibody portion used in the ADC of the present invention comprises the heavy chain sequence and the light chain sequence of Cetuximab.

In some embodiments, the Ab unit of the ADC of the present invention comprises the three CDRs of the heavy chain variable region (VH) sequence of SEQ ID NO: 44 and the three CDRs of the light chain variable region (VL) sequence of SEQ ID NO: 43, and preferably, wherein the CDRs are defined according to Kabat or IMGT or a combination thereof.

In some embodiments, the Ab unit of the ADC of the present invention comprises 3 heavy chain complementarity determining regions (HCDR) and 3 light chain complementarity determining regions (LCDR), wherein:
According to Kabat definition, HCDR1 comprises or consists of the amino acid sequence of SEQ ID NO: 40, HCDR2 comprises or consists of the amino acid sequence of SEQ ID NO: 41, HCDR3 comprises or consists of the amino acid sequence of SEQ ID NO: 42, LCDR1 comprises or consists of the amino acid sequence of SEQ ID NO: 37, LCDR2 comprises or consists of the amino acid sequence of SEQ ID NO: 38, and LCDR3 comprises or consists of the amino acid sequence of SEQ ID NO: 39.

In one embodiment, the Ab unit of the ADC of the present invention comprises a heavy chain variable region, wherein the heavy chain variable region comprises the amino acid sequence shown in SEQ ID NO: 44, or an amino acid sequence having at least 80%, 85%, 90%, 95%, 96%, 97%, 98%, or 99% sequence identity thereto.

In one embodiment, the Ab unit of the ADC of the present invention comprises a light chain variable region, wherein the light chain variable region comprises the amino acid sequence shown in SEQ ID NO: 43, or an amino acid sequence having at least 80%, 85%, 90%, 95%, 96%, 97%, 98%, or 99% sequence identity thereto.

In some preferred embodiments, the Ab unit of the ADC of the present invention comprises a heavy chain variable region and a light chain variable region, wherein the heavy chain variable region comprises the amino acid sequence described in SEQ ID NO: 44, and wherein the light chain variable region comprises the amino acid sequence shown in SEQ ID NO: 43.

In some embodiments, the Ab unit of the ADC of the present invention preferably further comprises an antibody heavy chain constant region and/or light chain constant region. Preferably, the heavy chain constant region is a heavy chain constant region derived from a human immunoglobulin. Preferably, the light chain constant region is a light chain constant region derived from a human immunoglobulin. In some aspects, the heavy chain constant region comprised in the Ab unit can be of any isotype or subtype, e.g., an IgG1, IgG2, IgG3, or IgG4 isotype heavy chain constant region, and preferably an IgG1, IgG2, or IgG4 heavy chain constant region, especially a human IgG1 heavy chain constant region. In further aspects, the light chain constant region comprised in the Ab unit can be a κ light chain constant region or a λ light chain constant region, especially a human κ light chain constant region.

In some embodiments, the Ab unit of the ADC of the present invention comprises a human IgG1 heavy chain constant region. Preferably, the heavy chain constant region comprises the amino acid sequence of SEQ ID NO: 34, or an amino acid sequence comprising at least one, two, or three, but not more than 20, 10, or 5 amino acid changes relative to the amino acid sequence of SEQ ID NO: 34, or a sequence having at least 95-99% identity to the amino acid sequence of SEQ ID NO: 34.

In some embodiments, the Ab unit of the ADC of the present invention comprises a human κ light chain constant region. Preferably, the light chain constant region comprises the amino acid sequence of SEQ ID NO: 12, or an amino acid sequence comprising at least one, two, or three, but not more than 20, 10, or 5 amino acid changes relative to the amino acid sequence of SEQ ID NO: 12, or a sequence having at least 95-99% identity to the amino acid sequence of SEQ ID NO: 12.

In some embodiments, the Ab unit of the ADC of the present invention is a full-length antibody comprising a heavy chain constant region and a light chain constant region. In some embodiments, the Ab unit is a tetrameric structure formed by two light chains and two heavy chains. In further embodiments, the Ab unit is an IgG antibody, especially an IgG1 antibody.

In some preferred embodiments, the Ab unit of the ADC of the present invention comprises a heavy chain and a light chain, wherein: the heavy chain comprises the amino acid sequence shown in SEQ ID NO: 36, or an amino acid sequence having at least 80%, 85%, 90%, 95%, 96%, 97%, 98%, or 99% sequence identity thereto, or consists thereof. In other preferred embodiments, the Ab unit of the ADC of the present invention comprises a heavy chain and a light chain, wherein: the light chain comprises the amino acid sequence shown in SEQ ID NO: 35, or an amino acid sequence having at least 80%, 85%, 90%, 95%, 96%, 97%, 98%, or 99% sequence identity thereto.

In some more preferred embodiments, the Ab unit of the ADC of the present invention comprises:
(a) a heavy chain comprising the amino acid sequence of SEQ ID NO: 36, and
(b) a light chain comprising the amino acid sequence of SEQ ID NO: 35.

Cancers that can be treated with the EGFR-targeting ADCs of the present disclosure include, but are not limited to, various primary and metastatic solid tumors, such as lung cancer (e.g., lung adenocarcinoma, lung squamous cell carcinoma, and non-small cell lung cancer), head and neck cancer (e.g., head and neck squamous cell carcinoma), nasopharyngeal cancer, esophageal cancer, biliary tract cancer, colon cancer, colorectal cancer, pancreatic cancer, gastric cancer, and glioblastoma.

### Ab Unit Targeting EGFR and MET

In a particularly preferred embodiment, the antibody conjugates provided herein include an antibody or antigen-binding fragment that specifically binds to human EGFR and MET (also known as c-Met) (anti-EGFR/MET antibody), i.e., the antibody portion specifically targets the tumor-associated antigens EGFR and MET. Accordingly, in some aspects, the present disclosure provides an antibody-drug conjugate (ADC) comprising an antibody or antigen-binding fragment thereof that specifically binds EGFR and MET as the Ab unit of the ADC.

Drug resistance is a major challenge for targeted cancer therapies. For example, MET amplification or protein overexpression has been identified as an important mechanism of clinical resistance to EGFR inhibitors. Similarly, emerging evidence suggests that activation of the EGFR pathway may lead to resistance to c-MET-targeted inhibitors. Benedettini, E., et al., Met activation in non-small cell lung cancer is associated with de novo resistance to EGFR inhibitors and the development of brain metastasis. The American journal of pathology, 2010. 177(1): p. 415-423. Bertotti, 2015 Bertotti, A. and F. Sassi, Molecular Pathways: Sensitivity and Resistance to Anti-EGFR Antibodies. Clinical Cancer Research, 2015. Furthermore, co-expression of cMet and EGFR has been observed in various cancers, including non-small cell lung cancer, pancreatic cancer, colorectal cancer, head and neck squamous cell carcinoma, breast cancer, and esophagogastric cancer. Therefore, drug molecules based on multispecific antibodies (such as bispecific antibodies) targeting EGFR and c-MET have been proposed as a promising therapeutic option for EGFR/cMET singlepositive and double-positive tumors. Examples of EGFR/cMET dual-targeting antibodies include, but are not limited to, Amivantamab, AZD9592, Pamvatamig (MCLA-129), Bafisontamab (EMB01), and LY-3164530, which are approved or in clinical development.

In some embodiments, the antibody or antibody fragment thereof (e.g., antigen-binding fragment) that specifically binds to human EGFR and MET is selected from: Amivantamab, AZD9592, Pamvatamig (MCLA-129), Bafisontamab (EMB01), and LY-3164530, or an antibody fragment or site-specific mutant thereof, or other anti-human EGFR and MET antibody that recognizes the same epitope or competitively binds to human EGFR and MET. These antibodies can promote internalization at the relevant targets, enter tumor cells, and be degraded, and are therefore suitable drug delivery vehicles.

Amivantamab, also known as JNJ-61186372, is an anti-EGFR-MET bispecific antibody. This antibody has an EGFR antibody arm composed of one heavy chain and one light chain, and a MET antibody arm composed of one heavy chain and one light chain, and is a 1+1-type asymmetric IgG-like structure. This antibody binds to EGFR and MET, acts as an EGFR antagonist and MET inhibitor, and induces internalization of EGFR and MET on the cell surface. Amivantamab is indicated for the treatment of various cancers, especially non-small cell lung cancer, with EGFR exon 20 insertion mutations. Patients with non-small cell lung cancer often develop resistance to drugs targeting EGFR and MET alone. The development of amivantamab, by targeting both EGFR and MET, reduces the likelihood of developing resistance and has shown superior efficacy in animal models and clinical trials compared to EGFR inhibitors and MET inhibitors alone. The FDA has approved this antibody for the treatment of patients with metastatic non-small cell lung cancer (NSCLC) harboring EGFR exon 20 insertion mutations who have progressed on or after platinum-based chemotherapy. The antibody sequence of Amivantamab can be found in US20230174677A1 and CAS No. 2171511-58-1.

AZD9592 is currently in Phase 1 clinical studies as a drug molecule based on a MET-EGFR dual-target antibody, applicable to colorectal cancer, head and neck tumors, and non-small cell lung cancer. The bispecific antibody RAA22/B09-57 targeting EGFR and cMet involved in this drug molecule simultaneously targets both EGFR and c-Met and is a 1+1-type asymmetric bispecific antibody. This antibody exerts its effect by inhibiting hepatocyte growth factor receptor MET and epidermal growth factor receptor EGFR. The antibody sequence of RAA22/B09-57 can be found in US2023/0183358A1.

Pamvatamig, also known as MCLA-129, is a 1+1-type asymmetric bispecific antibody. The construction of this bispecific antibody employs DEKK mutations to prevent heavy chain mispairing and uses a common light chain to prevent light chain mispairing. Functionally, MCLA-129 can not only inhibit the phosphorylation of cMet and EGFR in tumors but also reverse tumor resistance to erlotinib. This bispecific antibody is suitable for targeting advanced malignant solid tumors, colorectal cancer, esophageal squamous cell carcinoma, metastatic non-small cell lung cancer, head and neck squamous cell carcinoma, gastric cancer, and advanced non-small cell lung cancer with EGFR mutations. The antibody sequence of Pamvatamig is disclosed in US11773170B2 and CAS No. 2750004-05-6.

Bafisontamab (EMB01) is an EGFR and cMet bispecific antibody constructed using the FIT-Ig^{®} bispecific antibody platform. Structurally, EMB-01 is a 2+2-type symmetric bispecific antibody structure. EMB-01 induces significantly stronger antitumor activity in PDX and CDX tumor models than anti-EGFR or anti-c-Met monoclonal antibodies alone and exhibits significant antitumor activity in anti-EGFR monoclonal antibody-resistant tumor models. The antibody sequence of EMB-01 is disclosed in WO2017136820 (as molecule FIT013a) and INN code 11851.

LY-3164530 is a bispecific antibody targeting c-MET/EGFR, constructed by fusing an scFv (cetuximab) to the N-terminus of the heavy chain of emibetuzumab (LY2875358), forming a 2+2 symmetric bispecific antibody. This antibody is of the IgG4 subtype and therefore has little or no relevant effector functions such as ADCC. In tumor mouse models, this bispecific antibody exhibits a certain effect in inhibiting tumor cell growth. The antibody sequence of LY-3164530 is disclosed under CAS 2069210-01-9.

Exemplary antibodies targeting EGFR and MET useful in the ADCs of the present disclosure can be antibodies or antigen-binding fragments comprising all 6 CDR sequences of an antibody selected from the group that specifically binds EGFR and all 6 CDR sequences that specifically binds MET (preferably, comprising the anti-EGFR heavy and light chain variable region sequences and the anti-MET heavy and light chain variable region sequences of an antibody selected from the group): Amivantamab, AZD9592, Pamvatamig (MCLA-129), Bafisontamab (EMB01), and LY-3164530. The present disclosure also contemplates anti-EGFR and MET antibodies disclosed in the following documents: WO2019031965A1, WO2022104236A2, WO2023069888A1, WO2023122588A2, WO2023172133A1, WO2023172134A1, WO2024002938A1, and WO2024153168A2.
> Amivantamab anti-EGFR heavy chain amino acid sequence (SEQ ID NO: 45) 
QVQLVESGGGVVQPGRSLRLSCAASGFTFSTYGMHWVRQAPGKGLEWVAVIWDDGSYKY
YGDSVKGRFTISRDNSKNTLYLQMNSLRAEDTAVYYCARDGITMVRGVMKDYFDYWGQG
TLVTVSSASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKRVEPKSCDKTHTCPPCPAPEL
LGGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPPSR
EEMTKNQVSLTCLVKGFYPSDIAVEWESNGQPENNYKTTPPVLDSDGSFLLYSKLTVDKSR
WQQGNVFSCSVMHEALHNHYTQKSLSLSPGK
> Amivantamab anti-EGFR light chain amino acid sequence (SEQ ID NO: 46) 
AIQLTQSPSSLSASVGDRVTITCRASQDISSALVWYQQKPGKAPKLLIYDASSLESGVPSRFS
GSESGTDFTLTISSLQPEDFATYYCQQFNSYPLTFGGGTKVEIKRTVAAPSVFIFPPSDEQLKS
GTASVVCLLNNFYPREAKVQWKVDNALQSGNSQESVTEQDSKDSTYSLSSTLTLSKADYE
KHKVYACEVTHQGLSSPVTKSFNRGEC
> Amivantamab anti-MET heavy chain amino acid sequence (SEQ ID NO: 47) 
QVQLVQSGAEVKKPGASVKVSCETSGYTFTSYGISWVRQAPGHGLEWMGWISAYNGYTN
YAQKLQGRVTMTTDTSTSTAYMELRSLRSDDTAVYYCARDLRGTNYFDYWGQGTLVTVSS
ASTKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKRVEPKSCDKTHTCPPCPAPELLGGPSVF
LFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSREEMTKNQ
VSLTCLVKGFYPSDIAVEWESNGQPENNYKTTPPVLDSDGSFFLYSRLTVDKSRWQQGNVFS
CSVMHEALHNHYTQKSLSLSPGK
> Amivantamab anti-MET light chain amino acid sequence (SEQ ID NO: 48) 
DIQMTQSPSSVSASVGDRVTITCRASQGISNWLAWFQHKPGKAPKLLIYAASSLLSGVPSRF
SGSGSGTDFTLTISSLQPEDFATYYCQQANSFPITFGQGTRLEIKRTVAAPSVFIFPPSDEQLKS
GTASVVCLLNNFYPREAKVQWKVDNALQSGNSQESVTEQDSKDSTYSLSSTLTLSKADYE
KHKVYACEVTHQGLSSPVTKSFNRGEC
> Amivantamab anti-EGFR heavy chain HCDR1 amino acid sequence (SEQ ID NO: 49)
   TYGMH
> Amivantamab anti-EGFR heavy chain HCDR2 amino acid sequence (SEQ ID NO: 50)
   VIWDDGSYKYYGDSVKG
> Amivantamab anti-EGFR heavy chain HCDR3 amino acid sequence (SEQ ID NO: 51)
   DGITMVRGVMKDYFDY
> Amivantamab anti-EGFR light chain LCDR1 amino acid sequence (SEQ ID NO: 52)
   RASQDISSALV
> Amivantamab anti-EGFR light chain LCDR2 amino acid sequence (SEQ ID NO: 53)
   DASSLES
> Amivantamab anti-EGFR light chain LCDR3 amino acid sequence (SEQ ID NO: 54)
   QQFNSYPLT
> Amivantamab anti-MET heavy chain HCDR1 amino acid sequence (SEQ ID NO: 55)
   SYGIS
> Amivantamab anti-MET heavy chain HCDR2 amino acid sequence (SEQ ID NO: 56)
   WISAYNGYTNYAQKLQG
> Amivantamab anti-MET heavy chain HCDR3 amino acid sequence (SEQ ID NO: 57)
   DLRGTNYFDY
> Amivantamab anti-MET light chain LCDR1 amino acid sequence (SEQ ID NO: 58)
   RASQGISNWLA
> Amivantamab anti-MET light chain LCDR2 amino acid sequence (SEQ ID NO: 59)
   AASSLLS
> Amivantamab anti-MET light chain LCDR3 amino acid sequence (SEQ ID NO: 60)
   QQANSFPIT
> Amivantamab anti-EGFR heavy chain variable region VH amino acid sequence (SEQ ID NO: 61) 
QVQLVESGGGVVQPGRSLRLSCAASGFTFSTYGMHWVRQAPGKGLEWVAVIWDDGSYKY
YGDSVKGRFTISRDNSKNTLYLQMNSLRAEDTAVYYCARDGITMVRGVMKDYFDYWGQG
 TLVTVSS
> Amivantamab anti-EGFR light chain variable region VL amino acid sequence (SEQ ID NO: 62)
> Amivantamab anti-MET heavy chain variable region VH amino acid sequence (SEQ ID NO: 63) 
QVQLVQSGAEVKKPGASVKVSCETSGYTFTSYGISWVRQAPGHGLEWMGWISAYNGYTN
YAQKLQGRVTMTTDTSTSTAYMELRSLRSDDTAVYYCARDLRGTNYFDYWGQGTLVTVSS
> Amivantamab anti-MET light chain variable region VL amino acid sequence (SEQ ID NO: 64)

DIQMTQSPSSVSASVGDRVTITCRASQGISNWLAWFQHKPGKAPKLLIYAASSLLSGVPSRF
SGSGSGTDFTLTISSLQPEDFATYYCQQANSFPITFGQGTRLEIK

In one embodiment, the antibody portion used in the ADC of the present invention comprises all 6 CDR sequences of the anti-EGFR arm and all 6 CDR sequences of the anti-MET arm of Amivantamab. In another embodiment, the antibody portion used in the ADC of the present invention comprises the heavy chain variable region sequence and light chain variable region sequence of the anti-EGFR arm and the heavy chain variable region sequence and light chain variable region sequence of the anti-MET arm of Amivantamab. In yet another embodiment, the antibody portion used in the ADC of the present invention comprises the anti-EGFR heavy chain sequence and light chain sequence and the anti-MET heavy chain sequence and light chain sequence of Amivantamab.

In some embodiments, the Ab unit of the ADC of the present invention comprises a first set of complementarity determining regions that bind EGFR and a second set of complementarity determining regions that bind MET, wherein the first set of complementarity determining regions comprises the three CDRs of the heavy chain variable region (VH) sequence of SEQ ID NO: 61 and the three CDRs of the light chain variable region (VL) sequence of SEQ ID NO: 62, and the second set of complementarity determining regions comprises the three CDRs of the heavy chain variable region (VH) sequence of SEQ ID NO: 63 and the three CDRs of the light chain variable region (VL) sequence of SEQ ID NO: 64. Preferably, the CDRs are defined according to Kabat or IMGT or a combination thereof.

In some embodiments, the Ab unit of the ADC of the present invention comprises a first set of complementarity determining regions that bind EGFR and a second set of complementarity determining regions that bind MET, wherein:
The first set of complementarity determining regions comprises 3 heavy chain complementarity determining regions (HCDR) and 3 light chain complementarity determining regions (LCDR), wherein:
According to Kabat definition, HCDR1 comprises or consists of the amino acid sequence of SEQ ID NO: 49, HCDR2 comprises or consists of the amino acid sequence of SEQ ID NO: 50, HCDR3 comprises or consists of the amino acid sequence of SEQ ID NO: 51, LCDR1 comprises or consists of the amino acid sequence of SEQ ID NO: 52, LCDR2 comprises or consists of the amino acid sequence of SEQ ID NO: 53, and LCDR3 comprises or consists of the amino acid sequence of SEQ ID NO: 54;
The second set of complementarity determining regions comprises 3 heavy chain complementarity determining regions (HCDR) and 3 light chain complementarity determining regions (LCDR), wherein:
   According to Kabat definition, HCDR1 comprises or consists of the amino acid sequence of SEQ ID NO: 55, HCDR2 comprises or consists of the amino acid sequence of SEQ ID NO: 56, HCDR3 comprises or consists of the amino acid sequence of SEQ ID NO: 57, LCDR1 comprises or consists of the amino acid sequence of SEQ ID NO: 58, LCDR2 comprises or consists of the amino acid sequence of SEQ ID NO: 59, and LCDR3 comprises or consists of the amino acid sequence of SEQ ID NO: 60.

In one embodiment, the Ab unit of the ADC of the present invention comprises a first heavy chain variable region and a first light chain variable region that bind EGFR and a second heavy chain variable region and a second light chain variable region that bind MET. In some embodiments, the first heavy chain variable region comprises the amino acid sequence shown in SEQ ID NO: 61, or an amino acid sequence having at least 80%, 85%, 90%, 95%, 96%, 97%, 98%, or 99% sequence identity thereto. In some embodiments, the first light chain variable region comprises the amino acid sequence shown in SEQ ID NO: 62, or an amino acid sequence having at least 80%, 85%, 90%, 95%, 96%, 97%, 98%, or 99% sequence identity thereto. In some embodiments, the second heavy chain variable region comprises the amino acid sequence shown in SEQ ID NO: 63, or an amino acid sequence having at least 80%, 85%, 90%, 95%, 96%, 97%, 98%, or 99% sequence identity thereto. In some embodiments, the second light chain variable region comprises the amino acid sequence shown in SEQ ID NO: 64, or an amino acid sequence having at least 80%, 85%, 90%, 95%, 96%, 97%, 98%, or 99% sequence identity thereto.

In some embodiments, the Ab unit of the ADC of the present invention comprises a first heavy chain variable region and a first light chain variable region that bind EGFR and a second heavy chain variable region and a second light chain variable region that bind MET, wherein:
the first heavy chain variable region comprises the amino acid sequence described in SEQ ID NO: 61, and the first light chain variable region comprises the amino acid sequence shown in SEQ ID NO: 62; and
the second heavy chain variable region comprises the amino acid sequence described in SEQ ID NO: 63, and the second light chain variable region comprises the amino acid sequence shown in SEQ ID NO: 64.

In some embodiments, the Ab unit of the ADC of the present disclosure preferably further comprises an antibody heavy chain constant region and/or light chain constant region. Preferably, the heavy chain constant region is a heavy chain constant region derived from a human immunoglobulin. Preferably, the light chain constant region is a light chain constant region derived from a human immunoglobulin. In some aspects, the heavy chain constant region comprised in the Ab unit can be of any isotype or subtype, e.g., an IgG1, IgG2, IgG3, or IgG4 isotype heavy chain constant region, and preferably an IgG1, IgG2, or IgG4 heavy chain constant region, especially a human IgG1 heavy chain constant region. In further aspects, the light chain constant region comprised in the Ab unit can be a κ light chain constant region or a λ light chain constant region, especially a human κ light chain constant region.

In some embodiments, the Ab unit of the ADC of the present disclosure comprises a human IgG1 heavy chain constant region. Preferably, the heavy chain constant region comprises the amino acid sequence of SEQ ID NO: 65 or 66, or an amino acid sequence comprising at least one, two, or three, but not more than 20, 10, or 5 amino acid changes relative to the amino acid sequence of SEQ ID NO: 65 or 66, or a sequence having at least 95-99% identity to the amino acid sequence of SEQ ID NO: 65 or 66.

An exemplary amino acid sequence of a human IgG1 heavy chain constant region (SEQ ID NO: 65)

ASTKGPSVFPLAPSSKSTSGGTAALGCLVKDYFPEPVTVSWNSGALTSGVHTFPAVLQS
SGLYSLSSVVTVPSSSLGTQTYICNVNHKPSNTKVDKRVEPKSCDKTHTCPPCPAPELLGGPS
VFLFPPKPKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPPSREEMTK
NQVSLTCLVKGFYPSDIAVEWESNGQPENNYKTTPPVLDSDGSFLLYSKLTVDKSRWQQGN
VFSCSVMHEALHNHYTQKSLSLSPGK

An exemplary amino acid sequence of a human IgG1 heavy chain constant region (SEQ ID NO: 66)

ASTKGPSVFPLAPSSKSTSGGTAALGCLVKDYFPEPVTVSWNSGALTSGVHTFPAVLQS
SGLYSLSSVVTVPSSSLGTQTYICNVNHKPSNTKVDKRVEPKSCDKTHTCPPCPAPELLGGPS
VFLFPPKPKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPPSREEMTK
NQVSLTCLVKGFYPSDIAVEWESNGQPENNYKTTPPVLDSDGSFFLYSRLTVDKSRWQQGN
VFSCSVMHEALHNHYTQKSLSLSPGK

In some embodiments, the Ab unit of the ADC of the present disclosure comprises a human κ light chain constant region. Preferably, the light chain constant region comprises the amino acid sequence of SEQ ID NO: 12, or an amino acid sequence comprising at least one, two, or three, but not more than 20, 10, or 5 amino acid changes relative to the amino acid sequence of SEQ ID NO: 12, or a sequence having at least 95-99% identity to the amino acid sequence of SEQ ID NO: 12.

In some embodiments, the Ab unit of the ADC of the present disclosure comprises or consists of a first heavy chain, a first light chain, a second heavy chain, and a second light chain, wherein from N-terminus to C-terminus:
- the first heavy chain comprises an anti-EGFR heavy chain variable region and a first heavy chain constant region;
- the first light chain comprises an anti-EGFR light chain variable region and a first light chain constant region;
- the second heavy chain comprises an anti-MET heavy chain variable region and a second heavy chain constant region;
- the second light chain comprises an anti-MET light chain variable region and a second light chain constant region.

In some embodiments, the first and second heavy chain constant regions are heavy chain constant regions as defined above, preferably human IgG1 constant regions; the first and second light chain constant regions are light chain constant regions as defined above, preferably human κ light chain constant regions. In some embodiments, the first heavy chain constant region comprises or consists of the amino acid sequence of SEQ ID NO: 65, and the second heavy chain constant region comprises or consists of the amino acid sequence of SEQ ID NO: 66. In some embodiments, the first and second light chain constant regions each comprise or consist of the amino acid sequence of SEQ ID NO: 12.

In some embodiments, the Ab unit of the ADC of the present disclosure is a full-length antibody comprising a heavy chain constant region and a light chain constant region. In some embodiments, the Ab unit is a tetrameric structure formed by two light chains and two heavy chains. In further embodiments, the Ab unit is an IgG antibody, especially an IgG1 antibody.

In some preferred embodiments, the Ab unit of the ADC of the present disclosure comprises a first heavy chain, a first light chain, a second heavy chain, and a second light chain, wherein:
the first heavy chain comprises the amino acid sequence shown in SEQ ID NO: 45, or an amino acid sequence having at least 80%, 85%, 90%, 95%, 96%, 97%, 98%, or 99% sequence identity thereto, or consists thereof; and the first light chain comprises the amino acid sequence shown in SEQ ID NO: 46, or an amino acid sequence having at least 80%, 85%, 90%, 95%, 96%, 97%, 98%, or 99% sequence identity thereto;
the second heavy chain comprises the amino acid sequence shown in SEQ ID NO: 47, or an amino acid sequence having at least 80%, 85%, 90%, 95%, 96%, 97%, 98%, or 99% sequence identity thereto, or consists thereof; and the second light chain comprises the amino acid sequence shown in SEQ ID NO: 48, or an amino acid sequence having at least 80%, 85%, 90%, 95%, 96%, 97%, 98%, or 99% sequence identity thereto.

In some more preferred embodiments, the Ab unit of the ADC of the present disclosure comprises:
(a) an anti-EGFR heavy chain comprising the amino acid sequence of SEQ ID NO: 45 and an anti-EGFR light chain comprising the amino acid sequence of SEQ ID NO: 46, and
(b) an anti-MET heavy chain comprising the amino acid sequence of SEQ ID NO: 47 and an anti-MET light chain comprising the amino acid sequence of SEQ ID NO: 48.

Cancers that can be treated with the **EGFR and MET**-targeting ADCs of the present disclosure are EGFR and/or MET positive cancers, such as solid tumors and hematological tumors, including but not limited to: non-small cell lung cancer, breast cancer, lung cancer, colorectal cancer, pancreatic cancer, ovarian cancer, gastric cancer, prostate cancer, head and neck tumors, liver cancer, bladder cancer, melanoma, esophageal cancer, renal tumors, thyroid cancer, cervical cancer, lymphoma, uterine cancer, skin tumors, colon cancer, sarcoma, leukemia, glioblastoma, etc.

### Ab Unit Targeting Nectin4

In a particularly preferred embodiment, the antibody conjugates provided herein include an antibody or antigen-binding fragment that specifically binds to human Nectin-4 (anti-Nectin-4 antibody), i.e., the antibody portion specifically targets the tumor-associated antigen Nectin-4. Accordingly, in some aspects, the present disclosure provides an antibody-drug conjugate (ADC) comprising an antibody or antigen-binding fragment thereof that specifically binds Nectin-4 as the Ab unit of the ADC.

Nectin-4 (Nectin cell adhesion molecule 4), also known as poliovirus receptor-related (PRR) protein, is a type I transmembrane cell adhesion molecule belonging to the Nectin family. Nectin-4 forms physical connections between adjacent cells and is essential for achieving intercellular communication, migration, and other important cellular processes. The Nectin-4 protein is specifically expressed in embryos and placentas, is expressed at low levels in only a few normal adult tissues (including skin), but is abnormally highly expressed in tumor tissues. The overexpression of Nectin-4 in various tumor cells serves as a marker for cancer recurrence and metastasis and is associated with poor prognosis in various cancers. Cancers overexpressing Nectin-4 include, for example, breast cancer, cervical cancer, ovarian cancer, gastric cancer, esophageal cancer, head and neck cancer, lung cancer, non-small cell lung cancer, melanoma, bladder cancer, thyroid cancer, as well as hepatocellular carcinoma and urothelial carcinoma. Due to the specific expression of the Nectin-4 target, Nectin-4 has become a potential biomarker and promising therapeutic target. See, e.g., Jeffrey L Wong et al., Expert Opin Biol Ther. 2021 May 24;21(7):863-873. doi: 10.1080/14712598.2021.1929168, "Targeting nectin-4 by antibody-drug conjugates for the treatment of urothelial carcinoma".

Antibodies targeting human Nectin-4 useful in the ADCs of the present disclosure can be prepared using antibody preparation techniques known in the art. For example, anti-Nectin4 antibodies can be obtained by immunizing animals with human Nectin4 (UniProtKB Accession No. Q96NY8) or a polypeptide comprising the amino acid sequence of the extracellular domain of Nectin-4, harvesting antibodies from the immunized animals, and purifying and preferably humanizing them. Furthermore, fully human sequence anti-human Nectin4 antibodies can be obtained using yeast display libraries expressing human immunoglobulin sequences, transgenic animals, or the like.

In some embodiments, the antibody or antibody fragment thereof (e.g., antigen-binding fragment) that specifically binds to human Nectin-4 can be selected from Enfortumab, LY4052031, 9MW2821, BAT8007, SBT6290, or an antibody fragment thereof, or other anti-human Nectin4 antibody that recognizes the same epitope or competitively binds to human Nectin4.

Exemplary antibodies targeting human Nectin4 useful in the ADCs of the present disclosure can be antibodies or antigen-binding fragments comprising all 6 CDR sequences of an antibody selected from the group (preferably, comprising the heavy chain variable region and light chain variable region sequences of an antibody selected from the group): Enfortumab, LY4052031, 9MW2821, BAT8007, SBT6290. The present disclosure also contemplates anti-human Nectin4 antibodies disclosed in the following documents: WO2012047724, WO2022228406, WO2022228563, WO2024088390, WO2024012536, WO2024038075, WO2024017992, US 12049500B2, US11179473B2, US11292837B2, US10675357B2, CN119013302A.

Enfortumab is a monospecific bivalent IgG antibody that targets and binds Nectin4. This antibody can directly bind to Nectin4-expressing cancer cells and trigger antibody internalization. Its sequence can be found in NCATS Inxight Drugs, as well as FDA UNII U1HUE4W970 and CAS NO. 1448664-46-7.
> Enfortumab light chain amino acid sequence (SEQ ID NO: 67) 
DIQMTQSPSSVSASVGDRVTITCRASQGISGWLAWYQQKPGKAPKFLIYAASTLQSGV
PSRFSGSGSGTDFTLTISSLQPEDFATYYCQQANSFPPTFGGGTKVEIKRTVAAPSVFIFPPSD
EQLKSGTASVVCLLNNFYPREAKVQWKVDNALQSGNSQESVTEQDSKDSTYSLSSTLTLS
KADYEKHKVYACEVTHQGLSSPVTKSFNRGEC
> Enfortumab heavy chain amino acid sequence (SEQ ID NO: 68) 
EVQLVESGGGLVQPGGSLRLSCAASGFTFSSYNMNWVRQAPGKGLEWVSYISSSSSTI
YYADSVKGRFTISRDNAKNSLSLQMNSLRDEDTAVYYCARAYYYGMDVWGQGTTVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKRVEPKSCDKTHTCPPCPAPELLGGPSV
FLFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSREEMTK
NQVSLTCLVKGFYPSDIAVEWESNGQPENNYKTTPPVLDSDGSFFLYSKLTVDKSRWQQG
NVFSCSVMHEALHNHYTQKSLSLSPGK
>Enfortumab light chain LCDR1 sequence (SEQ ID NO: 69)
   RASQGISGWLA
>Enfortumab light chain LCDR2 sequence (SEQ ID NO: 70)
   AASTLQS
>Enfortumab light chain LCDR3 sequence (SEQ ID NO: 71)
   QQANSFPPT
>Enfortumab heavy chain HCDR1 sequence (SEQ ID NO: 72)
   SYNMN
>Enfortumab heavy chain HCDR2 sequence (SEQ ID NO: 73)
   YISSSSSTIYYADSVKG
>Enfortumab heavy chain HCDR3 sequence (SEQ ID NO: 74)
   AYYYGMDV
>Enfortumab light chain variable region VL (SEQ ID NO: 75) 
DIQMTQSPSSVSASVGDRVTITCRASQGISGWLAWYQQKPGKAPKFLIYAASTLQSGV
PSRFSGSGSGTDFTLTISSLQPEDFATYYCQQANSFPPTFGGGTKVEIK
>Enfortumab heavy chain variable region VH (SEQ ID NO: 76)

EVQLVESGGGLVQPGGSLRLSCAASGFTFSSYNMNWVRQAPGKGLEWVSYISSSSSTI
YYADSVKGRFTISRDNAKNSLSLQMNSLRDEDTAVYYCARAYYYGMDVWGQGTTVTVS
S

In one embodiment, the antibody portion used in the ADC of the present disclosure comprises all 6 CDR sequences of Enfortumab. In another embodiment, the antibody portion used in the ADC of the present disclosure comprises the heavy chain variable region sequence and the light chain variable region sequence of Enfortumab. In yet another embodiment, the antibody portion used in the ADC of the present disclosure comprises the heavy chain sequence and the light chain sequence of Enfortumab.

In some embodiments, the Ab unit of the ADC of the present disclosure comprises the three CDRs of the heavy chain variable region (VH) sequence of SEQ ID NO: 76 and the three CDRs of the light chain variable region (VL) sequence of SEQ ID NO: 75, and preferably, wherein the CDRs are defined according to Kabat or IMGT or a combination thereof.

In some embodiments, the Ab unit of the ADC of the present disclosure comprises 3 heavy chain complementarity determining regions (HCDR) and 3 light chain complementarity determining regions (LCDR), wherein:
According to Kabat definition, HCDR1 comprises or consists of the amino acid sequence of SEQ ID NO: 72, HCDR2 comprises or consists of the amino acid sequence of SEQ ID NO: 73, HCDR3 comprises or consists of the amino acid sequence of SEQ ID NO: 74, LCDR1 comprises or consists of the amino acid sequence of SEQ ID NO: 69, LCDR2 comprises or consists of the amino acid sequence of SEQ ID NO: 70, and LCDR3 comprises or consists of the amino acid sequence of SEQ ID NO: 71.

In one embodiment, the Ab unit of the ADC of the present disclosure comprises a heavy chain variable region, wherein the heavy chain variable region comprises the amino acid sequence shown in SEQ ID NO: 76, or an amino acid sequence having at least 80%, 85%, 90%, 95%, 96%, 97%, 98%, or 99% sequence identity thereto.

In one embodiment, the Ab unit of the ADC of the present disclosure comprises a light chain variable region, wherein the light chain variable region comprises the amino acid sequence shown in SEQ ID NO: 75, or an amino acid sequence having at least 80%, 85%, 90%, 95%, 96%, 97%, 98%, or 99% sequence identity thereto.

In some preferred embodiments, the Ab unit of the ADC of the present disclosure comprises a heavy chain variable region and a light chain variable region, wherein the heavy chain variable region comprises the amino acid sequence described in SEQ ID NO: 76, and wherein the light chain variable region comprises the amino acid sequence shown in SEQ ID NO: 75.

In some embodiments, the Ab unit of the ADC of the present disclosure preferably further comprises an antibody heavy chain constant region and/or light chain constant region. Preferably, the heavy chain constant region is a heavy chain constant region derived from a human immunoglobulin. Preferably, the light chain constant region is a light chain constant region derived from a human immunoglobulin. In some aspects, the heavy chain constant region comprised in the Ab unit can be of any isotype or subtype, e.g., an IgG1, IgG2, IgG3, or IgG4 isotype heavy chain constant region, and preferably an IgG1, IgG2, or IgG4 heavy chain constant region, especially a human IgG1 heavy chain constant region. In further aspects, the light chain constant region comprised in the Ab unit can be a κ light chain constant region or a λ light chain constant region, especially a human κ light chain constant region.

In some embodiments, the Ab unit of the ADC of the present disclosure comprises a human IgG1 heavy chain constant region. Preferably, the heavy chain constant region comprises the amino acid sequence of SEQ ID NO: 23, or an amino acid sequence comprising at least one, two, or three, but not more than 20, 10, or 5 amino acid changes relative to the amino acid sequence of SEQ ID NO: 23, or a sequence having at least 95-99% identity to the amino acid sequence of SEQ ID NO: 23.

In some embodiments, the Ab unit of the ADC of the present disclosure comprises a human κ light chain constant region. Preferably, the light chain constant region comprises the amino acid sequence of SEQ ID NO: 12, or an amino acid sequence comprising at least one, two, or three, but not more than 20, 10, or 5 amino acid changes relative to the amino acid sequence of SEQ ID NO: 12, or a sequence having at least 95-99% identity to the amino acid sequence of SEQ ID NO: 12.

In some embodiments, the Ab unit of the ADC of the present disclosure is a full-length antibody comprising a heavy chain constant region and a light chain constant region. In some embodiments, the Ab unit is a tetrameric structure formed by two light chains and two heavy chains. In further embodiments, the Ab unit is an IgG antibody, especially an IgG1 antibody.

In some preferred embodiments, the Ab unit of the ADC of the present disclosure comprises a heavy chain and a light chain, wherein: the heavy chain comprises the amino acid sequence shown in SEQ ID NO: 68, or an amino acid sequence having at least 80%, 85%, 90%, 95%, 96%, 97%, 98%, or 99% sequence identity thereto, or consists thereof. In other preferred embodiments, the Ab unit of the ADC of the present disclosure comprises a heavy chain and a light chain, wherein: the light chain comprises the amino acid sequence shown in SEQ ID NO: 67, or an amino acid sequence having at least 80%, 85%, 90%, 95%, 96%, 97%, 98%, or 99% sequence identity thereto.

In some more preferred embodiments, the Ab unit of the ADC of the present disclosure comprises:
(a) a heavy chain comprising the amino acid sequence of SEQ ID NO: 68, and
(b) a light chain comprising the amino acid sequence of SEQ ID NO: 67.

Cancers that can be treated with the Nectin-4-targeting ADCs of the present disclosure are Nectin4-positive cancers, including but not limited to breast cancer, cervical cancer, ovarian cancer, gastric cancer, esophageal cancer, head and neck cancer, lung cancer, non-small cell lung cancer, melanoma, bladder cancer, thyroid cancer, as well as hepatocellular carcinoma and urothelial carcinoma.

### Drug P Unit

The drug P unit of the antibody-drug conjugate is also referred to herein as the payload of the ADC drug.

In some embodiments, the drug P unit useful in the ADCs of the present disclosure is a KRas mutant inhibitor, including but not limited to inhibitors of G12C mutant, G12D mutant, G12V mutant, G12A mutant, G12R mutant, G12S mutant, and G13D mutant. In some embodiments, the drug P unit useful in the ADCs of the present disclosure is a KRas mutant inhibitor described in the applicant's previously filed PCT application PCT/CN2023/122129, more specifically the KRas mutant inhibitors defined below.

In other embodiments, the drug P unit useful in the ADCs of the present disclosure is a pan-Ras inhibitor, which is capable of inhibiting almost all Ras isoforms regardless of mutation status, such as some of the drug P units exemplified or illustrated below, including but not limited to P8, P9, P12, P13, P16-23, P25-37, etc.

**Embodiment 1:** The drug P unit useful in the ADCs of the present disclosure is specifically a compound of formula (I), a stereoisomer, tautomer, stable isotopic variant, pharmaceutically acceptable salt, or solvate thereof, 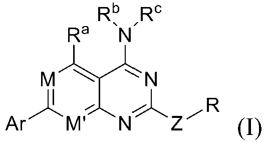 wherein:
M is selected from N or C-R₁;
M' is selected from N or C-R₁';
R₁ and R₁' are each independently selected from H, halogen, CN, -C₁₋₆ alkyl optionally substituted by halogen, and -OC₁₋₆ alkyl optionally substituted by halogen;
R^{a} is selected from H, halogen, CN, -C₁₋₆ alkyl optionally substituted by halogen, -C₂₋₆ alkynyl optionally substituted by halogen, and -OC₁₋₆ alkyl optionally substituted by halogen or deuterium;
R^{b} and R^{c} together with the N atom to which they are attached form 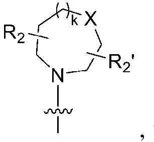 wherein X is selected from CH₂, N, and O;
R₂ and R₂' are each independently selected from H, OH, or -C₁₋₆ alkyl optionally substituted by halogen; or R₂ and R₂' attached to non-adjacent ring carbon atoms together form an intra-ring bridge -(CH₂)₁₋₂- or -CH₂=CH₂-; or R₂ and R₂' attached to the same ring carbon atom together with the ring carbon atom to which they are attached form a 4-6 membered spirocycloalkyl or a 4-6 membered spiroheterocycloalkyl containing 1 or 2 heteroatoms selected from N and O;
Ar is selected from 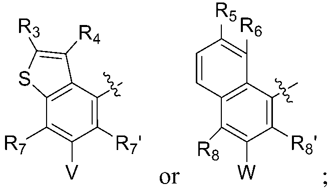
R₃ is selected from H, -NH₂, -NHC₁₋₆ alkyl, -N(C₁₋₆ alkyl)₂, and -OC₁₋₆ alkyl;
R₄ is selected from -CN, halogen, -NO₂, -C₂₋₆ alkynyl optionally substituted by halogen, and - C₁₋₆ alkyl optionally substituted by halogen;
R₅ is selected from H, -CN, halogen, -NO₂, and halogen-substituted -C₁₋₆ alkyl;
R₆ is selected from H, halogen, CN, -C₁₋₆ alkyl, and -C₂₋₆ alkynyl, wherein the -C₁₋₆ alkyl and - C₂₋₆ alkynyl are each independently optionally substituted by halogen;
R₇, R₇', R₈, and R₈' are each independently selected from H, halogen, CN, -NO₂, and -C₁₋₆ alkyl optionally substituted by halogen;
V and W are each independently selected from H, halogen, -C₁₋₆ alkyl, OH, and NH₂;
Z is selected from O, N, and CH₂;
R is 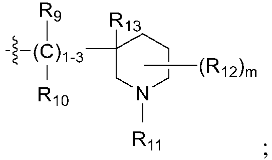
R₉ and R₁₀ are each independently selected from H, deuterium, -C₁₋₆ alkyl, and -(CH₂)ₙ-C₃₋₆ cycloalkyl, wherein the -C₁₋₆ alkyl and -C₃₋₆ cycloalkyl are each independently optionally substituted by deuterium, halogen, or -O-C₁₋₆ alkyl, or R₉ and R₁₀ attached to the same carbon atom together with the carbon atom to which they are attached form a C₃₋₄ cycloalkyl;
R₁₁ is selected from H, -C₁₋₆ alkyl, -C₂₋₆ alkenyl, -C₂₋₆ alkynyl, and -(CH₂)ₙ-C₃₋₆ cycloalkyl, wherein the C₁₋₆ alkyl, -C₂₋₆ alkenyl, -C₂₋₆ alkynyl, or C₃₋₆ cycloalkyl are each independently optionally substituted by deuterium, halogen, CN, or -O-C₁₋₆ alkyl;
R₁₂ is selected from H, halogen, -CN, -OH, -NH₂, -NHC₁₋₆ alkyl, -N(C₁₋₆ alkyl)₂, -O-C₁₋₆ alkyl, -O-C₃₋₆ cycloalkyl, -C₁₋₆ alkyl, -C₂₋₆ alkenyl, -C₂₋₆ alkynyl, -(CH₂)ₙ-C₃₋₆ cycloalkyl, and =C(R^{d})₂, wherein R^{d} is each independently selected from H, halogen, and -C₁₋₆ alkyl optionally substituted by halogen, wherein each occurrence of C₁₋₆ alkyl, -C₂₋₆ alkenyl, -C₂₋₆ alkynyl, or C₃₋₆ cycloalkyl is each independently optionally substituted by halogen, CN, or -OC₁₋₆ alkyl;
R₁₃ is selected from H, -C₁₋₆ alkyl, and -(CH₂)ₙ-C₃₋₆ cycloalkyl, wherein the -C₁₋₆ alkyl and -C₃₋₆ cycloalkyl are each independently optionally substituted by halogen or -O-C₁₋₆ alkyl;
k is selected from 0 or 1;
m is selected from 0 to 6; and
n is selected from 0 to 2;

Thus, formula (I) can be specifically represented as:

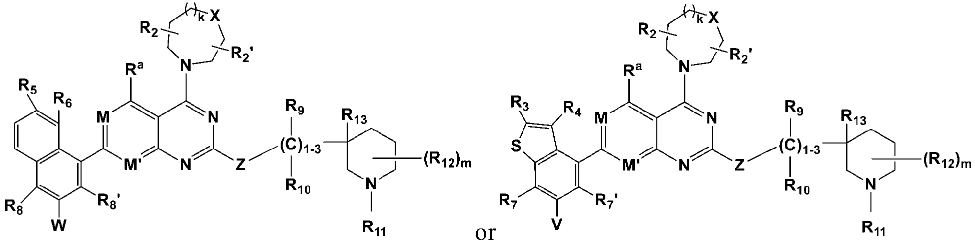

**Embodiment 1.1:** The compound of formula (I) according to Embodiment 1, a stereoisomer, tautomer, stable isotopic variant, pharmaceutically acceptable salt, or solvate thereof, wherein M is C-R₁.

**Embodiment 1.1.1:** The compound of formula (I) according to Embodiment 1.1, a stereoisomer, tautomer, stable isotopic variant, pharmaceutically acceptable salt, or solvate thereof, wherein R₁ is H.

**Embodiment 1.1.2:** The compound of formula (I) according to Embodiment 1.1, a stereoisomer, tautomer, stable isotopic variant, pharmaceutically acceptable salt, or solvate thereof, wherein R₁ is halogen, e.g., F, Cl.

**Embodiment 1.1.3:** The compound of formula (I) according to Embodiment 1.1, a stereoisomer, tautomer, stable isotopic variant, pharmaceutically acceptable salt, or solvate thereof, wherein R₁ is CN.

**Embodiment 1.1.4:** The compound of formula (I) according to Embodiment 1.1, a stereoisomer, tautomer, stable isotopic variant, pharmaceutically acceptable salt, or solvate thereof, wherein R₁ is - C₁₋₆ alkyl optionally substituted by halogen, preferably -C₁₋₃ alkyl optionally substituted by halogen, more preferably -C₁₋₃ alkyl substituted by 1-3 halogens, most preferably -C₁₋₃ alkyl substituted by 1-3 F, e.g., -CF₃; examples of R₁ include, but are not limited to -CH₃, -CH₂CH₃, -CH₂CH₂CH₃, - CH(CH₃)(CH₃), -CH₂CH₂CH₂CH₃, -CH₂CH(CH₃)CH₃, -C(CH₃)₃, -CH₂Cl, -CH₂F, -CHF₂, -CF₃, - CH₂CH₂F, -CH₂CHF₂, -CH₂CF₃, -CH₂CH₂CH₂F, -CH₂CH₂CHF₂, -CH₂CH₂CF₃, -C₂F₅.

**Embodiment 1.1.5:** The compound of formula (I) according to Embodiment 1.1, a stereoisomer, tautomer, stable isotopic variant, pharmaceutically acceptable salt, or solvate thereof, wherein R₁ is - OC₁₋₆ alkyl optionally substituted by halogen, preferably -OC₁₋₃ alkyl optionally substituted by halogen, e.g., -OC₁₋₃ alkyl, such as -OCH₃, -OCH₂CH₃.

**Embodiment 1.1.6:** The compound of formula (I) according to Embodiment 1.1, a stereoisomer, tautomer, stable isotopic variant, pharmaceutically acceptable salt, or solvate thereof, wherein R₁ is selected from halogen and -C₁₋₆ alkyl optionally substituted by halogen, preferably halogen and -C₁₋₃ alkyl substituted by halogen, e.g., F, Cl, and -CF₃.

**Embodiment 1.1.7:** The compound of formula (I) according to any one of Embodiments 1.1 to 1.1.6, a stereoisomer, tautomer, stable isotopic variant, pharmaceutically acceptable salt, or solvate thereof, wherein R^{a} is H.

**Embodiment 1.1.8:** The compound of formula (I) according to any one of Embodiments 1.1 to 1.1.6, a stereoisomer, tautomer, stable isotopic variant, pharmaceutically acceptable salt, or solvate thereof, wherein R^{a} is CN or halogen, e.g., F, Cl.

**Embodiment 1.1.9:** The compound of formula (I) according to any one of Embodiments 1.1 to 1.1.6, a stereoisomer, tautomer, stable isotopic variant, pharmaceutically acceptable salt, or solvate thereof, wherein R^{a} is -C₁₋₆ alkyl optionally substituted by halogen, as generally or specifically defined in Embodiment 1.1.4.

**Embodiment 1.1.10:** The compound of formula (I) according to any one of Embodiments 1.1 to 1.1.6, a stereoisomer, tautomer, stable isotopic variant, pharmaceutically acceptable salt, or solvate thereof, wherein R^{a} is -C₂₋₆ alkynyl optionally substituted by halogen, preferably -C₂₋₄ alkynyl optionally substituted by halogen, more preferably -C₂₋₄ alkynyl, e.g., but not limited to,

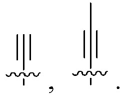

**Embodiment 1.1.11:** The compound of formula (I) according to any one of Embodiments 1.1 to 1.1.6, a stereoisomer, tautomer, stable isotopic variant, pharmaceutically acceptable salt, or solvate thereof, wherein R^{a} is -OC₁₋₆ alkyl optionally substituted by halogen or deuterium, preferably -OC₁₋₃ alkyl optionally substituted by halogen or deuterium, more preferably -OC₁₋₃ alkyl optionally substituted by deuterium, e.g., -OCH₃, -OCD₃, -OCH₂CH₃, most preferably -OCH₃.

**Embodiment 1.1.12:** The compound of formula (I) according to any one of Embodiments 1.1 to 1.1.11, a stereoisomer, tautomer, stable isotopic variant, pharmaceutically acceptable salt, or solvate thereof, wherein M' is N.

**Embodiment 1.1.13:** The compound of formula (I) according to any one of Embodiments 1.1 to 1.1.11, a stereoisomer, tautomer, stable isotopic variant, pharmaceutically acceptable salt, or solvate thereof, wherein M' is C-R₁'.

**Embodiment 1.1.14:** The compound of formula (I) according to Embodiment 1.1.13, a stereoisomer, tautomer, stable isotopic variant, pharmaceutically acceptable salt, or solvate thereof, wherein R₁' is selected from halogen, e.g., F, Cl, preferably F.

**Embodiment 1.1.15:** The compound of formula (I) according to Embodiment 1.1.13, a stereoisomer, tautomer, stable isotopic variant, pharmaceutically acceptable salt, or solvate thereof, wherein R₁' is CN.

**Embodiment 1.1.16:** The compound of formula (I) according to Embodiment 1.1.13, a stereoisomer, tautomer, stable isotopic variant, pharmaceutically acceptable salt, or solvate thereof, wherein R₁' is selected from -C₁₋₆ alkyl optionally substituted by halogen and -OC₁₋₆ alkyl optionally substituted by halogen, each as generally or specifically defined in Embodiments 1.1.4 and 1.1.5.

**Embodiment 1.2:** The compound of formula (I) according to Embodiment 1, a stereoisomer, tautomer, stable isotopic variant, pharmaceutically acceptable salt, or solvate thereof, wherein M is N.

**Embodiment 1.2.1:** The compound of formula (I) according to Embodiment 1.2, a stereoisomer, tautomer, stable isotopic variant, pharmaceutically acceptable salt, or solvate thereof, wherein R^{a} is H.

**Embodiment 1.2.2:** The compound of formula (I) according to Embodiment 1.2, a stereoisomer, tautomer, stable isotopic variant, pharmaceutically acceptable salt, or solvate thereof, wherein R^{a} is CN, or R^{a} is halogen, e.g., F, Cl.

**Embodiment 1.2.3:** The compound of formula (I) according to Embodiment 1.2, a stereoisomer, tautomer, stable isotopic variant, pharmaceutically acceptable salt, or solvate thereof, wherein R^{a} is - C₁₋₆ alkyl optionally substituted by halogen, as generally or specifically defined in Embodiment 1.1.4.

**Embodiment 1.2.4:** The compound of formula (I) according to Embodiment 1.2, a stereoisomer, tautomer, stable isotopic variant, pharmaceutically acceptable salt, or solvate thereof, wherein R^{a} is - C₂₋₆ alkynyl optionally substituted by halogen, preferably -C₂₋₄ alkynyl optionally substituted by halogen, more preferably -C₂₋₄ alkynyl, e.g., but not limited to,

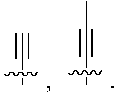

**Embodiment 1.2.5:** The compound of formula (I) according to Embodiment 1.2, a stereoisomer, tautomer, stable isotopic variant, pharmaceutically acceptable salt, or solvate thereof, wherein R^{a} is - OC₁₋₆ alkyl optionally substituted by halogen or deuterium, preferably -OC₁₋₃ alkyl optionally substituted by halogen or deuterium, more preferably -OC₁₋₃ alkyl optionally substituted by deuterium, e.g., -OCH₃, -OCD₃, -OCH₂CH₃, most preferably -OCH₃.

**Embodiment 1.2.6:** The compound of formula (I) according to any one of Embodiments 1.2 to 1.2.5, a stereoisomer, tautomer, stable isotopic variant, pharmaceutically acceptable salt, or solvate thereof, wherein M' is N.

**Embodiment 1.2.7:** The compound of formula (I) according to any one of Embodiments 1.2 to 1.2.5, a stereoisomer, tautomer, stable isotopic variant, pharmaceutically acceptable salt, or solvate thereof, wherein M' is C-R₁'.

**Embodiment 1.2.8:** The compound of formula (I) according to Embodiment 1.2.7, a stereoisomer, tautomer, stable isotopic variant, pharmaceutically acceptable salt, or solvate thereof, wherein R₁' is selected from halogen, e.g., F, Cl, preferably F.

**Embodiment 1.2.9:** The compound of formula (I) according to Embodiment 1.2.7, a stereoisomer, tautomer, stable isotopic variant, pharmaceutically acceptable salt, or solvate thereof, wherein R₁' is CN.

**Embodiment 1.2.10:** The compound of formula (I) according to Embodiment 1.2.7, a stereoisomer, tautomer, stable isotopic variant, pharmaceutically acceptable salt, or solvate thereof, wherein R₁' is selected from -C₁₋₆ alkyl optionally substituted by halogen and -OC₁₋₆ alkyl optionally substituted by halogen, each as generally or specifically defined in Embodiments 1.1.4 and 1.1.5.

**Embodiment 1.3:** The compound of formula (I) according to Embodiment 1, a stereoisomer, tautomer, stable isotopic variant, pharmaceutically acceptable salt, or solvate thereof, wherein the structural fragment 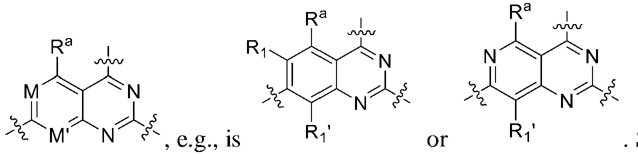 Specifically,
M is C-R₁, wherein R₁ is selected from halogen (preferably F or Cl) or -C₁₋₆ alkyl optionally substituted by halogen (preferably -C₁₋₃ alkyl substituted by halogen, more preferably -CF₃), R^{a} is H, and M' is C-R₁' and R₁' is halogen, preferably F; or
M is N, R^{a} is selected from H, -C₂₋₆ alkynyl (preferably -C₂₋₄ alkynyl, more preferably -C=CH), and -OC₁₋₆ alkyl (preferably -OC₁₋₃ alkyl optionally substituted by deuterium, more preferably - OCH₃, -OCD₃, -OCH₂CH₃), and M' is C-R₁' and R₁' is halogen, preferably F;

Specific examples include, but are not limited to:

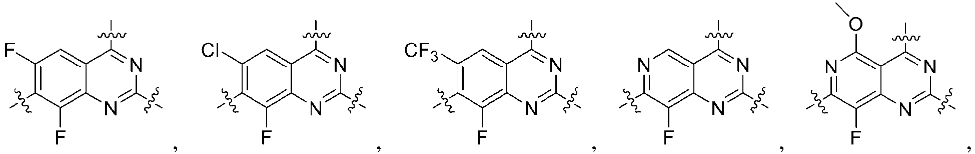

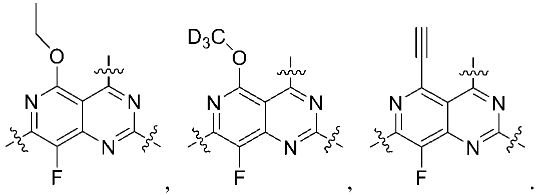

**Embodiment 2.1:** The compound of formula (I) according to any one of Embodiments 1 to 1.3, a stereoisomer, tautomer, stable isotopic variant, pharmaceutically acceptable salt, or solvate thereof, wherein Ar is

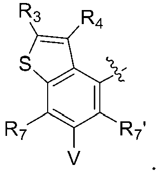

**Embodiment 2.1.1:** The compound of formula (I) according to Embodiment 2.1, a stereoisomer, tautomer, stable isotopic variant, pharmaceutically acceptable salt, or solvate thereof, wherein R₃ is H; or R₃ is selected from -NH₂, -NHC₁₋₆ alkyl, and N(C₁₋₆ alkyl)₂, preferably selected from -NH₂, - NHC₁₋₃ alkyl, and N(C₁₋₃ alkyl)₂, most preferably -NH₂.

**Embodiment 2.1.2:** The compound of formula (I) according to Embodiment 2.1, a stereoisomer, tautomer, stable isotopic variant, pharmaceutically acceptable salt, or solvate thereof, wherein R₃ is - OC₁₋₆ alkyl, preferably -OC₁₋₃ alkyl, e.g., -OCH₃, -OCH₂CH₃.

**Embodiment 2.1.3:** The compound of formula (I) according to any one of Embodiments 2.1 to 2.1.2, a stereoisomer, tautomer, stable isotopic variant, pharmaceutically acceptable salt, or solvate thereof, wherein R₄ is CN.

**Embodiment 2.1.4:** The compound of formula (I) according to any one of Embodiments 2.1 to 2.1.2, a stereoisomer, tautomer, stable isotopic variant, pharmaceutically acceptable salt, or solvate thereof, wherein R₄ is halogen, selected from F, Cl, Br, I; or R₄ is NO₂.

**Embodiment 2.1.5:** The compound of formula (I) according to any one of Embodiments 2.1 to 2.1.2, a stereoisomer, tautomer, stable isotopic variant, pharmaceutically acceptable salt, or solvate thereof, wherein R₄ is -C₂₋₆ alkynyl optionally substituted by halogen, preferably -C₂₋₄ alkynyl optionally substituted by halogen, e.g., but not limited to,

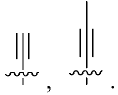

**Embodiment 2.1.6:** The compound of formula (I) according to any one of Embodiments 2.1 to 2.1.2, a stereoisomer, tautomer, stable isotopic variant, pharmaceutically acceptable salt, or solvate thereof, wherein R₄ is -C₁₋₆ alkyl optionally substituted by halogen, preferably -C₁₋₃ alkyl optionally substituted by halogen, e.g., but not limited to, -CH₃, -CH₂CH₃, -CH₂CH₂CH₃, -CH(CH₃)(CH₃), - CH₂CH₂CH₂CH₃, -CH₂CH(CH₃)CH₃, -C(CH₃)₃, -CH₂Cl, -CH₂F, -CHF₂, -CF₃, -CCl₃, -CH₂CH₂F, - CH₂CHF₂, -CH₂CF₃, -CH₂CH₂CH₂F, -CH₂CH₂CHF₂, -CH₂CH₂CF₃, -C₂F₅.

**Embodiment 2.1.7:** The compound of formula (I) according to any one of Embodiments 2.1 to 2.1.6, a stereoisomer, tautomer, stable isotopic variant, pharmaceutically acceptable salt, or solvate thereof, wherein R₇ and R₇' are each H; or R₇ and R₇' are each halogen, preferably F.

**Embodiment 2.1.8:** The compound of formula (I) according to any one of Embodiments 2.1 to 2.1.6, a stereoisomer, tautomer, stable isotopic variant, pharmaceutically acceptable salt, or solvate thereof, wherein one of R₇ and R₇' is H, and the other is selected from halogen, CN, and NO₂, wherein the halogen is preferably F; e.g., R₇ is H and R₇' is halogen, preferably F, or R₇' is H and R₇ is halogen, preferably F.

**Embodiment 2.1.9:** The compound of formula (I) according to any one of Embodiments 2.1 to 2.1.6, a stereoisomer, tautomer, stable isotopic variant, pharmaceutically acceptable salt, or solvate thereof, wherein one of R₇ and R₇' is H, and the other is selected from -C₁₋₆ alkyl optionally substituted by halogen, preferably -C₁₋₃ alkyl optionally substituted by halogen, e.g., but not limited to, -CH₃, - CH₂CH₃, -CH₂CH₂CH₃, -CH(CH₃)(CH₃), -CH₂CH₂CH₂CH₃, -CH₂CH(CH₃)CH₃, -C(CH₃)₃, -CH₂Cl, -CH₂F, -CHF₂, -CF₃, -CCl₃, -CH₂CH₂F, -CH₂CHF₂, -CH₂CF₃, -CH₂CH₂CH₂F, -CH₂CH₂CHF₂, - CH₂CH₂CF₃, -C₂F₅.

**Embodiment 2.1.10:** The compound of formula (I) according to any one of Embodiments 2.1 to 2.1.6, a stereoisomer, tautomer, stable isotopic variant, pharmaceutically acceptable salt, or solvate thereof, wherein one of R₇ and R₇' is selected from halogen, -NO₂, CN, and -C₁₋₆ alkyl optionally substituted by halogen, and the other is selected from -C₁₋₆ alkyl optionally substituted by halogen, wherein the -C₁₋₆ alkyl optionally substituted by halogen is preferably -C₁₋₃ alkyl optionally substituted by halogen, as specifically exemplified in Embodiment 2.1.9.

**Embodiment 2.1.11:** The compound of formula (I) according to any one of Embodiments 2.1 to 2.1.10, a stereoisomer, tautomer, stable isotopic variant, pharmaceutically acceptable salt, or solvate thereof, wherein V is H.

**Embodiment 2.1.12:** The compound of formula (I) according to any one of Embodiments 2.1 to 2.1.10, a stereoisomer, tautomer, stable isotopic variant, pharmaceutically acceptable salt, or solvate thereof, wherein V is -OH, or V is -NH₂.

**Embodiment 2.1.13:** The compound of formula (I) according to any one of Embodiments 2.1 to 2.1.10, a stereoisomer, tautomer, stable isotopic variant, pharmaceutically acceptable salt, or solvate thereof, wherein V is halogen; or V is -C₁₋₆ alkyl, preferably -C₁₋₃ alkyl.

**Embodiment 2.1.14:** The compound of formula (I) according to Embodiment 2.1, a stereoisomer, tautomer, stable isotopic variant, pharmaceutically acceptable salt, or solvate thereof, wherein Ar is 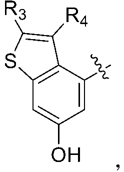 wherein R₃ is H or halogen, R₄ is selected from halogen, -C₂₋₆ alkynyl (preferably -C₂₋₄ alkynyl), and -C₁₋₆ alkyl (preferably -C₁₋₃ alkyl), e.g., but not limited to,

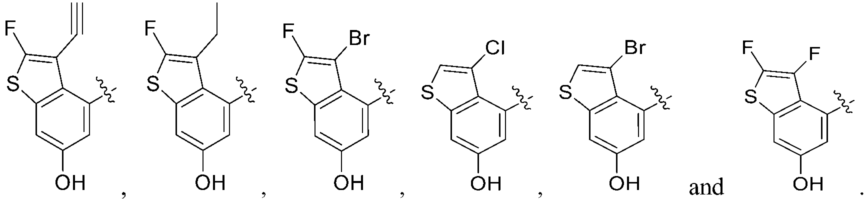

**Embodiment 2.1.15:** The compound of formula (I) according to Embodiment 2.1, a stereoisomer, tautomer, stable isotopic variant, pharmaceutically acceptable salt, or solvate thereof, wherein Ar is 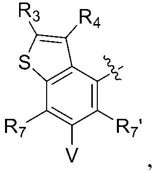 wherein R₃ is selected from -NH₂, -NHC₁₋₆ alkyl, and N(C₁₋₆ alkyl)₂, preferably selected from -NH₂, -NHC₁₋₃ alkyl, and N(C₁₋₃ alkyl)₂, most preferably -NH₂; R₄ is selected from -CN, halogen, -NO₂, and -C₂₋₆ alkynyl substituted by halogen; V is selected from H, -C₁₋₆ alkyl (preferably -C₁₋₃ alkyl), and halogen; R₇ and R₇' are each H, or each are halogen, or one of them is H and the other is halogen or halogen-substituted C₁₋₆ alkyl (preferably halogen-substituted -C₁₋₃ alkyl), or one is halogen and the other is halogen-substituted C₁₋₆ alkyl (preferably halogen-substituted -C₁₋₃ alkyl), wherein the halogen is preferably F;

Preferably, Ar is 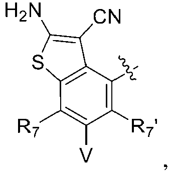 V is H, R₇ and R₇' are each H, or one of them is H and the other is halogen, wherein the halogen is preferably F;

For example, but not limited to:

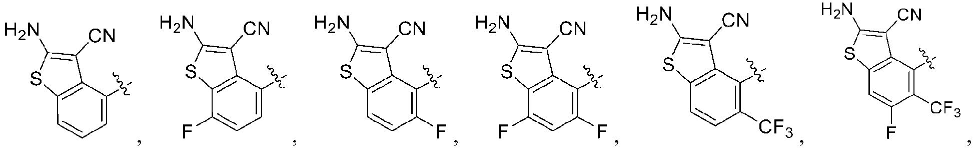

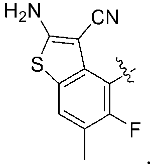

**Embodiment 2.1.16:** The compound of formula (I) according to any one of Embodiments 2.1 to 2.1.15, a stereoisomer, tautomer, stable isotopic variant, pharmaceutically acceptable salt, or solvate thereof, wherein the asterisk in 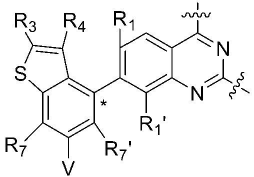 can have axial chirality as appropriate (e.g., depending on the values of R₁ and R₁'), including 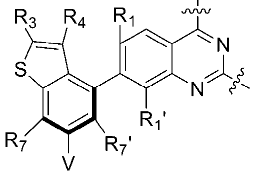 or

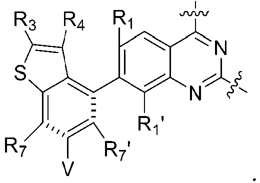

**Embodiment 2.2:** The compound of formula (I) according to any one of Embodiments 1 to 1.3, a stereoisomer, tautomer, stable isotopic variant, pharmaceutically acceptable salt, or solvate thereof, wherein Ar is

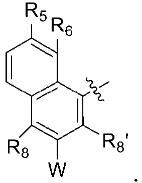

**Embodiment 2.2.1:** The compound of formula (I) according to Embodiment 2.2, a stereoisomer, tautomer, stable isotopic variant, pharmaceutically acceptable salt, or solvate thereof, wherein R₅ is H; or R₅ is halogen, selected from F, Cl, Br, I; preferably R₅ is halogen, most preferably F; or R₅ is NO₂; or R₅ is halogen-substituted -C₁₋₃ alkyl.

**Embodiment 2.2.2:** The compound of formula (I) according to Embodiment 2.2 or 2.2.1, a stereoisomer, tautomer, stable isotopic variant, pharmaceutically acceptable salt, or solvate thereof, wherein R₆ is H; or R₆ is halogen, selected from F, Cl, Br, I.

**Embodiment 2.2.3:** The compound of formula (I) according to Embodiment 2.2 or 2.2.1, a stereoisomer, tautomer, stable isotopic variant, pharmaceutically acceptable salt, or solvate thereof, wherein R₆ is -C₁₋₆ alkyl optionally substituted by halogen, preferably -C₁₋₃ alkyl optionally substituted by halogen, more preferably -C₁₋₃ alkyl, e.g., but not limited to, -CH₃, -CH₂CH₃, - CH₂CH₂CH₃, -CH(CH₃)(CH₃), -CH₂CH₂CH₂CH₃, -CH₂CH(CH₃)CH₃, -C(CH₃)₃, -CH₂Cl, -CH₂F, - CHF₂, -CF₃, -CCl₃, -CH₂CH₂F, -CH₂CHF₂, -CH₂CF₃, -CH₂CH₂CH₂F, -CH₂CH₂CHF₂, -CH₂CH₂CF₃, -C₂F₅.

**Embodiment 2.2.4:** The compound of formula (I) according to Embodiment 2.2 or 2.2.1, a stereoisomer, tautomer, stable isotopic variant, pharmaceutically acceptable salt, or solvate thereof, wherein R₆ is -C₂₋₆ alkynyl optionally substituted by halogen, preferably -C₂₋₄ alkynyl optionally substituted by halogen, more preferably -C₂₋₄ alkynyl, e.g., but not limited to, 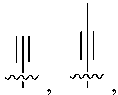 preferably

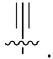

**Embodiment 2.2.5:** The compound of formula (I) according to any one of Embodiments 2.2 to 2.2.4, a stereoisomer, tautomer, stable isotopic variant, pharmaceutically acceptable salt, or solvate thereof, wherein R₈ and R₈' are each H; or R₈ and R₈' are each halogen, preferably F.

**Embodiment 2.2.6:** The compound of formula (I) according to any one of Embodiments 2.2 to 2.2.4, a stereoisomer, tautomer, stable isotopic variant, pharmaceutically acceptable salt, or solvate thereof, wherein one of R₈ and R₈' is H, and the other is selected from halogen, CN, and NO₂, wherein the halogen is preferably F.

**Embodiment 2.2.7:** The compound of formula (I) according to any one of Embodiments 2.2 to 2.2.4, a stereoisomer, tautomer, stable isotopic variant, pharmaceutically acceptable salt, or solvate thereof, wherein one of R₈ and R₈' is H, and the other is selected from -C₁₋₆ alkyl optionally substituted by halogen, preferably -C₁₋₃ alkyl optionally substituted by halogen, as generally or specifically defined in Embodiment 2.1.9.

**Embodiment 2.2.8:** The compound of formula (I) according to any one of Embodiments 2.2 to 2.2.4, a stereoisomer, tautomer, stable isotopic variant, pharmaceutically acceptable salt, or solvate thereof, wherein one of R₈ and R₈' is selected from H, halogen, -NO₂, CN, and -C₁₋₆ alkyl optionally substituted by halogen, and the other is selected from -C₁₋₆ alkyl optionally substituted by halogen, wherein the -C₁₋₆ alkyl optionally substituted by halogen is preferably -C₁₋₃ alkyl optionally substituted by halogen, as generally or specifically defined in Embodiment 2.1.9.

**Embodiment 2.2.9:** The compound of formula (I) according to any one of Embodiments 2.2 to 2.2.8, a stereoisomer, tautomer, stable isotopic variant, pharmaceutically acceptable salt, or solvate thereof, wherein W is -OH.

**Embodiment 2.2.10:** The compound of formula (I) according to any one of Embodiments 2.2 to 2.2.8, a stereoisomer, tautomer, stable isotopic variant, pharmaceutically acceptable salt, or solvate thereof, wherein W is -NH₂.

**Embodiment 2.2.11:** The compound of formula (I) according to Embodiment 2.2, a stereoisomer, tautomer, stable isotopic variant, pharmaceutically acceptable salt, or solvate thereof, wherein Ar is 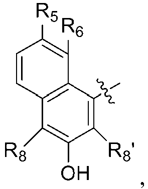 wherein R₅ is H or halogen, R₆ is selected from halogen, -C₂₋₆ alkynyl, and -C₁₋₆ alkyl; R₈ and R₈' are each H; or R₈ and R₈' are each halogen; or one of R₈ and R₈' is H and the other is selected from halogen, CN, and NO₂; or one of R₈ and R₈' is H and the other is selected from -C₁₋₆ alkyl optionally substituted by halogen; or one of R₈ and R₈' is selected from H, halogen, -NO₂, CN, and -C₁₋₆ alkyl optionally substituted by halogen, and the other is selected from -C₁₋₆ alkyl optionally substituted by halogen;

Preferably, Ar is 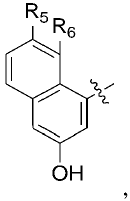 wherein R₅ is halogen, preferably F, R₆ is selected from -C₂₋₆ **alkynyl** (preferably -C₂₋₄ alkynyl),

For example, but not limited to

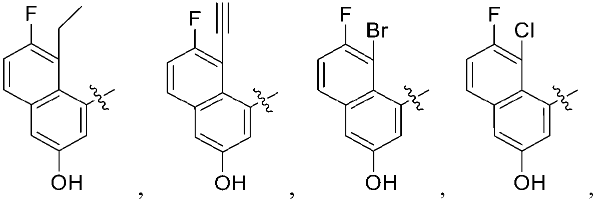

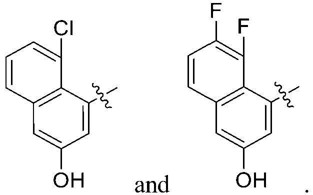

**Embodiment 2.2.12:** The compound of formula (I) according to any one of Embodiments 2.2 to 2.2.11, a stereoisomer, tautomer, stable isotopic variant, pharmaceutically acceptable salt, or solvate thereof, wherein * in 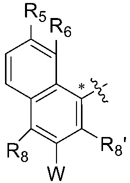 can have axial chirality as appropriate, as exemplified in the Definitions section.

**Embodiment 3.1:** The compound of formula (I) according to any one of Embodiments 1 to 2.2.12, a stereoisomer, tautomer, stable isotopic variant, pharmaceutically acceptable salt, or solvate thereof, wherein in 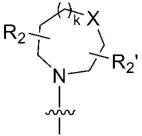 formed by R^{b} and R^{c} together with the N atom to which they are attached, k is 0 and X is selected from CH₂, N, and O, i.e., R^{b} and R^{c} together with the N atom to which they are attached form 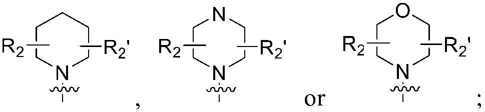 or
k is 1 and X is selected from CH₂, N, and O, i.e., R^{b} and R^{c} together with the N atom to which they are attached form

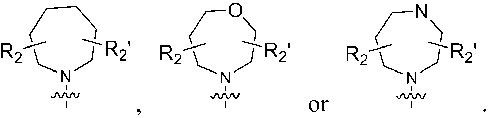

**Embodiment 3.1.1:** The compound of formula (I) according to Embodiment 3.1, a stereoisomer, tautomer, stable isotopic variant, pharmaceutically acceptable salt, or solvate thereof, wherein R^{b} and R^{c} together with the N atom to which they are attached form 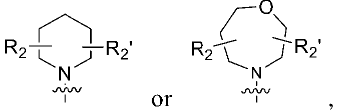 wherein R₂ and R₂' are each independently selected from H, OH, and -C₁₋₆ alkyl optionally substituted by halogen, preferably OH and -C₁₋₆ alkyl optionally substituted by halogen, more preferably selected from OH and -C₁₋₃ alkyl; or R₂ and R₂' attached to the same ring carbon atom together with the ring carbon atom to which they are attached form a 4-6 membered spirocycloalkyl or a 4-6 membered spiroheterocycloalkyl containing 1 or 2 heteroatoms selected from N and O.

**Embodiment 3.1.1.1:** The compound of formula (I) according to Embodiment 3.1.1, a stereoisomer, tautomer, stable isotopic variant, pharmaceutically acceptable salt, or solvate thereof, wherein R₂ and R₂' can each independently be attached to any ring carbon atom, preferably 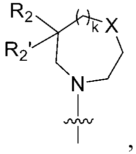 more preferably

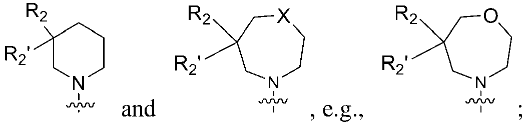

Further, one of R₂ and R₂' is -OH and the other is -C₁₋₆ alkyl, preferably -C₁₋₃ alkyl, more preferably methyl; or R₂ and R₂' together with the ring carbon atom to which they are attached form a 4-6 membered spirocycloalkyl or a 4-6 membered spiroheterocycloalkyl containing 1 or 2 heteroatoms selected from N and O;

Further, where chemically feasible, substituents R₂ and R₂' can each be in stereoisomeric form, e.g., R or S configuration;

Specific examples include, but are not limited to:

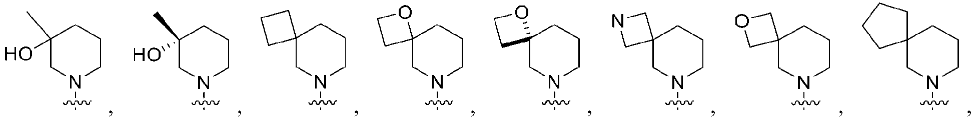

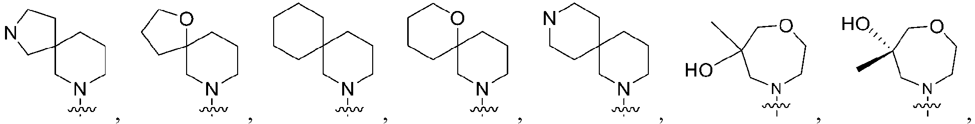

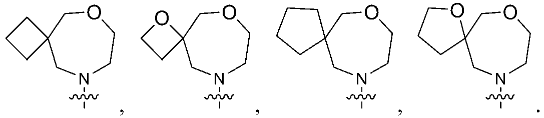

**Embodiment 3.1.2:** The compound of formula (I) according to Embodiment 3.1, a stereoisomer, tautomer, stable isotopic variant, pharmaceutically acceptable salt, or solvate thereof, wherein R^{b} and R^{c} together with the N atom to which they are attached form 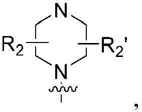 wherein R₂ and R₂', attached to non-adjacent ring carbon atoms together form an intra-ring bridge -CH₂-, -CH₂CH₂-, or - CH₂=CH₂-;

Specific examples include 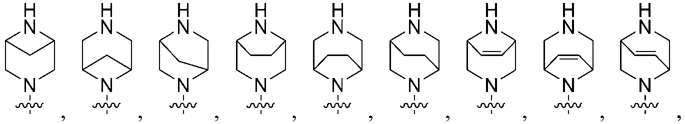 preferably

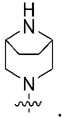

**Embodiment 4.1:** The compound of formula (I) according to any one of Embodiments 1 to 3.1.2, a stereoisomer, tautomer, stable isotopic variant, pharmaceutically acceptable salt, or solvate thereof, wherein Z is O; or Z is N; or Z is CH₂; preferably Z is O.

**Embodiment 5.1:** The compound of formula (I) according to any one of Embodiments 1 to 4.1, a stereoisomer, tautomer, stable isotopic variant, pharmaceutically acceptable salt, or solvate thereof, wherein R is 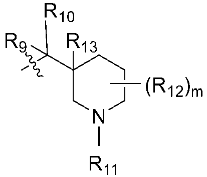 , more preferably

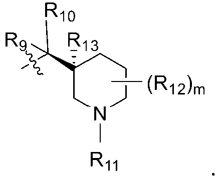

**Embodiment 5.1.1:** The compound of formula (I) according to any one of Embodiments 1 to 5.1, a stereoisomer, tautomer, stable isotopic variant, pharmaceutically acceptable salt, or solvate thereof, wherein R₉ and R₁₀ are both H; or one or both are deuterium.

**Embodiment 5.1.1.1:** The compound of formula (I) according to any one of Embodiments 1 to 5.1, a stereoisomer, tautomer, stable isotopic variant, pharmaceutically acceptable salt, or solvate thereof, wherein R₉ and R₁₀ are each independently selected from H and -C₁₋₆ alkyl, preferably -C₁₋₃ alkyl, optionally substituted by deuterium, halogen, or -OC₁₋₆ alkyl, e.g., one of them is H and the other is the defined alkyl, or both are the defined alkyl; examples of such alkyl include, but are not limited to, -CH₃, -CD₃, -CH₂CH₃, -CH₂CH₂CH₃, -CH(CH₃)(CH₃), -CH₂-OCH₃, -CH₂-O-CH₂CH₃, - CH₂CH₂-O-CH₃, -CH₂CH₂-O-CH₂CH₃, -CH₂F, -CH₂Cl, -CHF₂, -CF₃, -CCl₃, -CH₂CH₂F, -CH₂CHF₂, -CH₂CF₃, -CH₂CH₂CH₂F, -CH₂CH₂CHF₂, -CH₂CH₂CF₃.

**Embodiment 5.1.1.2:** The compound of formula (I) according to any one of Embodiments 1 to 5.1, a stereoisomer, tautomer, stable isotopic variant, pharmaceutically acceptable salt, or solvate thereof, wherein R₉ and R₁₀ are each independently selected from H and -(CH₂)ₙ-C₃₋₆ cycloalkyl (preferably -C₃₋₆ cycloalkyl), wherein the C₃₋₆ cycloalkyl is optionally substituted by halogen or C₁₋₆ alkoxy, e.g., one of them is H and the other is the defined cycloalkyl; examples include, but are not limited to,

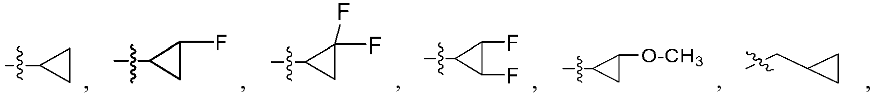

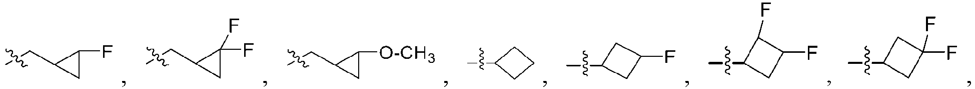

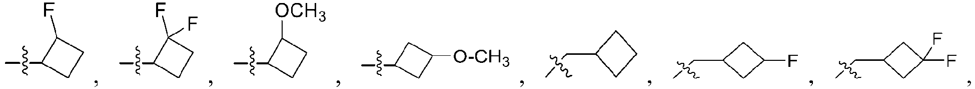

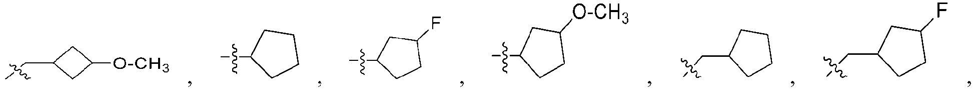

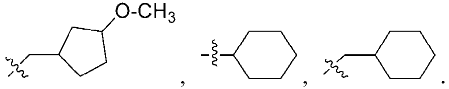

**Embodiment 5.1.1.3:** The compound of formula (I) according to any one of Embodiments 1 to 5.1, a stereoisomer, tautomer, stable isotopic variant, pharmaceutically acceptable salt, or solvate thereof, wherein R₉ and R₁₀ attached to the same carbon atom together with the carbon atom to which they are attached form a C₃₋₄ cycloalkyl, e.g., cyclopropyl, cyclobutyl.

**Embodiment 5.1.2:** The compound of formula (I) according to any one of Embodiments 1 to 5.1.1.3, a stereoisomer, tautomer, stable isotopic variant, pharmaceutically acceptable salt, or solvate thereof, wherein R₁₁ is H.

**Embodiment 5.1.2.1:** The compound of formula (I) according to any one of Embodiments 1 to 5.1.1.3, a stereoisomer, tautomer, stable isotopic variant, pharmaceutically acceptable salt, or solvate thereof, wherein R₁₁ is -C₁₋₆ alkyl, preferably -C₁₋₃ alkyl, optionally substituted by deuterium, halogen, CN, or -C₁₋₆ alkoxy, preferably optionally substituted by deuterium, halogen, or -C₁₋₃ alkoxy; examples include, but are not limited to, -CH₃, -CD₃, -CH₂CH₃, -CH₂CD₃, -CH₂CH₂CH₃, - CH(CH₃)(CH₃), -CH₂CH₂CH₂CH₃, -CH₂CH(CH₃)CH₃, -C(CH₃)₃, -CH₂-OCH₃, -CH₂-O-CH₂CH₃, - CH₂CH₂-O-CH₃, -CH₂CH₂-O-CH₂CH₃, -CH(CH₃)CH₂-OCH₃, -CH₂CH(CH₃)-OCH₃, -CH₂F, - CH₂Cl, -CHF₂, -CF₃, -CH₂CH₂F, -CH₂CHF₂, -CH(CH₃)F, -CH(CH₃)CH₂F, -CH₂CH(CH₃)F, - CH₂CF₃, -CH₂CH₂CH₂F, -CH₂CH₂CHF₂, -CH₂CH₂CF₃, -C₂F₅, -CH₂CN, -CH₂CH₂CN;

More preferably, R₁₁ is -C₁₋₃ alkyl, wherein the hydrogen atom is optionally replaced by one or more isotope deuterium, e.g., -CH₃, -CD₃.

**Embodiment 5.1.2.2:** The compound of formula (I) according to any one of Embodiments 1 to 5.1.1.3, a stereoisomer, tautomer, stable isotopic variant, pharmaceutically acceptable salt, or solvate thereof, wherein R₁₁ is -C₂₋₆ alkenyl or -C₂₋₆ alkynyl, preferably -C₂₋₄ alkenyl or -C₂₋₄ alkynyl, optionally substituted by halogen, CN, or -C₁₋₆ alkoxy; examples include, but are not limited to, ethenyl, propenyl, ethynyl, each optionally substituted by halogen or -C₁₋₆ alkoxy.

**Embodiment 5.1.2.3:** The compound of formula (I) according to any one of Embodiments 1 to 5.1.1.3, a stereoisomer, tautomer, stable isotopic variant, pharmaceutically acceptable salt, or solvate thereof, wherein R₁₁ is -(CH₂)ₙ-C₃₋₆ cycloalkyl, preferably -C₃₋₆ cycloalkyl, wherein the -C₃₋₆ cycloalkyl is optionally substituted by deuterium, halogen, CN, or -C₁₋₆ alkoxy; examples include, but are not limited to,

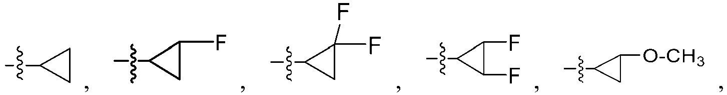

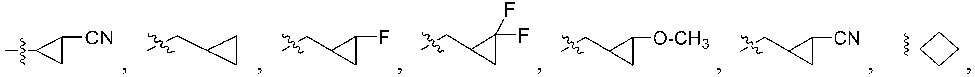

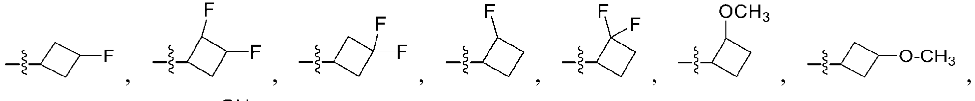

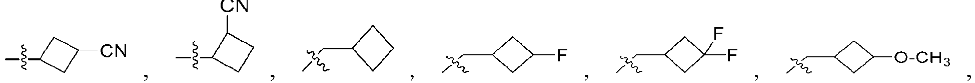

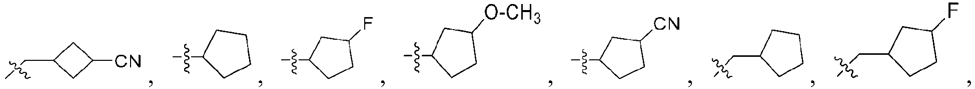

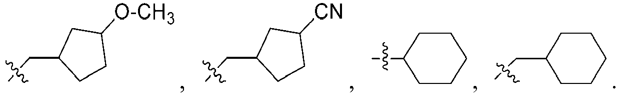

**Embodiment 5.1.3:** The compound of formula (I) according to any one of Embodiments 1 to 5.1.2.3, a stereoisomer, tautomer, stable isotopic variant, pharmaceutically acceptable salt, or solvate thereof, wherein R₁₂ is H.

**Embodiment 5.1.3.1:** The compound of formula (I) according to any one of Embodiments 1 to 5.1.2.3, a stereoisomer, tautomer, stable isotopic variant, pharmaceutically acceptable salt, or solvate thereof, wherein R₁₂ is halogen, e.g., F, Cl, Br, I, preferably F; or R₁₂ is CN.

**Embodiment 5.1.3.2:** The compound of formula (I) according to any one of Embodiments 1 to 5.1.2.3, a stereoisomer, tautomer, stable isotopic variant, pharmaceutically acceptable salt, or solvate thereof, wherein R₁₂ is -NH₂, -NHC₁₋₆ alkyl, -N(C₁₋₆ alkyl)₂, wherein the -C₁₋₆ alkyl is preferably -C₁₋₃ alkyl, optionally substituted by halogen, CN, or -OC₁₋₆ alkyl.

**Embodiment 5.1.3.3:** The compound of formula (I) according to any one of Embodiments 1 to 5.1.2.3, a stereoisomer, tautomer, stable isotopic variant, pharmaceutically acceptable salt, or solvate thereof, wherein R₁₂ is -OH; or R₁₂ is -O-C₁₋₆ alkyl, preferably -O-C₁₋₃ alkyl, wherein the alkyl is optionally substituted by halogen, CN, or -OC₁₋₆ alkyl; examples include, but are not limited to, -O-CH₃, -O-CH₂CH₃, -O-CH₂CH₂CH₃, -O-CH(CH₃)(CH₃), -O-CH₂CH₂CH₂CH₃, -O-CH₂CH(CH₃)CH₃, -O-C(CH₃)₃, -O-CH₂Cl, -O-CH₂CN, -O-CH₂F, -O-CHF₂, -O-CF₃, -O-CCl₃, -O-CH₂CH₂F, -O-CH₂CH₂CN, -O-CH₂CHF₂, -O-CH₂CF₃, -O-CH₂CH₂CH₂F, -O-CH₂CH₂CHF₂, -O-CH₂CH₂CF₃, -O-C(CH₃)₂CF₃, -O-C₂F₅, -O-CH₂-OCH₃, -O-CH₂-O-CH₂CH₃, -O-CH₂CH₂-O-CH₃, -O-CH₂CH₂-O-CH₂CH₃.

**Embodiment 5.1.3.4:** The compound of formula (I) according to any one of Embodiments 1 to 5.1.2.3, a stereoisomer, tautomer, stable isotopic variant, pharmaceutically acceptable salt, or solvate thereof, wherein R₁₂ is -C₁₋₆ alkyl, preferably -C₁₋₃ alkyl, optionally substituted by halogen, CN, or - OC₁₋₆ alkyl, e.g., but not limited to, -CH₃, -CH₂CH₃, -CH₂CH₂CH₃, -CH(CH₃)(CH₃), - CH₂CH₂CH₂CH₃, -CH₂CH(CH₃)CH₃, -C(CH₃)₃, -CH₂-OCH₃, -CH₂-O-CH₂CH₃, -CH₂CH₂-O-CH₃, - CH₂CH₂-O-CH₂CH₃, -CH(CH₃)CH₂-OCH₃, -CH₂CH(CH₃)-OCH₃, -CH₂F, -CH₂Cl, -CHF₂, -CF₃, - CH₂CH₂F, -CH₂CHF₂, -CH₂CF₃, -CH₂CH₂CH₂F, -CH₂CH₂CHF₂, -CH₂CH₂CF₃, -C₂F₅, -CH₂CN, - CH₂CH₂CN.

**Embodiment 5.1.3.5:** The compound of formula (I) according to any one of Embodiments 1 to 5.1.2.3, a stereoisomer, tautomer, stable isotopic variant, pharmaceutically acceptable salt, or solvate thereof, wherein R₁₂ is -O-C₃₋₆ cycloalkyl, wherein the C₃₋₆ cycloalkyl is optionally substituted by halogen, CN, or -O-C₁₋₆ alkyl; examples include, but are not limited to,

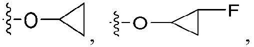

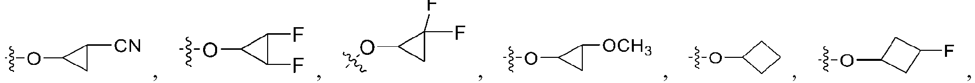

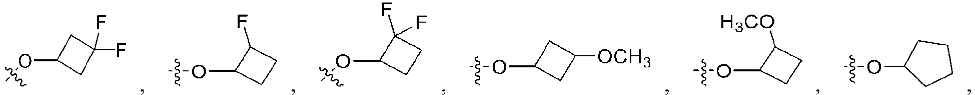

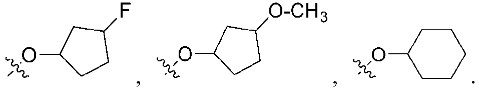

**Embodiment 5.1.3.6:** The compound of formula (I) according to any one of Embodiments 1 to 5.1.2.3, a stereoisomer, tautomer, stable isotopic variant, pharmaceutically acceptable salt, or solvate thereof, wherein R₁₂ is -(CH₂)ₙ-C₃₋₆ cycloalkyl, preferably -C₃₋₆ cycloalkyl, wherein the -C₃₋₆ cycloalkyl is optionally substituted by halogen, CN, or -C₁₋₆ alkoxy; as exemplified in Embodiment 5.1.2.3.

**Embodiment 5.1.3.7:** The compound of formula (I) according to any one of Embodiments 1 to 5.1.2.3, a stereoisomer, tautomer, stable isotopic variant, pharmaceutically acceptable salt, or solvate thereof, wherein R₁₂ is -C₂₋₆ alkenyl or -C₂₋₆ alkynyl, preferably -C₂₋₄ alkenyl or -C₂₋₄ alkynyl, optionally substituted by halogen, CN, or -C₁₋₆ alkoxy; examples include, but are not limited to, ethenyl, propenyl, ethynyl, each optionally substituted by halogen or -C₁₋₆ alkoxy.

**Embodiment 5.1.3.8:** The compound of formula (I) according to any one of Embodiments 1 to 5.1.2.3, a stereoisomer, tautomer, stable isotopic variant, pharmaceutically acceptable salt, or solvate thereof, wherein R₁₂ is =C(R^{d})₂, wherein R^{d} is each independently selected from H, F, Cl, Br, I, -C₁₋₆ alkyl optionally substituted by halogen (preferably -C₁₋₃ alkyl); examples include, but are not limited to, =CH₂, =CF₂, =CHF, =CCl₂, =C(CH₃)₂, =C(CF₃)₂.

**Embodiment 5.1.3.9:** The compound of formula (I) according to any one of Embodiments 1 to 5.1.2.3, a stereoisomer, tautomer, stable isotopic variant, pharmaceutically acceptable salt, or solvate thereof, wherein R₁₂ is selected from -C₁₋₆ alkyl optionally substituted by halogen (preferably -C₁₋₃ alkyl) and =C(R^{d})₂, wherein R^{d} is each independently selected from H and halogen (preferably F).

**Embodiment 5.1.3.10:** The compound of formula (I) according to any one of Embodiments 1 to 5.1.2.3, a stereoisomer, tautomer, stable isotopic variant, pharmaceutically acceptable salt, or solvate thereof, wherein m is 0, 1, or 2, preferably 1.

**Embodiment 5.1.3.11:** The compound of formula (I) according to any one of Embodiments 1 to 5.1.2.3, a stereoisomer, tautomer, stable isotopic variant, pharmaceutically acceptable salt, or solvate thereof, wherein the ring carbon atom to which R₁₂ is attached can be chiral, having R configuration or S configuration.

**Embodiment 5.1.3.12:** The compound of formula (I) according to any one of Embodiments 5.1.3.8 to 5.1.3.9, a stereoisomer, tautomer, stable isotopic variant, pharmaceutically acceptable salt, or solvate thereof, wherein when R₁₂ is =C(R^{d})₂, the double bond can have geometric isomers, including E and Z forms, preferably E isomer.

**Embodiment 5.1.4:** The compound of formula (I) according to any one of Embodiments 1 to 5.1.3.12, a stereoisomer, tautomer, stable isotopic variant, pharmaceutically acceptable salt, or solvate thereof, wherein R₁₃ is H.

**Embodiment 5.1.4.1:** The compound of formula (I) according to any one of Embodiments 1 to 5.1.3.12, a stereoisomer, tautomer, stable isotopic variant, pharmaceutically acceptable salt, or solvate thereof, wherein R₁₃ is halogen, preferably F.

**Embodiment 5.1.4.2:** The compound of formula (I) according to any one of Embodiments 1 to 5.1.3.12, a stereoisomer, tautomer, stable isotopic variant, pharmaceutically acceptable salt, or solvate thereof, wherein R₁₃ is -C₁₋₆ alkyl, preferably -C₁₋₃ alkyl, optionally substituted by halogen or -OC₁₋₆ alkyl, e.g., but not limited to, -CH₃, -CH₂CH₃, -CH₂CH₂CH₃, -CH(CH₃)(CH₃), -CH₂CH₂CH₂CH₃, - CH₂CH(CH₃)CH₃, -C(CH₃)₃, -CH₂-OCH₃, -CH₂-O-CH₂CH₃, -CH₂CH₂-O-CH₃, -CH₂CH₂-O-CH₂CH₃, -CH(CH₃)CH₂-OCH₃, -CH₂CH(CH₃)-OCH₃, -CH₂F, -CH₂Cl, -CHF₂, -CF₃, -CH₂CH₂F, - CH₂CHF₂, -CH₂CF₃, -CH₂CH₂CH₂F, -CH₂CH₂CHF₂, -CH₂CH₂CF₃, -C₂F₅.

**Embodiment 5.1.4.3:** The compound of formula (I) according to any one of Embodiments 1 to 5.1.3.12, a stereoisomer, tautomer, stable isotopic variant, pharmaceutically acceptable salt, or solvate thereof, wherein R₁₃ is -(CH₂)ₙ-C₃₋₆ cycloalkyl, wherein the C₃₋₆ cycloalkyl is optionally substituted by halogen or -OC₁₋₆ alkyl; examples include, but are not limited 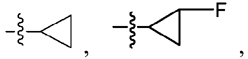 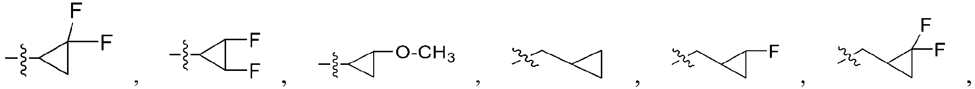 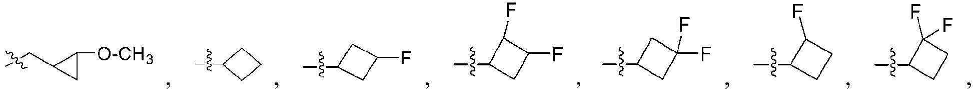 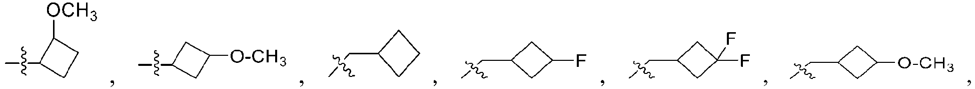 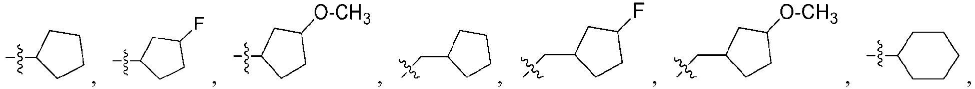 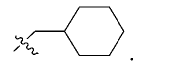 .

**Embodiment 5.1.4.4.:** The compound of formula (I) according to any one of Embodiments 1 to 5.1.3.12, a stereoisomer, tautomer, stable isotopic variant, pharmaceutically acceptable salt, or solvate thereof, wherein R₁₃ is -C₁₋₆ alkyl, preferably -C₁₋₃ alkyl, more preferably -CH₃.

**Embodiment 5.2:** The compound of formula (I) according to any one of Embodiments 1 to 5.1.4.4, a stereoisomer, tautomer, stable isotopic variant, pharmaceutically acceptable salt, or solvate thereof, wherein R is 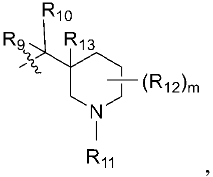 more preferably 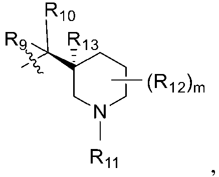 wherein R₉ and R₁₀ are both H, or one or both are deuterium; R₁₁ is -C₁₋₃ alkyl, wherein the hydrogen atom is optionally replaced by one or more isotope deuterium; R₁₂ is selected from -C₁₋₆ alkyl optionally substituted by halogen (preferably -C₁₋₃ alkyl) and =C(R^{d})₂, wherein R^{d} is each independently selected from H and halogen (preferably F); R₁₃ is -C₁₋₆ alkyl (preferably -C₁₋₃ alkyl); m is 1 or 2;

Specifically, R is 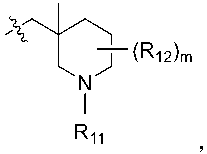 wherein R₁₁, R₁₂, and m have the meanings defined, examples of R₁₁ include, but are not limited to, methyl, ethyl, isopropyl, -CD₃, -CH₂CD₃; and/or examples of R₁₂ include, but are not limited to, fluoromethyl, difluoromethyl, methyl, fluoro methylene, difluoro methylene, methylene.

**Embodiment 5.3:** The compound of formula (I) according to any one of Embodiments 1 to 5.1.4.4, a stereoisomer, tautomer, stable isotopic variant, pharmaceutically acceptable salt, or solvate thereof, wherein R is 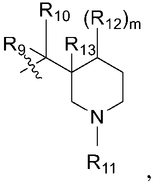 preferably 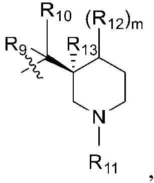 wherein R₉ and R₁₀ are both H, or one or both are deuterium; R₁₁ is -C₁₋₃ alkyl, wherein the hydrogen atom is optionally replaced by one or more isotope deuterium; R₁₂ is selected from -C₁₋₆ alkyl optionally substituted by halogen (preferably -C₁₋₃ alkyl) and =C(R^{d})₂, wherein R^{d} is each independently selected from H and halogen (preferably F); R₁₃ is -C₁₋₆ alkyl (preferably -C₁₋₃ alkyl); m is 1 or 2;

Specifically, R is 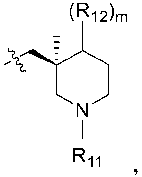 wherein R₁₁, R₁₂, and m have the meanings defined, examples of R₁₁ include, but are not limited to, methyl, ethyl, isopropyl, -CD₃, -CH₂CD₃; and/or examples of R₁₂ include, but are not limited to, fluoromethyl, difluoromethyl, methyl, fluoro methylene, difluoro methylene, methylene.

**Embodiment 5.4:** The compound of formula (I) according to any one of Embodiments 1 to 5.1, a stereoisomer, tautomer, stable isotopic variant, pharmaceutically acceptable salt, or solvate thereof, wherein examples of R include, but are not limited to

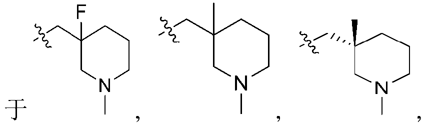

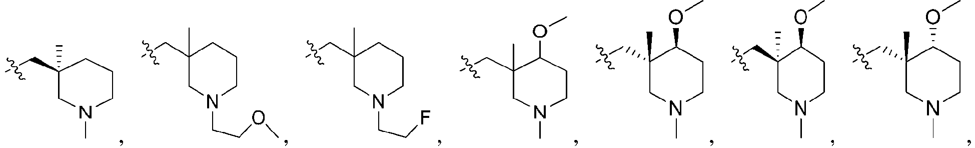

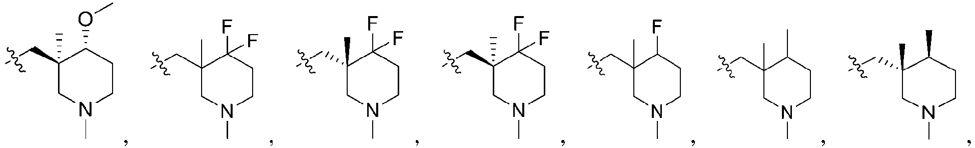

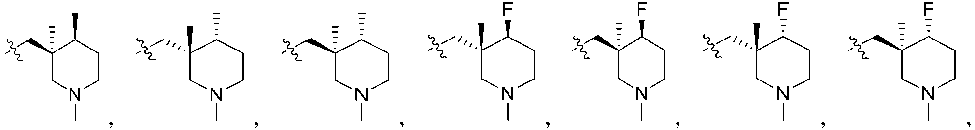

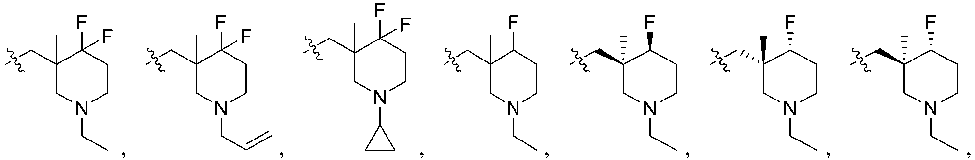

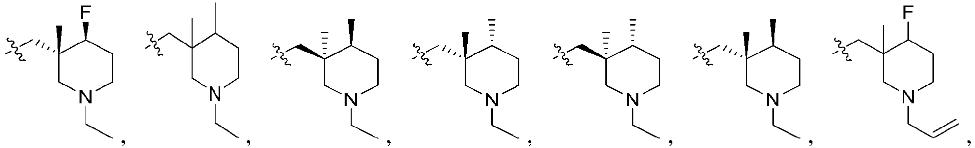

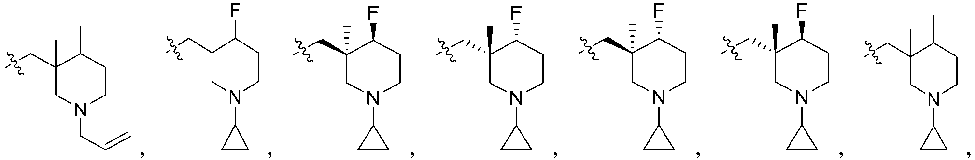

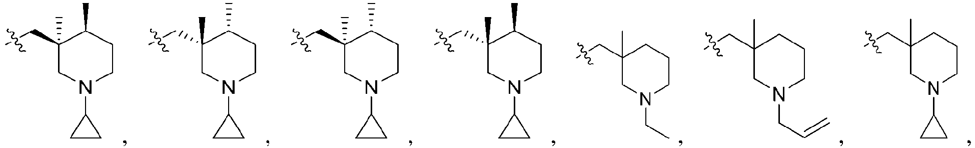

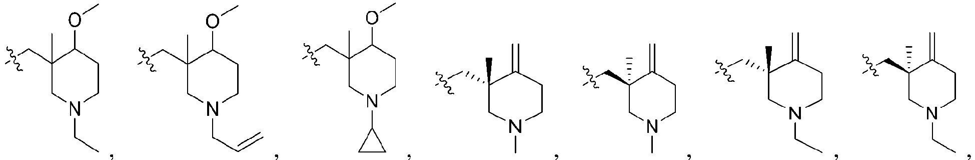

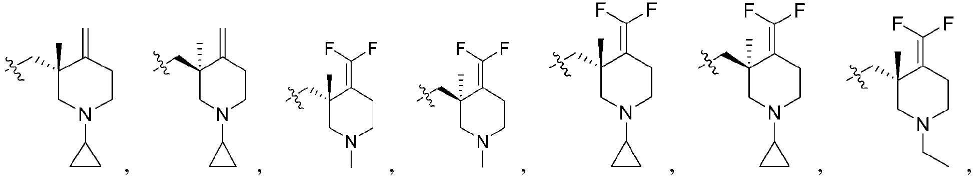

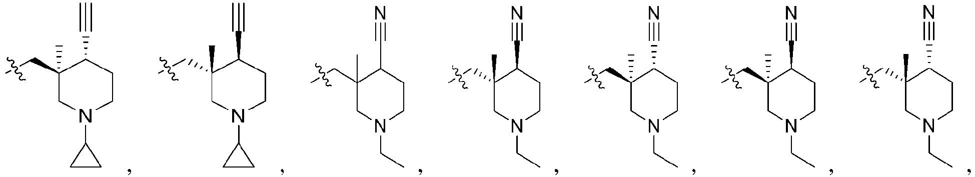

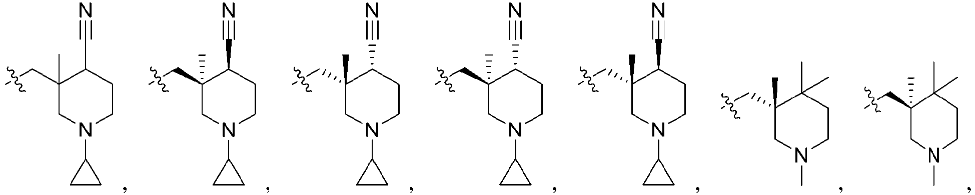

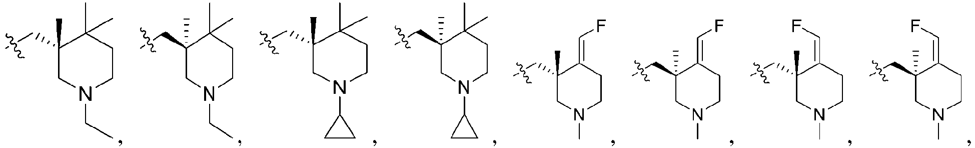

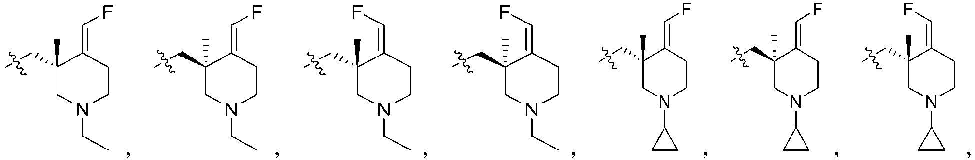

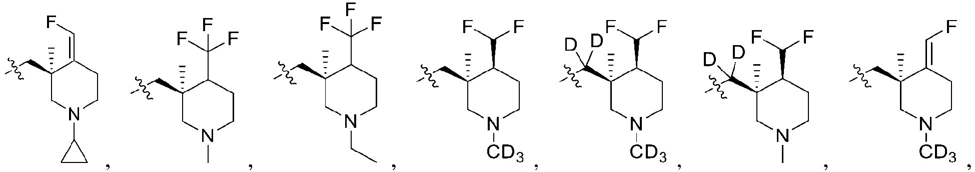

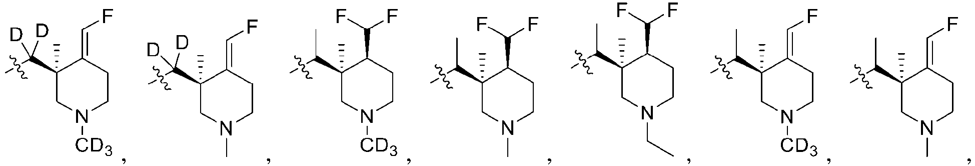

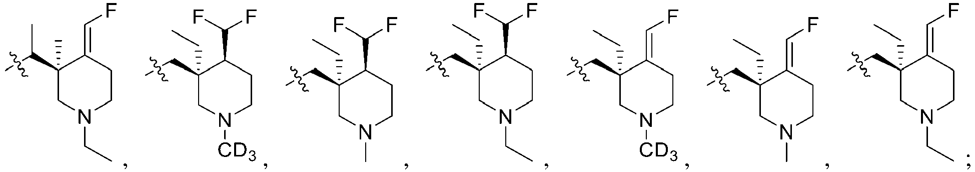

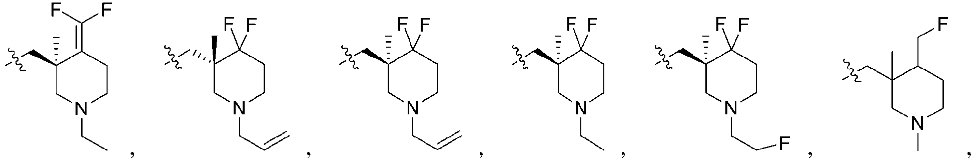

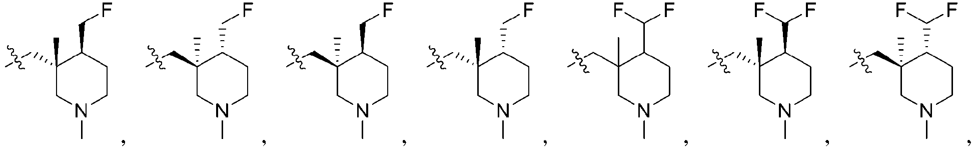

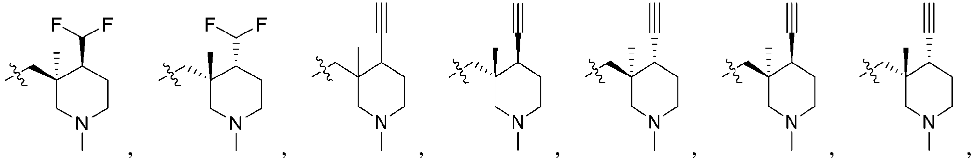

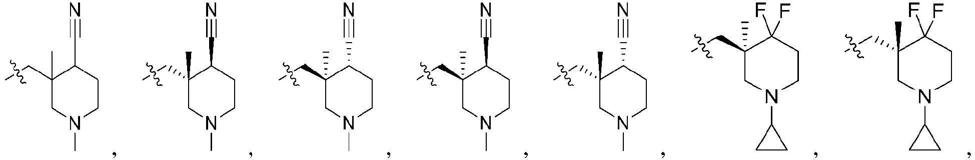

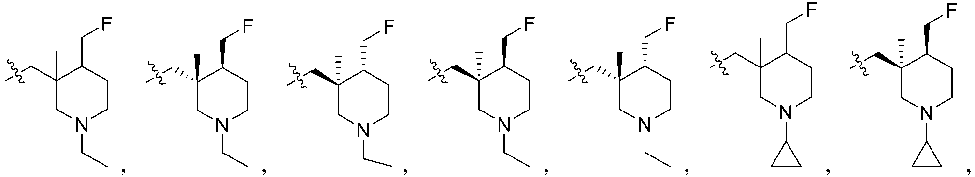

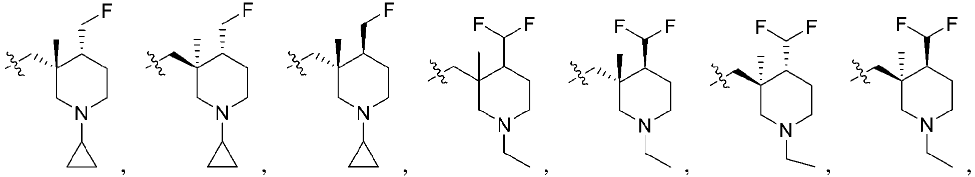

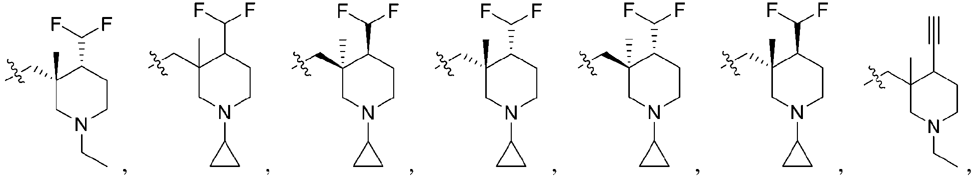

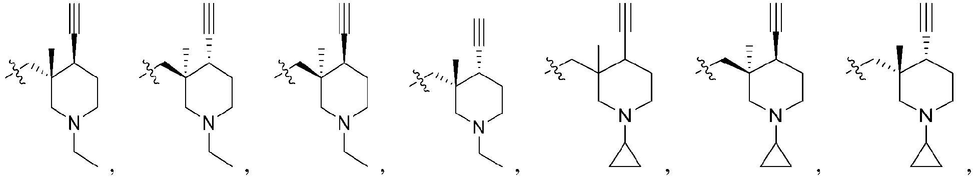

Preferably

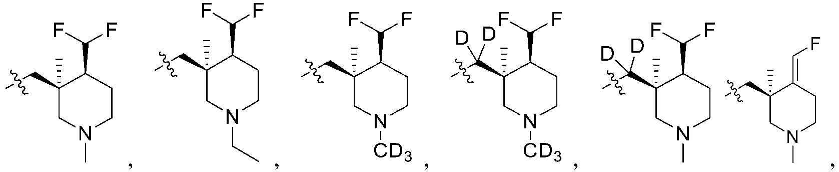

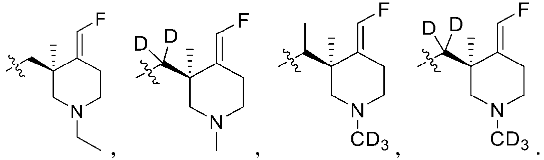

**Embodiment 6.1:** The compound of formula (I) according to Embodiment 1, a stereoisomer, tautomer, stable isotopic variant, pharmaceutically acceptable salt, or solvate thereof, having a sub-general formula as shown in **Table 1** below:

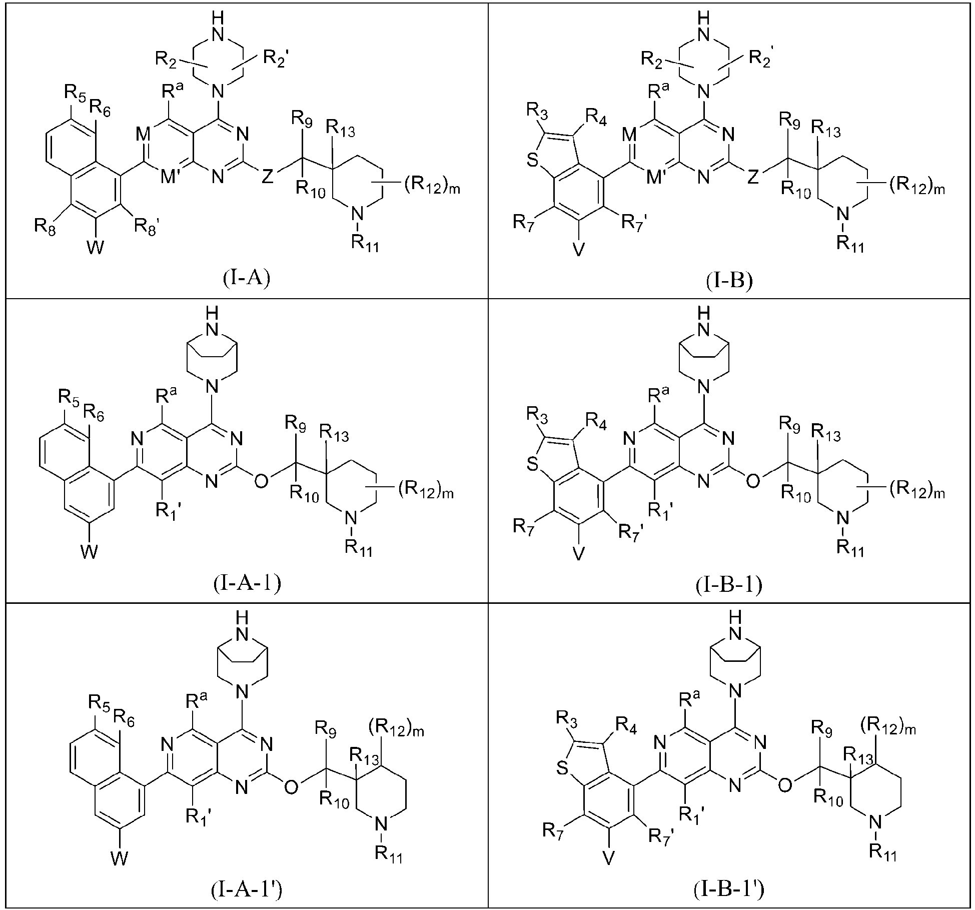

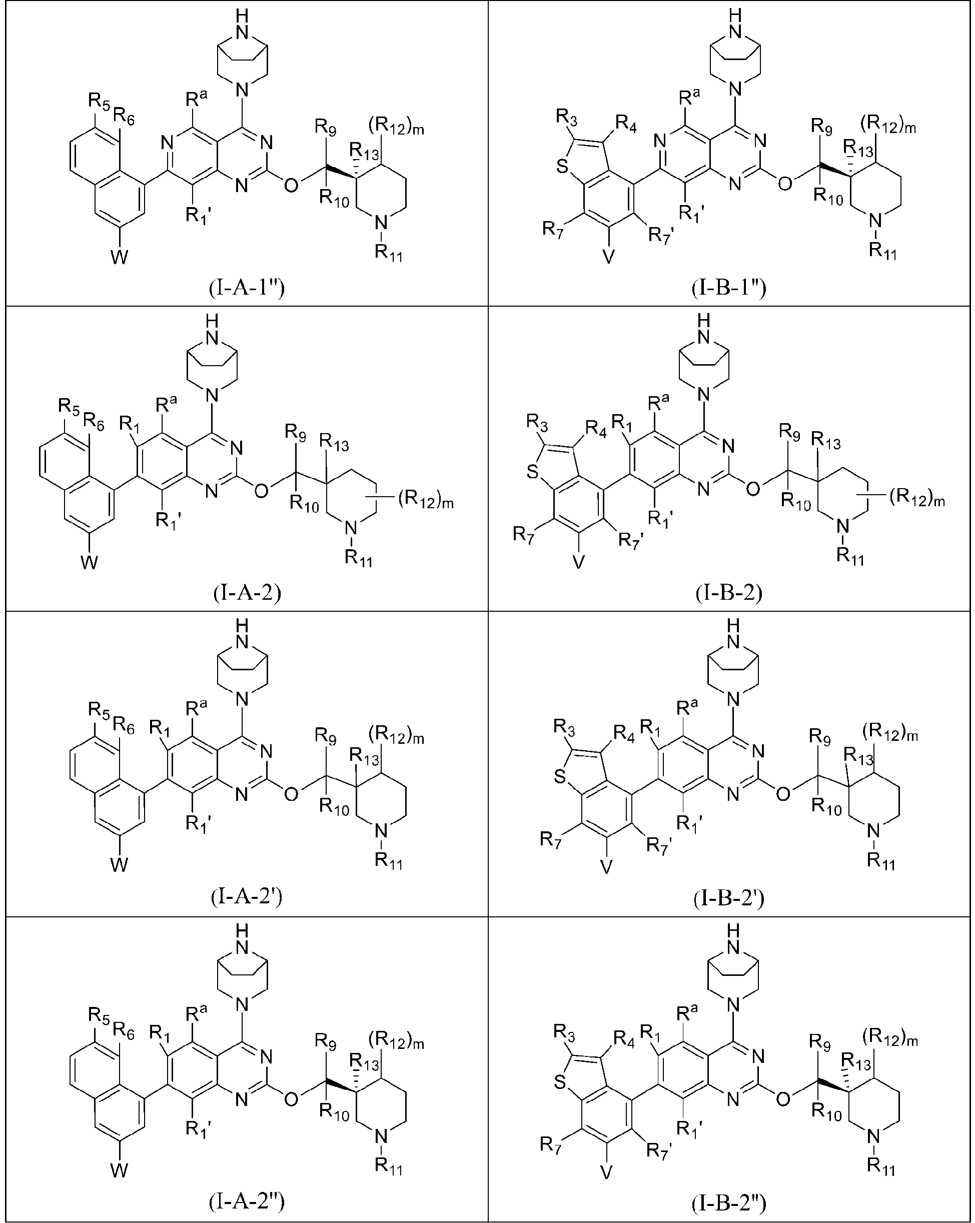

wherein each substituent has the meaning as generally or specifically defined in the respective corresponding embodiments above, and also encompasses any combination of the generally or specifically defined meanings of each substituent;

Preferably, wherein
when M is C-R₁, R₁ is selected from halogen (preferably F or Cl) or -C₁₋₆ alkyl optionally substituted by halogen (preferably -C₁₋₃ alkyl substituted by halogen, more preferably -CF₃), R^{a} is H, and M' is C-R₁' and R₁' is halogen (preferably F);
when M is N, R^{a} is selected from H, -C₂₋₆ alkynyl (preferably -C₂₋₄ alkynyl, more preferably - C≡CH), and -OC₁₋₆ alkyl (preferably -OC₁₋₃ alkyl optionally substituted by deuterium, more preferably -OCH₃, -OCD₃, -OCH₂CH₃), and M' is C-R₁' and R₁' is halogen (preferably F);
R₂ and R₂' attached to non-adjacent ring carbon atoms together form an intra-ring bridge -(CH₂)₁₋₂- or -CH₂=CH₂-;
R₃ is selected from -NH₂, -NHC₁₋₆ alkyl, and N(C₁₋₆ alkyl)₂ (preferably selected from -NH₂, - NHC₁₋₃ alkyl, and N(C₁₋₃ alkyl)₂, more preferably -NH₂); R₄ is selected from -CN, halogen, -NO₂, and -C₂₆ alkynyl substituted by halogen; V is selected from H, -C₁₋₆ alkyl (preferably -C₁₋₃ alkyl), and halogen; R₇ and R₇' are each H, or each are halogen, or one of them is H and the other is halogen or halogen-substituted C₁₋₆ alkyl (preferably halogen-substituted -C₁₋₃ alkyl), or one is halogen and the other is halogen-substituted C₁₋₆ alkyl (preferably halogen-substituted -C₁₋₃ alkyl), wherein the halogen is preferably F;
Preferably, R₃ is -NH₂, R₄ is -CN, V is H, R₇ and R₇' are each H, or one of them is H and the other is halogen (preferably F);
R₅ is H or halogen (preferably F); R₆ is selected from halogen, -C₂₋₆ alkynyl (preferably -C₂₋₄ alkynyl), and -C₁₋₆ alkyl (preferably -C₁₋₃ alkyl); R₈ and R₈' are each H, or R₈ and R₈' are each halogen, or one of R₈ and R₈' is H and the other is selected from halogen, CN, and NO₂, or one of R₈ and R₈' is H and the other is selected from -C₁₋₆ alkyl optionally substituted by halogen, or one of R₈ and R₈' is selected from H, halogen, -NO₂, CN, and -C₁₋₆ alkyl optionally substituted by halogen, and the other is selected from -C₁₋₆ alkyl optionally substituted by halogen; W is OH;
Preferably, R₅ is halogen (preferably F), R₆ is selected from -C₂₋₆ alkynyl (preferably -C₂₋₄ alkynyl), R₈ and R₈' are each H, W is OH;
Z is selected from O, N, and CH₂;
R₉ and R₁₀ are both H; or one or both are deuterium;
R₁₁ is -C₁₋₆ alkyl (preferably -C₁₋₃ alkyl), wherein the hydrogen atom is optionally replaced by one or more isotope deuterium;
R₁₂ is selected from -C₁₋₆ alkyl optionally substituted by halogen (preferably -C₁₋₃ alkyl) and =C(R^{d})₂, wherein R^{d} is each independently selected from H and halogen (preferably F);
m is 0, 1, or 2 (preferably 1 or 2);
R₁₃ is -C₁₋₆ alkyl (preferably -C₁₋₃ alkyl, more preferably -CH₃).

**Embodiment 6.2:** The compound of formula (I) according to Embodiment 1, a stereoisomer, tautomer, stable isotopic variant, pharmaceutically acceptable salt, or solvate thereof, having a sub-general formula as shown in **Table 2** below:

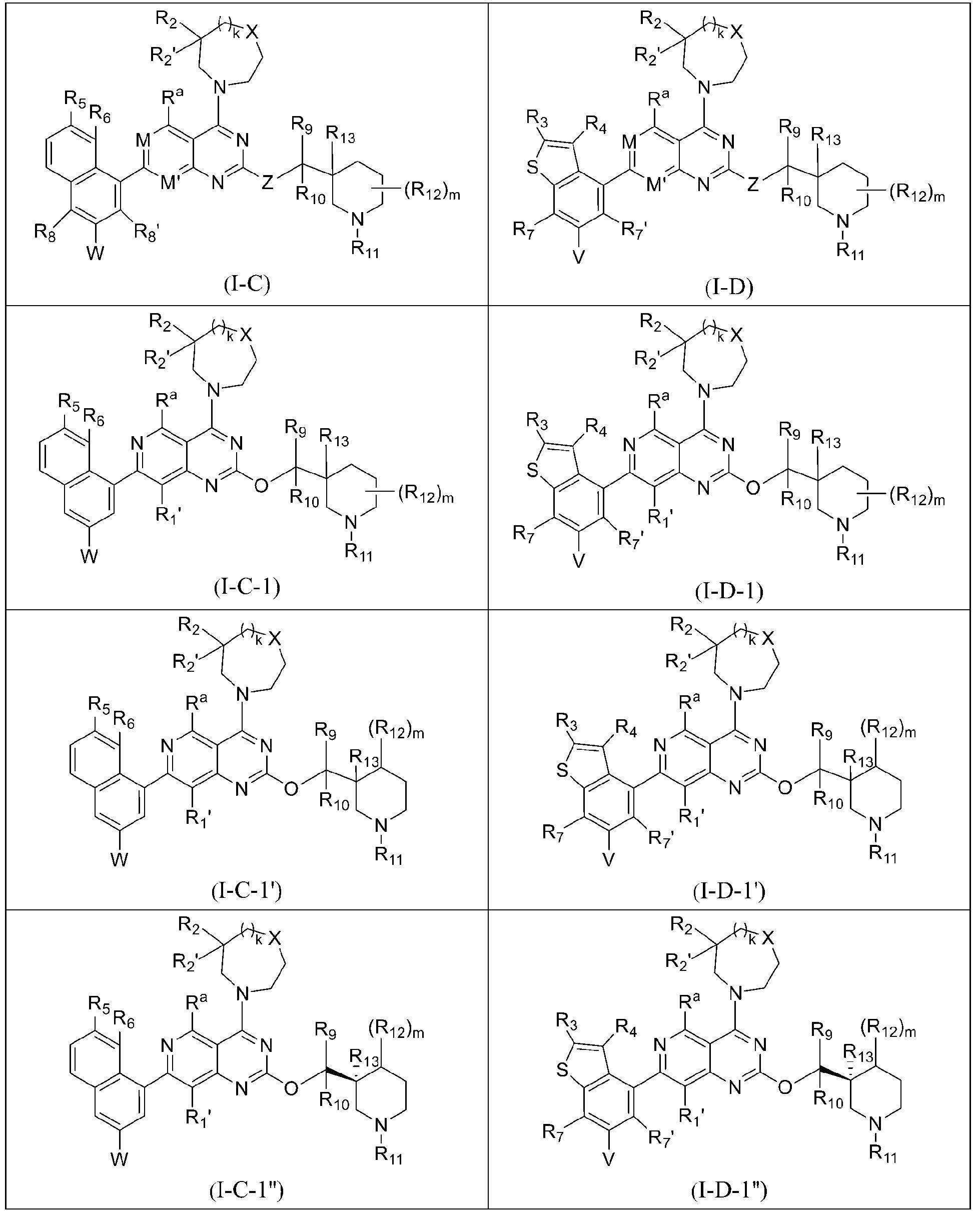

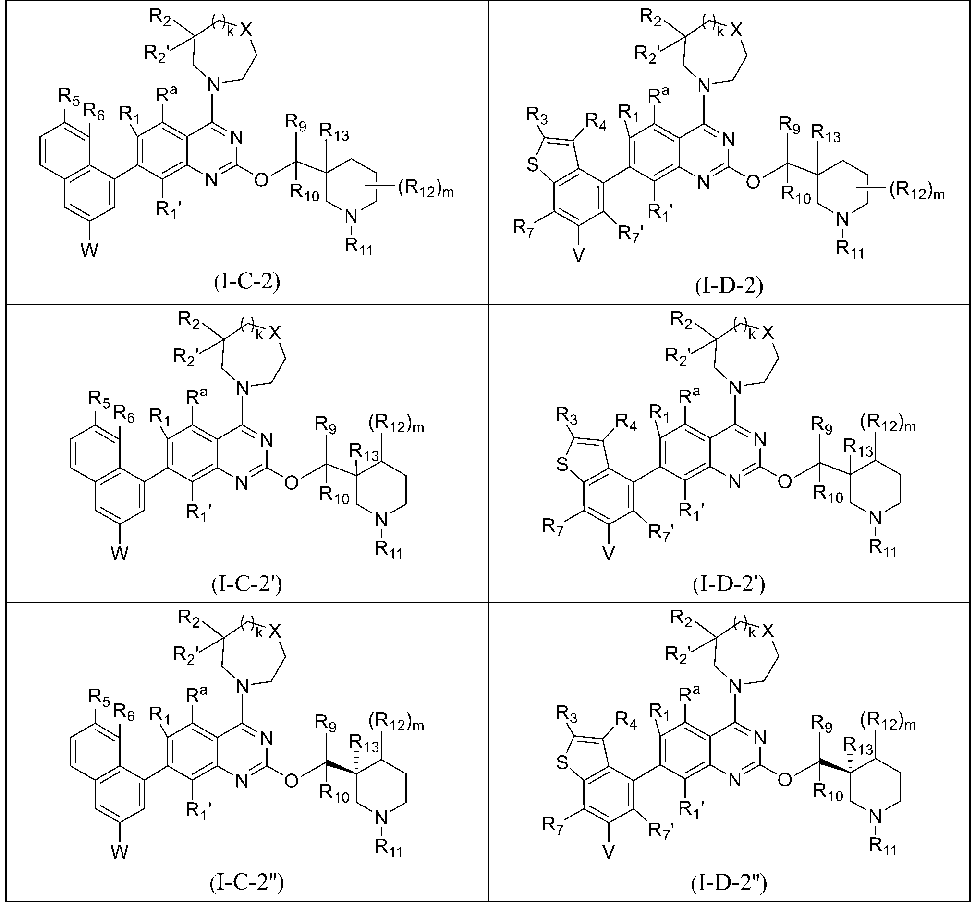

wherein each substituent has the meaning as generally or specifically defined in the respective corresponding embodiments above, and also encompasses any combination of the generally or specifically defined meanings of each substituent;

Preferably, wherein
when M is C-R₁, R₁ is selected from halogen (preferably F or Cl) or -C₁₋₆ alkyl optionally substituted by halogen (preferably -C₁₋₃ alkyl substituted by halogen, more preferably -CF₃), R^{a} is H, and M' is C-R₁' and R₁' is halogen (preferably F);
when M is N, R^{a} is selected from H, -C₂₋₆ alkynyl (preferably -C₂₋₄ alkynyl, more preferably - C≡CH), and -OC₁₋₆ alkyl (preferably -OC₁₋₃ alkyl optionally substituted by deuterium, more preferably -OCH₃, -OCD₃, -OCH₂CH₃), and M' is C-R₁' and R₁' is halogen (preferably F);
the structural fragment 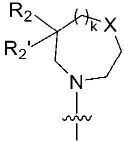 is selected from 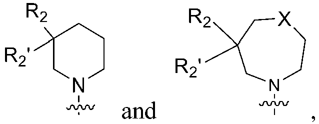 wherein X is selected from C, N, and O; one of R₂ and R₂' is -OH and the other is -C₁₋₆ alkyl, or R₂ and R₂' together with the ring carbon atom to which they are attached form a 4-6 membered spirocycloalkyl or a 4-6 membered spiroheterocycloalkyl containing 1 or 2 heteroatoms selected from N and O;
Preferably, 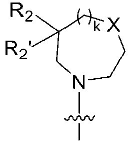 is selected from 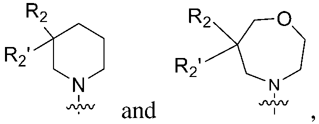 wherein one of R₂ and R₂' is -OH and the other is -C₁₋₃ alkyl, preferably methyl, more preferably 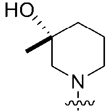 and 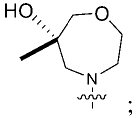
R₃ is selected from -NH₂, -NHC₁₋₆ alkyl, and N(C₁₋₆ alkyl)₂ (preferably selected from -NH₂, - NHC₁₋₃ alkyl, and N(C₁₋₃ alkyl)₂, more preferably -NH₂); R₄ is selected from -CN, halogen, -NO₂, and -C₂₋₆ alkynyl substituted by halogen; V is selected from H, -C₁₋₆ alkyl (preferably -C₁₋₃ alkyl), and halogen; R₇ and R₇' are each H, or each are halogen, or one of them is H and the other is halogen or halogen-substituted C₁₋₆ alkyl (preferably halogen-substituted -C₁₋₃ alkyl), or one is halogen and the other is halogen-substituted C₁₋₆ alkyl (preferably halogen-substituted -C₁₋₃ alkyl), wherein the halogen is preferably F;
Preferably, R₃ is -NH₂, R₄ is -CN, V is H, R₇ and R₇' are each H, or one of them is H and the other is halogen (preferably F);
R₅ is H or halogen (preferably F); R₆ is selected from halogen, -C₂₋₆ alkynyl (preferably -C₂₋₄ alkynyl), and -C₁₋₆ alkyl (preferably -C₁₋₃ alkyl); R₈ and R₈' are each H, or R₈ and R₈' are each halogen, or one of R₈ and R₈' is H and the other is selected from halogen, CN, and NO₂, or one of R₈ and R₈' is H and the other is selected from -C₁₋₆ alkyl optionally substituted by halogen, or one of R₈ and R₈' is selected from H, halogen, -NO₂, CN, and -C₁₋₆ alkyl optionally substituted by halogen, and the other is selected from -C₁₋₆ alkyl optionally substituted by halogen; W is OH;
Preferably, R₅ is halogen (preferably F), R₆ is selected from -C₂₋₆ alkynyl (preferably -C₂₋₄ alkynyl), R₈ and R₈' are each H, W is OH;
Z is selected from O, N, and CH₂;
R₉ and R₁₀ are both H; or one or both are deuterium;
R₁₁ is -C₁₋₆ alkyl (preferably -C₁₋₃ alkyl), wherein the hydrogen atom is optionally replaced by one or more isotope deuterium;
R₁₂ is selected from -C₁₋₆ alkyl optionally substituted by halogen (preferably -C₁₋₃ alkyl) and =C(R^{d})₂, wherein R^{d} is each independently selected from H and halogen (preferably F);
m is 0, 1, or 2 (preferably 1 or 2);
R₁₃ is -C₁₋₆ alkyl (preferably -C₁₋₃ alkyl, more preferably -CH₃).

**Embodiment 6.3:** A compound of formula (I'), a stereoisomer, tautomer, stable isotopic variant, pharmaceutically acceptable salt, or solvate thereof, having a sub-general formula as shown in **Table 2-1** below:

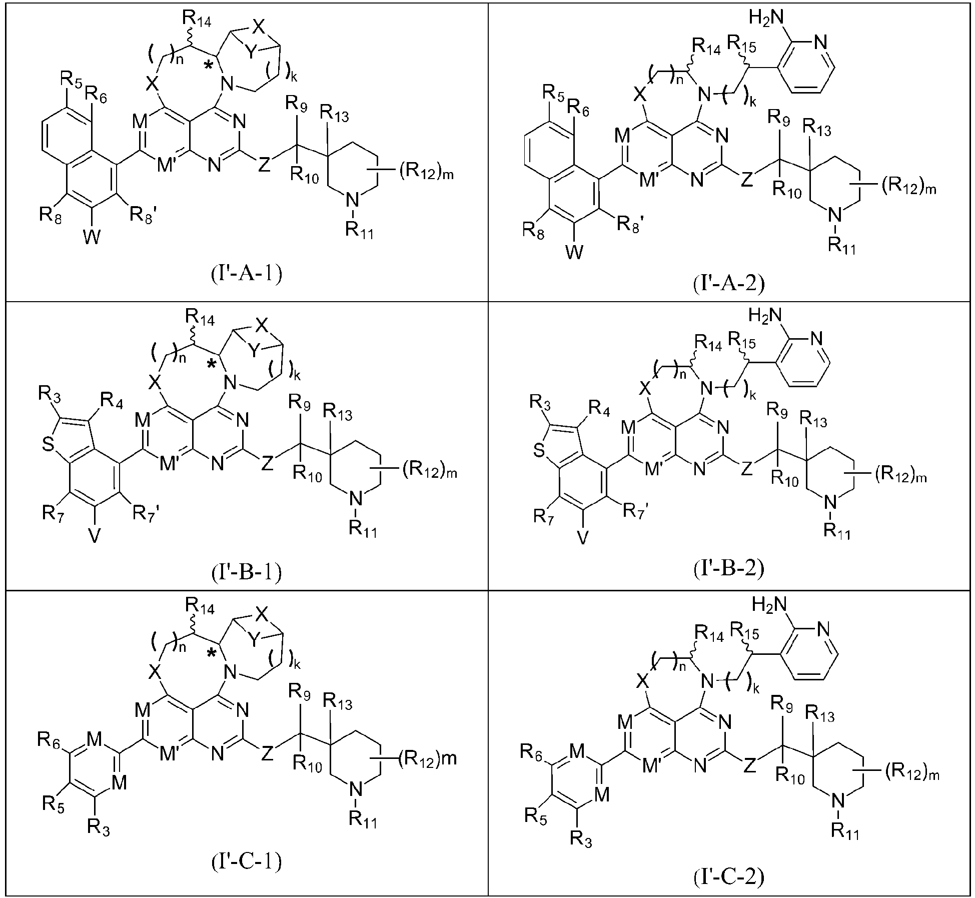

wherein
M, M', X, Z, V, W, R₁, R₁', R₃, R₄, R₅, R₆, R₇, R₇', R₈, R₈', R₉, R₁₀, R₁₁, R₁₂, R₁₃, k, m, n are respectively as defined above for each embodiment of the compound of formula (I);
the structural fragment 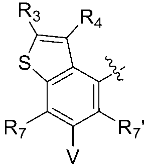 is as defined above in any one of Embodiments 2.1 to 2.1.16 for formula (I);
the structural fragment 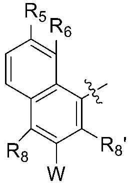 is as defined above in any one of Embodiments 2.2 to 2.2.12 for formula (I);
the structural fragment 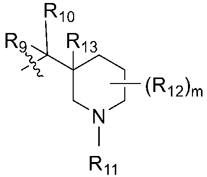 is as defined above in any one of Embodiments 5.1 to 5.4 for formula (I);
Y is selected from -CH₂-, -CH₂CH₂-, -CH₂OCH₂-, or is absent;
R₁₄ and R₁₅ are each independently selected from H and -C₁₋₆ alkyl.

**Embodiment 6.3.1:** The compound of formula (I') according to **Embodiment 6.3,** a stereoisomer, tautomer, stable isotopic variant, pharmaceutically acceptable salt, or solvate thereof, wherein,
in the fragment 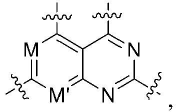 when M is C-R₁, R₁ is selected from halogen (preferably F or Cl) or -C₁₋₆ alkyl optionally substituted by halogen (preferably -C₁₋₃ alkyl substituted by halogen, more preferably -CF₃), and M' is C-R₁' and R₁' is halogen (preferably F); or when M is N, M' is C-R₁' and R₁' is halogen (preferably F);
in the fragment 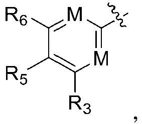 both M are C-R₁, or one M is N and the other M is C-R₁, R₁ are each independently selected from H, halogen (preferably F), and halogen-substituted -C₁₋₆ alkyl (preferably -C₁₋₃ alkyl substituted by one or more F, more preferably -CF₃), preferably the M ortho to R₃ is selected from N and C-R₁ (preferably C-halogen, more preferably C-F), and the M ortho to R₆ is C-R₁ (preferably C-halogen-substituted -C₁₋₃ alkyl, more preferably -C-CF₃); R₃ is selected from -NH₂, -NHC₁₋₆ alkyl, and N(C₁₋₆ alkyl)₂ (preferably selected from -NH₂, -NHC₁₋₃ alkyl, and N(C₁₋₃ alkyl)₂, more preferably -NH₂); R₅ is H or halogen (preferably F); R₆ is selected from halogen (preferably Cl) and -C₁₋₆ alkyl (preferably -C₁₋₃ alkyl);
in the fragment 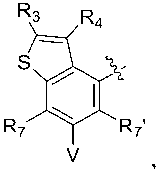 R₃ is selected from -NH₂, -NHC₁₋₆ alkyl, and N(C₁₋₆ alkyl)₂ (preferably selected from -NH₂, -NHC₁₋₃ alkyl, and N(C₁₋₃ alkyl)₂, more preferably -NH₂); R₄ is selected from -CN, halogen, -NO₂, and -C₂₋₆ alkynyl substituted by halogen; V is selected from H, - C₁₋₆ alkyl (preferably -C₁₋₃ alkyl), and halogen; R₇ and R₇' are each H, or each are halogen, or one of them is H and the other is halogen or halogen-substituted C₁₋₆ alkyl (preferably halogen-substituted - C₁₋₃ alkyl), or one is halogen and the other is halogen-substituted C₁₋₆ alkyl (preferably halogen-substituted -C₁₋₃ alkyl), wherein the halogen is preferably F;
Preferably, R₃ is -NH₂, R₄ is -CN, V is H, R₇ and R₇' are each H, or one of them is H and the other is halogen (preferably F);
in the fragment 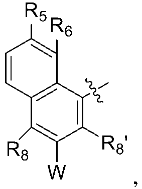 R₅ is H or halogen (preferably F); R₆ is selected from halogen, - C₂₋₆ alkynyl (preferably -C₂₋₄ alkynyl), and -C₁₋₆ alkyl (preferably -C₁₋₃ alkyl); R₈ and R₈' are each H, or R₈ and R₈' are each halogen, or one of R₈ and R₈' is H and the other is selected from halogen, CN, and NO₂, or one of R₈ and R₈' is H and the other is selected from -C₁₋₆ alkyl optionally substituted by halogen, or one of R₈ and R₈' is selected from H, halogen, -NO₂, CN, and -C₁₋₆ alkyl optionally substituted by halogen, and the other is selected from -C₁₋₆ alkyl optionally substituted by halogen; W is OH;
Preferably, R₅ is halogen (preferably F), R₆ is selected from -C₂₋₆ alkynyl (preferably -C₂₋₄ alkynyl), R₈ and R₈' are each H, W is OH;
X is selected from O and NH;
Y is selected from -CH₂CH₂- and is absent;
Z is selected from O;
R₉ and R₁₀ are both H; or one or both are deuterium;
R₁₁ is -C₁₋₆ alkyl (preferably -C₁₋₃ alkyl), wherein the hydrogen atom is optionally replaced by one or more isotope deuterium;
R₁₂ is selected from -C₁₋₆ alkyl optionally substituted by halogen (preferably -C₁₋₃ alkyl) and =C(R^{d})₂, wherein R^{d} is each independently selected from H and halogen (preferably F);
m is 0, 1, or 2 (preferably 1 or 2);
n and k are 0 or 1 (preferably 0);
R₁₃ is -C₁₋₆ alkyl (preferably -C₁₋₃ alkyl, more preferably -CH₃);
R₁₄ and R₁₅ are H or -C₁₋₃ alkyl, more preferably -CH₃.

**Embodiment 6.4:** The compound according to any one of Embodiments 6.1 to 6.3.1, a stereoisomer, tautomer, stable isotopic variant, pharmaceutically acceptable salt, or solvate thereof, wherein the R fragment 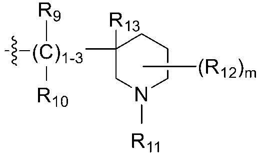 is selected from

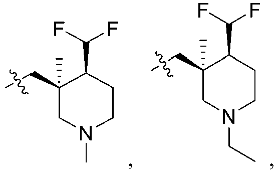

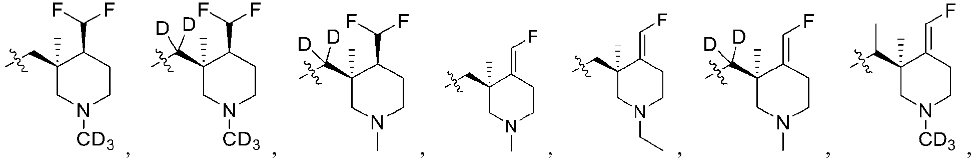

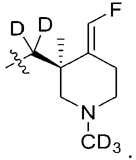

**Embodiment 6.5:** The compound according to any one of Embodiments 6.1 to 6.4, a stereoisomer, tautomer, stable isotopic variant, pharmaceutically acceptable salt, or solvate thereof, wherein the Ar moiety, i.e., the left-side fragment, optionally has axial chirality, as shown below: 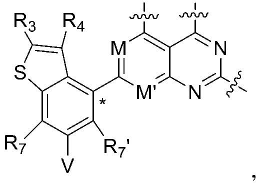 e.g., 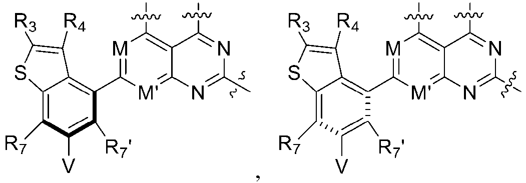 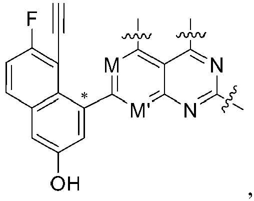 e.g., 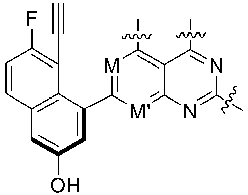 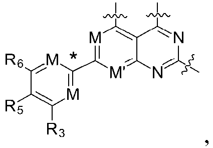 e.g.,

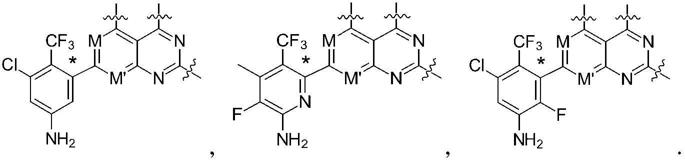

**Embodiment 7:** A KRas mutant inhibitor compound selected from the following compounds, stereoisomers, tautomers, stable isotopic variants, pharmaceutically acceptable salts, or solvates thereof: 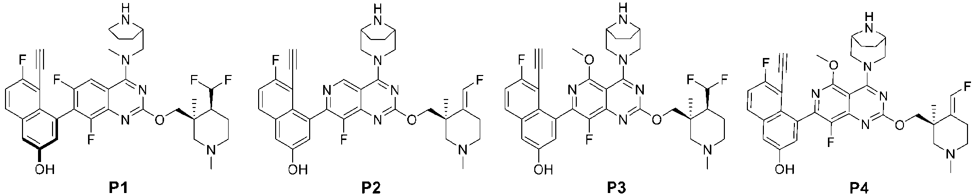 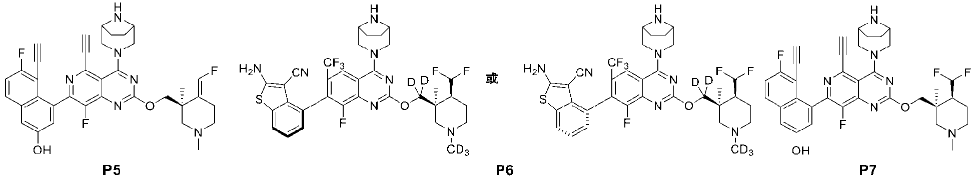 or a pharmaceutically acceptable salt or solvate thereof.

It should be noted that the KRas mutant inhibitor compounds defined above, which are suitable for the ADCs of the present disclosure, are bonded to the linker unit L of the ADC via the ring NH present in the 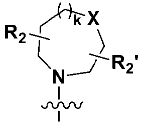 moiety thereof, and/or via the OH present on the side-chain naphthalene or benzothiophene ring.

**Embodiment 8:** A Ras mutant inhibitor compound selected from the following compounds, stereoisomers, tautomers, stable isotopic variants, pharmaceutically acceptable salts, or solvates thereof: 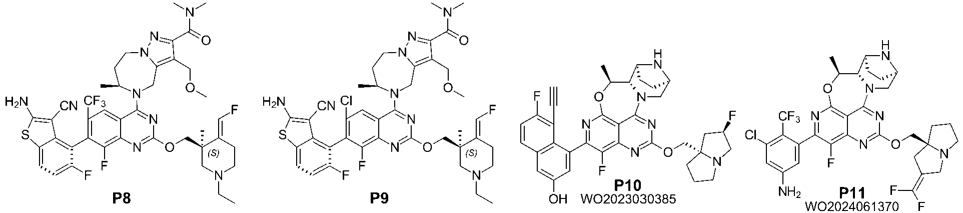 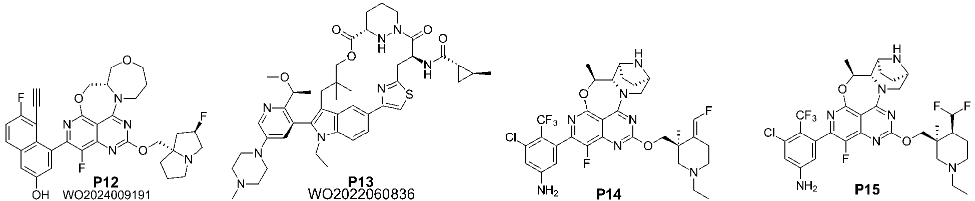 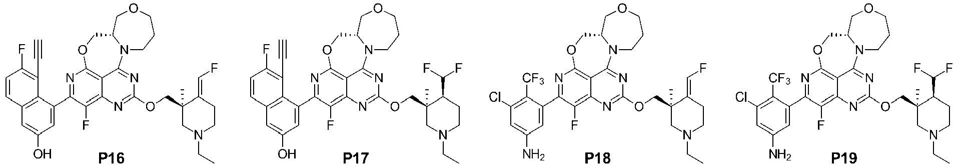 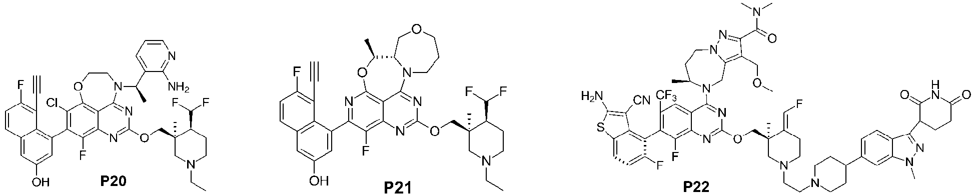 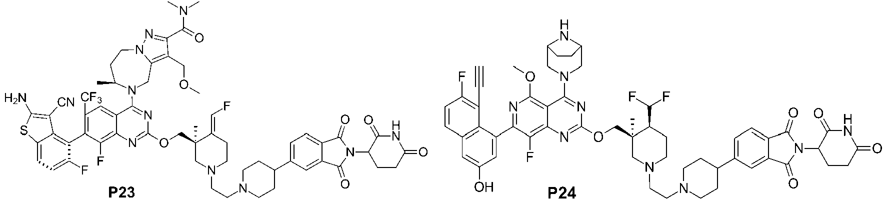 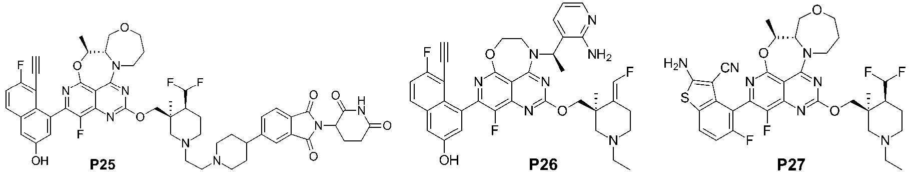 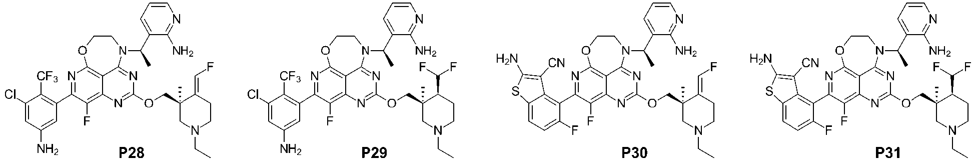 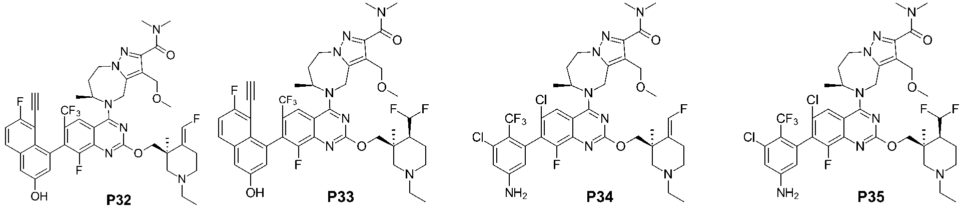 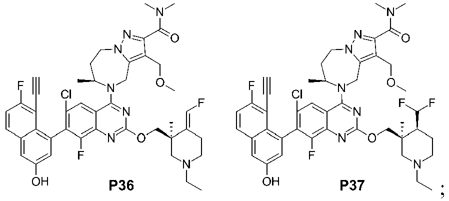 wherein each Ras mutant inhibitor compound is linked to the linker unit L of the ADC via the - NH₂, -NH-, N, or -OH present in its structure; or selected from Ras inhibitor compounds disclosed in the following patent publications, with representative compounds as follows:
Ras protein degraders 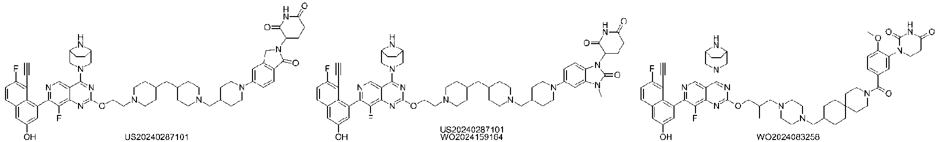 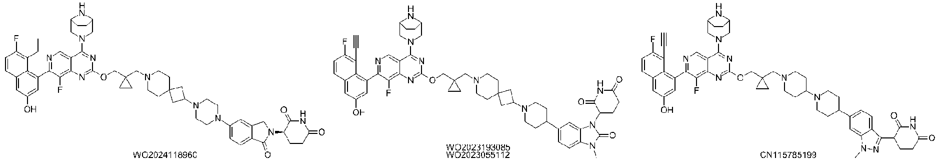 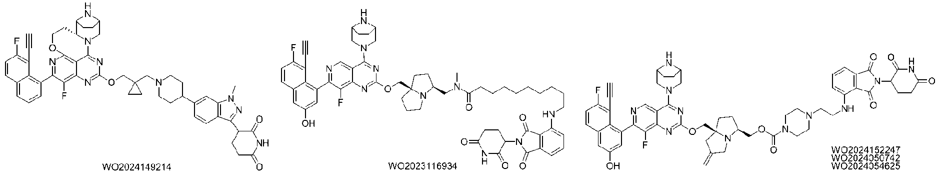 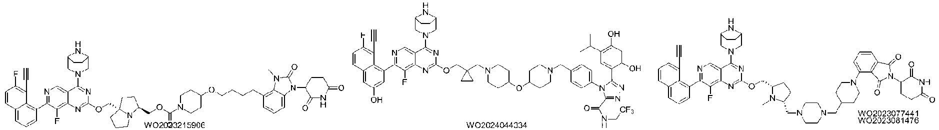 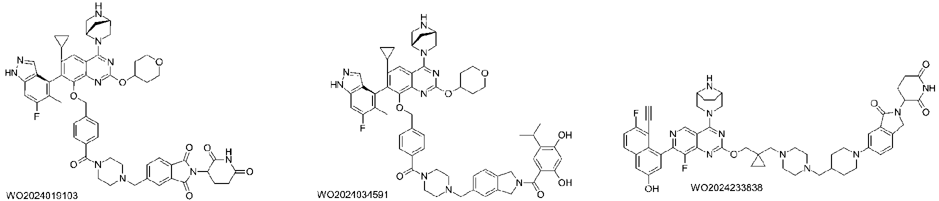 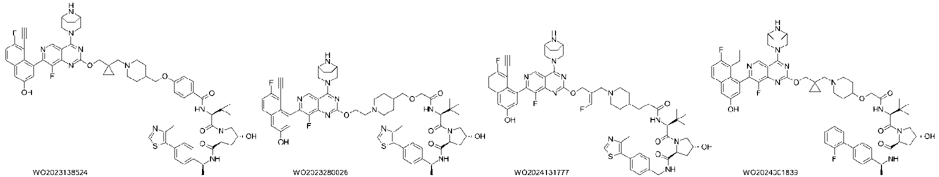 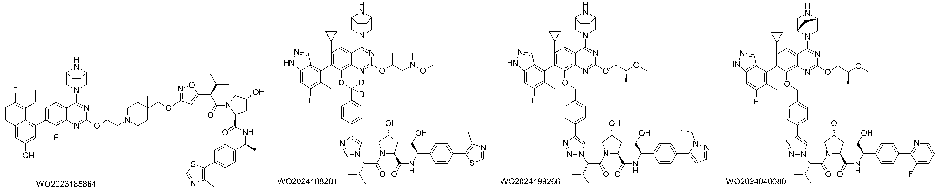 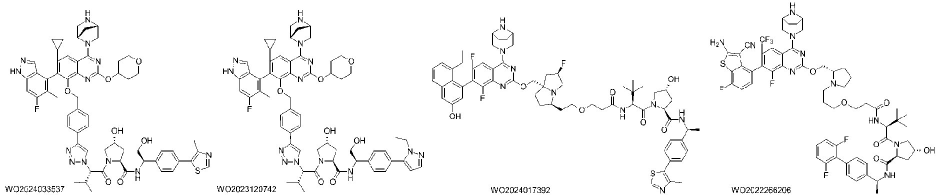 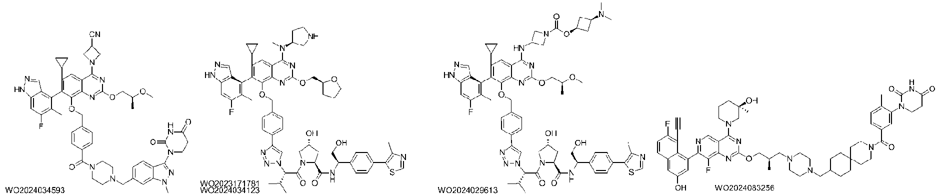 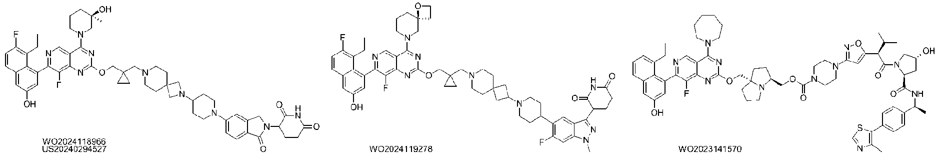 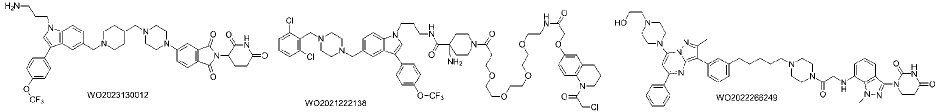 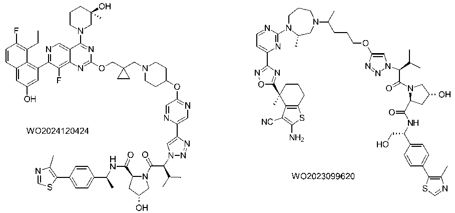
Ras molecular glue 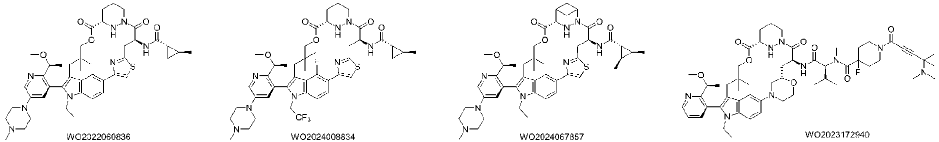 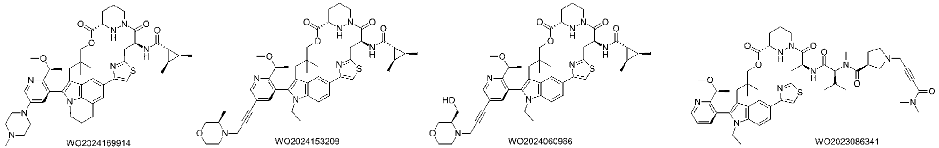 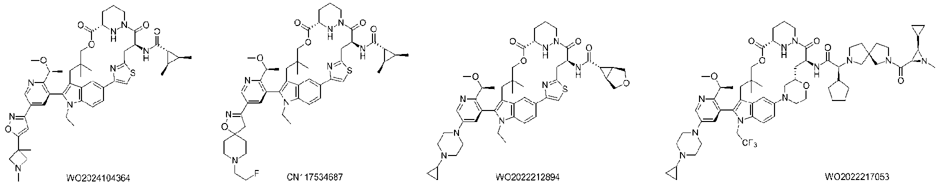 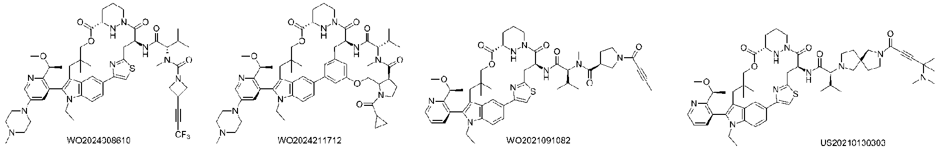 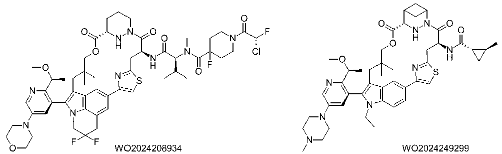
Tri/tetracyclic Ras inhibitors 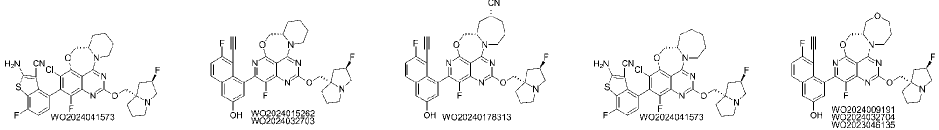 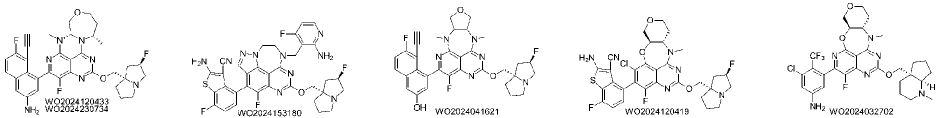 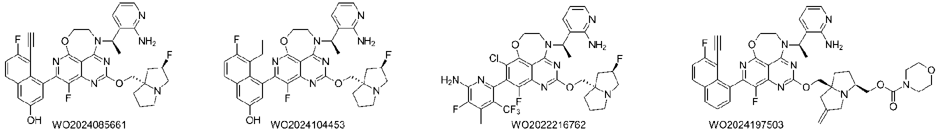 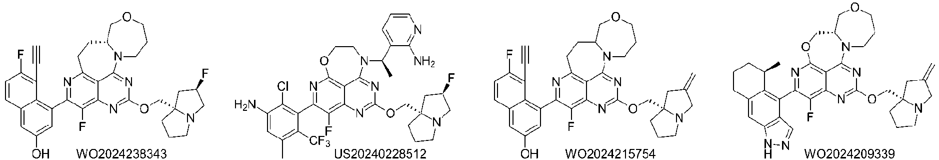 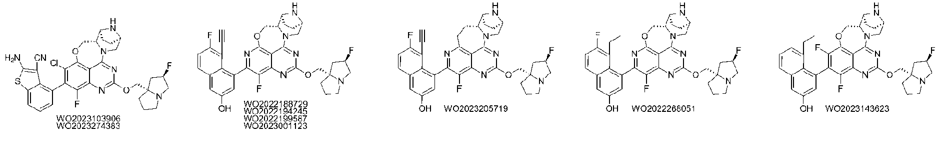 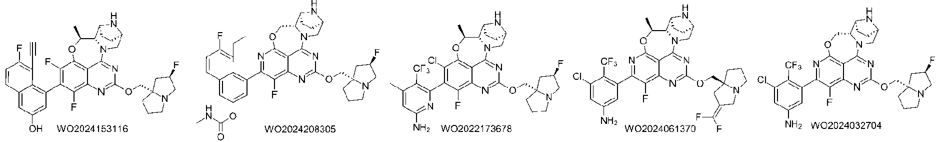 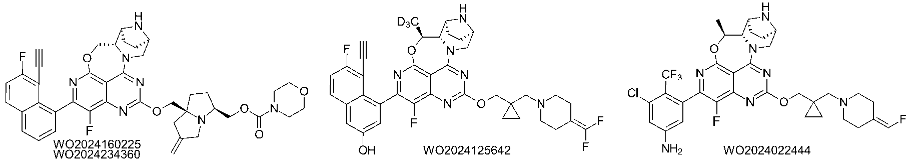
Small molecule Ras inhibitors 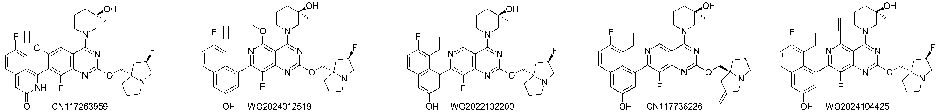 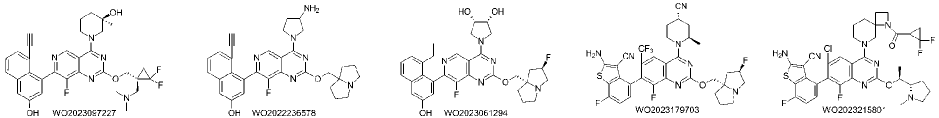 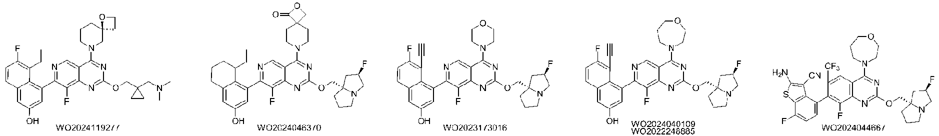 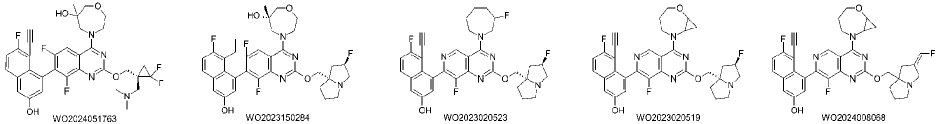 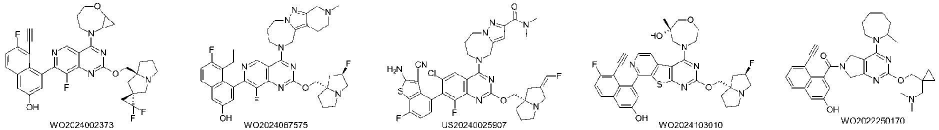 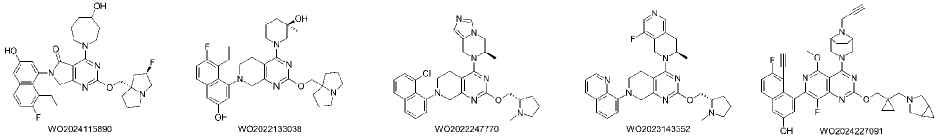 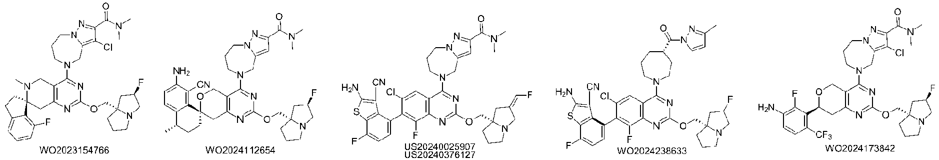 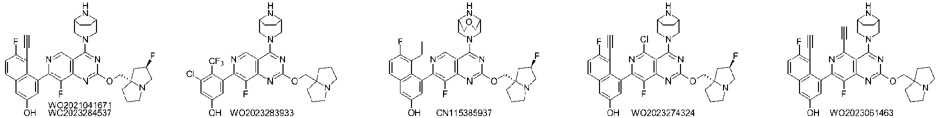 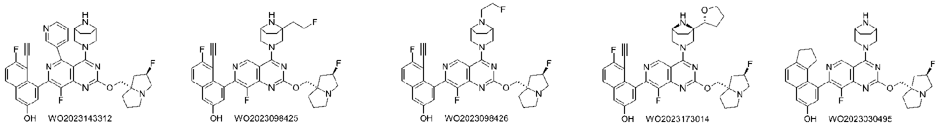 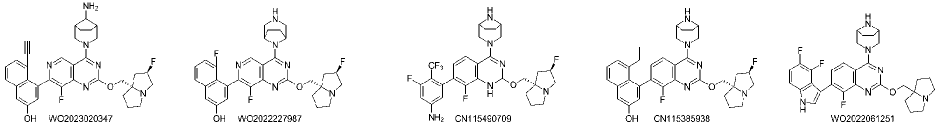 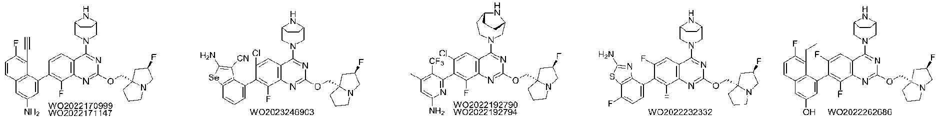 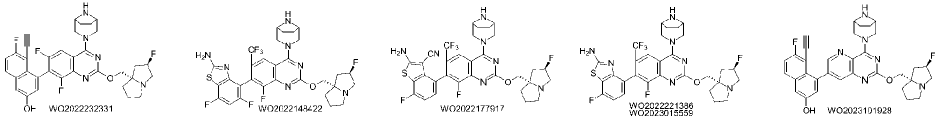 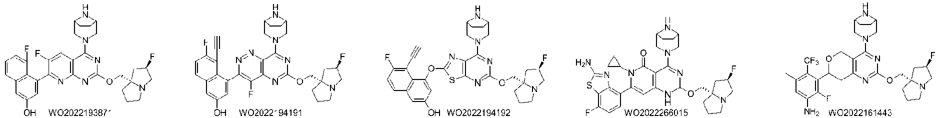 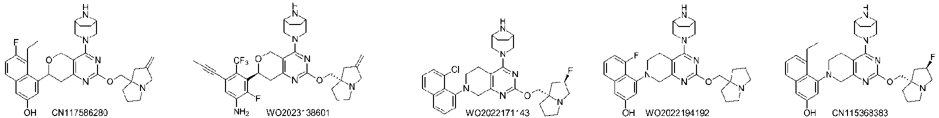 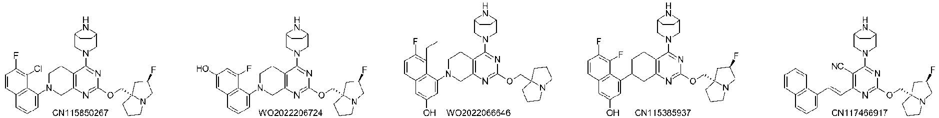 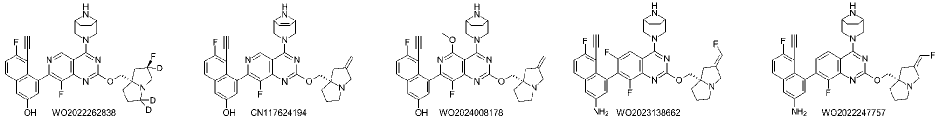 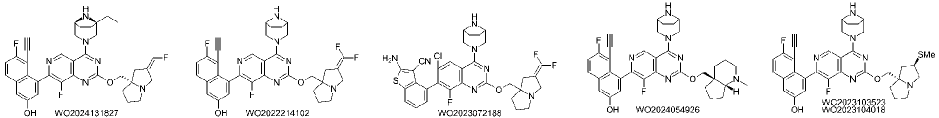 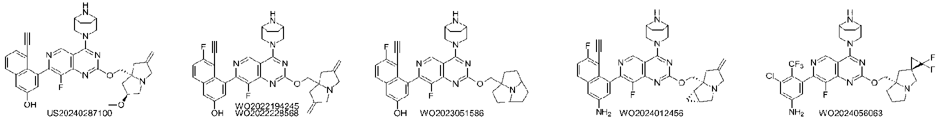 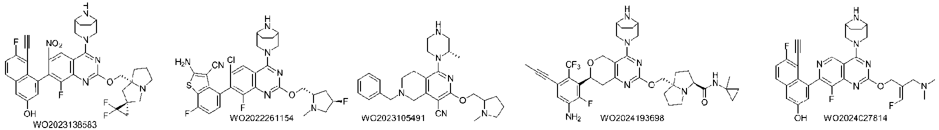 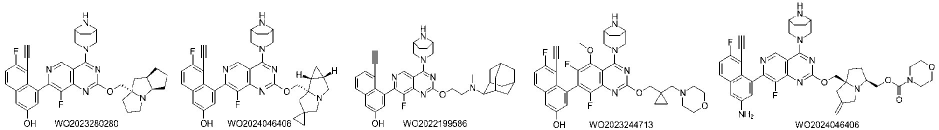 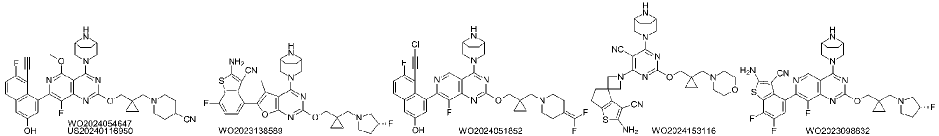 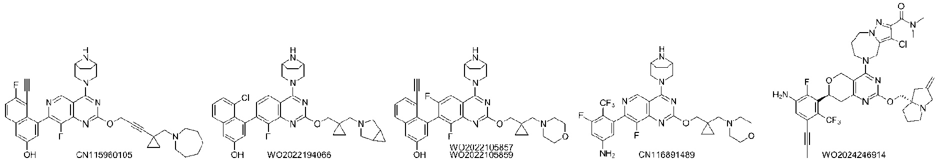
Other types of small molecule Ras inhibitors

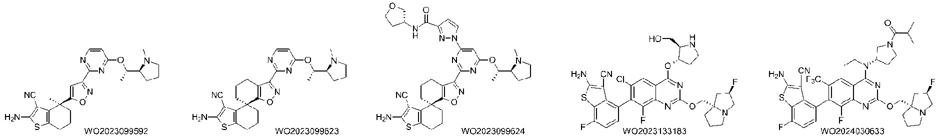

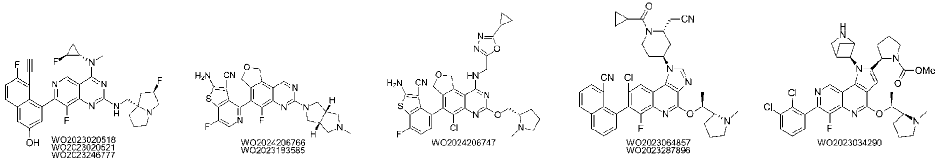

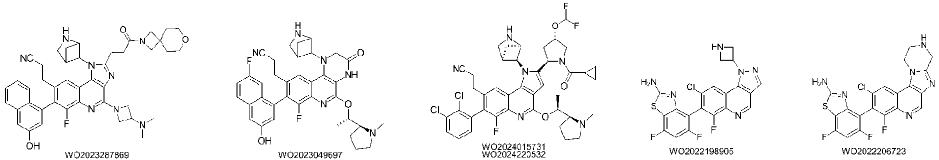

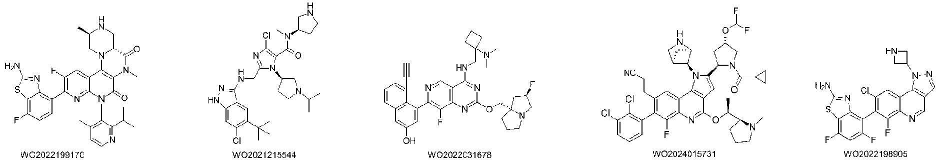

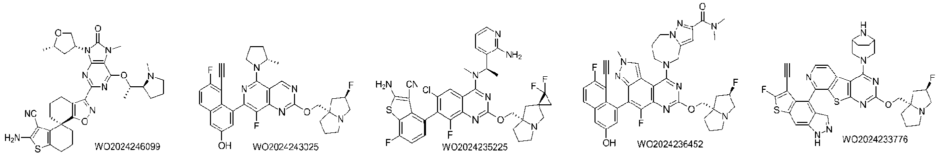

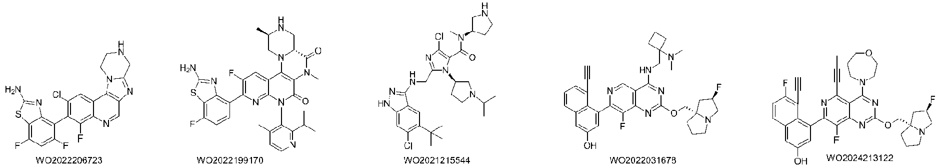

It should be noted that the Ras mutant inhibitor compounds of the present disclosure encompass each of the above independent embodiments or each specific embodiment, and also encompass embodiments constituted by any combination or sub-combination of the above various embodiments or specific embodiments, and also encompass embodiments constituted by any combination of any of the above preferred or exemplified embodiments.

It should be noted that the present disclosure encompasses ADC compounds formed by any of the above general, specific, or preferred Ras mutant inhibitor compounds with the linker units and antibodies defined herein.

### Linker Unit L

In the ADCs described herein, the Ras inhibitor, e.g., KRas mutant inhibitor, is linked to the antibody or antigen-binding fragment via a linker unit. The linker unit connects the Ras inhibitor, e.g., KRas mutant inhibitor, to the antibody or antigen-binding fragment by forming a covalent bond with the Ras inhibitor, e.g., KRas mutant inhibitor, at one position thereof and forming a covalent bond with the antibody or antigen-binding fragment at another position thereof. The linker units can be monovalent with respect to the Ras inhibitor, e.g., KRas mutant inhibitor, such that they covalently link a single Ras inhibitor, e.g., KRas mutant inhibitor, to a single site on the antibody or fragment thereof, or they can be multivalent with respect to the Ras inhibitor, e.g., KRas mutant inhibitor, such that they covalently link more than one Ras inhibitor, e.g., KRas mutant inhibitor, to a single site on the antibody or fragment thereof. As used herein, the expression "linker unit" is intended to include un-conjugated, partially conjugated (i.e., coupled only to the Ras inhibitor, e.g., KRas mutant inhibitor, or only to Ab), and fully conjugated forms (i.e., coupled to both the Ras inhibitor, e.g., KRas mutant inhibitor, and Ab) of the linker unit.

The number of Ras inhibitors, e.g., KRas mutant inhibitors, linked to the antibody or antigen-binding fragment of the ADC can vary (referred to as "drug-to-antibody ratio" or "DAR") and will be limited by the number of available linking sites on the antibody or antigen-binding fragment and the number of Ras inhibitors, e.g., KRas mutant inhibitors, attached to a single linker. In ADCs comprising multiple Ras inhibitors, e.g., KRas mutant inhibitors, each Ras inhibitor, e.g., KRas mutant inhibitor, may be the same or different. ADCs with a DAR of 10 or even higher are contemplated as long as the ADC does not exhibit unacceptable levels of aggregation under conditions of use and/or storage. In some embodiments, the ADCs described herein can have a DAR in the range of about 1 to 10, 1 to 9, 1 to 8, 1 to 7, 1 to 6, 1 to 5, or 1 to 4. In some embodiments, the ADCs described herein can have a DAR in the range of about 2 to 10, 2 to 9, 2 to 8, 2 to 7, 2 to 6, 2 to 5, or 2 to 4. In certain specific embodiments, the ADC can have a DAR of about 1, 2, 3, or 4. In other specific embodiments, the ADC can have a DAR of about 5, 6, 7, or 8. In some specific embodiments, the ADC can have a DAR of about 1.

The linker unit L suitable for the ADC of the present disclosure can be any linker capable of coupling the drug of the present disclosure to the antibody. Suitably, the incorporation of the linker should ensure sufficient stability of the ADC of the present disclosure in the circulatory system, should not be cleaved prematurely in the circulation to trigger off-target toxicity, while allowing for rapid and effective release of the KRas mutant inhibitor at the target site (e.g., tumor cells or tumor environment). For example, the linker unit can be chemically stable to the extracellular environment and serum, or can include intentionally labile linker units and can release the Ras inhibitor, e.g., KRas mutant inhibitor, in the extracellular environment or tumor microenvironment.

In some embodiments, the linker unit includes a bond designed to release the Ras inhibitor, e.g., KRas mutant inhibitor, after intracellular internalization of the ADC. In some specific embodiments, the linker unit includes a bond designed to be cleaved and/or digested intracellularly or otherwise specifically or non-specifically degraded.

In some embodiments, the linker unit in the ADC of formula (X) of the present disclosure is a non-degradable linker. Examples of non-degradable linker units include, but are not limited to, thioether linkers, N-succinimidyl-4-(N-maleimidomethyl)cyclohexane-1-carboxylate (SMCC), and maleimidocaproyl (MC). Generally, such linkers are more stable, and ADCs containing such linkers must be internalized by cells, where intracellular lysosomal proteases degrade the antibody portion of the ADC to release the active drug molecule.

In further embodiments, the linker unit in the ADC of formula (X) of the present disclosure is a degradable linker unit, which contains one or more chemically or enzymatically cleavable chemical bonds. Drug release from ADCs containing such linkers is triggered by the nature of the cleavage site in the linker. Therefore, the cleavage site of such linkers can be designed according to the characteristics of the target therapeutic site (e.g., tumor cell lysosomes and/or tumor environment).

In some embodiments, the degradable linker unit contains a chemically labile group that exploits the differential properties between plasma and some cytoplasmic compartments, such as the acidic environment of endosomes or lysosomes or the high thiol concentration in the cytosol (e.g., glutathione); in some cases, the plasma stability of linkers containing chemically labile groups can be increased or decreased by using substituents to alter the steric hindrance around the group.

In some embodiments, the chemically labile group of the degradable linker unit is an acid-labile group that can remain intact during circulation at neutral pH and hydrolyze under acidic conditions to release the Ras inhibitor, e.g., KRas mutant inhibitor, for example, in an acidic tumor environment or upon internalization into endosomal (pH 5.0-6.5) and lysosomal (pH 4.5-5.0) cellular compartments. This pH-dependent release mechanism can be optimized by chemical modification to finely tune the release of the Ras inhibitor, e.g., KRas mutant inhibitor, for a specific pH. Examples of such acid-labile groups include hydrazones, hydrazines, acetals, orthoesters, or imine groups.

In some embodiments, the degradable linker unit contains a reducible group, such as a disulfide group. This group is reduced when the ADC is internalized into cells due to the more reducing environment provided by the cytosol (e.g., reductive glutathione) in cells, thereby releasing the drug. Irregular blood flow in tumor cells can induce hypoxic conditions, leading to increased reductase activity and elevated glutathione concentrations, which favors the selective release of drugs in tumor cells for linkers containing disulfide bonds.

In some embodiments, the degradable linker unit is an enzyme-degradable linker unit and is more stable in plasma and extracellular environments than chemically labile linkers. Such linkers can be peptide-based or include a peptide region, or be non-peptide linkers, such as peptidomimetics, or sugars, esters, and amides. Such linkers can be cleaved by tumor-specific enzymes, for example, tumor-specific proteases with increased abundance in tumors and/or the tumor environment, including but not limited to lysosomal proteases, such as cathepsins (e.g., cathepsin B), legumain, MMP-2/9, plasmin, esterases, amidases, glutathione, etc.

Generally, an enzyme-cleavable linker unit can be composed of a self-immolative linker, a cleavable linker, optionally a property-modulating unit, optionally a linker unit, and an antibody linker. The self-immolative linker connects the drug P to the cleavable linker, facilitating the release of the active drug molecule from the remainder of the ADC, such as p-aminobenzyl, p-hydroxybenzyl, p-aminobenzyloxycarbonyl, p-hydroxybenzyloxycarbonyl, etc.; the cleavable linker contains, under enzyme-based release mechanisms, peptides or peptide analogs, esters (e.g., carbamates, sulfates), amides, disulfide-containing moieties, sugars, etc., that can be recognized by enzymes; the addition of a property-modulating unit may be beneficial for improving the properties of the ADC, such as stability in the bloodstream, efficacy of the ADC at the target site, and optimizing the hydrophilicity of the ADC. For example, when the drug is highly hydrophobic, the addition of a moiety such as a PEG unit can be considered (but not required) to optimize the hydrophilicity of the ADC, e.g., reducing precipitation and aggregation; the antibody linker connects the antigen-targeting antibody or antigen-binding fragment to the remainder of the conjugate and has a functional group capable of forming a bond with a functional group on the antibody.

In one aspect, the linker unit L in the ADC of formula (X) of the present disclosure has the structure of formula (II):

-A(S)-B(D¹)-D-E(D¹)-G- (II),

wherein
A is a self-immolative linker;
S is an optional solubilizing sugar unit;
B is a cleavable linker, which is absent, or is selected from a peptide residue of 2-8 amino acids, preferably a dipeptide, tripeptide, or tetrapeptide; an amide bond-containing moiety; a carbamatecontaining moiety; a thioether bond-containing moiety;
D and D¹ are each independently an optional property-modulating unit, which when present is selected from polyethylene glycol (PEG), hydrophilic peptide, cyclodextrin unit, polyamine, polyamide, polysaccharide, dendrimer, and bifunctional hydrocarbon chain;
E is an optional present linker unit;
G is an antibody linker connected to Ab.

Accordingly, the composition of the ADC of the present disclosure can be represented as: [PA(S)-B(D¹)-D-E(D¹)-G]_{q}- Ab.

In another aspect, the linker unit L in the ADC of formula (X) of the present disclosure has the structure of formula (II'):

-J-B(D¹)-D-E(D¹)-G- (II'),

wherein
J is a self-immolative linker;
B is a cleavable linker, which is absent, or is selected from a peptide residue of 2-8 amino acids, preferably a dipeptide, tripeptide, or tetrapeptide; an amide bond-containing moiety; a carbamatecontaining moiety; a thioether bond-containing moiety;
D and D¹ are each independently an optional property-modulating unit, which when present is selected from polyethylene glycol (PEG), hydrophilic peptide, cyclodextrin unit, polyamine, polyamide, polysaccharide, dendrimer, and bifunctional hydrocarbon chain;
E is an optional present linker unit;
G is an antibody linker connected to Ab.

Accordingly, the composition of the ADC of the present disclosure can be represented as: [P-J-B(D¹)-D-E(D¹)-G]_{q}- Ab.

General, specific, or preferred embodiments for each possible component of the linker unit L are described below. It should be noted that the present disclosure encompasses linker units L obtained by any combination of the general, specific, or preferred embodiments of each component with the general, specific, or preferred embodiments of any one or more of the remaining components; accordingly, the present disclosure encompasses ADC compounds obtained by any combination of the linker units obtained by said any combination with the general, specific, or preferred embodiments of the antibody and drug moieties defined herein.

### G-Antibody Linker

The role of the antibody linker is to connect the antigen-targeting antibody or antigen-binding fragment to the remainder of the conjugate and has a functional group capable of forming a bond with a functional group on the antibody.

In some embodiments, the antibody linker has a nucleophilic group capable of interacting with a reactive electrophilic group on the antibody to form a covalent bond between the antibody and the linker unit. The electrophilic group on the antibody includes, but is not limited to, aldehyde and ketone carbonyl groups, and the nucleophilic group on the antibody linker includes, but is not limited to, hydrazide, hydroxylamine, amino, hydrazine, thiosemicarbazone, carboxylic acid hydrazide, and aryl hydrazide.

In other embodiments, the antibody linker has an electrophilic group capable of interacting with a reactive nucleophilic group on the antibody to form a covalent bond between the antibody and the linker unit. The nucleophilic group on the antibody includes, but is not limited to, thiol, hydroxyl, or amino functional groups, and the electrophilic group on the antibody linker includes, but is not limited to, maleimide, haloacetamide groups, activated disulfides, active esters such as NHS esters or HOBt esters, haloformates, acyl halides, alkyl halides, or benzyl halides such as haloacetamide. In some embodiments, the bond formed between the antibody linker and the antibody is a thioether, amide, ester, carbamate, carbonate, urea, disulfide, or ether.

G in the linker unit L of formula (II) or (II') of the ADC of the present disclosure has the following structure:

-G₁-G₂-G₃-,

wherein:
G₁ is a bonding atom from Ab, such as S, N, or C atom;
G2 is selected from:
   - a 5-10 membered heterocyclyl group containing 1 or 2 heteroatoms selected from N, S, and O, wherein ring carbon atoms are optionally oxidized;
   - 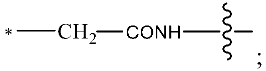
   - 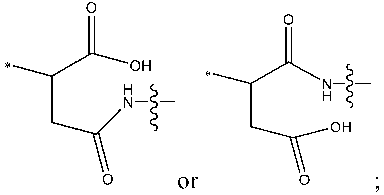
   - 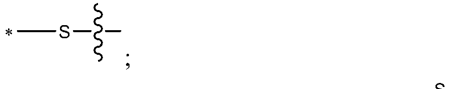
   - 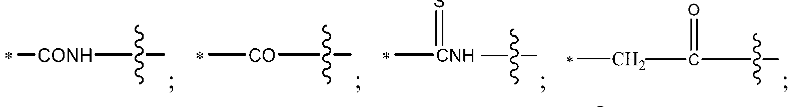
   - 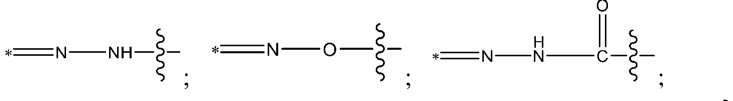
wherein the left * indicates the point of attachment to G₁, and the right 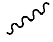 indicates the point of attachment to G₃;
G₃ is selected from a bond, -C₁-₁₀ alkylene-C(=O)-, -C₃-₁₀ alkynylene-C(=O)-, -C₃-₁₀ alkenylene-C(=O)-, -C₁-₁₀ heteroalkylene-C(=O)-, -C₃-₈ cycloalkylene-C(=O)-, -O-C₁-₁₀ alkylene-C(=O)-, -C₆-₁₀ arylene-C(=O)-, -C₁-₁₀ alkylene-C₆-₁₀ arylene-C(=O)-, -C₆-₁₀ arylene-C₁₋₁₀ alkylene-C(=O)-, -C₁-₁₀ alkylene-C₃-₈ cycloalkylene-C(=O)-, -C₃-₈ cycloalkylene-C₁-₁₀ alkylene-C(=O)-, -C₃-₈ heterocyclylene-C(=O)-, -C₁-₁₀ alkylene-C₃-₈ heterocyclylene-C(=O)-, -C₃-₈ heterocyclylene-C₁-₁₀ alkylene-C(=O)-, -C₁-₁₀ alkylene-C(=O)-NH-C₁-₁₀ alkylene-O-C₁-₁₀ alkylene-C(=O)-, -C₃-₁₀ alkynylene-C(=O)-NH-C₁-₁₀ alkylene-O-C₁-₁₀ alkylene-C(=O)-, -C₃-₁₀ alkenylene-C(=O)-NH-C₁-₁₀ alkylene-O-C₁-₁₀ alkylene-C(=O)-, -C₁-₁₀ heteroalkylene-C(=O)-NH-C₁-₁₀ alkylene-O-C₁-₁₀ alkylene-C(=O)-, -C₃-₈ cycloalkylene-C(=O)-NH-C₁-₁₀ alkylene-O-C₁-₁₀ alkylene-C(=O)-, -O-C₁-₁₀ alkylene-C(=O)-NH-C₁-₁₀ alkylene-O-C₁-₁₀ alkylene-C(=O)-, -C₆-₁₀ arylene-C(=O)-NH-C₁-₁₀ alkylene-O-C₁-₁₀ alkylene-C(=O)-, -C₁-₁₀ alkylene-C₆-₁₀ arylene-C(=O)-NH-C₁-₁₀ alkylene-O-C₁-₁₀ alkylene-C(=O)-, -C₆-₁₀ arylene-C₁₋₁₀ alkylene-C(=O)-NH-C₁-₁₀ alkylene-O-C₁-₁₀ alkylene-C(=O)-, -C₁-₁₀ alkylene-C₃-₈ cycloalkylene-C(=O)-NH-C₁-₁₀ alkylene-O-C₁-₁₀ alkylene-C(=O)-, -C₃-₈ cycloalkylene-C₁-₁₀ alkylene-C(=O)-NH-C₁-₁₀ alkylene-O-C₁-₁₀ alkylene-C(=O)-, -C₃₋₈ heterocyclylene-C(=O)-NH-C₁-₁₀ alkylene-O-C₁-₁₀ alkylene-C(=O)-, -C₁-₁₀ alkylene-C₃-₈ heterocyclylene-C(=O)-NH-C₁-₁₀ alkylene-O-C₁-₁₀ alkylene-C(=O)-, -C₃-₈ heterocyclylene-C₁-₁₀ alkylene-C(=O)-NH-C₁-₁₀ alkylene-O-C₁-₁₀ alkylene-C(=O)-, -C₁-₁₀ alkylene-NH-, -C₁-₁₀ heteroalkylene-NH-, -C₃-₈ cycloalkylene-NH-, -O-C₁-₁₀ alkylene-NH-, -C₆-₁₀ arylene-NH-, -C₁-₁₀ alkylene-C₆-₁₀ arylene-NH-, -C₆-₁₀ arylene-C₁₋₁₀ alkylene-NH-, -C₁-₁₀ alkylene-C₃-₈ cycloalkylene-NH-, -C₃-₈ cycloalkylene-C₁-₁₀ alkylene-NH-, -C₃₋₈ heterocyclylene-NH-, -C₁-₁₀ alkylene-C₃-₈ heterocyclylene-NH-, and -C₃-₈ heterocyclylene-C₁-₁₀ alkylene-NH-, wherein each group attached to -C(=O)- or -NH- in G₃ is optionally substituted by Bu, and G₃ is connected via its -C(=O)- or -NH-to the linker unit E (when present), or the property-modulating unit D (when present and E is absent), or the cleavable linker B (when both E and D are absent), and the other end group is connected to G₂.

In some embodiments, each group attached to -C(=O)- or -NH- in G₃ is optionally substituted by the following Bu group: H, deuterium, halogen, NO₂, CN, -ORₕ, -ORₕ, -N(Rₕ)₂, -CORₕ, -CO₂ Rₕ, -C-(O)C(O)Rₕ, -C(O)CH₂C(O)Rₕ, -S(O)Rₕ, -SO₂Rₕ, C(O)N(Rₕ)₂, -SO₂N(Rₕ)₂, -OC(O)Rₕ, - N(Rₕ)SO₂Rₕ, and -C₁₋₆ alkyl optionally substituted by the preceding groups, wherein Rₕ is H or -C₁₋₆ alkyl optionally substituted by halogen, or wherein two Rₕ groups attached to the same N atom together with the nitrogen to which they are attached form a 4-7 membered heterocyclyl group. Preferably, the substituent on G₃ is an aminoalkyl moiety, such as -(CH₂)₁₋₆NH₂, -(CH₂)₁₋₆NHRₕ, or -(CH₂)₁₋₆N(Rₕ)₂, or wherein two Rₕ groups attached to the same N atom together with the nitrogen to which they are attached form an azetidinyl, pyrrolidinyl, or piperidinyl group.

In other embodiments, each group attached to -C(=O)- or -NH- in G₃ is optionally substituted by the Bu group of formula (A): 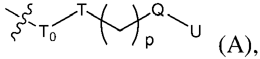 wherein,
T₀ is -C₁₋₆ alkylene-;
T is selected from -C(R^{a})₂-, -O-, -NR^{a}-, or is absent;
Q is selected from -CO-, -O-, -NR^{a}-, or is absent;
U is selected from glycosyl or a derivative thereof, 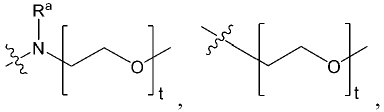 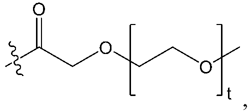 and a hydrophilic peptide comprising 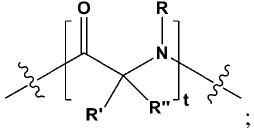
R^{a} is selected from H or -C₁₋₆ alkyl, preferably H or -CH₃;
the subunit 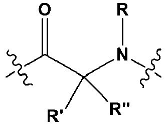 is selected from natural amino acid residues and unnatural amino acid residues;
p is an integer from 0 to 4, for example 0-2, 1-2, 2-4;
t is an integer from 0 to 20, for example 0-8, 0-6, 0-4, 0-2, 1-20, 1-10, 2-10, 2-8, 2-6, 2-4, 4-14, 6-10, 6-12, 8-10, 8-14.

The glycosyl or derivative thereof among the substituents carried by G₃ of the antibody linker of the ADC of the present disclosure is selected from monosaccharide groups, disaccharide groups, oligosaccharide groups, or polysaccharide groups or derivatives thereof, preferably the monosaccharides or disaccharides or derivatives thereof as generally, specifically, or preferably defined in the "Linker Unit L" section herein for the solubilizing sugar unit S, more preferably the monosaccharides or disaccharide groups or derivatives thereof exemplified in Tables 3 and 4.

In some embodiments, U is a monosaccharide or disaccharide or derivative thereof as defined herein and is linked via a glycosidic bond (-O-sugar residue); in other embodiments, U is an amino sugar or derivative thereof as defined herein and is linked via an amino group, preferably an amino sugar derivative of a monosaccharide or disaccharide; in other embodiments, U is a sugar acid or uronic acid or derivative thereof as defined herein and is linked via a carbonyl group.

In the hydrophilic peptide carried by the ADC of the present disclosure, the amino acid residues as subunits can be the conventional 20 natural amino acids. Correspondingly, the combinations of values for R, R', R" in the repeating subunit -CO-CR'R"-NR- correspond to alanine (Ala), arginine (Arg), aspartic acid (Asp), asparagine (Asn), histidine (His), glycine (Gly), glutamic acid (Glu), glutamine (Gln), phenylalanine (Phe), lysine (Lys), leucine (Leu), serine (Ser), tyrosine (Tyr), threonine (Thr), isoleucine (Ile), proline (Pro), tryptophan (Trp), valine (Val), cysteine (Cys), methionine (Met). Preferred are polar natural amino acids, such as arginine, serine, threonine, tyrosine, cysteine, aspartic acid, asparagine, glutamic acid, glutamine, lysine, histidine, glycine, tryptophan. The hydrophilic peptide can be a repeat of a single unit or a mixed repeat of different subunits.

In the hydrophilic peptide carried by the ADC of the present disclosure, the amino acid residues as subunits can also be amino acids other than the conventional 20 natural amino acids, such as ornithine (Orn), β-alanine (β-Ala), citrulline (Cit), sarcosine (Sar), and those amino acids where R, R', R" in the repeating subunit -CO-CR'R"-NR- differ from the corresponding groups or moieties in the 20 natural amino acids. For example, R, R', R" are selected from alkyl, aryl, acyl, amido, ketone, azido, hydroxyl, mercapto, hydrazine, cyano, quaternary ammonium, halogen, hydrazide, alkenyl, alkynyl, ether, thiol, seleno, sulfonic acid, sulfate, borate, phosphono, phosphate, heterocycle, enone, imine, aldehyde, ester, thioacid, hydroxylamine, amino, etc., or any combination thereof, or groups containing alkyl, aryl, acyl, amido, ketone, azido, hydroxyl, mercapto, hydrazine, cyano, quaternary ammonium, halogen, hydrazide, alkenyl, alkynyl, ether, thiol, seleno, sulfonic acid, sulfate, borate, phosphono, phosphate, heterocycle, enone, imine, aldehyde, ester, thioacid, hydroxylamine, amino, etc. Preferred are those amino acids where R, R', and/or R" contain hydrophilic groups, such as R, R', R" each independently is carboxyl, sulfonic acid, sulfate, phosphate, amino, amido, quaternary ammonium, ether, mercapto, or hydroxyl, or is a group containing carboxyl, sulfonic acid, sulfate, phosphate, amino, amido, quaternary ammonium, ether, mercapto, and/or hydroxyl, such as alkyl or aryl, e.g., C₁₋₆ alkyl.

In some embodiments, the amino acid units of the hydrophilic peptide comprise natural amino acid repeating units, preferably selected from arginine, serine, threonine, tyrosine, cysteine, aspartic acid, asparagine, glutamic acid, glutamine, lysine, histidine, glycine, tryptophan; in other embodiments, the hydrophilic peptide comprises amino acid repeating units other than the 20 natural amino acids listed above, such as ornithine (Orn), citrulline (Cit), sarcosine (Sar); in other embodiments, the hydrophilic peptide is a mixed hydrophilic peptide comprising said natural amino acids and said non-conventional amino acids.

In some embodiments, in the subunit 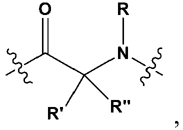 preferably R, R', R" are each independently H, -C₁₋₆ alkyl, carboxyl, sulfonic acid, sulfate, phosphate, amino, amido, quaternary ammonium, ether, mercapto, or hydroxyl, or a group containing a hydrophilic group such as carboxyl, sulfonic acid, sulfate, phosphate, amino, amido, quaternary ammonium, ether, mercapto, and/or hydroxyl, such as aryl or C₁₋₆ alkyl; more preferably R, R', R" are each independently H, -C₁₋₆ alkyl, carboxyl, sulfonic acid, phosphate, amino, amido, quaternary ammonium, mercapto, or hydroxyl, or a group containing a hydrophilic group such as carboxyl, sulfonic acid, phosphate, amino, amido, quaternary ammonium, mercapto, and/or hydroxyl, such as aryl or C₁₋₆ alkyl.

In some embodiments, the G₃ part of the antibody linker of the ADC of the present disclosure is substituted by a Bu group of formula (A), and wherein U in formula (A) is a hydrophilic peptide comprising 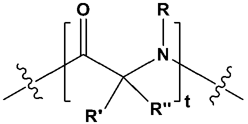 as generally or specifically defined above.

In some embodiments, the hydrophilic peptide is linked via the carboxyl terminus, and in other embodiments, the hydrophilic peptide is linked via the amino terminus.

It should be noted that the hydrophilic peptide is involved in the definition of multiple structural fragments throughout the present disclosure, and its definition herein applies to the definition given for the fragment of formula (A).

For the ADC of the present disclosure, the hydrophilic peptide carried, for example but not limited to, is 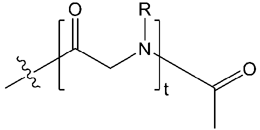 linked via the carboxyl terminus or 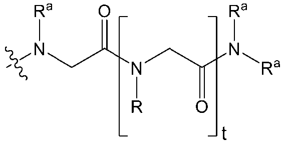 linked via the amino terminus, wherein R is as defined above, including but not limited to the amino residues of the various amino acids listed for the hydrophilic peptide herein, R^{a} is selected from H or -C₁₋₆ alkyl, preferably H or -CH₃; preferably comprising 4-14 units of polysarcosine, polyarginine, or polyglycine, more preferably comprising 6-12 units of polysarcosine.

It should be noted that each example of 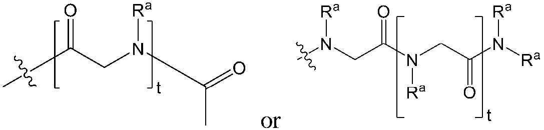 exemplified below for the hydrophilic peptide is a specific example of the hydrophilic peptide, especially 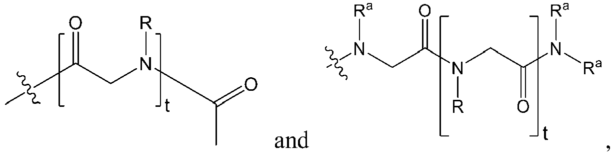 wherein when R^{a} on the N of the repeating unit is methyl, it represents the polysarcosine preferably carried by the ADC of the present disclosure.

In some embodiments, U is selected from 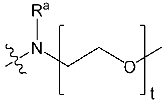 and the hydrophilic peptide linked via the amino terminus, e.g., 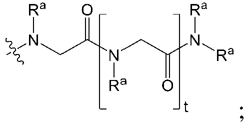 in other embodiments, U is selected from 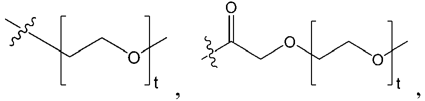 and the hydrophilic peptide linked via the carboxyl terminus, e.g., 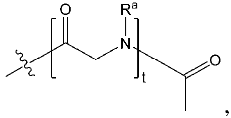 wherein R^{a} is selected from H or -C₁₋₆ alkyl, preferably H or - CH₃.

In some embodiments, T₀ is -C₁₋₄ alkylene-, preferably -C₁₋₂ alkylene-, more preferably methylene.

In some embodiments, in the 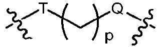 fragment, T is -O-, e.g., 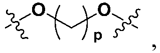 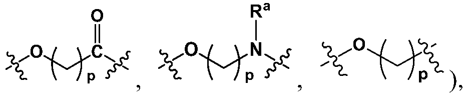 or T is -C(R^{a})₂-, e.g., 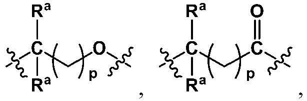 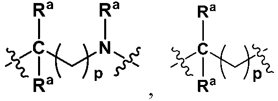 ), or T is -NR^{a}-, e.g., 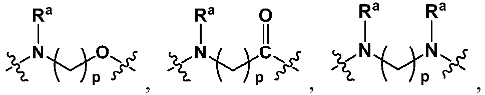 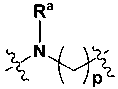 ), , or T is absent, e.g.,

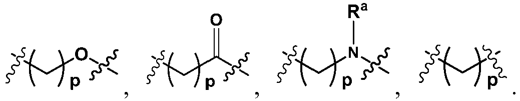

In some embodiments, in the 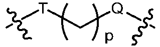 fragment, T is absent, p is 0-4, e.g., 0-2, and Q is -CO-, i.e., 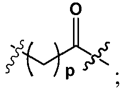 or T is -C(R^{a})₂-, p is 0-4, e.g., 0-2, and Q is -CO-, i.e., 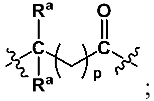 for example but not limited to 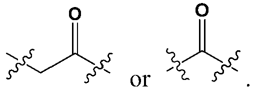 Further, in such embodiments, U is preferably selected from 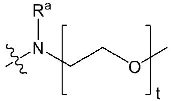 and a hydrophilic peptide such as 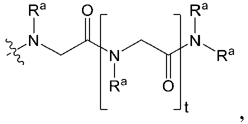 or an amino sugar or derivative thereof as defined herein linked via an amino group, preferably an amino sugar derivative of a monosaccharide or disaccharide, more preferably the amino sugar or derivative thereof shown in Table 3 or Table 4.

In some embodiments, in the 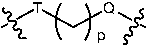 fragment, T is absent, p is 0-4, e.g., 0-2, and Q is selected from -O- or-NR^{a}-, i.e., 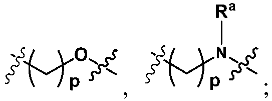 or T is -C(R^{a})₂-, p is 0-4, e.g., 0-2, and Q is selected from -O- and -NR^{a}-, i.e., 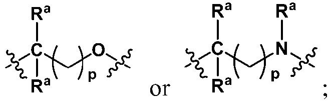 for example but not limited to 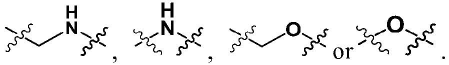 Further, in such embodiments, U is preferably selected from a sugar acid or derivative thereof as defined herein linked via a carbonyl group, preferably a sugar acid derivative of a monosaccharide or disaccharide, more preferably the sugar acid derivatives shown in Table 3 or Table 4; 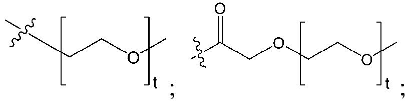 and a hydrophilic peptide such as

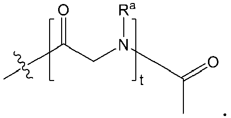

In some embodiments, Q is absent, and the 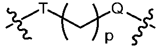 fragment is selected from 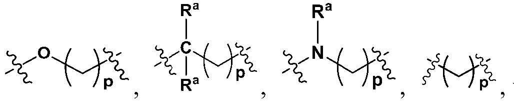 wherein p is 0-4, preferably 1-4, e.g., 1-2, such as 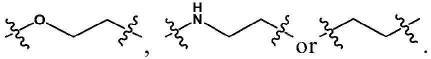 Further, in such embodiments, U can be a glycosyl or derivative thereof linked via a glycosidic bond, e.g., 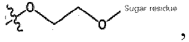 where the sugar residue is linked via ethylene glycol; or U is an amino sugar or derivative thereof linked via an amino group, e.g.,

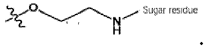

In some embodiments, t is an integer from 1~20, e.g., 2-10, 2-8, 2-6, 2-4, 4-10, 4-14, 6-10, 6-12, 8-10, 8-14.

In some embodiments, G₁ is an S atom; in other embodiments, G₁ is an N atom; in other embodiments, G₁ is a C atom. In the ADC of the present disclosure, preferred G₁ is a sulfur atom of Ab.

In some embodiments, G₂ is a 5-10 membered heterocyclyl group, preferably a 5-6 membered heterocyclyl group containing 1 or 2 heteroatoms selected from N, O, and S, wherein ring carbon atoms are optionally oxidized, for example but not limited to 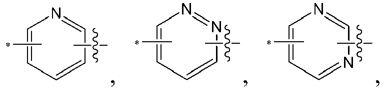 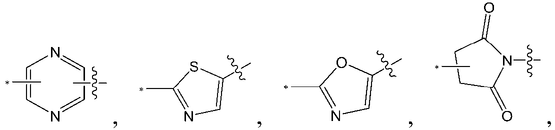 wherein the left * indicates the point of attachment to G₁, and the right wavy line indicates the point of attachment to G₃.

In some embodiments, G₂ is selected from 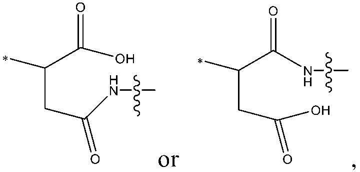 which are the acid-amide moieties formed after partial hydrolysis of the succinimide moiety.

In a preferred embodiment, G₂ is a maleimidyl group 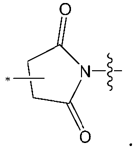 In another preferred embodiment, G₂ is

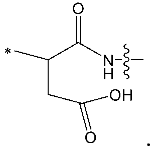

In some embodiments, G₂ is selected from 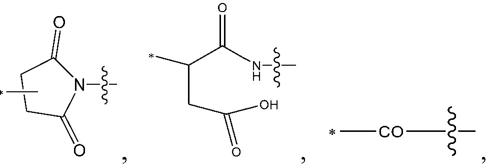 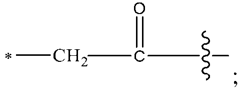 preferably selected from

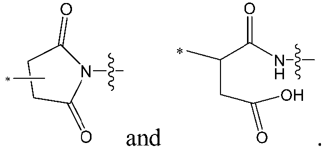

In some embodiments, G₃ is selected from -C₁-₁₀ alkylene-C(=O)-, -C₃-₁₀ alkynylene-C(=O)-, - C₃-₁₀ alkenylene-C(=O)-, -C₁-₁₀ heteroalkylene-C(=O)-, -C₃-₈ cycloalkylene-C(=O)-, -O-C₁-₁₀ alkylene-C(=O)-, -C₆-₁₀ arylene-C(=O)-, -C₁-₁₀ alkylene-C₆-₁₀ arylene-C(=O)-, -C₆-₁₀ arylene-C₁₋₁₀ alkylene-C(=O)-, -C₁-₁₀ alkylene-C₃-₈ cycloalkylene-C(=O)-, -C₃-₈ cycloalkylene-C₁-₁₀ alkylene-C(=O)-, -C₃₋₈ heterocyclylene-C(=O)-, -C₁-₁₀ alkylene-C₃-₈ heterocyclylene-C(=O)-, -C₃-₈ heterocyclylene-C₁-₁₀ alkylene-C(=O)-, -C₁-₁₀ alkylene-NH-, -C₁-₁₀ heteroalkylene-NH-, -C₃-₈ cycloalkylene-NH-, -O-C₁-₁₀ alkylene-NH-, -C₆-₁₀ arylene-NH-, -C₁-₁₀ alkylene-C₆-₁₀ arylene-NH-, -C₆-₁₀ arylene-C₁₋₁₀ alkylene-NH-, -C₁-₁₀ alkylene-C₃-₈ cycloalkylene-NH-, -C₃-₈ cycloalkylene-C₁-₁₀ alkylene-NH-, -C₃₋₈ heterocyclylene-NH-, -C₁-₁₀ alkylene-C₃-₈ heterocyclylene-NH-, and -C₃-₈ heterocyclylene-C₁-₁₀ alkylene-NH-; preferably selected from -C₁-₁₀ alkylene-C(=O)-, -C₃-₁₀ alkynylene-C(=O)-, -C₃-₁₀ alkenylene-C(=O)-, -C₁-₁₀ heteroalkylene-C(=O)-, -C₁-₁₀ alkylene-C₆-₁₀ arylene-C(=O)-, -C₆-₁₀ arylene-C₁₋₁₀ alkylene-C(=O)-, -C₁-₁₀ alkylene-C₃-₈ cycloalkylene-C(=O)-, - C₃-₈ cycloalkylene-C₁-₁₀ alkylene-C(=O)-, -C₁-₁₀ alkylene-C₃-₈ heterocyclylene-C(=O)-, -C₃-₈ heterocyclylene-C₁-₁₀ alkylene-C(=O)-; more preferably -C₁-₁₀ alkylene-C(=O)-; wherein each group attached to -C(=O)- or NH in G₃ is optionally substituted by Bu as defined above for G₃, and G₃ is connected via its -C(=O)- or NH to the linker unit E (when present), or the property-modulating unit D (when present and E is absent), or the cleavable linker B (when both E and D are absent), and the other end group is connected to G₂.

In some embodiments, G₃ is -C₁-₁₀ alkylene-C(=O)-, preferably -C₁-₅ alkylene-C(=O)-, wherein the alkylene moiety is optionally substituted by the Bu moiety described above.

In some embodiments, G₃ is -C₁-₁₀ alkylene-NH, preferably -C₁-₅ alkylene-NH-, wherein the alkylene moiety is optionally substituted by the Bu moiety described above.

In exemplary embodiments, -G₂-G₃- can be the following groups: 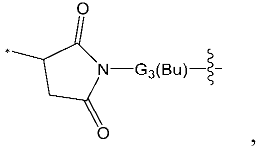 e.g., 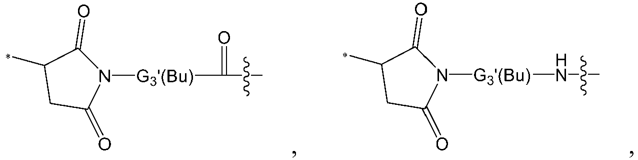 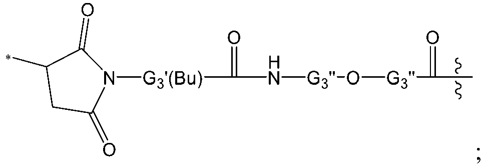 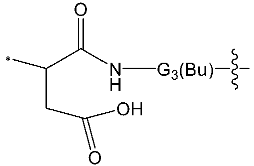 e.g., 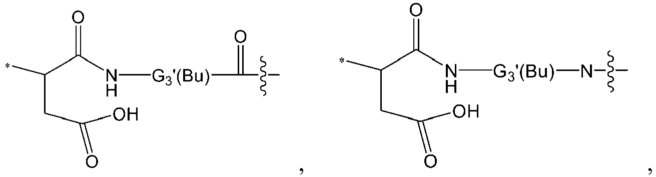 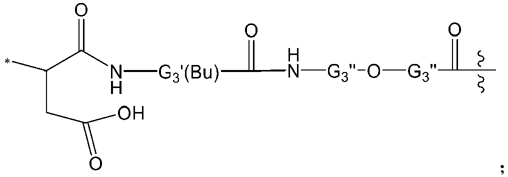 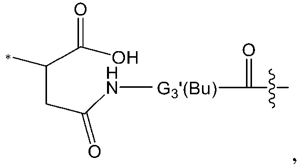 wherein G₃ and Bu are each as generally or preferably defined above;
G₃' and G₃" are each independently selected from -C₁-₁₀ alkylene, -C₃-₁₀ alkynylene, -C₃-₁₀ alkenylene, -C₁-₁₀ heteroalkylene, -C₃-₈ cycloalkylene, -C₆-₁₀ arylene, -C₁-₁₀ alkylene-C₆-₁₀ arylene, -C₆-₁₀ arylene-C₁₋₁₀ alkylene, -C₁-₁₀ alkylene-C₃-₈ cycloalkylene, -C₃-₈ cycloalkylene-C₁-₁₀ alkylene, -C₃₋₈ heterocyclylene, -C₁-₁₀ alkylene-C₃-₈ heterocyclylene, -C₃-₈ heterocyclylene-C₁-₁₀ alkylene;
Preferably, G₃' and G₃" are each independently selected from -C₁-₁₀ alkylene, -C₃-₁₀ alkynylene, -C₃-₁₀ alkenylene, -C₁-₁₀ heteroalkylene, -C₁-₁₀ alkylene-C₆-₁₀ arylene, -C₆-₁₀ arylene-C₁₋₁₀ alkylene, -C₁-₁₀ alkylene-C₃-₈ cycloalkylene, -C₃-₈ cycloalkylene-C₁-₁₀ alkylene, -C₁-₁₀ alkylene-C₃-₈ heterocyclylene, -C₃-₈ heterocyclylene-C₁-₁₀ alkylene;
More preferably, G₃' is -C₁-₁₀ alkylene-, preferably -C₁-₅ alkylene-; G₃" is -C₁-₅ alkylene-, preferably -C₁₋₂ alkylene-;
wherein G₃' and G₃" are optionally substituted by the Bu moiety as generally or preferably defined above;
wherein the left * indicates the point of attachment to G₁, and the right 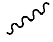 indicates the point of attachment to E (when present), or the property-modulating unit D (when present and E is absent), or the cleavable linker B (when both E and D are absent).

In some embodiments, -G₂-G₃- is 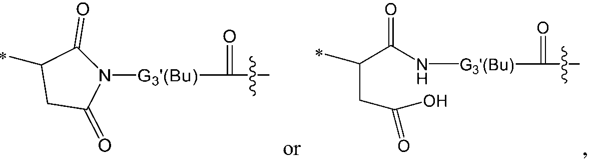 wherein G₃' is -C₁-₁₀ alkylene-, preferably -C₁-₅ alkylene-, Bu is absent or is a structural fragment of formula (A).

In specific embodiments, -G₂-G₃- can be 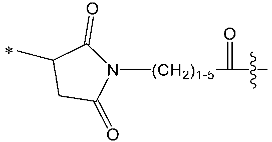 or 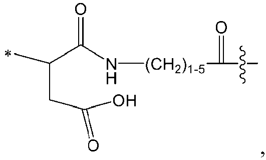 e.g., 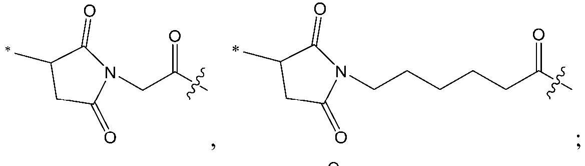 or -G₂-G₃- can be 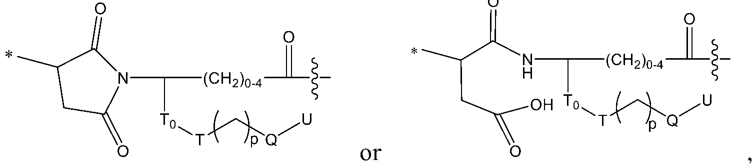 e.g., 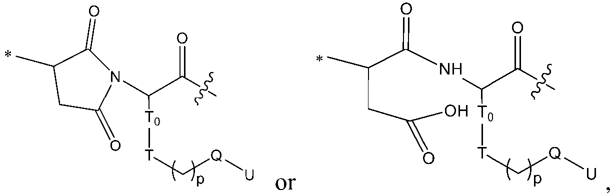 wherein T₀, T, Q, U, p, and the fragment of formula (A) are respectively as generally or specifically defined above for formula (A).

In further specific embodiments, -G₂-G₃- is 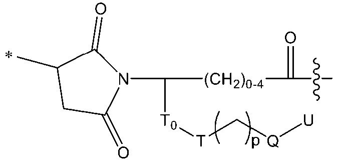 or 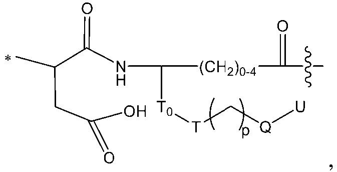 e.g., 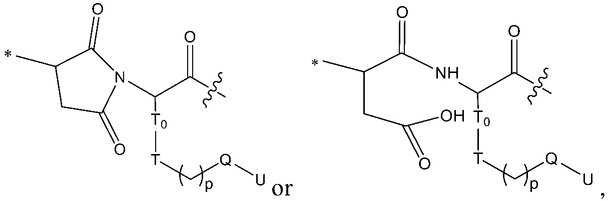 wherein T₀ is -C₁₋₄ alkylene- (e.g., -C₁₋₂ alkylene-, such as methylene), T is absent and p is 0-4 (e.g., 0-2) and Q is selected from -O- or -NR^{a}-, or T₀ is -C₁₋₄ alkylene- (e.g., -C₁₋₂ alkylene-, such as methylene), T is -C(R^{a})₂-, p is 0-4 (e.g., 0-2) and Q is -NR^{a}- or -O-, and U is preferably selected from a sugar acid (preferably a sugar acid of a monosaccharide or disaccharide) or derivative thereof linked via a carbonyl group, 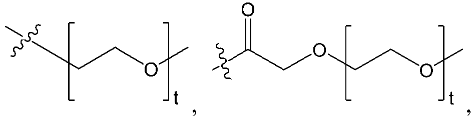 a hydrophilic peptide such as 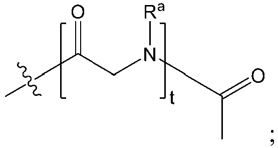 or
wherein T₀ is -C₁₋₄ alkylene- (e.g., -C₁₋₂ alkylene-, such as methylene), T is absent and p is 0-4 (e.g., 0-2) and Q is -CO-, or T₀ is -C₁₋₄ alkylene- (e.g., -C₁₋₂ alkylene-, such as methylene), T is - C(R^{a})₂-, p is 0-4 (e.g., 0-2) and Q is -CO-, and U is preferably selected from an amino sugar (preferably an amino monosaccharide or amino disaccharide) or derivative thereof linked via an amino group, 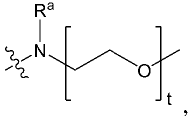 or a hydrophilic peptide such as 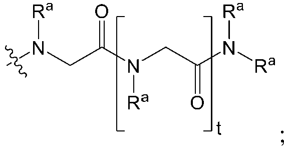 or
wherein T₀ is -C₁₋₄ alkylene- (e.g., -C₁₋₂ alkylene-, such as methylene), T is selected from - C(R^{a})₂-, -O-, -NR^{a} -, or is absent, Q is absent, p is 0-4 (e.g., 0-2, 1-2), and U is preferably selected from a glycosyl (preferably a monosaccharide or disaccharide) or derivative thereof linked via a glycosidic bond, or an amino sugar (preferably an amino monosaccharide or amino disaccharide) or derivative thereof linked via an amino group;
wherein t is an integer from 1 to 20, e.g., 2-10, 2-8, 2-6, 2-4, 4-10, 4-14, 6-10, 6-12, 8-10, 8-14.

In some embodiments, -G₁-G₂-G₃- is selected from the following groups: 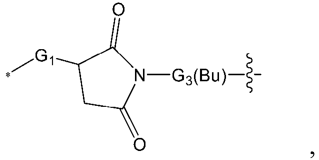 e.g., 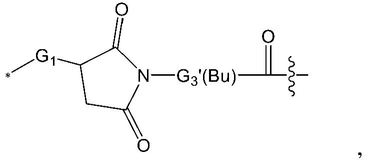 specifically 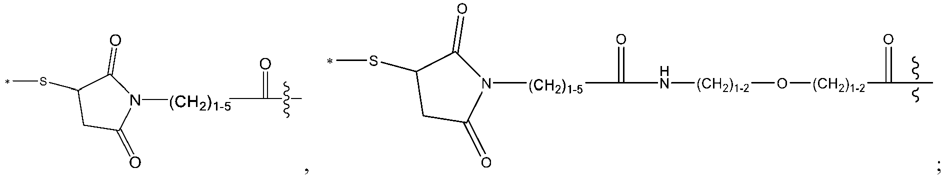 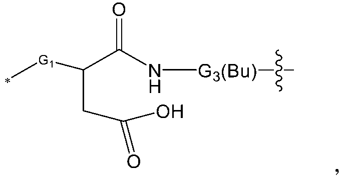 e.g., 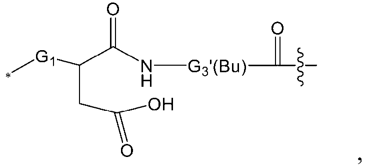 specifically 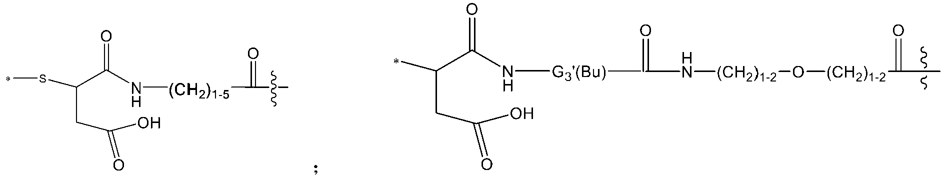 and 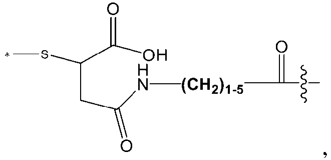 wherein G₃, G₃', and Bu are respectively as generally or preferably defined above; the left * indicates the point of attachment to the remainder of the antibody, and the right 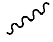 indicates the point of attachment to the linker unit E (when present), or the property-modulating unit D (when present and E is absent), or the cleavable linker B (when both E and D are absent).

In some embodiments, -G₁-G₂-G₃- is 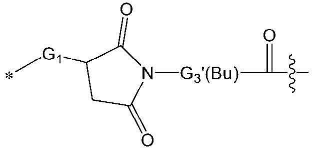 or 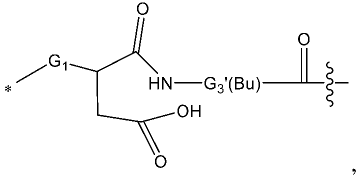 wherein G₃' is -C₁-₁₀ alkylene-, preferably -C₁-₅ alkylene-, Bu is absent or is a structural fragment of formula (A).

In specific embodiments, -G₁-G₂-G₃- can be 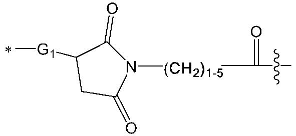 or 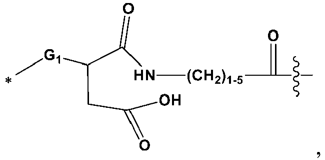 e.g., 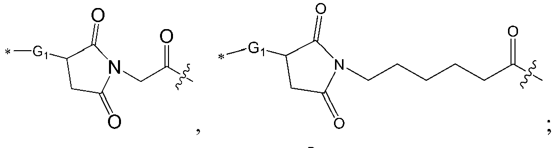 or - G₁-G₂-G₃- can be 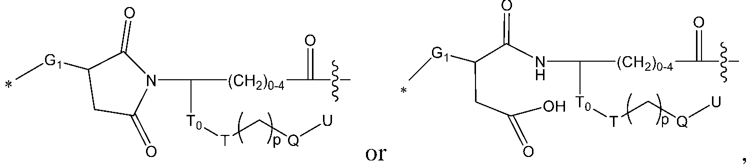 e.g., 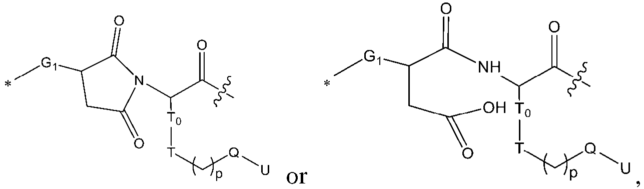 wherein T₀, T, Q, U, p, and the fragment of formula (A) are respectively as generally or specifically defined above for formula (A).

In further specific embodiments, -G₁-G₂-G₃- is 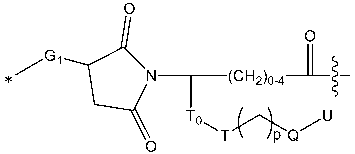 or 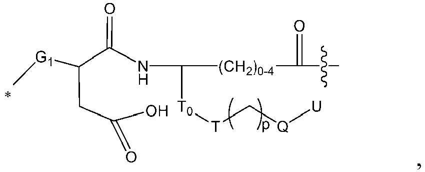 e.g., 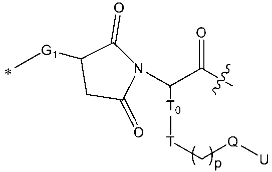 or 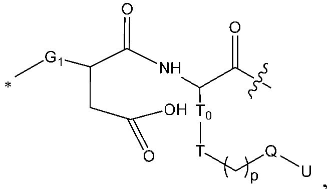 wherein T₀ is -C₁₋₄ alkylene- (e.g., -C₁₋₂ alkylene-, such as methylene), T is absent and p is 0-4 (e.g., 0-2) and Q is selected from -O- or -NR^{a}-, or T₀ is -C₁₋₄ alkylene- (e.g., -C₁₋₂ alkylene-, such as methylene), T is -C(R')₂-, p is 0-4 (e.g., 0-2) and Q is -NR^{a}-or -O-, and U is preferably selected from a sugar acid (preferably a sugar acid of a monosaccharide or disaccharide) or derivative thereof linked via a carbonyl group, 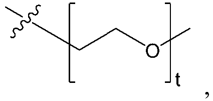 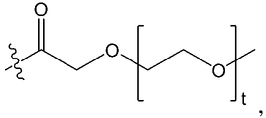 a hydrophilic peptide such as 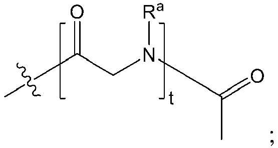 or
wherein T₀ is -C₁₋₄ alkylene- (e.g., -C₁₋₂ alkylene-, such as methylene), T is absent and p is 1-4 (e.g., 1-2) and Q is -CO-, or T₀ is -C₁₋₄ alkylene- (e.g., -C₁₋₂ alkylene-, such as methylene), T is - C(R^{a})₂-, p is 0-4 (e.g., 0-2) and Q is -CO-, and U is preferably selected from an amino sugar (preferably an amino monosaccharide or amino disaccharide) or derivative thereof linked via an amino group, 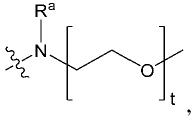 or a hydrophilic peptide such as 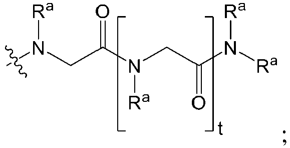 or
wherein T₀ is -C₁₋₄ alkylene- (e.g., -C₁₋₂ alkylene-, such as methylene), T is selected from - C(R^{a})₂-, -O-, -NR^{a}-,, or is absent, Q is absent, p is 0-4 (e.g., 0-2, 1-2), and U is preferably selected from a glycosyl (preferably a monosaccharide or disaccharide) or derivative thereof linked via a glycosidic bond, or an amino sugar (preferably an amino monosaccharide or amino disaccharide) or derivative thereof linked via an amino group;
wherein t is an integer from 1~20, e.g., 2-10, 2-8, 2-6, 2-4, 4-10, 4-14, 6-10, 6-12, 8-10, 8-14.

In exemplary embodiments, G (-G₁-G₂-G₃-) in formula (II) or (II') of the present disclosure has the following structures:

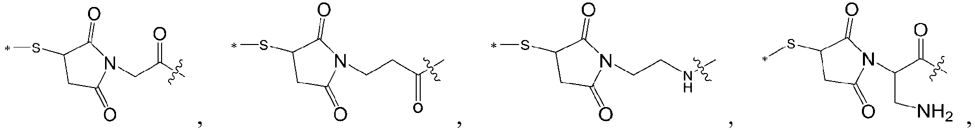

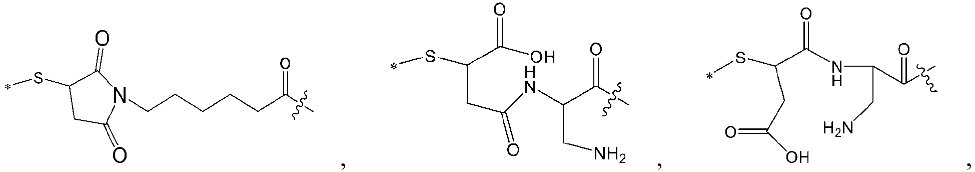

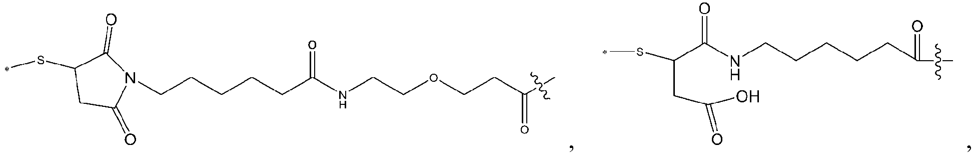

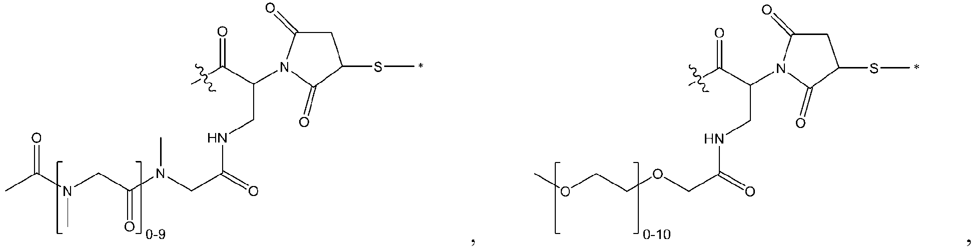

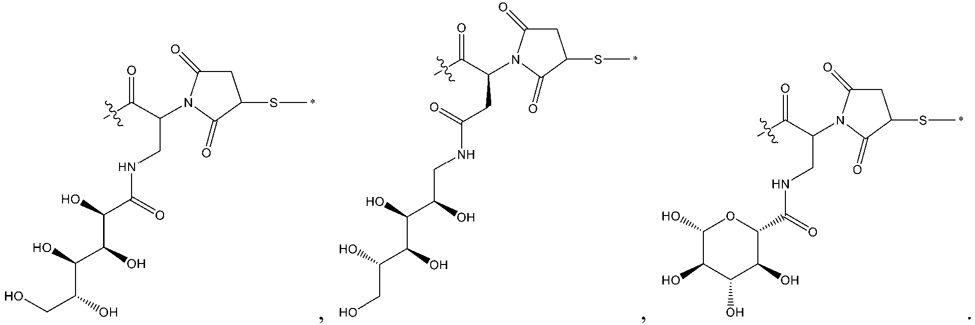

In some preferred embodiments, G in formula (II) or (II') of the present disclosure has the following structures: 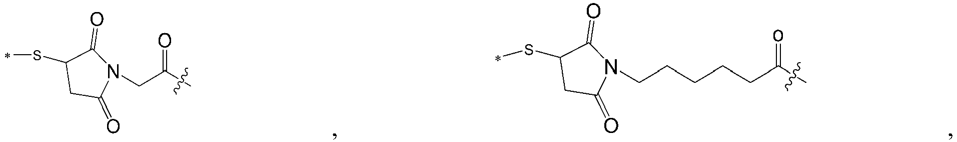 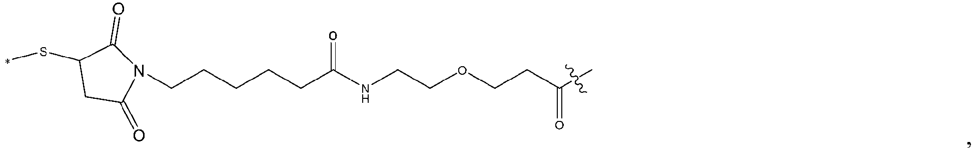 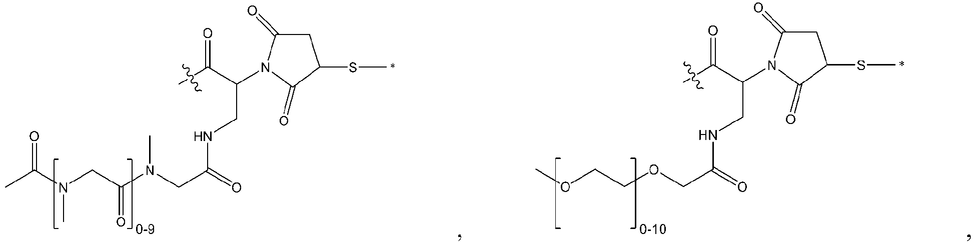 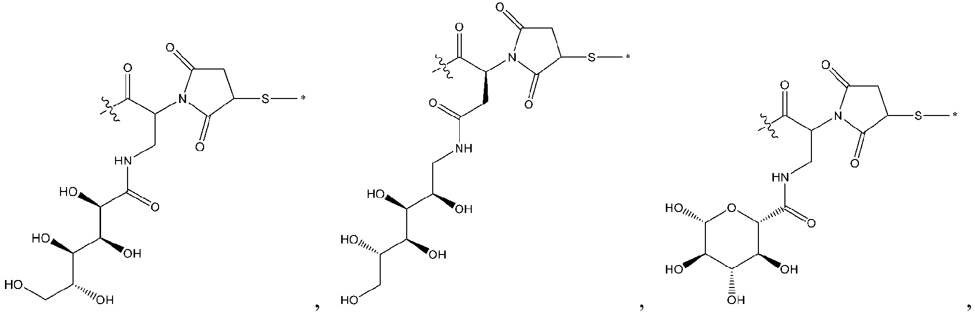 and the respective fragments above where the maleimide moiety is 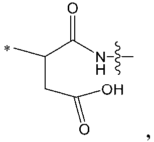 e.g., 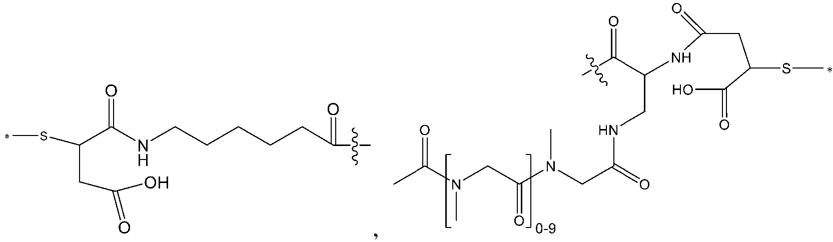 etc.

### E-Linker Unit

The linker unit E in the linker unit L of formula (II) or (II') of the ADC of the present disclosure connects the antibody linker G to the property-modulating unit D, or when D is absent, connects the antibody linker G to the cleavable linker B, to add extra distance between the antibody linker and the cleavable linker B, potentially aiding in the activation of B.

In some embodiments, the linker unit E is selected from a direct bond, -C₁-₅ alkylene-, -NH-, - NH-C₁₋₅ alkylene-heteroaryl (such as a 5- or 6-membered nitrogen-containing heteroaryl, e.g., triazolyl), and the following groups: 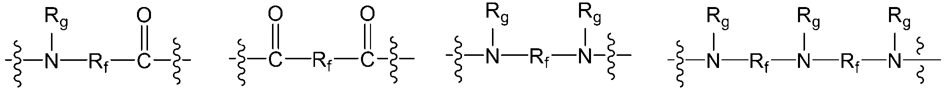 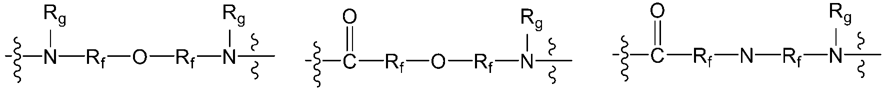
wherein when present, E is connected via its -C(=O)- or -NH- end to the property-modulating unit D (when present) or the cleavable linker B as appropriate, and the other end is connected via - C(=O)- or -NH- to G₃ as appropriate;
R_{g} is each independently selected from H and -C₁₋₆ alkyl, preferably H or -C₁₋₃ alkyl;
R_{f} is selected from -C₁₋₆ alkylene-, -arylene-, -C₁₋₁₀ heteroalkylene-, -C₃₋₈ heterocyclylene-, -C₁₋₁₀ alkylene-C₆₋₁₀ arylene-, -C₆₋₁₀ arylene-C₁₋₁₀ alkylene-, -C₁₋₁₀ alkylene-C₃₋₈ cycloalkylene-, -C₃₋₈ cycloalkylene-C₁₋₁₀ alkylene-, -C₁₋₁₀ alkylene-C₃₋₈ heterocyclylene-, -C₃₋₈ heterocyclylene-C₁-₁₀ alkylene-;
It should be noted that, when present, each specific definition of E above can be repeated consecutively 1-10 times, preferably 1-4 times, in different head-to-tail orientations, such as 1, 2, 3, and 4 times.

In a specific embodiment, the linker unit E is 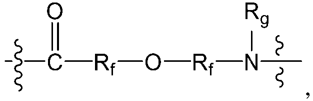 wherein R_{f} is each independently selected from -C₁₋₆ alkylene-, preferably -C₂₋₄ alkylene, R_{g} is H or C₁₋₃ alkyl, e.g., 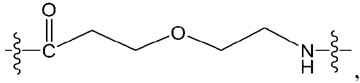 wherein the left wavy line is connected to the cleavable linker B, and the right wavy line is connected to the antibody linker G.

In another specific embodiment, the linker unit E is a direct bond; in another specific embodiment, the linker unit E is -C₁₋₄ alkylene-; in another specific embodiment, the linker unit E is -NH-.

### D and D¹-Property-Modulating Unit

The addition of the property-modulating unit D or D¹ in the linker unit L of formula (II) or (II') of the ADC of the present disclosure may be beneficial for improving the properties of the ADC, such as stability in the bloodstream, improving hydrophilicity, leading to reduced clearance and increased exposure. However, increasing the number of D or D¹ units can also lead to increased molecular weight and hydrodynamic radius of the ADC, which can reduce diffusion, and reduced diffusivity may decrease the ability of the ADC to penetrate tumors. Due to these two competing pharmacokinetic effects, a moderate amount of D or D¹ is needed to reduce the clearance of the ADC, thereby increasing plasma exposure, but not so large as to reduce its diffusivity to the extent that it interferes with the ability of the ADC to reach the intended target cell population.

Property-modulating unit D is present in the linker unit in a tandem manner, and D¹ is present in a branched manner. D can be directly connected between B and E, or can be connected between B and E via an additional linking group, such as amino, carbonyl, alkylcarbonyl, amide, ester, urea, disulfide bridge, carbamate, hydrazone, imine, oxime, triazolyl, maleimidyl, alkenyl, alkynyl, or alkylene. For example, D can be connected to the cleavable linker B via -NH-, -C(=O)-, -C₁₋₄ alkylene, -C₁₋₄ alkylene-C(=O)-, -NH-C(=O)-(CH₂OCH₂)-C(=O)-, -C₁₋₄ alkylene-NH-C(=O)-(CH₂OCH₂)-C(=O)-.

In some embodiments, D or D¹ is absent.

In some embodiments, D or D¹ is selected from polyamines, for example but not limited to polyethyleneimine, polylysine, spermine, dimeric polyamines, arginine, amidine, protamine, cationic lipids, cationic porphyrins, quaternary ammonium salts of polyamines, α-helical peptides.

In some embodiments, D or D¹ is selected from peptides, preferably hydrophilic peptides.

In some embodiments, D or D¹is a cyclodextrin unit.

In some embodiments, D or D¹ is a polyamide.

In some embodiments, D or D¹ is a polysaccharide, dendrimer, or bifunctional hydrocarbon chain.

In some embodiments, D or D¹ is PEG.

In the embodiments of the present disclosure, polydisperse PEG (non-uniform mixture of sizes and molecular weights), monodisperse PEG (single chain length and molecular weight), and discrete PEG can be used as part of the property-modulating unit in the ADC of the present disclosure. Preferred PEG is discrete PEG, which are compounds synthesized in a stepwise manner rather than via a polymerization process, providing a single molecule with a defined chain length.

The ADC of the present disclosure can contain one or more PEG chains consisting of at least two ethylene oxide (CH₂CH₂O) subunits. PEG chains can be linked together, for example, in a linear, branched, or star configuration. Typically, at least one PEG chain is functionalized so that it can be covalently linked to other components in the linker unit, with functionalization methods including, for example, via amine, thiol, NHS ester, maleimide, alkyne, azide, carbonyl, or other functional groups. For example, one end of a PEG chain can be functionalized to covalently attach to an appropriate site on the linker unit, and the other end (or multiple ends) can be free and unbound, i.e., not attached to other components in the ADC, and can take the form of methoxy, carboxylic acid, alcohol, or other suitable functional groups; alternatively, the PEG chain can be covalently linked in a tandem manner between two components of the linker unit, for example, between the linker unit E and the cleavable linker B. The linkage of the PEG chain to the components within the linker unit can be via cleavable bonds, which can be bonds that are substantially insensitive to cleavage during circulation in plasma but sensitive to cleavage in intracellular or intratumoral environments. Exemplary linkages include, but are not limited to, amide bonds, ether bonds, ester bonds, hydrazone bonds, oxime bonds, disulfide bonds, peptide bonds, or triazole bonds, etc.

Methods for attaching PEG units to the linker unit of ADCs are well known to those skilled in the art. For example, PEG can be covalently bound to amino acid residues via reactive groups. Reactive groups are those to which an activated PEG molecule can bind, such as free amino or carboxyl groups, e.g., thiol groups on cysteine residues can also be used as reactive groups for linking PEG. In some embodiments, methoxylated PEG ("mPEG") with different reactive moieties can also be used to link PEG to amino groups. Non-limiting examples of such reactive moieties include succinimidyl succinate (SS), succinimidyl carbonate (SC), mPEG-imidate, p-nitrophenyl carbonate (NPC), succinimidyl propionate (SPA), and cyanuric chloride. Correspondingly, non-limiting examples of such mPEG include mPEG-succinimidyl succinate (mPEG-SS), mPEG-succinimidyl carbonate (mPEG-SC), mPEG-imidate, mPEG-p-nitrophenyl carbonate (mPEG-NPC), mPEG-succinimidyl propionate (mPEG-SPA), mPEG-N-hydroxy-succinimide (mPEG-NHS), mPEG-cyanuric chloride, mPEG₂-lysinol-NPC, and mPEG2-Lys-NHS.

In some embodiments, the PEG unit comprises one or more linear PEG chains, each PEG chain having 2-12 ethylene oxide (CH₂CH₂O) subunits, for example at least 2, at least 3, at least 4, at least 5, at least 6, at least 7, at least 8, at least 9, at least 10, at least 11, at least 12 ethylene oxide (CH₂CH₂O) subunits. In preferred embodiments, the PEG unit comprises at least 2, at least 4, at least 6, at least 8, at least 10, or at least 12 ethylene oxide (CH₂CH₂O) subunits, for example, the PEG unit comprises 2 to 12, 2 to 10, 2 to 8, 2 to 6, 2 to 4, 4 to 12, 4 to 10, 4 to 8, 4 to 6, 6 to 12, 6 to 10, 6 to 8, 8 to 12, or 8 to 10 subunits.

Exemplary embodiments of the PEG moiety D linking the cleavable linker B and the linker unit E include, but are not limited to, the following:

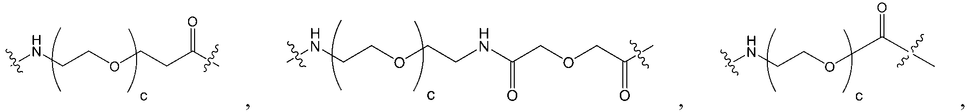

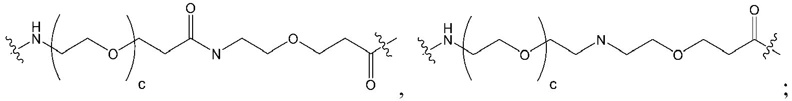

Exemplary embodiments of linear PEG units (i.e., one end linked to the linker unit, the other end being a cap) include: 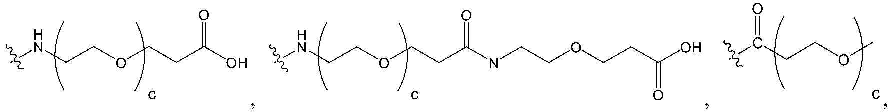 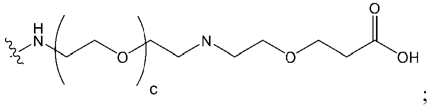 wherein the left wavy line indicates the point of attachment to E or G₃ (or to E or B in the case of branched PEG as appropriate), the right wavy line indicates the point of attachment to B, and each subscript c is independently selected from integers 2-12. In some embodiments, c is 2, 4, 8, or 12. In some embodiments, c is 2. In some embodiments, c is 4. In some embodiments, c is 8. In some embodiments, c is 12.

### B-Cleavable Linker

In one aspect, the cleavable linker B in the linker unit L of formula (II) or (II') of the ADC of the present disclosure is an enzyme-cleavable unit, i.e., a substrate that can be cleaved or broken by an enzyme. To limit or minimize the non-targeted toxicity of the ADC while ensuring the release of the toxin molecule at the target tumor site, the cleavable linker is designed to be a substrate specifically cleaved by enzymes present in the vicinity or inside the target cell, preferably where the level or activity of said enzyme in the vicinity or inside the target cell is higher than that in other parts of the body, thereby ensuring that the ADC is specifically recognized, cleaved, and releases the KRas mutant inhibitor in the target cell/tissue, exerting its biological function.

Therefore, the ADCs of the present disclosure are stable in biological fluids until they reach their target, e.g., tumor cells/tissues. Due to the specific release of the ADC, administration of the ADC of the present disclosure will result in less toxicity compared to administration of the KRas mutant inhibitor alone, and the activity of the KRas mutant inhibitor is maximized.

In some embodiments, B in the linker unit L of formula (II) or (II') of the ADC of the present disclosure is a dipeptide, tripeptide, or oligopeptide consisting of an amino acid sequence specifically recognized and cleaved by a protease, preferably specifically recognized and cleaved by a nonmammalian protease or an endogenous mammalian protease present in or near the target cell. Such enzymes include, for example but not limited to: β-site APP-cleaving enzyme 1 (BACE1), cathepsin D (CTSD), calpain-1 (CAPN1), legumain, caspase 1 (Casp1), caspase 2 (Casp2), caspase 3 (Casp3), caspase 5 (CASPS), caspase 6 (Casp6), caspase 7 (Casp7), caspase 8 (Casp8), caspase 9 (Casp9), cathepsin B (CTSB), cathepsin K (CTSK), cathepsin L (CTSL), cathepsin S (CTSS), esterases (e.g., cholinesterase, alkaline phosphatase, phosphodiesterase, sulfatase), amidase angiotensin I converting enzyme (ACE), angiotensin I converting enzyme 2 (ACE2), ADAM metallopeptidase domain 10 (ADAM10), dipeptidyl peptidase 3 (DPP3), insulin-degrading enzyme (IDE), matrix metalloproteinase 1 (MMP1), matrix metalloproteinase 12 (MMP12), matrix metalloproteinase 13 (MMP13), matrix metalloproteinase 14 (membrane-inserted) (MMP14), matrix metalloproteinase 2 (MMP2), matrix metalloproteinase 3 (MMP3), matrix metalloproteinase 7 (MMP7), matrix metalloproteinase 8 (MMP8), matrix metalloproteinase 9 (MMP9), membrane metallo-endopeptidase (NEPRILYSIN) (MME), ADAM metallopeptidase domain 17 (TACE) (ADAM17), dipeptidyl peptidase 4 (DPP4), dipeptidyl peptidase 8 (DPP8), dipeptidyl peptidase 9 (DPP9), coagulation factor Xa (factor Xa), coagulation factor VIIA (factor VII), fibroblast activation protein α (FAP), furin (paired basic amino acid cleaving enzyme), granzyme A (granzyme 1, cytotoxic T lymphocyte-associated (GZMA), serine esterase 3), granzyme B (granzyme 2, cytotoxic T lymphocyte-associated (GZMB), serine esterase 1), granzyme K (granzyme 3, trypsin II) (GZMK), kallikrein-1 (KLK1), kallikrein-2 (KLK2), plasma kallikrein (PSA, KLK3), kallikrein-11 (KLK11), kallikrein-13 (KLK13), kallikrein-15 (KLK15), matriptase (ST14), spinesin (TMPRSSS), plasmin (PLG), prolyl oligopeptidase (PREP), thrombin (F2), tPA, tissue-type plasminogen activator (PLAT), UPA, urokinase-type plasminogen activator (PLAU), HtrA serine peptidase 2 (hTRA2), caseinolytic mitochondrial matrix peptidase proteolytic subunit (CIPP/X), constitutive proteasome chymotrypsin-like form (PSMBS, β5), constitutive proteasome trypsin-like form (PSMB7, β7), constitutive proteasome caspase-like form (PSMB6, β6), immunoproteasome chymotrypsin-like form (PSMB8, LMP7), immunoproteasome trypsin-like form (PSMB₁₀, MECL1), immunoproteasome caspase-like form (PSMB9, LMP2).

In some embodiments, B in the linker unit L of formula (II) or (II') of the ADC of the present disclosure is specifically recognized and cleaved by cathepsin B, for example Cit-Val, Ala-Val, Gly-Phe-Gly-Gly, Gly-Phe-Leu-Gly.

In some embodiments, B in the linker unit L of formula (II) or (II') of the ADC of the present disclosure is specifically recognized and cleaved by legumain, for example Ala-Ala, Asn-Ala-Ala.

In some embodiments, B in the linker unit L of formula (II) or (II') of the ADC of the present disclosure is specifically recognized and cleaved by, for example, matrix metalloproteinases 2/9 (MMP-2/9) present at high concentrations in tumors, such as Pro-Leu-Gly-Leu-Ala-Gly (PLGLAG), Gly-Pro-Leu-Gly-Ile-Ala-Gly-Gln (GPLGIAGQ), Gly-Pro-Val-Gly-Leu-Ile-Gly-Lys (GPVGLIGK).

In some embodiments, B as part of the linker unit L of formula (II) or (II') of the ADC of the present disclosure is a cleavable peptide containing two or more (e.g., 2-12) consecutive or nonconsecutive amino acids, for example, can comprise or consist of a dipeptide, tripeptide, tetrapeptide, pentapeptide, hexapeptide, heptapeptide, octapeptide, nonapeptide, decapeptide, undecapeptide, or dodecapeptide unit. Each amino acid of the peptide-containing unit can be independently selected from natural or unnatural amino acids and/or can be the D- or L-isomer, provided that B contains a cleavable bond that, when cleaved, will trigger the release of the KRas mutant inhibitor. In certain embodiments, B consists only of natural amino acids. In other embodiments, B consists only of unnatural amino acids. In some embodiments, B consists of natural amino acids attached to unnatural amino acids. In some embodiments, B consists of natural amino acids attached to the D-isomer of a natural amino acid.

In some embodiments, the amino acids contained in the cleavable peptide in B of the linker unit L of formula (II) or (II') of the ADC of the present disclosure are, for example, selected from: alanine (Ala), arginine (Arg), aspartic acid (Asp), asparagine (Asn), histidine (His), glycine (Gly), glutamic acid (Glu), glutamine (Gln), phenylalanine (Phe), lysine (Lys), substituted lysine, leucine (Leu), serine (Ser), tyrosine (Tyr), threonine (Thr), isoleucine (Ile), proline (Pro), tryptophan (Trp), valine (Val), cysteine (Cys), methionine (Met), selenocysteine, ornithine (Orn), β-alanine (β-Ala), citrulline (Cit), and derivatives thereof. In some embodiments, each amino acid is independently selected from alanine, arginine, aspartic acid, asparagine, histidine, glycine, glutamic acid, glutamine, phenylalanine, lysine, leucine, serine, tyrosine, threonine, isoleucine, proline, tryptophan, valine, cysteine, methionine, and derivatives thereof. In some embodiments, each amino acid is independently selected from alanine, arginine, aspartic acid, asparagine, histidine, glycine, glutamic acid, glutamine, phenylalanine, lysine, leucine, serine, tyrosine, threonine, isoleucine, proline, tryptophan, valine, N-methylglycine, β-alanine, and derivatives thereof.

In some embodiments, each amino acid is independently selected from the following L-(natural) amino acids: alanine, arginine, aspartic acid, asparagine, histidine, glycine, glutamic acid, glutamine, phenylalanine, lysine, leucine, serine, tyrosine, threonine, isoleucine, tryptophan, and valine. In another embodiment, each amino acid is independently selected from the D-isomers of the following natural amino acids: alanine, arginine, aspartic acid, asparagine, histidine, glycine, glutamic acid, glutamine, phenylalanine, lysine, leucine, serine, tyrosine, threonine, isoleucine, tryptophan, and valine.

In some embodiments, B or the cleavable peptide contained in B has the structure of formula: 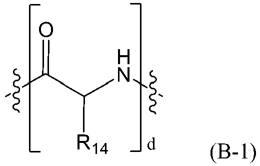
wherein the N-terminus is connected to D (when present), E (when present), or the G part of the linker unit, and the C-terminus is connected to the A or J part of the linker unit or directly to the drug P;
d is an integer from 2 to 12;
R₁₄ in each occurrence is independently selected from hydrogen, methyl, isopropyl, isobutyl, sec-butyl, benzyl, p-hydroxybenzyl, -CH₂OH, -CH(OH)CH₃, -CH₂CH₂SCH₃, -CH₂CONH₂, - CH₂COOH, -CH₂CH₂CONH₂, -CH₂CH₂COOH, -(CH₂)₃NHC( = NH)NH₂, -(CH₂)₃NH₂, - (CH₂)₃NHCOCH₃, -(CH₂)₃NHCHO, -(CH₂)₄NHC(=NH)NH₂, -(CH₂)₄NH₂, -(CH₂)₄NHCOCH₃, - (CH₂)₄NHCHO, -(CH₂)₃NHCONH₂, -(CH₂)₄NHCONH₂, -CH₂CH₂CH(OH)CH₂NH₂, 2-pyridylmethyl-, 3-pyridylmethyl-, 4-pyridylmethyl-, phenyl, cyclohexyl, 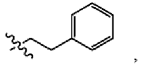 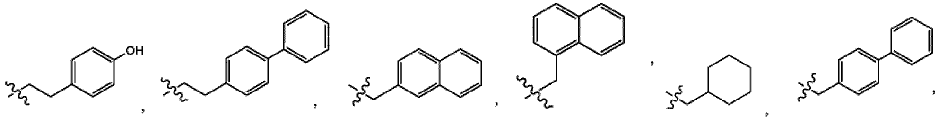 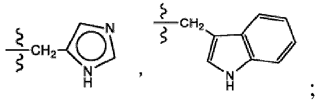 or R₁₄ is a spiro ring 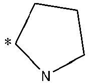 attached via the * position.

In some embodiments, each R₁₄ is independently hydrogen, methyl, isopropyl, isobutyl, sec-butyl, benzyl, -CH₂CONH₂, -CH₂CH₂COOH, or -(CH₂)₃NHCONH₂. Preferably, said amino acid is selected from alanine, glycine, asparagine, isoleucine, leucine, valine, phenylalanine, citrulline, glutamic acid.

In some embodiments, d is an integer from 2 to 8, e.g., 2 to 6, 2 to 4, 2 to 3, such as 2, 3, 4, 6, 8.

Alternatively, in some embodiments, B or the cleavable peptide contained in B has the structure from the C-terminus to the N-terminus as follows:

-(AA)_{d}- (B-2)

wherein AA is an amino acid selected from alanine (Ala), arginine (Arg), aspartic acid (Asp), asparagine (Asn), histidine (His), glycine (Gly), glutamic acid (Glu), glutamine (Gln), phenylalanine (Phe), lysine (Lys), substituted lysine, leucine (Leu), serine (Ser), tyrosine (Tyr), threonine (Thr), isoleucine (Ile), proline (Pro), tryptophan (Trp), valine (Val), cysteine (Cys), methionine (Met), selenocysteine, ornithine (Orn), β-alanine (β-Ala), citrulline (Cit), and derivatives thereof; preferably selected from alanine, glycine, asparagine, isoleucine, leucine, valine, phenylalanine, citrulline, glutamic acid; more preferably selected from alanine, glycine, asparagine, valine, phenylalanine, citrulline, glutamic acid;
d is an integer from 2 to 12; preferably an integer from 2 to 8, more preferably an integer from 2 to 4;
wherein each AA is linked by peptide bonds,
wherein the N-terminus of the peptide is connected to D (when present), E (when present), or the G part of the linker unit, and the C-terminus of the peptide is connected to the A or J part of the linker unit or directly to the drug P.

In some embodiments, the carbonyl terminus of the peptide is connected to the self-immolative linker A or directly to the drug P, and the amino terminus of the peptide is connected to the property-modulating unit D (when present), the linker unit (when present), or the antibody linker G.

In some embodiments, d in formula (B-1) or (B-2) is 2, and B comprises a dipeptide, for example selected from the following dipeptides: Ala-Ala, Ala-(D)Asp, Ala-Cit, Ala-Lys, Ala-Val, Asn-Cit, Asp-Cit, Asn-Lys, Asn-(D)Lys, Asp-Val, Cit-Ala, Cit-Asn, Cit-Asp, Cit-Cit, Cit-Lys, Cit-Ser, Cit-Val, Glu-Val, Glu-Gly, phenylGly-(D)Lys, His-Val, Ile-Cit, Ile-Pro, Ile-Val, Leu-Cit, Lys-Cit, Me3Lys-Pro, Met-Lys, Met-(D)Lys, Phe-Arg, Phe-Cit, Phe-Lys, Pro-(D)Lys, Ser-Cit, Trp-Cit, Ala-Val, Val-(D)Asp, NorVal-(D)Asp, Cit-Val, Val-Glu, Val-Lys, and salts thereof. In a specific embodiment, the dipeptide is Ala-Ala. In a specific embodiment, the dipeptide is Cit-Val. In another specific embodiment, the dipeptide is Ala-Val. In another specific embodiment, the dipeptide is Glu-Gly.

In some embodiments, d in formula (B-1) or (B-2) is 3, and B comprises a tripeptide, for example Gly-Gly-Gly, Gly-Gly-Arg, Phe-Lys-Gly, Leu-Lys-Gly, Leu-Leu-Gly, Glu-Val-Cit, Cit-Ala-Glu, Val-Lys-Gly, Val-Lys-Ala, Val-Gly-Gly, Val-Cit-Gly, Val-Gln-Gly, Val-Glu-Gly, Val-Lys-Gly, Val-Lys-Leu, Ala-Ala-Ala, Asn-Ala-Ala. In a specific embodiment, the tripeptide is Asn-Ala-Ala. In a specific embodiment, the tripeptide is Glu-Val-Cit. In a specific embodiment, the tripeptide is Cit-Ala-Glu.

In some embodiments, d in formula (B-1) or (B-2) is 4, and B comprises a tetrapeptide, for example Gly-Gly-Gly-Gly, Gly-Gly-Leu-Gly, Gly-Phe-Leu-Gly, Gly-Val-Lys-Gly, Ala-Leu-Ala-Leu, Gly-Phe-Leu-Gly, Ala-Leu-Ala-Leu, Gly-Phe-Gly-Gly, Val-Lys-Gly-Gly. In a specific embodiment, the tetrapeptide is Gly-Gly-Leu-Gly. In another specific embodiment, the tetrapeptide is Gly-Phe-Gly-Gly.

In some embodiments, d in formula (B-1) or (B-2) is 6 or 8, and B comprises a hexapeptide or octapeptide, for example Pro-Leu-Gly-Leu-Ala-Gly (PLGLAG), Gly-Pro-Leu-Gly-Ile-Ala-Gly-Gln (GPLGIAGQ), Gly-Pro-Val-Gly-Leu-Ile-Gly-Lys (GPVGLIGK).

Preferably, the peptides specifically exemplified above are connected via the carbonyl group of the leftmost amino acid to the self-immolative linker A or J or to the drug P, and via the N-terminus of the rightmost amino acid to D (when present), E (when present), or the G part of the linker unit.

In some embodiments, B is a peptide comprising substituted lysine, such as a dipeptide, tripeptide, tetrapeptide, hexapeptide, or octapeptide. In one embodiment, the substituted lysine is: 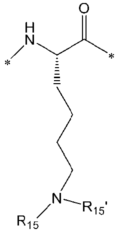 wherein R₁₅ and R₁₅' are each independently selected from: H, C₁₋₆ alkyl, -CO-NH₂, -CONH(C₁₋₆ alkyl), and -CONH(C₁₋₆ alkyl)₂, wherein said alkyl is optionally substituted by a group selected from halogen, C₁₋₆ alkoxy, C₁₋₆ haloalkyl, and C₃₋₆ cycloalkyl.

In preferred embodiments, B is selected from the following peptides: Ala-Ala, Cit-Val, Ala-Val, Glu-Gly, Asn-Ala-Ala, Cit-Ala-Glu, Gly-Phe-Gly-Gly, for example as shown below: 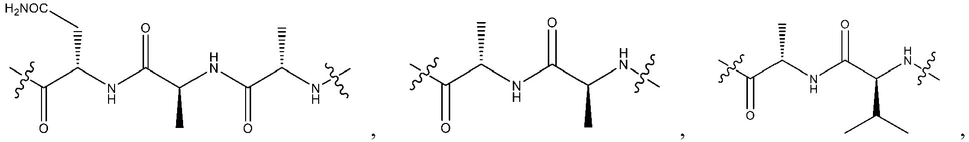 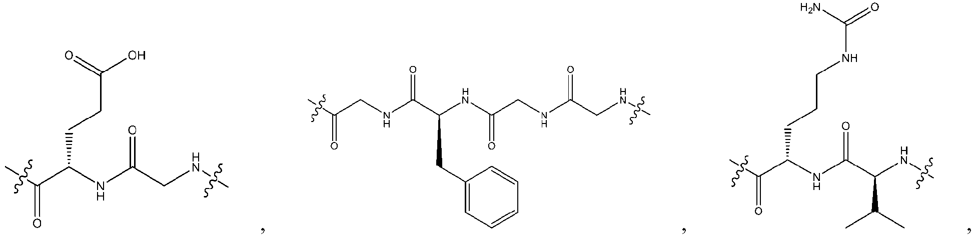 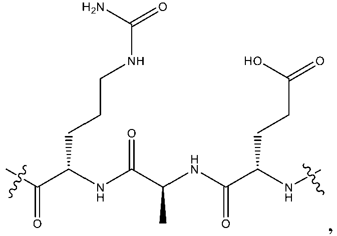 wherein the left carbonyl group is connected to the self-immolative linker A or J or to the drug P, and the right N-terminus is connected to D (when present), E (when present), or the G part of the linker unit.

As described above, by designing antibody-drug conjugates targeted to tumor distribution, the linker unit containing the peptide B unit will promote the release of the KRas mutant inhibitor in tumor cells and the environment. Thus, in some embodiments, after the ADC is internalized into tumor cells, the amide bonds in the peptide of the B unit will be recognized and degraded by enzymes in the tumor cells, releasing the drug moiety P. In other embodiments, the amide bonds in the peptide of the B unit will be recognized and degraded by enzymes in the tumor environment, releasing the drug moiety P.

In other embodiments, B in the linker unit L of the ADC of the present disclosure comprises a reducible group.

In some embodiments, B in the linker unit L of the ADC of the present disclosure comprises a disulfide group. This group is reduced by glutathione when the ADC is internalized into tumor cells, as tumor cells can induce hypoxic conditions due to irregular blood flow, leading to elevated glutathione concentrations, thereby selectively releasing the KRas mutant inhibitor drug in tumor cells.

In other embodiments, B in the linker unit L of the ADC of the present disclosure comprises a nitro group, which can be reduced under hypoxic conditions or by nitroreductase.

In another aspect, B in the linker unit L of the ADC of the present disclosure comprises a specific sugar and derivatives thereof. Specific sugars and derivatives thereof applicable are as generally and specifically defined below for the solubilizing sugar unit S.

In some embodiments, B in the linker unit L of the ADC of the present disclosure comprises, for example, galactose. In this case, since β-galactosidase is primarily active intracellularly, it will promote the release of the KRas mutant inhibitor within the target cell.

In other embodiments, B in the linker unit L of the ADC of the present disclosure comprises, for example, glucuronic acid. Since β-glucuronidase is highly concentrated in the microenvironment of various solid tumors, including lung, breast, and gastrointestinal cancers, the inclusion of glucuronic acid in B of the ADC linker unit will enable specific release of the KRas mutant inhibitor drug in the tumor microenvironment.

In other embodiments, B in the linker unit L of the ADC of the present disclosure can be absent, for example, the self-immolative linker is directly connected to the antibody linker.

### S-Solubilizing Sugar Unit

The solubilizing sugar unit S in the linker unit L of formula (II) of the ADC of the present disclosure is connected to the self-immolative linker A and is specifically hydrolyzed in vivo by enzymes such as β-glucuronidase or β-galactosidase, undergoing a self-immolative reaction to release the drug P. In the antibody-drug conjugates of the present disclosure, introducing a sugar group into the linker unit helps improve the hydrophilicity of the conjugated chain, increases the payload loading rate to the theoretical maximum value, while improving the solubility and purity of the conjugate, reducing aggregation, improving the druggability of the conjugate, and ultimately enhancing the effect of inhibiting tumor cell proliferation.

In the present disclosure, the solubilizing sugar unit S can be a monosaccharide, disaccharide, oligosaccharide, or even a polysaccharide, and their respective derivatives.

The "monosaccharide" of the solubilizing sugar unit and the "monosaccharide unit" as the basic structural unit of disaccharides, oligosaccharides, or polysaccharides refer to polyhydroxy aldehydes (aldoses) or polyhydroxy ketones (ketoses) containing 3 or more carbon atoms and their derivatives:

H-[CHOH]ₐ-CHO

or

H-[CHOH]ₐ-CO-[CHOH]_{b}-H

wherein a ≥ 2, b ≥ 1. Monosaccharides suitable for the ADC of the present disclosure can be three-carbon sugar (triose), four-carbon sugar (tetrose), five-carbon sugar (pentose), six-carbon sugar (hexose), seven-carbon sugar (heptose), etc., preferably five-carbon sugar or six-carbon sugar (aldose or ketose), more preferably six-carbon sugar (aldose or ketose), and their derivatives, including for example phosphate esters, sugar alcohols, sugar acids, deoxy sugars, amino sugars, acylated amino sugars, and glycosides of monosaccharides, preferably uronic acids, amino sugars, or acylated amino sugars (preferably acetamido sugars).

Phosphate esters of monosaccharides suitable for the ADC of the present disclosure are sugar compounds formed by esterification of one or more hydroxyl groups of a monosaccharide with phosphoric acid, typically D-glucose-1 or 6-phosphate, D-fructose-1 or 6-phosphate, D-fructose-1,6-diphosphate, D-ribose-5-phosphate.

Sugar alcohols suitable for the ADC of the present disclosure are sugar compounds formed by reduction of the carbonyl group of a sugar to OH, typically D-glucitol, sorbitol, D-mannitol, galactitol, inositol, xylitol, ribitol, allitol, D-arabinitol.

Sugar acids suitable for the ADC of the present disclosure include aldonic acids formed by oxidation of the aldehyde group of an aldose to a carboxyl group, uronic acids formed by oxidation of the primary alcohol group of an aldose to a carboxyl group, and aldaric acids formed by oxidation of both the aldehyde and primary alcohol groups of an aldose to carboxyl groups. Typical examples include D-glucuronic acid, D-galacturonic acid, D-mannuronic acid, D-iduronic acid.

Deoxy sugars suitable for the ADC of the present disclosure are sugars in which one or two hydroxyl groups in the monosaccharide molecule are replaced by hydrogen atoms. Typical examples include 6-deoxy sugars, such as 6-deoxy-allose, 6-deoxy-altrose, 6-deoxy-gulose, 6-deoxy-talose, rhamnose, fucose, 2-deoxyribose.

Amino sugars suitable for the ADC of the present disclosure are sugar derivatives where one or more hydroxyl groups of a monosaccharide molecule are replaced by amino groups, also known as sugar amines. The most common are 2-amino hexoses, such as glucosamine, galactosamine, mannosamine, D-gulosamine, D-fucosamine; 3-amino sugars, such as kanosamine, 3-aminoribose, 3-amino-3,6-dideoxy-D-glucose or galactose; 4-amino sugars, such as 4-amino-4,6-dideoxy-D-glucose or galactose; 6-amino sugars, such as 6-amino-6-deoxy-D-glucose; and diamino sugars, such as 2,6-diamino-2,6-dideoxy-L-idose, 2,6-diamino-2,6-dideoxy-D-glucose.

Acylated amino sugars suitable for the ADC of the present disclosure are sugar derivatives where the amino group of an amino sugar is acylated. Typical examples include 2-acetamido-2-deoxy-D-galactose, 2-acetamido-2-deoxy-D-glucose, 2-acetamido-2-deoxy-D-mannose, 3-acetamido-3-deoxy-D-glucose, 3-acetamido-3,6-dideoxy-D-mannose.

Glycosides suitable for the ADC of the present disclosure are sugar-containing derivatives formed by condensation of the hemiacetal hydroxyl group of a monosaccharide with the hydroxyl, amine, or thiol group of another molecule (such as an alcohol, sugar, purine, or pyrimidine), also known as glycosides, i.e., a derivative composed of a sugar residue (sugar lacking the hemiacetal hydroxyl) and an aglycone linked via a glycosidic bond. Glycosidic bonds can link through oxygen, sulfur, or nitrogen atoms, and their glycosides are referred to as O-glycosides, S-glycosides, N-glycosides, or C-glycosides, respectively. The aglycone of a glycoside can be a sugar, thus forming disaccharides, oligosaccharides, and polysaccharides.

Sugar derivatives suitable for the ADC of the present disclosure can also be derivatives formed by derivatizing a sugar molecule in more than one of the above ways, for example, acylated amino uronic acids, such as 2-acetamido-2-deoxy-D-galacturonic acid, 2-acetamido-2-deoxy-D-glucuronic acid, 2-acetamido-2-deoxy-D-mannuronic acid.

Exemplary D-series five-carbon sugar or six-carbon sugar suitable for the ADC of the present disclosure are shown below:

| | |
|---|---|
| 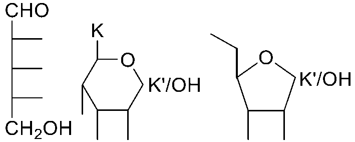 | 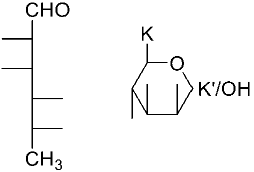 |
| Ribose K=K'=H | Rhamnose K=CH₃, K'=H |
| 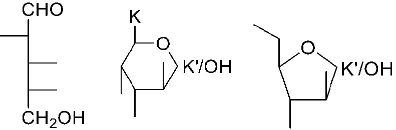 | 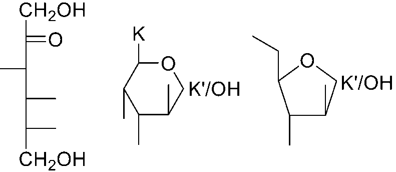 |
| Arabinose K=K'=H | Fructose K=H, K'= CH₂OH |
| 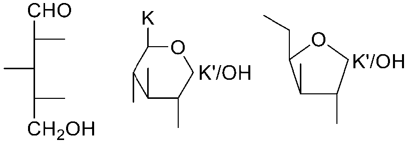 | 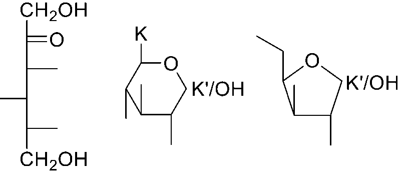 |
| Xylose K=K'=H | Sorbose K=H, K'= CH₂OH |
| 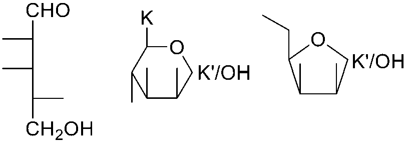 | 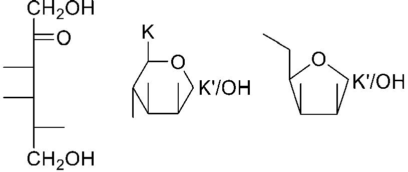 |
| Lyxose K=K'=H | Tagatose K=H, K'= CH₂OH |
| 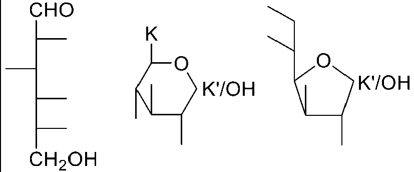 | 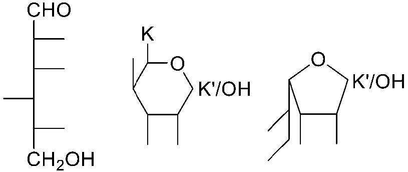 |
| Glucose K=CH₂OH, K'=H | Gulose K=CH₂OH, K'=H |
| 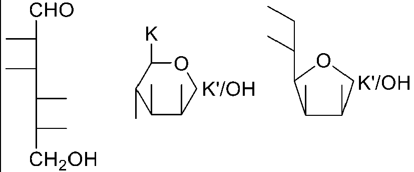 | 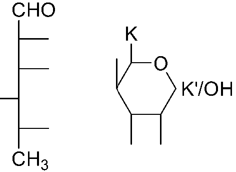 |
| Mannose K=CH₂OH, K'=H | 6-Deoxygulose K=CH₃, K'=H |
| 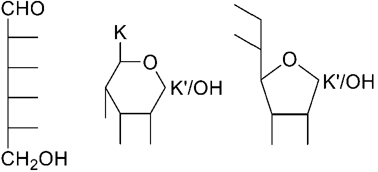 | 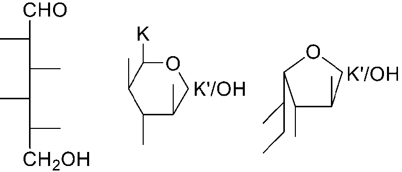 |
| Allose K=CH₂OH, K'=H | Idose K=CH₂OH, K'=H |
| 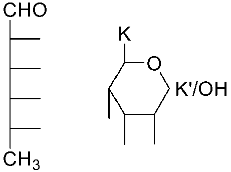 | 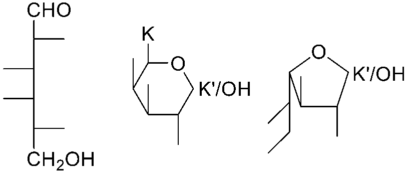 |
| 6-Deoxyalose K=CH₃, K'=H | Galactose K=CH₂OH, K'=H |
| 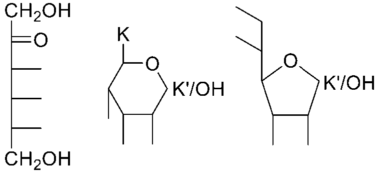 | 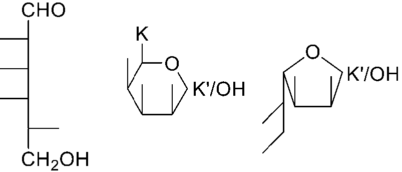 |
| Psicose K=H, K'=CH₂OH | Talose K=CH₂OH, K'=H |
| 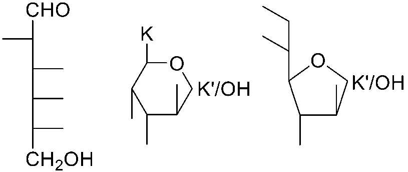 | 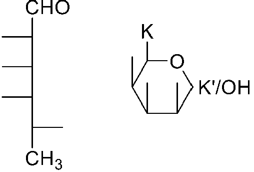 |
| Altrose K=CH₂OH, K'=H | 6-Deoxytalose K=CH₃, K'=H |
| 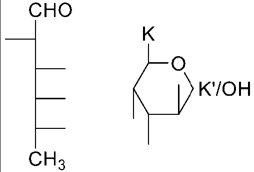 | 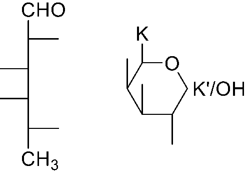 |
| 6-Deoxyaltrose K=CH₃, K'=H | Fucose K=CH₃, K'=H |
| 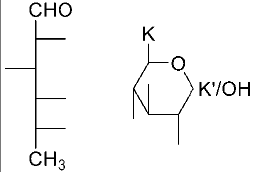 | |
| Quinovose K=CH₃, K'=H | |

including their respective α or β anomers, and their respective various derivatives as described above, including for example phosphate esters, sugar alcohols, sugar acids, deoxy sugars, amino sugars, acylated amino sugars, and glycosides; preferred derivatized forms are uronic acids (D-galacturonic acid, D-glucuronic acid, D-mannuronic acid), amino sugars (2-amino-2-deoxy-D-galactose, 2-amino-2-deoxy-D-glucose, 2-amino-2-deoxy-D-mannose), acylated amino sugars (2-acetamido-2-deoxy-D-galactose, 2-acetamido-2-deoxy-D-glucose, 2-acetamido-2-deoxy-D-mannose), amino uronic acids (2-amino-2-deoxy-D-galacturonic acid, 2-amino-2-deoxy-D-glucuronic acid, 2-amino-2-deoxy-D-mannuronic acid), acylated amino uronic acids (2-acetamido-2-deoxy-D-galacturonic acid, 2-acetamido-2-deoxy-D-glucuronic acid, 2-acetamido-2-deoxy-D-mannuronic acid).

In the ADC of the present disclosure, the solubilizing monosaccharide unit S can be connected to the self-immolative linker A via any free hydroxyl group, preferably via the OH at position 1 of the sugar unit.

Monosaccharides or derivatives thereof suitable for the ADC of the present disclosure can be selected from glyceraldehyde, erythrose, erythrulose, arabinose, ribose, ribulose, xylose, xylulose, lyxose, glucose, mannose, allose, psicose, altrose, quinovose, rhamnose, fructose, sorbose, tagatose, gulose, idose, galactose, talose, lactose, maltose, sucrose, or their respective phosphate esters, sugar alcohols, uronic acids, deoxy sugars, amino sugars, acylated amino sugars, and/or glycoside forms.

Examples of monosaccharides or derivatives thereof suitable for the ADC of the present disclosure include, but are not limited to, glyceraldehyde, glyceraldehyde phosphate, dihydroxyacetone phosphate, erythrose, erythrulose, arabinose, ribose, ribulose, deoxyribose, ribitol, ribose phosphate, xylose, xylulose, xylitol, lyxose, glucose, glucosamine, acetylglucosamine, glucuronic acid, acetylglucosaminic acid, glucose phosphate, mannose, mannuronic acid, mannitol, mannosamine, acetylmannosamine, acetylmannosaminuronic acid, fructose, fructose phosphate, galactose, galactitol, galacturonic acid, galactosamine, acetylgalactosamine, acetylgalactosaminuronic acid, allose, deoxyallose, altrose, deoxyaltrose, quinovose, rhamnose, psicose, sorbose, sorbitol, tagatose, gulose, deoxygulose, idose, talose, fucose, deoxytalose, etc.

The above monosaccharides and their derivatives include their D-configuration, L-configuration, racemates (DL), and meso forms, preferably the D-configuration, and also include any optically active forms ((+), (-), (±)). The monosaccharides and their derivatives described in the present disclosure include their chain isomers, cyclic isomers, or mixtures thereof, wherein the cyclic forms include α-anomeric forms and β-anomeric forms or mixtures thereof, and include, for example, pyranose or furanose forms.

Preferred monosaccharides or derivatives thereof are selected from glucose, galactose, mannose, or their respective uronic acid, amino sugar, acylated amino sugar, amino uronic acid, or acylated amino uronic acid forms, for example glucose, galactose, mannose, glucosamine, galactosamine, mannosamine, galacturonic acid, glucuronic acid, mannuronic acid, glucosaminuronic acid, galactosaminuronic acid, mannosaminuronic acid, acetylgalactosamine, acetylglucosamine, acetylmannosamine, acetylgalactosaminuronic acid, acetylglucosaminuronic acid, acetylmannosaminuronic acid; further preferred are D-glucose, D-galactose, D-mannose, D-glucosamine, D-galactosamine, D-mannosamine, D-galacturonic acid, D-glucuronic acid, D-mannuronic acid, D-glucosaminuronic acid, D-galactosaminuronic acid, D-mannosaminuronic acid, 2-acetamido-2-deoxy-D-galactose, 2-acetamido-2-deoxy-D-glucose, 2-acetamido-2-deoxy-D-mannose, 2-acetamido-2-deoxy-D-galacturonic acid, 2-acetamido-2-deoxy-D-glucuronic acid, 2-acetamido-2-deoxy-D-mannuronic acid.

In preferred embodiments, the solubilizing monosaccharide units and derivatives thereof in the ADC of the present disclosure include those shown in **Table 3** below:

| | | |
|---|---|---|
| 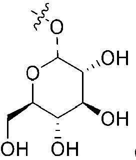 Glucose | 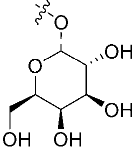 Galactose | 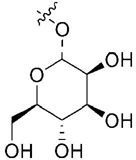 Mannose |
| 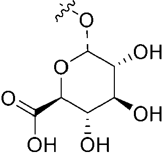 | 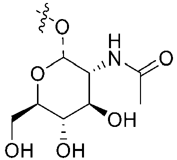 | 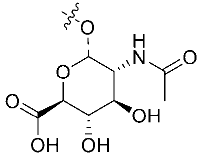 |
| D-Glucuronic acid | 2-Acetamido-2-deoxy-D-glucose | 2-Acetamido-2-deoxy-D-glucuronic acid |
| 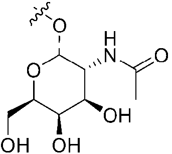 | 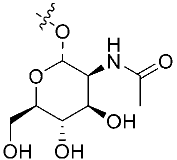 | 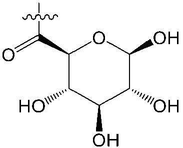 |
| 2-Acetamido-2-deoxy-D-galactose | 2-Acetamido-2-deoxy-D-mannose | |
| 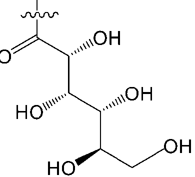 | 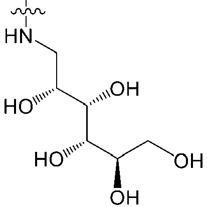 | 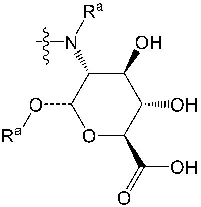 |
| 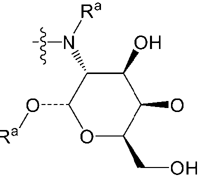 | 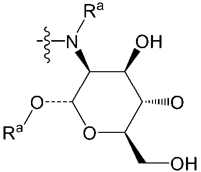 | 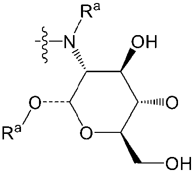 |
| wherein the wavy bond indicates the connection bond of the sugar unit to the linker unit; the dashed line at position 1 of the sugar unit indicates that the configuration of the glycosidic bond can be α or β, or a mixture thereof; wherein R^{a} is H or C₁₋₆ alkyl, for example methyl. | | |

The "disaccharide" in the linker unit L suitable for the ADC of the present disclosure refers to a sugar compound formed by linking two monosaccharides or derivatives thereof via a glycosidic bond. Depending on the linkage mode, there are two types: one is a non-reducing sugar formed by dehydration of the hemiacetal (hemiketal) hydroxyl groups of two sugars to form a glycosyl glycoside; the other is a reducing sugar formed by glycosidation of the hemiacetal (hemiketal) hydroxyl group of one sugar with the non-hemiacetal (non-hemiketal) hydroxyl group of another sugar, known as glycosyl sugar. The monosaccharide units constituting the disaccharide can be the various monosaccharides or derivatives thereof as described above. Depending on the type of disaccharide, the two monosaccharide units of the disaccharide can be linked via, for example, α-1,4 glycosidic bonds, α-1,6 glycosidic bonds, β-1,4 glycosidic bonds, α-1-2β glycosidic bonds, β-1,6 glycosidic bonds, α-1,1 glycosidic bonds, β-1,3 glycosidic bonds, or β-2,1 glycosidic bonds.

Each monosaccharide unit constituting the disaccharide can be derivatized as described above, for example, each monosaccharide unit is independently in the form of a uronic acid, amino sugar, acylated amino sugar, acylated amino uronic acid, or amino uronic acid. Specific examples of disaccharides include, but are not limited to, maltose, isomaltose, lactose, sucrose, chitobiose, rutinose, trehalose, xylobiose, gentiobiose, etc., and their respective derivatives as described above.

Preferred disaccharides suitable for the ADC of the present disclosure include lactose or derivatives thereof, maltose or derivatives thereof, sucrose or derivatives thereof, including their α and β anomers, wherein the derivatized forms are, for example, uronic acids, amino sugars, acylated amino sugars, acylated amino uronic acids, amino uronic acids, specifically D-lactose, D-maltose, D-sucrose, or their respective acetamido (e.g., 2-acetamido) and/or 6-carboxy derivatives; specific examples include, but are not limited to, those shown in **Table 4** below:

| | | |
|---|---|---|
| 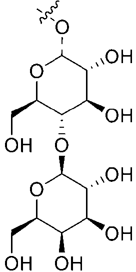 | 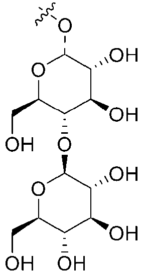 | 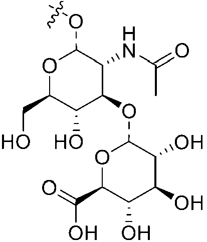 |
| Lactose | Maltose | Maltose derivative - acetamido and carboxylic acid derivative |
| 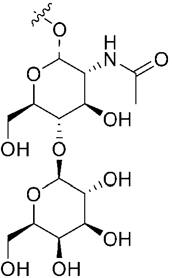 | 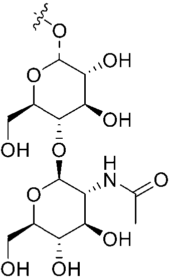 | 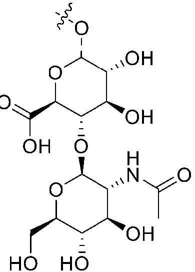 |
| Lactose derivative - acetamido derivative | Maltose derivative - acetamido derivative | Maltose derivative - acetamido and carboxylic acid derivative |

wherein the wavy bond indicates the connection bond of the sugar unit to the linker unit; the dashed line at position 1 of the sugar unit indicates that the configuration of the glycosidic bond can be α, β, or a mixture thereof.

The solubilizing sugar unit in the linker unit L suitable for the ADC of the present disclosure can also be low-polymerized sugars composed of 3~9 monosaccharides linked via glycosidic bonds and their derivatives, i.e., oligosaccharides and their derivatives, wherein the monosaccharides, glycosidic bonds, and derivatives are as defined above, for example but not limited to, mannotriose, selaginotriose, gentianose, plantose, stachyose, raffinose, etc.

The solubilizing sugar unit in the linker unit L suitable for the ADC of the present disclosure can also be polysaccharides composed of 10 or more monosaccharide units linked via glycosidic bonds. The glycosidic bonds can be α-type, β-type, or α/β mixed. Structurally, they can be linear, branched, or cyclic. Polysaccharides can be homopolysaccharides composed of one type of monosaccharide, where the sugar units are selected from three carbon sugar, four-carbon sugar, five-carbon sugar, six-carbon sugar, seven-carbon sugar, and eight carbon sugar or derivatives thereof such as deoxy sugar units, for example dextran, xylan, polysialic acid, etc.; or heteropolysaccharides composed of two or more types of monosaccharides, such as hyaluronic acid, heparin, etc. Examples of polysaccharides include, but are not limited to, dextran, levan, hyaluronic acid, cyclodextrins (α, β, γ, etc.), hydroxyethyl starch, xylan, water-soluble starch, water-soluble cellulose, carboxymethyl cellulose, galactosamine, polysialic acid, rhamnan, Ganoderma lucidum polysaccharide, lentinan, chitin, chitosan, alginate, carrageenan, gellan gum, pullulan, scleroglucan, xanthan gum, xyloglucan, amylose, etc.

The solubilizing sugar unit in the linker unit L suitable for the ADC of the present disclosure is connected to the linker unit L, specifically to the self-immolative linker A in the linker unit, via a glycosidic bond, which can be α-type, β-type, or a mixed form thereof.

### A-Self-Immolative Linker

The self-immolative linker A in the linker unit L of formula (II) of the ADC of the present disclosure can be a covalent bond, or a functional group that facilitates the connection of the cleavable linker B to the drug P, or a functional group that provides additional structural components to further promote the release of the drug P from the remainder of the ADC.

In some embodiments, the self-immolative linker A in the linker unit L of formula (II) of the ADC of the present disclosure is a covalent bond, i.e., the cleavable linker B and the drug P in the linker unit are directly connected, for example via an amide bond.

In other embodiments, the self-immolative linker A between the drug P and the cleavable linker B in the ADC of the present disclosure is a functional group that facilitates the connection of the cleavable linker B to the drug P, or can provide additional structural components to further promote the release of the drug P from the remainder of the ADC. Specifically, A connected to the drug P has the sub-general formulas shown in **Table 5** below:

| | | |
|---|---|---|
| 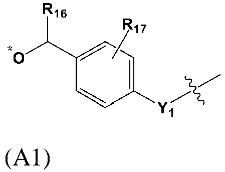 | 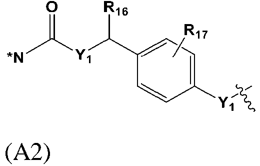 | 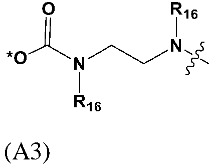 |
| 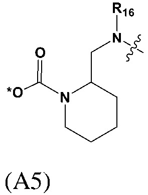 | 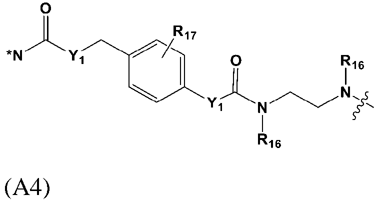 | 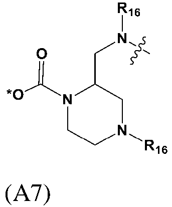 |
| 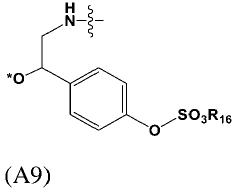 | 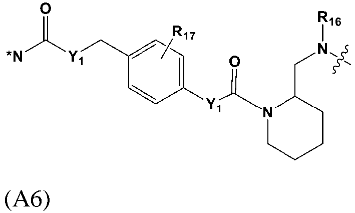 | 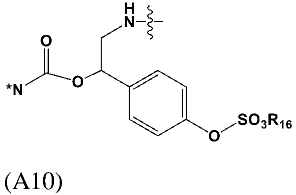 |
| 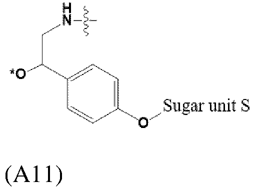 | 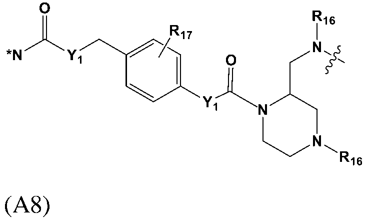 | 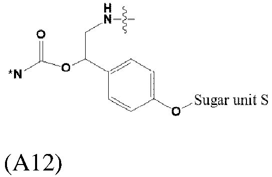 |
| wherein the O or N atom marked with * does not constitute part of the self-immolative linker A, but is included for illustrative purposes to indicate the atom in the KRas mutant inhibitor connected to the self-immolative linker A; | | |
| the wavy bond is connected to the remainder of the linker unit. | | |

wherein,
each occurrence of Y₁ is independently selected from NH or O;
each occurrence of R₁₆ is independently selected from H and -C₁₋₆ alkyl, preferably H or C₁₋₃ alkyl, more preferably H or methyl;
each occurrence of R₁₇ is independently selected from H, -NO₂, -NH₂, -CF₃, or is -O-sugar unit S, or is a fragment of formula (Al): 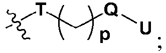
each occurrence of sugar unit S is as defined herein for the solubilizing sugar unit S in the linker unit, and
each occurrence of the moiety of formula (A1): 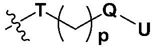 is as generally or specifically defined herein for the self-immolative linker J part.

The sugar unit carried by the self-immolative linker A can be linked to A via an α-glycosidic bond, β-glycosidic bond, or a mixture thereof.

In some embodiments, R₁₆ is H. In other embodiments, R₁₆ is -C₁₋₆ alkyl, preferably C₁₋₃ alkyl, more preferably methyl. Preferably R₁₆ is H.

In some embodiments, R₁₇ is H. In other embodiments, R₁₇ is -O-sugar unit S. In other embodiments, R₁₇ is a fragment of formula (Al):

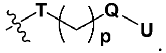

In a specific embodiment, A connected to the KRas mutant inhibitor has the formula: 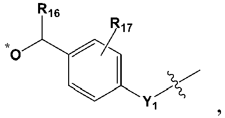 specific examples include 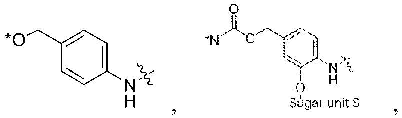 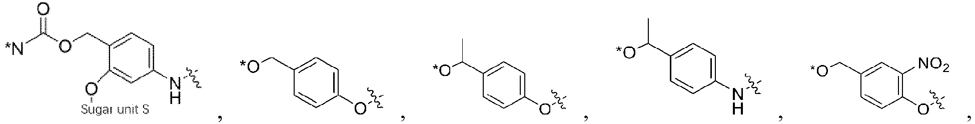 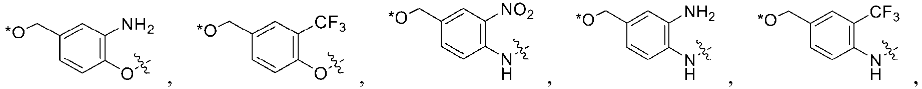 or the respective fragments where R₁₇ is correspondingly 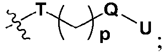 more specifically, this group of self-immolative linkers A is connected via the OH present on the side-chain naphthalene or benzothiophene ring of the drug P unit.

In a specific embodiment, A connected to the KRas mutant inhibitor has the formula: 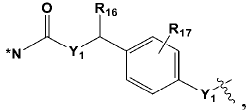 specific examples include 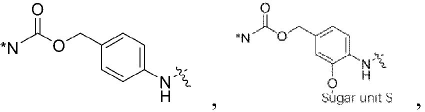 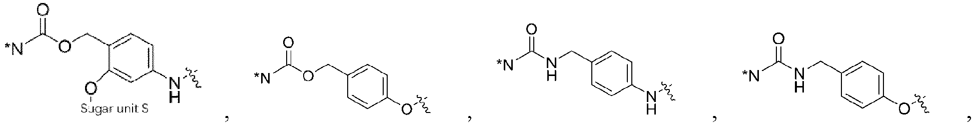 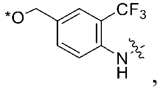 or the respective fragments where R₁₇ is correspondingly 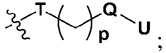 more specifically, this group of self-immolative linkers A is connected via the ring NH in the 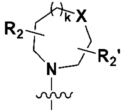 fragment of the drug P unit.

In a specific embodiment, A connected to the KRas mutant inhibitor has the formula: 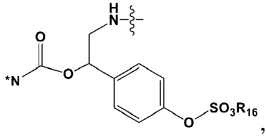 wherein R₁₆ is H.

In some specific embodiments, A connected to the KRas mutant inhibitor has formula (A9), (A10), (A11), or (A12). In this case, the cleavable linker B can be absent, and such self-immolative linkers can be directly connected to the property-modulating unit D, the linker unit E, or the antibody linker G, and such self-immolative linkers can directly release the KRas mutant inhibitor upon activation by enzymes such as sulfatase or β-glucuronidase.

In preferred embodiments of the present disclosure, A connected to the KRas mutant inhibitor has the formula: 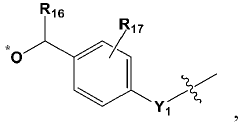 e.g. 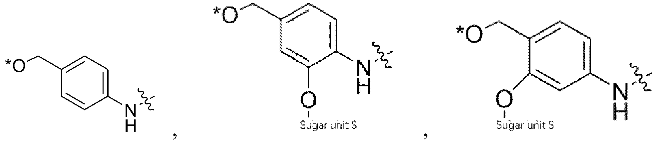 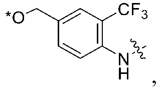 or the respective fragments where R₁₇ is correspondingly 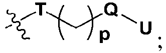 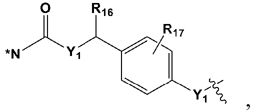 e.g., 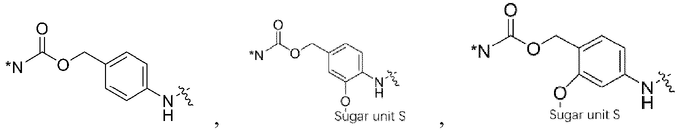 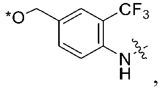 or the respective fragments where R₁₇ is correspondingly

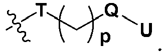

In the above general or preferred embodiments of the self-immolative linker A, the sugar unit S present can be selected from the monosaccharides or derivatives thereof, disaccharides or derivatives thereof, or oligosaccharides or derivatives thereof as generally or specifically defined in the solubilizing sugar unit S section above, preferably monosaccharides or derivatives thereof (glucose, galactose, mannose, glucosamine, galactosamine, mannosamine, galacturonic acid, glucuronic acid, mannuronic acid, acetylgalactosamine, acetylglucosamine, acetylmannosamine, acetylgalactosaminuronic acid, acetylglucosaminuronic acid, acetylmannosaminuronic acid, glucosaminuronic acid, galactosaminuronic acid, mannosaminuronic acid; further preferred are D-glucose, D-galactose, D-mannose, D-glucosamine, D-galactosamine, D-mannosamine, D-galacturonic acid, D-glucuronic acid, D-mannuronic acid, D-glucosaminuronic acid, D-galactosaminuronic acid, D-mannosaminuronic acid, 2-acetamido-2-deoxy-D-galactose, 2-acetamido-2-deoxy-D-glucose, 2-acetamido-2-deoxy-D-mannose, 2-acetamido-2-deoxy-D-galacturonic acid, 2-acetamido-2-deoxy-D-glucuronic acid, 2-acetamido-2-deoxy-D-mannuronic acid, such as those shown in Table 3 above) and disaccharides or derivatives thereof (lactose or derivatives thereof, maltose or derivatives thereof, sucrose or derivatives thereof, including their α and β anomers, wherein the derivatized forms are, for example, uronic acids, acylated amino sugars, acylated amino uronic acids, specifically D-lactose, D-maltose, D-sucrose, or their respective 2-acetamido and/or 6-carboxy derivatives, such as those shown in Table 4 above).

The individual monosaccharide or derivative thereof, or the monosaccharide or derivative thereof as a structural unit of a disaccharide, is preferably a five-carbon sugar or six-carbon sugar (aldose or ketose), more preferably a six-carbon sugar (aldose or ketose), or a derivative thereof, including phosphate esters of monosaccharides, sugar alcohols, sugar acids, deoxy sugars, amino sugars, acylated amino sugars, and glycosides, preferably sugar acids, amino sugars, or acylated amino sugars.

The linker unit of the ADC of the present disclosure includes structural fragments obtained by any combination, as appropriate, of the self-immolative linker A as generally or specifically defined above with the monosaccharide or derivative thereof S as generally or specifically defined above. Preferred examples of the self-immolative linker A carrying a monosaccharide or derivative thereof include, but are not limited to, those shown in **Table 3-1** below:

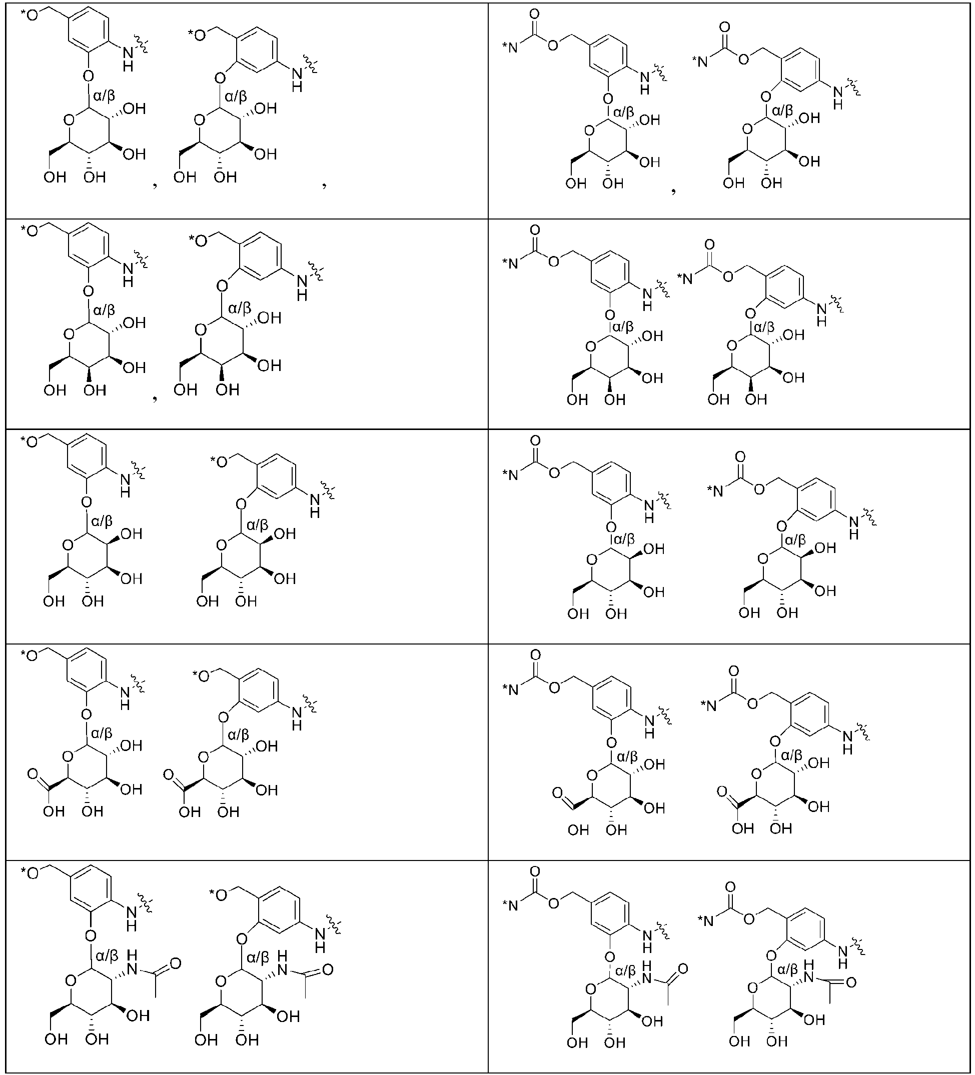

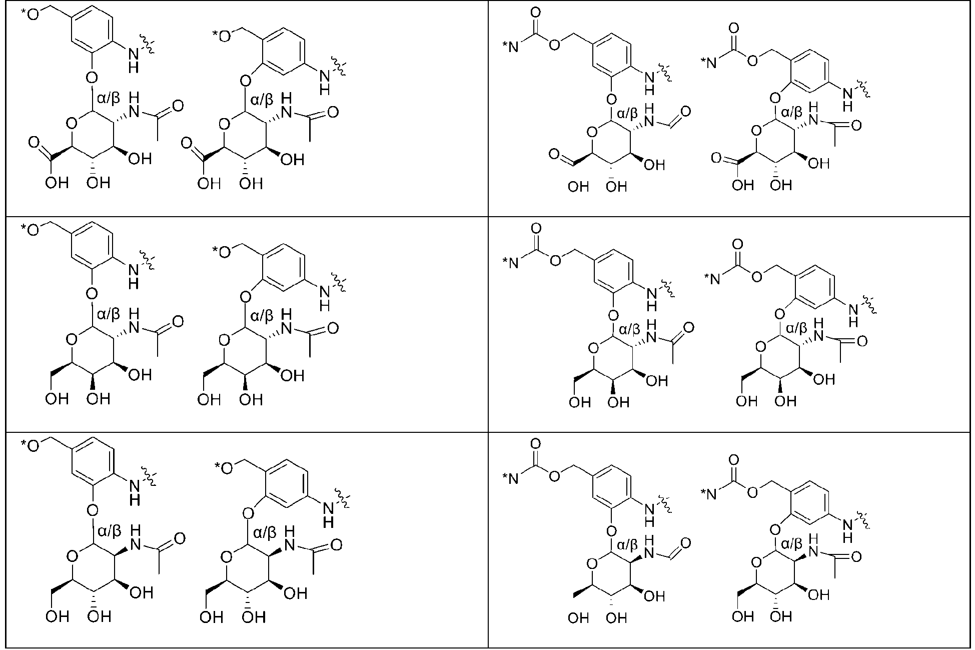

The linker unit L of the ADC of the present disclosure includes structural fragments obtained by any combination, as appropriate, of the self-immolative linker A as generally or specifically defined above with the disaccharide or derivative thereof as generally or specifically defined above. Preferred examples of the self-immolative linker A carrying a disaccharide or derivative thereof include, but are not limited to, those shown in **Table 4-1** below:

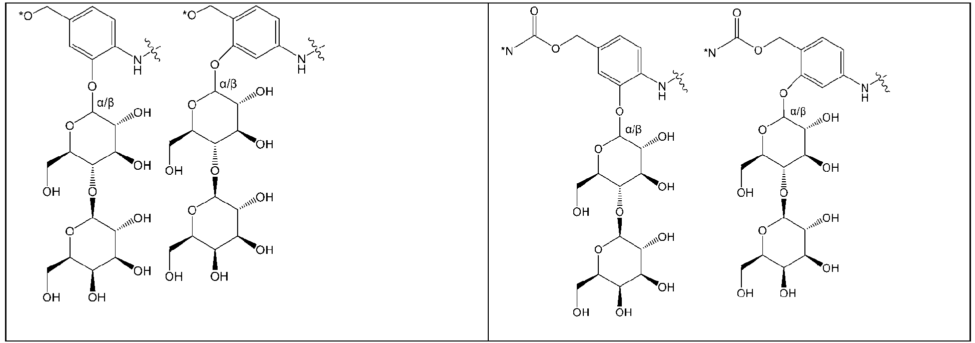

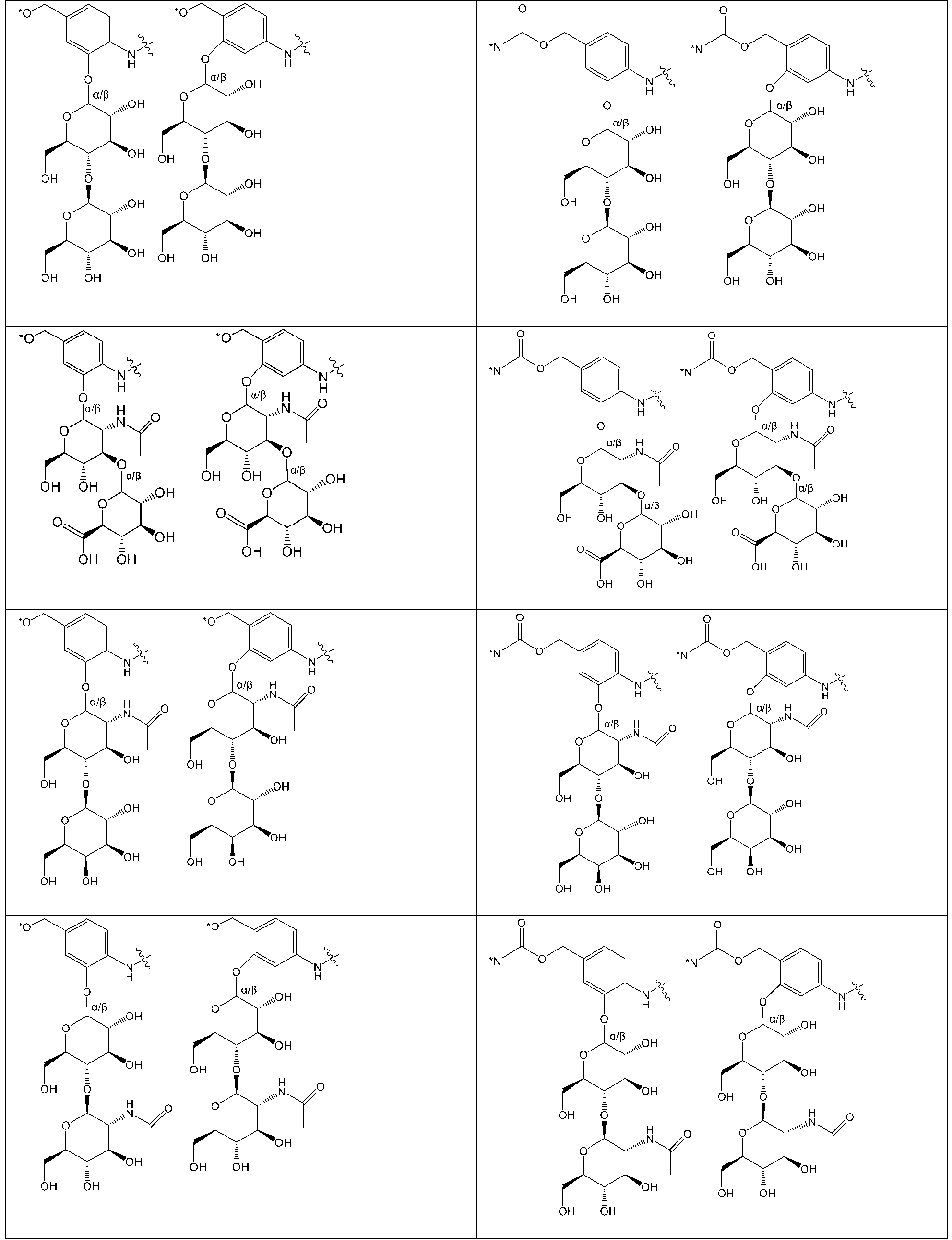

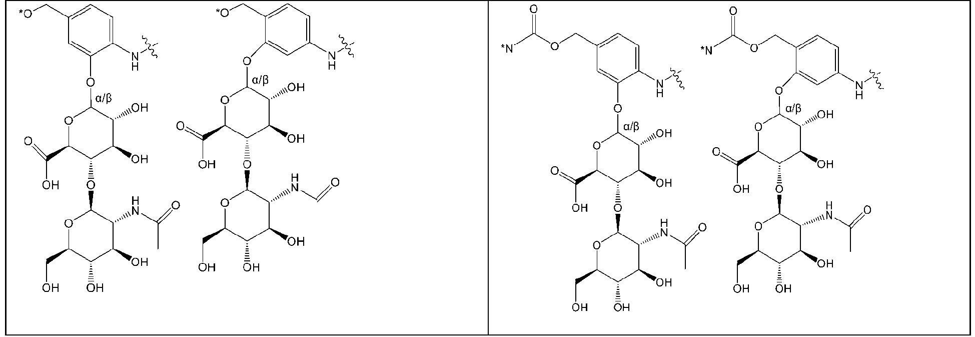

In the specific examples above, the α/β indicated at position 1 of the sugar unit means that the configuration of the glycosidic bond can be α, β, or a mixture thereof.

### J-Self-Immolative Linker

The self-immolative linker J in the linker unit L of formula (II') of the ADC of the present disclosure is a functional group that facilitates the connection of the cleavable linker B to the drug P, or provides additional structural components to further promote the release of the drug P from the remainder of the ADC.

In some embodiments, the self-immolative linker J in the linker unit L of formula (II') of the ADC of the present disclosure is a covalent bond, i.e., the cleavable linker B and the drug P in the linker unit are directly connected, for example via an amide bond.

Specifically, J connected to the drug P is selected from -J₁-, -J₂-, -J₁-J₂ -, -J₂-J₁-, wherein
-J₁- is selected from 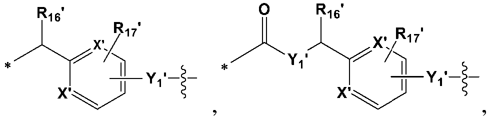 wherein the * indicates the point of attachment to drug P or to -J₂-, and the wavy line indicates the point of attachment to the cleavable linker B;
-J₂- is selected from 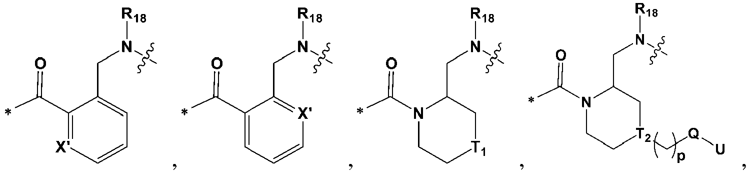 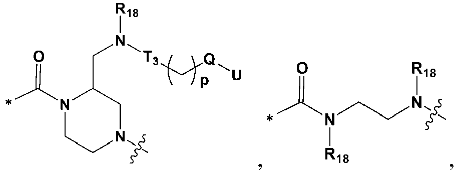 wherein the * indicates the point of attachment to drug P or to -J₁-, and the wavy line indicates the point of attachment to the cleavable linker B;
each occurrence of X' is independently selected from CH or N;
each occurrence of Y₁' is independently selected from -NH-, -NC₁₋₆ alkyl-, or -O-;
each occurrence of R₁₆' is independently selected from H and -C₁₋₆ alkyl, preferably H or C₁₋₃ alkyl, more preferably H;
each occurrence of R₁₇' is independently selected from H, -NO₂, -NH₂, -CF₃, or a fragment of formula (A1): 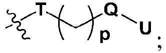
each occurrence of R₁₈ is independently selected from -C₁₋₆ alkyl, preferably C₁₋₃ alkyl, more preferably methyl;
T is selected from -C(R^{a})₂-, -O-, -NR^{a}-, or is absent;
T₁ is selected from -C(R^{a})₂-, -O-, -NR^{a}-;
T₂ is selected from -CR^{a}- and -N-;
T₃ is a peptide of 2 to 6 amino acids linked via the carboxyl terminus to N;
Q is selected from -CO-, -O-, -NR^{a}-, or is absent;
U is selected from glycosyl or a derivative thereof, 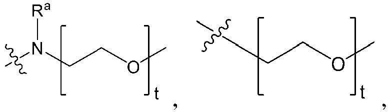 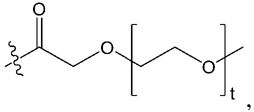 and a hydrophilic peptide comprising 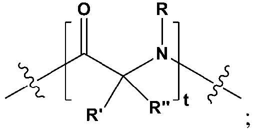
R^{a} is each independently selected from H or -C₁₋₆ alkyl, preferably H or -CH₃;
the subunit 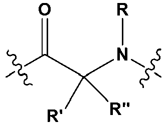 is selected from natural amino acid residues and unnatural amino acid residues;
p is an integer from 0 to 4, for example 0-2, 1-4, 1-2, 2-4;
t is an integer from 0 to 20, for example 0-8, 0-6, 0-4, 0-2, 1-20, 1-10, 2-10, 2-8, 2-6, 2-4, 4-14, 6-10, 6-12, 8-10, 8-14.

The glycosyl or derivative thereof appearing in the self-immolative linker J is selected from monosaccharide groups, disaccharide groups, oligosaccharide groups, or polysaccharide groups or derivatives thereof, preferably the monosaccharides or disaccharides or derivatives thereof as generally, specifically, or preferably defined in the "Linker Unit L" section herein for the solubilizing sugar unit S, more preferably the monosaccharides or disaccharide groups or derivatives thereof exemplified in Tables 3 and 4.

In some embodiments, U appearing in the self-immolative linker J is a monosaccharide or disaccharide or derivative thereof as defined herein and is linked via a glycosidic bond (-O-sugar residue); in other embodiments, U is an amino sugar or derivative thereof as defined herein and is linked via an amino group, preferably an amino sugar derivative of a monosaccharide or disaccharide; in other embodiments, U is a sugar acid or derivative thereof as defined herein and is linked via a carbonyl group.

It should be noted that when U appearing in the self-immolative linker J is a hydrophilic peptide, it is as defined accordingly herein, more specifically as generally or specifically defined for the hydrophilic peptide in the antibody linker section above, preferably 4-14 units of polysarcosine, polyarginine, polyglycine, more preferably 6-12 units of polysarcosine.

In some embodiments, the self-immolative linker J is -J₁-, specifically 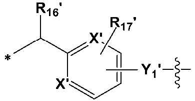 (J-1), for example but not limited to: 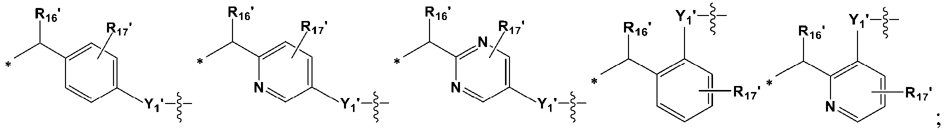
preferably wherein Y₁' is selected from -O- and -NH-, R₁₆' is H, R₁₇' is selected from H or a fragment of formula (A1) 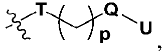
for example but not limited to: :

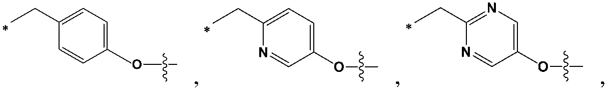

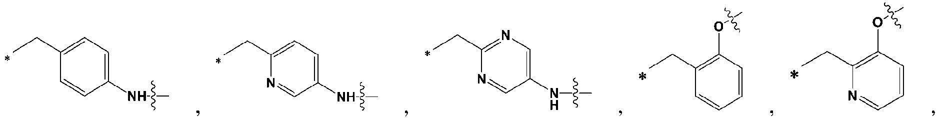

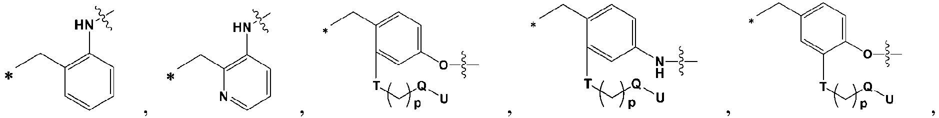

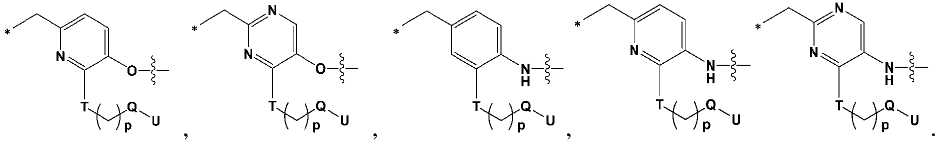

In some embodiments, the self-immolative linker J is -J₁-, specifically 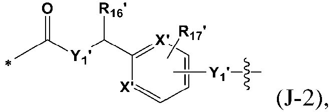 specifically for example: 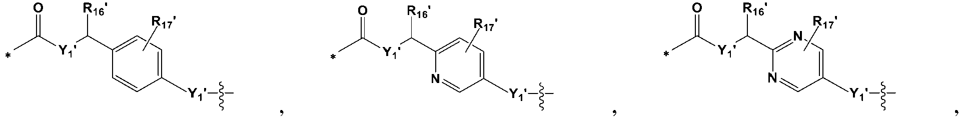 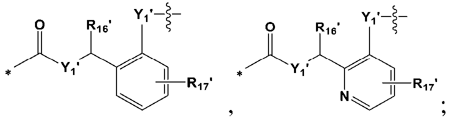 preferably 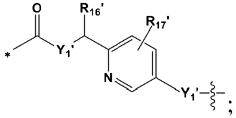
preferably wherein Y₁' is selected from -O- and -NH-, R₁₆' is H, R₁₇' is selected from H or a fragment of formula (A1) 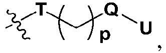
for example but not limited to: 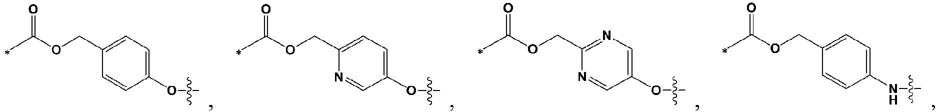 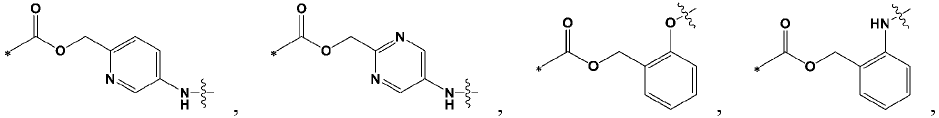 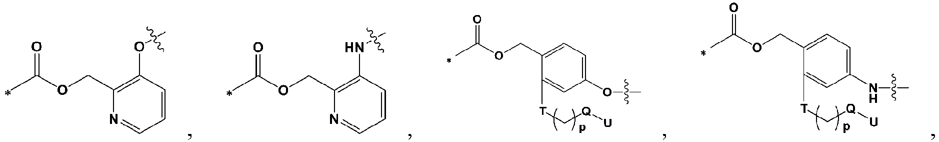 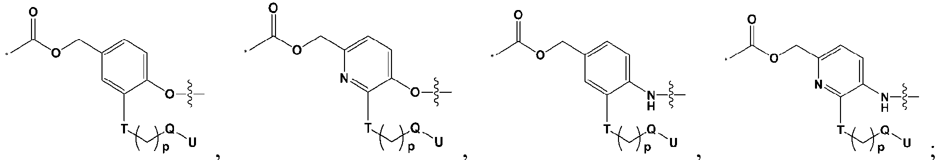 preferably

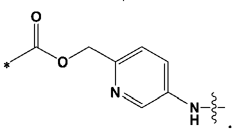

In some embodiments, the self-immolative linker J is -J₂-, specifically 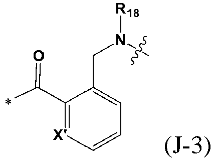 or 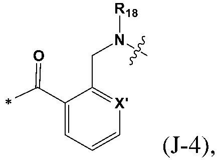 specifically for example: 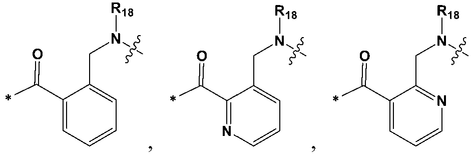 preferably R₁₈ is -C₁₋₃ alkyl, more preferably methyl.

In some embodiments, the self-immolative linker J is -J₂-, specifically 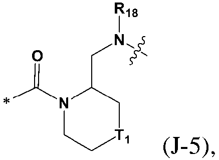 specifically for example: 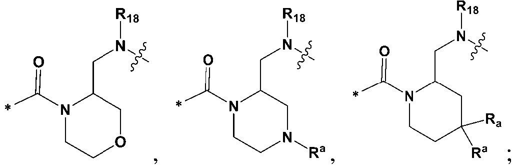 wherein R₁₈ is -C₁₋₆ alkyl, preferably C₁₋₃ alkyl, more preferably methyl, preferably -CH₃), for example but not limited to:

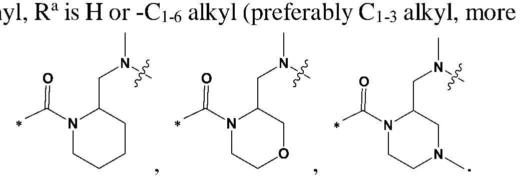

In some embodiments, the self-immolative linker J is -J₂-, specifically 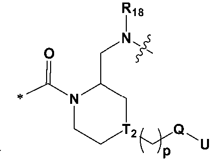 (J-6), preferably R₁₈ is -C₁₋₃ alkyl, more preferably methyl, T₂ is -N-, i.e., 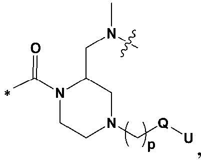 p is 0-4, preferably 0-2; Q is absent or is -NR^{a}- or -O-, preferably absent; U is preferably selected from 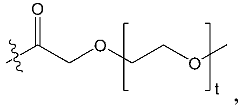 a hydrophilic peptide such as 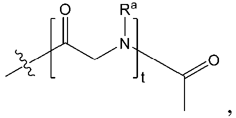 or a uronic acid (preferably a sugar acid of a monosaccharide or disaccharide) or derivative thereof, more preferably a hydrophilic peptide such as 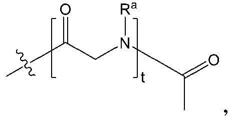 wherein t is preferably 2-14, such as 4-14, 6-12, more preferably 10; for example but not limited to

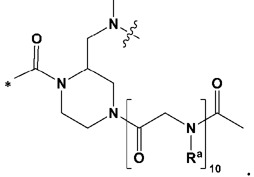

In some embodiments, the self-immolative linker J is -J₂-, specifically 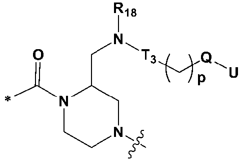 (J-6'), preferably R₁₈ is -C₁₋₃ alkyl, more preferably methyl, T₃ is a dipeptide, tripeptide, or tetrapeptide (preferably the dipeptides, tripeptides, or tetrapeptides defined herein for the cleavable linker B) linked via -CO- to N, p is 0-4, preferably 0-2; Q is absent or is -NR^{a}- or -O-, preferably absent; U is preferably selected from 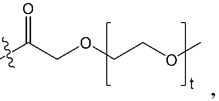 a hydrophilic peptide such as 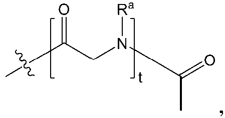 or a uronic acid (preferably a sugar acid of a monosaccharide or disaccharide) or derivative thereof, more preferably a hydrophilic peptide such as 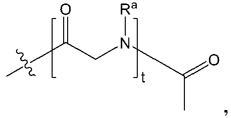 wherein t is preferably 2-14, such as 4-14, 6-12, more preferably 10; for example but not limited to

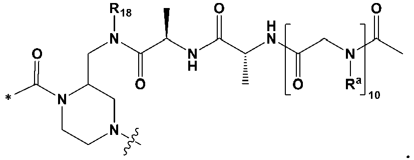

In some embodiments, the self-immolative linker J is -J₂-, specifically 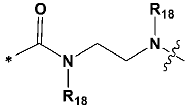 (J-7), preferably R₁₈ is -C₁₋₃ alkyl, more preferably methyl, i.e.,

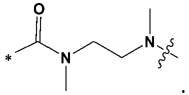

In some embodiments, the self-immolative linker J is -J₁-J₂-, selected from:

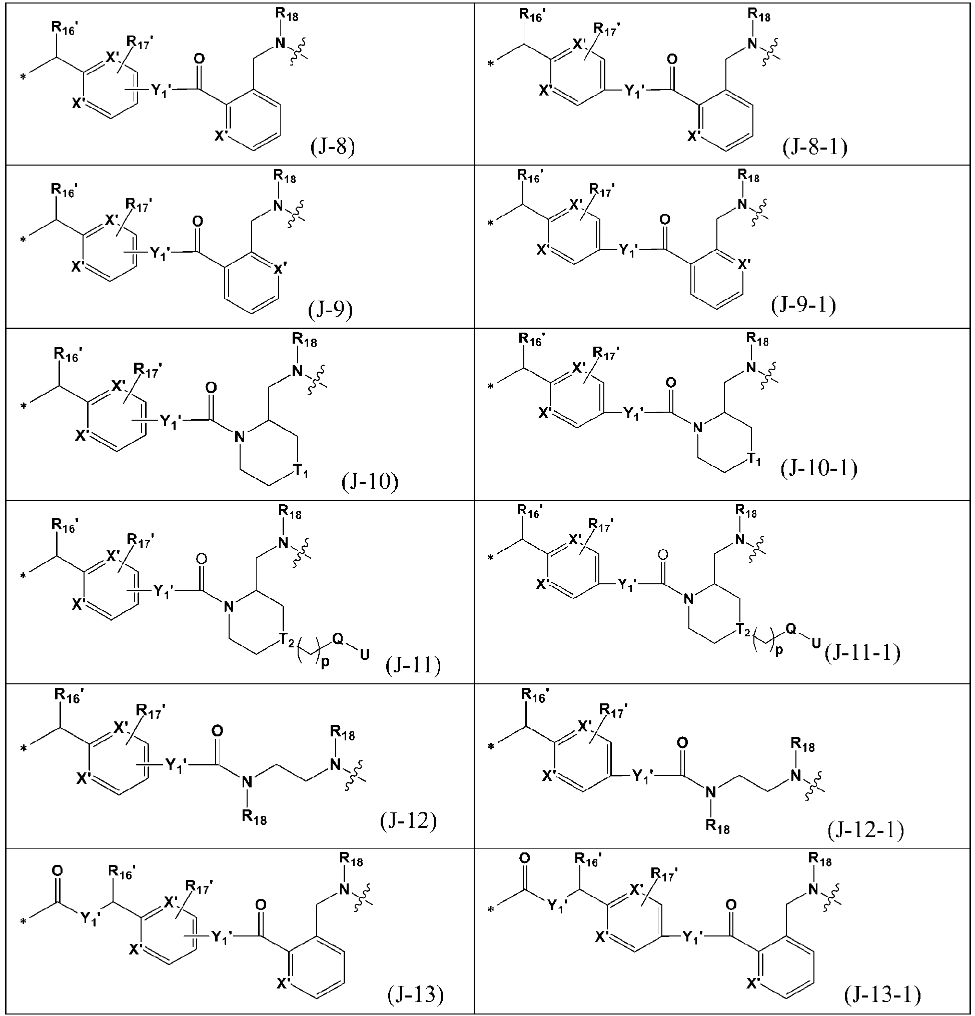

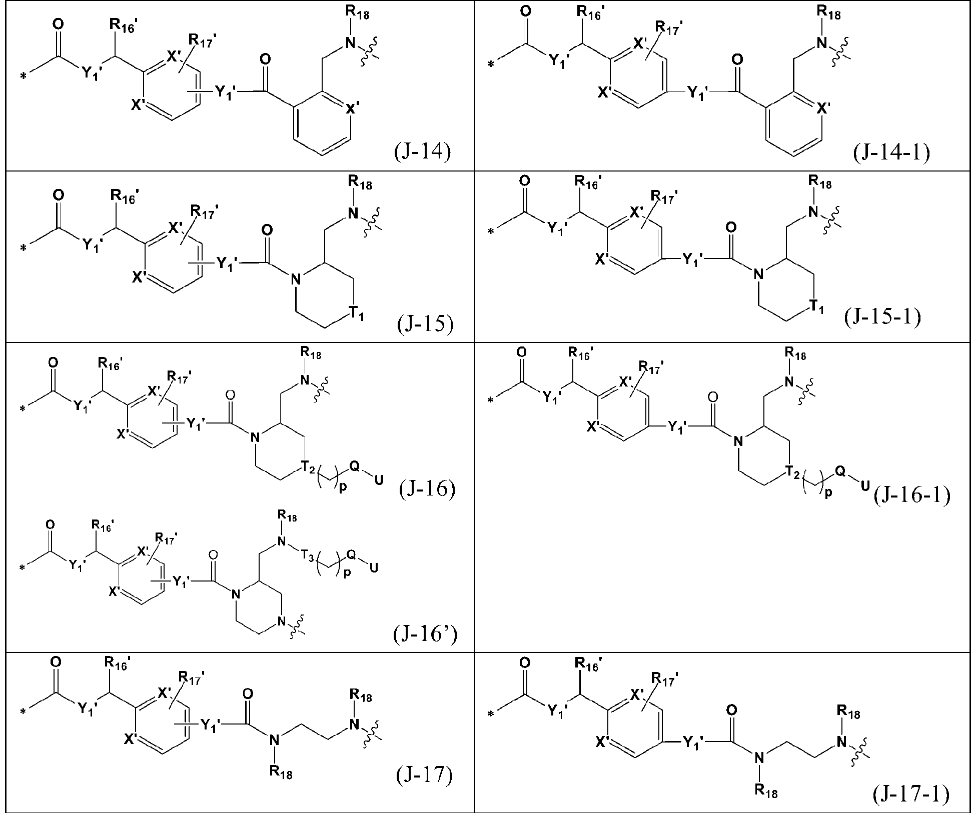

In other embodiments, the self-immolative linker J is -J₂-J₁-, selected from:

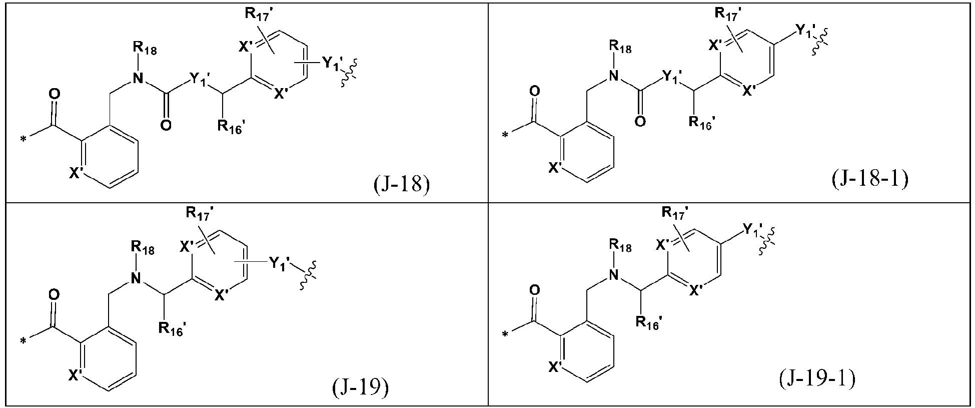

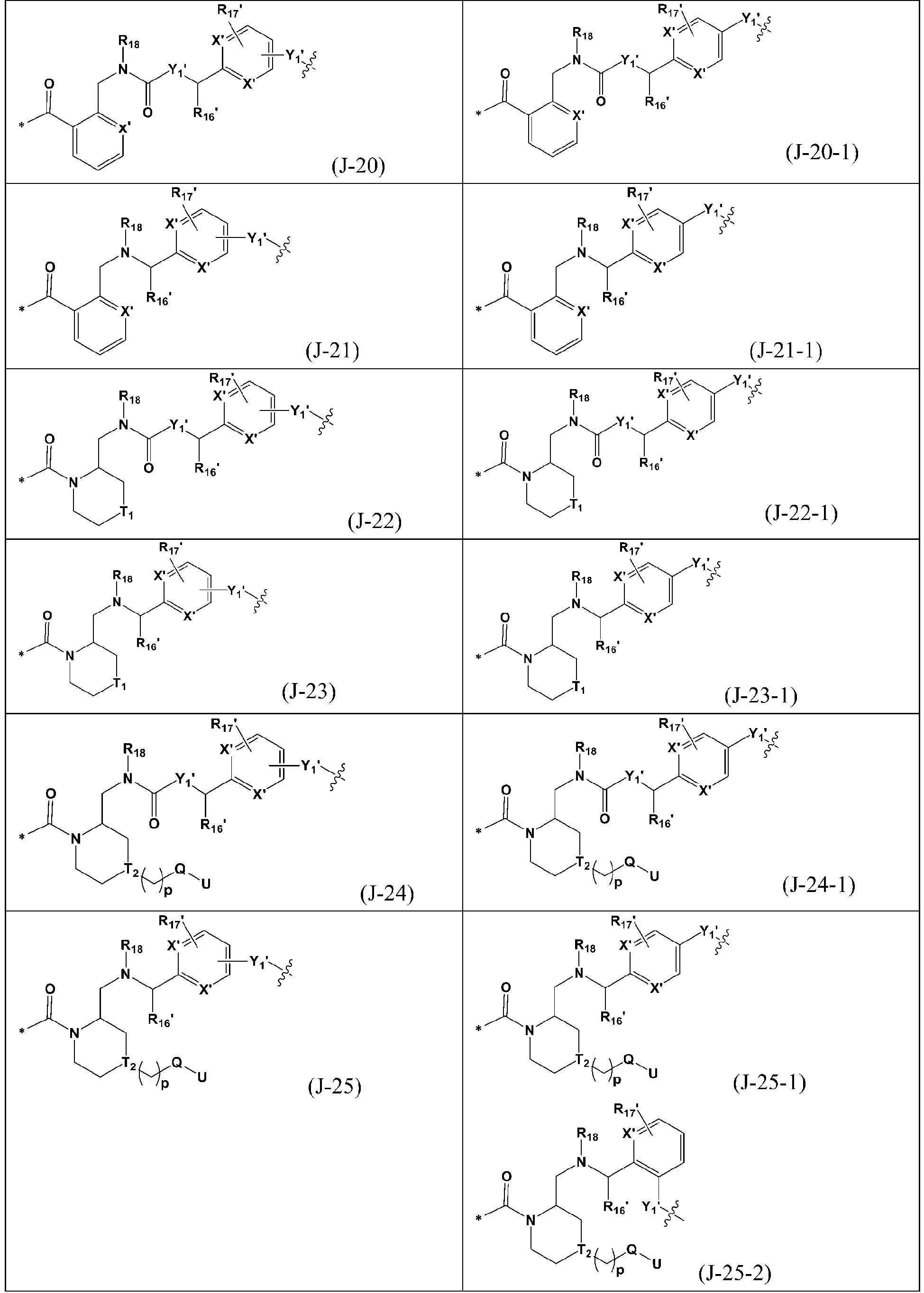

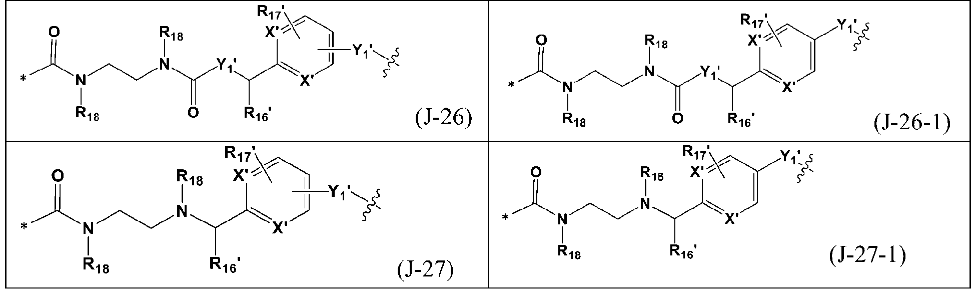

wherein the * indicates the point of attachment to drug P, and the wavy line indicates the point of attachment to the remainder of the linker unit; each J₁ and J₂ component and each substituent therein are as generally or specifically defined above for J₁ and J₂, respectively;

In the various sub-formulas above, the Y₁' connected to the six-membered aromatic ring containing X' is, in some embodiments, attached para to the other attachment point of the ring, and in other embodiments, attached ortho to the other attachment point;

Preferably,
each occurrence of X' is independently selected from -N- or -C-;
each occurrence of Y₁' is independently selected from -NH- or -O-;
each occurrence of R₁₆' is independently selected from H or C₁₋₃ alkyl, preferably H;
each occurrence of R₁₇' is independently selected from H or a fragment of formula (A1) 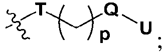
each occurrence of R₁₈ is independently selected from -C₁₋₃ alkyl, preferably methyl;
each occurrence of T is selected from -O-, -NR^{a}-, or is absent;
each occurrence of T₁ is selected from -CH₂-, -O-, -NCH₃-;
each occurrence of T₂ is selected as -N-;
each occurrence of T₃ is a 2-4 peptide linked via the carboxyl terminus to N, selected from Ala-Ala, Cit-Val, Ala-Val, Glu-Gly, Asn-Ala-Ala, Cit-Ala-Glu, Gly-Phe-Gly-Gly;
each occurrence of Q is absent or is -NR^{a}- or -O-;
each occurrence of U is selected from 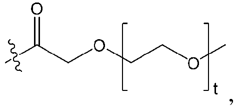 a hydrophilic peptide such as 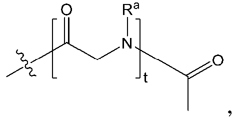 or a glycosyl (preferably a monosaccharide or disaccharide as defined herein or a derivative thereof such as a uronic acid) or derivative thereof, preferably a hydrophilic peptide such as 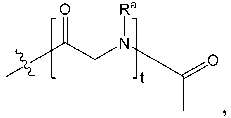 wherein t is 2-14, such as 4-14, 6-12, preferably 10;
each occurrence of p is 0-4, preferably 0-2.

Specifically, -J₁-J₂- or -J₂-J₁- can be any chemically feasible combination of each example of - J₁- shown above with each example of -J₂- shown above.

In some embodiments, J₁ is 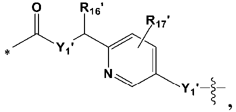 preferably

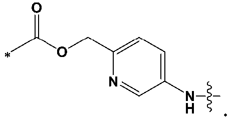

For 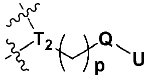 appearing in the self-immolative linker J, in some embodiments, T₂ is - CR^{a}-, e.g., -CH-; in other embodiments, T₂ is -N-; preferably T₂ is -N-. In some embodiments, p is 0-4, e.g., 0-2, such as 0, 2. In some embodiments, Q is absent; in other embodiments, Q is -O- or -NR^{a}-; in other embodiments, Q is -C(O)-.

In a specific embodiment of 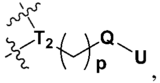 T₂ is -N-, p is 0, Q is absent, U is selected from 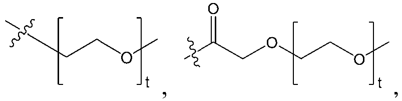 a hydrophilic peptide such as 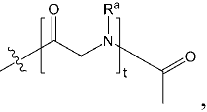 or a glycosyl (preferably a uronic acid) or derivative thereof (preferably a uronic acid of a monosaccharide or disaccharide or derivative thereof), preferably a hydrophilic peptide such as 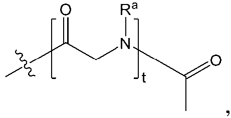 wherein t is preferably 2-14, such as 4-14, 6-12, more preferably 10.

In a specific embodiment of 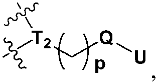 T₂ is -N-, p is selected from 1-4, such as 1-2, Q is -O- or -NR^{a}-, U is selected from 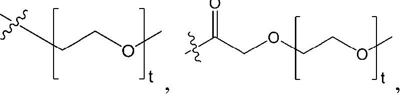 a hydrophilic peptide such as 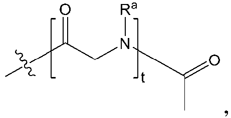 or a glycosyl (preferably a uronic acid) or derivative thereof (preferably a uronic acid of a monosaccharide or disaccharide or derivative thereof), preferably a hydrophilic peptide such as 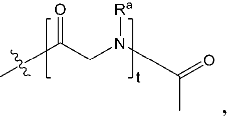 wherein t is preferably 2-14, such as 4-14, 6-12, more preferably 10.

In a specific embodiment of 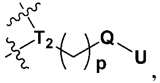 T₂ is -N-, p is selected from 0-4, such as 0-2 or 1-2, Q is -C(O)-, U is selected from 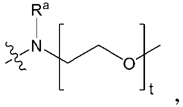 a hydrophilic peptide such as 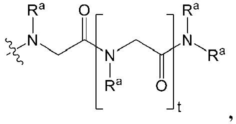 or a glycosyl (preferably an amino sugar) or derivative thereof (preferably an amino monosaccharide or amino disaccharide or derivative thereof).

It should be understood that the fragment of formula (Al) 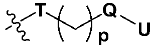 as R₁₇' in the self-immolative linker J has the exact same meaning as the corresponding fragment in formula (A) as a substituent for the G₃ component of the antibody linker, including all general, preferred, or specific embodiments thereof.

In a specific embodiment, in the moiety of formula (A1) 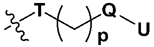 as R₁₇' in the self-immolative linker J, both T and Q are absent, p is 0, and U is -O-glycosyl or a derivative thereof, i.e., the glycosyl is linked to the benzene or pyridine ring in J₁ via a glycosidic bond, preferably to the benzene ring. Examples of the self-immolative linker J carrying a glycosyl or derivative thereof linked via a glycosidic bond include, but are not limited to, those shown in Tables 3-1 and 4-1 in the "A-Solubilizing Sugar Unit" section above.

In a specific embodiment, in the fragment of formula (A1) 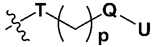 as R₁₇' in the self-immolative linker J, at least one of T and Q is present, or p is not 0.

In other specific embodiments, in the fragment of formula (A1) 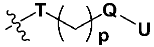 as R₁₇' in the self-immolative linker J, T is -C(R^{a})₂-, -O-, or -NR^{a}-, preferably -O- or -NR^{a}-, more preferably -O-; p is 1-4, preferably 2-4, more preferably 2; Q is absent; U is a glycosyl or derivative thereof linked via a glycosidic bond, preferably the monosaccharide or disaccharide groups or derivatives thereof exemplified in Tables 3 and 4 above. In this case, the moiety of formula (A1) is, for example but not limited to, 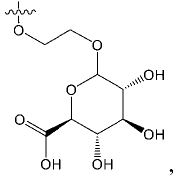 and the self-immolative linker J is, for example but not limited to,

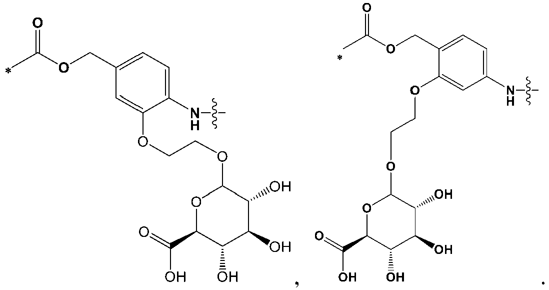

In other specific embodiments, in the fragment of formula (A1) 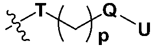 as R₁₇' in the self-immolative linker J, T is -C(R^{a})₂-, -O-, or -NR^{a}-, preferably -O- or -NR^{a}-, more preferably -O-; p is 1-4, preferably 2-4, more preferably 2; Q is -CO-, -O-, or -NR^{a}-, preferably -O- or -NR^{a}-, more preferably -NR^{a}-; U is a glycosyl or derivative thereof, for example a uronic acid or amino sugar or derivative thereof (preferably a uronic acid or derivative thereof linked via a carbonyl group, more preferably the uronic acid derivatives of monosaccharides or disaccharides exemplified in Tables 3 and 4 above). In this case, the fragment of formula (A1) is, for example but not limited to: 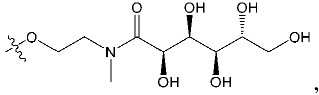 and the self-immolative linker J is, for example but not limited to, 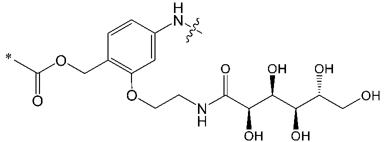 (as in Example 52 or 64), or U is 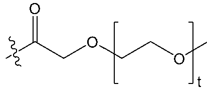 or a hydrophilic peptide such as 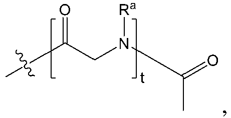 wherein t is preferably 2-14, such as 4-14, 6-12, more preferably 10. In this case, the fragment of formula (A1) is, for example but not limited to: 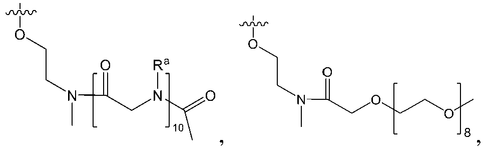 and the self-immolative linker J is, for example but not limited to,

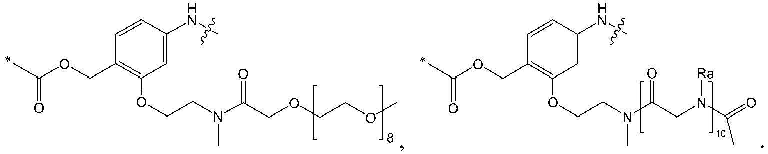

In other specific embodiments, the self-immolative linker J is (J-11), for example 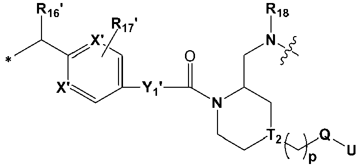 (J-11-1), wherein Y₁' is selected from -O- and -NH-; X' is selected from CH or N, preferably CH; R₁₆' is H; R₁₇' is selected from H or a fragment of formula (A1) 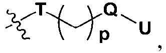 preferably H; R₁₈ is preferably -C₁₋₃ alkyl, more preferably methyl; T₂ is preferably N; p is 0-4, preferably 0-2; Q is absent or is -NR^{a}- or -O-, preferably absent; U is preferably selected from 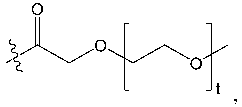 a hydrophilic peptide such as 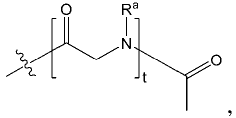 or a glycosyl (preferably a uronic acid, more preferably a uronic acid of a monosaccharide or disaccharide) or derivative thereof, more preferably a hydrophilic peptide such as 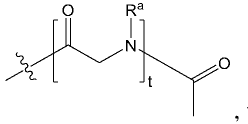 wherein t is preferably 2-14, such as 4-14, 6-12, more preferably 10; for example but not limited to

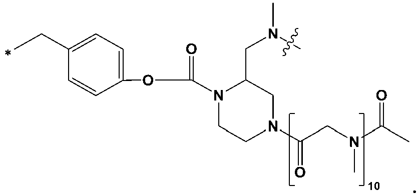

In other specific embodiments, the self-immolative linker J is (J-13), for example 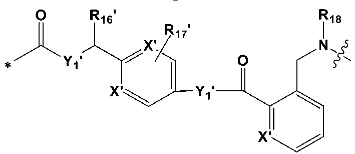 (J-13-1), wherein Y₁' is selected from -O- and -NH-; X' is selected from CH or N, preferably CH; R₁₆' is H; R₁₇' is selected from H or a fragment of formula (A1) 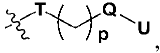 preferably H; R₁₈ is preferably -C₁₋₃ alkyl, more preferably methyl, for example but not limited to

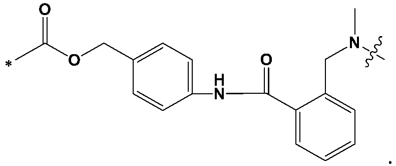

In other specific embodiments, the self-immolative linker J is (J-15), for example 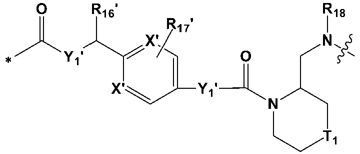 (J-15-1), wherein Y₁' is selected from -O- and -NH-; X' is selected from CH or N; R₁₆' is H; R₁₇' is selected from H or a fragment of formula (A1) 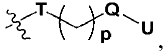 preferably H; R₁₈ is preferably -C₁₋₃ alkyl, more preferably methyl; T₁ is selected from O, NR^{a}, C(R^{a})₂; for example but not limited to

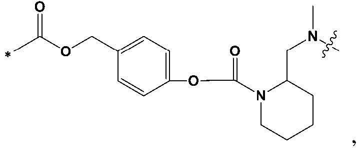

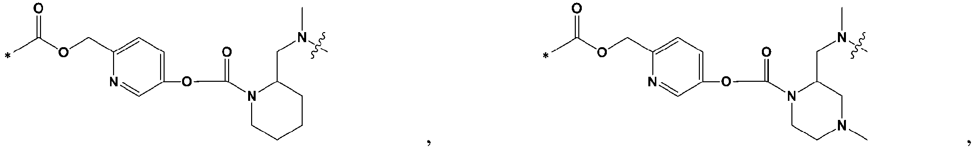

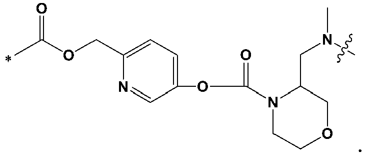

In other specific embodiments, the self-immolative linker J is (J-16), for example 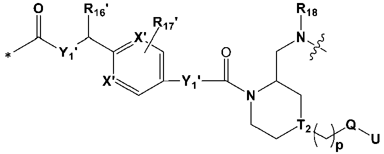 (J-16-1), wherein Y₁' is selected from -O- and -NH-; X' is selected from CH or N; R₁₆' is H; R₁₇' is selected from H or a fragment of formula (A1) 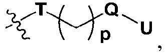 preferably H; R₁₈ is preferably -C₁₋₃ alkyl, more preferably methyl; T₂ is preferably N; p is 0-4, preferably 0-2; Q is absent or is -NR^{a}- or -O-, preferably absent; U is preferably selected from 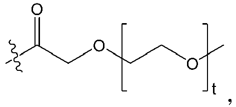 a hydrophilic peptide such as 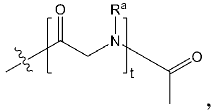 or a glycosyl or derivative thereof (preferably a uronic acid, more preferably a uronic acid of a monosaccharide or disaccharide) or derivative thereof, more preferably a hydrophilic peptide such as 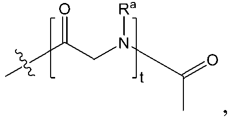 wherein t is preferably 2-14, such as 4-14, 6-12, more preferably 10; for example but not limited to

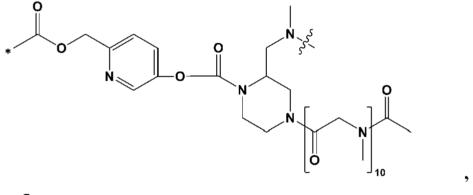

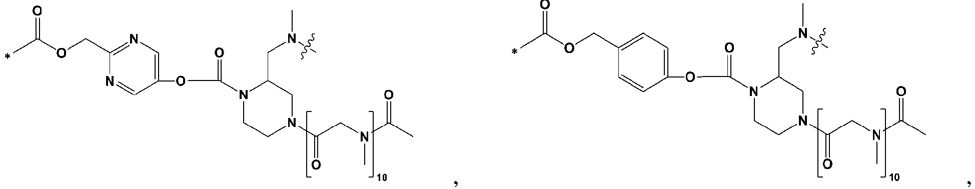

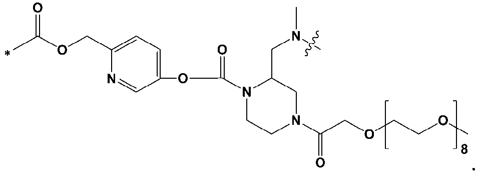

In other specific embodiments, the self-immolative linker J is 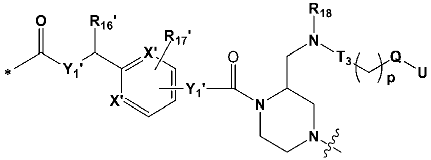 (J-16'), wherein Y₁' is selected from -O- and -NH-; X' is selected from CH or N; R₁₆' is H; R₁₇' is selected from H or a fragment of formula (A1) 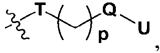 preferably H; R₁₈ is preferably -C₁₋₃ alkyl, more preferably methyl; T₃ is a dipeptide, tripeptide, or tetrapeptide (preferably the dipeptides, tripeptides, or tetrapeptides defined herein for the cleavable linker B, more preferably selected from Ala-Ala, Cit-Val, Ala-Val, Glu-Gly, Asn-Ala-Ala, Cit-Ala-Glu, Gly-Phe-Gly-Gly) linked via -CO- to N, p is 0-4, preferably 0-2; Q is absent or is -NR^{a}- or -O-, preferably absent; U is preferably selected from 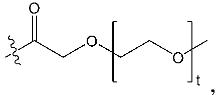 a hydrophilic peptide such as 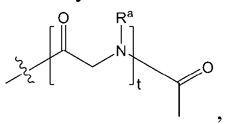 or a uronic acid (preferably a uronic acid of a monosaccharide or disaccharide) or derivative thereof, more preferably a hydrophilic peptide 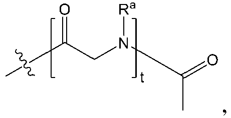 wherein t is preferably 2-14, such as 4-14, 6-12, more preferably 10; for example but not limited to

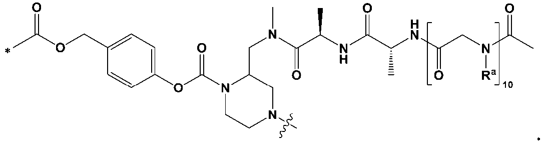

In other specific embodiments, the self-immolative linker J is (J-17), for example 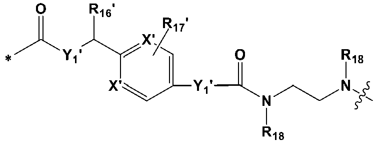 (J-17-1), wherein Y₁' is selected from -O- and -NH-; X' is selected from CH or N; R₁₆' is H; R₁₇' is selected from H or a fragment of formula (A1) 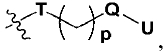 preferably H; R₁₈ is preferably -C₁₋₃ alkyl, more preferably methyl; for example but not limited to

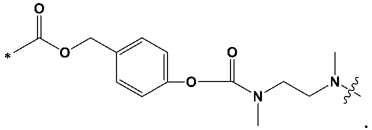

In other specific embodiments, the self-immolative linker J is (J-18), for example 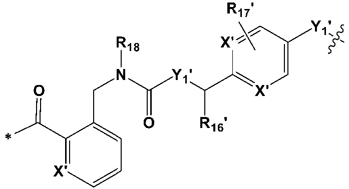 (J-18-1), wherein Y₁' is selected from -O- and -NH-; X' is selected from CH or N; R₁₆' is H; R₁₇' is selected from H or a fragment of formula (A1) 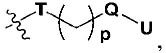 preferably H; R₁₈ is preferably -C₁₋₃ alkyl, more preferably methyl; for example but not limited to

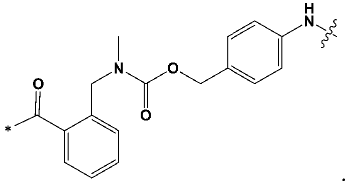

In other specific embodiments, the self-immolative linker J is (J-24), for example 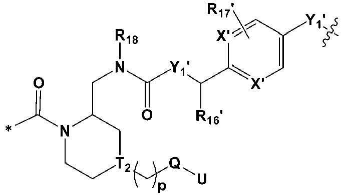 (J-24-1), wherein Y₁' is selected from -O- and -NH-; X' is selected from CH or N; R₁₆' is H; R₁₇' is selected from H or a fragment of formula (A1) 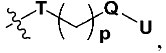 preferably H; R₁₈ is preferably -C₁₋₃ alkyl, more preferably methyl; T₂ is preferably N; p is 0-4, preferably 0-2; Q is absent or is -NR^{a}- or -O-, preferably absent; U is preferably selected from 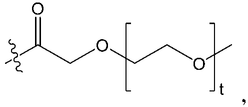 a hydrophilic peptide such as 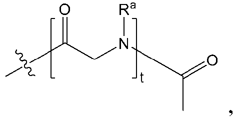 or a glycosyl (preferably a uronic acid, more preferably a uronic acid of a monosaccharide or disaccharide) or derivative thereof, more preferably a hydrophilic peptide such as 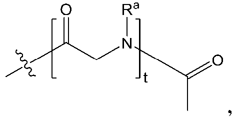 wherein t is preferably 2-14, such as 4-14, 6-12, more preferably 10; for example but not limited to

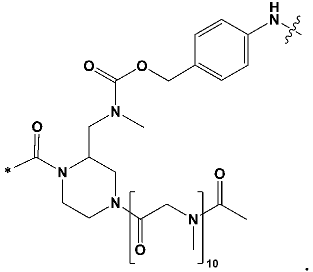

In other specific embodiments, the self-immolative linker J is 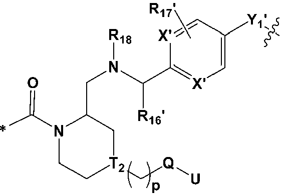 (J-25) or 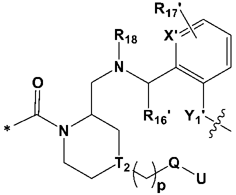 (J-25-1), wherein Y₁' is selected from -O- and -NH-; X' is selected from CH or N; R₁₆' is H; R₁₇' is selected from H or a fragment of formula (A1) 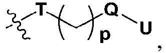 preferably H; R₁₈ is preferably -C₁₋₃ alkyl, more preferably methyl; T₂ is preferably N; p is 0-4, preferably 0-2; Q is absent or is -NR^{a}- or -O-, preferably absent; U is preferably selected from 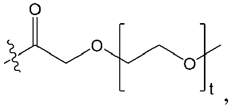 a hydrophilic peptide such as 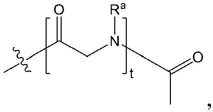 or a glycosyl (preferably a uronic acid, more preferably a uronic acid of a monosaccharide or disaccharide) or derivative thereof, more preferably a hydrophilic peptide such as 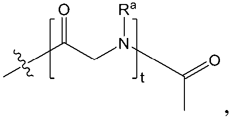 wherein t is preferably 2-14, such as 4-14, 6-12, more preferably 10; for example but not limited to

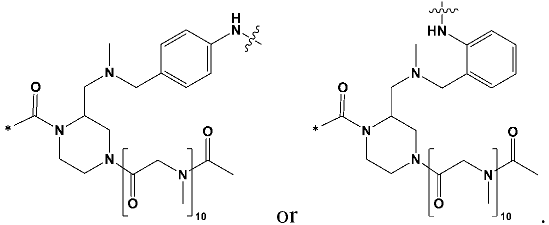

It should be understood that the ADC of the present disclosure encompasses ADC technical solutions formed by combining each self-immolative linker J as generally, specifically, or preferably defined above with the general, specific, or preferred definitions of any other components in the ADC as defined herein.

When the ADC of the present disclosure is represented as [P-J-B(D¹)-D-E(D¹)-G]_{q}-Ab, J therein is preferably selected from (J-1), (J-2), (J-3), (J-4), (J-6), (J-6'), (J-11), (J-14), (J-15), (J-16), (J-16'), (J-17), (J-18), (J-20), (J-24), (J-25) as defined above, and their respective sub-formulas or specific embodiments.

### Specific Technical Solutions for the Linker Unit L

It should be noted that the linker unit L of the ADC of the present disclosure includes any combination of the general or specific definition of any one of the components of the linker unit with the general or specific definitions of one or more of the remaining components.

In some embodiments, the linker unit L in the ADC of formula (X) of the present disclosure has the general formulas shown in **Table** 6 below:

| | |
|---|---|
| (II-1) | -A(S)-B-D-E-G- |
| (II-2) | -A(S)-B-E-G- |
| (II-3) | -A(S)-B-G- |
| (II-4) | -A-B-G- |
| (II-5) | -B-G- |
| (II-6) | -B-E-G- |
| (II-7) | -B-D-E-G- |
| (II-8) | -A(S)-G- |

wherein A, S, B, D, E, and G are each as generally or specifically defined in the respective sections above.

In some embodiments, in the linker unit formulas (II), (II-1), (11-2), (II-3), (II-4), (II-5), (II-6), (11-7), or (11-8) of the ADC of the present disclosure,
A is selected from 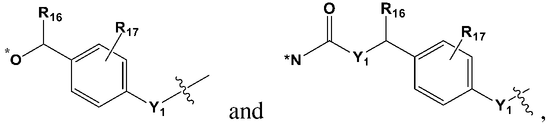 wherein the end marked with * is connected to the drug P, and the N or O marked with * is an atom from the drug P; Y₁ is each independently N or O, R₁₆ is H, and R₁₇ is H, or is a fragment of formula (A1) as generally or specifically defined herein for the self-immolative linker J part: 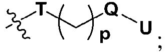
S is selected from glyceraldehyde, erythrose, erythrulose, arabinose, ribose, ribulose, xylose, xylulose, lyxose, glucose, mannose, allose, psicose, altrose, quinovose, rhamnose, fructose, sorbose, tagatose, gulose, idose, galactose, talose, lactose, maltose, sucrose, or their respective phosphate esters, sugar alcohols, uronic acids, deoxy sugars, amino sugars, acylated amino sugars, and/or glycoside forms;
Preferably, S is selected from glucose, galactose, mannose, galacturonic acid, glucuronic acid, mannuronic acid, acetylgalactosamine, acetylglucosamine, acetylmannosamine, acetylgalactosaminuronic acid, acetylglucosaminuronic acid, acetylmannosaminuronic acid; lactose, maltose, sucrose, or their respective acetamido derivatives, uronic acid derivatives, acetamido uronic acid derivatives;
Further preferably, S is selected from D-glucose, D-galactose, D-mannose, D-galacturonic acid, D-glucuronic acid, D-mannuronic acid, 2-acetamido-2-deoxy-D-galactose, 2-acetamido-2-deoxy-D-glucose, 2-acetamido-2-deoxy-D-mannose, 2-acetamido-2-deoxy-D-galacturonic acid, 2-acetamido-2-deoxy-D-glucuronic acid, 2-acetamido-2-deoxy-D-mannuronic acid; D-lactose, D-maltose, D-sucrose, or their respective 2-acetamido and/or 6-carboxy derivatives;
B is of formula (B-1): 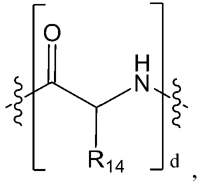 wherein the N-terminus is connected to D (when present), E (when present), or the G part of the linker unit, and the C-terminus is connected to the A part of the linker unit or directly to the drug P;
Preferably, B is of formula (B-2): -(AA)_{d}-, wherein
d is an integer from 2 to 12, preferably an integer from 2 to 8, e.g., 2 to 6, 2 to 4, 2 to 3;
R₁₄ in each occurrence is independently selected from hydrogen, methyl, isopropyl, isobutyl, sec-butyl, benzyl, -CH₂CONH₂, -CH₂CH₂COOH, or -(CH₂)₃NHCONH₂;
AA is an amino acid selected from: alanine, glycine, asparagine, isoleucine, leucine, valine, phenylalanine, citrulline, glutamic acid; preferably selected from alanine, glycine, asparagine, valine, phenylalanine, citrulline, glutamic acid; more preferably -(AA)_{d}- is the following polypeptide from C-terminus to N-terminus: Ala-Ala, Cit-Val, Ala-Val, Glu-Gly, Asn-Ala-Ala, Cit-Ala-Glu, Gly-Phe-Gly-Gly: wherein the N-terminus of the peptide is connected to the G part of the linker unit, and the C-terminus of the peptide is directly connected to the drug P;
D is selected from 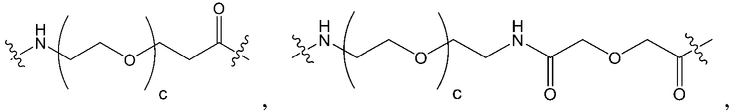 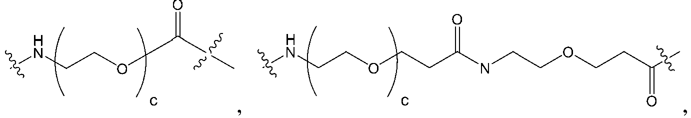 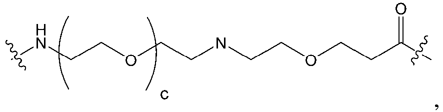 wherein the amino terminus is connected to E (when present) or G, the carbonyl terminus is connected to B, and each subscript c is independently selected from integers 2-12;
E is 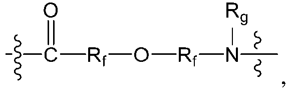 wherein the carbonyl terminus is connected to B, the amino terminus is connected to G, R_{f} is each independently selected from -C₁₋₆ alkylene-, preferably -C₂₋₄ alkylene, R_{g} is H or C₁₋₃ alkyl, most preferably 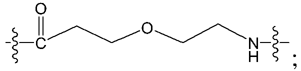
G is selected from 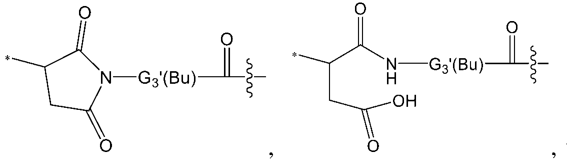 wherein the * end indicates the point of attachment to the antibody Ab (via the G₁ atom in Ab responsible for attachment to L), the 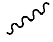 end indicates the point of attachment to the linker unit, G₃' is -C₁-₁₀ alkylene-, preferably -C₁-₅ alkylene-, Bu is H, or is a fragment of formula (A):

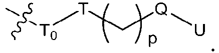

In a specific embodiment, the linker unit of the ADC of the present disclosure has formula (II-3): -A(S)-B-G-, wherein:
A is selected from 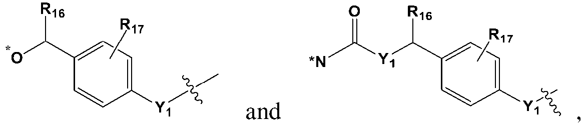 preferably 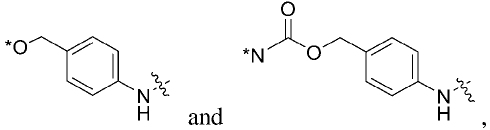 or wherein R₁₇ is correspondingly a fragment of formula (A1) 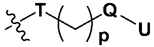 as generally or specifically defined herein for the self-immolative linker J part, wherein the end marked with * is connected to the drug P, and the N or O marked with * is an atom from the drug P;
S is selected from glucose, galactose, mannose, galacturonic acid, glucuronic acid, mannuronic acid, acetylgalactosamine, acetylglucosamine, acetylmannosamine, acetylgalactosaminuronic acid, acetylglucosaminuronic acid, acetylmannosaminuronic acid; lactose, maltose, sucrose, or their respective acetamido derivatives, uronic acid derivatives, acetamido uronic acid derivatives; preferably S is selected from D-glucose, D-galactose, D-mannose, D-galacturonic acid, D-glucuronic acid, D-mannuronic acid, 2-acetamido-2-deoxy-D-galactose, 2-acetamido-2-deoxy-D-glucose, 2-acetamido-2-deoxy-D-mannose, 2-acetamido-2-deoxy-D-galacturonic acid, 2-acetamido-2-deoxy-D-glucuronic acid, 2-acetamido-2-deoxy-D-mannuronic acid; D-lactose, D-maltose, D-sucrose, or their respective 2-acetamido and/or 6-carboxy derivatives;
B is of formula (B-2): -(AA)_{d}-, wherein
d is an integer from 2 to 12, preferably an integer from 2 to 4;
AA is an amino acid selected from: alanine, glycine, asparagine, valine, phenylalanine, citrulline, glutamic acid; preferably -(AA)_{d}- is the following polypeptide from C-terminus to N-terminus: Ala-Ala, Cit-Val, Ala-Val, Glu-Gly, Asn-Ala-Ala, Cit-Ala-Glu, Gly-Phe-Gly-Gly, as specifically exemplified above; wherein the N-terminus of the peptide is connected to the G part, and the C-terminus of the peptide is connected to the self-immolative linker A;
G is selected from 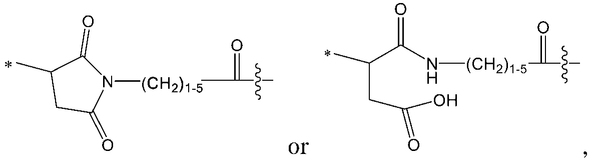 or G is selected from 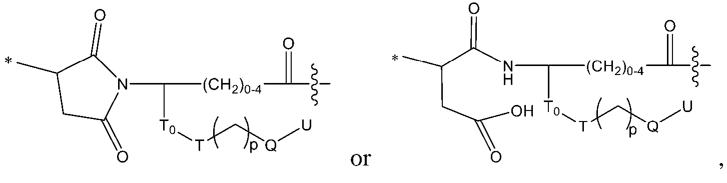 wherein the * end indicates the point of attachment to the antibody Ab (via the G₁ atom in Ab responsible for attachment to L), the 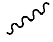 end indicates the point of attachment to the linker unit;
Thus, the linker unit of formula (II-3) is, for example, of formula (II-3-1): 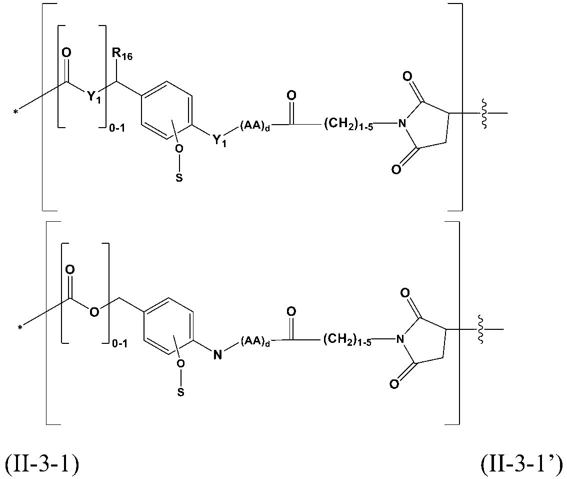 and the corresponding fragments where the maleimide moiety is

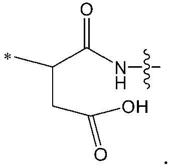

In a specific embodiment, the linker unit of the ADC of the present disclosure has formula (II-4): -A-B-G-, wherein:
A is selected from 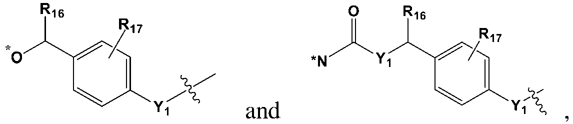 preferably 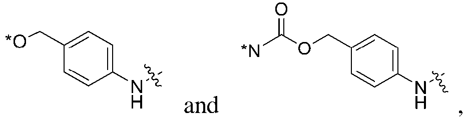 or wherein R₁₇ is correspondingly a fragment of formula (A1) 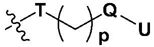 as generally or specifically defined herein for the self-immolative linker J part, wherein the end marked with * is connected to the drug P, and the N or O marked with * is an atom from the drug P;
B is of formula (B-2): -(AA)_{d}-, wherein
d is an integer from 2 to 12, preferably an integer from 2 to 4;
AA is an amino acid selected from: alanine, glycine, asparagine, valine, phenylalanine, citrulline, glutamic acid; preferably -(AA)_{d}- is the following polypeptide from C-terminus to N-terminus: Ala-Ala, Cit-Val, Ala-Val, Glu-Gly, Asn-Ala-Ala, Cit-Ala-Glu, Gly-Phe-Gly-Gly; wherein the N-terminus of the peptide is connected to the G part of the linker unit, and the C-terminus of the peptide is connected to the self-immolative linker A;
G is selected from 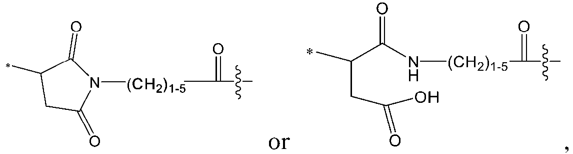 or G is selected from 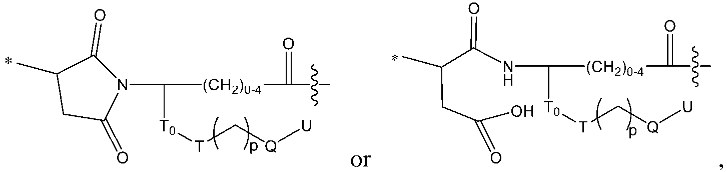 wherein the * end indicates the point of attachment to the antibody Ab (via the G₁ atom in Ab responsible for attachment to L), the 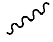 end indicates the point of attachment to the linker unit;
Thus, the linker unit of formula (II-4) has, for example, formula (II-4-1): 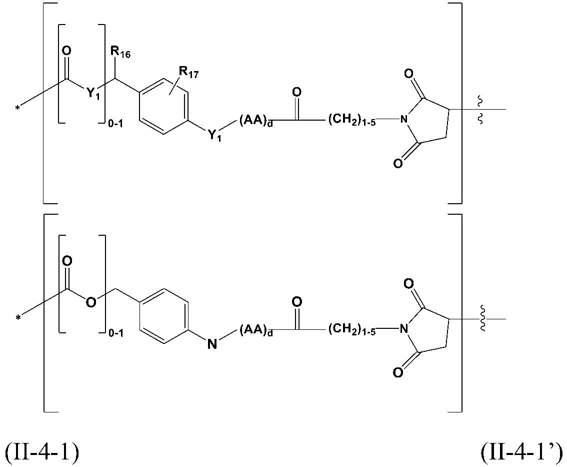 and the corresponding fragments where the maleimide moiety is

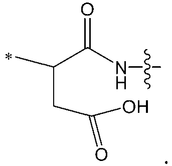

In another specific embodiment, the linker unit of the ADC of the present disclosure has formula (II-5): -B-G-, wherein:
B is of formula (B-2): -(AA)_{d}-, wherein
d is an integer from 2 to 12, preferably an integer from 2 to 4;
AA is an amino acid selected from: alanine, glycine, asparagine, valine, phenylalanine, citrulline, glutamic acid; preferably -(AA)_{d}- is the following polypeptide from C-terminus to N-terminus: Ala-Ala, Cit-Val, Ala-Val, Glu-Gly, Asn-Ala-Ala, Cit-Ala-Glu, Gly-Phe-Gly-Gly; wherein the N-terminus of the peptide is connected to the G part of the linker unit, and the C-terminus of the peptide is directly connected to the drug P;
G is selected from 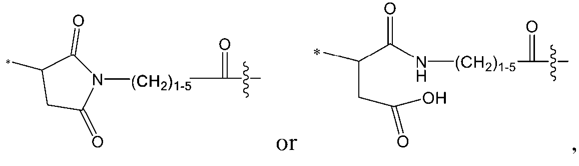 or G is selected from 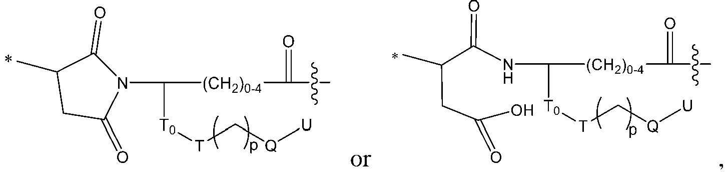 wherein the * end indicates the point of attachment to the antibody Ab (via the G₁ atom in Ab responsible for attachment to L), the 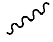 end indicates the point of attachment to the linker unit;
Thus, the linker unit of formula (II-5) has, for example, formula (II-5-1): 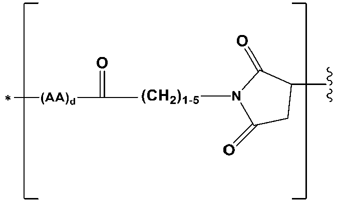 (11-5-1) and the corresponding fragments where the maleimide moiety is

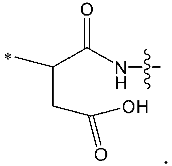

In other embodiments, the linker unit L of formula (II') in the ADC of formula (X) of the present disclosure has the general formulas shown in Table 6-1 below:

| | |
|---|---|
| (II'-1) | -J-B-D-E-G- |
| (II'-2) | -J-B-E-G- |
| (II'-3) | -J-B-G- |
| (II'-4) | -J-G- |

wherein J, B, D, E, and G are each as generally or specifically defined for the respective parts above.

In some embodiments, in the linker unit formulas (II'), (II'-1), (II'-2), (II'-3), (II'-4) of the ADC of the present disclosure,
J is as defined in the **"J---Self-Immolative** Linker" section above, selected from -J₁-, -J₂-, -J₁-J₂-, -J₂-J₁-, specifically selected from the formulas (J-1), (J-2), (J-3), (J-4), (J-5), (J-6), (J-6'), (J-7), (J-8), (J-9), (J-10), (J-11), (J-12), (J-13), (J-14), (J-15), (J-16), (J- 16'), (J-17), (J-18), (J-19), (J-20), (J-21), (J-22), (J-23), (J-24), (J-25), (J-26), (J-27) as generally or specifically defined above, and their respective sub-formulas or specific embodiments, preferably selected from (J-1), (J-2), (J-3), (J-4), (J-6), (J-6'), (J-11), (J-14), (J-15), (J-16), (J-16'), (J-17), (J-18), (J-20), (J-24), (J-25), and their respective sub-formulas or specific embodiments;
B is of formula (B-2): -(AA)_{d}-, wherein
d is an integer from 2 to 12, preferably an integer from 2 to 4;
preferably -(AA)_{d}- is the following polypeptide from C-terminus to N-terminus: Ala-Ala, Cit-Val, Ala-Val, Glu-Gly, Asn-Ala-Ala, Cit-Ala-Glu, Gly-Phe-Gly-Gly; wherein the N-terminus of the peptide is connected to D (when present), E (when present and D is absent), or G (when both D and E are absent) of the linker unit, and the C-terminus of the peptide is connected to J;
D and E are respectively as defined for the sub-formulas in Table 6 above;
G is 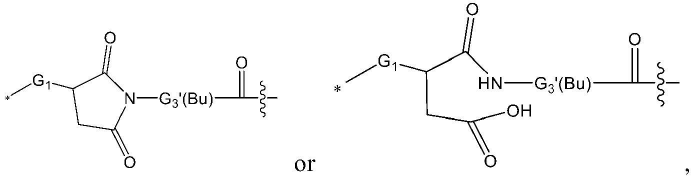 wherein G₁ is the atom in Ab responsible for attachment to L, G₃' is-C₁-₁₀ alkylene-, preferably -C₁-₅ alkylene-, Bu is absent or is a structural fragment of formula (A); preferably, G is 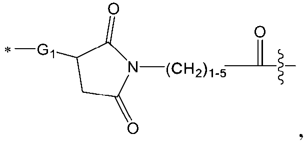 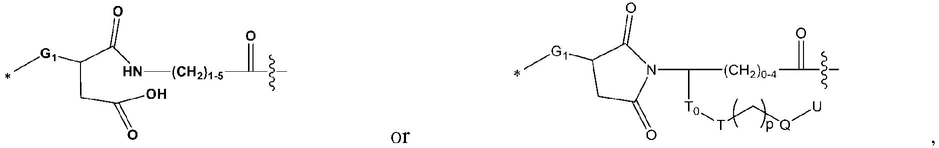 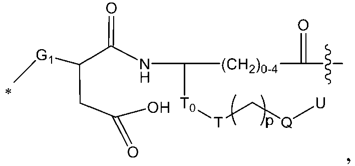 wherein T₀, T, Q, U, p are respectively as defined for formula (A), preferably, wherein T₀ is -C₁₋₄ alkylene- (e.g., -C₁₋₂ alkylene-, such as methylene), T is absent or is - C(R^{a})₂-, p is 0-4 (e.g., 0-2) and Q is selected from -O- or -NR^{a}-, and U is preferably selected from a uronic acid (preferably a uronic acid of a monosaccharide or disaccharide as defined herein) or derivative thereof linked via a carbonyl group, 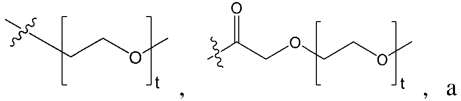 hydrophilic peptide such as 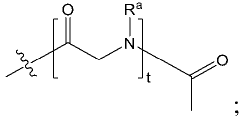 or wherein T₀ is -C₁₋₄ alkylene- (e.g., -C₁₋₂ alkylene-, such as methylene), T is absent or is -C(R^{a})₂-, p is 0-4 (e.g., 0-2) and Q is -CO-, U is preferably selected from an amino sugar (preferably an amino monosaccharide or amino disaccharide) or derivative thereof linked via an amino group, 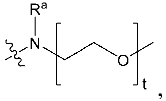 or a hydrophilic peptide such as 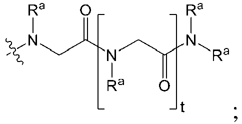 or wherein T₀ is -C₁₋₄ alkylene- (e.g., -C₁₋₂ alkylene-, such as methylene), T is selected from -C(R^{a})₂-, -O-, -NR^{a}-, or is absent, Q is absent, p is 0-4 (e.g., 0-2, 1-2), and U is preferably selected from a glycosyl (preferably a monosaccharide or disaccharide) or derivative thereof linked via a glycosidic bond, or an amino sugar (preferably an amino monosaccharide or amino disaccharide) or derivative thereof linked via an amino group; wherein t is an integer from 1~20, e.g., 2-10, 2-8, 2-6, 2-4, 4-10, 4-14, 6-10, 6-12, 8-10, 8-14.

It should be noted that when U appearing in the linker unit is a hydrophilic peptide, it is as defined accordingly herein, more specifically as generally or specifically defined for the hydrophilic peptide in the antibody linker section above, preferably 4-14 units of polysarcosine, polyarginine, or polyglycine, more preferably 6-12 units of polysarcosine.

In some embodiments, the linker unit L of the ADC of the present disclosure has the general formula (II'-3'): 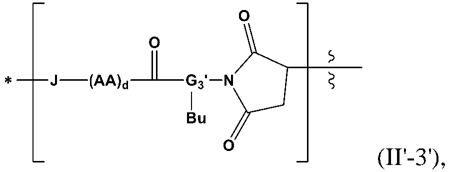 specifically: 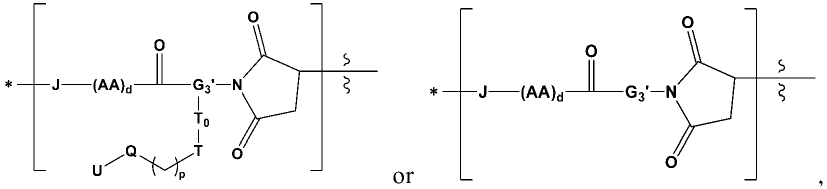 or has the general formula (II'-4'): 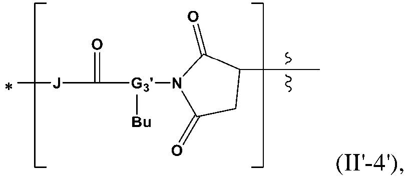 specifically 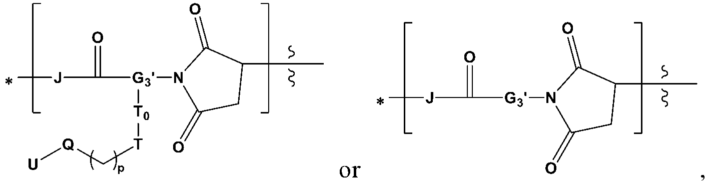
wherein -(AA)_{d}-, Bu, G₃', T, Q, U, p are as generally or specifically defined above;
J is as generally or specifically defined above, preferably selected from (J-1), (J-2), (J-3), (J-4), (J-6), (J-6'), (J-11), (J-14), (J-15), (J-16), (J-16'), (J-17), (J-18), (J-20), (J-24), (J-25), or their respective sub-formulas;
and the corresponding fragments where the maleimide moiety is

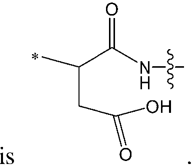

The linker unit L of formula (II'-3') or (II'-4') is, for example but not limited to:

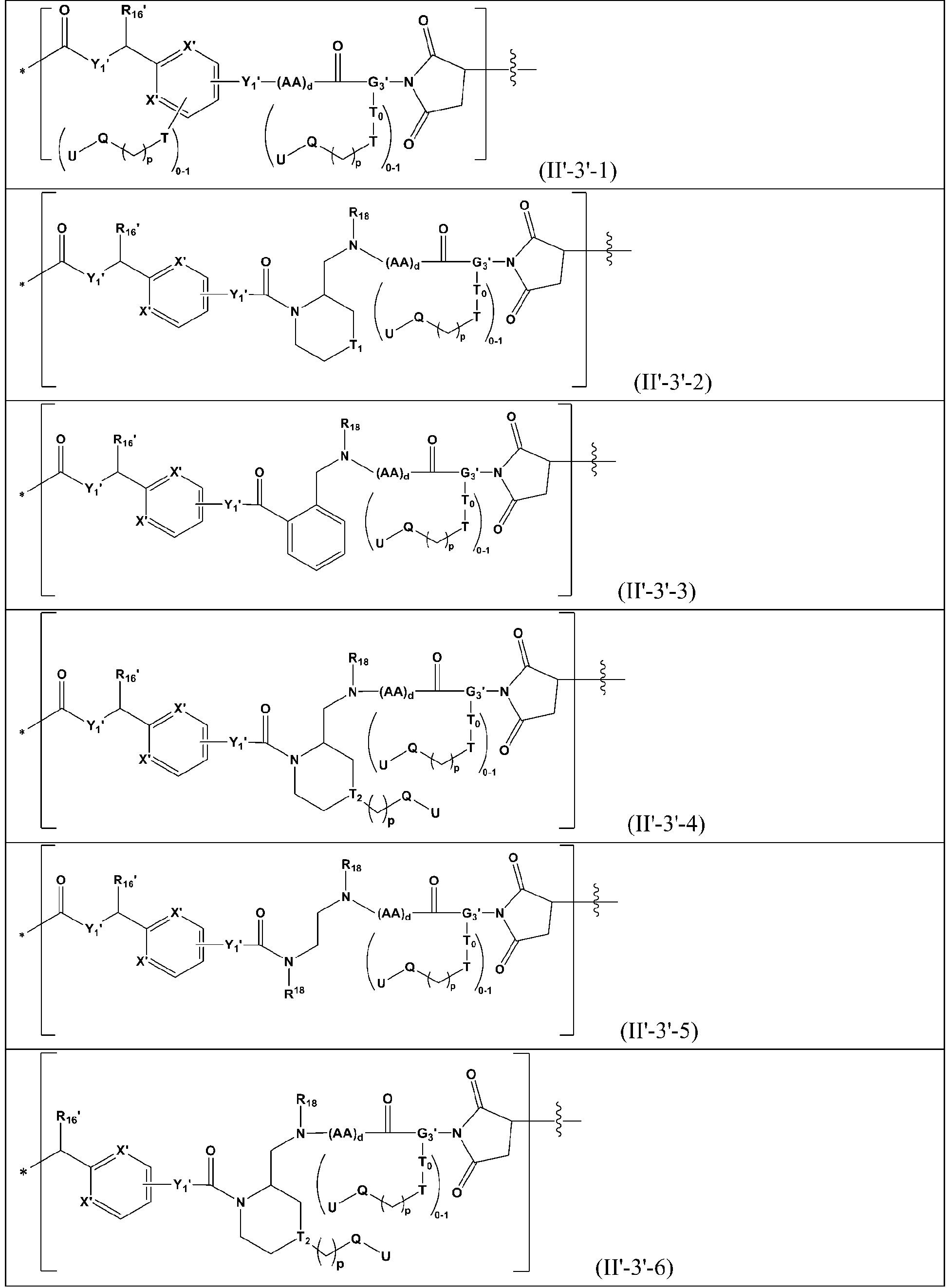

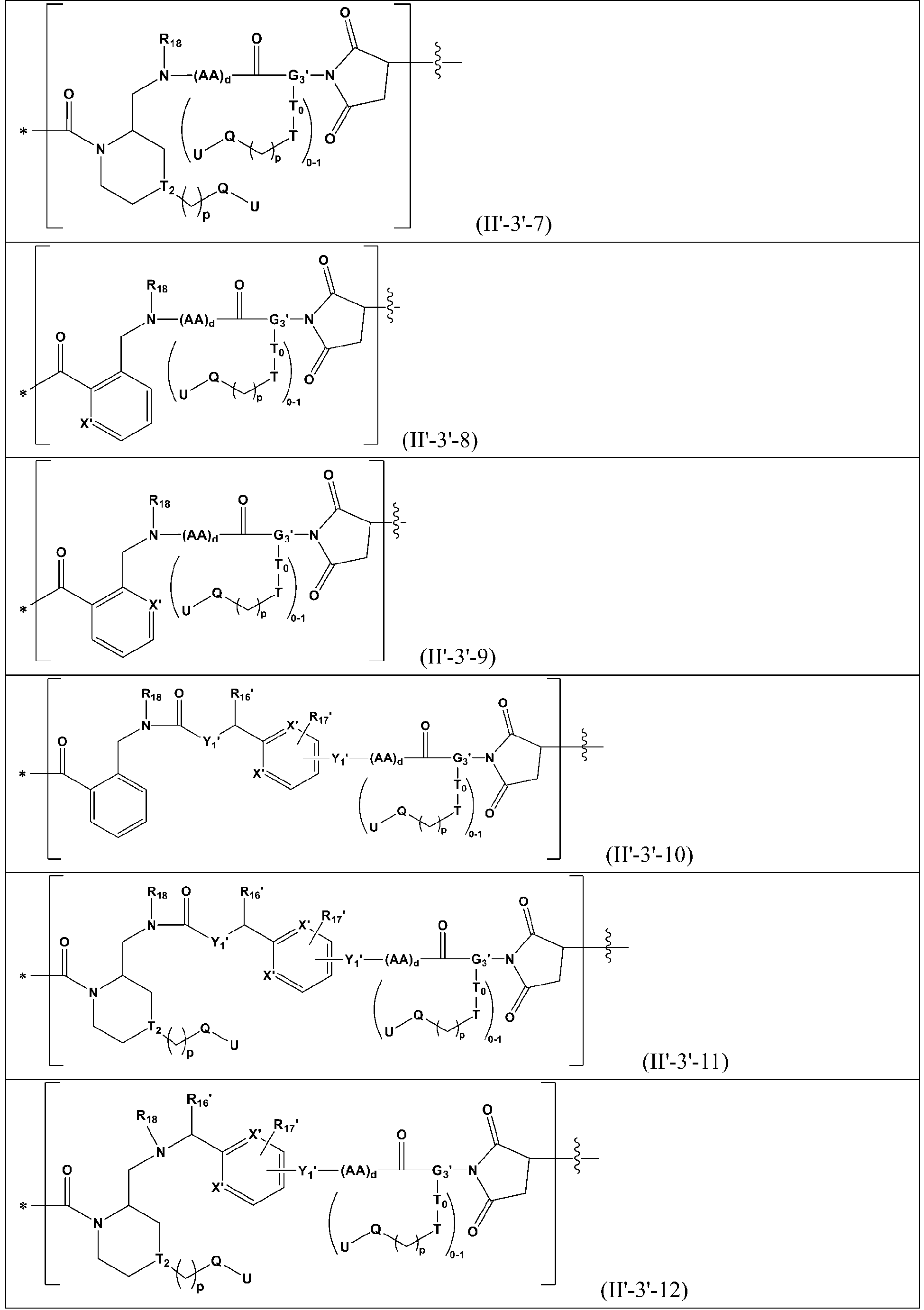

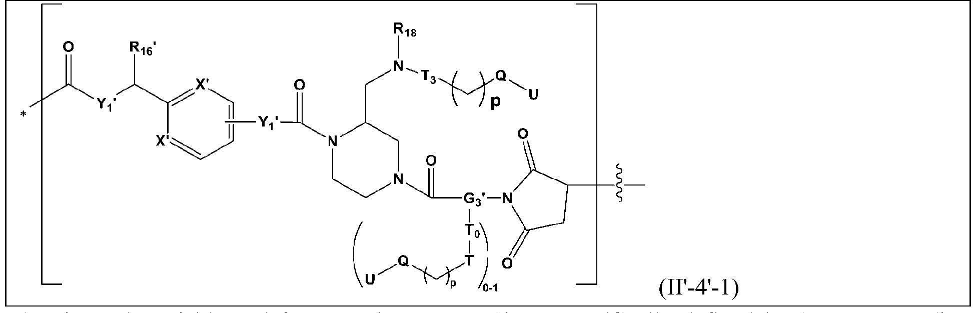

wherein each variable and fragment is as generally or specifically defined in the corresponding sections above.

In the various sub-formulas of the linker unit L shown above, the Y₁' connected to -(AA)_{d}- can be attached para to the other attachment point of the six-membered aromatic ring, or ortho to the other attachment point;

In some embodiments of the various sub-formulas of the linker unit L shown above, G₃' carries the 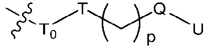 fragment; in other embodiments, G₃' does not carry the 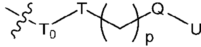 fragment;

Specifically,
Y₁' is selected from -O- and -NH-;
X' is selected from CH or N;
G₃' is -C₁-₁₀ alkylene-, preferably -C₁-₅ alkylene-;
R₁₆' is H;
R₁₇' is selected from H or a fragment of formula (A1) 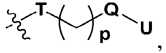 preferably H;
R₁₈ is preferably -C₁₋₃ alkyl, more preferably methyl;
T₁ is selected from -C(R^{a})₂-, -O-, -N R^{a}-;
in 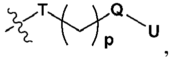 T is selected from -O- or -NR^{a}-, preferably -O-, p is 2-4, preferably 2, Q is absent or is -O- or -NR^{a}-, U is a monosaccharide or disaccharide group or derivative thereof (e.g., a monosaccharide or disaccharide group linked via a glycosidic bond, a uronic acid linked via a carbonyl group), 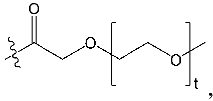 or a hydrophilic peptide such as 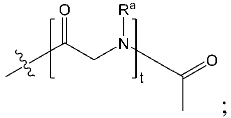
in 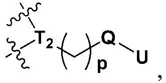 T₂ is -N-, p is 0, Q is absent, U is selected from 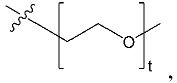 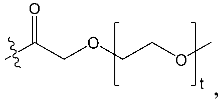 a hydrophilic peptide such as 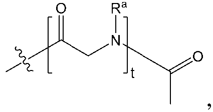 or a glycosyl (preferably a uronic acid) or derivative thereof (preferably a uronic acid of a monosaccharide or disaccharide or derivative thereof), preferably a hydrophilic peptide such as 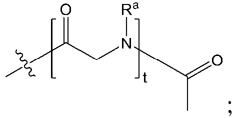
in 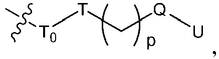 T₀ is -C₁₋₄ alkylene-, preferably -C₁₋₂ alkylene-, more preferably methylene; T is absent, p is 0, Q is -NR^{a}- or -CO-, U is selected from a glycosyl (preferably a uronic acid or amino sugar, more preferably a uronic acid or amino sugar of a monosaccharide or disaccharide) or derivative thereof, 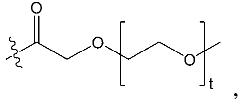 a hydrophilic peptide such as 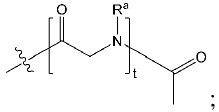
in 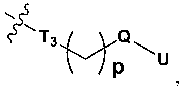 T₃ is a dipeptide, tripeptide, or tetrapeptide (preferably the dipeptides, tripeptides, or tetrapeptides defined herein for the cleavable linker B, more preferably selected from Ala-Ala, Cit-Val, Ala-Val, Glu-Gly, Asn-Ala-Ala, Cit-Ala-Glu, Gly-Phe-Gly-Gly) linked via -CO- to N, p is 0-4, preferably 0-2; Q is absent or is -NR^{a}- or -O-, preferably absent; U is preferably selected from 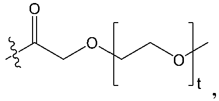 a hydrophilic peptide such as 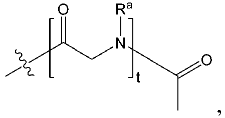 or a uronic acid (preferably a uronic acid of a monosaccharide or disaccharide) or derivative thereof, more preferably a hydrophilic peptide 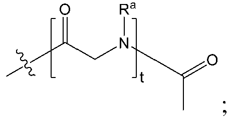
R^{a} is selected from H or -C₁₋₃ alkyl, preferably H or -CH₃;
t is preferably 2-14, such as 4-14, 6-12, more preferably 10.

It should be noted that the general formulas or sub-formulas exemplified above for (II'-3') and (II'-4') also include the corresponding general formulas where the maleimide moiety is

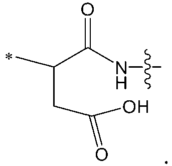

In the various sub-formulas of the linker unit L shown above, preferred G (-G₁-G₂-G₃-) is selected from the following: 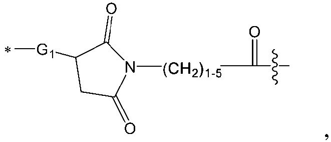 e.g., 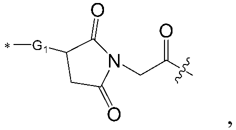 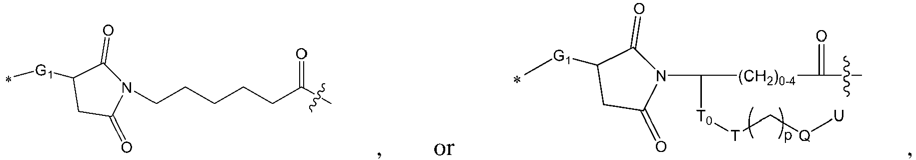 e.g., 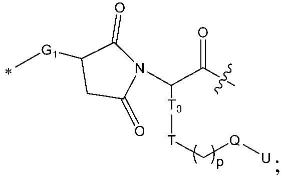 specifically 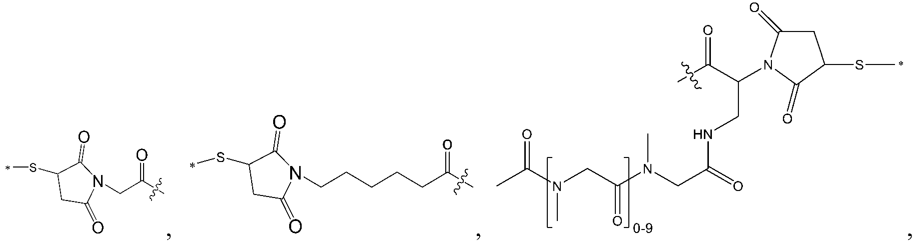 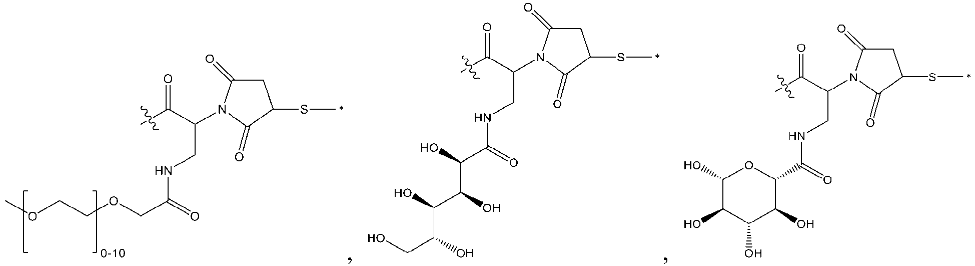 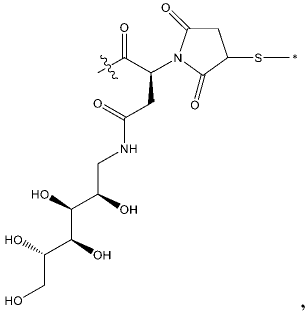 wherein S is an exemplary linking atom from Ab;
and the corresponding fragments where the maleimide moiety is

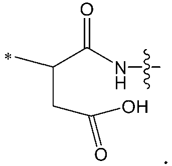

In the various sub-formulas of the linker unit L shown above, preferred -(AA)_{d}- polypeptides are selected from the following: 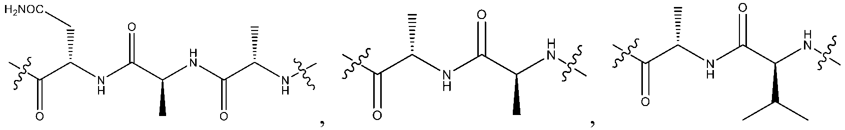 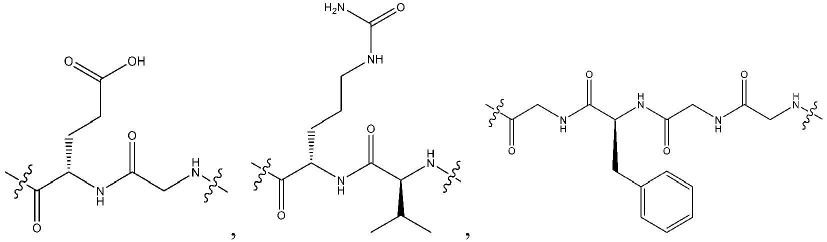 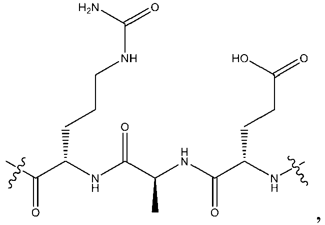 wherein the left carbonyl group is connected to the self-immolative linker A or the drug P, and the right N-terminus is connected to D (when present), E (when present), or the G part of the linker unit.

In a preferred group of embodiments, in the sub-formulas of the linker unit shown in Table 6-1 above, both J and G carry a fragment containing U ( 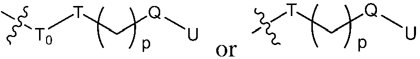 or 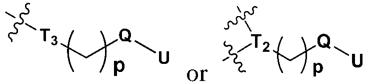 ); in another preferred group of embodiments, one of J and G carries the corresponding moiety containing U; in another preferred group of embodiments, J is 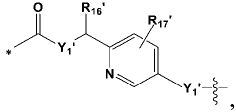 e.g., 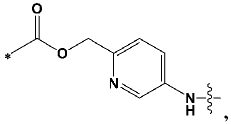 and G carries 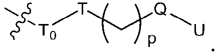 For these embodiments, U is preferably a hydrophilic peptide as defined herein, more preferably 4-14 units of polysarcosine, polyarginine, or polyglycine, more preferably 6-12 units of polysarcosine.

In specific embodiments, the linker unit L of the ADC of the present disclosure is selected from the structures shown in Table 7 below:

| | |
|---|---|
| 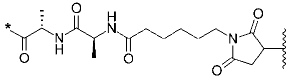 | 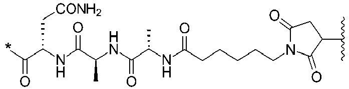 |
| 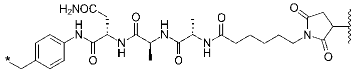 | 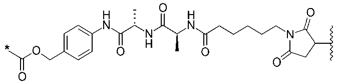 |
| 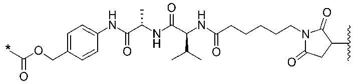 | 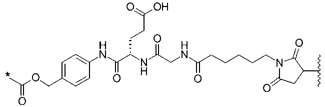 |
| 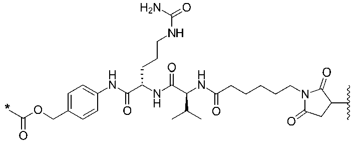 | 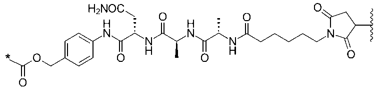 |
| 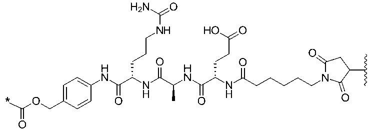 | 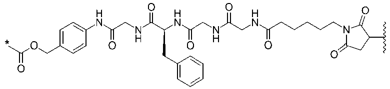 |
| 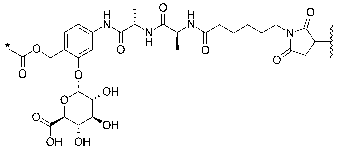 | 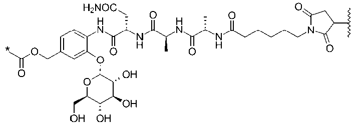 |
| 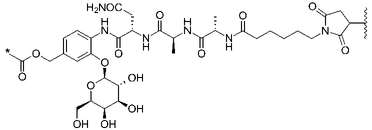 | 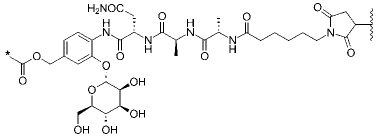 |
| 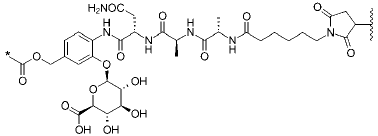 | 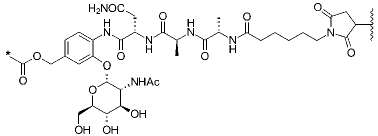 |
| 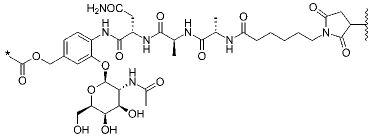 | 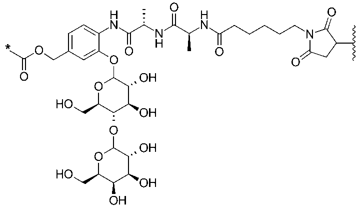 |
| 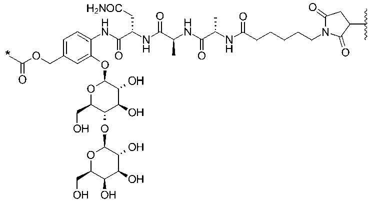 | 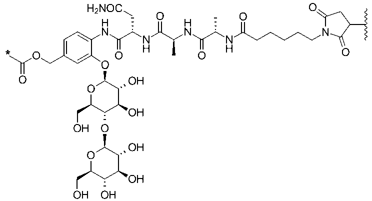 |
| 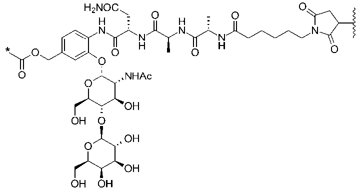 | |
| 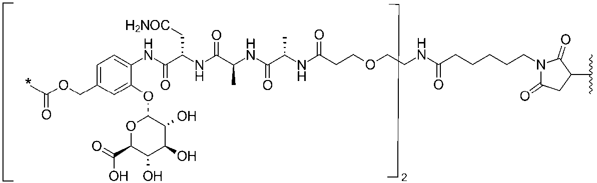 | |
| 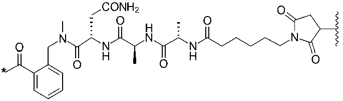 | 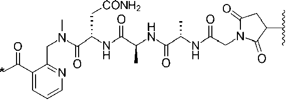 |
| 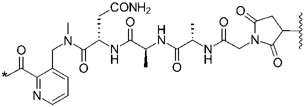 | 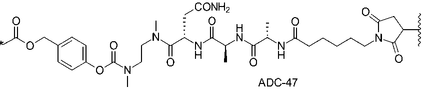 |
| 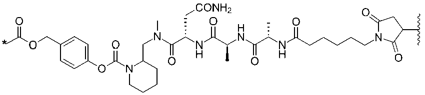 | 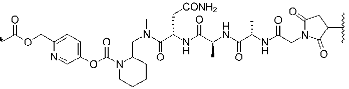 |
| 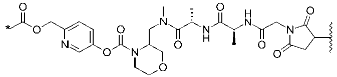 | 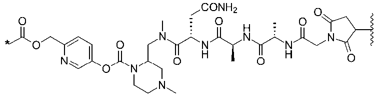 |
| 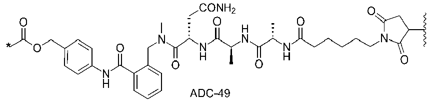 | 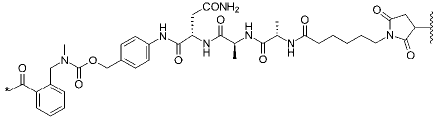 |
| 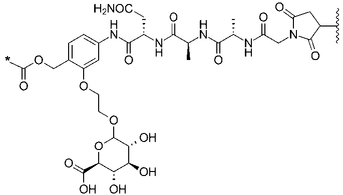 | 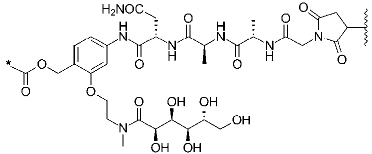 |
| 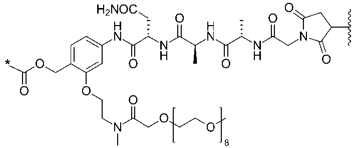 | 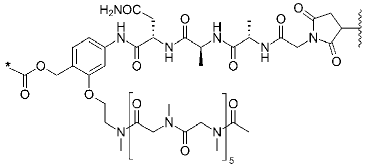 |
| 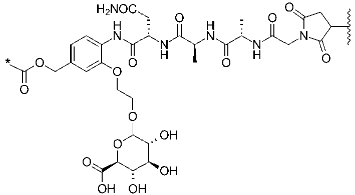 | 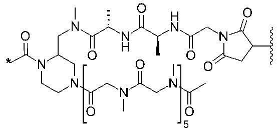 |
| 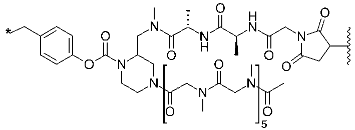 | 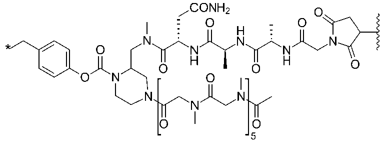 |
| 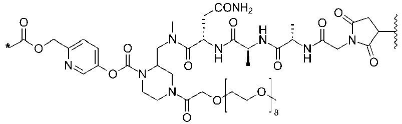 | 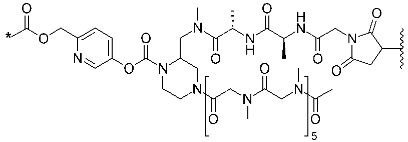 |
| 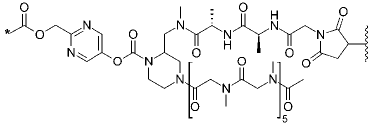 | 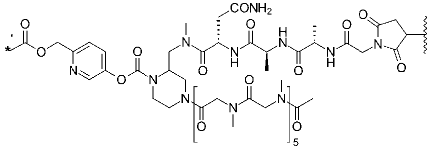 |
| 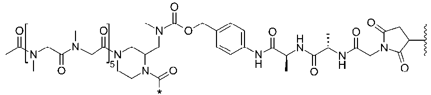 | 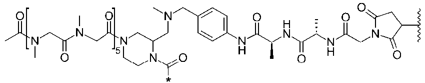 |
| 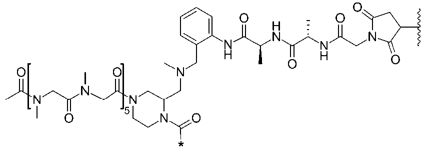 | 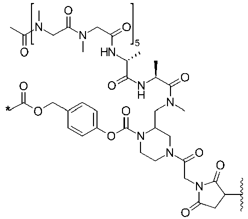 |
| 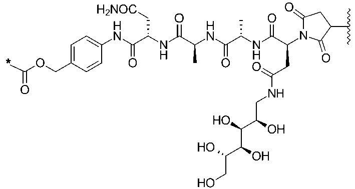 | 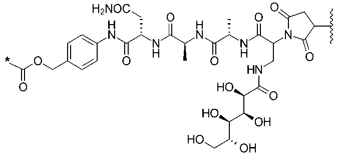 |
| 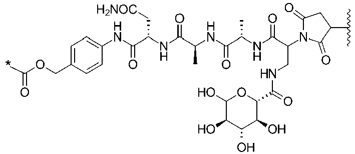 | 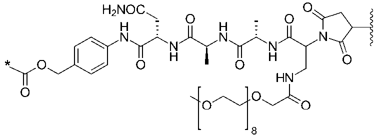 |
| 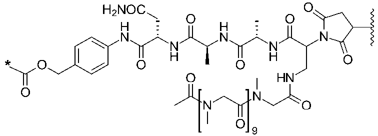 | 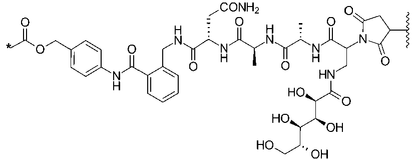 |
| 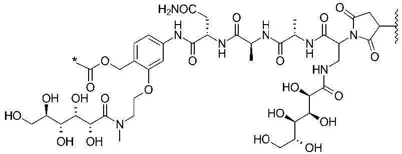 | 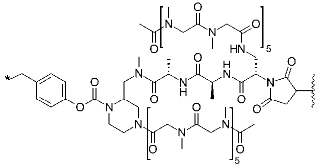 |
| 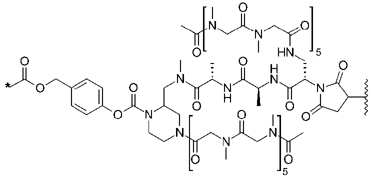 | 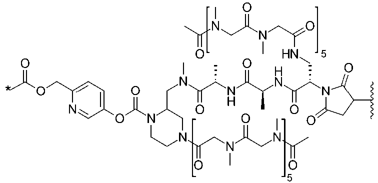 |
| 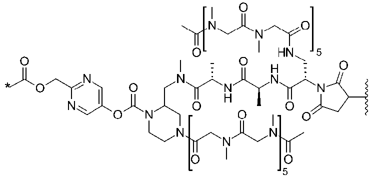 | 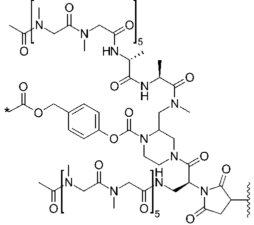 |
| 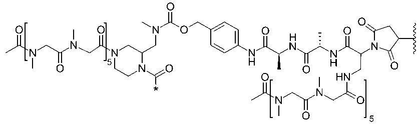 | 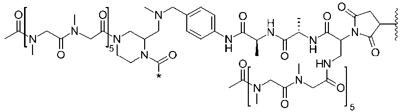 |
| 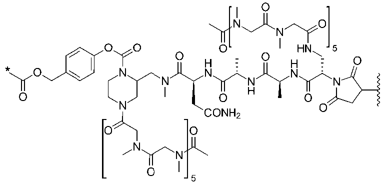 | 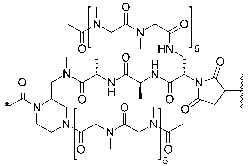 |
| 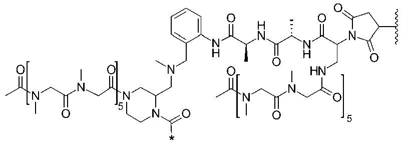 | |
| and the corresponding fragments where the maleimide moiety is 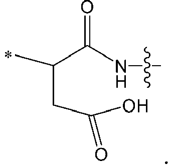 | |

It should be noted that the linker units of the general formulas in **Table 6-1** defined above for the linker unit L of formula (II') in the ADC of formula (X) of the present disclosure, and their specific embodiments (Table 7), are not limited to use with the drug P units and/or the antigen-targeting antibody or antigen-binding fragment Ab described in the present disclosure; they can also be used as appropriate with other drugs and/or antibodies or antigen-binding fragments not mentioned in the present disclosure. In other words, the linker units of the general formulas in **Table 6-1** and their specific embodiments (Table 7) independently constitute part of the technical solutions of the present invention.

### Ras Mutant Inhibitor-Linker Unit Conjugation Fragment of the Present Disclosure

Based on the above, the [P-L]-in the ADC of formula (X) of the present disclosure can be represented as: 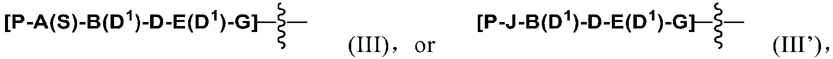 wherein P, A, S, B, D, D¹, E, G, J are respectively as generally or specifically defined above.

In some embodiments, in the [P-L]-of formula (III) or (III'), P is a compound as generally or specifically defined in the various embodiments of the Drug P Unit section, or a stereoisomer, tautomer, stable isotopic variant, pharmaceutically acceptable salt, or solvate thereof.

In formula (III) or (III'), the drug P unit is connected to the linker unit via an N atom or O atom in a substituent carried on its parent ring or side chain, for example, the drug P unit is connected to the linker unit via the ring N atom in the 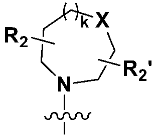 fragment of formula (I) (such as the N atom of the 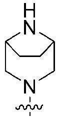 fragment), or via an OH, primary amino, secondary amino, or tertiary amino group carried on the drug side chain.

In formula (III) or (III'), the drug P unit can be a compound of formula (I), (I-A), (I-A-1), (I-A-1'), (I-A-1"), (I-A-2), (I-A-2'), (I-A-2"), (I-B), (I-B-1), (I-B-1'), (I-B-1"), (I-B-2), (I-B-2'), (I-B-2"), (I-C), (I-C-1), (I-C- I'), (I-C-1"), (I-C-2), (I-C-2'), (I-C-2"), (I-D), (I-D-1), (I-D-1'), (I-D-1"), (I-D-2), (I-D-2'), or (I-D-2") as defined above, or a compound listed in Embodiment 8 of the Drug P Unit; preferably a compound of formula (I), (I-A), (I-A-1), (I-A-1'), (I-A-1"), (I-A-2), (I-A-2'), (I-A-2"), (I-B), (I-B-1), (I-B-1'), (I-B-1"), (I-B-2), (I-B-2'), or (I-B-2"), or a compound listed in Embodiment 8 of the Drug P Unit; more preferably a compound of formula (I-A-1'), (I-A-1"), (I-A-2'), (I-A-2"), (I-B-1'), (I-B-1"), (I-B-2'), or (I-B-2"), or a compound listed in Embodiment 8 of the Drug P Unit; further more preferably a compound of formula (I-A-1"), (I-A-2"), (I-B-1"), or (I-B-2"), or a compound listed in Embodiment 8 of the Drug P Unit; most preferably any of the P1 to P7 compounds defined in Embodiment 7 of the Drug P Unit above or any of the P8 to P37 compounds listed in Embodiment 8 of the Drug P Unit; or their respective stereoisomers, tautomers, stable isotopic variants, pharmaceutically acceptable salts, or solvates, wherein each substituent has the meaning as generally or specifically defined in the Drug P Unit section above for each respective formula.

In formula (III), the linker unit -A(S)-B(D¹)-D-E(D¹)-G- can be a structural fragment of formula (II), (II-1), (11-2), (II-3), (II-3-1), (11-3-1'), (II-4), (11-4-1), (II-4-1'), (II-5), (II-5-1), (II-6), (II-7), or (II-8) as defined above, preferably a structural fragment of formula (II-3), (11-3-1), (II-3-1'), (II-4), (11-4-1), (11-4-1'), (II-5), (II-5-1), more preferably a structural fragment of formula (II-3-1), (11-3-1'), (11-4-1), (11-4-1'), or (II-5-1), most preferably a structural fragment of formula (II-3-1'), (11-4-1'), or (II-5-1), wherein each component or variable has the meaning as generally or specifically defined in the Linker Unit L section above for each respective formula.

In formula (III'), the linker unit -J-B(D¹)-D-E(D¹)-G- can be a structural fragment of formula (II'), (II'-1), (II'-2), (II'-3), (II'-3') as defined above, preferably a structural fragment of formula (II'-3), (II'-3'), (II'-4), (II'-4'), more preferably a structural fragment of formula (II'-3'-1) to (II'-3'-12) and (II'-4'-1), wherein each component or variable has the meaning as generally or specifically defined in the Linker Unit L section above for each respective formula.

It should be noted that the present disclosure encompasses KRas mutant inhibitor-linker unit conjugation fragments obtained by any combination of any embodiment of the drug P unit as generally or specifically defined with any embodiment of the linker unit L as generally or specifically defined.

In some embodiments of formula (III) or (III'), the drug P unit is a compound of formula (I), (I-A), (I-A-1), (I-A-1'), (I-A-1"), (I-A-2), (I-A-2'), (I-A-2"), (I-B), (I-B-1), (I-B-1'), (I-B-1"), (I-B-2), (I-B-2'), or (I-B-2") as defined above, or a compound listed in **Embodiment 8 of the Drug P Unit,** or a stereoisomer, tautomer, stable isotopic variant, pharmaceutically acceptable salt, or solvate thereof, wherein each substituent has the meaning as generally or specifically defined in the Drug P Unit section above for each respective formula; and the linker unit -A(S)-B(D¹)-D-E(D¹)-G- is a structural fragment of formula (II-3-1), (II-3-1'), (11-4-1), (II-4-1'), or (II-5-1) as defined above, or the linker unit -J-B(D¹)-D-E(D¹)-G- is a structural fragment of formula (II'-3), (II'-3'), (II'-4), or (II'-4') as defined above, wherein each component or variable has the meaning as generally or specifically defined in the Linker Unit L section above for each respective formula.

In some embodiments of formula (III) or (III'), the drug P unit is a compound of formula (I-A-1"), (I-A-2"), (I-B-1"), or (I-B-2") as defined in **Embodiment 6.1 of the Drug P Unit** above, or a compound listed in **Embodiment 8 of the Drug P Unit,** or a stereoisomer, tautomer, stable isotopic variant, pharmaceutically acceptable salt, or solvate thereof, wherein each substituent has the meaning as generally or specifically defined in the Drug P Unit section above for each respective formula; and the linker unit -A(S)-B(D¹)-D-E(D¹)-G- is a structural fragment of formula (II-3-1'), (II-4-1'), or (II-5-1) as defined above, or the linker unit -J-B(D¹)-D-E(D¹)-G- is a structural fragment of formula (II'-3') or (II'-4') as defined above, wherein each component or variable has the meaning as generally or specifically defined in the Linker Unit L section above for each respective formula.

In some embodiments of formula (III) or (III'), the drug P unit is any of the P1, P2, P3, P4, P5, P6, or P7 compounds defined in **Embodiment 7 of the Drug P Unit** above or any of the P8 to P37 compounds listed in **Embodiment 8 of the Drug P Unit,** or a stereoisomer, tautomer, stable isotopic variant, pharmaceutically acceptable salt, or solvate thereof; and the linker unit -A(S)-B(D¹)-D-E(D¹)-G- is a structural fragment of formula (II-3-1'), (II-4-1'), or (II-5-1) as defined above, or the linker unit -J-B(D¹)-D-E(D¹)-G- is a structural fragment of formula (II'-3'-1) to (II'-3'-12) or (II'-4'-1) as defined above, wherein each component or variable has the meaning as generally or specifically defined in the Linker Unit L section above for each respective formula.

In some embodiments, specific formula (III) conjugation fragments include, but are not limited to: 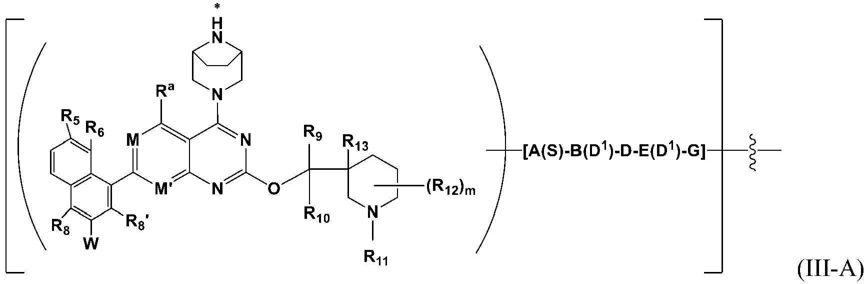 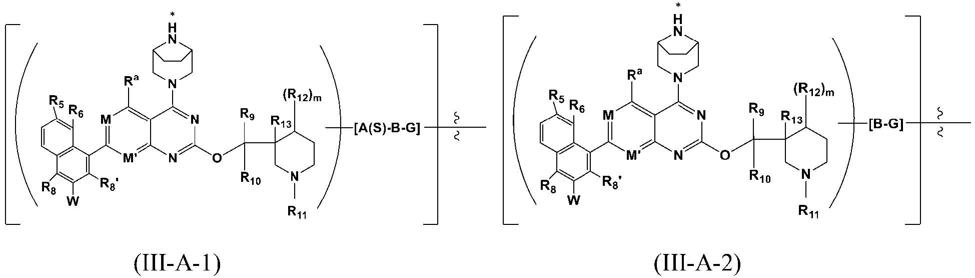 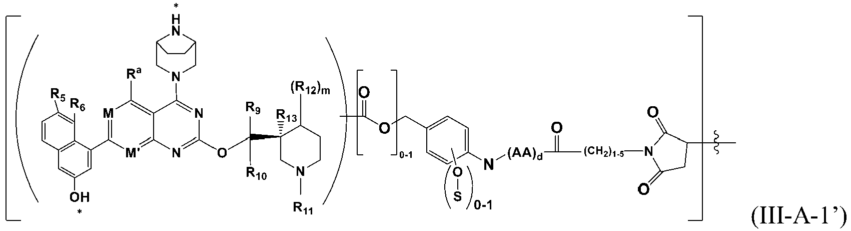 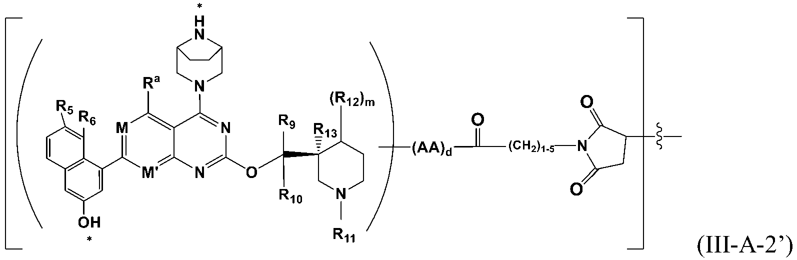 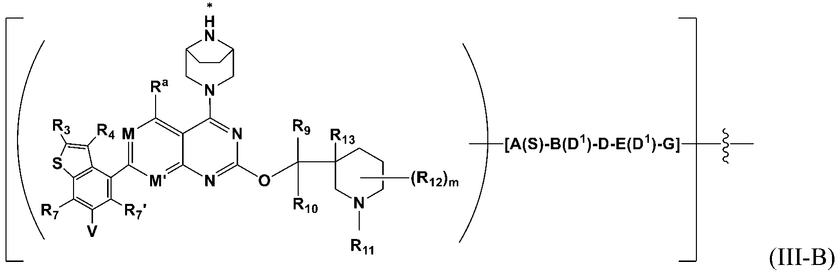 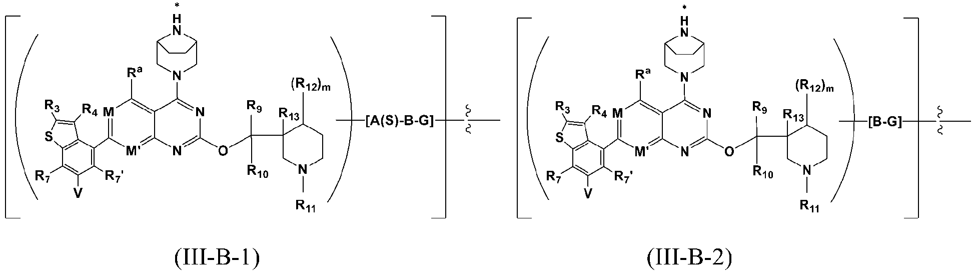 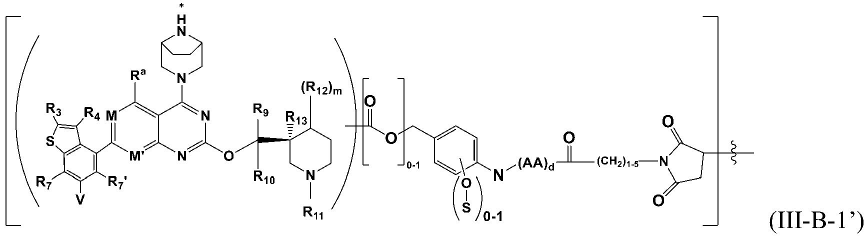 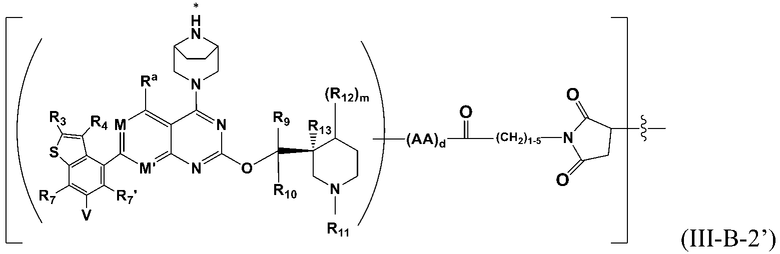 and the corresponding respective general formula fragments where the maleimide moiety is 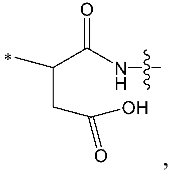 or a stereoisomer, tautomer, stable isotopic variant, pharmaceutically acceptable salt, or solvate thereof, wherein M, M', R^{a}, R₃, R₄, R₅, R₆, R₇, R₇', R₈, R₈', R₉, R₁₀, R₁₁, R₁₂, R₁₃, W, V, m, A, S, B, D¹, D, E, G, AA, d respectively have the meanings as generally or specifically defined in the Drug P Unit and Linker Unit L sections above; preferably have the general or specific drug P unit, the general or specific linker unit L, and combinations of the general or specific drug P unit and linker unit L as defined for formula (III) above;
wherein the wavy line indicates the point of attachment to Ab; the atom marked with * is the attachment site in the drug P unit for connection to the linker unit. It should be noted that when more than one * is present, it indicates that the fragment can be bonded to the linker unit at any of the marked * positions.

In some embodiments, specific formula (III') conjugation fragments include, but are not limited to: 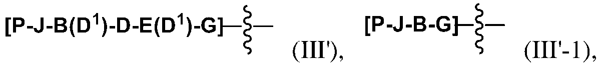 specifically having the structures shown in Table 8:

| |
|---|
| 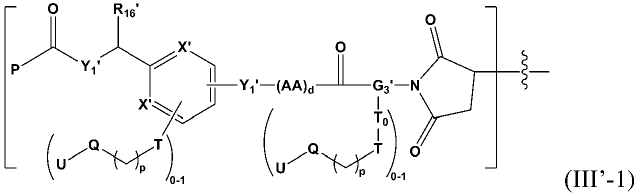 |
| 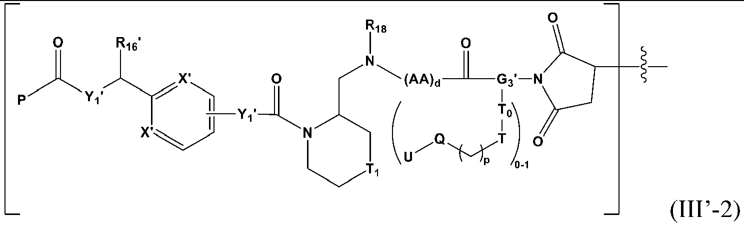 |
| 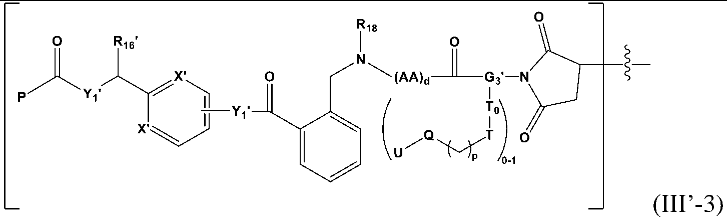 |
| 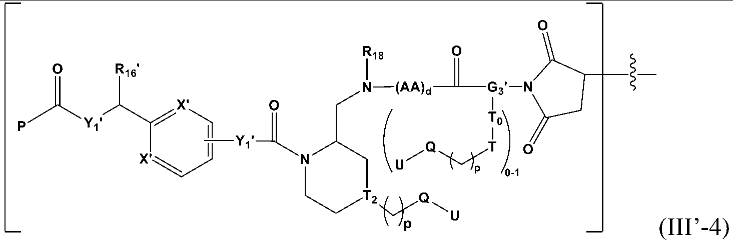 |
| 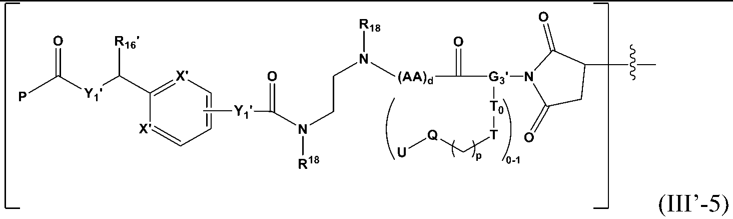 |
| 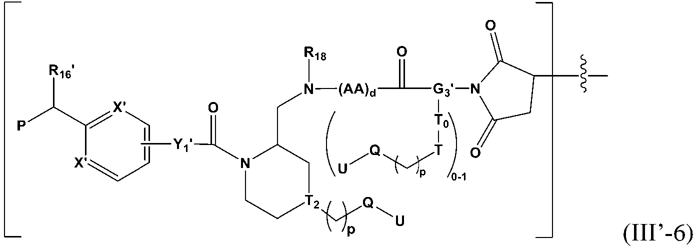 |
| 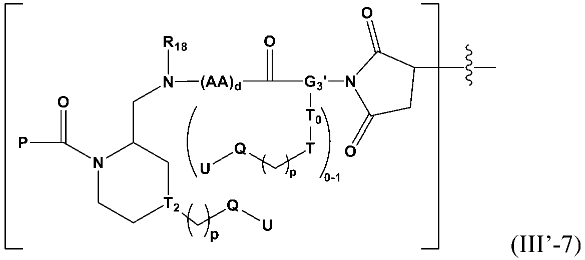 |
| 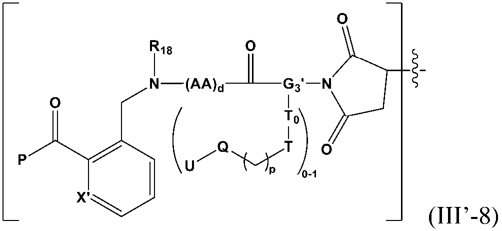 |
| 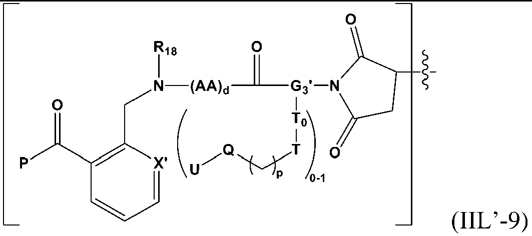 |
| 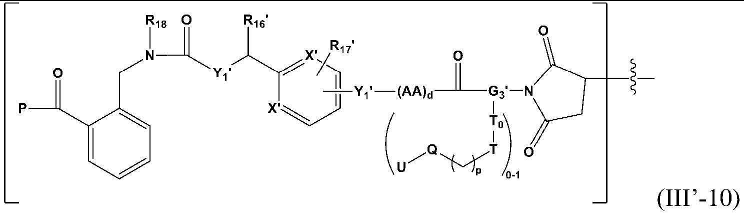 |
| 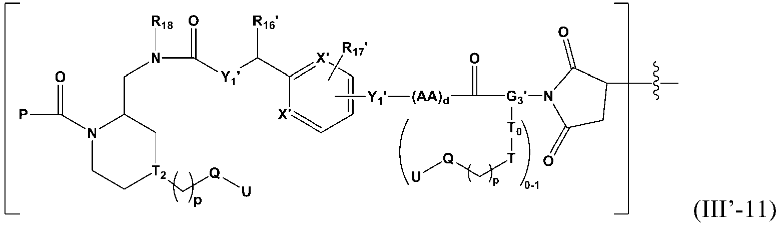 |
| 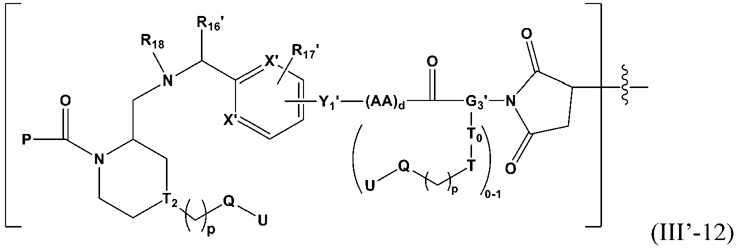 |
| 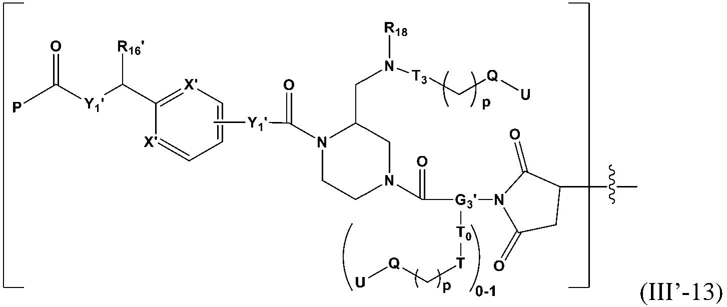 |
| and the corresponding respective general formula fragments where the maleimide moiety is 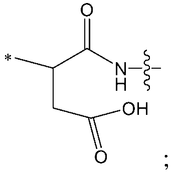 |

wherein the drug P unit is selected from the Ras inhibitor compounds defined in any one of **Embodiments 1-8** of the **Drug P Unit,** preferably a KRas inhibitor compound, more preferably the P1-P37 compounds defined in Embodiments 7-8, or a stereoisomer, tautomer, stable isotopic variant, pharmaceutically acceptable salt, or solvate thereof; and
wherein each variable in the linker unit is as generally or specifically defined in the corresponding sections above, and the linker unit has the structure as generally or specifically exemplified in the corresponding sections above.

The Ras mutant inhibitor-linker unit conjugation fragment of the present disclosure includes technical solutions formed by the general, preferred, or specific definitions of each group in the above various formulas, and also includes technical solutions formed by any combination of the general, preferred, or specific definitions of each group.

Specific examples of the Ras mutant inhibitor-linker unit conjugate of the present disclosure include, but are not limited to, the structures shown in Table 8-1 below:

| | |
|---|---|
| 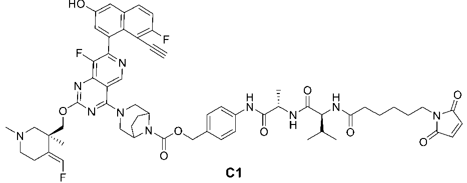 | 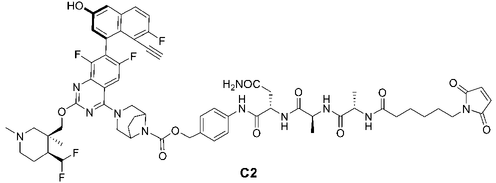 |
| 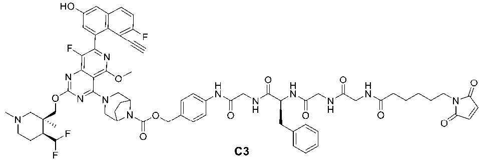 | 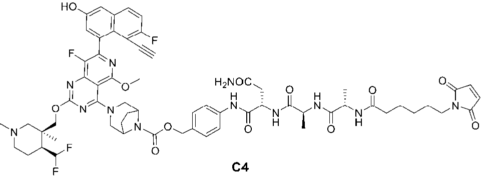 |
| 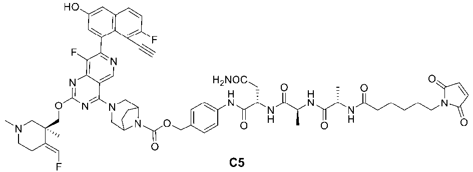 | 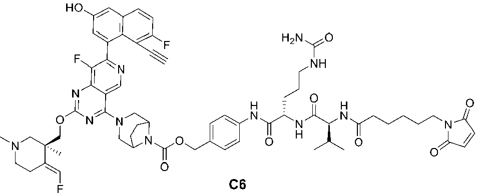 |
| 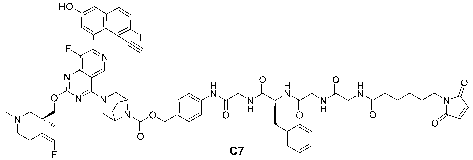 | 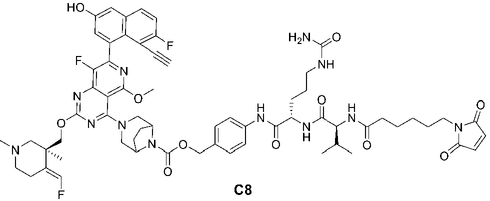 |
| 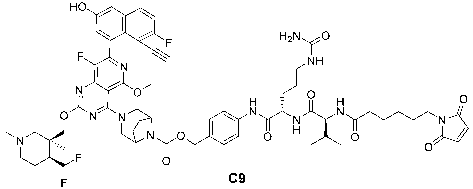 | 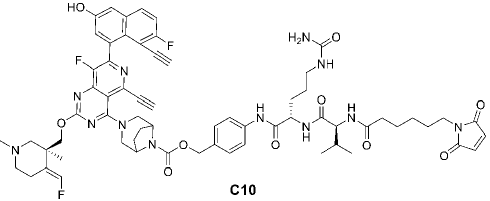 |
| 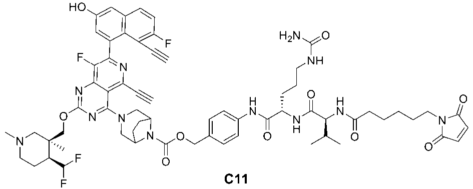 | 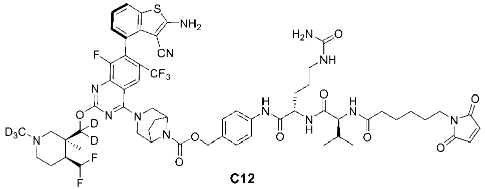 |
| 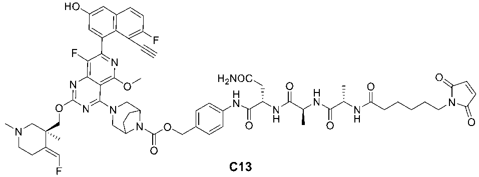 | 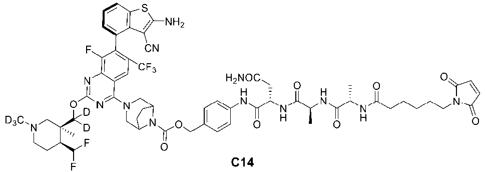 |
| 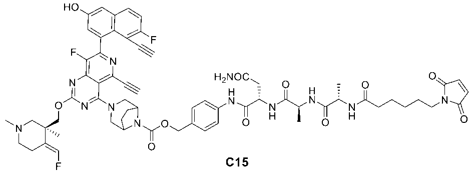 | 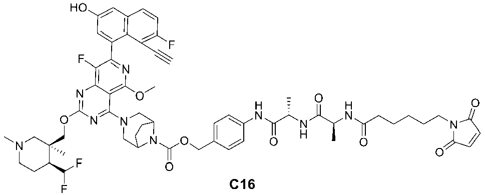 |
| 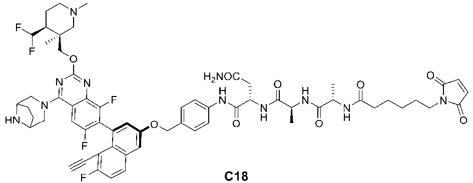 | 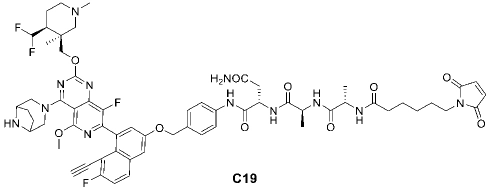 |
| 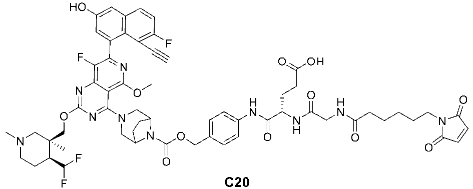 | 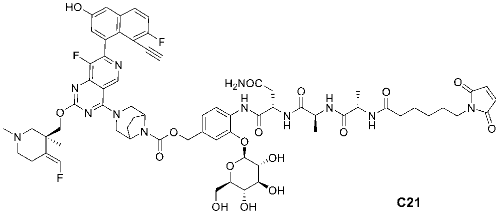 |
| 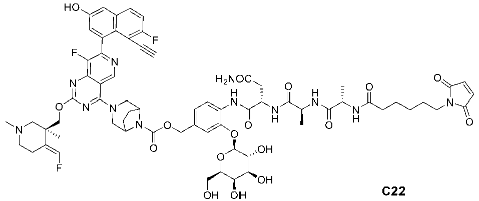 | 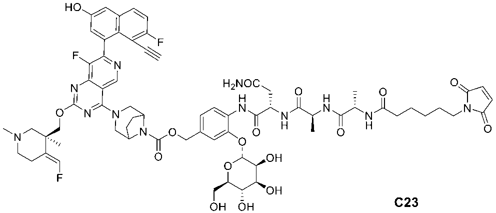 |
| 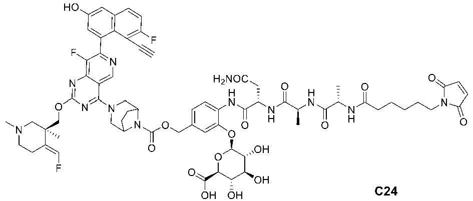 | 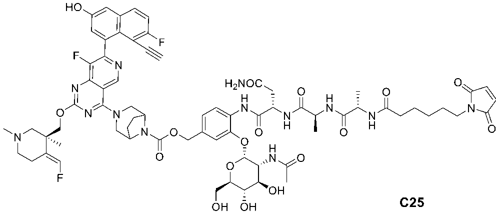 |
| 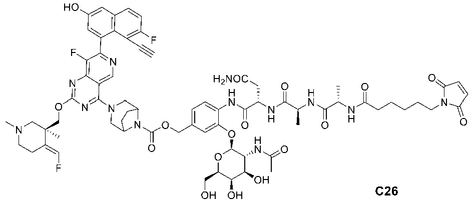 | 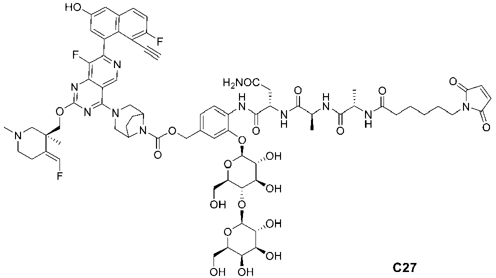 |
| 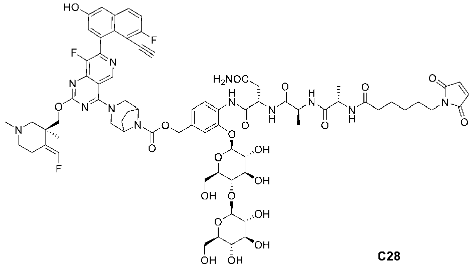 | 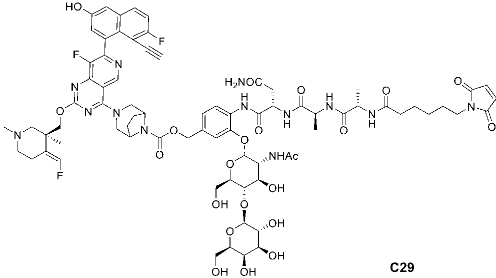 |
| 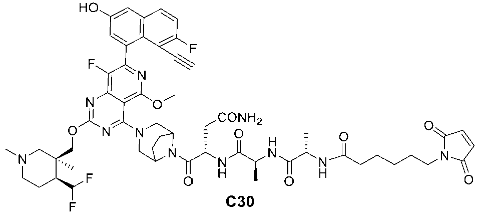 | 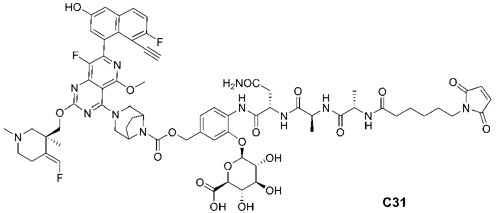 |
| 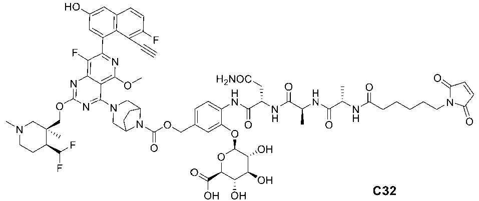 | 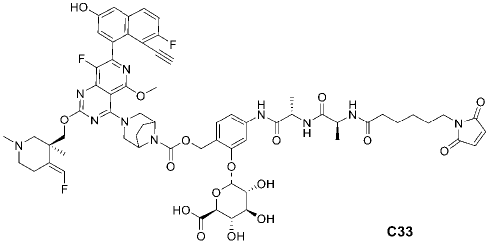 |
| 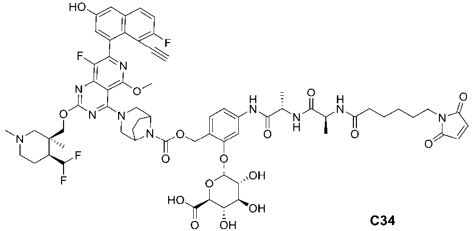 | 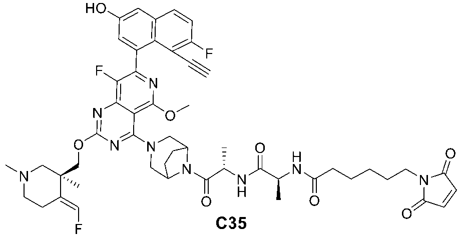 |
| 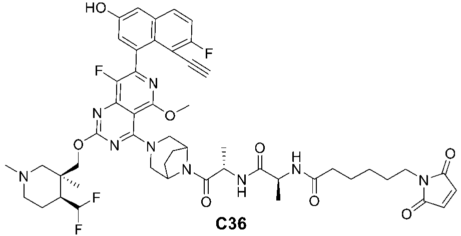 | 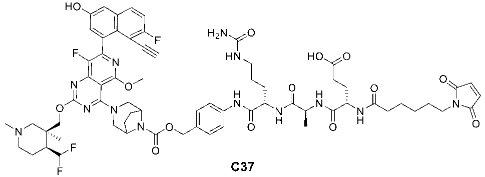 |
| 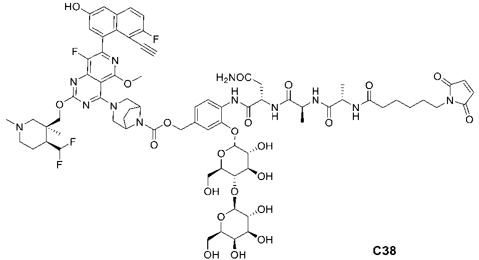 | |
| 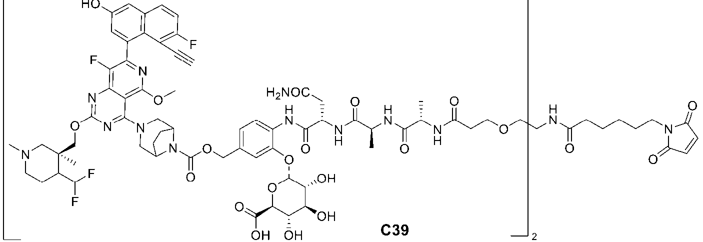 | |
| and C17 and C40-C86 as shown in the Examples section below; | |
| 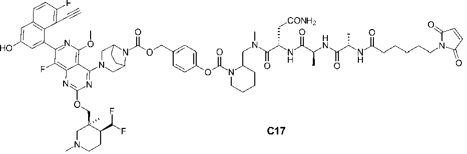 | 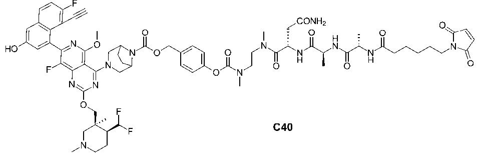 |
| 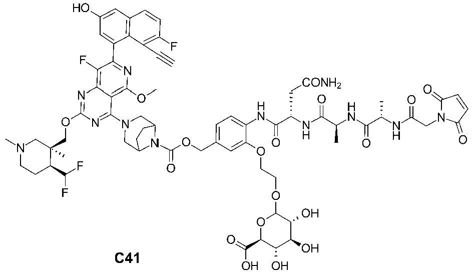 | 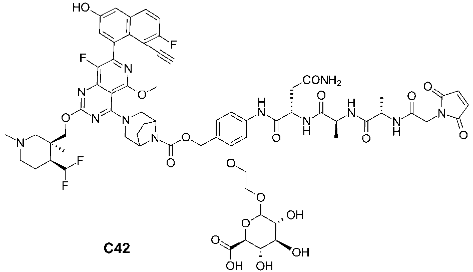 |
| 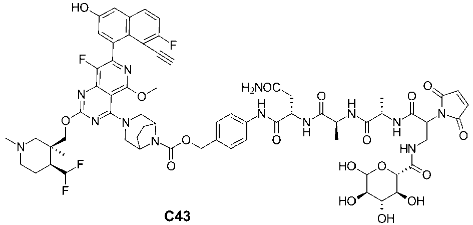 | 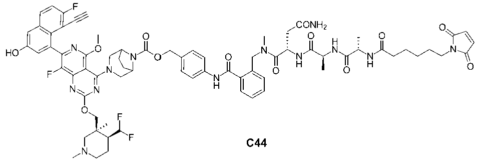 |
| 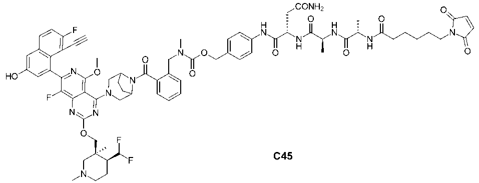 | 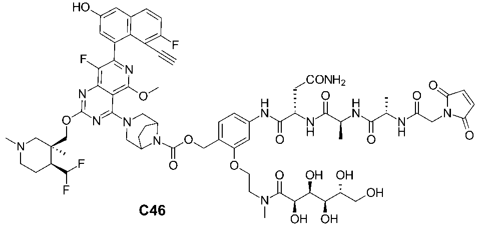 |
| 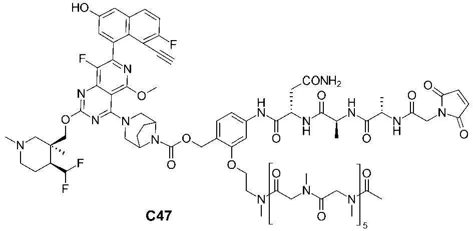 | 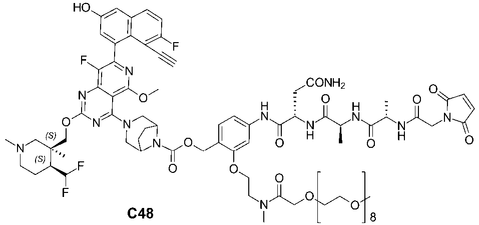 |
| 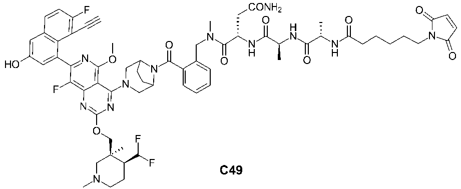 | 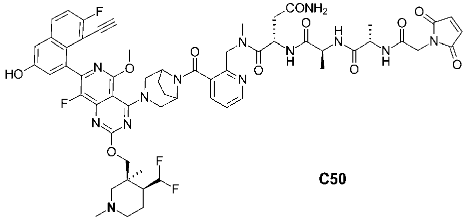 |
| 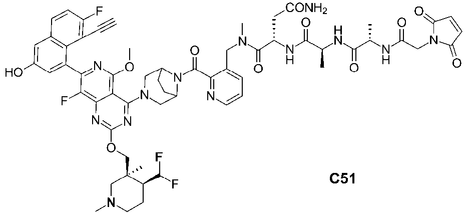 | 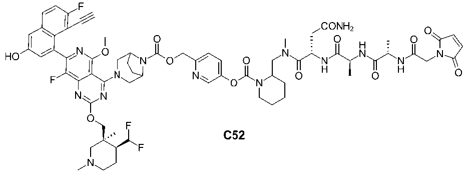 |
| 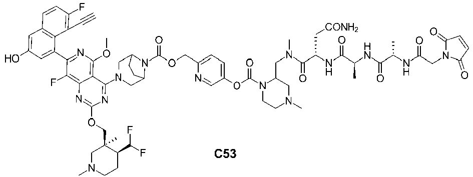 | 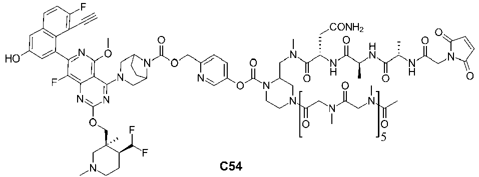 |
| 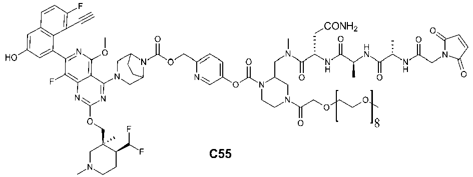 | 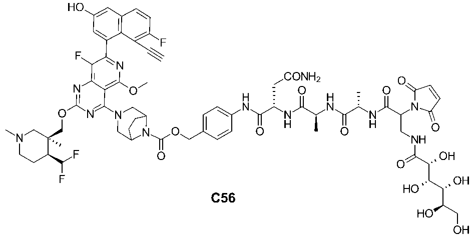 |
| 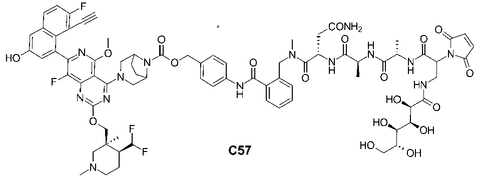 | 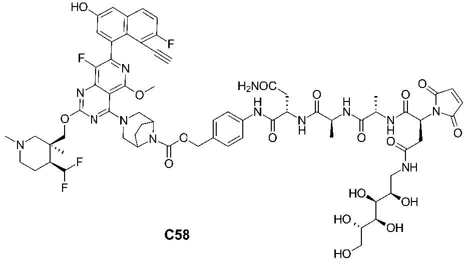 |
| 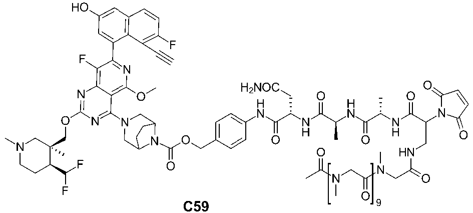 | 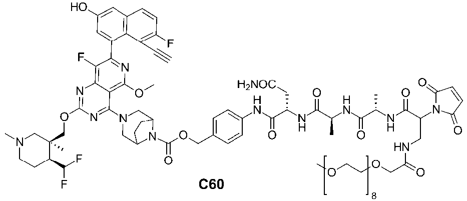 |
| 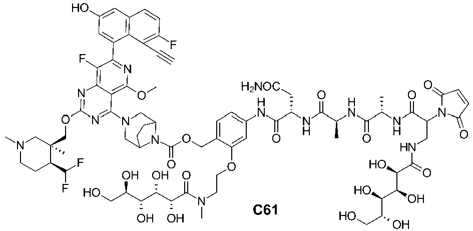 | 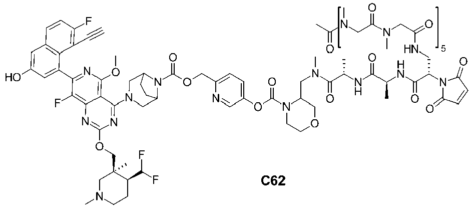 |
| 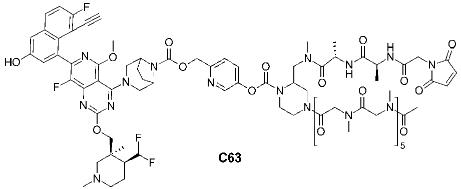 | 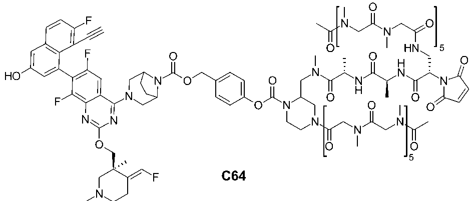 |
| 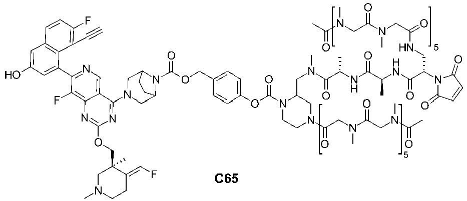 | 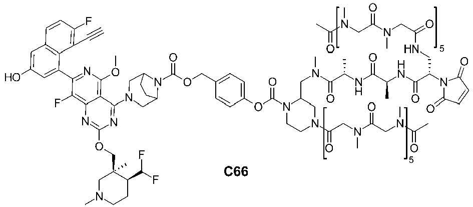 |
| 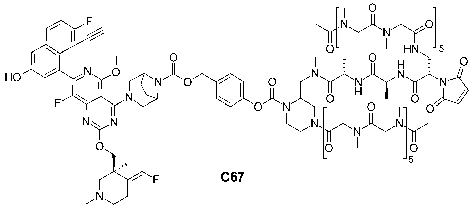 | 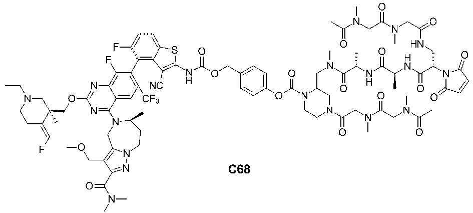 |
| 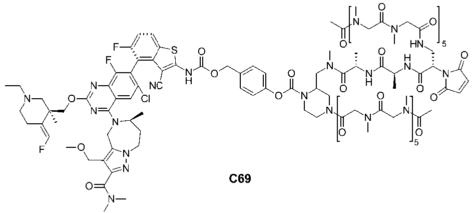 | 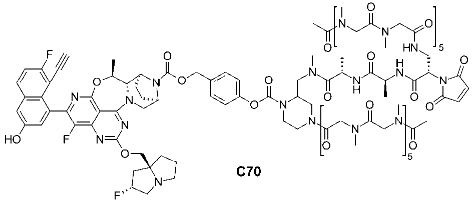 |
| 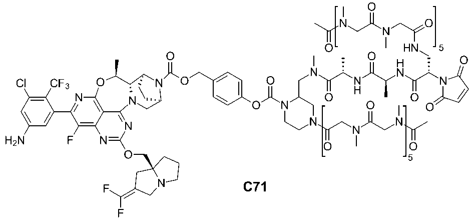 | 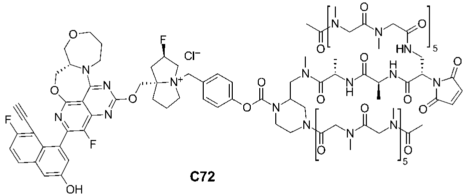 |
| 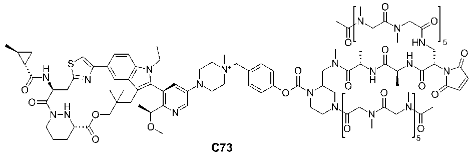 | 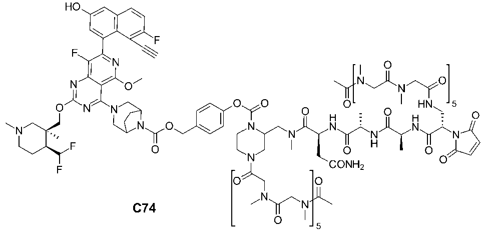 |
| 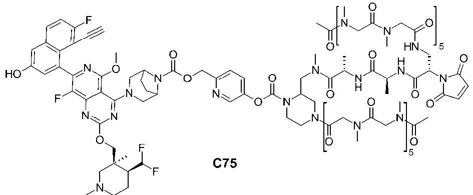 | 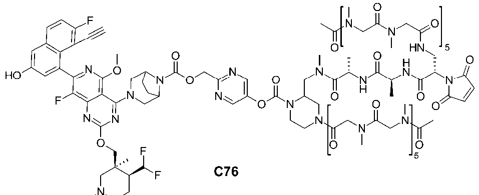 |
| 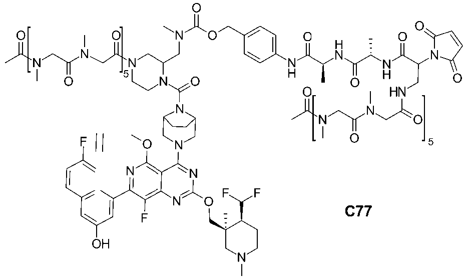 | 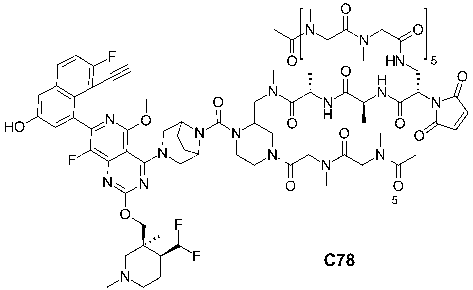 |
| 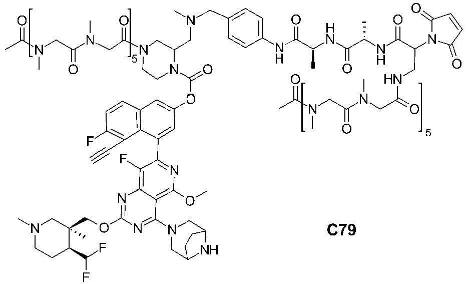 | 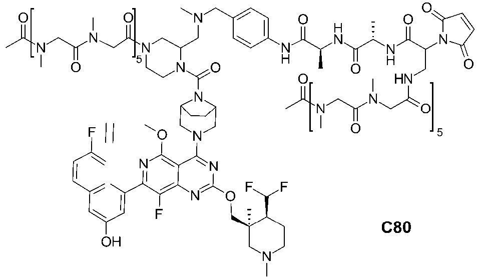 |
| 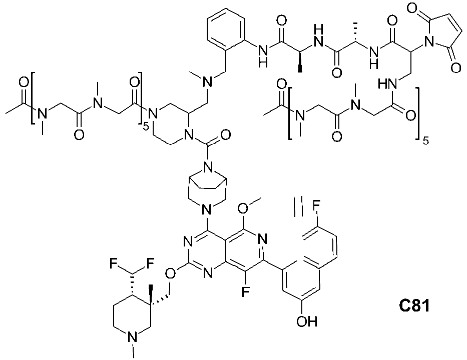 | 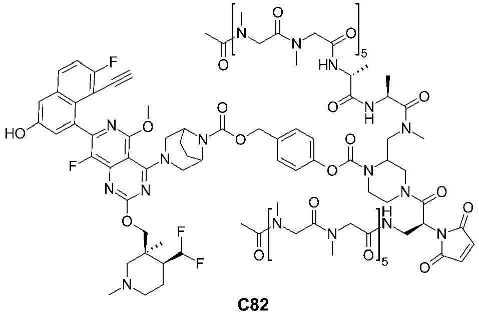 |
| 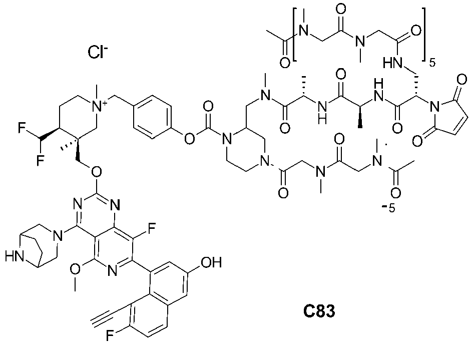 | 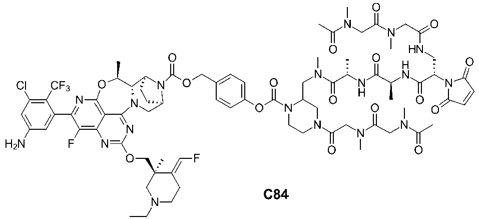 |
| 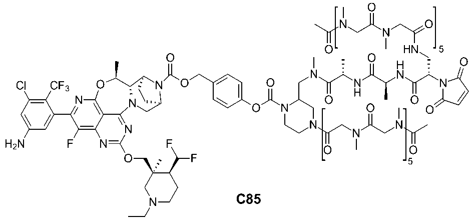 | 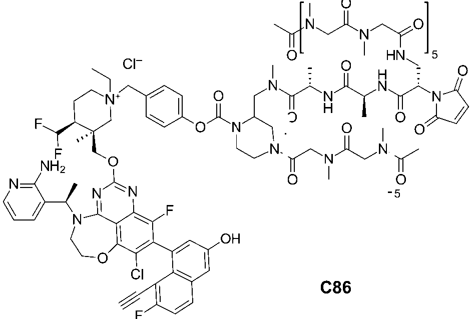 |
| and the corresponding fragments where the maleimide moiety is 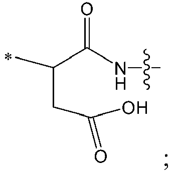 | |

or a stereoisomer, tautomer, stable isotopic variant, pharmaceutically acceptable salt, or solvate thereof.

### ADC of the Present Disclosure

Based on the above, the ADC of formula (X) of the present disclosure: [P-L]_{q}-Ab, wherein P, L, q, and Ab are respectively as generally or specifically defined in the various sections above; wherein the P-L moiety is as generally or specifically defined in the "Ras **Mutant Inhibitor-Linker Unit Conjugation Fragment of the Present Disclosure"** section above, preferably a fragment shown in Table 8, more preferably a fragment shown in Table 8-1.

Specifically, the ADC of formula (X) of the present disclosure can be: 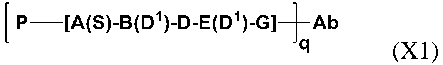 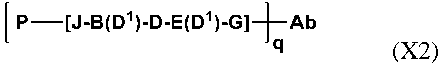
or a stereoisomer, tautomer, stable isotopic variant, pharmaceutically acceptable salt, or solvate thereof,
wherein P is a Ras inhibitor compound as defined in any one of **Embodiments 1-8 of the Drug P Unit** above, preferably the P1~P37 compounds defined in Embodiments 7-8, or a stereoisomer, tautomer, stable isotopic variant, pharmaceutically acceptable salt, or solvate thereof. In some embodiments, the drug P is a compound of formula (I), i.e., a compound defined in any one of Embodiments 1 to 7, preferably P1~P7 compounds; in other embodiments, the drug P is a compound defined in Embodiment 8, preferably P8~P37 compounds;
wherein A, S, B, D¹, D, E, G, J respectively have the meanings as generally or specifically defined in the **Linker Unit L** section above, wherein q has the meaning as generally or specifically defined in the "I: Antibody-Drug Conjugate" section above, for example 1-10, 1-8, 2-8, 3-10, 3-8, 4-10, 4-8, 6-8, or 6-10; and Ab has the meaning as generally or specifically defined in the "Ab-**Antibody or Antigen-Binding Fragment"** section above.

In formula (X1) or (X2), the drug P unit, for example a compound of formula (I), is connected to the linker unit via an N atom or O atom in a substituent carried on its parent ring or side chain, for example, the drug P unit is connected to the linker unit via the ring N atom in the 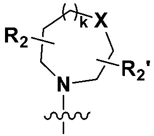 fragment of formula (I) (such as the N atom of the 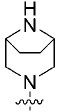 fragment), or via an OH, primary amino, secondary amino, or tertiary amino group carried on the drug side chain.

In formula (X), specifically formula (X1) or (X2), the general, preferred, more preferred, and most preferred definitions of the drug P unit, the general, preferred, more preferred, and most preferred definitions of the linker unit -A(S)-B(D¹)-D-E(D¹)-G- or -J-B(D¹)-D-E(D¹)-G-, and exemplary combinations thereof, are as defined in the "Ras **Mutant Inhibitor-Linker Unit Conjugation Fragment of the Present** Disclosure" section above.

In preferred embodiments, the drug P unit in formula (X1) or (X2) is a compound of formula (I-A-1"), (I-A-2"), (I-B-1"), or (I-B-2") as defined in **Embodiment 6.1 of the Drug P Unit** above, or a compound listed in **Embodiment 8 of the Drug P Unit,** or a stereoisomer, tautomer, stable isotopic variant, pharmaceutically acceptable salt, or solvate thereof, wherein each substituent has the meaning as generally or specifically defined in the Drug P Unit section above for each respective formula; and the linker unit -A(S)-B(D¹)-D-E(D¹)-G- is a structural fragment of formula (II-3-1'), (II-4-1'), or (II-5-1) as defined above, or the linker unit -J-B(D¹)-D-E(D¹)-G- is a structural fragment of formula (II'-3), (II'-3'), (II'-4), or (II'-4') as defined above, wherein each component or variable has the meaning as generally or specifically defined in the Linker Unit L section above for each respective formula.

In more preferred embodiments, the drug P unit in formula (X1) or (X2) is any of the P1~P7 compounds defined in **Embodiment 7 of the Drug P Unit** above or any of the P8~P37 compounds listed in **Embodiment 8 of the Drug P Unit,** or a stereoisomer, tautomer, stable isotopic variant, pharmaceutically acceptable salt, or solvate thereof; and the linker unit -A(S)-B(D¹)-D-E(D¹)-G- is a structural fragment of formula (II-3-1'), (II-4-1'), or (II-5-1) as defined above, or the linker unit -J-B(D¹)-D-E(D¹)-G- is a structural fragment of any of formulas (II'-3'-1) to (II'-3'-12) or (II'-4'-1) as defined above, wherein each component or variable has the meaning as generally or specifically defined in the Linker Unit L section above for each respective formula.

In some embodiments, the drug P unit in formula (X2) is any of the P1~P7 compounds defined in **Embodiment 7 of the Drug P Unit** above or any of the P8 to P37 compounds listed in **Embodiment 8 of the Drug P Unit,** or a stereoisomer, tautomer, stable isotopic variant, pharmaceutically acceptable salt, or solvate thereof; and the linker unit -J-B(D¹)-D-E(D¹)-G- is a structural fragment of any of formulas (II'-3'-1) to (II'-3'-12) or (II'-4'-1) as defined above, wherein each component or variable has the meaning as generally or specifically defined in the Linker Unit L section above for each respective formula.

It should be noted that the ADC of the present disclosure includes Ras mutant inhibitor-linker unit conjugation fragments obtained by any combination of any embodiment of the drug P unit as generally or specifically defined with any embodiment of the linker unit L as generally or specifically defined.

In some embodiments, the ADC of formula (X1) of the present disclosure includes, but is not limited to: 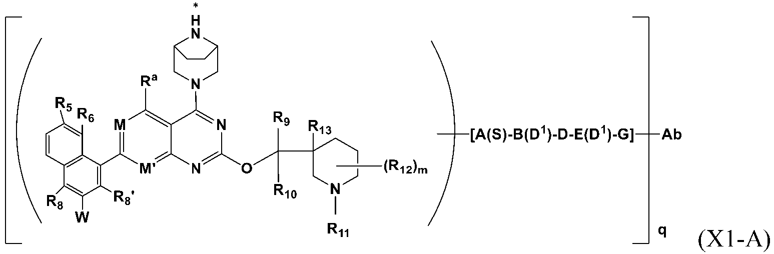 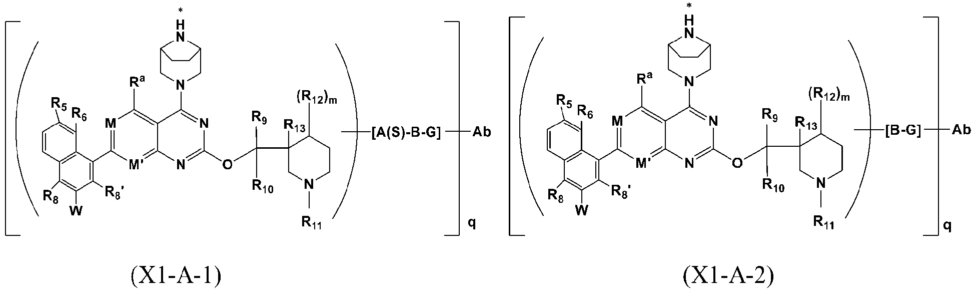 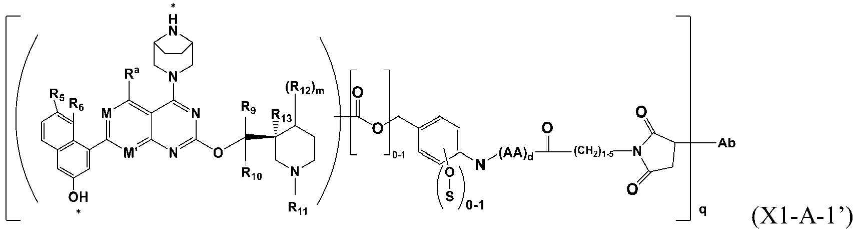 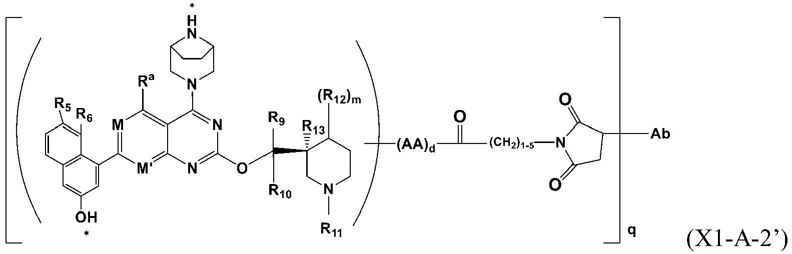 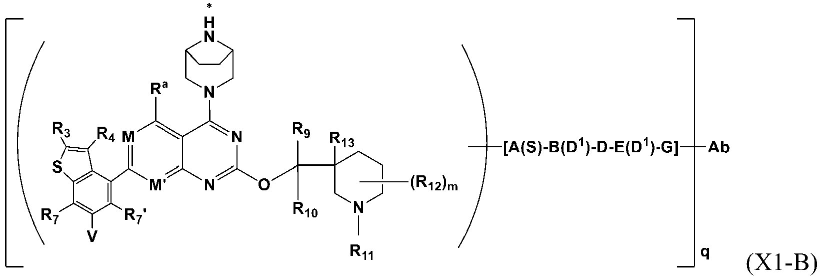 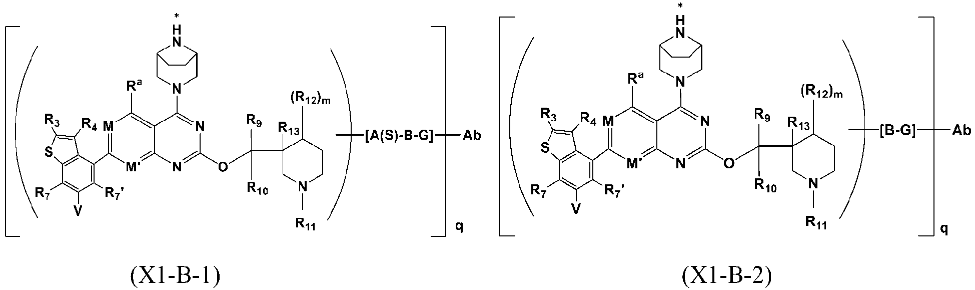 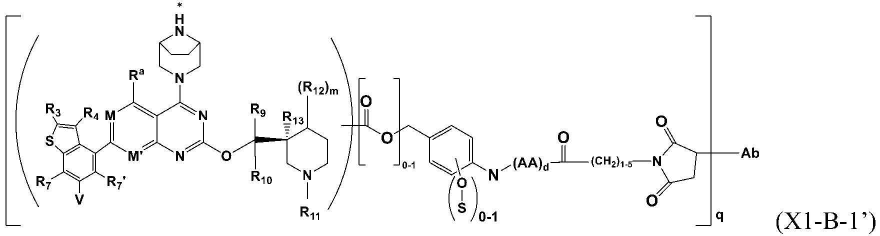 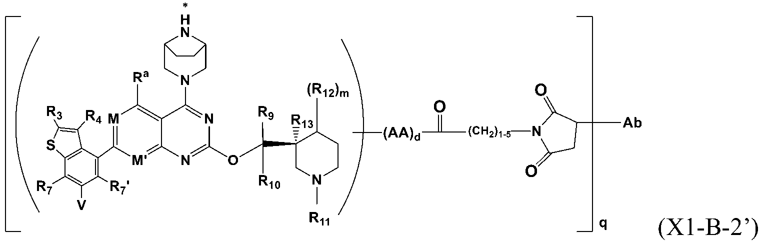
and the corresponding respective general formula fragments where the maleimide moiety is 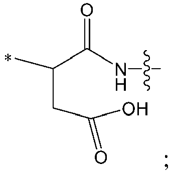
or a stereoisomer, tautomer, stable isotopic variant, pharmaceutically acceptable salt, or solvate thereof,
wherein M, M', R^{a}, R₃, R₄, R₅, R₆, R₇, R₇', R₈, R₈', R₉, R₁₀, R₁₁, R₁₂, R₁₃, W, V, m, A, S, B, D¹, D, E, G, q, Ab respectively have the meanings as generally or specifically defined in the respective sections above, including technical solutions formed by the general, preferred, or specific definitions of each unit or group, as well as technical solutions formed by any combination of the general, preferred, or specific definitions.

In some embodiments, the ADC of formula (X2) of the present disclosure includes, but is not limited to: 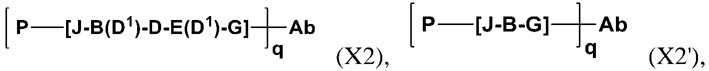 specifically having the structures shown in Table 9:

| |
|---|
| 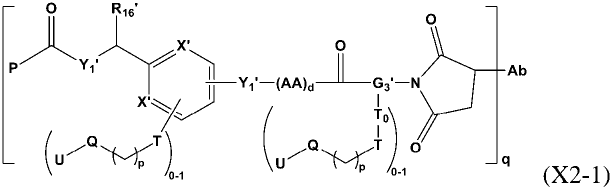 |
| 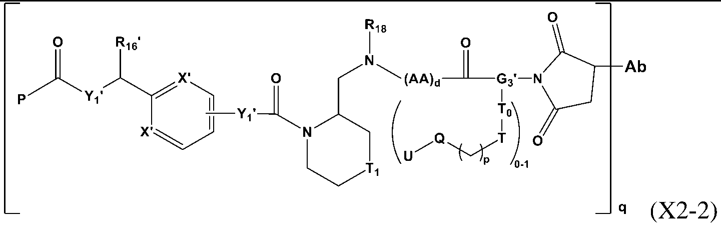 |
| 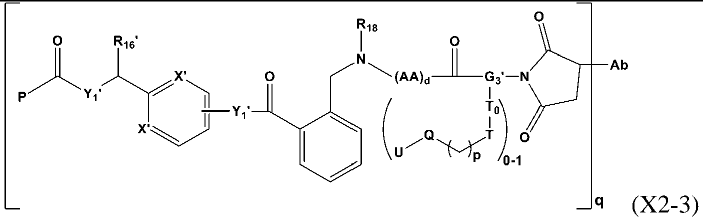 |
| 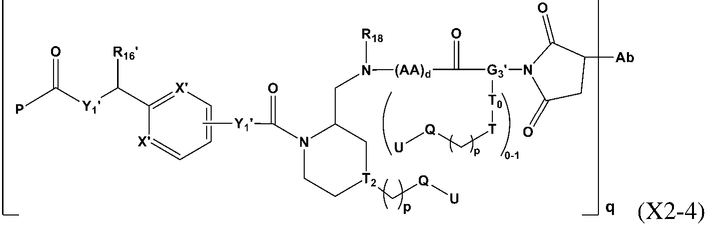 |
| 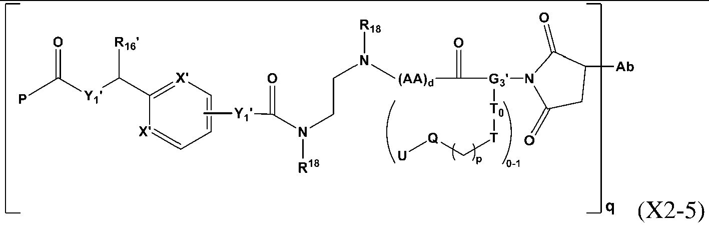 |
| 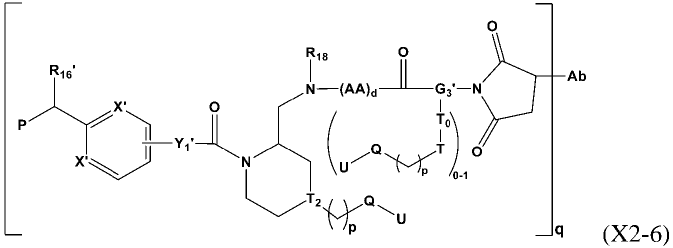 |
| 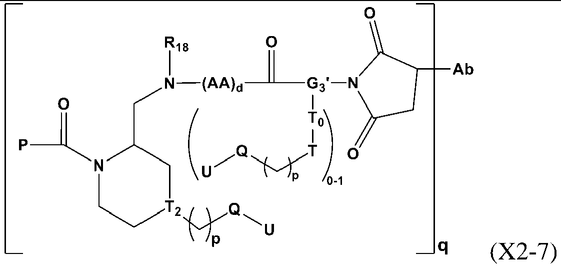 |
| 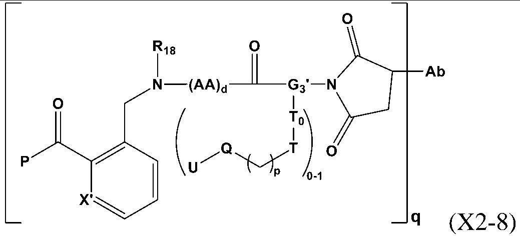 |
| 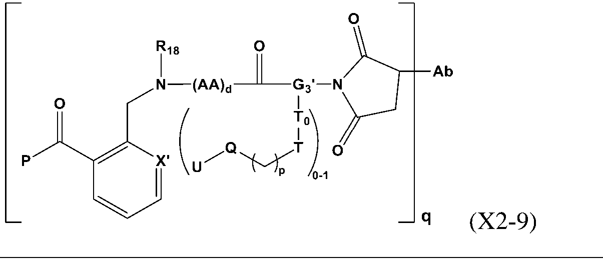 |
| 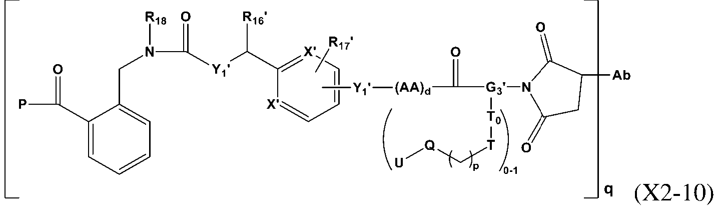 |
| 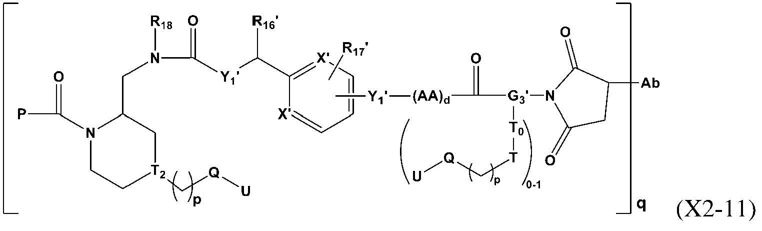 |
| 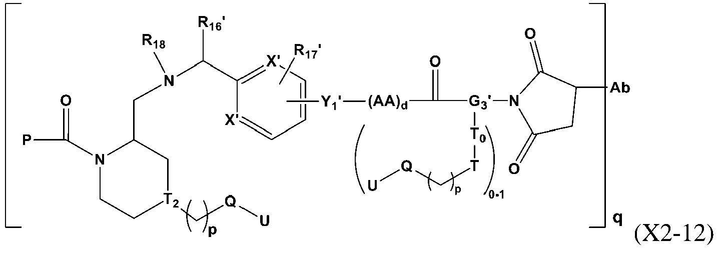 |
| 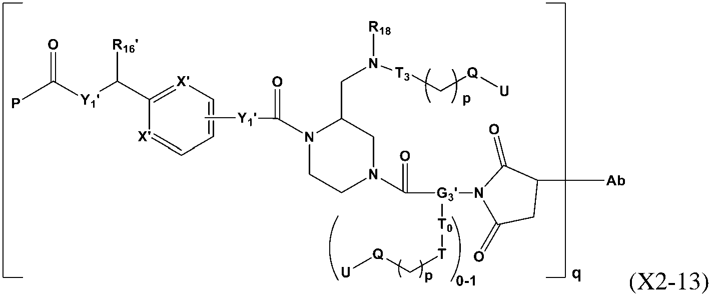 |
| and the corresponding respective general formula fragments where the maleimide moiety is |
| 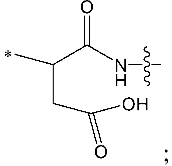 |

wherein the drug P unit is selected from the Ras inhibitor compounds defined in any one of Embodiments 1-8 of the Drug P Unit, preferably the P1-P37 compounds defined in Embodiments 7-8, or a stereoisomer, tautomer, stable isotopic variant, pharmaceutically acceptable salt, or solvate thereof; and
wherein each variable in the linker unit is as generally or specifically defined in the corresponding sections above, and the linker unit has the structure as generally or specifically exemplified in the corresponding sections above.

In some embodiments, q in the ADC of formula (X1) or (X2) and their various sub-formulas of the present disclosure represents an average DAR value that is an integer of at least 1, for example 1 to 10, or a range formed by any two values between 1 and 10, such as about 1, 2, 3, 4, 5, 6, 7, 8, 9, 10, about 1 to 2, 1 to 3, 1 to 4, 1 to 5, 1 to 6, 1 to 7, 1 to 8, 1 to 9, 1 to 10, 2 to 10, 2 to 8, 2 to 6, 2 to 4, 3 to 10, 3 to 8, 4 to 6, 4 to 8, 4 to 10, 6 to 8, or 6 to 10.

In some preferred embodiments, q in the ADC of formula (X1) or (X2) and their various sub-formulas of the present disclosure represents an average DAR value of 1 to 8, or a range formed by any two values between 1 and 8, such as about 1, 2, 3, 4, 5, 6, 7, 8, 1 to 2, 1 to 3, 1 to 4, 1 to 5, 1 to 6, 1 to 7, 1 to 8, 2 to 8, 2 to 6, 2 to 4, 3 to 10, 3 to 8, 4 to 6, 4 to 8, or 6 to 8.

In some preferred embodiments, q represents an average DAR of about 3. In some preferred embodiments, q represents an average DAR of about 6. In some preferred embodiments, q represents an average DAR of about 8.

In some embodiments, q represents an average DAR of about 1-10, 1-8, 2-8, 3-10, 3-8, 4-10, 4-8, 6-8, or 6-10.

The Ab in the ADC of formula (X) and its various sub-formulas of the present disclosure is an antibody or antigen-binding fragment that binds to a tumor-specific antigen or tumor-associated antigen.

In some preferred embodiments, the Ab in the ADC of formula (X) and its various sub-formulas of the present disclosure binds to a tumor-specific antigen or tumor-associated antigen selected from: HER2, Her3, HER1 (ErbB1), HER4 (ErbB4), TROP2, Nectin-4, tissue factor, PD-L1, PD-1, PD-L1/PD-L2, MET, CLDN18.2, KIT, CTLA-4, RPR1, ephrin A2 receptor (EphA2), folate receptor (FRa), mesothelin, endothelin receptor, GCPII, IL-13Ra, BCMA, GD2, CLL-1, CA-IX, MUC1, 5T4, AOC3, ALK, AXL, C242, CA-125, CCL11, CCR5, CD2, CD3, CD4, CDS, CD15, CA15-3, CD16, CD18, CD19, CD20, CD21, CD22, CD25, CD30, CD32, CD33, CD37, CD38, CD44, CD52, CD56, CD64, CD66e, CD70, CD72, CD74, CD79a, CD79b, CD123, CD138, CD142, CD174, CD276, CDH3, CCD79b, CLDN9/CLDN6, CA19-9, DPEP3, AGS-16, IGF1R, IGF2R, VEGFR1, VEGFR2, VEGFR3, PDGFR-α, PDGFR-β, EGFR, EGFRvIII, ENPP3, FcRH5, FRα, KAAG1, LIV-1, Mesothelin, cMet, ROR1, SLTRK6, TF, BMPR1B, E16, TOP1, STEAP1, 0772P, MUC16, Napi3b, Sema 5b, PSCA hIg, ETBR, RNF124, prostate cancer-associated gene 1, TrpM4, teratocarcinoma-derived growth factor 1, C3DR, FcRH2, NCA, MDP, IL20R-α, Brevican, EphB2R, ASLG659, prostate stem cell antigen precursor, GEDA, BAFF-R, CXCR5, HLA-DOB, P2X5, LY64, FcRH1, IRTA2, TENB2, integrin α5β6, integrin α4β7, FGF2, FGFR1, FGFR2, FGFR3, FGFR4, PSMA, Somatostatin receptor, RANK, SLAMF7, ITGB6, CEACAM5, CA9, EGFRvlll, IL2RA, AXL receptor tyrosine kinase, TGF-βR, TNFRSF8, cancer/testis-associated antigens, CLEC14A, GRP78, stem cell-specific antigens, ASG-5, PRR4, GUCY2C, SLC39A6, TPBG, tumor-associated antigen CA242, FOLR1, GPNMB, HAVCR1, prostate tumor target Mindin, VTCN1, PTK7 protein tyrosine kinase 7, macrophage stimulating 1 receptor, TACSTD2, CA6, DLL3, DLL4, EpCAM, FAP, DKK-1, Endoglin, VCAM1, GPC3, DR5, ASCT2, B7H1, B7H3, B7H4.

In some preferred embodiments, the Ab in the ADC of formula (X) and its various sub-formulas of the present disclosure binds to an antigen selected from AXL, B7H1, B7H3, B7H4, BCMA, CD16, CD19, CD22, CD25, CD30, CD32, CD33, CD44, CD64, CD70, CD74, CD79, CD138, CD142, CD276, CDH3, CEACAM5, Claudin 18.2, CLDN9/CLDN6, DPEP3, EGFR, ENPP3, EphA, FcRH5, FOLR1, FRα, GCPII, HER2, HER3, KAAG1, KIT, LIV-1, Mesothelin, cMet, MUC1, Nectin-4, PD-L1, PD-L1/PD-L2, PSMA, ROR1, RPR1, TF, TOP1, TROP2.

In some more preferred embodiments, the antibody in the ADC of the present disclosure is one that binds to an antigen preferentially expressed or overexpressed in cancer cells, such as HER2 (ErbB2), PD-1, PD-L1, EGFR, TROP2, Nectin-4, Claudin 18.2, EphA-2, Met, or a combination of EGFR and Met, more preferably HER2 (ErbB2), EGFR, TROP2, Claudin 18.2, Nectin-4, or a combination of EGFR and Met.

In any of the ADC embodiments generally or specifically defined above that do not carry a solubilizing unit, e.g., a solubilizing sugar unit, the corresponding antibody can be an antibody as generally or specifically defined herein, preferably a hydrophilic antibody; in any of the ADC embodiments generally or specifically defined above that carry a solubilizing unit, e.g., a solubilizing sugar unit, the corresponding antibody can be an antibody as generally or specifically defined herein, preferably a hydrophobic antibody.

In a preferred group of embodiments, the Ab in the ADC of formula (X) and its various sub-formulas of the present disclosure is an antibody or antigen-binding fragment that specifically binds to HER2.

In this preferred group of embodiments, the Ab in the ADC of the present disclosure comprises 3 heavy chain complementarity determining regions (HCDR) and 3 light chain complementarity determining regions (LCDR) that specifically bind to HER2, wherein: according to Kabat definition,
HCDR1 comprises or consists of the amino acid sequence of SEQ ID NO: 6,
HCDR2 comprises or consists of the amino acid sequence of SEQ ID NO: 7,
HCDR3 comprises or consists of the amino acid sequence of SEQ ID NO: 8,
LCDR1 comprises or consists of the amino acid sequence of SEQ ID NO: 3,
LCDR2 comprises or consists of the amino acid sequence of SEQ ID NO: 4, and
LCDR3 comprises or consists of the amino acid sequence of SEQ ID NO: 5.

In this preferred group of embodiments, the Ab in the ADC of the present disclosure comprises a heavy chain variable region and a light chain variable region, wherein the heavy chain variable region comprises the amino acid sequence described in SEQ ID NO: 10, and wherein the light chain variable region comprises the amino acid sequence shown in SEQ ID NO: 9.

In this preferred group of embodiments, the Ab in the ADC of the present disclosure comprises:
(a) a heavy chain comprising the amino acid sequence of SEQ ID NO: 2, and
(b) a light chain comprising the amino acid sequence of SEQ ID NO: 1.

In this preferred group of embodiments, the Ab in the ADC of the present disclosure is Trastuzumab.

In another preferred group of embodiments, the Ab in the ADC of formula (X) and its various sub-formulas of the present disclosure is an antibody or antigen-binding fragment that specifically binds to Trop-2.

In this preferred group of embodiments, the Ab in the ADC of the present disclosure comprises 3 heavy chain complementarity determining regions (HCDR) and 3 light chain complementarity determining regions (LCDR) that specifically bind to Trop-2, wherein: according to Kabat definition,
HCDR1 comprises or consists of the amino acid sequence of SEQ ID NO: 18,
HCDR2 comprises or consists of the amino acid sequence of SEQ ID NO: 19,
HCDR3 comprises or consists of the amino acid sequence of SEQ ID NO: 20,
LCDR1 comprises or consists of the amino acid sequence of SEQ ID NO: 15,
LCDR2 comprises or consists of the amino acid sequence of SEQ ID NO: 16, and
LCDR3 comprises or consists of the amino acid sequence of SEQ ID NO: 17.

In this preferred group of embodiments, the Ab in the ADC of the present disclosure comprises a heavy chain variable region and a light chain variable region, wherein the heavy chain variable region comprises the amino acid sequence described in SEQ ID NO: 22, and wherein the light chain variable region comprises the amino acid sequence shown in SEQ ID NO: 21.

In this preferred group of embodiments, the Ab in the ADC of the present disclosure comprises:
(a) a heavy chain comprising the amino acid sequence of SEQ ID NO: 14, and
(b) a light chain comprising the amino acid sequence of SEQ ID NO: 13.

In this preferred group of embodiments, the Ab in the ADC of the present disclosure is Sacituzumab.

In another preferred group of embodiments, the Ab in the ADC of formula (X) and its various sub-formulas of the present disclosure is an antibody or antigen-binding fragment that specifically binds to Claudin 18.2.

In this preferred group of embodiments, the Ab in the ADC of formula (X) and its various sub-formulas of the present disclosure comprises 3 heavy chain complementarity determining regions (HCDR) and 3 light chain complementarity determining regions (LCDR) that specifically bind to Claudin18.2, wherein: according to Kabat definition,
HCDR1 comprises or consists of the amino acid sequence of SEQ ID NO: 29,
HCDR2 comprises or consists of the amino acid sequence of SEQ ID NO: 30,
HCDR3 comprises or consists of the amino acid sequence of SEQ ID NO: 31,
LCDR1 comprises or consists of the amino acid sequence of SEQ ID NO: 26,
LCDR2 comprises or consists of the amino acid sequence of SEQ ID NO: 27, and
LCDR3 comprises or consists of the amino acid sequence of SEQ ID NO: 28.

In this preferred group of embodiments, the Ab in the ADC of the present disclosure comprises a heavy chain variable region and a light chain variable region, wherein the heavy chain variable region comprises the amino acid sequence described in SEQ ID NO: 33, and wherein the light chain variable region comprises the amino acid sequence shown in SEQ ID NO: 32.

In this preferred group of embodiments, the Ab in the ADC of the present disclosure comprises:
(a) a heavy chain comprising the amino acid sequence of SEQ ID NO: 25, and
(b) a light chain comprising the amino acid sequence of SEQ ID NO: 24.

In this preferred group of embodiments, the Ab in the ADC of the present disclosure is Zolbetuximab.

In another preferred group of embodiments, the Ab in the ADC of formula (X) and its various sub-formulas of the present disclosure is an antibody or antigen-binding fragment that specifically binds to EGFR.

In this preferred group of embodiments, the Ab in the ADC of the present disclosure comprises 3 heavy chain complementarity determining regions (HCDR) and 3 light chain complementarity determining regions (LCDR) that specifically bind to EGFR, wherein: according to Kabat definition,
HCDR1 comprises or consists of the amino acid sequence of SEQ ID NO: 40,
HCDR2 comprises or consists of the amino acid sequence of SEQ ID NO: 41,
HCDR3 comprises or consists of the amino acid sequence of SEQ ID NO: 42,
LCDR1 comprises or consists of the amino acid sequence of SEQ ID NO: 37,
LCDR2 comprises or consists of the amino acid sequence of SEQ ID NO: 38, and
LCDR3 comprises or consists of the amino acid sequence of SEQ ID NO: 39.

In this preferred group of embodiments, the Ab in the ADC of the present disclosure comprises a heavy chain variable region and a light chain variable region, wherein the heavy chain variable region comprises the amino acid sequence described in SEQ ID NO: 44, and wherein the light chain variable region comprises the amino acid sequence shown in SEQ ID NO: 43.

In this preferred group of embodiments, the Ab in the ADC of the present disclosure comprises:
(a) a heavy chain comprising the amino acid sequence of SEQ ID NO: 36, and
(b) a light chain comprising the amino acid sequence of SEQ ID NO: 35.

In this preferred group of embodiments, the Ab in the ADC of the present disclosure is Cetuximab.

In another preferred group of embodiments, the Ab in the ADC of formula (X) and its various sub-formulas of the present disclosure is an antibody or antigen-binding fragment that specifically binds to EGFR and MET.

In this preferred group of embodiments, the Ab in the ADC of the present disclosure comprises a first set of complementarity determining regions that specifically bind EGFR and a second set of complementarity determining regions that specifically bind MET, wherein:
The first set of complementarity determining regions comprises 3 heavy chain complementarity determining regions (HCDR) and 3 light chain complementarity determining regions (LCDR), wherein: according to Kabat definition,
HCDR1 comprises or consists of the amino acid sequence of SEQ ID NO: 49,
HCDR2 comprises or consists of the amino acid sequence of SEQ ID NO: 50,
HCDR3 comprises or consists of the amino acid sequence of SEQ ID NO: 51,
LCDR1 comprises or consists of the amino acid sequence of SEQ ID NO: 52,
LCDR2 comprises or consists of the amino acid sequence of SEQ ID NO: 53, and
LCDR3 comprises or consists of the amino acid sequence of SEQ ID NO: 54,

The second set of complementarity determining regions comprises 3 heavy chain complementarity determining regions (HCDR) and 3 light chain complementarity determining regions (LCDR), wherein: according to Kabat definition,
HCDR1 comprises or consists of the amino acid sequence of SEQ ID NO: 55,
HCDR2 comprises or consists of the amino acid sequence of SEQ ID NO: 56,
HCDR3 comprises or consists of the amino acid sequence of SEQ ID NO: 57,
LCDR1 comprises or consists of the amino acid sequence of SEQ ID NO: 58,
LCDR2 comprises or consists of the amino acid sequence of SEQ ID NO: 59, and
LCDR3 comprises or consists of the amino acid sequence of SEQ ID NO: 60.

In this preferred group of embodiments, the Ab in the ADC of the present disclosure comprises an anti-EGFR heavy chain variable region and light chain variable region, and an anti-MET heavy chain variable region and light chain variable region, wherein the anti-EGFR heavy chain variable region and light chain variable region respectively comprise the amino acid sequences shown in SEQ ID NO: 61 and SEQ ID NO: 62, and wherein the anti-MET heavy chain variable region and light chain variable region respectively comprise the amino acid sequences shown in SEQ ID NO: 63 and SEQ ID NO: 64.

In this preferred group of embodiments, the Ab in the ADC of the present disclosure comprises:
(a) an anti-EGFR heavy chain and light chain comprising the amino acid sequences of SEQ ID NO: 45 and SEQ ID NO: 46, respectively, and
(b) an anti-MET heavy chain and light chain comprising the amino acid sequences of SEQ ID NO: 47 and SEQ ID NO: 48, respectively.

In this preferred group of embodiments, the Ab in the ADC of the present disclosure is Amivantamab.

In another preferred group of embodiments, the Ab in the ADC of formula (X) and its various sub-formulas of the present disclosure is an antibody or antigen-binding fragment that specifically binds to Nectin-4.

In this preferred group of embodiments, the Ab in the ADC of the present disclosure comprises 3 heavy chain complementarity determining regions (HCDR) and 3 light chain complementarity determining regions (LCDR) that specifically bind to Nectin-4, wherein: according to Kabat definition,
HCDR1 comprises or consists of the amino acid sequence of SEQ ID NO: 72,
HCDR2 comprises or consists of the amino acid sequence of SEQ ID NO: 73,
HCDR3 comprises or consists of the amino acid sequence of SEQ ID NO: 74,
LCDR1 comprises or consists of the amino acid sequence of SEQ ID NO: 69,
LCDR2 comprises or consists of the amino acid sequence of SEQ ID NO: 70, and
LCDR3 comprises or consists of the amino acid sequence of SEQ ID NO: 71.

In this preferred group of embodiments, the Ab in the ADC of the present disclosure comprises a heavy chain variable region and a light chain variable region, wherein the heavy chain variable region comprises the amino acid sequence described in SEQ ID NO: 76, and wherein the light chain variable region comprises the amino acid sequence shown in SEQ ID NO: 75.

In this preferred group of embodiments, the Ab in the ADC of the present disclosure comprises:
(a) a heavy chain comprising the amino acid sequence of SEQ ID NO: 68, and
(b) a light chain comprising the amino acid sequence of SEQ ID NO: 67.

In this preferred group of embodiments, the Ab in the ADC of the present disclosure is Enfortumab.

It should be noted that the ADC of the present disclosure encompasses ADCs obtained by any combination of any embodiment of the drug P unit as generally or specifically defined, any embodiment of the linker unit L as generally or specifically defined, and any embodiment of the antibody Ab as generally or specifically defined.

Specific examples of the ADC of the present disclosure include, but are not limited to, those shown in Table 9-1:

| | |
|---|---|
| ADC-1 | 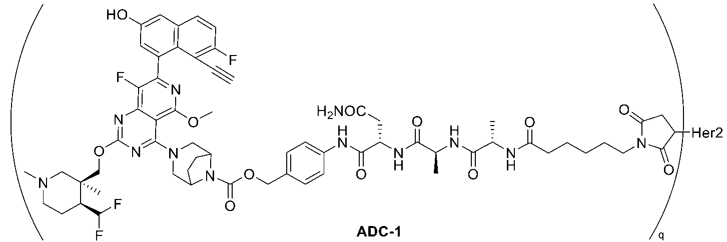 |
| ADC-2 | 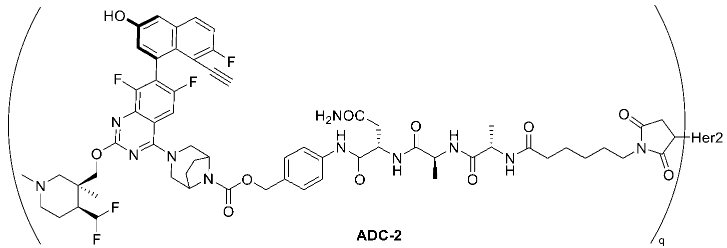 |
| ADC-3 | 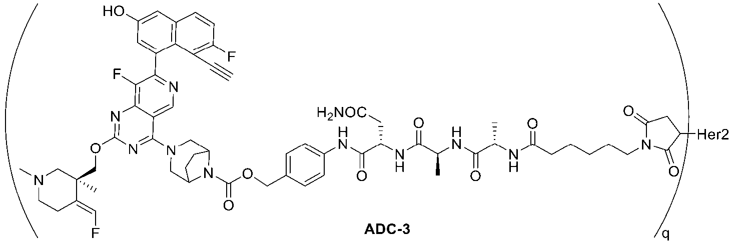 |
| ADC-4 | 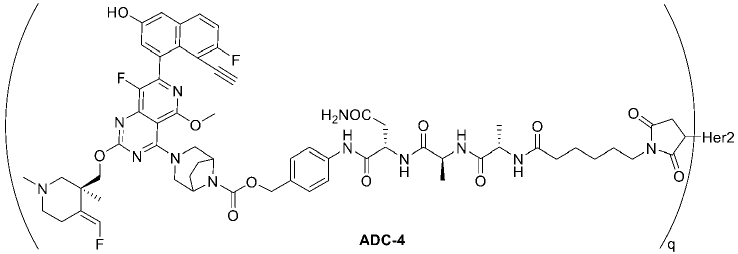 |
| ADC-5 | 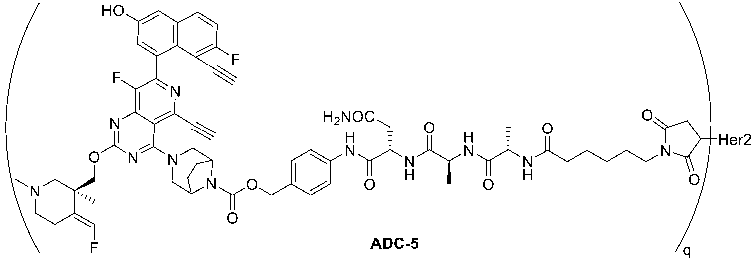 |
| ADC-6 | 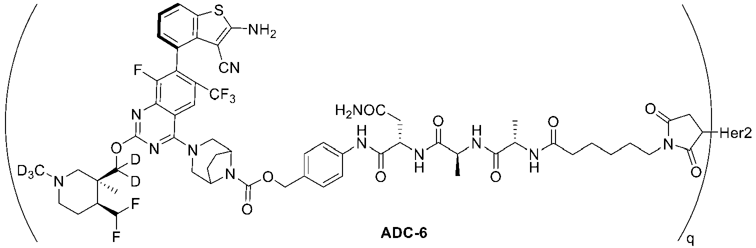 |
| ADC-7 | 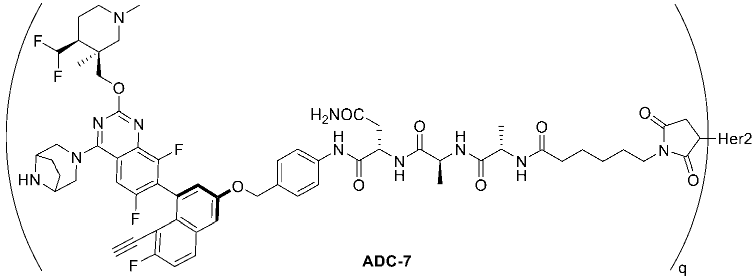 |
| ADC-8 | 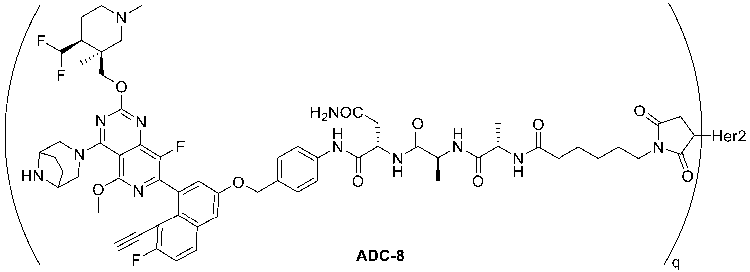 |
| ADC-9 | 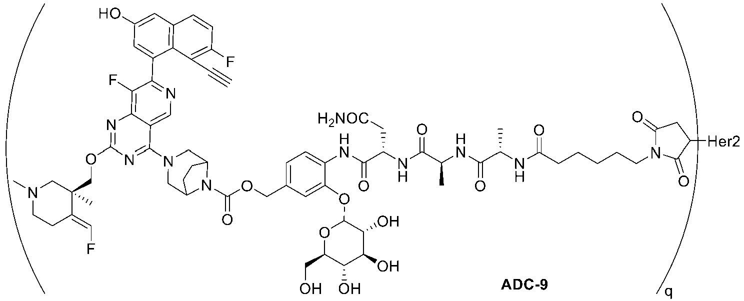 |
| ADC-10 | 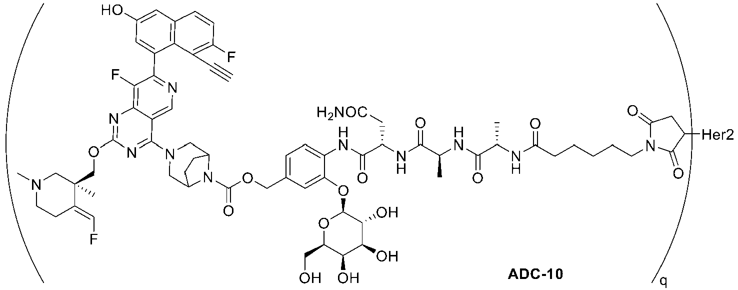 |
| ADC-11 | 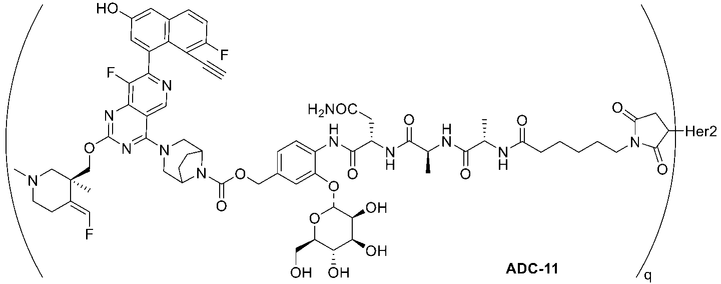 |
| ADC-12 | 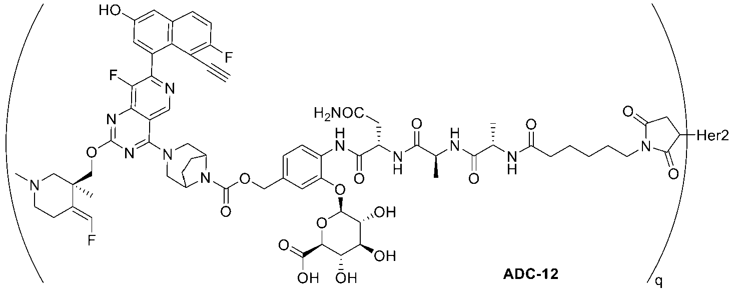 |
| ADC-13 | 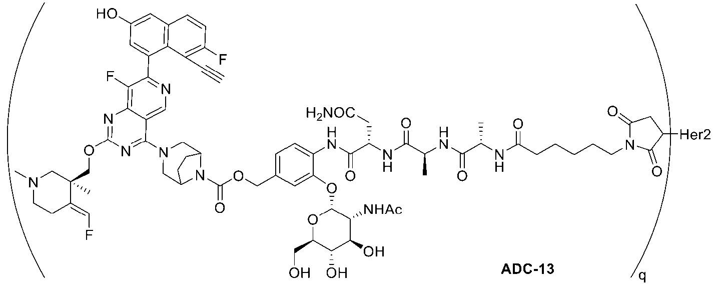 |
| ADC-14 | 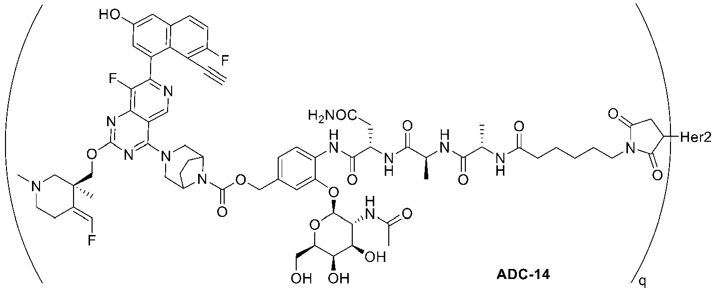 |
| ADC-15 | 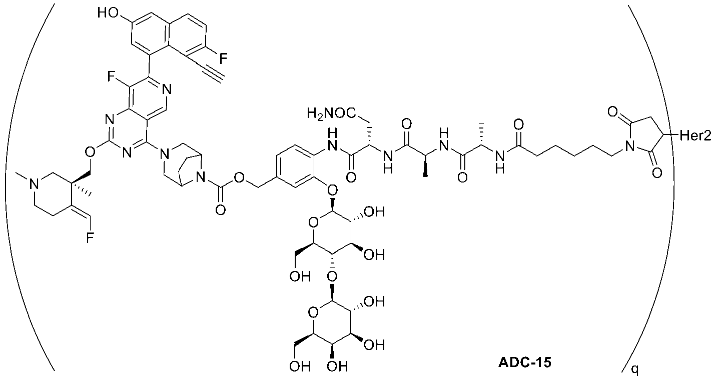 |
| ADC-16 | 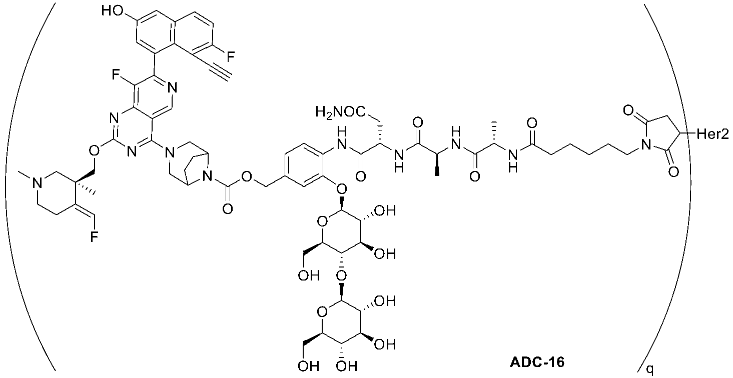 |
| ADC-17 | 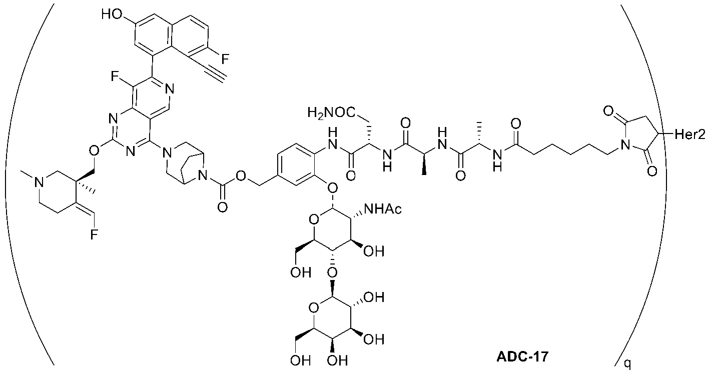 |
| ADC-18 | 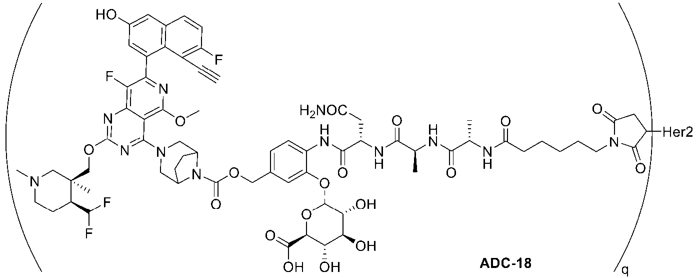 |
| ADC-19 | 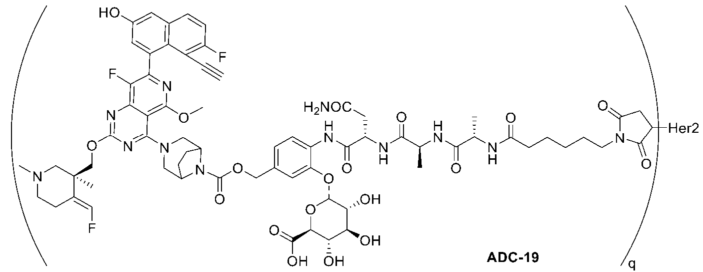 |
| ADC-20 | 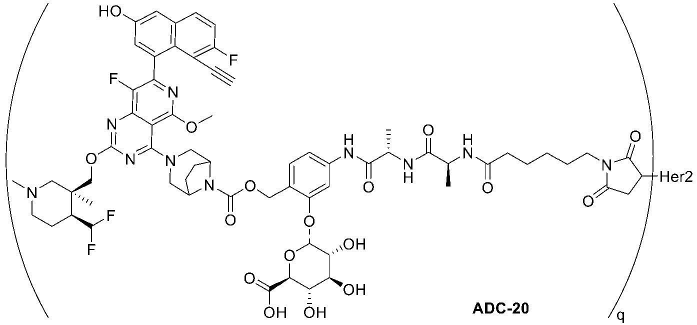 |
| ADC-21 | 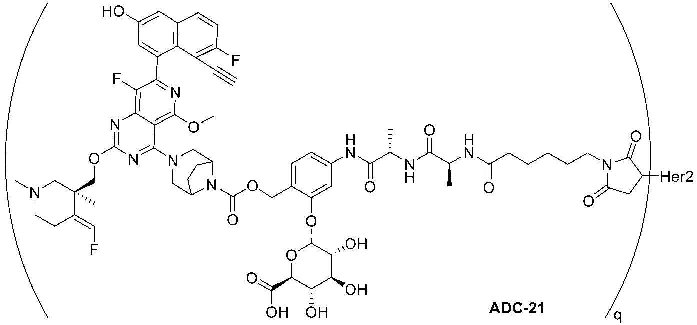 |
| ADC-22 | 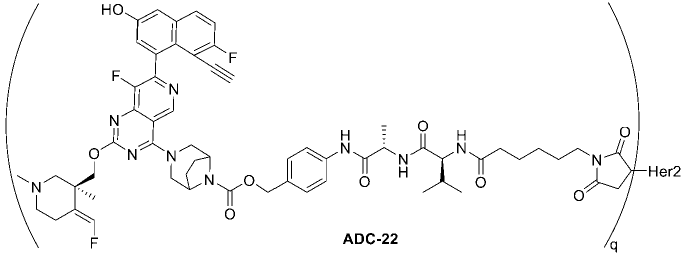 |
| ADC-23 | 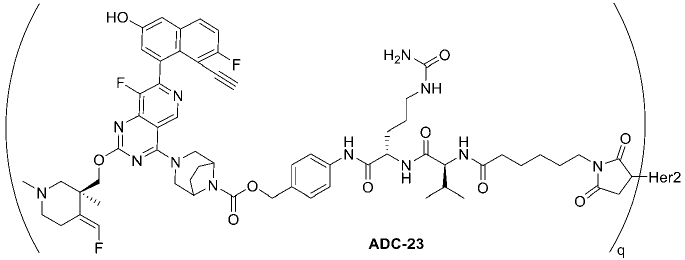 |
| ADC-24 | 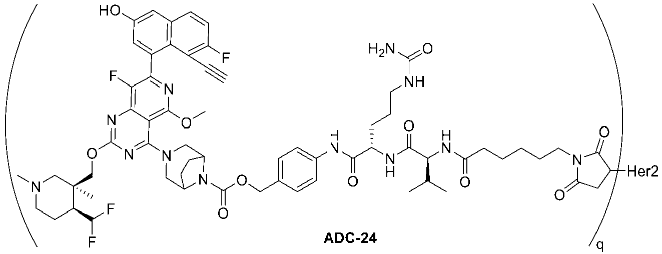 |
| ADC-25 | 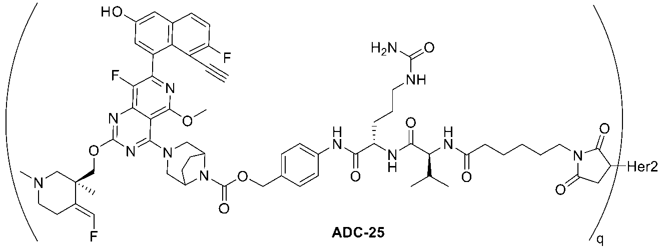 |
| ADC-26 | 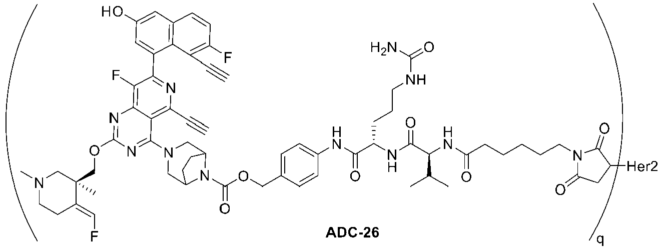 |
| ADC-27 | 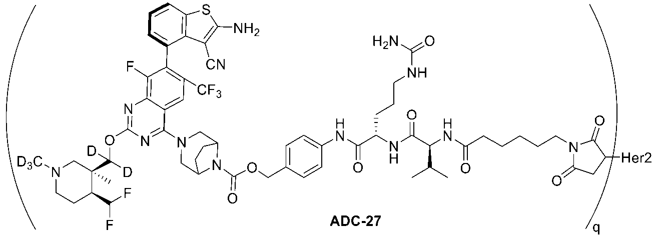 |
| ADC-28 | 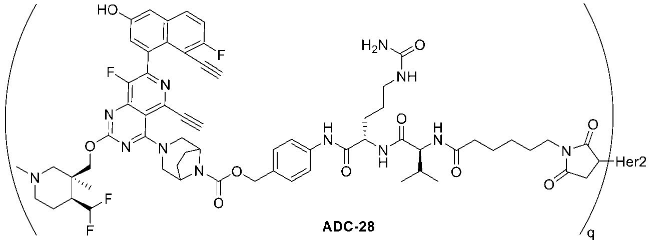 |
| ADC-29 | 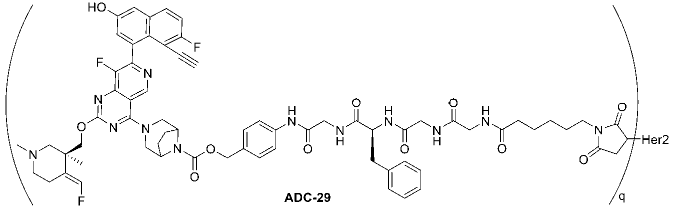 |
| ADC-30 | 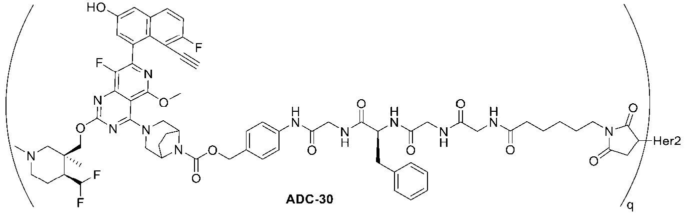 |
| ADC-31 | 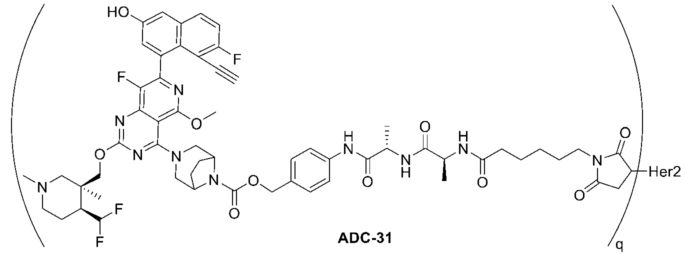 |
| ADC-32 | 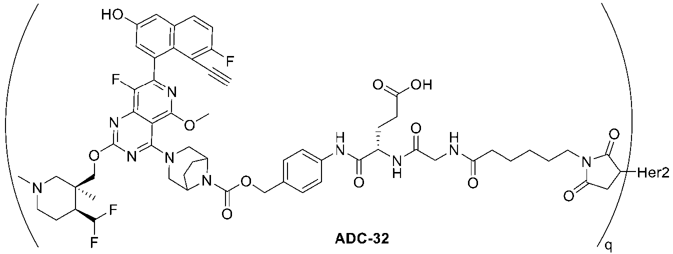 |
| ADC-33 | 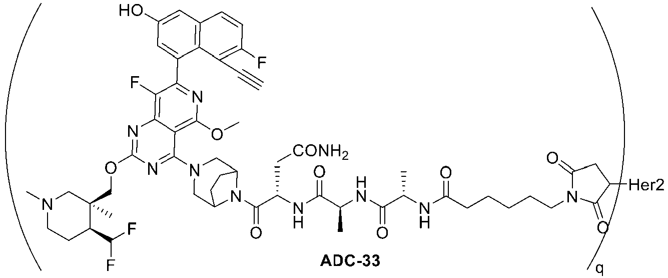 |
| ADC-34 | 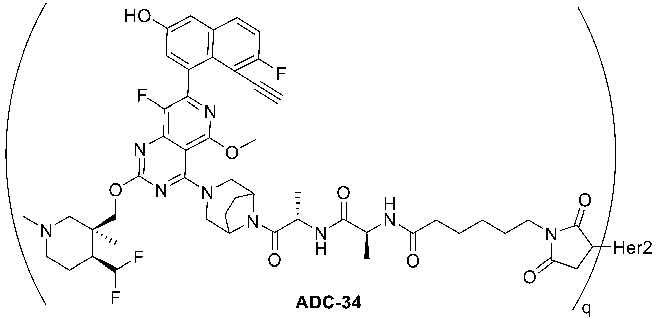 |
| ADC-35 | 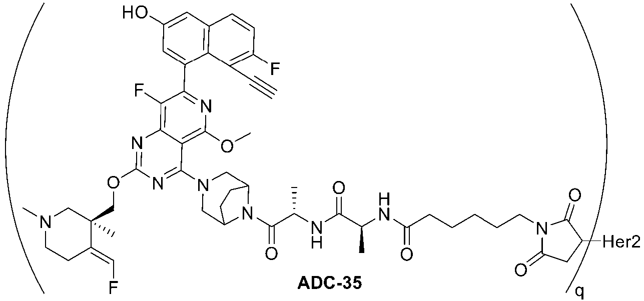 |
| ADC-36 | 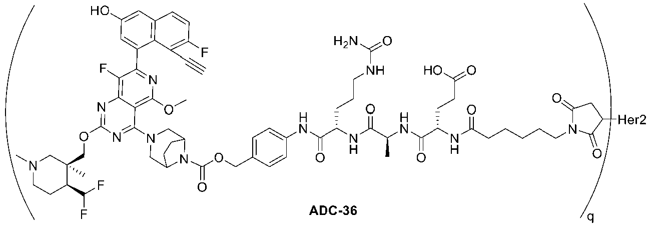 |
| ADC-37 | 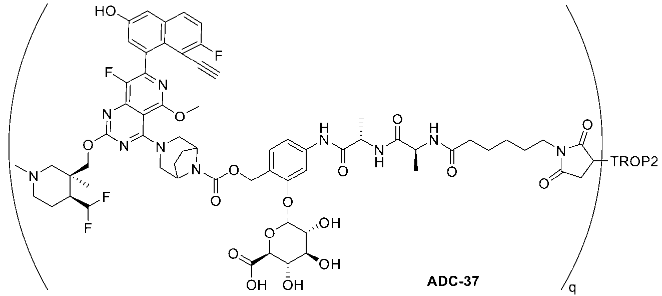 |
| ADC-38 | 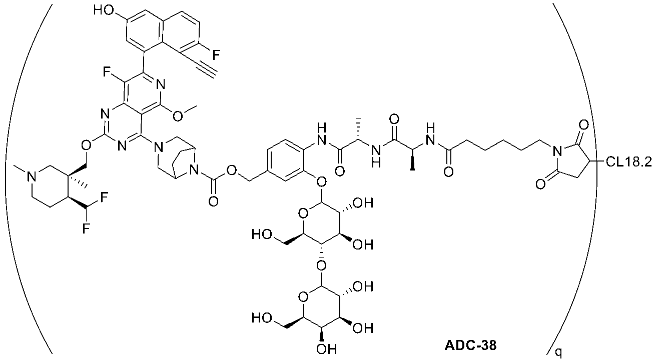 |
| ADC-39 | 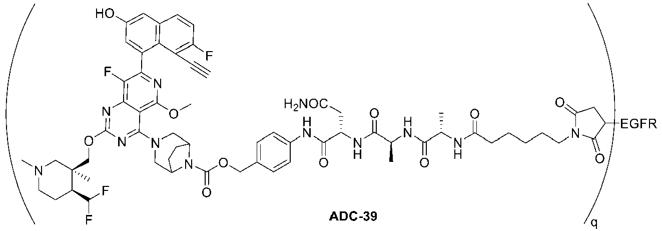 |
| ADC-40 | 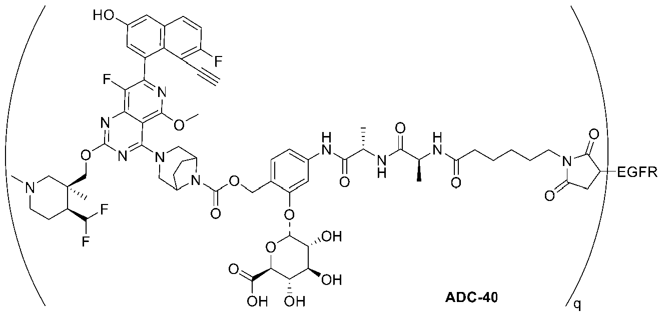 |
| ADC-41 | 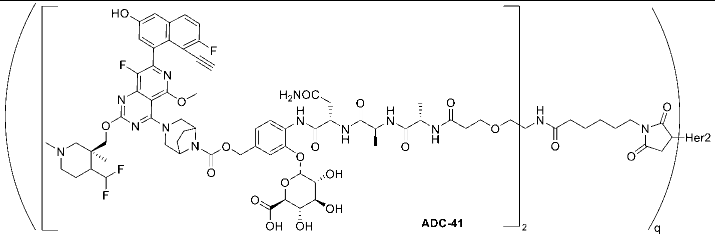 |
| ADC-42 | 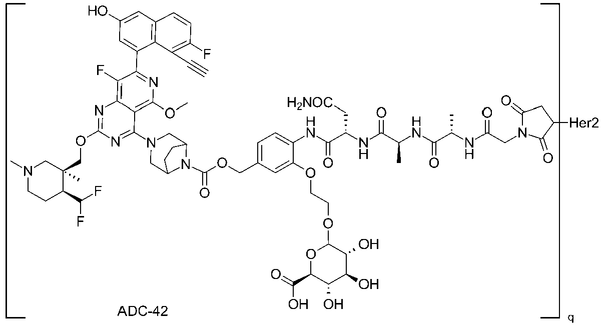 |
| ADC-43 | 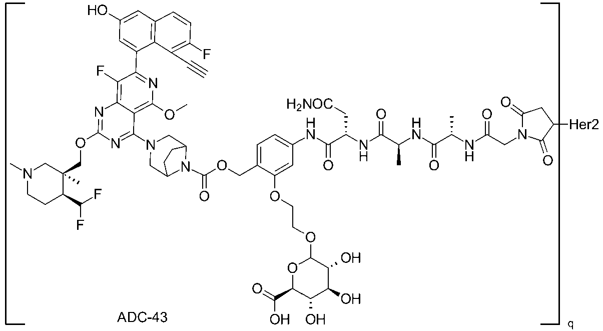 |
| ADC-44 | 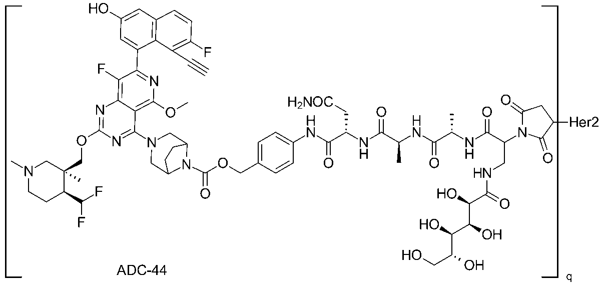 |
| ADC-45 | 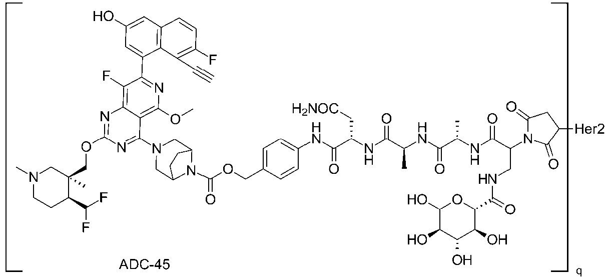 |
| ADC-46 | 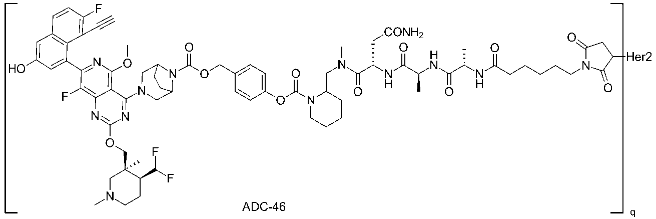 |
| ADC-47 | 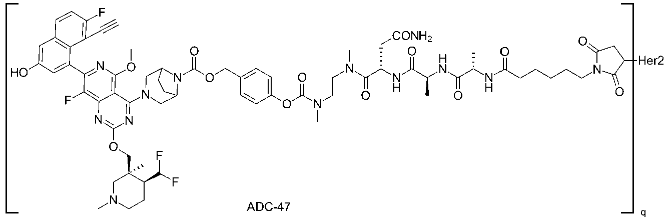 |
| ADC-48 | 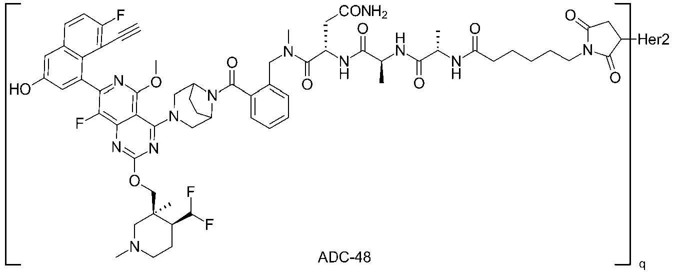 |
| ADC-49 | 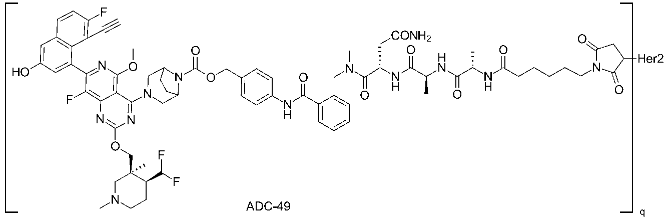 |
| ADC-50 | 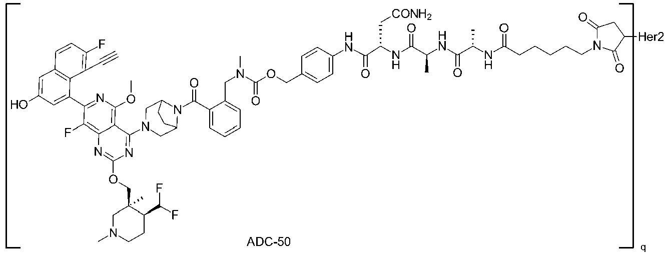 |
| ADC-51 | 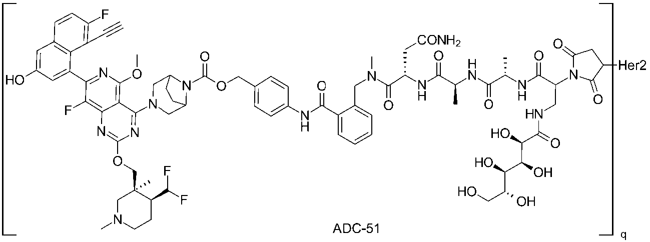 |
| ADC-52 | 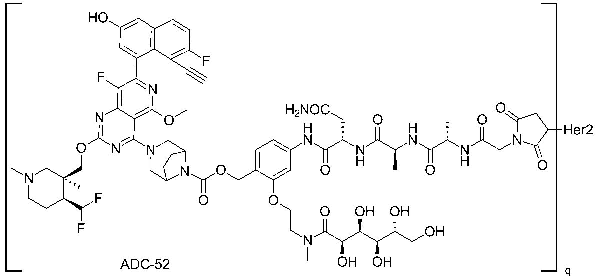 |
| ADC-53 | 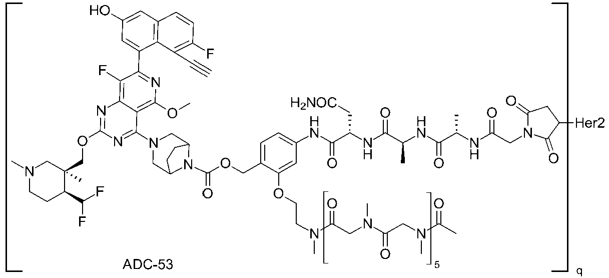 |
| ADC-54 | 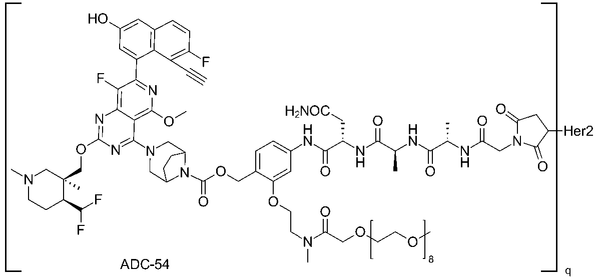 |
| ADC-55 | 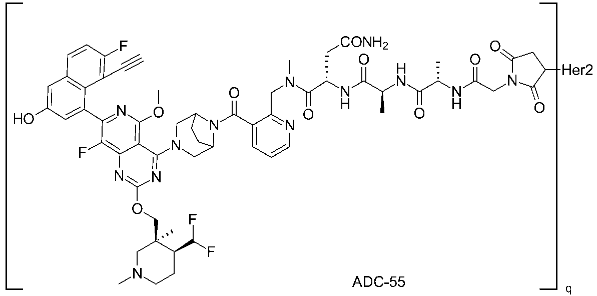 |
| ADC-56 | 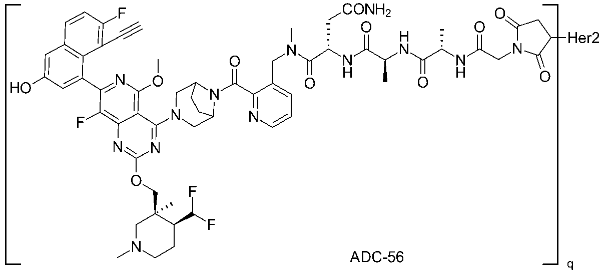 |
| ADC-57 | 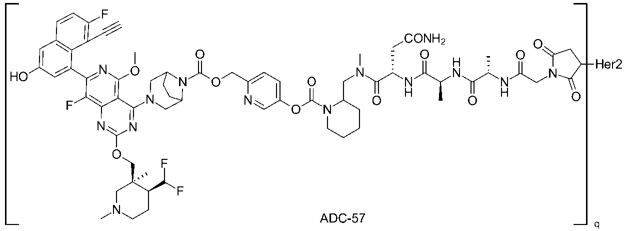 |
| ADC-58 | 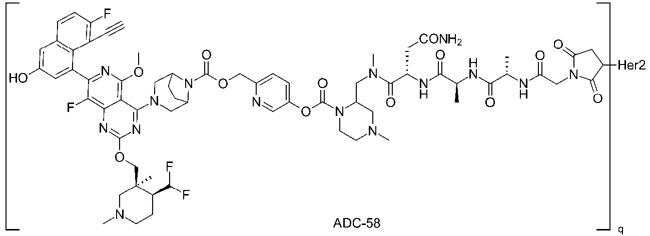 |
| **ADC-**59 | 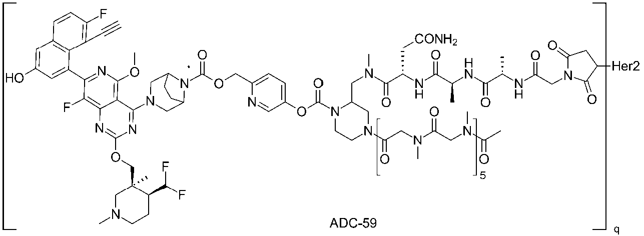 |
| ADC-60 | 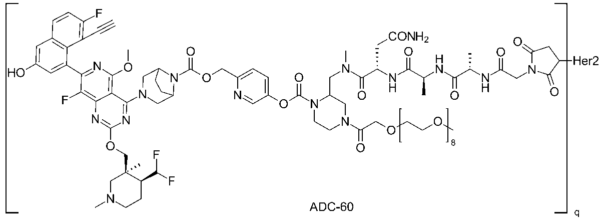 |
| ADC-61 | 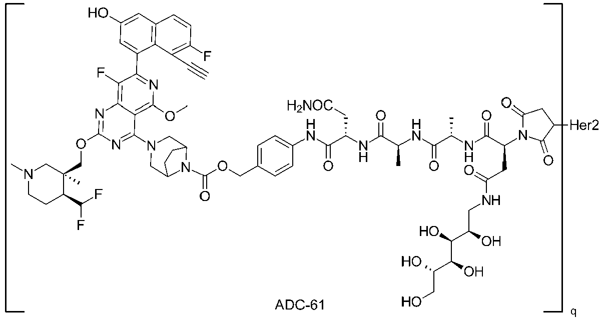 |
| ADC-62 | 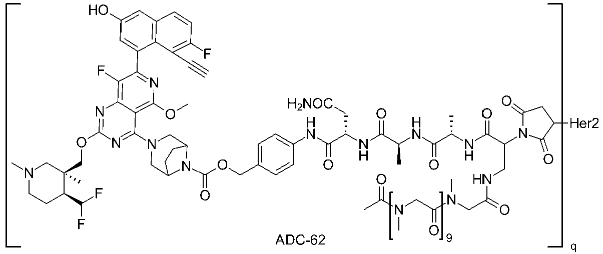 |
| ADC-63 | 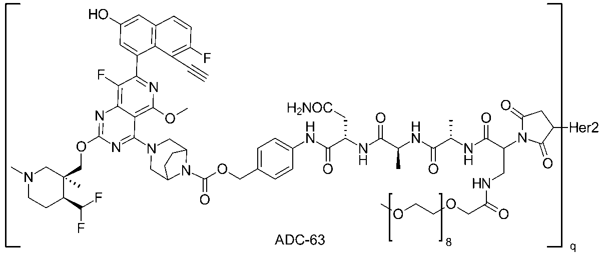 |
| ADC-64 | 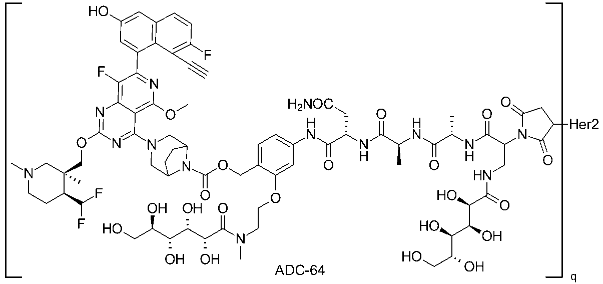 |
| ADC-65 | 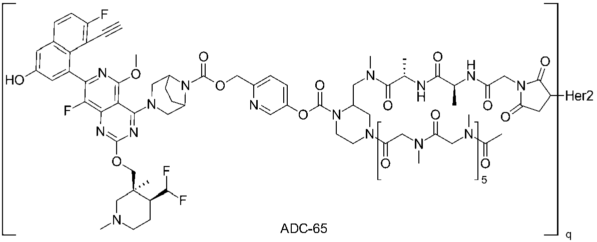 |
| ADC-66 | 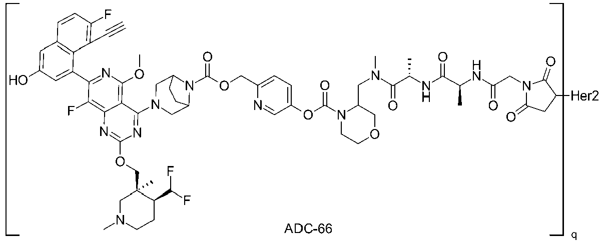 |
| ADC-67 | 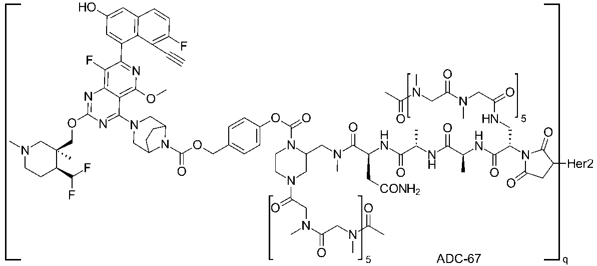 |
| ADC-68 | 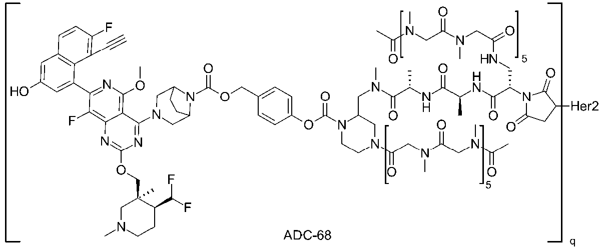 |
| ADC-69 | 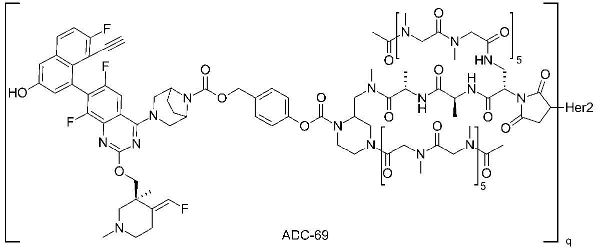 |
| ADC-70 | 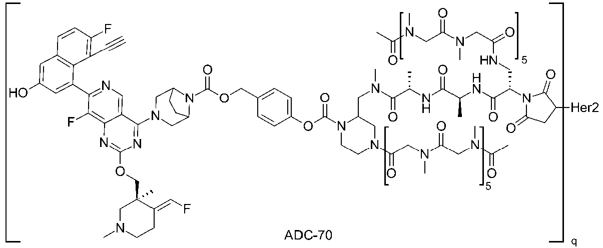 |
| ADC-71 | 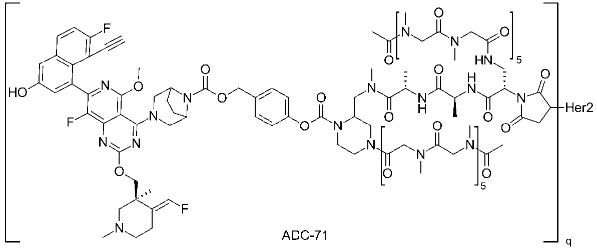 |
| ADC-72 | 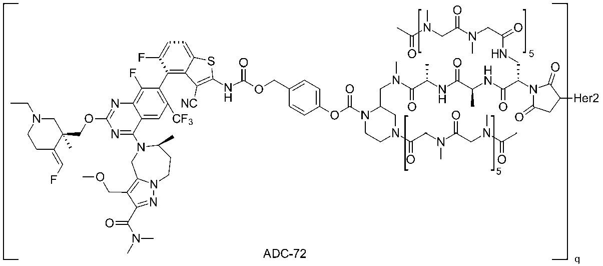 |
| ADC-73 | 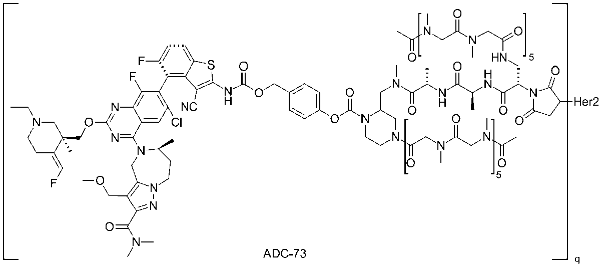 |
| ADC-74 | 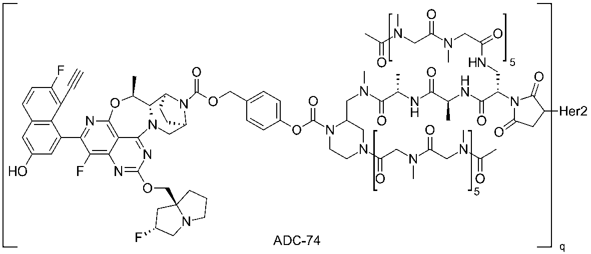 |
| ADC-75 | 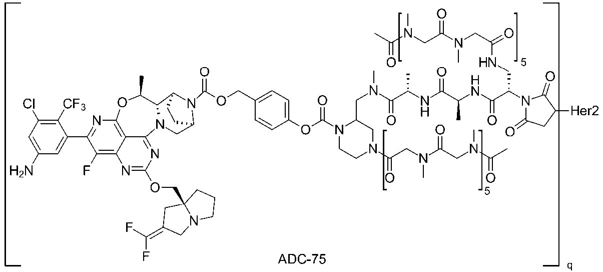 |
| ADC-76 | 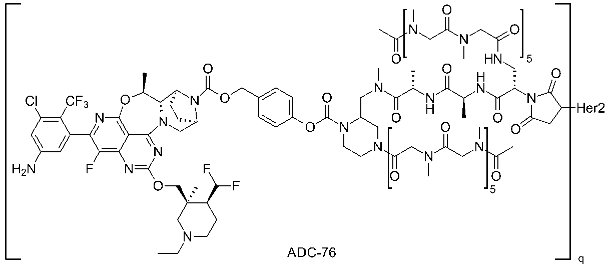 |
| ADC-77 | 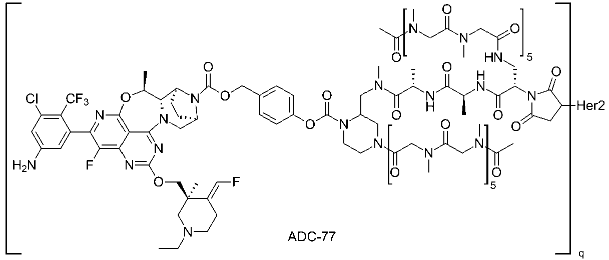 |
| ADC-78 | 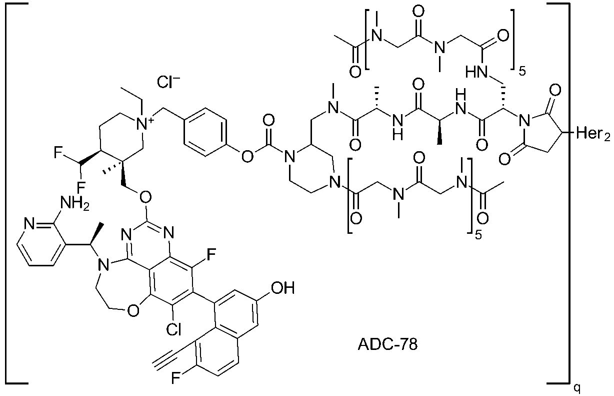 |
| ADC-79 | 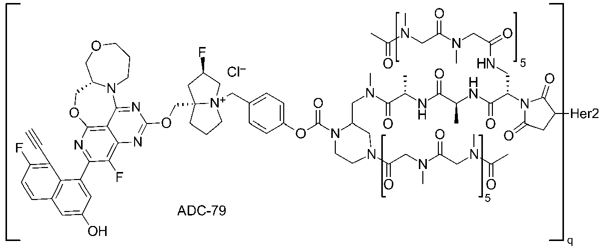 |
| ADC-80 | 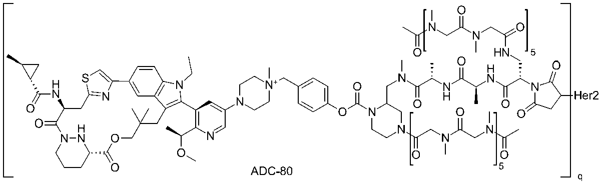 |
| ADC-81 | 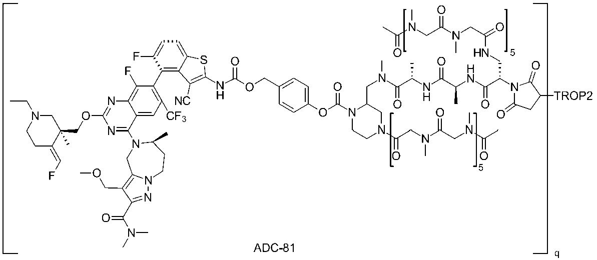 |
| ADC-82 | 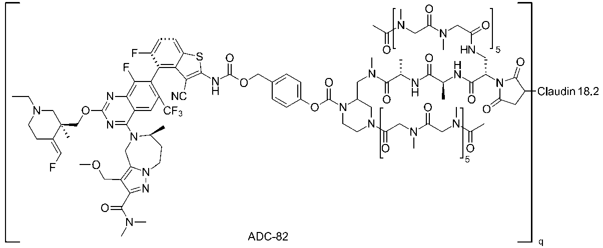 |
| ADC-83 | 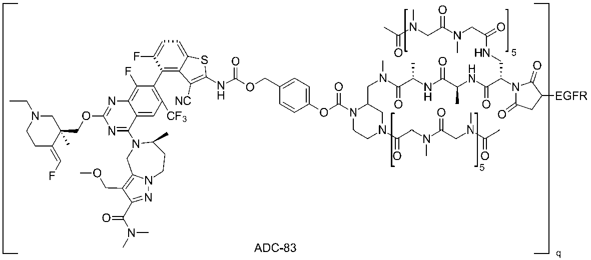 |
| ADC-84 | 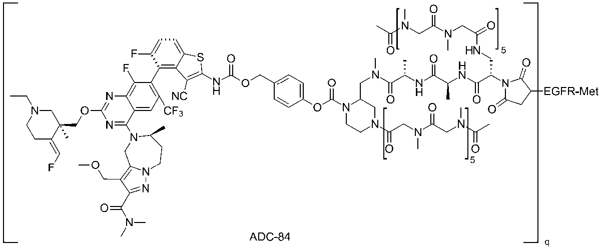 |
| ADC-85 | 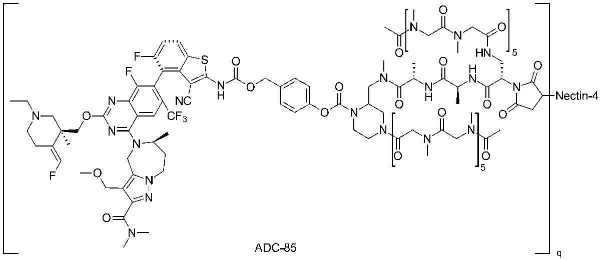 |
| ADC-86 | 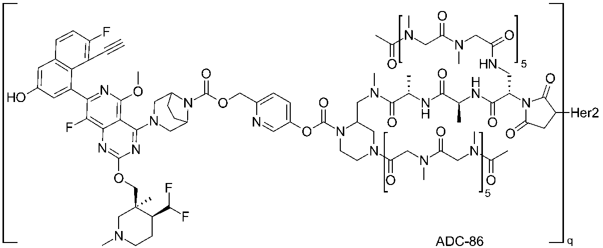 |
| ADC-87 | 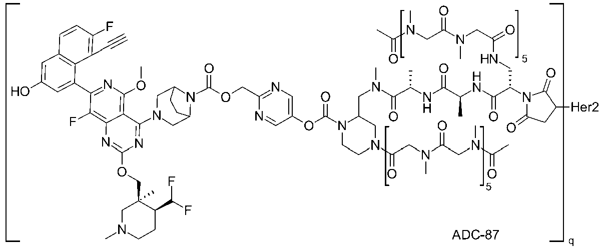 |
| ADC-88 | 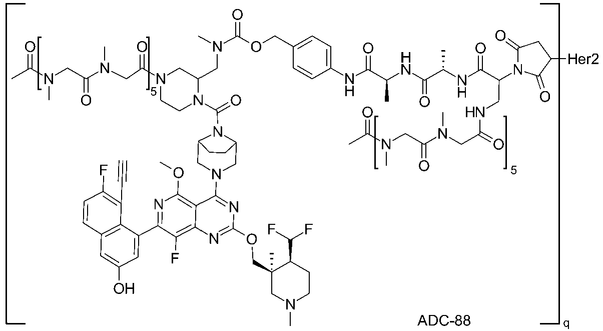 |
| ADC-89 | 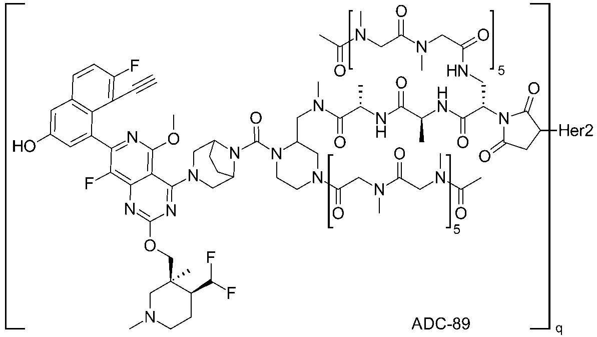 |
| ADC-90 | 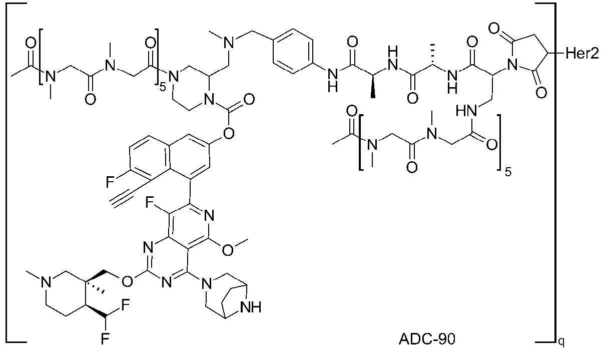 |
| ADC-91 | 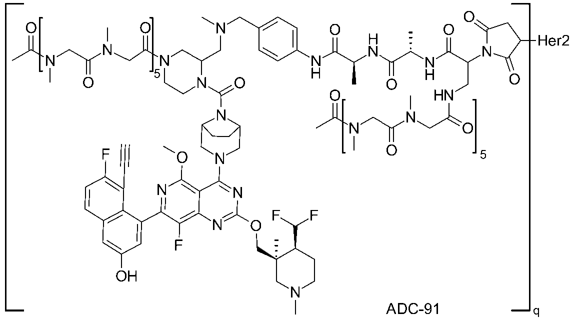 |
| ADC-92 | 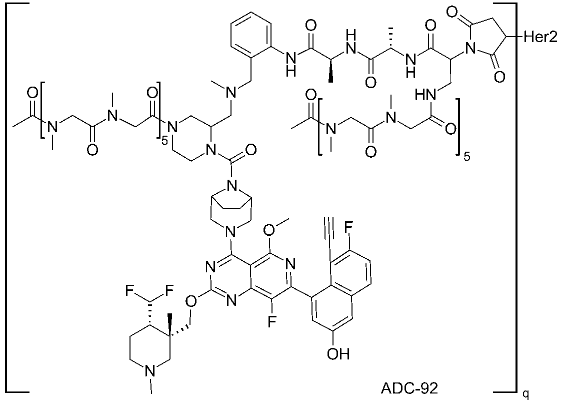 |
| ADC-93 | 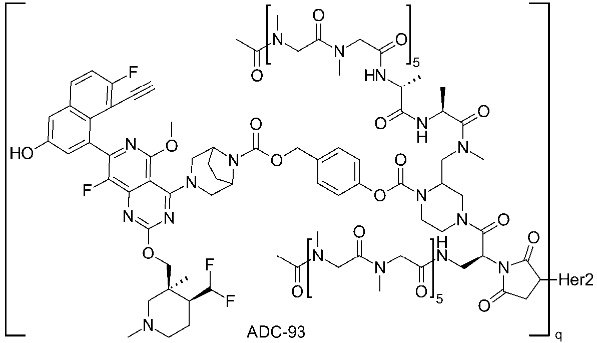 |
| ADC-94 | 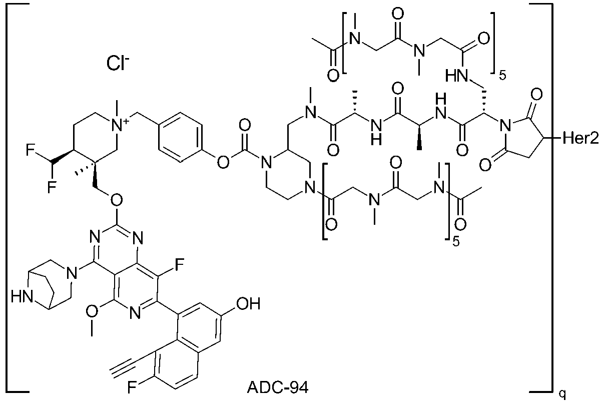 |
| and the corresponding ADCs where the maleimide moiety is 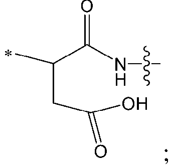 | |
| wherein, | |
| the antibody indicated as Her2 is specifically Trastuzumab; | |
| the antibody indicated as CL18.2 is specifically Zolbetuximab; | |
| the antibody indicated as TROP2 is specifically Sacituzumab; | |
| the antibody indicated as EGFR is specifically Cetuximab; | |
| the antibody indicated as EGFR-Met is specifically Amivantamab; | |
| the antibody indicated as Nectin-4 is specifically Enfortumab; | |
| q represents 1 to 20, for example 1-10, 1-8, 2-8, 3-10, 3-8, 4-10, 4-8, 6-8, or 6-10, | |

stereoisomers, tautomers, stable isotopic variants, pharmaceutically acceptable salts, or solvates thereof;

In preferred embodiments, the ADCs of the present disclosure are those specifically implemented in Examples 1~94 in the "6.4 Synthesis of ADCs" section of the Examples below;

In the above ADC technical solutions, the average DAR is 1-10, 1-8, 2-8, 3-10, 3-8, 4-10, 4-8, 6-8, or 6-10.

In the above ADC technical solutions, q represents about 1-10, 1-8, 2-8, 3-10, 3-8, 4-10, 4-8, 6-8, or 6-10.

### II: Beneficial Effects of the ADC of the Present Disclosure

The ADCs according to the present disclosure possess at least one or more of the following advantages:
➢ Using enzyme inhibitors as the drug payload for ADCs, particularly inhibitors targeting Ras, e.g., KRas mutant proteins, significantly distinguishes from classical ADC drug designs that typically use potent cytotoxins as payloads. They combine the selectivity of enzyme inhibitors and the selectivity of antibodies, i.e., dual selectivity, which can significantly reduce drug toxicity. Ras inhibitors suitable for the ADCs of the present disclosure include not only G12D inhibitors but also pan-Ras inhibitors, such as the pan-Ras inhibitors exemplified in the context of the present disclosure, for example but not limited to P8-P9, P12-P13, P16-P23, P25-P37.
➢ Ingeniously incorporating solubilizing units, such as solubilizing sugar units and/or hydrophilic peptides, into different components of the ADC linker unit improves the hydrophilicity of non-polar payloads, maximizes conjugation reaction efficiency (DAR), facilitates purification of the conjugation product, and effectively inhibits ADC macromolecule aggregation, thereby improving efficacy.
➢ Using new linker technology to stabilize imines or induce imine ring opening to linear free acids, preventing the conjugated chain from falling off via Retro-Michael, reducing the number of free payload molecules in the system and lowering toxicity.
➢ ADC macromolecules can delay the metabolism of drug payload molecules, improving the systemic stability and PK properties of the drug.
➢ Utilizing antibody internalization via tumor cell receptors addresses delivery and membrane permeability issues in targeted organs.
➢ ADC drugs have a long half-life in vivo and sustained target inhibition, helping to prolong efficacy and delay the emergence of drug resistance.
➢ No significant aggregation, allowing for high drug loading. Higher DAR values can be achieved through solubilizing units, e.g., branched solubilizing sugar units; DAR can be as high as 8.
➢ Demonstrated significant tumor growth inhibitory activity in animal models compared to the separate antibody and separate drug P unit alone.
➢ Showed a wide safety window in in vivo pharmacodynamic models.

Specifically, the targeted inhibitory activity of the ADC compounds of the present disclosure can be that, upon adding the compound of the present disclosure to the culture medium of tumor cells with high expression of a specific target, the proliferation ability of said tumor cells with high expression of the specific target is reduced by more than 1%, more than 5%, more than 20%, more than 50%, or even 90%, 95% compared to adding negative control or control drug. For example, the targeted inhibitory activity can be an IC50 value (nM) of 0.1~500 nM, 1~100 nM, e.g., 1~50 nM, 1~10 nM against tumor cells with high expression of the specific target; for example, said specific target includes but is not limited to HER2, EGFR, TROP2, and Claudin 18.2.

Specifically, the in vivo tumor inhibitory effect of the ADC compounds of the present disclosure can be that, upon administration to an animal compared to a control drug, the tumor volume in the animal is reduced by more than 1%, more than 5%, more than 20%, more than 50%, or even 90% or 95% at 1, 3, 5, 7, 14, 21, or 30 days. For example, compared to administering a negative control and control drug, the tumor volume in said animal at 1, 3, 5, 7, 14, 21, or 30 days is reduced by more than 1.1-fold, more than 1.5-fold, more than 2-fold, more than 5-fold, more than 10-fold, or even 100-fold or 500-fold. Said animals include but are not limited to mammals. Said administration includes but is not limited to oral, intravenous injection or infusion, intraperitoneal injection, or local administration.

Specifically, the distribution of the ADC compounds of the present disclosure in animal tumor tissues after administration, compared to other tissues and organs, or compared to the distribution of the Ras, e.g., Ras mutant protein, inhibitor, can be increased by more than 1%, more than 5%, more than 20%, more than 50%, or even 90%, 95%. For example, the distribution can be increased by more than 1.1-fold, more than 1.5-fold, more than 2-fold, more than 5-fold, more than 10-fold, or even 100-fold or 500-fold. Said animals include but are not limited to mammals. Said tissues or organs include but are not limited to heart, liver, spleen, lung, kidney, brain, etc. Said administration includes but is not limited to oral, intravenous injection or infusion, intraperitoneal injection, or local administration.

Specifically, the ADC compounds of the present disclosure have good in vivo safety, and the in vivo free toxin release rate after administration to animals does not exceed 50%, does not exceed 20%, does not exceed 10%, or even does not exceed 1%, does not exceed 0.1%. For example, the in vivo safety is that the compound administration concentration at which the animals show no toxic manifestations can be 0.5 mg/kg or more, 5 mg/kg or more, 10 mg/kg or more, 50 mg/kg or more, 100 mg/kg or more, or even 500 mg/kg or more. Said animals include but are not limited to mammals. Said administration includes but is not limited to oral, intravenous injection or infusion, intraperitoneal injection, or local administration.

Specifically, the ADC compounds of the present disclosure have plasma stability, and the release rate of the Ras, e.g., KRas mutant protein, inhibitor after being added to plasma does not exceed 50%, does not exceed 20%, does not exceed 10%, or even does not exceed 1% at 1, 3, 5, 7, 14, 21, or 30 days.

### III. Pharmaceutical Compositions

Another aspect of the present disclosure provides pharmaceutical compositions comprising the ADC compounds of the present disclosure and one or more pharmaceutically acceptable excipients. The composition can contain components conventional in pharmaceutical formulations, such as diluents, carriers, pH adjusters, preservatives, solubilizers, stabilizers, wetting agents, emulsifiers, sweeteners, colorants, flavoring agents, salts for altering osmotic pressure, buffers, masking agents, antioxidants, and other active agents. Suitable carriers and excipients are well known to those skilled in the art and are described in detail, for example, in Gennaro A.R. et al., Remington: The Science and Practice of Pharmacy (2000) Lippincott, Williams & Wilkins, Philadelphia.

The present disclosure also provides methods for preparing said compositions using the ADC compounds of the present disclosure. Typical pharmaceutical compositions or dosage forms are prepared by mixing the compounds of the present disclosure with carriers or excipients, and can be formulated in a manner consistent with good medical practice known to those skilled in the art, see, for example, Gennaro A.R. et al., Remington: The Science and Practice of Pharmacy (2000) Lippincott, Williams & Wilkins, Philadelphia, or pharmacopoeias of various countries.

The pharmaceutical composition can be in any suitable form, such as tablets, powders, capsules, sterile injectable preparations, solutions, dispersions, suspensions, syrups, sprays, suppositories, gels, emulsions, patches, etc., and can be administered to patients by various routes, such as intravenous, intratumoral, subcutaneous, intramuscular, oral, intranasal, intrathecal, transdermal, or topical. The route of administration in any given case may depend on the specific antibody and/or ADC, the subject, the nature and severity of the disease, and the physical condition of the subject. In certain embodiments, the pharmaceutical composition will be administered intravenously, intratumorally, subcutaneously, or intramuscularly in the form of a liquid formulation.

In preferred embodiments, the ADCs described herein and the pharmaceutical compositions comprising them are administered systemically, e.g., subcutaneously, intraperitoneally, intramuscularly, intravenously, particularly intravenously, especially in the form of sterile injectable preparations, such as sterile injectable solutions or suspensions in non-toxic parenterally acceptable diluents or solvents, or prepared as lyophilized powders; acceptable vehicles or solvents include, for example, water, 1,3-butanediol, Ringer's solution, or isotonic sodium chloride solution; in addition, sterile fixed oils can be used conventionally as solvents or suspension media, and any mild fixed oil can be used for this purpose, including, for example, synthetic mono- or diglycerides, fatty acids, etc. In other embodiments, the ADCs described herein and/or the Ras, e.g., KRas mutant, inhibitors and the pharmaceutical compositions comprising them are administered locally at the tumor site, e.g., intratumorally or in the tumor microenvironment.

The dosage of the compounds of the present disclosure can vary within a wide range, and of course, in each specific case, it can be adjusted by the clinician according to individual needs. For prophylactic or therapeutic purposes, the appropriate dosage of the ADC of the present disclosure (when used alone or in combination with one or more other therapeutic agents) will depend on the type of disease to be treated, the type of antibody, the severity and course of the disease, whether the antibody is administered for prophylactic or therapeutic purposes, previous therapy, the patient's clinical history and response to the antibody, and the judgment of the attending physician.

The pharmaceutical compositions or formulations of the present disclosure may also contain more than one active ingredient, which is required for the specific indication being treated, preferably those with complementary activities that do not adversely affect each other. For example, it may be desirable to also provide other therapeutic agents, including chemotherapeutic agents, angiogenesis inhibitors, cytokines, cytotoxic agents, other antibodies, small molecule drugs, or immunomodulators (e.g., immune checkpoint inhibitors or agonists), etc. Said active ingredients are suitably combined in amounts effective for the intended use.

### IV: Pharmaceutical Combinations and Kits

In some embodiments, the present disclosure also provides pharmaceutical combinations or combination products comprising the ADCs and/or Ras, e.g., KRas mutant, inhibitors of the present disclosure or pharmaceutically acceptable salts or solvates thereof, and one or more other therapeutic agents. The combination is for use in preventing or treating diseases mediated by Ras mutant proteins, especially KRas mutant proteins (such as G12C mutant, G12D mutant, G12V mutant, G12A mutant, G12R mutant, G12S mutant, and G13D mutant proteins), more specifically for use in treating or preventing hyperproliferative diseases, especially as anti-tumor agents.

Another object of the present disclosure is to provide a kit comprising the pharmaceutical combination of the present disclosure, preferably the kit is in the form of pharmaceutical dosage units. Thus, dosage units can be provided according to the dosing regimen or drug administration intervals.

In one embodiment, the kit of the present disclosure comprises within the same packaging:
- a first container containing a pharmaceutical composition comprising the ADC of the present disclosure or a pharmaceutically acceptable salt or solvate thereof;
- a second container containing a pharmaceutical composition comprising another therapeutic agent.

The mode and order of administration of the components of the combination product can be determined by those skilled in the art depending on factors such as the disease to be treated and the condition of the individual. The combination products of the present disclosure can be used in the treatment methods of the present disclosure. In some embodiments, the present disclosure provides combination products wherein the other therapeutic agent is, for example, an anti-proliferative drug that acts through the same or different target and/or mechanism of action as the Ras, e.g., KRas mutant, inhibitor of the present disclosure. In some embodiments, the combination product is for use in preventing or treating diseases mediated by Ras mutant proteins, especially KRas mutant proteins (such as G12C mutant, G12D mutant, G12V mutant, G12A mutant, G12R mutant, G12S mutant, and G13D mutant proteins), more specifically for use in treating or preventing hyperproliferative diseases, especially tumors.

### V. Uses and Methods

As shown in the Examples below, the Ras, e.g., KRas mutant, inhibitors and ADCs of the present disclosure can effectively inhibit the proliferation of tumor cells carrying Ras, e.g., KRas mutant, proteins, and both exhibit excellent anti-tumor activity and safety in animal models. In the ADCs, the Ras, e.g., KRas mutant, inhibitor is conjugated via a linker unit to an antibody or antigen-binding fragment that binds to a tumor-associated antigen or immune cell antigen, and is targeted and delivered to tumor-associated cells or the tumor microenvironment to centrally inhibit the Ras, e.g., KRas mutant, protein, thereby promoting tumor cell apoptosis.

Given that the drug payload of the ADC of the present disclosure, i.e., the Ras, e.g., KRas mutant, inhibitor, can target and inhibit Ras, e.g., KRas mutant, proteins (such as but not limited to G12C mutant, G12D mutant, G12V mutant, G12A mutant, G12R mutant, G12S mutant, and G13D mutant proteins), another aspect of the present disclosure provides therapeutic uses and methods of the ADCs and/or Ras, e.g., KRas mutant, inhibitors of the present disclosure.

Thus, in one aspect, the present disclosure provides the ADCs and/or Ras, e.g., KRas mutant, inhibitors of the present disclosure (i.e., compounds defined in the Drug P unit, such as ADCs of formula X and its sub-formulas) or KRas mutant inhibitors (i.e., KRas mutant inhibitors of formula I and its sub-formulas), for use in therapy.

In another aspect, the present disclosure provides the ADCs and/or Ras, e.g., KRas mutant, inhibitors of the present disclosure, or stereoisomers, tautomers, stable isotopic variants, pharmaceutically acceptable salts or solvates thereof, or pharmaceutical compositions comprising them, for use as therapeutic agents for treating or preventing diseases mediated by Ras mutant proteins, especially KRas mutant proteins (such as G12C mutant, G12D mutant, G12V mutant, G12A mutant, G12R mutant, G12S mutant, and G13D mutant proteins), more specifically for use in treating or preventing hyperproliferative diseases, especially as anti-tumor therapeutic agents.

In another aspect, the present disclosure provides use of the ADCs and/or Ras, e.g., KRas mutant, inhibitors of the present disclosure, or stereoisomers, tautomers, stable isotopic variants, pharmaceutically acceptable salts or solvates thereof, or pharmaceutical compositions comprising them, in the prevention or treatment of diseases mediated by Ras mutant proteins, especially KRas mutant proteins (such as G12C mutant, G12D mutant, G12V mutant, G12A mutant, G12R mutant, G12S mutant, and G13D mutant proteins), more specifically in the treatment or prevention of hyperproliferative diseases, especially tumors.

In another aspect, the present disclosure provides methods for treating or preventing a disease mediated by a Ras mutant protein, especially a KRas mutant protein (such as G12C mutant, G12D mutant, G12V mutant, G12A mutant, G12R mutant, G12S mutant, and G13D mutant proteins), in a subject, comprising administering to a human or animal the ADCs and/or Ras, e.g., KRas mutant, inhibitors of the present disclosure, or stereoisomers, tautomers, stable isotopic variants, pharmaceutically acceptable salts or solvates thereof, or pharmaceutical compositions comprising them; specifically, the present disclosure provides methods for treating or preventing hyperproliferative diseases, especially tumors, in a subject, comprising administering to a human or animal the ADCs and/or Ras, e.g., KRas mutant, inhibitors of the present disclosure, or stereoisomers, tautomers, stable isotopic variants, pharmaceutically acceptable salts or solvates thereof, or pharmaceutical compositions comprising them.

In another aspect, the present disclosure provides use of the ADCs and/or Ras, e.g., KRas mutant, inhibitors of the present disclosure, or stereoisomers, tautomers, stable isotopic variants, pharmaceutically acceptable salts or solvates thereof, or pharmaceutical compositions comprising them, in the manufacture of a medicament for the prevention or treatment of diseases mediated by Ras mutant proteins, especially KRas mutant proteins (such as G12C mutant, G12D mutant, G12V mutant, G12A mutant, G12R mutant, G12S mutant, and G13D mutant proteins); specifically, the present disclosure provides use of the ADCs and/or Ras, e.g., KRas mutant, inhibitors of the present disclosure, or stereoisomers, tautomers, stable isotopic variants, pharmaceutically acceptable salts or solvates thereof, or pharmaceutical compositions comprising them, in the manufacture of a medicament for the treatment or prevention of hyperproliferative diseases, especially tumors.

In another aspect, the ADCs and/or Ras, e.g., KRas mutant, inhibitors provided by the present disclosure can be used alone or in combination with each other and/or in combination with other therapeutic agents. Therefore, the present disclosure provides pharmaceutical combinations comprising the ADCs and/or Ras, e.g., KRas mutant, inhibitors of the present disclosure, or stereoisomers, tautomers, stable isotopic variants, pharmaceutically acceptable salts or solvates thereof, and at least one other therapeutic agent; the combination is for use in the prevention or treatment of diseases mediated by Ras mutant proteins, especially KRas mutant proteins (such as G12C mutant, G12D mutant, G12V mutant, G12A mutant, G12R mutant, G12S mutant, and G13D mutant proteins), more specifically in the treatment or prevention of hyperproliferative diseases, especially tumors; and methods for treating or preventing diseases mediated by Ras mutant proteins, especially KRas mutant proteins (such as G12C mutant, G12D mutant, G12V mutant, G12A mutant, G12R mutant, G12S mutant, and G13D mutant proteins), more specifically hyperproliferative diseases, especially tumors, in a subject, comprising administering to a human or animal the pharmaceutical combination of the present disclosure.

In another aspect, the present disclosure also provides methods for preparing the ADCs of the present disclosure or stereoisomers, tautomers, stable isotopic variants, pharmaceutically acceptable salts or solvates thereof by conjugating a Ras, e.g., KRas, inhibitor as defined herein to an antibody or antigen-binding fragment via a linker unit. The Ras, e.g., KRas, inhibitor compounds defined herein can be conjugated to the antibody or antigen-binding fragment via a cleavable or non-cleavable linker unit. In specific embodiments, upon cleavage of the linker unit, the Ras, e.g., KRas, inhibitor is released into the tumor cell, cancer-associated immune cell, or tumor microenvironment.

For the above uses and methods, the hyperproliferative disease refers to a physiological condition in a subject characterized by uncontrolled or dysregulated cell growth or death, whether malignant or benign, especially refers to tumors or cancers, including solid tumors and hematological tumors, and all precancerous cells and cancer cells and tissues. For various aspects of the present disclosure, the cancer or tumor includes but is not limited to lung adenocarcinoma, lung cancer, bone cancer, pancreatic cancer, skin cancer, head and neck cancer, cutaneous or intraocular melanoma, anal region cancer, testicular cancer, urethral cancer, ureteral cancer, penile cancer, prostate cancer, bladder cancer, uterine cancer, ovarian cancer, fallopian tube cancer, endometrial cancer, cervical cancer, vaginal cancer, vulvar cancer, gastric cancer, colon cancer, rectal cancer, breast cancer, esophageal cancer, small intestine cancer, lip cancer, laryngeal cancer, nasopharyngeal cancer, oral cancer, salivary gland cancer, peritoneal cancer, gastrointestinal stromal tumor, mesothelioma, biliary tract cancer, hepatocellular carcinoma, seminoma, soft tissue sarcoma, urothelial carcinoma, sweat gland carcinoma, endocrine system cancer, thyroid cancer, medullary thyroid cancer, papillary thyroid cancer, parathyroid cancer, kidney cancer, renal parenchymal cancer, renal cell carcinoma, renal pelvic cancer, adrenal cancer, brain cancers such as glioblastoma, astrocytoma, meningioma, medulloblastoma, and peripheral neuroectodermal tumors; chronic or acute leukemias, Hodgkin's disease, lymphocytic lymphoma, Hodgkin lymphoma, non-Hodgkin lymphoma, Burkitt lymphoma, acute lymphoblastic leukemia (ALL), chronic lymphocytic leukemia (CLL), chronic myeloid leukemia (CML) and lymphocytic carcinoma, acute myeloid leukemia (AML), myeloid leukemia (chronic myeloid leukemia (CML)), adult T-cell lymphoma, diffuse large B-cell lymphoma (DLBCL), primary CNS lymphoma, central nervous system (CNS) tumors, spinal tumors, brainstem glioma, or pituitary adenoma.

For various aspects of the present disclosure, preferably, the cancer or tumor is associated with a Ras mutation, especially a KRas mutation, preferably a KRas G12D mutation, including but not limited to the above tumor types and their preferred ranges. Particularly preferred tumors of the present invention include lung cancer, lung adenocarcinoma, colon cancer, rectal cancer, pancreatic cancer, endometrial cancer, cholangiocarcinoma, leukemia, and ovarian cancer.

It should be noted that in the above various use and method technical solutions, the Ras inhibitor can be a pan-Ras inhibitor, i.e., an inhibitor not limited to a specific isoform and mutation site, and the Ras inhibitor can also be a specific KRas inhibitor, specifically, for example, a KRas G12D inhibitor.

### VI: Preparation of the ADC of the Present Disclosure

### 1. General Synthetic Methods

The compounds of the present disclosure, their stereoisomers, tautomers, stable isotopic variants, pharmaceutically acceptable salts, or solvates can be prepared by various methods well known in the field of organic synthesis, including the methods given below, the methods given in the Examples, or methods analogous thereto as understood by those skilled in the art.

General synthetic schemes for synthesizing the compounds of the present disclosure are illustrated below. For each reaction step, appropriate reaction conditions are known to those skilled in the art or can be routinely determined. Specifically, the method steps used to synthesize the compounds of the present disclosure can be carried out under reaction conditions known per se (including those specifically mentioned), in the absence or usually in the presence of a solvent or diluent (including, for example, solvents or diluents that are inert to the reagents used and can dissolve the reagents used), in the absence or presence of a catalyst, condensing agent, or neutralizing agent (e.g., ion exchangers, such as cation exchangers, e.g., H⁺ form), depending on the reaction and/or the nature of the reactants, at reduced, normal, or elevated temperatures (e.g., about -100 °C to about 190 °C, including, for example, about -78 °C to about 150 °C, such as about 0 °C to about 125 °C, room temperature, -20 °C to 40 °C, or reflux temperature), at atmospheric pressure or in a closed vessel, under pressure when appropriate, and/or under an inert atmosphere such as oxygen or nitrogen.

Unless otherwise specified, the starting materials and reagents used in preparing these compounds are generally commercially available or can be prepared by the methods described below, methods analogous to those described below, or methods known in the art. If desired, the starting materials and intermediates in the synthetic reaction schemes can be isolated and purified using conventional techniques, including but not limited to filtration, distillation, crystallization, chromatography, etc. The materials can be characterized using conventional methods including physical constants and spectral data.

Unless otherwise specified in the description of the method, solvents suitable for any particular reaction include: those specifically mentioned, or for example water; esters such as lower alkyl esters of lower fatty acids, e.g., ethyl acetate; ethers such as aliphatic ethers, e.g., diethyl ether, or cyclic ethers, e.g., tetrahydrofuran or dioxane; liquid aromatic hydrocarbons such as benzene or toluene; alcohols such as methanol, ethanol, or 1- or 2-propanol, such as acetonitrile; halogenated hydrocarbons such as dichloromethane or chloroform; amides such as N,N-dimethylformamide or N,N-dimethylacetamide; bases such as heterocyclic nitrogen bases, e.g., pyridine; carboxylic anhydrides such as lower aliphatic carboxylic anhydrides, e.g., acetic anhydride; cyclic, linear, or branched hydrocarbons such as cyclohexane, hexane, or isopentane; or mixtures of these solvents, e.g., aqueous solutions. Such solvent mixtures can also be used for work-up, e.g., work-up by chromatography or partition.

Those skilled in the art will recognize the presence of stereocenters in the compounds of formula I. At all stages of the reaction, mixtures of the resulting isomers can be separated into individual isomers, e.g., diastereomers or enantiomers, or into any desired isomer mixture, e.g., racemates or mixtures of diastereomers, see, e.g., E. L. Eliel, S. H. Wilen, and L. N. Mander's "Stereochemistry of Organic Compounds" (Wiley-Interscience, 1994**).**

### 2. Synthesis of KRAS Mutant Inhibitors

Scheme 1 below illustrates a general synthetic route that can be used to prepare the KRAS variant inhibitors and their various specific embodiments described herein. The variables in the general formulas in the following schemes have the same meanings as in the compounds or their specific embodiments defined herein, unless otherwise specified.

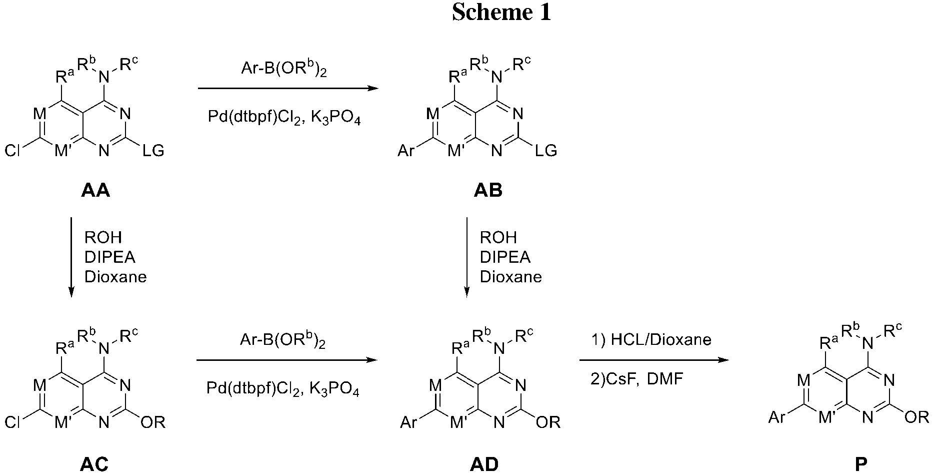

Intermediate AA is subjected to a coupling reaction such as a Suzuki coupling reaction to obtain intermediate AB, which is then treated with ROH in the presence of a base to replace the leaving group -LG in intermediate AB, yielding intermediate AD. Alternatively, intermediate AA is treated with ROH in the presence of a base to replace the leaving group -LG, yielding intermediate AC, which is then subjected to a coupling reaction such as a Suzuki coupling reaction to obtain intermediate AD. Then, all possible protecting groups carried by intermediate AD are removed using standard methods to obtain the KRAS variant inhibitor P.

Specifically, the coupling reaction involves, for example, subjecting intermediate AA or AC to Suzuki coupling with an arylboronic acid or boronate ester in the presence of a catalyst such as a palladium catalyst and a base, wherein the palladium catalyst is, for example but not limited to, Pd(dbpf)Cl₂ or Pd(dtbpf)Cl₂, etc., preferably Pd(dtbpf)Cl₂; the base can be selected from sodium hydroxide, potassium hydroxide, sodium carbonate, potassium carbonate, or cesium carbonate, preferably potassium carbonate; the coupling reaction is preferably carried out in a suitable organic solvent, which can be selected from tetrahydrofuran, ethers (such as diethyl ether, ethylene glycol monomethyl ether, etc.), N-methylpyrrolidone, N,N-dimethylformamide, dimethylacetamide, dichloromethane, 1,4-dioxane, dimethyl sulfoxide, and any combinations thereof, preferably 1,4-dioxane; the coupling reaction is preferably carried out at a suitable temperature, for example, 50-200 °C, 80-150 °C, preferably 90-120 °C.

Wherein, the leaving group LG in the intermediate AA can be, for example, Cl, F (obtained by treating the corresponding Cl-substituted compound with KF), SMe, SOMe (obtained by oxidizing SMe with m-chloroperoxybenzoic acid (mCPBA)), SO₂Me (obtained by oxidizing SMe with mCPBA), etc.; the base used in the substitution reaction can be, for example, sodium hydride, DIPEA, sodium alkoxide, potassium alkoxide, etc., preferably DIPEA and sodium tert-butoxide; the deprotection reaction is determined according to the type of the substituent, for example, deprotecting Boc with hydrochloric acid in a suitable solvent such as dioxane, or deprotecting silane ether protecting groups such as TMS, TBDMS, TES, TIPS, etc. with cesium fluoride in a suitable solvent such as DMF.

It should be noted that when the coupling product has axial chirality, its isomers can be resolved at any intermediate stage by methods well known to those skilled in the art.

Scheme 2 illustrates the synthesis route for the compound of formula AA in Scheme 1, as well as the synthesis of intermediates further used therein.

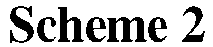

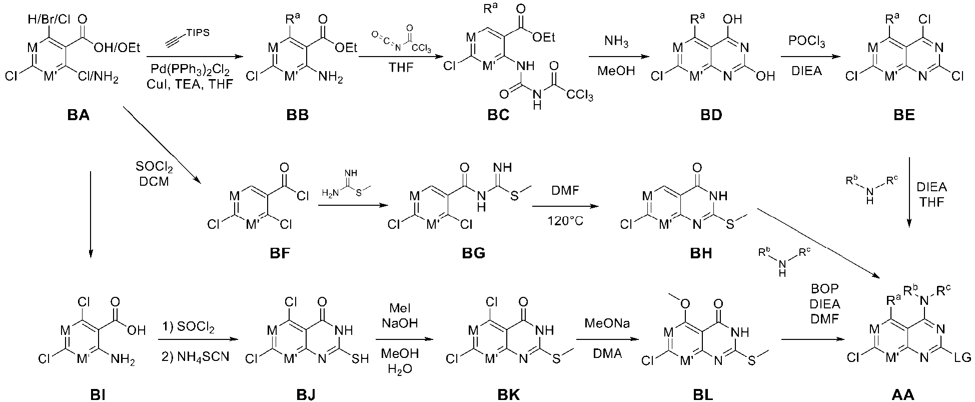

When BA is a bromide, the protected alkynyl group R^{a} can be introduced under the co-catalysis of palladium and copper salts (such as Pd(PPh₃)₂Cl₂ and CuI) to obtain BB; BB is then reacted with 2,2,2-trichloroacetyl isocyanate in a suitable solvent such as THF to obtain BC; BC is cyclized in the presence of a base such as ammonia in a suitable solvent such as methanol to generate intermediate BD; the hydroxyl group in BD is then activated with a chlorinating agent such as phosphorus oxychloride in a suitable solvent such as DIEA to generate the chlorinated product BE; BE is subsequently substituted with an organic aliphatic amine such as HNR^{b}R^{c} to obtain key intermediate AA.

When BA is a free acid, it can be directly treated with an acyl chlorinating agent such as thionyl chloride to generate BF, which is then reacted with S-methylthiourea to obtain BG; BG is cyclized in a suitable solvent such as DMF at elevated temperatures such as 120 °C to generate BH; BH is reacted with an organic aliphatic amine such as HNR^{b}R^{c} in the presence of a condensing agent such as BOP and a base such as DIEA to obtain key intermediate AA.

When BA is a free acid such as BI, it can first be treated with an acyl chlorinating agent such as thionyl chloride, followed by reaction with ammonium thiocyanate instead of S-methylthiourea to obtain intermediate BJ; the thiol group is methylated with an alkylating agent such as methyl iodide in the presence of a base such as NaOH in a suitable solvent such as methanol and water to obtain BK; BK is treated with a conventional manner such as sodium methoxide in a suitable solvent such as DMA to introduce the methoxy group, yielding BL; BL is reacted in the presence of a condensing agent such as BOP and a base such as DIEA to yield key intermediate AA.

The boronic acids or boronate esters (ArB(OR^{b})₂, including cyclic esters) used in Scheme 1 are commercially available or can be prepared by those skilled in the art using general synthetic methods.

Scheme 3 illustrates the synthesis route for the ROH compound in Scheme 1, as well as the synthesis of intermediates further used therein.

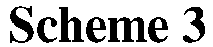

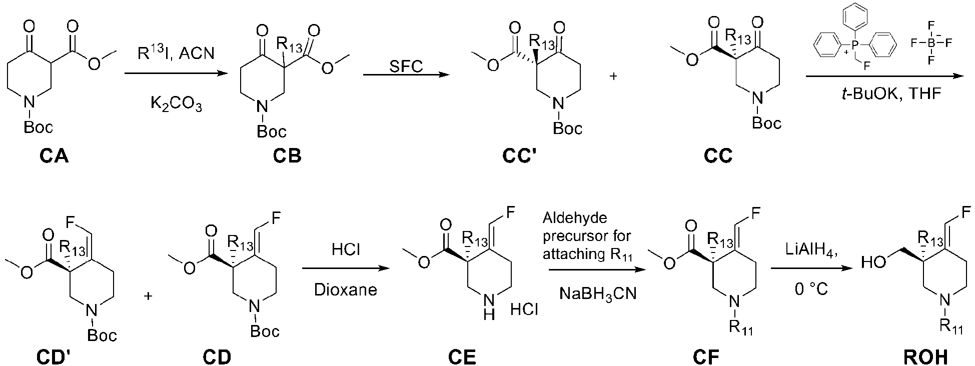

CA (CAS: 161491-24-3) is treated with an alkylating agent in the presence of a base such as potassium carbonate in a suitable solvent such as acetonitrile to obtain intermediate CB; CB is a mixture of chiral isomers, which is resolved by SFC to obtain optically pure intermediates CC and CC'. For the present disclosure, intermediate CC is preferred. Intermediate CC undergoes a Wittig reaction to convert the ketone carbonyl to an alkene double bond, yielding geometric isomers CD and CD'. For the present disclosure, CD is preferred. CD is treated with an acid such as hydrochloric acid in a suitable solvent such as dioxane to remove the Boc protecting group on the N atom, followed by reductive amination or N-alkylation to introduce the R¹¹ group, yielding intermediate CF. Finally, the ester group in CF is reduced with a reducing agent such as lithium aluminum hydride to obtain the alcohol intermediate ROH.

Scheme 4 illustrates another synthetic route for the compound ROH in Scheme 1, as well as the synthesis of intermediates further used therein.

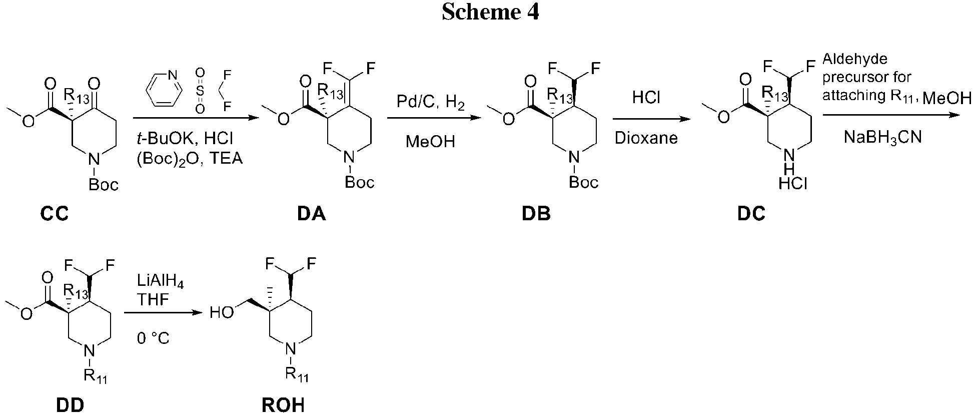

Intermediate CC obtained from Scheme 3 is reacted with 2-((difluoromethyl)sulfonyl)pyridine in the presence of a strong base such as potassium tert-butoxide to generate DA; DA is hydrogenated, for example, using palladium on carbon and H₂ in a suitable solvent such as methanol, to obtain DB; following the method of Scheme 3 above, DB is deprotected to obtain DC; DC is then subjected to reductive amination or N-alkylation to introduce the R₁₁ group, yielding DD; finally, the ester group in DD is reduced to obtain the alcohol intermediate ROH.

Scheme 5 illustrates the synthesis route for the compound of formula ROH in Scheme 1 when it is deuterated, as well as the synthesis of intermediates further used therein.

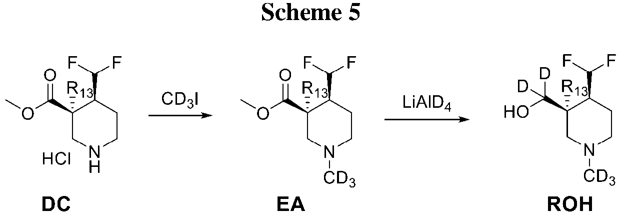

Intermediate DC obtained from Scheme 4 is treated with a deuterating agent such as trideuterated iodomethane to obtain EA; the ester group in EA is then reduced with a deuterated reducing agent such as lithium aluminum deuteride to obtain the corresponding deuterated alcohol ROH.

### 3. Synthesis of Linker Unit L and KRas Mutant Inhibitor-Linker Unit Conjugation Fragment

The drug payload KRas mutant inhibitor of the ADC of the present disclosure is linked to the antibody via the linker unit L defined herein, as shown in the following specific examples: 
Linker unit
KRAS Inhibitor—Self-immolative linker – cleavable linker— Antibody Linker —Antibody
Solubilizing sugar unit
or
Linker unit
KRAS Inhibitor—Self-immolative linker – cleavable linker— Antibody Linker —Antibody
Optional solubilizing unit Optional solubilizing unit
 wherein the KRas mutant inhibitor is linked to the self-immolative linker via an amine nitrogen atom or a hydroxyl oxygen atom; the solubilizing sugar unit can be linked to the self-immolative linker via an α or β glycosidic bond, or the optional solubilizing unit is linked to the self-immolative linker or the antibody linker in the manner defined above; wherein the self-immolative linker, cleavable linker, antibody linker, solubilizing sugar unit, and solubilizing unit are respectively as generally or specifically defined in the Summary of the Invention section above for each unit.

It should be noted that the various self-immolative linkers, cleavable linkers, antibody linkers, and solubilizing sugar units are commercially available or can be routinely prepared by those skilled in the art using methods known in the art.

Scheme 6 below illustrates a general synthetic route that can be used to prepare the conjugation fragment of the KRas mutant inhibitor and the linker unit, using the case where the linking atom of the KRas mutant inhibitor is N as an example. The variables in the general formulas in the following schemes have the same meanings as in the compounds or their specific embodiments defined herein, unless otherwise specified.

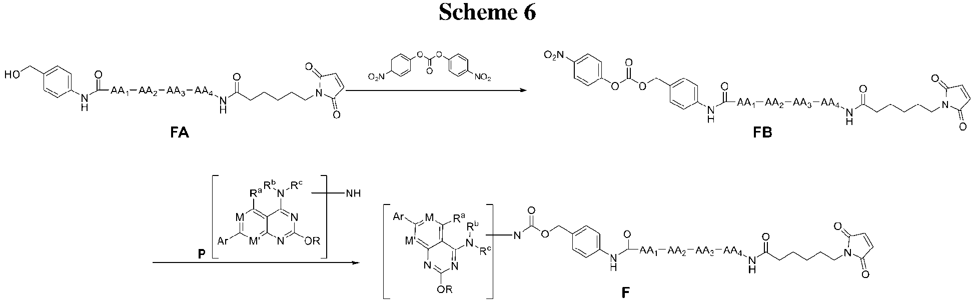

Intermediate FA is commercially available and is activated, e.g., by treatment with bis(p-nitrophenyl) carbonate, to obtain the active carbonate FB; FB is reacted with the -NH group in the drug P unit to generate intermediate F, which can be directly used for linking to the antibody molecule.

It should be noted that in Scheme 6, AA₁, AA₂, AA₃, AA₄ represent individual amino acids constituting the cleavable linker; their number is merely exemplary, and for convenience of illustration, the carbonyl group of AA₁ and the amino terminus of AA₄ are shown.

Scheme 7 below illustrates a general synthetic route that can be used to prepare the conjugation fragment of the KRas mutant inhibitor and the linker unit, wherein the linking atom of the KRas mutant inhibitor is N. The variables in the general formulas in the following schemes have the same meanings as in the compounds or their specific embodiments defined herein, unless otherwise specified.

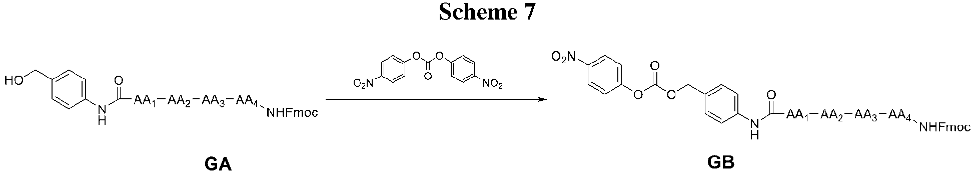

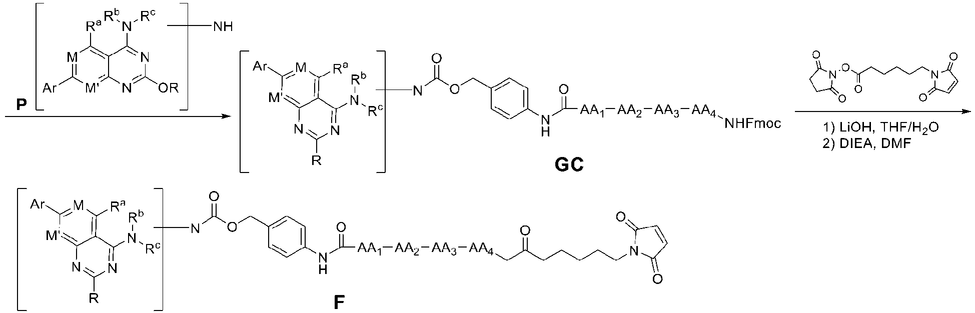

Intermediate GA is commercially available and is activated, e.g., by treatment with bis(p-nitrophenyl) carbonate, to obtain the active carbonate GB; intermediate GB is reacted with the -NH group in the drug P unit to generate intermediate GC; GC is then treated with a base such as LiOH aqueous solution to remove the Fmoc group and, when a sugar unit is present, the acetyl groups in the sugar unit, to obtain intermediate F, which can be used for direct linking to the antibody molecule.

Scheme 8 below illustrates a general synthetic route that can be used to prepare the conjugation fragment of the KRas mutant inhibitor and the linker unit, wherein the KRas mutant inhibitor is linked via its hydroxyl O atom to the linker unit. The variables in the general formulas in the following schemes have the same meanings as in the compounds or their specific embodiments defined herein, unless otherwise specified.

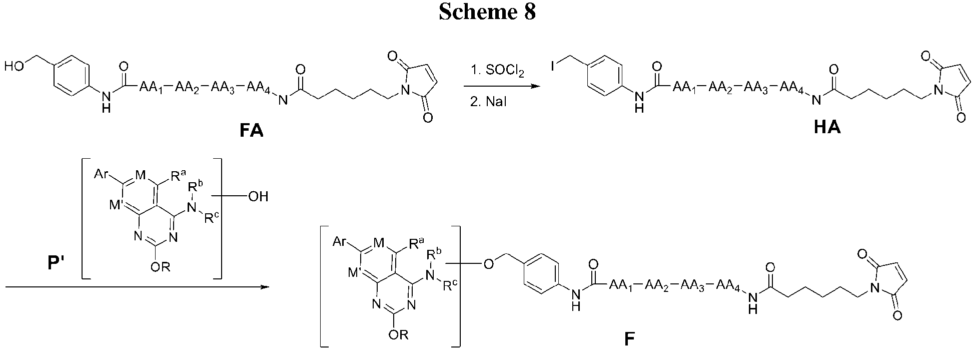

Intermediate FA is commercially available and is treated with a halogenating reagent such as thionyl chloride to convert the benzyl alcohol to a benzyl halide, preferably benzyl iodide HA; intermediate HA is reacted in a suitable solvent such as DMF, wherein the iodide is preferentially reacted with the -OH group in the protected drug P' unit directly over the succinimide, followed by deprotection of the amine protecting group using suitable reagents (e.g., TBDMSOTf in 2,6-lutidine to deprotect Boc), to obtain intermediate F, which is used for linking to the antibody molecule.

The sugar solubilizing unit contained in the ADC of the present disclosure can be incorporated into any position of the linker unit and payload. Scheme 9 below illustrates a general synthetic route that can be used to prepare a linker unit L in the ADC of the present disclosure, wherein the solubilizing sugar unit is linked to the self-immolative linker A of the linker unit L. The variables in the general formulas in the following schemes have the same meanings as in the compounds or their specific embodiments defined herein, unless otherwise specified.

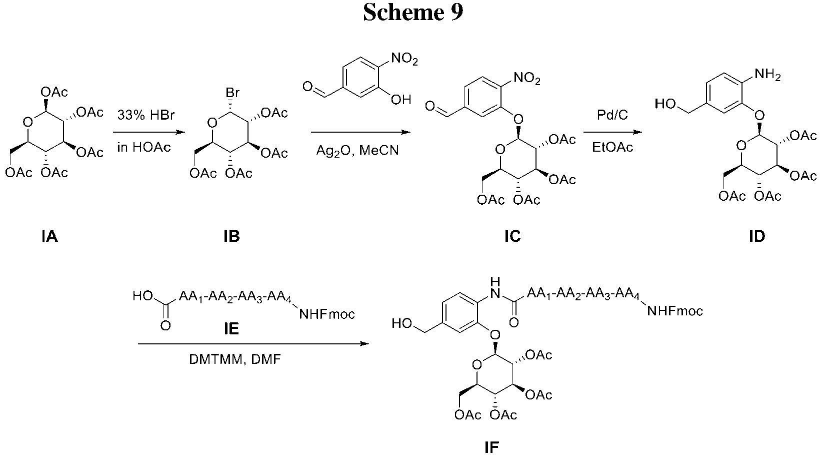

Acetylated glucose IA is commercially available and is first activated by a suitable method, for example, converted to the 1-bromo derivative IB under conditions conventionally known to those skilled in the art; IB is then condensed with a phenolic hydroxyl group to obtain glycoside intermediate IC; the nitro and aldehyde groups on the benzene ring of IC can be simultaneously reduced by hydrogenation to obtain ID; ID is then condensed with commercially available peptide IE to obtain intermediate IF. IF can be used to replace intermediate GA in Scheme 7, wherein the acetyl protecting groups on the sugar unit can be removed together with the amino protecting group Fmoc, ultimately preparing the conjugation fragment F of the KRas mutant inhibitor and the linker unit described herein.

### 4. Selection and Source of Antibodies

The antibodies used in the conjugates of the present disclosure are commercially available and include antibodies targeting the following targets: AXL, B7H1, B7H3, B7H4, BCMA, CD16, CD19, CD22, CD25, CD30, CD32, CD33, CD44, CD64, CD70, CD74, CD79, CD138, CD142, CD276, CDH3, CEACAM5, Claudin 18.2, CLDN9/CLDN6, DPEP3, EGFR, ENPP3, EphA, FcRH5, FOLR1, FRα, GCPII, HER2, HER3, KAAG1, KIT, LIV-1, Mesothelin, cMet, MUC1, Nectin-4, PD-L1, PD-L1/PD-L2, PSMA, ROR1, RPR1, TF, TOP1, TROP2, or a combination of EGFR and Met, etc.

### 5. Synthesis of KRas Mutant Inhibitor-Linker Unit-Antibody ADC

The preparation of antibody-drug conjugates can be achieved by any method known to those skilled in the art. In some embodiments, conjugation of the drug-linker unit to the antibody is accomplished by reaction with amino acid residues of the antibody. In some embodiments, a linker unit L bearing a leaving group is used to couple the drug P unit to cysteine residues of the antibody to prepare the ADC of the present disclosure. In some embodiments, the conditions for treating the antibody with a reducing agent such as tris(2-hydroxyethyl)phosphine (TCEP) can be controlled to disrupt the interchain disulfide bonds of the antibody and expose free thiol groups for conjugation with the drug-linker unit conjugation fragment. For IgG1-type antibodies, up to four linking disulfide bonds can be reduced, generating up to 8 reactive thiol groups for conjugation. Conjugates prepared by this method can carry zero, one, two, three, four, five, six, seven, or eight drug molecules per antibody molecule.

When the prepared conjugate is a composition of conjugates with different drug conjugation sites and/or numbers, the drug loading of the conjugate is represented by the average DAR. At this time, the average DAR of the produced antibody-drug conjugate composition can be characterized by conventional means such as mass spectrometry, ELISA assays, and HPLC, and the quantitative distribution of the antibody-drug conjugate in terms of q can also be determined. The isolation, purification, and characterization of homogeneous antibody-drug conjugates where q is a specific value from those with other drug loadings can be achieved by means such as reverse-phase HPLC or electrophoresis.

Scheme 9 below illustrates a general synthetic route that can be used to prepare the ADC of the present disclosure and its various specific embodiments. The variables in the general formulas in the following schemes have the same meanings as in the compounds or their specific embodiments defined herein, unless otherwise specified.

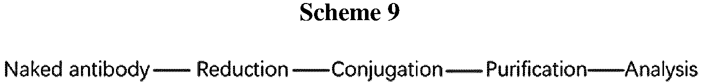

The antibody molecule is first reduced, preferably by reducing the disulfide bonds of the antibody molecule. The heavy and light chains of the antibody molecule are linked together by 4 pairs of disulfide bonds. These disulfide bonds can be reduced with a reducing agent (such as tris(2-carboxyethyl)phosphine, TCEP, etc.) to up to 0-8 thiol groups. Each thiol group may react with the succinimide to generate an antibody conjugate carrying one or more KRas mutant inhibitor-linker unit conjugation fragments. The average drug loading DAR (drug-to-antibody ratio) of this compound can be determined routinely by experiment.

Unless otherwise specified, the experimental materials and reagents used in the above synthetic methods and schemes can be obtained from commercial sources, prepared according to prior art methods, or prepared according to methods similar to those disclosed in the present application. Unless otherwise specified, the synthetic conditions used in the above synthetic methods and schemes can be routinely determined by those skilled in the art.

The present disclosure also relates to the following preparation methods: wherein compounds obtainable as intermediates in any step of the preparation methods and schemes described herein are used as starting materials and the remaining method steps are carried out, or wherein the starting materials are formed in situ under the reaction conditions or are used in the form of derivatives, e.g., in protected form or salt form, or compounds obtainable according to the methods of the present disclosure are generated under the method conditions and are further treated in situ.

### 6. Synthetic Examples

The present disclosure is further illustrated below with reference to examples. It should be noted that the following examples are not intended to limit the scope of protection of the present disclosure.

Unless there is a clear error in the structural formula, in the event of any inconsistency between the chemical name of any compound of the present disclosure and the structural formula given, the structural formula shall prevail.

In the following examples, experimental methods for which specific conditions are not indicated are generally carried out under conventional conditions for such reactions or under conditions recommended by the manufacturer. Unless otherwise specified, the experimental materials and reagents used in the following examples are commercially available, can be prepared according to prior art methods, or can be prepared according to methods similar to those disclosed in the present application.

Unless otherwise indicated, percentages and parts are percentages by weight and parts by weight; ratios of liquids are by volume; all temperatures are given in degrees Celsius unless otherwise indicated.

In the following examples, ¹H NMR and ¹⁹F NMR spectra were typically recorded on Bruker 400 MHz NMR and 500 MHz NMR spectrometers, with chemical shifts expressed in δ (ppm); mass spectra were recorded on an Agilent 1290 liquid chromatography + 6120B mass spectrometry LCMS liquid chromatography-mass spectrometry system. Silica gel column purification was performed using Biotage SelektSEL-2SV or ISO-1SV; preparative liquid chromatography purification was performed using Gilson 281 (column: waters Xbridge 19 mm * 250 mm, 5 µm or WELCH C18, 21.2 mm * 250 mm, 10 µm. Mobile phase: A: water (10 mM NH₄HCO₃ or 0.05% formic acid), B: acetonitrile (or containing 0.05% formic acid). Flow rate: 20-30 mL/min. Detection wavelength: 214 nm/254 nm), unless otherwise indicated.

**SFC-1:** Waters UPCC, analytical column: Daicel Chiralpak^{®}IG, 100 * 3 mm, 3 µm; Mobile phase A: CO₂, Mobile phase B: MeOH; Flow rate: 1.5 mL/min; Column temperature: 35 °C; Back pressure: 1800 psi; Gradient: 0-8.0 min A/B = 90/10.

**SFC-2:** Waters UPCC, analytical column: Daicel Chiralpak^{®}IC, 100 * 3 mm, 3 µm; Mobile phase A: CO₂, Mobile phase B: MeOH (0.1% DEA); Flow rate: 1.5 mL/min; Column temperature: 35 °C; Back pressure: 1800 psi; Gradient: 0-0.5 min A/B = 95/5, 0.5-5.0 min A/B = 95/5-60/40, 5.0-8.0 min A/B = 60/40.

**SFC-3:** Waters UPCC, analytical column: REGIS(S,S)WHELK-O1, 100 * 3 mm, 3 µm; Mobile phase A: CO₂, Mobile phase B: MeOH (+0.1% 7.0 M Ammonia); Flow rate: 1.5 mL/min; Column temperature: 35 °C; Back pressure: 1800 psi; Gradient: 0-5.0 min A/B = 80/20.

The following abbreviations are used in the synthetic examples below; abbreviations not listed have the meanings commonly understood by those skilled in the art.

A (absorbance); AA (amino acid); AAN (alanine-alanine-asparagine tripeptide); ACN or CH₃CN or MeCN (acetonitrile); AcOH (acetic acid); ADC (antibody-drug conjugate); Ag₂CO₃ (silver carbonate); BH₃ (borane); BIDIME (S-3-tert-butyl-4-(2,6-dimethoxyphenyl)-2,3-dihydrobenzo[*d*][1,3]oxaphosphole); Boc (tert-butoxycarbonyl); (Boc)₂O (di-tert-butyl dicarbonate); BOP (benzotriazol-1-yloxytris(dimethylamino)phosphonium hexafluorophosphate); BTC (butyltriphenylphosphonium chloride); t-Bu (tert-butyl); BuLi (butyl lithium); t-BuOK (potassium tert-butoxide); CBr₄ (carbon tetrabromide); Cbz (benzyloxycarbonyl); CDCl₃ (deuterated chloroform); CD₃I (deuterated iodomethane); CD₃OD-*d₄* (tetradeuterated methanol); cm (centimeter); cm² (square centimeter); CsCO₃ (cesium carbonate); DAR (drug-to-antibody ratio); CH₃I or MeI (methyl iodide); CO (carbon monoxide); m-CPBA (meta-chloroperoxybenzoic acid); CsF (cesium fluoride); CSI (chlorosulfonyl isocyanate); CuI (copper iodide); DCC (*N,N'-*dicyclohexylcarbodiimide); DCE (dichloroethane); DCM (dichloromethane); DEA or Et₂NH (diethylamine); DephosPdCl₂ (bis(diphenylphosphinophenyl ether)palladium dichloride); DIAD (diisopropyl azodicarboxylate); DIEA or DIPEA (N,N-diisopropylethylamine); DMA (dimethylacetamide); DMAP (p-dimethylaminopyridine); DMF (N,N-dimethylformamide); DMSO (dimethyl sulfoxide); DMSO-*d₆* (hexadeuterated dimethyl sulfoxide); DMTMM (4-(4,6-dimethoxytriazin-2-yl)-4-methylmorpholine hydrochloride); D₂O (deuterated water); DPPA (diphenylphosphoryl azide); DTT (dithiothreitol); E (trans olefin); EA or EtOAc (ethyl acetate); ED₅₀ (50% inhibitory dose); EDTA-K₂ (potassium ethylenediaminetetraacetate); ELISA (enzyme-linked immunosorbent assay); eq (equivalent); EtI (iodoethane); EtOH (ethanol); EVCit (glutamic acid-valine-citrulline tripeptide); ¹⁹F (fluorine nucleus); FCC (flash column chromatography); Fmoc (9-fluorenylmethyloxycarbonyl); g (gram); GE (glycine-glutamic acid dipeptide); GGFG (glycine-phenylalanine-glycine-glycine tetrapeptide); h (hour); ¹H (proton); H₂ (hydrogen); HATU (tetramethyluronium hexafluorophosphate); HCl (hydrogen chloride); HCl-MeOH or HCl/MeOH (hydrogen chloride methanol solution); HCOH (formaldehyde); HF (hydrogen fluoride); HFIP (hexafluoroisopropanol); HLM (human liver microsomes); HMDSLi (lithium bis(trimethylsilyl)amide); HMPA (hexamethylphosphoramide); H₂O (water); HOBt (1-hydroxybenzotriazole); HPLC (high performance liquid chromatography); H₂SO₄ (sulfuric acid); Hz (hertz); I (path length); I₂ (iodine); IC₅₀ (50% inhibitory concentration); *in vitro (in vitro* experiment); *in vivo (in vivo* experiment); IV or i.v. (intravenous administration); J (NMR coupling constant); K₂CO₃ (potassium carbonate); KF (potassium fluoride); KOAc (potassium acetate); K₃PO₄ (potassium phosphate); L (liter); L (relative stereochemical configuration); LCMS (liquid chromatography-mass spectrometry); LC-MS/MS (liquid chromatography-tandem mass spectrometry); LDA (lithium diisopropylamide); LiAlD₄ (lithium aluminum deuteride); LiAlH₄ (lithium aluminum hydride); LiCl (lithium chloride); LiOH (lithium hydroxide); M or MW (molecular weight); M (molar concentration); MeOH (methanol); MeONa (sodium methoxide); Methanol-*d₄* (tetradeuterated methanol); mg (milligram); MHz (megahertz); min (minute); mL (milliliter); mm (millimeter); mm³ (cubic millimeter); mM (millimolar concentration); mmol (millimole); MOM (methoxymethyl ether); MMPP (magnesium monoperoxyphthalate hexahydrate); MTBE (methyl tert-butyl ether); MS (mass spectrometry); MS-ESI (electron spray ionization mass spectrometry); MW (microwave); m/z (mass-to-charge ratio); N₂ (nitrogen); NaBH₄ (sodium borohydride); NaBH₃CN (sodium cyanoborohydride); NaCl (sodium chloride, table salt); Na₂CO₃ (sodium bicarbonate); NaH (sodium hydride); NaHCO₃ (sodium bicarbonate); NaI (sodium iodide); NaOAc (sodium acetate); NaOH (sodium hydroxide); Na₂SO₃ (sodium sulfite); Na₂SO₄ (sodium sulfate); Na₂S₂O₃ (sodium thiosulfate); NBS (bromosuccinimide); NCS (chlorosuccinimide); NH₃ (ammonia); NH₄Cl (ammonium chloride); NH₄HCO₃ (ammonium bicarbonate); NHS *(N-*hydroxysuccinimide); NH₄SCN (ammonium thiocyanate); NIS (iodosuccinimide); nm (nanometer); NMR (nuclear magnetic resonance); NOE (nuclear Overhauser effect); NPC (bis(p-nitrophenyl) carbonate); ³¹P (phosphorus nucleus); PABC (p-aminobenzyl alcohol); Pb(OAc)₄ (lead tetraacetate); PBS (phosphate buffered saline); Pd/C (palladium on carbon); PdCl₂(dtbpf) or Pd(dtbpf)Cl₂ (1,1'-bis(di-tert-butylphosphino)ferrocene palladium dichloride); PdCl₂(dppf) or Pd(dppf)Cl₂ (1,1'-bis(diphenylphosphino)ferrocene palladium dichloride); Pd(OAc) (palladium acetate); Pd(OH)₂ (palladium hydroxide); Pd(PPh₃)₄ or Pd-118 (tetrakistriphenylphosphine palladium); Pd(PPh₃)₂Cl₂ (bistriphenylphosphine palladium chloride); Pd(PPh₃)₄Cl₂ (tetrakistriphenylphosphine palladium chloride); PE (petroleum ether); PPh₃ (triphenylphosphine); PO or P.O. or p.o. (oral administration); POCl₃ (phosphorus oxychloride); psi (pounds per square inch); Py (pyridine); R (R configuration of chiral molecule); *Ra* (R configuration of axially chiral molecule); RP-HPLC (reverse phase high performance liquid chromatography); r.t. (room temperature); Rt (chromatographic retention time); S (S configuration of chiral molecule); *Sa* (S configuration of axially chiral molecule); s/d/t/q/m (NMR singlet/doublet/triplet/quartet/multiplet); Selectfluor (1-fluoro-4-methyl-1,4-diazabicyclo[2.2.2]octane tetrafluoroborate); SeO₂ (selenium dioxide); SFC (supercritical fluid chromatography); SiO₂ (silica gel); SO₂Cl₂ (sulfuryl chloride); TCEP (tris(2-carboxyethyl)phosphine); TEA or Et₃N (triethylamine); TBSCl or TBDSCl (tert-butyldimethylsilyl chloride); Teoc (2-(trimethylsilyl)ethoxycarbonyl); Teoc-Osu (N-[2-(trimethylsilyl)ethoxycarbonyloxy]succinimide); TFA (trifluoroacetic acid); TFE (trifluoroethanol); TGI (tumor growth inhibition); THF (tetrahydrofuran); TIPS (triisopropylsilyl); TIPSCI (triisopropylsilyl chloride); TLC (thin layer chromatography); TMPMgCl (2,2,6,6-tetramethylpiperidinyl magnesium chloride); TMSI (trimethylsilyl iodide); T₄P (1-butylphosphoric anhydride); Trt (trityl); TsOH (p-toluenesulfonic acid); TsOH·H₂O (p-toluenesulfonic acid monohydrate); VA (valine-alanine dipeptide); VCit (valine-citrulline dipeptide); v/v (volume ratio); %wt (weight percentage); w/w (weight ratio); Z (cis olefin); δ (chemical shift); µL (microliter); µm (micrometer); µM (micromolar); µmol (micromole).

### 6.1. Preparation of Payload Molecules

### Preparation of Intermediate A1: (S,E)-(4-(fluoromethylene)-1,3-dimethylpiperidin-3-yl)methanol

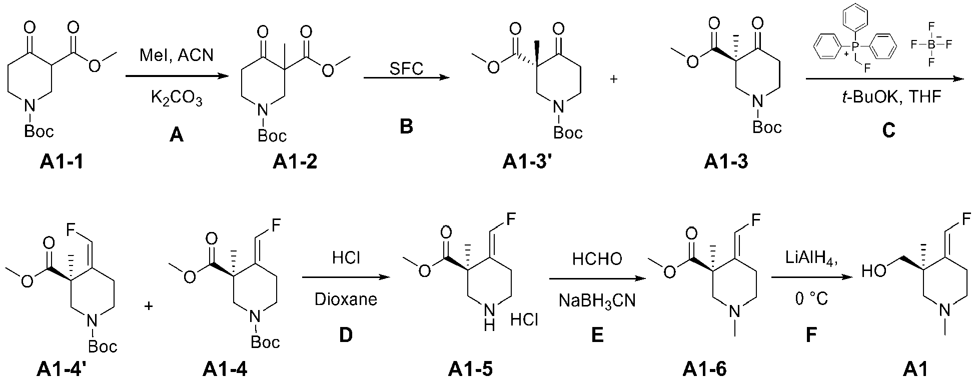

### Step A: 1-(tert-butyl) 3-methyl 3-methyl-4-oxopiperidine-1,3-dicarboxylate

At room temperature, iodomethane (13.79 g, 97.17 mmol) was added dropwise to a stirred mixture of 1-(tert-butyl) 3-methyl 4-oxopiperidine-1,3-dicarboxylate (5.0 g, 19.43 mmol), potassium carbonate (8.06 g, 58.30 mmol), and anhydrous acetonitrile (50 mL). After the addition, the mixture was stirred at room temperature overnight. After the reaction was complete as monitored by TLC, the reaction solution was poured into saturated aqueous ammonium chloride (100 mL) and extracted with ethyl acetate (3 × 100 mL). The collected organic phases were washed with saturated aqueous sodium chloride (50 mL), concentrated, and the crude product was purified by FCC (SiO₂, EA/PE = 0-20%) to give 1-(tert-butyl) 3-methyl 3-methyl-4-oxopiperidine-1,3-dicarboxylate (4.85 g, 92% yield) as a colorless oil. ¹H NMR (400 MHz, Methanol-*d*₄) δ 4.51 (d, *J* = 13.7 Hz, 1H), 4.23-4.05 (m, 1H), 3.73 (s, 3H), 3.47-3.27 (m, 1H), 3.17 (d, *J =* 13.7 Hz, 1H), 2.75 (s, 1H), 2.57-2.41 (m, 1H), 1.50 (s, 9H), 1.28 (s, 3H). MS-ESI [M+H-56]⁺: 216.0.

Step B: Compound 1-(tert-butyl) 3-methyl 3-methyl-4-oxopiperidine-1,3-dicarboxylate (120 g) was resolved by SFC (SFC150, Waters) (column: DAICEL CHIRALPAK^{®}IG, 250 * 50 mm, 10 µm; mobile phase: CO₂/MeOH = 90/10; flow rate: 120 mL/min) to give the first eluted isomer 1, compound **A1-3** (52.8 g, shorter retention time). Chiral analysis method SFC-1, Rt = 0.682 min. ¹H NMR (400 MHz, Chloroform-d) δ 4.59-4.42 (m, 1H), 4.26-3.98 (m, 1H), 3.73 (s, 3H), 3.42-3.24 (m, 1H), 3.16-3.01 (m, 1H), 2.93-2.63 (m, 1H), 2.58-2.40 (m, 1H), 1.49 (s, 9H), 1.31 (s, 3H). MS-ESI [M+H-56]⁺: 216.1; followed by the later eluted isomer 2, compound A1-3' (52.4 g, longer retention time). Chiral analysis method SFC-1, Rt = 1.035 min. ¹H NMR (400 MHz, Chloroform-d) δ 4.60-4.41 (m, 1H), 4.24-3.94 (m, 1H), 3.73 (s, 3H), 3.42-3.24 (m, 1H), 3.17-3.00 (m, 1H), 2.93-2.64 (m, 1H), 2.56-2.40 (m, 1H), 1.49 (s, 9H), 1.31 (s, 3H).

### Step C: (S,E)-1-(tert-butyl) 3-methyl 4-(fluoromethylene)-3-methylpiperidine-1,3-dicarboxylate and (S,Z)-1-(tert-butyl) 3-methyl 4-(fluoromethylene)-3-methylpiperidine-1,3-dicarboxylate

(Fluoromethylene)triphenylphosphonium tetrafluoroborate (10.56 g, 27.64 mmol) was dissolved in anhydrous tetrahydrofuran (50 mL), and the atmosphere was replaced with nitrogen three times. The reaction temperature was lowered to -70 °C using a dry ice-ethanol bath. A solution of potassium tert-butoxide in tetrahydrofuran (1M, 27.64 mL, 27.64 mmol) was added dropwise to the reaction system. The temperature was maintained and stirring continued for 1 hour. Then, a solution of (R)-1-(tert-butyl) 3-methyl 3-methyl-4-oxopiperidine-1,3-dicarboxylate (Intermediate **A1-3,** 5.0 g, 18.43 mmol) in anhydrous THF (15 mL) was added dropwise to the reaction system. After the addition, the resulting mixture was slowly warmed to room temperature and stirred overnight. After the reaction was complete as monitored by TLC, the reaction solution was slowly poured into water (100 mL) and extracted three times with ethyl acetate. After the organic phases were combined, washed with saturated brine, dried over anhydrous sodium sulfate, filtered and concentrated to dryness to give a crude product. The crude product was purified by FCC (SiO₂, EA/PE = 0-15%) to give the product (*S*,*E*)-1-(tert-butyl) 3-methyl 4-(fluoromethylene)-3-methylpiperidine-1,3-dicarboxylate (1.98 g, 37% yield) as a colorless oil. ¹H NMR (400 MHz, Chloroform-d) δ 6.55 (d, *J =* 84.7, 1H), 4.35 (d, *J =* 13.2 Hz, 1H), 4.10-3.82 (m, 1H), 3.69 (s, 3H), 3.00-2.85 (m, 1H), 2.76 (d, *J =* 13.1 Hz, 1H), 2.71-2.60 (m, 1H), 2.31-2.08 (m, 1H), 1.46 (s, 9H), 1.29 (s, 3H). MS-ESI [M+H-56]⁺: 232.1; the double bond configuration of compound **A1-4** was monitored by NOE signals between H1 and H2; and the product (*S*,*Z*)-1-(tert-butyl) 3-methyl 4-(fluoromethylene)-3-methylpiperidine-1,3-dicarboxylate (600 mg, 11% yield) as a colorless oil. ¹H NMR (400 MHz, Chloroform-d) δ 6.43 (d, *J =* 83.7, 1H), 3.86-3.75 (m, 1H), 3.71 (s, 3H), 3.62-3.47 (m, 1H), 3.42-3.29 (m, 2H), 2.24-2.06 (m, 2H), 1.46 (s, 9H), 1.43-1.39 (m, 3H). MS-ESI [M+H-56]⁺: 232.1; the double bond configuration of compound **A1-4'** was monitored by NOE signals between H1 and H3.

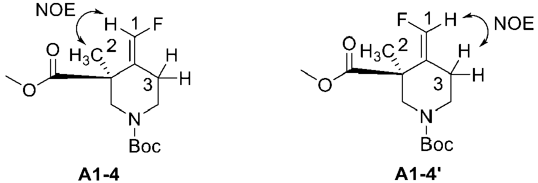

### Step D: (S,E)-methyl 4-(fluoromethylene)-3-methylpiperidine-3-carboxylate hydrochloride

At room temperature, hydrochloric acid in dioxane (4M, 10 mL) was added to (S,E)-1-(tert-butyl) 3-methyl 4-(fluoromethylene)-3-methylpiperidine-1,3-dicarboxylate (600 mg, 2.09 mmol), stirred at room temperature for 1 hour. The acid solution was removed by concentration to give *(S,E)-*methyl 4-(fluoromethylene)-3-methylpiperidine-3-carboxylate hydrochloride (572 mg, 100% yield) as a white solid. MS-ESI [M+H]⁺: 188.1.

### Step E: (S,E)-methyl 4-(fluoromethylene)-1,3-dimethylpiperidine-3-carboxylate

At room temperature, (S,E)-methyl 4-(fluoromethylene)-3-methylpiperidine-3-carboxylate hydrochloride (370 mg, 1.98 mmol) was dissolved in methanol (5 mL), and triethylamine was added dropwise until pH 10, stirred for 10 minutes, then glacial acetic acid was added dropwise until pH 4. Formaldehyde aqueous solution (481.15 mg, 5.93 mmol) was added to the reaction solution, and stirred at room temperature for 30 minutes. Sodium cyanoborohydride (136.62 mg, 2.17 mmol) was added to the reaction solution, and stirred at room temperature for 2 hours. After the reaction was complete as monitored by LCMS, the solvent was concentrated and removed under reduced pressure and the residue was evaporated twice with anhydrous tetrahydrofuran to give (S,E)-methyl 4-(fluoromethylene)-1,3-dimethylpiperidine-3-carboxylate (380 mg, 96% yield) as a white solid. MS-ESI [M+H]⁺: 202.1.

### Step F: (S,E)-(4-(fluoromethylene)-1,3-dimethylpiperidin-3-yl)methanol

Under ice-bath cooling, a solution of lithium aluminum hydride in tetrahydrofuran (1M, 2.83 mL, 107.5 mg, 2.83 mmol) was added dropwise to a solution of (E)-methyl 4-(fluoromethylene)-1,3-dimethylpiperidine-3-carboxylate (380 mg, 1.89 mmol) in anhydrous tetrahydrofuran (5 mL). The resulting mixture was stirred at room temperature for 20 minutes. After the reaction was complete as monitored by LCMS, the reaction solution was quenched with sodium sulfate decahydrate until no bubbles were generated. About 5 g of anhydrous sodium sulfate was added to remove water. The reaction solution was filtered through Celite, and the filter cake was washed three times with anhydrous tetrahydrofuran. The filtrate was collected and concentrated to dryness to give the product (S,E)-(4-(fluoromethylene)-1,3-dimethylpiperidin-3-yl)methanol (**A1**, 300 mg, 92% yield) as a colorless oil. MS-ESI [M+H]⁺: 174.1

### Preparation of Intermediate A2: ((3S,4S)-4-(difluoromethyl)-1,3-dimethylpiperidin-3-yl)methanol

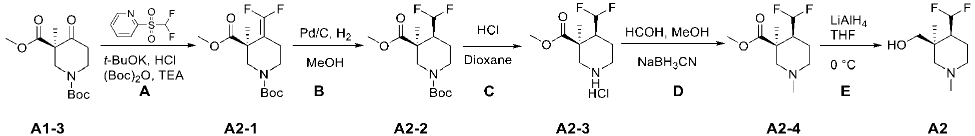

### Step A: (S)-1-(tert-butyl) 3-methyl 4-(difluoromethylene)-3-methylpiperidine-1,3-dicarboxylate

(R)-1-(tert-butyl) 3-methyl 3-methyl-4-oxopiperidine-1,3-dicarboxylate (A1-3, 3.00 g, 11.06 mmol) and 2-((difluoromethyl)sulfonyl)pyridine (3.20 g, 16.59 mmol) were dissolved in 30 mL of anhydrous DMF, and the atmosphere was replaced with nitrogen three times. The resulting mixture was cooled with a dry ice-ethanol bath, and a solution of potassium tert-butoxide in tetrahydrofuran (1M, 19.90 mL, 19.90 mmol) was added dropwise to the reaction system. The temperature was maintained, and the reaction was stirred for 1 hour. Saturated aqueous ammonium chloride (30 mL) was added dropwise to the reaction system, then warmed to -40 °C, and hydrochloric acid solution (6M, 20 mL, 120 mmol) was added dropwise. After the addition, the reaction solution was slowly warmed to 70 °C and stirred for two days. After completion as monitored by LCMS, saturated aqueous potassium carbonate solution was slowly added to adjust the pH to 9. The mixture was extracted three times with a mixture (DCM/MeOH = 10/1). The organic phases were combined, washed with saturated brine, dried over anhydrous sodium sulfate, filtered, and concentrated to dryness to give a crude product. The crude product was dissolved in 20 mL of dichloromethane, and di-tert-butyl dicarbonate (5 mL) and triethylamine (5 mL) were added at room temperature. The resulting mixture was stirred at room temperature for 1 hour. After the reaction was complete as monitored by TLC, the reaction solution was concentrated to dryness, and the crude product was purified by FCC (SiO₂, EA/PE = 0-7%) to give the product (S)-1-(tert-butyl) 3-methyl 4-(difluoromethylene)-3-methylpiperidine-1,3-dicarboxylate (1.0 g, 30% yield) as a colorless solid. MS-ESI [M+H-56]⁺: 250.1.

### Step B: (3S,4S)-1-(tert-butyl) 3-methyl 4-(difluoromethyl)-3-methylpiperidine-1,3-dicarboxylate

At room temperature, (S)-1-(tert-butyl) 3-methyl 4-(difluoromethylene)-3-methylpiperidine-1,3-dicarboxylate (250 mg, 0.82 mmol) was dissolved in methanol (20 mL), and the atmosphere was replaced with nitrogen three times. Palladium on carbon (87 mg, 0.08 mmol) was added, and the atmosphere was replaced with hydrogen three times. The mixture was stirred at room temperature overnight under 15 psi of hydrogen. After the reaction was complete as monitored by LCMS, the reaction solution was filtered through Celite, and the filter cake was washed three times with methanol. The filtrate was collected and concentrated to dryness to give the product (3*S*,4*S*)-1-(tert-butyl) 3-methyl 4-(difluoromethyl)-3-methylpiperidine-1,3-dicarboxylate (200 mg, 79% yield) as a colorless oil. MS-ESI [M+H]⁺: 252.1.

### Step C: (3S,4S)-methyl 4-(difluoromethyl)-3-methylpiperidine-3-carboxylate hydrochloride

At room temperature, hydrochloric acid in dioxane (4M, 5 mL) was added to (3S,4S)-1-(tert-butyl) 3-methyl 4-(difluoromethyl)-3-methylpiperidine-1,3-dicarboxylate (190 mg, 0.62 mmol). The resulting mixture was stirred at room temperature for 30 minutes, the acid solution was removed by concentration to give (3S,4S)-methyl 4-(difluoromethyl)-3-methylpiperidine-3-carboxylate hydrochloride (150 mg, 100% yield) as a white solid. MS-ESI [M+H]⁺: 208.1.

### Step D: (3S,4S)-methyl 4-(difluoromethyl)-1,3-dimethylpiperidine-3-carboxylate

At room temperature, (3S,4S)-methyl 4-(difluoromethyl)-3-methylpiperidine-3-carboxylate hydrochloride (792 mg, 3.25 mmol) was dissolved in methanol (10 mL), and formaldehyde aqueous solution (37% wt, 791 mg, 9.75 mmol) was added. The resulting mixture was stirred at room temperature for 30 minutes. Sodium cyanoborohydride (245 mg, 3.90 mmol) was added, and the mixture was stirred at room temperature for 1.5 hours. After the reaction was complete as monitored by LCMS, the system was concentrated to dryness and evaporated twice with anhydrous tetrahydrofuran to give the product (3*S*,4*S*)-methyl 4-(difluoromethyl)-1-ethyl-3-methylpiperidine-3-carboxylate (536 mg, 75% yield) as a colorless oil. MS-ESI [M+H]⁺: 222.1.

### Step E: ((3S,4S)-4-(difluoromethyl)-1,3-dimethylpiperidin-3-yl)methanol

Under ice-bath cooling, a solution of lithium aluminum hydride in tetrahydrofuran (1M, 3.60 mL, 3.60 mmol) was added dropwise to a solution of (3S,4S)-methyl 4-(difluoromethyl)-1,3-dimethylpiperidine-3-carboxylate (536 mg, 2.42 mmol) in anhydrous tetrahydrofuran (10 mL). The resulting mixture was stirred at room temperature for 15 minutes. After the reaction was complete as monitored by LCMS, the reaction was quenched with sodium sulfate decahydrate until no bubbles were generated. About 10 g of anhydrous sodium sulfate was added, the mixture was filtered through Celite, and the filter cake was washed three times with anhydrous tetrahydrofuran. The filtrate was collected and concentrated to dryness to give the product ((3S,4S)-4-(difluoromethyl)-1,3-dimethylpiperidin-3-yl)methanol (A2, 354 mg, 76% yield) as a colorless oil. MS-ESI [M+H]⁺: 194.1.

### Preparation of Intermediate A3: (S,E)-(4-(fluoromethylene)-3-methyl-1-(methyl-d₃)piperidin-3-yl)methylene-d₂-ol

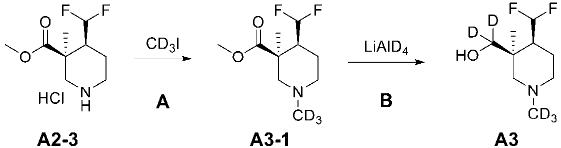

### Step A: (S,E)-methyl 4-(fluoromethylene)-3-methyl-1-(methyl-d₃)piperidine-3-carboxylate

At room temperature, potassium carbonate (37.9 g, 275 mmol) was added to a mixed solution of (S,E)-methyl 4-(fluoromethylene)-3-methylpiperidine-3-carboxylate hydrochloride (A2-3, 22.3 g, 91.5 mmol), deuterated iodomethane (15.9 g, 110 mmol), and acetonitrile (400 mL). After addition, the mixture was heated to 50 °C and stirred for 4 hours. After the reaction was complete as monitored by LCMS, filtered, and the filter cake was rinsed with acetonitrile (150 mL). The filtrate was collected, concentrated, and further purified by FCC (SiO₂, MeOH/DCM = 0-5%) to give (S,E)-methyl 4-(fluoromethylene)-3-methyl-1-(methyl-*d₃*)piperidine-3-carboxylate (14.0 g, 68% yield) as a colorless liquid. MS-ESI [M+H]⁺: 225.1.

### Step B: (S,E)-(4-(fluoromethylene)-3-methyl-1-(methyl-d₃)piperidin-3-yl)methylene-d₂-ol

Under ice-bath cooling, lithium aluminum deuteride powder (2.61 g, 62.2 mmol) was added to a solution of (S,E)-methyl 4-(fluoromethylene)-3-methyl-1-(methyl-ds)piperidine-3-carboxylate (14.0 g, 62.2 mmol) in anhydrous tetrahydrofuran (140 mL). The resulting mixture was stirred under ice-bath conditions for 15 minutes. After the reaction was complete as monitored by LCMS, the reaction was quenched with sodium sulfate decahydrate until no bubbles were generated. About 10 g of anhydrous sodium sulfate was added to remove water. The reaction solution was filtered through Celite, and the filter cake was washed three times with anhydrous tetrahydrofuran. The filtrate was collected and concentrated to dryness to give the product (S,E)-(4-(fluoromethylene)-3-methyl-1-(methyl-*d₃*)piperidin-3-yl)methylene-*d₂*-ol (A3, 12.0 g, 97% yield) as a colorless oil. This product was used directly in the next step without purification. MS-ESI [M+H]⁺: 199.2.

### Preparation of Intermediate A4: (7-fluoro-8-((triisopropylsilyl)ethynyl)-3-((triisopropylsilyl)oxy)naphthalen-1-yl)boronic acid

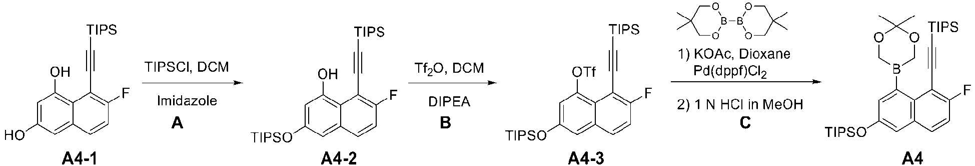

### Step A: 7-fluoro-8-((triisopropylsilyl)ethynyl)-3-((triisopropylsilyl)oxy)naphthalen-1-ol

Under ice-bath cooling and stirring, TIPSCl (177 g, 920 mmol) was added dropwise to a solution of 7-fluoro-8-((triisopropylsilyl)ethynyl)naphthalene-1,3-diol (300 g, 837 mmol) and imidazole (119 g, 1.76 mol) in dichloromethane (3 L). After the addition, the system was slowly warmed to room temperature and stirred for 6 hours. After the reaction was complete as monitored by TLC. Water (900 mL) was added, stirred for 30 minutes, and the layers were separated. The aqueous phase was extracted with dichloromethane (900 mL). The organic phases were combined, dried over anhydrous sodium sulfate, filtered, concentrated to dryness, and purified by silica gel chromatography (PE/EA = 50/1) to give the compound 7-fluoro-8-((triisopropylsilyl)ethynyl)-3-((triisopropylsilyl)oxy)naphthalen-1-ol (397 g, 92% yield).

### Step B: 7-fluoro-8-((triisopropylsilyl)ethynyl)-3-((triisopropylsilyl)oxy)naphthalen-1-yl trifluoromethanesulfonate

At -45 to -35 °C conditions, trifluoromethanesulfonic anhydride (326 g, 1.16 mol) was added dropwise to a solution of 7-fluoro-8-((triisopropylsilyl)ethynyl)-3-((triisopropylsilyl)oxy)naphthalen-1-ol (397 g, 0.77 mol) and DIPEA (298 g, 2.31 mol) in dichloromethane (4 L). After the addition, the temperature was maintained and stirring continued for 0.5 hours. The reaction was complete as monitored by TLC. The system was poured into water (800 mL), the layers were separated, and the aqueous phase was extracted with dichloromethane (1.2 L). The organic phases were combined, dried over anhydrous sodium sulfate, filtered, concentrated to dryness, and purified by silica gel chromatography (PE/EA = 50/1) to give the compound 7-fluoro-8-((triisopropylsilyl)ethynyl)-3-((triisopropylsilyl)oxy)naphthalen-1-yl trifluoromethanesulfonate (469 g, 94% yield).

### Step C: (7-fluoro-8-((triisopropylsilyl)ethynyl)-3-((triisopropylsilyl)oxy)naphthalen-1-yl)boronic acid

Under nitrogen protection, Pd(dppf)Cl₂ (13.2 g, 18.2 mmol) was added to a solution of 7-fluoro-8-((triisopropylsilyl)ethynyl)-3-((triisopropylsilyl)oxy)naphthalen-1-yl trifluoromethanesulfonate (235 g, 0.36 mol), 5,5,5',5'-tetramethyl-2,2'-bi(1,3,2-dioxaborinane) (164 g, 0.73 mol), and potassium acetate (107 g, 1.1 mol) in 1,4-dioxane (2.4 L). The system was heated to 85 °C and stirred for 20 hours. The reaction was complete as monitored by TLC. The mixture was cooled to room temperature, filtered through Celite, rinsed with EA, concentrated, and purified by silica gel chromatography (EA/PE = 0-5%) to give crude compound.

The crude compound was dissolved in methanol (1.2 L), and hydrochloric acid solution (1M, 2.4 L) was added. The resulting mixture was stirred at room temperature for 30 minutes. Ethyl acetate (2.4 L) was added, and stirring continued for 2 hours. The mixture was allowed to stand, then the layers were separated, and the organic phase was washed sequentially with water (2.4 L) and saturated brine (2 × 2.4 L). After concentration, (7-fluoro-8-((triisopropylsilyl)ethynyl)-3-((triisopropylsilyl)oxy)naphthalen-1-yl)boronic acid (A4, 183 g, 92% yield) was obtained. ¹H NMR (400 MHz, Chloroform-d) δ 7.66-7.60 (m, 1H), 7.34 (d, *J* = 2.5 Hz, 1H), 7.24-7.19 (m, 1H), 7.18 (d, *J* = 2.5 Hz, 1H), 4.52 (s, 2H), 1.35-1.29 (m, 3H), 1.24-1.21 (m, 3H), 1.20-1.17 (m, 18H), 1.12 (d, *J = 7.3* Hz, 18H). MS-ESI [M+H]⁺: 543.3.

### Preparation of Intermediate A5: tert-butyl (4-(5,5-dimethyl-1,3,2-dioxaborinan-2-yl)benzo[b]thiophen-2-yl)carbamate

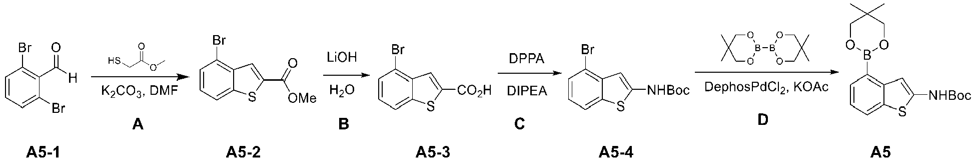

### Step A: methyl 4-bromobenzo[b]thiophene-2-carboxylate

At room temperature, compound 2,6-dibromobenzaldehyde (15.7 g, 59.5 mmol), methyl mercaptoacetate (5.6 mL, 65.4 mmol), potassium carbonate (15.7 g, 119.0 mmol), and DMF (200 mL) were sequentially added to a reaction flask containing a stir bar. The resulting mixture was heated to 110 °C and stirred for 10 hours. The reaction was complete as monitored by TLC, the reaction mixture was poured into 300 mL of water, and a large amount of yellow solid precipitated, filtered, and the filter cake was washed with water, and dried at 50 °C to give crude methyl 4-bromobenzo[b]thiophene-2-carboxylate (18 g). MS-ESI [M+H]⁺: 270.9.

### Step B: 4-bromobenzo[b]thiophene-2-carboxylic acid

Lithium hydroxide monohydrate (5.4 g, 129.7 mmol) and methyl 4-bromobenzo[b]thiophene-2-carboxylate (7.0 g, 25.9 mmol) were sequentially added to a mixed solution of tetrahydrofuran (10 mL) and water (2 mL). The resulting mixture was stirred at room temperature for 16 hours. After the reaction was complete as monitored by LCMS, the system was adjusted to pH 3 with 1M hydrochloric acid solution, extracted with ethyl acetate (3 × 60 mL), dried over anhydrous sodium sulfate, filtered, and concentrated under reduced pressure to give crude 1-benzothiophene-2-carboxylic acid (8.0 g). MS-ESI [M+H]⁺: 256.9.

### Step C: tert-butyl (4-bromobenzo[b]thiophen-2-yl)carbamate

4-Bromobenzo[*b*]thiophene-2-carboxylic acid (2.5 g), DPPA (3.2 mL, 14.7 mmol), DIEA (2.0 mL, 19.5 mmol), toluene (25 mL), and tert-butanol (1.4 mL, 14.7 mmol) were sequentially added to a reaction flask. The resulting mixture was stirred at 100 °C for 16 hours. After the reaction was complete as monitored by LCMS, the mixture was concentrated under reduced pressure, and the crude product was purified by FCC (SiO₂, EA/PE = 0-30%) to give tert-butyl (4-bromo-1-benzothiophen-2-yl)carbamate (3.0 g). MS-ESI [M+H-56]⁺: 271.9.

### Step D: tert-butyl (4-(5,5-dimethyl-1,3,2-dioxaborinan-2-yl)benzo[b]thiophen-2-yl)carbamate

tert-Butyl (4-bromobenzo[b]thiophen-2-yl)carbamate (2.9 g, 8.9 mmol), 5,5,5',5'-tetramethyl-2,2'-bis(1,3,2-dioxaborinane) (5.0 g, 22.3 mmol), DephosPdCl₂ (0.64 g, 0.9 mmol), potassium acetate (2.6 g, 26.7 mmol), and 1,4-dioxane (30 mL) were sequentially added to a reaction flask. The atmosphere was replaced with nitrogen three times, and the reaction solution was stirred at 95 °C for 1 hour. After the reaction was complete as monitored by LCMS, concentrated under reduced pressure, and the crude product was purified by FCC (SiO₂, EA/PE = 0-30%) to give tert-butyl (4-(5,5-dimethyl-1,3,2-dioxaborinan-2-yl)benzo[b]thiophen-2-yl)carbamate (**A5**, 3.1 g, 98% yield).

### Preparation of Intermediate A6: tert-butyl (1R,5S)-3-(2,6,8-trifluoro-7-(7-fluoro-8-((triisopropylsilyl)ethynyl)-3-((triisopropylsilyl)oxy)naphthalen-1-yl)quinazolin-4-yl)-3,8-diazabicyclo[3.2.1]octane-8-carboxylate

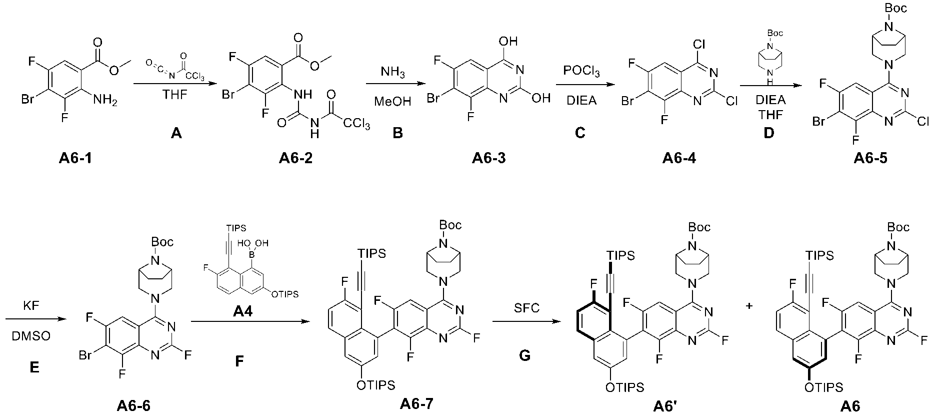

### Step A: methyl 4-bromo-3,5-difluoro-2-(3-(2,2,2-trichloroacetyl)ureido)benzoate

At room temperature, 2-amino-4-bromo-3,5-difluoromethyl benzoate (34 g, 128 mmol) and tetrahydrofuran (500 mL) were added to a round-bottom flask containing a stir bar. 2,2,2-Trichloroacetyl isocyanate (28.9 g, 153 mmol) was added dropwise to the system with stirring at room temperature. The resulting mixture was stirred at room temperature for 2 hours and then concentrated under reduced pressure to give methyl 4-bromo-3,5-difluoro-2-(3-(2,2,2-trichloroacetyl)ureido)benzoate (crude) as a brown solid, which was used directly in the next step. MS-ESI [M+H]⁺: 452.8.

### Step B: 7-bromo-6,8-difluoroquinazoline-2,4-diol

At room temperature, methyl 4-bromo-3,5-difluoro-2-(3-(2,2,2-trichloroacetyl)ureido)benzoate obtained in the previous step was added to a round-bottom flask containing a stir bar, and then ammonia in methanol solution (7M, 400 mL) was added. The resulting mixture was stirred at room temperature for 2 hours. The reaction was complete as monitored by LCMS. After concentration under reduced pressure, the resulting solid was slurried with methyl tert-butyl ether and filtered to give 7-bromo-6,8-difluoroquinazoline-2,4-diol (32 g, 90% yield over two steps) as a pale yellow solid. MS-ESI [M+H]⁺: 277.0.

### Step C: 7-bromo-2,4-dichloro-6,8-difluoroquinazoline

7-Bromo-6,8-difluoroquinazoline-2,4-diol (6 g, 22 mmol) and phosphorus oxychloride (50 mL) were added to a round-bottom flask containing a stir bar. DIEA (12 mL) was added dropwise to the system with stirring at room temperature. After the addition, the system was heated to 110 °C and stirred overnight. The reaction solution was concentrated under reduced pressure to about 30 mL, poured into water (500 mL), and a precipitate formed. The solid was collected by filtration and dried to give 7-bromo-2,4-dichloro-6,8-difluoroquinazoline (5 g, 74% yield) as a yellow solid. MS-ESI [M+H]⁺: 312.9.

### Step D: tert-butyl (1R,5S)-3-(7-bromo-2-chloro-6,8-difluoroquinazolin-4-yl)-3,8-diazacyclo[3.2.1]octane-8-carboxylate

7-Bromo-2,4-dichloro-6,8-difluoroquinazoline (8.0 g, 26 mmol), DIEA (8.0 mL), and tetrahydrofuran (80 mL) were added to a round-bottom flask containing a stir bar. tert-Butyl (1*R*,5*S*)-3,8-diazabicyclo[3.2.1]octane-8-carboxylate (6.0 g, 28 mmol) was slowly added to the reaction system with stirring at room temperature, and the resulting mixture was stirred for 1 hour. The reaction solution was concentrated, poured into water, and filtered. The filter cake was collected and dried to give tert-butyl (1*R*,5*S*)-3-(7-bromo-2-chloro-6,8-difluoroquinazolin-4-yl)-3,8-diazacyclo[3.2.1]octane-8-carboxylate (9.0 g, 72% yield) as a yellow solid. MS-ESI [M+H]⁺: 499.0.

### Step E: tert-butyl (1R,5S)-3-(7-bromo-2,6,8-trifluoroquinazolin-4-yl)-3,8-diazabicyclo[3.2.1]octane-8-carboxylate

A mixture of tert-butyl (1*R*,5*S*)-3-(7-bromo-2-chloro-6,8-difluoroquinazolin-4-yl)-3,8-diazabicyclo[3.2.1]octane-8-carboxylate (9.0 g, 18.4 mmol), potassium fluoride (21.4 g, 368 mmol), and dimethyl sulfoxide (100 mL) was stirred at 100 °C overnight. After the reaction was complete as monitored by LCMS, the reaction solution was cooled to room temperature. Under stirring, the reaction solution was slowly poured into water (1 L), and a yellow solid was precipitated, filtered, and the filter cake was washed with water (200 mL). The filter cake was collected and dried to give tert-butyl (1*R*,5*S*)-3-(7-bromo-2,6,8-trifluoroquinazolin-4-yl)-3,8-diazacyclo[3.2.1]octane-8-carboxylate (7.7 g, 88% yield) as a yellow solid. MS-ESI [M+H]⁺: 473.4.

### Step F: tert-butyl (1R,5S)-3-(2,6,8-trifluoro-7-(7-fluoro-8-((triisopropylsilyl)ethynyl)-3-((triisopropylsilyl)oxy)naphthalen-1-yl)quinazolin-4-yl)-3,8-diazabicyclo[3.2.1]octane-8-carboxylate

At room temperature, tert-butyl (1*R*,5*S*)-3-(7-bromo-2,6,8-trifluoroquinazolin-4-yl)-3,8-diazabicyclo[3.2.1]octane-8-carboxylate **(A4,** 3.0 g, 6.34 mmol), (7-fluoro-8-((triisopropylsilyl)ethynyl)-3-((triisopropylsilyl)oxy)naphthalen-1-yl)boronic acid (4.13 g, 7.61 mmol), palladium acetate (71 mg, 0.32 mmol), BIDIME (209 mg, 0.63 mmol), and potassium phosphate (4.04 g, 19.02 mmol) were dissolved in tert-amyl alcohol (30 mL). The atmosphere was replaced with nitrogen three times, and the mixture was heated to 110 °C and stirred overnight. After the reaction was complete as monitored, the mixture was filtered through Celite and washed with ethyl acetate (50 mL). The resulting organic phase was concentrated and purified by FCC (SiO₂, EA/PE = 0-50%) to give tert-butyl (1*R*,5*S*)-3-(2,6,8-trifluoro-7-(7-fluoro-8-((triisopropylsilyl)ethynyl)-3-((triisopropylsilyl)oxy)naphthalen-1-yl)quinazolin-4-yl)-3,8-diazabicyclo[3.2.1]octane-8-carboxylate (2.3 g, 41% yield) as a yellow solid. ¹H NMR (400 MHz, Chloroform-*d*) δ 7.74 (dd, *J* = 9.1, 5.6 Hz, 1H), 7.39 (dd, *J* = 9.8, 1.7 Hz, 1H), 7.34 (d, *J* = 2.6 Hz, 1H), 7.31-7.28 (m, 1H), 7.10 (d, *J* = 2.6 Hz, 1H), 4.86-4.61 (m, 1H), 4.54-4.26 (m, 2H), 4.20-4.01 (m, 1H), 3.92-3.69 (m, 1H), 3.58-3.36 (m, 1H), 2.11-1.97 (m, 3H), 1.53 (s, 9H), 1.36-1.23 (m, 4H), 1.17-1.08 (m, 18H), 0.95-0.79 (m, 18H), 0.61-0.49 (m, 3H). MS-ESI [M+H]⁺: 891.4.

**Step G:** Compound tert-butyl (1*R*,5*S*)-3-(2,6,8-trifluoro-7-(7-fluoro-8-((triisopropylsilyl)ethynyl)-3-((triisopropylsilyl)oxy)naphthalen-1-yl)quinazolin-4-yl)-3,8-diazabicyclo[3.2.1]octane-8-carboxylate (10 g) was resolved by SFC (SFC150, Waters) (column: DAICEL CHIRALPAK^{®}IC, 250 * 25 mm, 10 µm; mobile phase: CO₂/MeOH = 75/25; flow rate: 70 mL/min) to give the first eluted isomer 1, intermediate **A6'** (4.5 g, shorter retention time). Chiral analysis method SFC-2, Rt = 3.855 min. MS-ESI [M+H]⁺: 891.4; followed by the later eluted isomer 2, intermediate **A6** (4.9 g, longer retention time). Chiral analysis method SFC-2, Rt = 4.198 min. ¹H NMR (400 MHz, Chloroform-d) δ 7.74 (dd, *J* = 9.1, 5.7 Hz, 1H), 7.40 (dd, *J* = 9.7, 1.7 Hz, 1H), 7.34 (d, *J* = 2.5 Hz, 1H), 7.29 (d, *J* = 8.7 Hz, 1H), 7.10 (d, *J* = 2.6 Hz, 1H), 4.81-4.64 (m, 1H), 4.51-4.32 (m, 2H), 4.18-4.03 (m, 1H), 3.91-3.69 (m, 1H), 3.52-3.32 (m, 1H), 2.30-1.93 (m, 3H), 1.53 (s, 9H), 1.37-1.21 (m, 4H), 1.16-1.05 (m, 18H), 0.94-0.80 (m, 18H), 0.62-0.47 (m, 3H). ¹⁹F NMR (376 MHz, Chloroform-*d*) δ -47.14, -105.65, -112.24, -120.80. MS-ESI [M+H]⁺: 891.4.

### Preparation of Intermediate A7: tert-butyl (1R,5S)-3-(8-fluoro-7-(7-fluoro-8-((triisopropylsilyl)ethynyl)-3-((triisopropylsilyl)oxy)naphthalen-1-yl)-2-(methylsulfonyl)pyrido[4,3-d]pyrimidin-4-yl)-3,8-diazabicyclo[3.2.1]octane-8-carboxylate

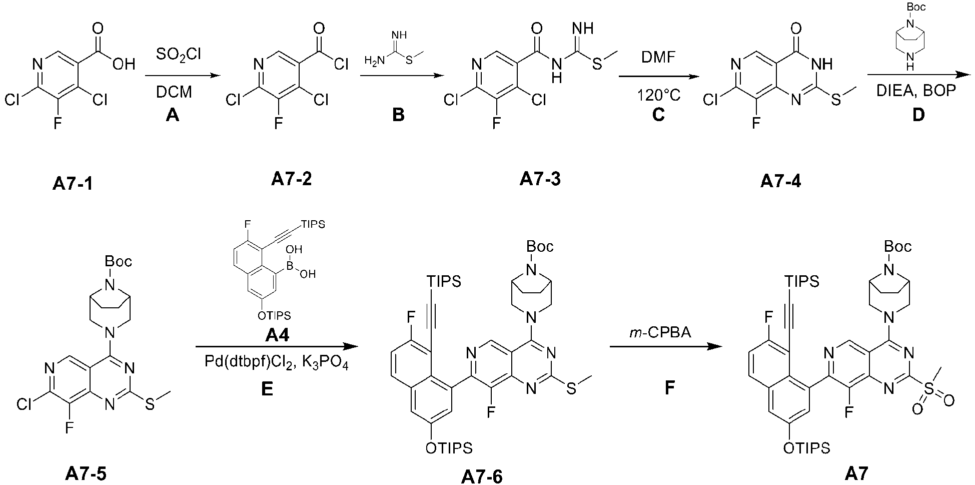

### Step A: 4,6-dichloro-5-fluoronicotinoyl chloride

Under stirring at room temperature, thionyl chloride (2.25 mL, 31 mmol) was slowly added to a solution of 4,6-dichloro-5-fluoronicotinic acid (5.0 g, 23.4 mmol) in dichloromethane (100 mL), followed by the addition of DMF (175 mg, 2.4 mmol). The resulting reaction solution was stirred at 50 °C for 2 hours. The reaction was complete as monitored by TLC. The reaction solution was concentrated, and a small amount of toluene was added and evaporated to give 4,6-dichloro-5-fluoronicotinoyl chloride (4.5 g, 83% yield) as a yellow solid, which was used directly in the subsequent reaction.

### Step B: methyl (4,6-dichloro-5-fluoronicotinoyl)carbamimidothioate

Under stirring at 0 °C, a mixed solution of 4,6-dichloro-5-fluoronicotinoyl chloride (4.5 g, 19.8 mmol) in ethylene glycol dimethyl ether (20 mL) was slowly added dropwise to a mixed solution of 2-methylisothiourea sulfate (15 g, 49.5 mmol) and aqueous sodium hydroxide (1M, 70 mL). The temperature was maintained and stirring continued for 1 hour. The precipitated solid was filtered and dried to give the product methyl (4,6-dichloro-5-fluoronicotinoyl)carbamimidothioate (5.0 g, 90% yield). MS-ESI [M+H]⁺: 282.1.

### Step C: 7-chloro-8-fluoro-2-(methylthio)pyrido[4,3-d]pyrimidin-4(3H)-one

Methyl (4,6-dichloro-5-fluoronicotinoyl)carbamimidothioate (5.0 g, 17.8 mmol) was dissolved in DMF (40 mL) and heated to 120 °C with stirring for 3 hours. After the reaction was complete as monitored by LCMS, the mixture was cooled to room temperature and water (200 mL) was added. The precipitated solid was filtered and dried to give the product methyl (4,6-dichloro-5-fluoronicotinoyl)carbamimidothioate (3.6 g, 82% yield). MS-ESI [M+H]⁺: 245.6.

### Step D: tert-butyl (1R,5S)-3-(7-chloro-8-fluoro-2-(methylthio)pyrido[4,3-d]pyrimidin-4-yl)-3,8-diazabicyclo[3.2.1]octane-8-carboxylate

At room temperature, to a solution of 7-chloro-8-fluoro-2-(methylthio)pyrido[4,3-*d*]pyrimidin-4(3*H*)-one (1.0 g, 4.0 mmol) in DMF (15 mL) were added tert-butyl 3,8-diazabicyclo[3.2.1]octane-8-carboxylate (1.0 g, 4.9 mmol), BOP (2.3 g, 5.3 mmol), and DIEA (1.0 g, 8.1 mmol). The reaction solution was stirred at 50 °C for 2 hours. After the reaction was complete as monitored by LCMS, the reaction solution was poured into 100 mL of ice water to quench the reaction. Extraction with ethyl acetate. The organic phases were combined, washed with saturated brine, dried over anhydrous sodium sulfate, filtered, and concentrated to dryness. The obtained crude product was purified by FCC (SiO₂, EA/PE = 0-20%) to give the product tert-butyl (1*R*,5*S*)-3-(7-chloro-8-fluoro-2-(methylthio)pyrido[4,3-*d*]pyrimidin-4-yl)-3,8-diazabicyclo[3.2.1]octane-8-carboxylate (980 mg, 55% yield) as a yellow solid. MS-ESI [M+H]⁺: 440.1.

### Step E: tert-butyl (1R,5S)-3-(8-fluoro-7-(7-fluoro-8-((triisopropylsilyl)ethynyl)-3-((triisopropylsilyl)oxy)naphthalen-1-yl)-2-(methylthio)pyrido[4,3-d]pyrimidin-4-yl)-3,8-diazabicyclo[3.2.1]octane-8-carboxylate

Under nitrogen, to a solution of tert-butyl (1*R*,5*S*)-3-(7-chloro-8-fluoro-2-(methylthio)pyrido[4,3-*d*]pyrimidin-4-yl)-3,8-diazabicyclo[3.2.1]octane-8-carboxylate (500 mg, 1.14 mmol) in 1,4-dioxane (10 mL) and water (2 mL) were added (7-fluoro-8-((triisopropylsilyl)ethynyl)-3-((triisopropylsilyl)oxy)naphthalen-1-yl)boronic acid (Intermediate **A4,** 925 mg, 1.7 mmol), potassium phosphate (482 mg, 2.3 mmol), and Pd(dtbpf)Cl₂ (127 mg, 0.11 mmol). The resulting reaction solution was stirred at 110 °C for 2 hours. After the reaction was complete as monitored by LCMS, the reaction solution was filtered through Celite, and the filtrate was concentrated to dryness. The crude product was purified by FCC (SiO₂, EA/PE = 0-30%) to give the product tert-butyl (1*R*,5*S*)-3-(8-fluoro-7-(7-fluoro-8-((triisopropylsilyl)ethynyl)-3-((triisopropylsilyl)oxy)naphthalen-1-yl)-2-(methylthio)pyrido[4,3-*d*]pyrimidin-4-yl)-3,8-diazabicyclo[3.2.1]octane-8-carboxylate (220 mg, 20% yield) as a yellow solid. MS-ESI [M+H]⁺: 902.0.

### Step F: tert-butyl (1R,5S)-3-(8-fluoro-7-(7-fluoro-8-((triisopropylsilyl)ethynyl)-3-((triisopropylsilyl)oxy)naphthalen-1-yl)-2-(methylsulfonyl)pyrido[4,3-d]pyrimidin-4-yl)-3,8-diazabicyclo[3.2.1]octane-8-carboxylate

At room temperature, to a solution of tert-butyl (1*R*,5*S*)-3-(8-fluoro-7-(7-fluoro-8-((triisopropylsilyl)ethynyl)-3-((triisopropylsilyl)oxy)naphthalen-1-yl)-2-(methylthio)pyrido[4,3-*d*]pyrimidin-4-yl)-3,8-diazabicyclo[3.2.1]octane-8-carboxylate (220 mg, 0.24 mmol) in dichloromethane (5 mL) was added meta-chloroperoxybenzoic acid (123 mg, 0.61 mmol), and the temperature was maintained and the reaction was stirred for 1 hour. After the reaction was complete as monitored by LCMS, 20 mL of saturated sodium bicarbonate solution was added to the reaction solution, and then extracted with dichloromethane. The organic phases were combined, washed with saturated brine, dried over anhydrous sodium sulfate, filtered, and concentrated to dryness to give the crude product tert-butyl (1*R*,5*S*)-3-(8-fluoro-7-(7-fluoro-8-((triisopropylsilyl)ethynyl)-3-((triisopropylsilyl)oxy)naphthalen-1-yl)-2-(methylsulfonyl)pyrido[4,3-*d*]pyrimidin-4-yl)-3,8-diazabicyclo[3.2.1]octane-8-carboxylate **(A7,** 230 mg, 99% yield). MS-ESI [M+H]⁺: 934.4.

### Preparation of Intermediate A8: tert-butyl (1R,5S)-3-(8-fluoro-7-(7-fluoro-8-((triisopropylsilyl)ethynyl)-3-((triisopropylsilyl)oxy)naphthalen-1-yl)-5-methoxy-2-(methylsulfonyl)pyrido[4,3-d]pyrimidin-4-yl)-3,8-diazabicyclo[3.2.1]octane-8-carboxylate

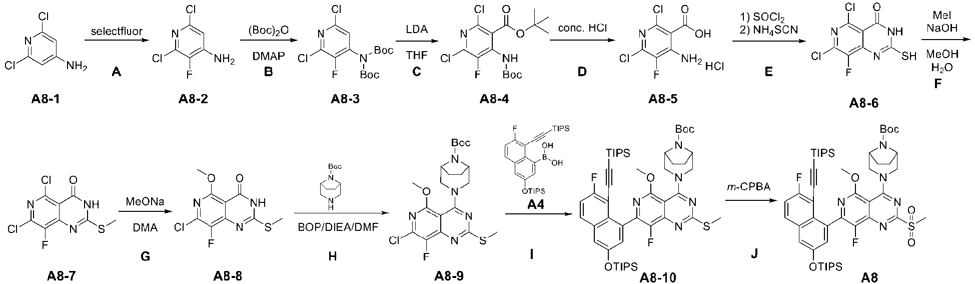

### Step A: 2,6-dichloro-3-fluoropyridin-4-amine

At room temperature, Selectfluor (68 g, 180 mmol) was added to a solution of 2,6-dichloropyridin-4-amine (25 g, 154 mmol) in methanol/water (5/1, 300 mL). The resulting mixture was stirred at 50 °C for 48 hours, concentrated under reduced pressure, diluted with ethyl acetate, washed sequentially with water and saturated brine, dried over anhydrous sodium sulfate, filtered, and concentrated. The resulting crude product was purified by FCC (SiO₂, EA/PE = 0-10%) to give 2,6-dichloro-3-fluoropyridin-4-amine (10 g) as a white solid. MS-ESI [M+H]⁺: 180.9.

### Step B: tert-butyl (tert-butoxycarbonyl)(2,6-dichloro-3-fluoropyridin-4-yl)carbamate

Under stirring at room temperature, 4-dimethylaminopyridine (307 mg, 2.75 mmol) and di-tert-butyl dicarbonate (30 g, 138 mmol) were added to a solution of 2,6-dichloro-3-fluoropyridin-4-amine (10 g, 55 mmol) in tetrahydrofuran (100 mL). The resulting mixture was heated to 60 °C and stirred for 16 hours. The reaction was complete as monitored by TLC, concentrated to obtain a crude product, which was slurried with methanol to give tert-butyl (tert-butoxycarbonyl)(2,6-dichloro-3-fluoropyridin-4-yl)carbamate (16 g) as a white solid. MS-ESI [M+H]⁺: 381.2.

### Step C: tert-butyl 4-((tert-butoxycarbonyl)amino)-2,6-dichloro-5-fluoronicotinate

Under a dry ice-ethanol bath, LDA (2M, 63 mL, 126 mmol) was slowly added to a solution of tert-butyl (tert-butoxycarbonyl)(2,6-dichloro-3-fluoropyridin-4-yl)carbamate (16 g, 42 mmol) in tetrahydrofuran (200 mL). The resulting mixture was stirred at this temperature for 1 hour. The reaction was complete as monitored by TLC. The reaction was quenched by adding a suitable amount of acetic acid, diluted with ethyl acetate, washed with water, dried over anhydrous sodium sulfate, filtered, and concentrated. The resulting crude product was purified by FCC (SiO₂, EA/PE = 0-20%) to give tert-butyl 4-((tert-butoxycarbonyl)amino)-2,6-dichloro-5-fluoronicotinate (13 g).

### Step D: 4-amino-2,6-dichloro-5-fluoronicotinic acid hydrochloride

At room temperature, concentrated hydrochloric acid (30 mL) was added to a solution of tert-butyl 4-((tert-butoxycarbonyl)amino)-2,6-dichloro-5-fluoronicotinate (13 g, 34 mmol) in dioxane (90 mL). The resulting mixture was stirred at room temperature for 3 hours. After the reaction was complete as monitored by LCMS, the mixture was concentrated to give 4-amino-2,6-dichloro-5-fluoronicotinic acid hydrochloride (8 g). MS-ESI [M+H]⁺: 224.9.

### Step E: 5,7-dichloro-8-fluoro-2-mercaptopyrido[4,3-d]pyrimidin-4(3H)-one

A mixed solution of 4-amino-2,6-dichloro-5-fluoronicotinic acid (8 g, 30.8 mmol) and thionyl chloride (200 mL) was stirred at 50 °C for 3 hours. Then it was concentrated, and the residue was dissolved in acetone (50 mL) to give solution 1. At room temperature, a mixed solution of ammonium thiocyanate (7 g, 92 mmol) in acetone (160 mL) was added dropwise to solution 1, and the resulting reaction solution was stirred at room temperature for another 1 hour. After the reaction was complete as monitored by LCMS, the reaction solution was poured into water, filtered, and the filter cake was dried to give 5,7-dichloro-8-fluoro-2-mercaptopyrido[4,3-*d*]pyrimidin-4(3*H*)-one (5 g). MS-ESI [M+H]⁺: 265.9.

### Step F: 5,7-dichloro-8-fluoro-2-(methylthio)pyrido[4,3-d]pyrimidin-4(3H)-one

At room temperature, a mixed solution of 5,7-dichloro-8-fluoro-2-mercaptopyrido[4,3-*d*]pyrimidin-4(3*H*)-one (5 g, 18.8 mmol), methanol (380 mL), aqueous sodium hydroxide (0.1M, 380 mL, 380 mmol), and iodomethane (5.3 g, 380 mmol) was stirred for 2 hours. After the reaction was complete as monitored by LCMS, the reaction solution was poured into 1000 mL of water and acidified to pH 6 with concentrated hydrochloric acid. The solution was filtered, and the filter cake was dried to give 5,7-dichloro-8-fluoro-2-(methylthio)pyrido[4,3-*d*]pyrimidin-4(3*H*)-one (4 g). MS-ESI [M+H]⁺: 279.9.

### Step G: 7-chloro-8-fluoro-5-methoxy-2-(methylthio)pyrido[4,3-d]pyrimidin-4(3H)-one

A mixture of 5,7-dichloro-8-fluoro-2-(methylthio)pyrido[4,3-*d*]pyrimidin-4(3*H*)-one (400 mg, 1.4 mmol), sodium methoxide (0.38 g, 7.5 mmol), DMA (10 mL), and methanol (2 mL) was stirred at 50 °C for 16 hours. After the reaction was complete as monitored by LCMS, the mixture was diluted with water, adjusted to pH 3 with concentrated hydrochloric acid, filtered, and the filter cake was collected and dried to give the product 7-chloro-8-fluoro-5-methoxy-2-(methylthio)pyrido[4,3-*d*]pyrimidin-4(3*H*)-one (250 mg). MS-ESI [M+H]⁺: 276.0.

### Step H: tert-butyl (1R,5S)-3-(7-chloro-8-fluoro-5-methoxy-2-(methylthio)pyrido[4,3-d]pyrimidin-4-yl)-3,8-diazabicyclo[3.2.1]octane-8-carboxylate

A mixed solution of 7-chloro-8-fluoro-5-methoxy-2-(methylthio)pyrido[4,3-d]pyrimidin-4(3*H*)-one (250 mg, 0.91 mmol), tert-butyl (1*R*,5*S*)-3,8-diazabicyclo[3.2.1]octane-8-carboxylate (290 mg, 1.4 mmol), BOP (620 mg, 1.4 mmol), and *N*,*N*-diisopropylethylamine (361 mg, 2.8 mmol) in DMF (5 mL) was stirred at 45 °C for 3 hours. After the reaction was complete as monitored by LCMS, the reaction solution was poured into water, filtered, and the filter cake was collected and dried to give tert-butyl (1*R*,5*S*)-3-(7-chloro-8-fluoro-5-methoxy-2-(methylthio)pyrido[4,3-*d*]pyrimidin-4-yl)-3,8-diazabicyclo[3.2.1]octane-8-carboxylate (300 mg) as a yellow solid. MS-ESI [M+H]⁺: 470.3.

### Step I: tert-butyl (1R,5S)-3-(8-fluoro-7-(7-fluoro-8-((triisopropylsilyl)ethynyl)-3-((triisopropylsilyl)oxy)naphthalen-1-yl)-5-methoxy-2-(methylthio)pyrido[4,3-d]pyrimidin-4-yl)-3,8-diazabicyclo[3.2.1]octane-8-carboxylate

Under nitrogen protection, a solution of tert-butyl (1*R*,5*S*)-3-(7-chloro-8-fluoro-5-methoxy-2-(methylthio)pyrido[4,3-*d*]pyrimidin-4-yl)-3,8-diazabicyclo[3.2.1]octane-8-carboxylate (500 mg, 1.1 mmol), (7-fluoro-8-((triisopropylsilyl)ethynyl)-3-((triisopropylsilyl)oxy)naphthalen-1-yl)boronic acid (Intermediate **A4,** 1.2 g, 2.2 mmol), potassium phosphate (678 mg, 3.2 mmol), and tetrakis(triphenylphosphine)palladium (60 mg, 0.05 mmol) in 1,4-dioxane/water (1/1, 5 mL) was heated to 100 °C and stirred for 2 hours. After the reaction was complete as monitored by LCMS, the mixture was concentrated under reduced pressure, and the crude product was purified by FCC (SiO₂, EA/PE = 0-50%) to give tert-butyl (1*R*,5*S*)-3-(8-fluoro-7-(7-fluoro-8-((triisopropylsilyl)ethynyl)-3-((triisopropylsilyl)oxy)naphthalen-1-yl)-5-methoxy-2-(methylthio)pyrido[4,3-*d*]pyrimidin-4-yl)-3,8-diazabicyclo[3.2.1]octane-8-carboxylate (260 mg, 28% yield) as a white solid. MS-ESI [M+H-156]⁺: 776.4.

### Step J: (1R,5S)-3-(8-fluoro-7-(7-fluoro-8-((triisopropylsilyl)ethynyl)-3-((triisopropylsilyl)oxy)naphthalen-1-yl)-5-methoxy-2-(methylsulfonyl)pyrido[4,3-d]pyrimidin-4-yl)-3,8-diazabicyclo[3.2.1]octane-8-carboxylate

A solution of (1*R*,5*S*)-3-(8-fluoro-7-(7-fluoro-8-((triisopropylsilyl)ethynyl)-3-((triisopropylsilyl)oxy)naphthalen-1-yl)-5-methoxy-2-(methylthio)pyrido[4,3-*d*]pyrimidin-4-yl)-3,8-diazabicyclo[3.2.1]octane-8-carboxylate (100 mg, 0.11 mmol) and meta-chloroperoxybenzoic acid (85%, 36 mg, 0.22 mmol) in dichloromethane (3 mL) was stirred at room temperature for 1 hour. After the reaction was complete as monitored by LCMS, the reaction solution was diluted with ethyl acetate (10 mL) and washed sequentially with saturated aqueous sodium bicarbonate and saturated brine. The resulting organic phase was concentrated and dried to give the product tert-butyl (1*R*,5*S*)-3-(8-fluoro-7-(7-fluoro-8-((triisopropylsilyl)ethynyl)-3-((triisopropylsilyl)oxy)naphthalen-1-yl)-5-methoxy-2-(methylsulfonyl)pyrido[4,3-*d*]pyrimidin-4-yl)-3,8-diazabicyclo[3.2.1]octane-8-carboxylate **(A8,** 80 mg, 77% yield) as a white solid. MS-ESI [M+H-156]⁺: 808.3.

### Preparation of Intermediate A9: tert-butyl (1R,5S)-3-(2,7-dichloro-8-fluoro-5-((triisopropylsilyl)ethynyl)pyrido[4,3-d]pyrimidin-4-yl)-3,8-diazabicyclo[3.2.1]octane-8-carboxylate

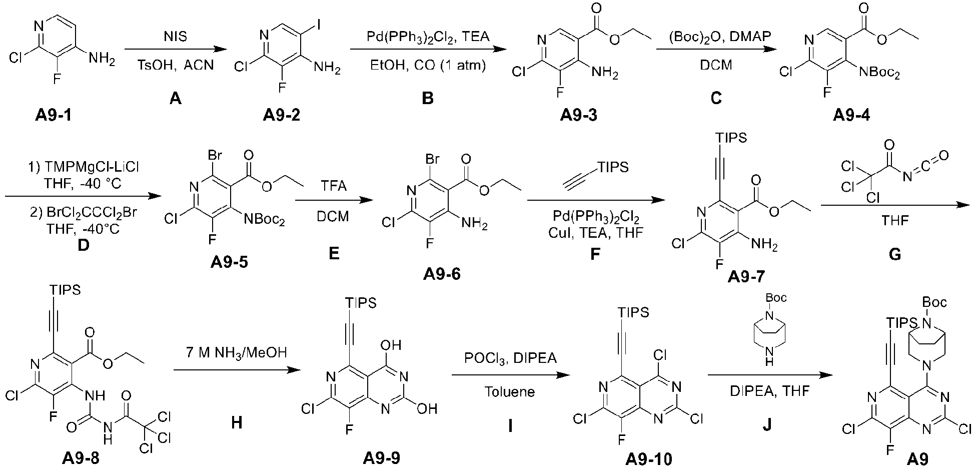

### Step A: 2-chloro-3-fluoro-5-iodopyridin-4-amine

At room temperature, 2-chloro-3-fluoro-4-aminopyridine (25.0 g, 171 mmol) was dissolved in 250 mL of acetonitrile. Then NIS (35.4 g, 205 mmol) and p-toluenesulfonic acid monohydrate (3.24 g, 17.1 mmol) were added to the above system. The resulting mixture was heated to 70 °C and stirred overnight. After the reaction was complete as monitored by LCMS, cooled to room temperature, the reaction solution was poured into water (500 mL) to quench, and extracted with ethyl acetate (3 × 800 mL). The organic phases were combined, washed with saturated sodium bicarbonate solution, saturated sodium thiosulfate solution, and brine, dried over anhydrous sodium sulfate, filtered, and concentrated. The resulting crude product was purified by FCC (SiO₂, EA/PE = 0-50%) to give 2-chloro-3-fluoro-5-iodopyridin-4-amine (42 g, 90% yield) as a white solid. ¹H NMR (400 MHz, DMSO- *d₆*) *δ 8.10* (s, 1H), 6.69 (s, 2H). ¹⁹F NMR (376 MHz, DMSO-*d₆*) δ -138.90. MS-ESI [M+H]⁺: 272.9.

### Step B: ethyl 4-amino-6-chloro-5-fluoronicotinate

At room temperature, 2-chloro-3-fluoro-5-iodopyridin-4-amine (40 g, 147 mmol) was dissolved in 400 mL of anhydrous ethanol. The system was replaced with carbon monoxide repeatedly three times. Then Pd(PPh₃)₂Cl₂ (10.3 g, 14.7 mmol) and triethylamine (95.0 g, 735 mmol) were added to the reaction flask, and the system was replaced with carbon monoxide three times. The resulting mixture was heated to 80 °C and stirred overnight under a carbon monoxide atmosphere. After the reaction was complete as monitored by LCMS, cooled to room temperature, the reaction solution was filtered through Celite. The filtrate was concentrated to dryness, and the resulting crude product was purified by FCC (SiO₂, EA/PE = 0-50%) to give ethyl 4-amino-6-chloro-5-fluoronicotinate (30 g, 93% yield) as a white solid. MS-ESI [M+H]⁺: 219.0.

### Step C: ethyl 4-(bis(tert-butoxycarbonyl)amino)-6-chloro-5-fluoronicotinate

Under stirring at room temperature, (Boc)₂O (65.9 g, 302 mmol) was added dropwise to a solution of ethyl 4-amino-6-chloro-5-fluoronicotinate (30.0 g, 137 mmol) and DMAP (3.4 g, 27.5 mmol) in anhydrous dichloromethane (600 mL). After the addition, the mixture was heated to 45 °C and reacted overnight. Imidazole (9.33 g, 137 mmol) was added, stirred for half an hour, then washed with saturated ammonium chloride solution (3 × 300 mL). The layers were separated, and the organic phase was washed with saturated brine (2 × 300 mL), dried over anhydrous sodium sulfate, filtered, and concentrated to dryness to give ethyl 4-(bis(tert-butoxycarbonyl)amino)-6-chloro-5-fluoronicotinate (45.9 g, 80% yield) as a yellow solid. MS-ESI [M+H]⁺: 419.1.

### Step D: ethyl 4-(bis(tert-butoxycarbonyl)amino)-2-bromo-6-chloro-5-fluoronicotinate

A solution of ethyl 4-(bis(tert-butoxycarbonyl)amino)-6-chloro-5-fluoronicotinate (45.0 g, 107 mmol) in anhydrous THF (450 mL) was cooled to -40 °C using a dry ice-acetonitrile bath. At this temperature, a solution of TMPMgCl-LiCl in tetrahydrofuran (1M, 161 mL, 161 mmol) was added dropwise with stirring. After the addition, stirring continued at this temperature for 4 hours, then a solution of dibromotetrachloroethane (42.0 g, 129 mmol) in tetrahydrofuran (100 mL) was added dropwise. Stirring continued at -40 °C for 4 hours. The reaction was quenched by adding saturated aqueous ammonium chloride (500 mL), and extracted with ethyl acetate (3 × 500 mL). The organic phases were combined, washed with saturated brine, dried over anhydrous sodium sulfate, filtered, and concentrated. The crude product was purified by FCC (SiO₂, EA/DCM = 0-50%) to give ethyl 4-(bis(tert-butoxycarbonyl)amino)-2-bromo-6-chloro-5-fluoronicotinate (17.0 g, 32% yield) as a white solid. MS-ESI [M+H]⁺: 497.0.

### Step E: ethyl 4-amino-2-bromo-6-chloro-5-fluoronicotinate

Under stirring at room temperature, trifluoroacetic acid (50 mL) was added in one portion to a solution of ethyl 4-(bis(tert-butoxycarbonyl)amino)-2-bromo-6-chloro-5-fluoronicotinate (9.5 g, 19.1 mmol) in dichloromethane (50 mL). The mixture was stirred at room temperature for 2 hours, then concentrated to dryness. The residue was neutralized with saturated sodium bicarbonate solution (200 mL) and extracted with ethyl acetate (3 × 200 mL). The organic phases were combined, washed with saturated brine, dried over anhydrous sodium sulfate, filtered, and concentrated to give ethyl 4-amino-2-bromo-6-chloro-5-fluoronicotinate (5.6 g, 99% yield) as a white solid. MS-ESI [M+H]⁺: 296.9.

### Step F: ethyl 4-amino-6-chloro-5-fluoro-2-((triisopropylsilyl)ethynyl)nicotinate

At room temperature, ethyl 4-amino-2-bromo-6-chloro-5-fluoronicotinate (5.60 g, 18.8 mmol), Pd(PPh₃)₂Cl₂ (1.32 g, 1.88 mmol), and copper iodide (717 mg, 3.76 mmol) were dissolved in anhydrous tetrahydrofuran (150 mL). The atmosphere was replaced with nitrogen three times. Triisopropylsilyl acetylene (4.46 g, 24.5 mmol) and triethylamine (5.71 g, 56.5 mmol) were added, and the atmosphere was replaced with nitrogen three more times. The resulting mixture was heated to 45 °C and stirred for 2 hours. After the reaction was complete as monitored by LCMS, cooled to room temperature, the reaction solution was filtered through Celite. The filtrate was concentrated to dryness, and the crude product was purified by FCC (SiO₂, EA/PE = 0-10%) to give ethyl 4-amino-6-chloro-5-fluoro-2-((triisopropylsilyl)ethynyl)nicotinate (7 g, 93% yield) as a white solid. MS-ESI [M+H]⁺: 399.1.

### Step G: ethyl 6-chloro-5-fluoro-4-(3-(2,2,2-trichloroacetyl)ureido)-2-((triisopropylsilyl)ethynyl)nicotinate

Under stirring at room temperature, 2,2,2-trichloroacetyl isocyanate (3.97 g, 21.1 mmol) was added to a solution of ethyl 4-amino-6-chloro-5-fluoro-2-((triisopropylsilyl)ethynyl)nicotinate (7.00 g, 17.5 mmol) in anhydrous tetrahydrofuran (150 mL). The resulting mixture was stirred at room temperature for 1 hour, and then concentrated to dryness to give ethyl 6-chloro-5-fluoro-4-(3-(2,2,2-trichloroacetyl)ureido)-2-((triisopropylsilyl)ethynyl)nicotinate (11 g, crude) as a white solid, which was used directly in the next step without purification. MS-ESI [M+H]⁺: 586.0.

### Step H: 7-chloro-8-fluoro-5-((triisopropylsilyl)ethynyl)pyrido[4,3-d]pyrimidine-2,4-diol

At room temperature, ethyl 6-chloro-5-fluoro-4-(3-(2,2,2-trichloroacetyl)ureido)-2-((triisopropylsilyl)ethynyl)nicotinate (11 g, crude) was dissolved in ammonia in methanol solution (7M, 50 mL, 350 mmol). The resulting mixture was stirred at room temperature for 2 hours. After concentration, the crude product was slurried with PE/EA (10/1, 110 mL) and filtered to give 7-chloro-8-fluoro-5-((triisopropylsilyl)ethynyl)pyrido[4,3-*d*]pyrimidine-2,4-diol (5.8 g, 84% yield) as a white solid. MS-ESI [M+H]⁺: 396.1.

### Step I: 2,4,7-trichloro-8-fluoro-5-((triisopropylsilyl)ethynyl)pyrido[4,3-d]pyrimidine

At room temperature, phosphorus oxychloride (1.55 g, 10.1 mmol) and triethylamine (1.31 g, 10.1 mmol) were added to a solution of 7-chloro-8-fluoro-5-((triisopropylsilyl)ethynyl)pyrido[4,3-*d*]pyrimidine-2,4-diol (1.00 g, 2.53 mmol) in anhydrous toluene (20 mL). The resulting mixture was heated to 100 °C and stirred overnight, then concentrated to give crude 2,4,7-trichloro-8-fluoro-5-((triisopropylsilyl)ethynyl)pyrido[4,3-*d*]pyrimidine, which was used directly in the next step.

### Step J: tert-butyl (1R,5S)-3-(2,7-dichloro-8-fluoro-5-((triisopropylsilyl)ethynyl)pyrido[4,3-d]pyrimidin-4-yl)-3,8-diazabicyclo[3.2.1]octane-8-carboxylate

Under nitrogen protection, a solution of 2,4,7-trichloro-8-fluoro-5-((triisopropylsilyl)ethynyl)pyrido[4,3-*d*]pyrimidine (crude from step I) in anhydrous dichloromethane (20 mL) was cooled to -40 °C. DIPEA (976 mg, 7.56 mmol) was added, followed by a solution of tert-butyl 3,8-diazabicyclo[3.2.1]octane-8-carboxylate (534 mg, 2.52 mmol) in dichloromethane (2 mL). The resulting mixture was stirred at -40 °C for 1 hour. The reaction was quenched by adding saturated ammonium chloride solution (30 mL), and extracted with dichloromethane (3 × 30 mL). The organic phases were combined, dried, and concentrated. The crude product was purified by FCC (SiO₂, EA/PE = 0-10%) to give tert-butyl (1*R*,5*S*)-3-(2,7-dichloro-8-fluoro-5-((triisopropylsilyl)ethynyl)pyrido[4,3-*d*]pyrimidin-4-yl)-3,8-diazabicyclo[3.2.1]octane-8-carboxylate **(A9,** 960 mg, 63% yield over two steps) as a white solid. MS-ESI [M+H]⁺: 608.3.

### Preparation of Intermediate A10: tert-butyl (1R,5S)-3-(7-(2-((tert-butoxycarbonyl)amino)-3-cyanobenzo[b]thiophen-4-yl)-6-chloro-2,8-difluoroquinazolin-4-yl)-3,8-diazabicyclo[3.2.1]octane-8-carboxylate

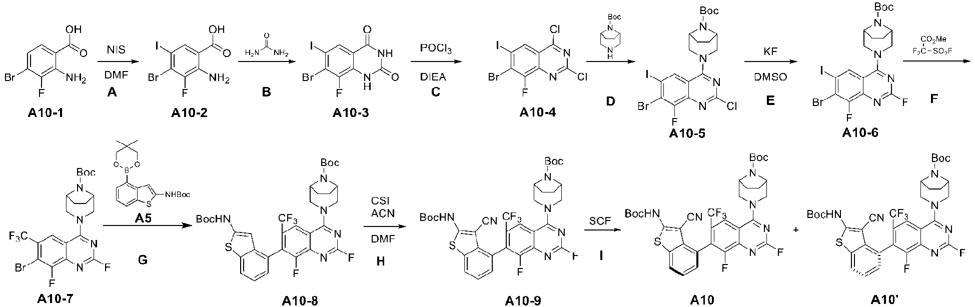

### Step A: 2-amino-4-bromo-3-fluoro-5-iodobenzoic acid

At room temperature, NIS (50.6 g, 225 mmol) was added to a mixed solution of 2-amino-4-bromo-3-fluorobenzoic acid (50 g, 215 mmol) and DMF (500 mL). The resulting mixture was stirred at 80 °C for 3 hours. After the reaction was complete as monitored by LCMS, the reaction solution was cooled, poured into water (2 L), and extracted with ethyl acetate (4 L). The organic layer was dried over anhydrous sodium sulfate, filtered, and concentrated to give 2-amino-4-bromo-3-fluoro-5-iodobenzoic acid (50 g, 65% yield) as a yellow solid. MS-ESI [M+H]⁺: 360.2.

### Step B: 7-bromo-8-fluoro-6-iodoquinazoline-2,4-(1H,3H)-dione

A mixture of 2-amino-4-bromo-3-fluoro-5-iodobenzoic acid (50 g, 139 mmol) and urea (168 g) was stirred at 200 °C for 2 hours. After the reaction was complete, the system was cooled to room temperature, water was added for slurrying, and the solid was collected by filtration and dried to give 7-bromo-8-fluoro-6-iodoquinazoline-2,4-(1*H*,3*H*)-dione (48 g, 90% yield) as a yellow solid. MS-ESI [M+H]⁺: 384.8.

### Step C: 7-bromo-2,4-dichloro-8-fluoro-6-iodoquinazoline

At room temperature, DIEA (40 mL) was slowly added to a mixed solution of 7-bromo-8-fluoro-6-iodoquinazoline-2,4-(1*H*,3*H*)-dione (20 g, 52 mmol) and phosphorus oxychloride (160 mL). The resulting mixture was stirred at 100 °C for 16 hours. After the reaction was complete as monitored by TLC, the reaction solution was concentrated, slowly poured into water, and the filter cake was collected and dried to give 7-bromo-2,4-dichloro-8-fluoro-6-iodoquinazoline (18.5 g, 85% yield) as a yellow solid.

### Step D: tert-butyl (1R,5S)-3-(7-bromo-2-chloro-8-fluoro-6-iodoquinazolin-4-yl)-3,8-diazabicyclo[3.2.1]octane-8-carboxylate

A mixture of 7-bromo-2,4-dichloro-8-fluoro-6-iodoquinazoline (18.5 g, 44 mmol), tert-butyl (1*R*,5*S*)-3,8-diazabicyclo[3.2.1]octane-8-carboxylate (9.3 g, 44 mmol), DIEA (17 g, 132 mmol), and tetrahydrofuran (200 mL) was stirred at room temperature for 1 hour. After the reaction was complete as monitored by LCMS, the reaction solution was poured into water (2 L), filtered, and the solid was collected and dried to give tert-butyl (1*R*,5*S*)-3-(7-bromo-2-chloro-8-fluoro-6-iodoquinazolin-4-yl)-3,8-diazabicyclo[3.2.1]octane-8-carboxylate (17.6 g, 67% yield) as a yellow solid. MS-ESI [M+H]⁺: 596.6.

### Step E: tert-butyl (1R,5S)-3-(7-bromo-2,8-difluoro-6-iodoquinazolin-4-yl)-3,8-diazabicyclo[3.2.1]octane-8-carboxylate

A mixture of tert-butyl (1*R*,5*S*)-3-(7-bromo-2-chloro-8-fluoro-6-iodoquinazolin-4-yl)-3,8-diazabicyclo[3.2.1]octane-8-carboxylate (10 g, 16.8 mmol), potassium fluoride (8 g, 138 mmol), and dimethyl sulfoxide (100 mL) was stirred at 110 °C for 16 hours. After the reaction was complete as monitored by LCMS, the reaction solution was poured into water (2 L), filtered, and the solid was collected and dried to give tert-butyl (1*R*,5*S*)-3-(7-bromo-2,8-difluoro-6-iodoquinazolin-4-yl)-3,8-diazabicyclo[3.2.1]octane-8-carboxylate (9 g, 92% yield) as a yellow solid. MS-ESI [M+H]⁺: 583.3.

### Step F: tert-butyl (1R,5S)-3-(7-bromo-2,8-difluoro-6-(trifluoromethyl)quinazolin-4-yl)-3,8-diazabicyclo[3.2.1]octane-8-carboxylate

A mixture of tert-butyl (1*R*,5*S*)-3-(7-bromo-2,8-difluoro-6-iodoquinazolin-4-yl)-3,8-diazabicyclo[3.2.1]octane-8-carboxylate (1 g, 1.7 mmol), methyl fluorosulfonyldifluoroacetate (660 mg, 0.44 mL, 3.4 mmol), copper iodide (1 g, 5.2 mmol), hexamethylphosphoramide (2 mL), and DMF (10 mL) was heated to 110 °C with microwave irradiation and stirred for 1 hour. After the reaction was complete, the reaction solution was filtered, extracted with ethyl acetate and water, and the organic phase was dried over anhydrous sodium sulfate, filtered, and concentrated. The crude product was purified by FCC (SiO₂, EA/PE = 0-20%) to give tert-butyl (1*R*,5*S*)-3-(7-bromo-2,8-difluoro-6-(trifluoromethyl)quinazolin-4-yl)-3,8-diazabicyclo[3.2.1]octane-8-carboxylate (400 mg, 45% yield) as a yellow solid. MS-ESI [M+H]⁺: 523.6.

### Step G: tert-butyl (1R,5S)-3-(7-(2-((tert-butoxycarbonyl)amino)benzo[b]thiophen-4-yl)-2,8-difluoro-6-(trifluoromethyl)quinazolin-4-yl)-3,8-diazabicyclo[3.2.1]octane-8-carboxylate

A mixture of tert-butyl (1*R*,5*S*)-3-(7-bromo-2,8-difluoro-6-(trifluoromethyl)quinazolin-4-yl)-3,8-diazabicyclo[3.2.1]octane-8-carboxylate (1.3 g, 2.5 mmol), tert-butyl (4-(4,4,5,5-tetramethyl-1,3,2-dioxaborolan-2-yl)benzo[*b*]thiophen-2-yl)carbamate (1.4 g, 3.75 mmol), Pd-118 (320 mg, 0.5 mmol), potassium phosphate (1.6 g, 7.5 mmol), and 1,4-dioxane/water (4/1, 25 mL) was stirred at 100 °C for 16 hours. After the reaction was complete as monitored by LCMS, the reaction solution was concentrated and purified by FCC (SiO₂, EA/PE = 0-30%) to give tert-butyl (1*R*,5*S*)-3-(7-(2-((tert-butoxycarbonyl)amino)benzo[*b*]thiophen-4-yl)-2,8-difluoro-6-(trifluoromethyl)quinazolin-4-yl)-3,8-diazabicyclo[3.2.1]octane-8-carboxylate (580 mg, 34% yield) as a yellow solid. MS-ESI [M+H]⁺: 692.6.

### Step H: tert-butyl (1R,5S)-3-(7-(2-((tert-butoxycarbonyl)amino)-3-cyanobenzo[b]thiophen-4-yl)-2,8-difluoro-6-(trifluoromethyl)quinazolin-4-yl)-3,8-diazabicyclo[3.2.1]octane-8-carboxylate

At 0 °C, CSI (262 mg, 0.16 mL, 1.9 mol) was added to a solution of tert-butyl (1*R*,5*S*)-3-(7-(2-((tert-butoxycarbonyl)amino)benzo[*b*]thiophen-4-yl)-2,8-difluoro-6-(trifluoromethyl)quinazolin-4-yl)-3,8-diazabicyclo[3.2.1]octane-8-carboxylate (430 mg, 0.62 mmol) in acetonitrile (5 mL). The reaction solution was stirred at 0 °C for 2 hours, then DMF (2.5 mL) was slowly added to the reaction system. The resulting reaction solution was stirred at 0 °C for 1 hour. After the reaction was complete as monitored by LCMS. The reaction was quenched by adding saturated ammonium chloride solution (10 mL), and extracted with ethyl acetate (20 mL). The organic phase was dried over anhydrous sodium sulfate, filtered, and concentrated. The crude product was purified by FCC (SiO₂, EA/PE = 0-50%) to give tert-butyl (1*R*,5*S*)-3-(7-(2-((tert-butoxycarbonyl)amino)-3-cyanobenzo[*b*]thiophen-4-yl)-2,8-difluoro-6-(trifluoromethyl)quinazolin-4-yl)-3,8-diazabicyclo[3.2.1]octane-8-carboxylate (280 mg, 63% yield) as a yellow solid. MS-ESI [M+H]⁺: 717.3.

**Step I:** Intermediate **A10-9** (1.2 g) was resolved by SFC (Waters SFC 150, column: REGIS(S,S)WHELK-Ol, 250 * 25 mm, 10 µm; mobile phase: CO₂/MeOH (0.1% 7.0M NH₃/MeOH) = 80/20; flow rate: 120 mL/min) to give the first eluted isomer 1, intermediate **A10** (0.48 g, shorter retention time). Chiral analysis method SFC-3, Rt = 1.923 min. MS-ESI [M+H]⁺: 717.3; followed by the later eluted isomer 2, intermediate **A10'** (0.49 g, longer retention time). Chiral analysis method SFC-3, Rt = 2.523 min. MS-ESI [M+H]⁺: 717.3.

### Preparation of Intermediate A11: 4-(4-((1R,5S)-3,8-diazabicyclo[3.2.1]octan-3-yl)-2-(((3S,4S)-4-(difluoromethyl)-1,3-dimethylpiperidin-3-yl)methoxy)-6,8-difluoroquinazolin-7-yl)-5-((triisopropylsilyl)ethynyl)-6-fluoro-2-((triisopropylsilyl)oxy)naphthalene

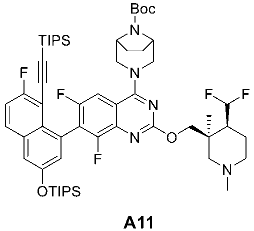

Intermediate **A11** was prepared following the synthetic method for intermediate **P2-1** of payload molecule **P2** below, using intermediate **A6** (tert-butyl (1*R*,5*S*)-3-(2,6,8-trifluoro-7-(7-fluoro-8-((triisopropylsilyl)ethynyl)-3-((triisopropylsilyl)oxy)naphthalen-1-yl)quinazolin-4-yl)-3,8-diazabicyclo[3.2.1]octane-8-carboxylate) instead of intermediate A7 (tert-butyl (1*R*,5*S*)-3-(8-fluoro-7-(7-fluoro-8-((triisopropylsilyl)ethynyl)-3-((triisopropylsilyl)oxy)naphthalen-1-yl)-2-(methylsulfonyl)pyrido[4,3-*d*]pyrimidin-4-yl)-3,8-diazabicyclo[3.2.1]octane-8-carboxylate), and using intermediate **A2** ((3*S*,4*S*)-4-(difluoromethyl)-1,3-dimethylpiperidin-3-yl)methanol instead of intermediate **A1** (*S*,*E*)-(4-(fluoromethylene)-1,3-dimethylpiperidin-3-yl)methanol.

### Preparation of Intermediate A12: 4-(4-((1R,5S)-3,8-diazabicyclo[3.2.1]-8-tert-butyl carboxylate-octan-3-yl)-2-(((3S,4S)-4-(difluoromethyl)-1,3-dimethylpiperidin-3-yl)methoxy)-8-fluoro-5-methoxypyrido[4,3-d]pyrimidin-7-yl)-5-(triisopropylsilyl)ethynyl-6-fluoro-2-(triisopropylsilyl)oxy)naphthalene

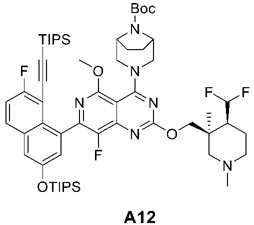

Intermediate **A12** was prepared following the synthetic method for intermediate **P2-1** of payload molecule **P2** below, using intermediate **A8** (tert-butyl (1*R*,5*S*)-3-(8-fluoro-7-(7-fluoro-8-((triisopropylsilyl)ethynyl)-3-((triisopropylsilyl)oxy)naphthalen-1-yl)-5-methoxy-2-(methylsulfonyl)pyrido[4,3-*d*]pyrimidin-4-yl)-3,8-diazabicyclo[3.2.1]octane-8-carboxylate) instead of intermediate **A7** (tert-butyl (1*R*,5*S*)-3-(8-fluoro-7-(7-fluoro-8-((triisopropylsilyl)ethynyl)-3-((triisopropylsilyl)oxy)naphthalen-1-yl)-2-(methylsulfonyl)pyrido[4,3-*d*]pyrimidin-4-yl)-3,8-diazabicyclo[3.2.1]octane-8-carboxylate), and using intermediate **A2** ((3*S*,4*S*)-4-(difluoromethyl)-1,3-dimethylpiperidin-3-yl)methanol instead of intermediate **A1** (*S*,*E*)-(4-(fluoromethylene)-1,3-dimethylpiperidin-3-yl)methanol.

### Preparation of Intermediate A13: [3-(7'-{[4-(difluoromethyl)-1-methyl-3-methylpiperidin-3-yl]methoxy}-8-ethynyl-1',7-difluoro-3-hydroxy-4'-methoxy-3',6',8'-triaza-1,2'-binaphthalen-5'-yl)-3,8-diazabicyclo[3.2.1]octan-8-yl]{O-[(methylamino)methyl]phenyl}methanone hydrochloride

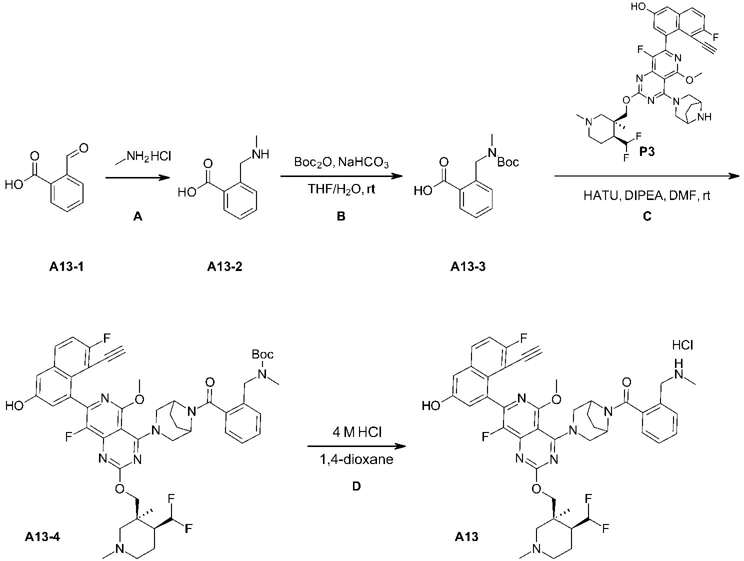

### Step A: o-[(methylamino)methyl]benzoic acid

At room temperature, DIPEA (4.3 g, 33.3 mmol) was added to a solution of methylamine hydrochloride (4.5 g, 66.6 mmol) in methanol (25 mL), followed by a solution of compound **A13-1** (5 g, 33.3 mmol) in methanol (25 mL). The reaction solution was stirred at room temperature for 1 minute until clear, then NaCNBH₃ (2.1 g, 33.3 mmol) was added. The reaction solution was stirred at room temperature for 5 minutes. A large amount of solid formed. The reaction solution was filtered, and the filtrate was concentrated, slurried with acetonitrile, and filtered to give **A13-2** (3.76 g, 68.3% yield) as a white solid. ¹H NMR (400 MHz, DMSO-*d₆*) δ 10.95 (s, 2H), 7.83-7.76 (m, 1H), 7.41-7.27 (m, 3H), 4.00 (s, 2H), 2.45 (s, 3H). MS-ESI [M+H]⁺: 166.0.

### Step B: O-{ [(tert-butyl)(methyl)(oxycarbonylamino)]methyl} benzoic acid

At room temperature, NaHCO₃ (4.58 g, 54.5 mmol) and di-tert-butyl dicarbonate (5.95 g, 27.2 mmol) were added to a solution of intermediate **A13-2** (3 g, 18.2 mmol) in THF (30 mL) and H₂O (30 mL). The reaction solution was stirred at room temperature for 12 hours. The reaction solution was diluted with H₂O (100 mL) and extracted with EA (3 × 50 mL). The organic phases were combined, dried over anhydrous Na₂SO₄ and concentrated under reduced pressure to give **A13-3** (4.2 g, 87.2% yield) as a white solid. MS-ESI [M+Na]⁺: 288.0.

### Step C: 2-(O-{[3-(7'-{[(3S,4S)-4-(difluoromethyl)-1-methyl-3-methylpiperidin-3-yl]methoxy}-8-ethynyl-1',7-difluoro-3-hydroxy-4'-methoxy-3',6',8'-triaza-1,2'-binaphthalen-5'-yl)-3,8-diazabicyclo[3.2.1]octan-8-yl]carbonyl}phenyl)methyl 2-methyl 2-methyl-2-propane carbamate

At room temperature, HATU (57 mg, 0.15 mmol) and DIPEA (39 mg, 0.3 mmol) were added to a solution of intermediate **A13-3** (39.9 mg, 0.15 mmol) in acetonitrile (5 mL), followed by intermediate **P3** (100 mg, 0.15 mmol). The reaction solution was stirred at room temperature for 1 hour, purified by FCC (DCM/MeOH = 0-15%) to give **A13-4** (100 mg, 72.9% yield) as a yellow solid. MS-ESI [M+H]⁺: 912.1.

### Step D: [3-(7'-1[4-(difluoromethyl)-1-methyl-3-methylpiperidin-3-yl]methoxy}-8-ethynyl-1',7-difluoro-3-hydroxy-4'-methoxy-3',6',8'-triaza-1,2'-binaphthalen-5'-yl)-3,8-diazabicyclo[3.2.1]octan-8-yl]{O-[(methylamino)methyl]phenyl}methanone hydrochloride

At room temperature, HCl-Dioxane (4M, 4 mL) was added to a solution of intermediate **A13-4** (160 mg, 0.18 mmol) in DCM (2 mL) and MeOH (2 mL). The reaction solution was stirred at room temperature for 5 minutes. The solvent was removed under reduced pressure to **A13** (150 mg, 100% yield) as a yellow solid. MS-ESI [M+H]⁺: 812.2.

### Preparation of Intermediate A14: (1S,5S)-3-(7'-{[(3R,4R)-4-(difluoromethylethyl)-1-methyl-3-methylmethylmethyl-3-methyl-3-methoxymethoxy]-1',7-difluoro-4'-methoxymethylmethoxy-3"-methoxy-4"-formylmethoxy-3"carbonyl chloride

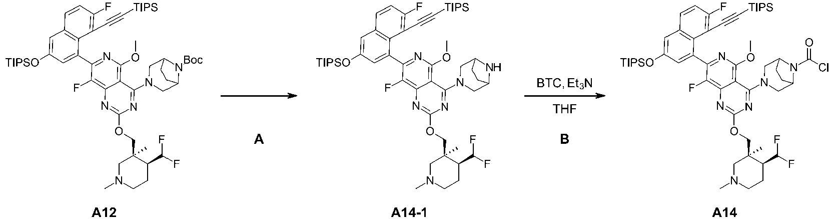

**Step A:** At room temperature, hydrogen chloride in 1,4-dioxane solution (4M, 60 mL) was added to **A12** (6.8 g, 6.31 mmol) in dichloromethane (40 mL). The mixture was stirred at room temperature for 2 hours. After the reaction was complete, the solvent was evaporated. Dichloromethane (300 mL) was added, and the mixture was washed with saturated sodium bicarbonate solution (3 × 50 mL). The organic phase was dried over anhydrous sodium sulfate, filtered, and concentrated to give **B14-1** (6.0 g, 97% yield) as a yellow solid. MS-ESI [M+H]⁺: 977.7.

**Step B:** Under nitrogen protection and ice-water bath, triphosgene (610 mg, 2.05 mmol) and triethylamine (830 mg, 8.22 mmol) were added to a solution of **B14-1** (2.0 g, 2.05 mmol) in tetrahydrofuran (30 mL). The reaction system was warmed to room temperature and stirred for 2 hours. After the reaction was complete, dilute hydrochloric acid (0.1M, 200 mL) was added, and the mixture was extracted with dichloromethane (3 × 100 mL). The organic phases were combined, dried over anhydrous sodium sulfate and filtered. The filtrate was concentrated to give **B14** (2.0 g, 94% yield) as a yellow solid. MS-ESI [M+H]⁺: 1039.5; 1041.5.

### Preparation of Intermediate A15: (S,E)-(1-ethyl-4-(fluoromethylene)-3-methylpiperidin-3-yl)methanol

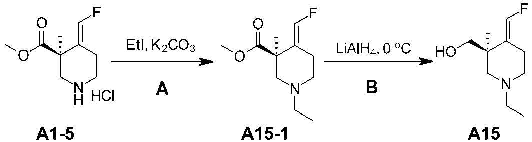

### Step A: (S,E)-methyl 1-ethyl-4-(fluoromethylene)-3-methylpiperidine-3-carboxylate

At room temperature, potassium carbonate (370.73 mg, 2.68 mmol) and iodoethane (278.92 mg, 1.79 mmol) were sequentially added to a solution of (S,E)-methyl 4-(fluoromethylene)-3-methylpiperidine-3-carboxylate hydrochloride (**A1-5**, 200 mg, 0.894 mmol) in acetonitrile (6 mL). The resulting mixture was heated to 90 °C and stirred overnight. After the reaction was complete as monitored by LCMS, the reaction solution was filtered through Celite, and the filter cake was washed twice with acetonitrile. The filtrate was collected and concentrated to dryness to give a crude product. The crude product was purified by FCC (SiO₂, EA/PE = 0-90%) to give (*S*,*E*)-methyl 1-ethyl-4-(fluoromethylene)-3-methylpiperidine-3-carboxylate (100 mg, 69% yield) as a colorless oil. MS-ESI [M+H]⁺: 216.1.

### Step B: (S,E)-(1-ethyl-4-(fluoromethylene)-3-methylpiperidin-3-yl)methanol

Under ice-bath cooling, 1M LiAlH₄-THF solution (0.7 mL, 0.7 mmol) was added dropwise to a solution of (*S*,*E*)-methyl 1-ethyl-4-(fluoromethylene)-3-methylpiperidine-3-carboxylate **(A15-1,** 100 mg, 0.46 mmol) in anhydrous tetrahydrofuran (5 mL). The resulting mixture was stirred at room temperature for 20 minutes. After the reaction was complete as monitored by LCMS, the reaction was quenched with sodium sulfate decahydrate until no bubbles were generated. About 5 g of anhydrous sodium sulfate was added to dry. The reaction solution was filtered through Celite, and the filter cake was washed three times with anhydrous tetrahydrofuran. The filtrate was collected and concentrated to dryness to give (*S*,*E*)-(1-ethyl-4-(fluoromethylene)-3-methylpiperidin-3-yl)methanol (80 mg, 92% yield) as a colorless oil. MS-ESI [M+H]⁺: 188.1.

### Preparation of Intermediate A16: ((3S,4S)-4-(difluoromethyl)-1-ethyl-3-methylpiperidin-3-yl)methanol

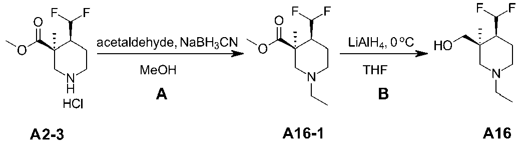

### Step A: (3S,4S)-methyl 4-(difluoromethyl)-1-ethyl-3-methylpiperidine-3-carboxylate

At room temperature, (3*S*,4*S*)-methyl 4-(difluoromethyl)-3-methylpiperidine-3-carboxylate hydrochloride **(A2-3,** 150 mg, 0.62 mmol) was dissolved in methanol (5 mL), and acetaldehyde aqueous solution (184 mg, 1.85 mmol, 40% wt) was added. The resulting mixture was stirred at room temperature for 30 minutes. Sodium cyanoborohydride (46 mg, 0.74 mmol) was added, and the mixture was stirred at room temperature for 3 hours. After the reaction was complete as monitored by LCMS, the system was concentrated to dryness and evaporated twice with anhydrous tetrahydrofuran to give the product (3*S*,4*S*)-methyl 4-(difluoromethyl)-1-ethyl-3-methylpiperidine-3-carboxylate (140 mg, 97% yield) as a colorless oil. MS-ESI [M+H]⁺: 236.1.

### Step B: ((3S,4S)-4-(difluoromethyl)-1-ethyl-3-methylpiperidin-3-yl)methanol

Under ice-bath cooling, 1M LiAlH₄-THF (0.9 mL, 0.89 mmol) was added dropwise to a solution of (3*S*,4*S*)-methyl 4-(difluoromethyl)-1-ethyl-3-methylpiperidine-3-carboxylate **(A16-1,** 140 mg, 0.60 mmol) in anhydrous THF (5 mL). The resulting mixture was stirred at room temperature for 15 minutes. After the reaction was complete as monitored by LCMS, the reaction was quenched with sodium sulfate decahydrate until no bubbles were generated. About 5 g of anhydrous sodium sulfate was added, and filtered through Celite. The filter cake was washed three times with anhydrous tetrahydrofuran. The filtrate was collected and concentrated to dryness to give ((3S,4S)-4-(difluoromethyl)-1-ethyl-3-methylpiperidin-3-yl)methanol (120 mg, 97% yield) as a colorless oil. MS-ESI [M+H]⁺: 208.1.

### Preparation of Intermediate A17: N,N-dimethyl-(S)-3-(methoxymethyl)-6-methyl-5,6,7,8-tetrahydro-4H-1,5,8a-triazazolin-2-carboxamide hydrochloride

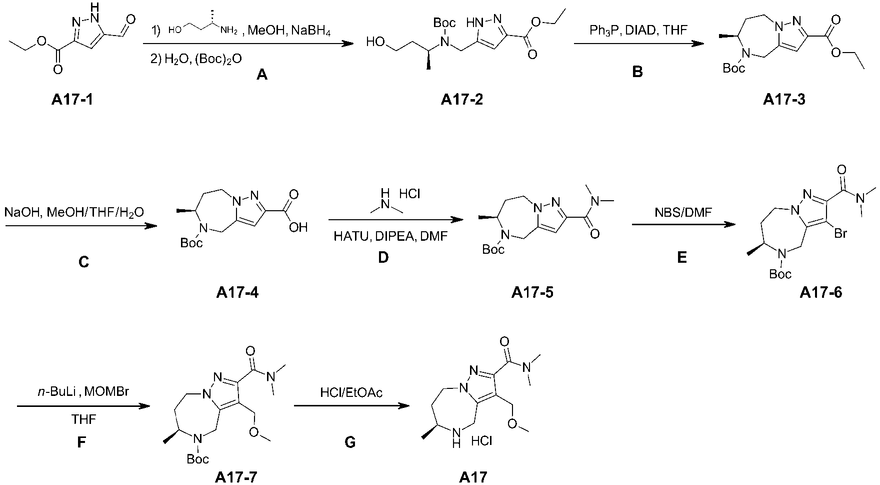

**Step A:** At room temperature, (S)-3-aminobutanol (19.9 g, 223 mmol) was added to a solution of ethyl 5-formylpyrazole-3-carboxylate **(A17-1,** 25.0 g, 149 mmol) in anhydrous methanol (250 mL) and stirred for 30 minutes. The reaction solution was cooled to 0-5 °C with ice water, and sodium borohydride (11.3 g, 297 mmol) was slowly added, keeping the internal temperature below 20 °C. After the addition, the mixture was stirred at room temperature for 2 hours. The reaction was complete as monitored by LCMS. An equal volume of water (250 mL) was added, and (Boc)₂O (64.9 g, 297 mmol) was added in one portion to the reaction system at room temperature, followed by stirring overnight. The reaction was complete as monitored by LCMS. The reaction solution was poured into H₂O (500 mL) and extracted with EtOAc (3 × 500 mL). The organic phases were combined, dried over anhydrous Na₂SO₄ and filtered. The filtrate was concentrated to dryness, and the crude product was purified by FCC (SiO₂, EA/PE = 0-100%) to give **A17-2** (26.2 g, 52% yield) as a colorless oil. MS-ESI [M+H]⁺: 342.4.

**Step B:** Under ice-water bath, DIAD (31.0 g, 153 mmol) was added dropwise to a mixture of compound **A17-2** (26.2 g, 76.4 mmol), triphenylphosphine (38.1 g, 153 mmol), and anhydrous THF (500 mL). The mixture was allowed to warm to room temperature and stirred overnight. The reaction was complete as monitored by LCMS. The reaction solution was poured into H₂O (400 mL) and extracted with EtOAc (3 × 400 mL). The organic phases were combined, dried over anhydrous Na₂SO₄ and filtered. The filtrate was concentrated to dryness, and the crude product was purified by FCC (SiO₂, EA/PE = 0-100%) to give **A17-3** (18.0 g, 73% yield) as a colorless oil. MS-ESI [M+H]⁺: 324.1.

**Step C:** Under ice bath, NaOH (11.3 g, 278 mmol) was added to a solution of compound **A17-3** (18.0 g, 55.7 mmol) in MeOH/THF/H₂O (1/1/1, 450 mL), and the mixture was stirred at room temperature for 1 hour. The reaction was complete as monitored by LCMS. The organic solvent was removed under reduced pressure. The remaining aqueous phase was cooled to 0-10 °C with an ice-water bath, adjusted to pH 3-4 with 2M HCl, and extracted with EtOAc (5 × 400 mL). The organic phases were combined, dried over anhydrous Na₂SO₄ and filtered. The filtrate was concentrated to dryness to give crude compound **A17-4** (16 g, 97% yield) as a white solid. MS-ESI [M+H]⁺: 296.1.

**Step D:** At room temperature, HATU (34.7 g, 91.4 mmol) was added to a mixture of compound **A17-4** (18.0 g, 60.9 mmol), dimethylamine hydrochloride (9.82 g, 122 mmol), DIPEA (39.4 g, 305 mmol), and DMF (250 mL). The mixture was stirred at room temperature for 1 hour. The reaction was complete as monitored by LCMS. The reaction solution was poured into 5% wt LiCl aqueous solution (500 mL) and extracted with EtOAc (3 × 400 mL). The organic phases were combined, dried over anhydrous Na₂SO₄ and filtered. The filtrate was concentrated to dryness, and the crude product was purified by FCC (SiO₂, EA/PE = 0-100%) to give **A17-5** (12.4 g, 63% yield) as a white solid. ¹H NMR (400 MHz, DMSO-*d₆*) δ 6.39 (s, 1H), 4.88-4.53 (m, 1H), 4.51-4.11 (m, 3H), 4.09-3.93 (m, 1H), 3.23 (s, 3H), 2.94 (s, 3H), 2.18-1.93 (m, 2H), 1.45-1.24 (m, 9H), 1.20 (d, *J* = 6.6 Hz, 3H). MS-ESI [M+H]⁺: 323.2.

**Step E:** At room temperature, NBS (5.0 g, 27.9 mmol) was added to a mixture of compound **A17-5** (6.0 g, 18.6 mmol) and DMF (60 mL). The mixture was stirred at room temperature for 3 hours. The reaction was complete as monitored by LCMS. The reaction solution was poured into 5% LiCl aqueous solution (200 mL) and extracted with EtOAc (3 × 200 mL). The organic phases were combined, dried over anhydrous Na₂SO₄ and filtered. The filtrate was concentrated to dryness, and the crude product was purified by FCC (SiO₂, EA/PE = 0-100%) and lyophilized to give **A17-6** (7.0 g, 94% yield) as a white solid. ¹H NMR (400 MHz, DMSO-*d₆*) δ 4.68-4.58 (m, 1H), 4.45-4.39 (m, 2H), 4.31-4.20 (m, 1H), 4.09-3.90 (m, 1H), 2.96 (s, 3H), 2.95 (s, 3H), 2.18-2.05 (m, 2H), 1.33 (s, 9H), 1.19 (d, *J* = 6.4 Hz, 3H). MS-ESI [M+H]⁺: 401.1; 403.1.

**Step F:** At room temperature, compound **A17-6** (1 g, 2.5 mmol) was dissolved in anhydrous tetrahydrofuran (15 mL) and cooled in a dry ice-ethanol bath for 10 minutes. n-Butyllithium (1.6M, 2.4 mL, 3.75 mmol) was slowly added dropwise to the reaction system. After the addition, stirring continued at dry ice-ethanol bath temperature for 10 minutes. Bromomethyl methyl ether (468 mg, 3.75 mmol) in anhydrous tetrahydrofuran (1.5 mL) was slowly added dropwise. The temperature was maintained for 10 minutes, then the mixture was allowed to warm to room temperature and stirred for 20 minutes. The reaction was complete as monitored by LCMS. The reaction was quenched with aqueous ammonium chloride (5 mL), diluted with 20 mL water, and extracted with EtOAc (3 × 30 mL). The organic phases were combined, dried over anhydrous Na₂SO₄ and filtered. The filtrate was concentrated to dryness, and the crude product was purified by FCC (SiO₂, EA/PE = 0-100%) to give **A17-7** (465 mg, 49% yield) as a white solid. MS-ESI [M+H]⁺: 367.2.

**Step G:** At room temperature, 4M HCl-ethyl acetate (10 mL, 40 mmol) was added to compound **A17-7** (465 mg, 1.27 mmol) and stirred at room temperature for 1 hour. The reaction was complete as monitored by LCMS. The acid solution was removed by concentration to give intermediate **A17** (385 mg, 100%) as a semi-solid oil. MS-ESI [M+H]⁺: 267.1.

### Preparation of Intermediate A18: (S)-4-[4-chloro-8-fluoro-2-(methylthio)-6-(trifluoromethyl)quinazolin-7-yl]-3-cyano-5-fluoro-5,6-dihydro-1-thiainden-2-yl 2-methylpropan-2-carboxylate

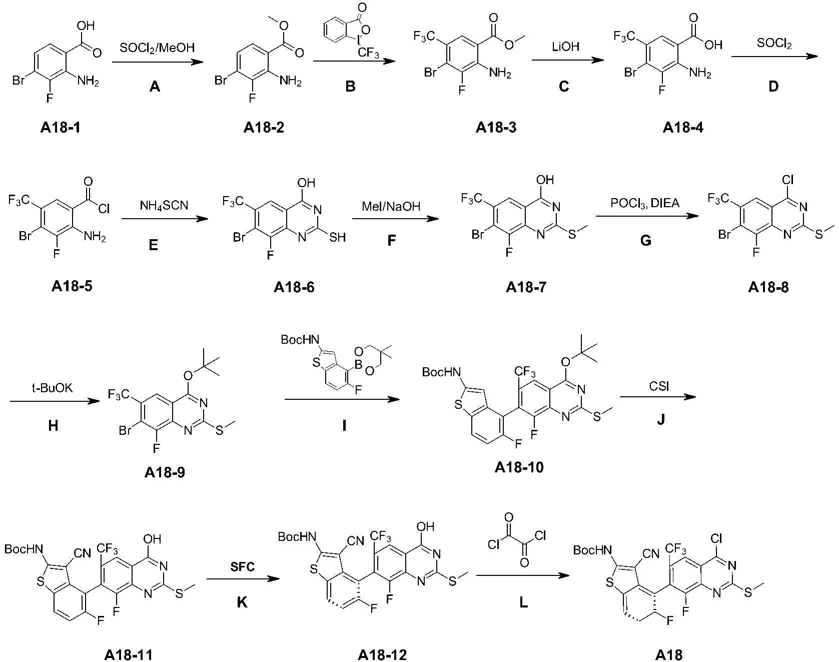

### Step A: methyl 2-amino-4-bromo-3-fluorobenzoate

At 0 °C, SOCl₂ (156 mL, 210 mmol) was added to a mixture of 2-amino-4-bromo-3-fluorobenzoic acid (50 g, 214 mmol) and methanol (300 mL). The reaction system was heated to 100 °C and stirred for 16 hours. The reaction was complete as monitored by TLC. The reaction solution was cooled to room temperature, concentrated under reduced pressure, diluted with EA (1 L), and washed with saturated NaHCO₃ aqueous solution (500 mL). The organic phase was collected, dried over anhydrous Na₂SO₄, filtered, concentrated, and purified by FCC (SiO₂, EA/PE = 0-20%) to give methyl 2-amino-4-bromo-3-fluorobenzoate **(A18-2,** 40 g) as a white solid. MS-ESI [M+H]⁺: 249.9.

### Step B: methyl 2-amino-4-bromo-3-fluoro-5-(trifluoromethyl)benzoate

At room temperature, 1-(trifluoromethyl)-1,2-benziodoxol-3(1*H*)-one (19 g, 60 mmol) was added to a mixed solution of methyl 2-amino-4-bromo-3-fluorobenzoate (5 g, 20 mmol) in ACN (20 mL). After replacing with N₂ three times, the mixture was warmed to 100 °C and stirred for 72 hours. After the reaction was complete as monitored by LCMS, the reaction solution was returned to room temperature, concentrated, diluted with EA (100 mL), washed with water (2 × 50 mL), dried over anhydrous Na₂SO₄, filtered, concentrated, and purified by FCC (SiO₂, EA/PE = 0-10%) to give methyl 2-amino-4-bromo-3-fluoro-5-(trifluoromethyl)benzoate **(A18-3,** 1.2 g) as a pale yellow solid. MS-ESI [M+H]⁺: 317.1.

### Step C: 2-amino-4-bromo-3-fluoro-5-(trifluoromethyl)benzoic acid

At room temperature, a solution of LiOH (15.17 g, 630 mmol) in water (100 mL) was added to a mixture of methyl 2-amino-4-bromo-3-fluoro-5-(trifluoromethyl)benzoate (20 g, 63 mmol), THF (100 mL), and MeOH (100 mL). The resulting mixture was stirred at room temperature for 16 hours. The reaction was complete as monitored by TLC and LCMS. 1M HCl was added to adjust pH to 6, and the mixture was extracted with EA (3 × 100 mL). The organic phase was collected, washed with saturated brine, and concentrated to give 2-amino-4-bromo-3-fluoro-5-(trifluoromethyl)benzoic acid **(A18-4,** 19 g, 99% yield) as a yellow solid. MS-ESI [M+H]⁺: 301.9.

### Step D: 2-amino-4-bromo-3-fluoro-5-(trifluoromethyl)benzoyl chloride

At room temperature, 2-amino-4-bromo-3-fluoro-5-(trifluoromethyl)benzoic acid (19 g, 63 mmol) was added to SOCl₂ (200 mL) and heated to 50 °C with stirring for 3 hours. The reaction was complete as monitored by LCMS. The reaction solution was returned to room temperature and concentrated to give 2-amino-4-bromo-3-fluoro-5-(trifluoromethyl)benzoyl chloride **(A18-5,** 20 g, 99% yield) as a yellow oil. MS-ESI [M-Cl+OMe]⁺: 315.9.

### Step E: 7-bromo-8-fluoro-2-mercapto-6-(trifluoromethyl)quinazolin-4-ol

At room temperature, 2-amino-4-bromo-3-fluoro-5-(trifluoromethyl)benzoyl chloride (20 g, 62.4 mmol) was added to acetone (200 mL). Ammonium thiocyanate (14.2 g, 187 mmol) dissolved in acetone (100 mL) was added to the above reaction solution and stirred for 2 hours. The reaction was complete as monitored by LCMS. The reaction solution was slowly poured into stirred water (1000 mL), and a flocculent insoluble precipitate formed. The precipitate was collected by filtration and dried to give 7-bromo-8-fluoro-2-mercapto-6-(trifluoromethyl)quinazolin-4-ol **(A18-6,** 20 g, 93% yield) as a yellow solid. MS-ESI [M+H]⁺: 342.9.

### Step F: 7-bromo-8-fluoro-2-(methylthio)-6-(trifluoromethyl)quinazolin-4-ol

At room temperature, MeI (20.68 g, 146 mmol) was added to a mixed solution of 7-bromo-8-fluoro-2-mercapto-6-(trifluoromethyl)quinazolin-4-ol (20 g, 58.3 mmol), MeOH (200 mL), and 0.1M NaOH (200 mL), and stirred for 2 hours. The reaction was complete as monitored by LCMS. The mixture was slowly poured into stirred water (1000 mL), adjusted to pH 6 with concentrated hydrochloric acid, and the resulting flocculent precipitate was collected by filtration and dried to give 7-bromo-8-fluoro-2-(methylthio)-6-(trifluoromethyl)quinazolin-4-ol **(A18-7,** 20 g, 96% yield) as a yellow solid. MS-ESI [M+H]⁺: 356.9.

### Step G: 7-bromo-4-chloro-8-fluoro-2-(methylthio)-6-(trifluoromethyl)quinazoline

At room temperature, POCl₃ (20.6 g, 134 mmol) was added to a mixed solution of 7-bromo-8-fluoro-2-(methylthio)-6-(trifluoromethyl)quinazolin-4-ol (16 g, 44.8 mmol), DIEA (17.37 g, 134 mmol), and THF (200 mL), and heated to 60 °C with stirring for 1 hour. The reaction was complete as monitored by LCMS. The reaction solution was concentrated and purified by FCC to give 7-bromo-4-chloro-8-fluoro-2-(methylthio)-6-(trifluoromethyl)quinazoline **(A18-8,** 15 g, 89.2% yield) as a yellow oil. MS-ESI [M+H]⁺: 374.8.

### Step H: 7-bromo-4-(tert-butoxy)-8-fluoro-2-(methylthio)-6-(trifluoromethyl)quinazoline

Under a dry ice-ethanol bath, *t*-BuOK (26.6 mL, 1M in THF, 26.6 mmol) was added to a mixed solution of 7-bromo-4-chloro-8-fluoro-2-(methylthio)-6-(trifluoromethyl)quinazoline (10 g, 26.6 mmol) and THF (200 mL). The mixture was stirred at this temperature for 1 hour, then allowed to warm to room temperature and stirred for another 1 hour. The reaction was complete as monitored by LCMS and TLC. The reaction solution was poured into half-saturated NH₄Cl solution (100 mL) and extracted with EA (3 × 100 mL). The organic phase was collected, washed with saturated brine (100 mL), dried over anhydrous Na₂SO₄, filtered, concentrated, and purified by FCC (SiO₂, EA/PE = 0-20%) to give 7-bromo-4-(tert-butoxy)-8-fluoro-2-(methylthio)-6-(trifluoromethyl)quinazoline **(A18-9,** 10 g, 90.1% yield) as a yellow solid. MS-ESI [M+H]⁺: 412.9.

### Step I: tert-butyl (4-(4-(tert-butoxy)-8-fluoro-2-(methylthio)-6-(trifluoromethyl)quinazolin-7-yl)-5-fluorobenzo[b]thiophen-2-yl)carbamate

At room temperature, 7-bromo-4-(tert-butoxy)-8-fluoro-2-(methylthio)-6-(trifluoromethyl)quinazoline (4.00 g, 9.68 mmol), tert-butyl (4-(5,5-dimethyl-1,3,2-dioxaborinan-2-yl)-5-fluorobenzo[*b*]thiophen-2-yl)carbamate (5.51 g, 14.52 mmol), RuphosPdG4 (1.64 g, 0.19 mmol), and DIEA (3.75 g, 29.04 mmol) were sequentially added to a mixed solution of 1,4-dioxane (80 mL) and water (16 mL). After replacing with N₂ three times, the mixture was heated to 80 °C and stirred for 4 hours. The reaction was complete as monitored by LCMS and TLC. The reaction solution was cooled to room temperature, filtered through Celite to remove the palladium catalyst, and the filtrate was collected. Water (100 mL) was added, and the mixture was extracted with EA (3 × 100 mL). The organic phase was collected, washed with saturated brine (50 mL), and the organic solution was concentrated. The resulting crude product was purified by FCC (SiO₂, EA/PE = 0-40%) to give tert-butyl (4-(4-(tert-butoxy)-8-fluoro-2-(methylthio)-6-(trifluoromethyl)quinazolin-7-yl)-5-fluorobenzo[*b*]thiophen-2-yl)carbamate **(A18-10,** 3 g, 51.6% yield) as a yellow solid. MS-ESI [M+H-56]⁺: 544.0.

### Step J: tert-butyl (3-cyano-5-fluoro-4-(8-fluoro-4-hydroxy-2-(methylthio)-6-(trifluoromethyl)quinazolin-7-yl)benzo[b]thiophen-2-yl)carbamate

Under a dry ice-acetonitrile bath, CSI (2.83 g, 20 mmol) was added to a mixed solution of tert-butyl (4-(4-(tert-butoxy)-8-fluoro-2-(methylthio)-6-(trifluoromethyl)quinazolin-7-yl)-5-fluorobenzo[*b*]thiophen-2-yl)carbamate (3 g, 5 mmol) in acetonitrile (500 mL) and stirred for 1 hour. The reaction was complete as monitored by LCMS. The reaction solution was poured into ice-cooled DMF (200 mL) and stirred for another 1 hour. The reaction was complete as monitored by LCMS. The reaction solution was poured into saturated NaHCO₃ solution (200 mL) and extracted with EA (3 × 100 mL). The organic phases were combined, washed with saturated brine (100 mL), dried over anhydrous Na₂SO₄, filtered, concentrated, and purified by FCC (SiO₂, EA/PE = 0-40%) to give tert-butyl (3-cyano-5-fluoro-4-(8-fluoro-4-hydroxy-2-(methylthio)-6-(trifluoromethyl)quinazolin-7-yl)benzo[*b*]thiophen-2-yl)carbamate **(A18-11,** 1.3 g, 45.7% yield) as a yellow solid. MS-ESI [M+H]⁺: 569.0.

**Step K:** Intermediate **A18-11** (5.0 g) was resolved by SFC (Waters SFC 150, column: DAICEL CHIRALCEL^{®}IC 250 * 25 mm, 10 µm; mobile phase CO₂/MeOH (0.1% 7.0M NH₃-MeOH) = 90/10; flow rate: 140 mL/min) to give the first eluted isomer 1 (2.1 g, shorter retention time, Rt = 2.692 min). MS-ESI [M+H]⁺: 569.0; followed by the later eluted isomer 2, intermediate **A18-12** (2.0 g, longer retention time, Rt = 3.424 min). MS-ESI [M+H]⁺: 569.0.

Chiral analysis method: Waters UPCC, analytical column: DAICEL CHIRALPAK^{®}IC, 100*3 mm, 3 µm; Mobile phase A: CO₂, Mobile phase B: MeOH (0.1% DEA); Flow rate: 1.5 mL/min; Column temperature: 35 °C; Back pressure: 1800 psi; Gradient: 0-8.0 min A/B = 85/15.

**Step L:** Under ice-water bath, DMF (25.7 mg, 0.35 mmol) was added to a solution of oxalyl chloride (178.6 mg, 1.41 mmol) in DCM (5 mL). The mixture was warmed to room temperature and stirred for 30 minutes. Then cooled to ice-water bath conditions, and intermediate **A18-12** (200 mg, 0.35 mmol) was added to the above reaction solution. The mixture was warmed to room temperature and stirred for 1 hour. The reaction was complete as monitored by LCMS and TLC. The reaction solution was poured into saturated NaHCO₃ (50 mL) and extracted with DCM (3 × 10 mL). The organic phase was collected, washed with saturated brine (10 mL), dried over anhydrous Na₂SO₄, and concentrated to give **A18** (200 mg, 97% yield) as a yellow solid. MS-ESI [M+H]⁺: 587.0.

### Preparation of Intermediate A19: (S)-3-cyano-4-[4,6-dichloro-8-fluoro-2-(methylthio)quinazolin-7-yl]-5-fluoro-5,6-dihydro-1-thiainden-2-yl 2-methylpropan-2-carboxylate

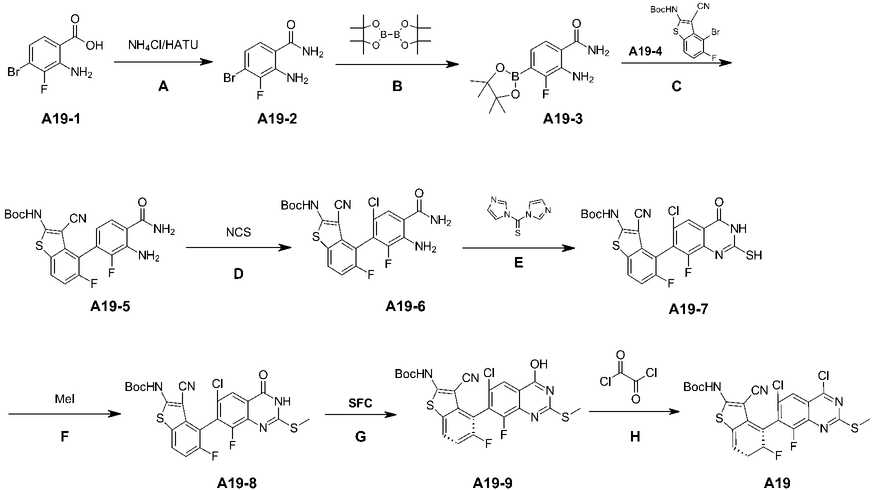

**Step A:** At room temperature, 2-amino-3-fluoro-4-bromobenzoic acid (60.0 g, 256 mmol), NH₄Cl (41.2 g, 769 mmol), HATU (146 g, 385 mmol), and DIEA (99.2 g, 769 mmol) were sequentially added to DMF (400 mL). The resulting reaction solution was stirred at room temperature for another 3 hours. The reaction was complete as monitored by LCMS and TLC. The reaction solution was poured into water (4 L), stirred at room temperature for 30 minutes, filtered, and the filter cake was dried in a vacuum oven to give compound **A19-2** (50.8 g, 85% yield) as a grey solid. MS-ESI [M+H]⁺: 233.1_{∘}

**Step B:** At room temperature, **A19-2** (22.1 g, 94.8 mmol), bis(pinacolato)diboron (28.9 g, 113.8 mmol), Pd(dppf)Cl₂ (3.47 g, 4.74 mmol), and potassium acetate (139 g, 142.2 mmol) were sequentially added to 1,4-dioxane (400 mL). After replacing with N₂ three times, the mixture was warmed to 90 °C and stirred for 4 hours. The reaction was complete as monitored by LCMS and TLC. The reaction solution was returned to room temperature, filtered through Celite to remove the palladium catalyst, and the filtrate was concentrated to give crude compound **A19-3,** which was used directly in the next step. MS-ESI [M+H]⁺: 281.1.

**Step C:** At room temperature, **A19-3** (26.6 g, 94.8 mmol), intermediate **A19-4** (23.5 g, 63.5 mmol), Pd-118 (4.14 g, 6.35 mmol), and K₃PO₄ (40.4 g, 190.5 mmol) were sequentially added to a solution of 1,4-dioxane (400 mL) and water (80 mL). After replacing with N₂ three times, the mixture was warmed to 90 °C and stirred for 2 hours. The reaction was complete as monitored by LCMS and TLC. The reaction solution was returned to room temperature, filtered through Celite to remove the palladium catalyst, and the filtrate was collected. Water (300 mL) was added, and the mixture was extracted with EA (3 × 200 mL). The organic phase was collected, washed with saturated brine, dried over anhydrous Na₂SO₄, filtered, concentrated, and purified by FCC (SiO₂, THF/PE = 0-55%) to give compound **A19-5** (22.1 g, 78% yield over two steps) as a yellow solid. MS-ESI [M+H]⁺: 445.3.

**Step D:** At room temperature, NCS (7.97 g, 59.7 mmol) was added to a solution of compound **A19-5** (22.1 g, 49.8 mmol) in DMF (200 mL). The resulting reaction solution was stirred at room temperature for 16 hours. The reaction was complete as monitored by LCMS and TLC. The reaction solution was concentrated, and the crude product was purified by FCC (SiO₂, THF/PE = 0-55%) to give compound **A19-6** (23.1 g, 97% yield) as a yellow solid. MS-ESI [M+H]⁺: 479.1.

**Step E:** At room temperature, sodium hydride (60% wt, 7.73 g, 193.2 mmol) was added to a solution of compound **A19-6** (23.1 g, 48.3 mmol) in tetrahydrofuran (400 mL). The resulting reaction solution was stirred at 40 °C for 0.5 hours. N,N'-Thiocarbonyldiimidazole (10.3 g, 58.0 mmol) was added, and the mixture was warmed to 60 °C and stirred for 2 hours. The reaction was complete as monitored by LCMS. The reaction solution was returned to room temperature, adjusted to pH 3-5 with hydrochloric acid, and extracted with EA (3 × 150 mL). The organic phase was collected, washed with saturated brine, dried over anhydrous Na₂SO₄, and concentrated to give compound **A19-7** (26.5 g, crude) as a brown solid. MS-ESI [M+H]⁺: 521.1.

**Step F:** At room temperature, compound **A19-7** (26.5 g, 51.0 mmol), NaOH (8.15 g, 20.4 mmol), and iodomethane (21.7 g, 153 mmol) were sequentially added to a solution of methanol (200 mL) and water (200 mL), and stirred at room temperature for 2 hours. The reaction was complete as monitored by LCMS. The reaction solution was adjusted to pH 3-5 with hydrochloric acid and extracted with EA (3 × 150 mL). The organic phase was collected, washed with saturated brine, dried over anhydrous Na₂SO₄, filtered, concentrated, and purified by FCC (SiO₂, THF/PE = 0-34%) to give intermediate **A19-8** (19 g, 74% yield over two steps) as a white solid. MS-ESI [M+H]⁺: 535.1.

**Step G:** Intermediate **A19-8** (10 g) was resolved by SFC (Waters SFC 150, column: DAICEL CHIRALCEL^{®}IC 250*50 mm 10 µm; mobile phase: CO₂/MeOH = 55/45; flow rate: 150 mL/min) to give the first eluted isomer 1 (4.4 g, 44% yield, shorter retention time, chiral analysis Rt = 2.501 min). MS-ESI [M+H]⁺: 535.1; followed by the later eluted isomer 2, intermediate **A19-9** (4.6 g, 46% yield, longer retention time, chiral analysis Rt = 3.208 min). MS-ESI [M+H]⁺: 535.1.

Chiral analysis method: Waters UPCC (CA-352), analytical column: DAICEL CHIRALPAK^{®}IC, 100*3 mm, 3 µm, Mobile phase A: CO₂, Mobile phase B: MeOH; Flow rate: 1.5 mL/min; Column temperature: 35 °C; Back pressure: 1800 psi; Gradient: 0-8 min A/B = 70/30.

**Step H:** Under ice bath, DMF (85 mg, 1.16 mmol) was added to a solution of oxalyl chloride (2.21 g, 17.4 mmol) in DCM (45 mL), and the temperature was maintained for 0.5 hours. Intermediate **A19-9** (3.1 g, 5.81 mmol) was added to the reaction solution. The resulting reaction solution was stirred at room temperature for another 1 hour. The reaction was complete as monitored by LCMS. The reaction solution was poured into water (50 mL) and extracted with DCM (3 × 50 mL). The organic phase was collected, washed with saturated brine, dried over anhydrous Na₂SO₄. The organic phase was concentrated, and purified by FCC (SiO₂, THF/PE = 0-15%) to give intermediate **A19** (2.34 g, 73% yield) as a white solid. MS-ESI [M+H]⁺: 553.0.

### Preparation of Intermediate A20: tert-butyl (4R,7S,8S)-13-chloro-14-fluoro-9-methyl-17-(methylthio)-10-oxa-2,12,16,18,20-pentaazapentacyclo[9.7.1.1^{4,7}.0^{2,8}.0^{15,19}]icosa-1(18),11,13,15(19),16-pentaene-20-carboxylate

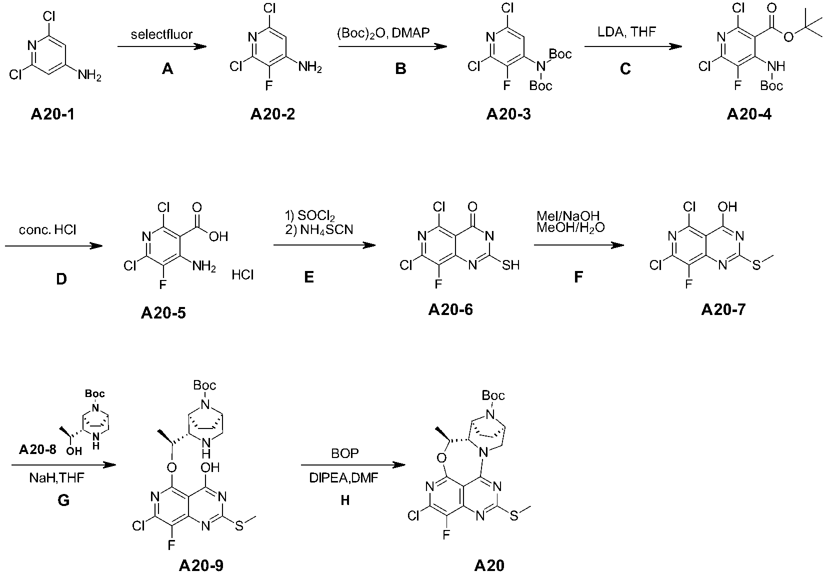

### Step A: 2,6-dichloro-3-fluoropyridin-4-amine

At room temperature, Selectfluor (68 g, 180 mmol) was added to a solution of 2,6-dichloropyridin-4-amine (25 g, 154 mmol) in methanol/water (300 mL, V/V = 5/1). The resulting mixture was stirred at 50 °C for 48 hours, concentrated under reduced pressure, diluted with EA, washed sequentially with water and saturated brine, dried over anhydrous sodium sulfate, filtered, and concentrated. The crude product was purified by FCC (SiO₂, EA/PE = 0-10%) to give 2,6-dichloro-3-fluoropyridin-4-amine **(A20-2,** 10 g) as a white solid. MS-ESI [M+H]⁺: 180.9.

### Step B: tert-butyl (tert-butoxycarbonyl)(2,6-dichloro-3-fluoropyridin-4-yl)carbamate

Under stirring at room temperature, 4-dimethylaminopyridine (307 mg, 2.75 mmol) and di-tert-butyl dicarbonate (30 g, 138 mmol) were added to a solution of 2,6-dichloro-3-fluoropyridin-4-amine (10 g, 55 mmol) in tetrahydrofuran (100 mL). The resulting mixture was heated to 60 °C and stirred for 16 hours. The reaction was complete as monitored by TLC, concentrated to give a crude product, which was slurried with methanol to give tert-butyl (tert-butoxycarbonyl)(2,6-dichloro-3-fluoropyridin-4-yl)carbamate **(A20-3,** 16 g) as a white solid. MS-ESI [M+H]⁺: 381.2.

### Step C: tert-butyl 4-((tert-butoxycarbonyl)amino)-2,6-dichloro-5-fluoronicotinate

Under a dry ice-ethanol bath, LDA (2.0M, 63 mL, 126 mmol) was slowly added to a solution of tert-butyl (tert-butoxycarbonyl)(2,6-dichloro-3-fluoropyridin-4-yl)carbamate (16 g, 42 mmol) in THF (200 mL). The resulting mixture was stirred at this temperature for 1 hour. The reaction was complete as monitored by TLC. The reaction was quenched by adding a suitable amount of acetic acid, diluted with EA, washed with water, dried over anhydrous sodium sulfate, filtered, and concentrated. The crude product was purified by FCC (SiO₂, EA/PE = 0-20%) to give tert-butyl 4-((tert-butoxycarbonyl)amino)-2,6-dichloro-5-fluoronicotinate **(A20-4,** 13 g).

### Step D: 4-amino-2,6-dichloro-5-fluoronicotinic acid hydrochloride

At room temperature, concentrated hydrochloric acid (30 mL) was added to a solution of tert-butyl 4-((tert-butoxycarbonyl)amino)-2,6-dichloro-5-fluoronicotinate (13 g, 34 mmol) in dioxane (90 mL). The resulting mixture was stirred at room temperature for 3 hours. After the reaction was complete as monitored by LCMS, concentrated to give 4-amino-2,6-dichloro-5-fluoronicotinic acid hydrochloride **(A20-5,** 8 g). MS-ESI [M+H]⁺: 224.9.

### Step E: 5,7-dichloro-8-fluoro-2-mercaptopyrido[4,3-d]pyrimidin-4(3H)-one

A mixed solution of 4-amino-2,6-dichloro-5-fluoronicotinic acid (8 g, 30.8 mmol) and thionyl chloride (200 mL) was stirred at 50 °C for 3 hours. Then it was concentrated, and the residue was dissolved in acetone (50 mL) to give solution 1. At room temperature, a mixed solution of ammonium thiocyanate (7 g, 92 mmol) in acetone (160 mL) was added dropwise to solution 1, and the resulting reaction solution was stirred at room temperature for another 1 hour. After the reaction was complete as monitored by LCMS, the reaction solution was poured into water, filtered, and the filter cake was dried to give 5,7-dichloro-8-fluoro-2-mercaptopyrido[4,3-*d*]pyrimidin-4(3*H*)-one **(A20-6,** 5 g). MS-ESI [M+H]⁺: 265.9.

### Step F: 5,7-dichloro-8-fluoro-2-(methylthio)pyrido[4,3-d]pyrimidin-4-ol

At room temperature, a mixed solution of 5,7-dichloro-8-fluoro-2-mercaptopyrido[4,3-*d*]pyrimidin-4(3*H*)-one (5 g, 18.8 mmol), methanol (380 mL), aqueous sodium hydroxide (1M, 380 mL, 380 mmol), and iodomethane (5.3 g, 380 mmol) was stirred for 2 hours. After the reaction was complete as monitored by LCMS, the reaction solution was poured into 1000 mL of water and acidified to pH 6 with concentrated hydrochloric acid. The solution was filtered, and the filter cake was dried to give 5,7-dichloro-8-fluoro-2-(methylthio)pyrido[4,3-*d*]pyrimidin-4-ol **(A20-7,** 4 g). MS-ESI [M+H]⁺: 279.9.

**Step G:** Under ice-water bath, 60% NaH (116 mg, 2.90 mmol) was added to a solution of tert-butyl (1*S*,2*S*,5*R*)-2-((*S*)-1-hydroxyethyl)-3,8-diazabicyclo[3.2.1]octane-8-carboxylate **(A20-8,** CAS: 2820537-22-0, 247 mg, 0.964 mmol) in anhydrous THF (10 mL), and the mixture was stirred at room temperature for 30 minutes. Intermediate **A20-7** (270 mg, 0.964 mmol) was then added, and the mixture was stirred at room temperature overnight. The reaction was complete as monitored by LCMS. The reaction was quenched with 1 mL of saturated ammonium chloride solution. The reaction solution was concentrated to dryness, and the crude product was purified by FCC (SiO₂, THF/PE = 0-100%) to give **A20-9** (420 mg, 87% yield) as a brown solid. MS-ESI [M+H]⁺: 500.3.

**Step H:** At room temperature, BOP (557 mg, 1.26 mmol) was added to a solution of **A20-9** (420 mg, 0.840 mmol), DIPEA (0.543 mg, 4.20 mmol), and anhydrous DMF (10 mL). The mixture was then heated to 60 °C and stirred overnight. The reaction was complete as monitored by LCMS. The reaction solution was poured into 5% LiCl aqueous solution (50 mL) and extracted with EtOAc (3 × 50 mL). The organic phases were combined, washed with saturated NaCl, dried over anhydrous Na₂SO₄, and filtered. The filtrate was concentrated to dryness, and the crude product was purified by FCC (SiO₂, THF/PE = 0-100%) to give **A20** (175 mg, 43% yield) as a white solid. MS-ESI [M+H]⁺: 482.1.

### Preparation of Intermediate A21: (8S)-13-chloro-14-fluoro-17-(methylsulfonyl)-6,10-dioxa-2,12,16,18-tetraazatetracyclo[9.7.1.0^{2,8}.0^{15,19}]nonadeca-1(18),11(19),12,14,16-pentaene

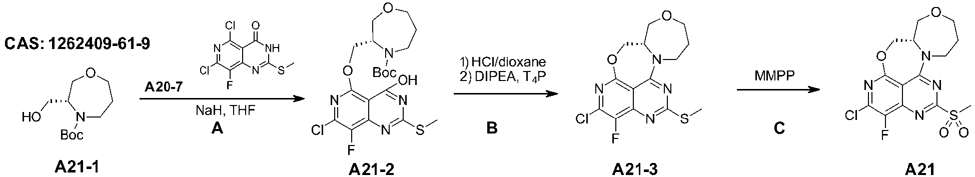

**Step A:** At room temperature, **A21-1** (3.47 g, 15.0 mmol) and compound **A20-7** (3.50 g, 12.5 mmol) were dissolved in THF (120 mL), cooled to -5 °C using an ice-salt bath, and then NaH (2.00 g, 60% purity, 50.0 mmol) was added in portions. The reaction solution was returned to room temperature and stirred for another 2 hours. Cooled with an ice bath, the reaction solution was adjusted to pH about 3 with 1M dilute hydrochloric acid and extracted with ethyl acetate (3 × 150 mL). The organic phases were combined, dried over anhydrous sodium sulfate, filtered, and concentrated to remove solvent. The crude product was purified by FCC (PE/EA = 0-100%) to give **A21-2** (2.90 g, 49% yield) as a yellow solid. MS-ESI [M-Boc+H]⁺: 375.1.

**Step B:** At room temperature, **A21-2** (2.90 g, 6.11 mmol) was added to HCl-dioxane (4M, 50 mL). The reaction solution was stirred at room temperature for 1 hour. The solvent was removed by concentration under reduced pressure, to give a yellow solid residue. The residue was dissolved in toluene (50 mL), then *N*,*N*-diisopropylethylamine (7.89 g, 61.16 mmol) was added, and finally phosphorus oxychloride (3.74 g, 24.4 mmol) was added. The reaction solution was stirred at room temperature for 1 hour. The reaction solution was treated with saturated aqueous sodium bicarbonate (100 mL) and extracted with ethyl acetate (3 × 100 mL). The organic phases were combined, dried over anhydrous sodium sulfate, filtered, and concentrated to remove solvent. The crude product was purified by FCC (DCM/MeOH = 0-15%) to give **A21-3** (1.95 g, 90% yield) as a white solid. MS-ESI [M+H]⁺: 357.1.

**Step C:** At room temperature, magnesium monoperoxyphthalate hexahydrate (12.2 g, 87% purity, 21.46 mmol) was added to a solution of intermediate **A21-3** (1.5 g, 4.20 mmol) in DCM/H₂O (1:1, 60 mL). The reaction solution was stirred at room temperature for 2 hours. The reaction solution was diluted with water (50 mL) and extracted with dichloromethane (3 × 50 mL). The organic phases were combined, dried over anhydrous sodium sulfate, filtered, and concentrated to remove solvent. The crude product was purified by FCC (PE/THF = 0-100%) to give **A21** (950 mg, 58% yield) as a white solid. MS-ESI [M+H]⁺: 389.1.

### Preparation of Intermediate A22: [2-(7'-{[(S)-(E)-4-fluoromethylene-1-methyl-3-methylpiperidin-3-yl]methoxy}-5'-(3,8-diazabicyclo[3.2.1]octan-3-yl)-1',7-difluoro-4'-methoxy-3-[tri(propan-2-yl)silyloxy]-3',6',8'-triaza-1,2'-binaphthalen-8-yl)ethynyl]tri(propan-2-yl)silane

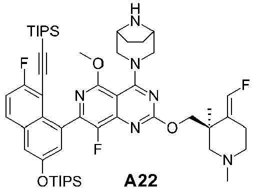

Intermediate **A22** was prepared following the synthetic method for intermediate **P2-2** of payload molecule **P2,** using intermediate **A8** (tert-butyl (1*R*,5*S*)-3-(8-fluoro-7-(7-fluoro-8-((triisopropylsilyl)ethynyl)-3-((triisopropylsilyl)oxy)naphthalen-1-yl)-5-methoxy-2-(methylsulfonyl)pyrido[4,3-*d*]pyrimidin-4-yl)-3,8-diazabicyclo[3.2.1]octane-8-carboxylate) instead of intermediate **A7** (tert-butyl (1*R*,5*S*)-3-(8-fluoro-7-(7-fluoro-8-((triisopropylsilyl)ethynyl)-3-((triisopropylsilyl)oxy)naphthalen-1-yl)-2-(methylsulfonyl)pyrido[4,3-*d*]pyrimidin-4-yl)-3,8-diazabicyclo[3.2.1]octane-8-carboxylate). ¹H NMR (400 MHz, CD₃OD) δ 7.88 (dd, *J* = 9.2, 5.6 Hz, 1H), 7.41 (d, *J* = 2.8 Hz, 1H), 7.35 (t, *J* = 8.8 Hz, 1H), 7.21 (t, *J* = 2.4 Hz, 1H), 6.67 (d, *J* = 86.8 Hz, 1H), 4.55 (dd, *J* = 39.2, 10.4 Hz, 1H), 4.35 (dd, *J* = 27.2, 10.4 Hz, 1H), 4.00 (d, *J* = 1.6 Hz, 3H), 3.74-3.68 (m, 2H), 3.66-3.45 (m, 3H), 3.25 (d, *J* = 12.8 Hz, 1H), 2.92-2.64 (m, 3H), 2.36-2.15 (m, 4H), 2.14-1.93 (m, 3H), 1.89-1.75 (m, 3H), 1.55-1.43 (m, 1H), 1.39-1.27 (m, 4H), 1.22 (s, 3H), 1.18-1.04 (m, 17H), 0.95-0.79 (m, 17H), 0.65-0.49 (m, 3H). MS-ESI [M+H]⁺: 957.8.

### Preparation of Intermediate A23: (2-{8-[(8S)-17-{[(2R,7aS)-2-fluorohexahydro-1H-pyrrolizin-7a-yl]methoxy}-14-fluoro-6,10-dioxo-2,12,16,18-tetraazatetracyclo[9.7.1.0^{2,8}.0^{15,19}]nonadeca-1(18),11(19),12,14,16-pentaen-13-yl]-2-fluoro-6-[tri(propan-2-yl) silyloxy] naphthalen-1-yl } ethynyl) tri(propan-2-yl) silane

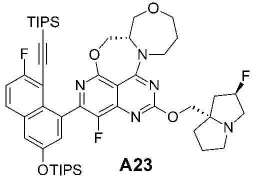

Intermediate **A23** was prepared following the synthetic method for intermediate **P16-2** of payload molecule **P16,** using intermediate [(2*R*,8*S*)-2-fluoro-1,2,3,5,6,7-hexahydropyrrolizin-7-yl]methanol instead of intermediate **A15** (*S*,*E*)-(1-ethyl-4-(fluoromethylene)-3-methylpiperidin-3-yl)methanol. MS-ESI [M+H]⁺: 930.1.

### Preparation of Payload Molecule P1: (Ra)-4-(4-((1R,5S)-3,8-diazabicyclo[3.2.1]octan-3-yl)-2-(((3S,4S)-4-(difluoromethyl)-1,3-dimethylpiperidin-3-yl)methoxy)-6,8-difluoroquinazolin-7-yl)-5-ethynyl-6-fluoronaphthalen-2-ol

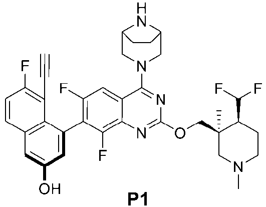

Payload molecule **P1** was prepared following the synthetic route for payload molecule **P2,** using intermediate **A6** (tert-butyl (1*R*,5*S*)-3-(2,6,8-trifluoro-7-(7-fluoro-8-((triisopropylsilyl)ethynyl)-3-((triisopropylsilyl)oxy)naphthalen-1-yl)quinazolin-4-yl)-3,8-diazabicyclo[3.2.1]octane-8-carboxylate) instead of intermediate **A7** (tert-butyl (1*R*,5*S*)-3-(8-fluoro-7-(7-fluoro-8-((triisopropylsilyl)ethynyl)-3-((triisopropylsilyl)oxy)naphthalen-1-yl)-2-(methylsulfonyl)pyrido[4,3-*d*]pyrimidin-4-yl)-3,8-diazabicyclo[3.2.1]octane-8-carboxylate), and using intermediate **A2** ((3S,4S)-4-(difluoromethyl)-1,3-dimethylpiperidin-3-yl)methanol instead of intermediate **A1** (*S*,*E*)-(4-(fluoromethylene)-1,3-dimethylpiperidin-3-yl)methanol. The crude compound (*Ra*)-4-(4-((1*R*,5*S*)-3,8-diazabicyclo[3.2.1]octan-3-yl)-2-(((3*S*,4*R*)-4-(difluoromethyl)-1,3-dimethylpiperidin-3-yl)methoxy)-6,8-difluoroquinazolin-7-yl)-5-ethynyl-6-fluoronaphthalen-2-ol was separated by Pre-HPLC (C18, ACN/(10 mmol NH₄HCO₃/H₂O) = 35-55%) to give isomer 1 (shorter retention time) (19 mg). ¹H NMR (400 MHz, DMSO-*d*₆) δ 7.99 (dd, *J* = 9.3, 6.0 Hz, 1H), 7.59-7.52 (m, 1H), 7.51-7.45 (m, 1H), 7.41 (d, *J* = 2.6 Hz, 1H), 7.15 (d, *J* = 2.6 Hz, 1H), 6.36-6.02 (m, 1H), 4.28-4.15 (m, 4H), 3.97 (s, 1H), 3.55-3.48 (m, 3H), 3.46-3.43 (m, 1H), 2.81-2.75 (m, 1H), 2.49-2.46 (m, 1H), 2.14 (s, 3H), 2.07-1.94 (m, 2H), 1.89-1.79 (m, 1H), 1.78-15 1.68 (m, 2H), 1.68-1.58 (m, 4H), 1.09 (s, 3H). ¹⁹F NMR (376 MHz, DMSO-*d*₆) δ -110.21, -115.79, -119.11, -120.78, - 124.72. MS-ESI [M+H]⁺: 652.3; and isomer 2 (longer retention time) as compound **P1** (30 mg). ¹H NMR (400 MHz, DMSO-*d*₆) δ 10.22 (s, 1H), 7.99 (dd, *J* = 9.2, 6.0 Hz, 1H), 7.58-7.52 (m, 1H), 7.52-7.45 (m, 1H), 7.41 (d, *J* = 2.6 Hz, 1H), 7.15 (d, *J* = 2.5 Hz, 1H), 6.44-6.13 (m, 1H), 4.46-4.39 (m, 1H), 4.38-4.31 (m, 1H), 4.30-4.19 (m, 2H), 3.99 (s, 1H), 3.56-3.48 20 (m, 3H), 3.47-3.42 (m, 1H), 2.87-2.78 (m, 2H), 2.11 (s, 3H), 1.89-1.76 (m, 2H), 1.74-1.54 (m, 7H), 1.11 (s, 3H). ¹⁹F NMR (376 MHz, DMSO-*d*₆) δ -110.19, -115.57, -118.92, -119.19, -124.59. MS-ESI [M+H]⁺: 652.3.

### Preparation of Payload Molecule P2: 4-(4-((1R,5S)-3,8-diazabicyclo[3.2.1]octan-3-yl)-8-fluoro-2-(((S,E)-4-(fluoromethylene)-1,3-dimethylpiperidin-3-yl)methoxy)pyrido[4,3-d]pyrimidin-7-yl)-5-ethynyl-6-fluoronaphthalen-2-ol

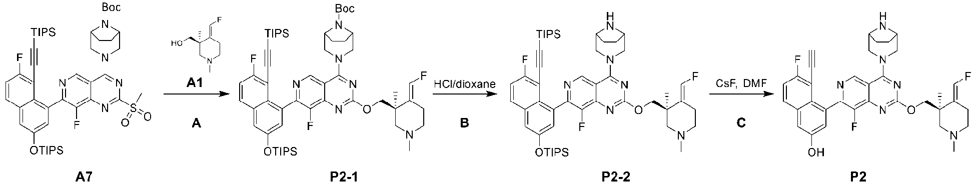

### Step A: tert-butyl (1R,5S)-3-(8-fluoro-7-(7-fluoro-8-((triisopropylsilyl)ethynyl)-3-((triisopropylsilyl)oxy)naphthalen-5 1-yl)-2-(((S,E)-4-(fluoromethylene)-1,3-dimethylpiperidin-3-yl)methoxy)pyrido[4,3-d]pyrimidin-4-yl)-3,8-diazabicyclo[3.2.1]octane-8-carboxylate

Under ice bath, sodium hydride (5 mg, 0.13 mmol) was added to a solution of compound (*S*,*E*)-(4-(fluoromethylene)-1,3-dimethylpiperidin-3-yl)methanol (Intermediate **A1**, 17 mg, 0.09 mol) in anhydrous tetrahydrofuran (2 mL). The temperature was maintained and the reaction was stirred for 0.5 hours, then tert-butyl (1*R*,5*S*)-3-(8-fluoro-7-(7-fluoro-8-((triisopropylsilyl)ethynyl)-3-((triisopropylsilyl)oxy)naphthalen-1-yl)-2-(methylsulfonyl)pyrido[4,3-*d*]pyrimidin-4-yl)-3,8-diazabicyclo[3.2.1]octane-8-carboxylate (Intermediate **A7,** 60 mg, 0.06 mmol) was added. The resulting reaction solution was stirred at room temperature for 2 hours. After the reaction was complete as monitored by LCMS, the reaction solution was poured into 30 mL of ice water to quench, extracted with ethyl acetate. The organic layers were combined, washed with saturated brine, dried over anhydrous sodium sulfate, filtered, and concentrated under reduced pressure to give crude tert-butyl (1*R*,5*S*)-3-(8-fluoro-7-(7-fluoro-8-((triisopropylsilyl)ethynyl)-3-((triisopropylsilyl)oxy)naphthalen-1-yl)-2-(((*S*,*E*)-4-(fluoromethylene)-1,3-dimethylpiperidin-3-yl)methoxy)pyrido[4,3-*d*]pyrimidin-4-yl)-3,8-15 diazabicyclo[3.2.1]octane-8-carboxylate (50 mg, 48% yield). MS-ESI [M+H]⁺: 1027.5.

### Step B: 4-((1R,5S)-3,8-diazabicyclo[3.2.1]octan-3-yl)-8-fluoro-7-(7-fluoro-8-((triisopropylsilyl)ethynyl)-3-((triisopropylsilyl)oxy)naphthalen-1-yl)-2-(((S,E)-4-(fluoromethylene)-1,3-dimethylpiperidin-3-yl)methoxy)pyrido[4,3-d]pyrimidine

At room temperature, a solution of hydrochloric acid in 1,4-dioxane (4M, 0.5 mL) was added to a solution of tert-butyl (1*R*,5*S*)-3-(8-fluoro-7-(7-fluoro-8-((triisopropylsilyl)ethynyl)-3-((triisopropylsilyl)oxy)naphthalen-1-yl)-2-(((*S*,*E*)-4-(fluoromethylene)-1,3-dimethylpiperidin-3-yl)methoxy)pyrido[4,3-*d*]pyrimidin-4-yl)-3,8-diazabicyclo[3.2.1]octane-8-carboxylate (50 mg, 0.05 mmol) in dichloromethane (0.5 mL). The reaction solution was stirred at room temperature for 1 hour. After the reaction was complete as monitored by LCMS, the reaction solution was concentrated to dryness to give the crude product 4-((1*R*,5*S*)-3,8-diazabicyclo[3.2.1]octan-3-yl)-8-fluoro-7-(7-fluoro-8-((triisopropylsilyl)ethynyl)-3-((triisopropylsilyl)oxy)naphthalen-1-yl)-2-(((*S*,*E*)-4-(fluoromethylene)-1,3-dimethylpiperidin-3-yl)methoxy)pyrido[4,3-*d*]pyrimidine (30 mg). MS-ESI [M+H]⁺: 927.4.

### Step C: 4-(4-((1R,5S)-3,8-diazabicyclo[3.2.1]octan-3-yl)-8-fluoro-2-(((S,E)-4-(fluoromethylene)-1,3-dimethylpiperidin-3-yl)methoxy)pyrido[4,3-d]pyrimidin-7-yl)-5-ethynyl-6-fluoronaphthalen-2-ol

At room temperature, cesium fluoride (49 mg, 0.33 mmol) was added to a solution of 4-((1*R*,5*S*)-3,8-diazabicyclo[3.2.1]octan-3-yl)-8-fluoro-7-(7-fluoro-8-((triisopropylsilyl)ethynyl)-3-((triisopropylsilyl)oxy)naphthalen-1-yl)-2-(((*S*,*E*)-4-(fluoromethylene)-1,3-dimethylpiperidin-3-yl)methoxy)pyrido[4,3-*d*]pyrimidine (30 mg, 0.03 mmol) in DMF (2 mL). The resulting reaction solution was stirred at 50 °C for 2 hours. After the reaction was complete as monitored by LCMS, the reaction solution was purified by pre-HPLC (C18, ACN/(10 mmol NH₄HCO₃/H₂O) = 30-70%) to give 4-(4-((1*R*,5*S*)-3,8-diazabicyclo[3.2.1]octan-3-yl)-8-fluoro-2-(((*S*,*E*)-4-(fluoromethylene)-1,3-dimethylpiperidin-3-yl)methoxy)pyrido[4,3-*d*]pyrimidin-7-yl)-5-ethynyl-6-fluoronaphthalen-2-ol **(P2,** 2 mg, 10% yield) as a white solid. ¹H NMR (400 MHz, Methanol-*d₄*) δ 8.99 (s, 1H), 7.85 (dd, *J* = 9.2, 5.7 Hz, 1H), 7.38-7.27 (m, 2H), 7.24-7.15 (m, 1H), 6.64 (d, *J* = 86.3 Hz, 1H), 4.68-4.55 (m, 3H), 4.44-4.32 (m, 1H), 3.75-3.63 (m, 3H), 3.33-3.31 (m, 1H), 2.93-2.83 (m, 1H), 2.83-2.74 (m, 1H), 2.71-2.62 (m, 1H), 2.38-2.27 (m, 1H), 2.25 (s, 3H), 2.03-1.77 (m, 6H), 1.20 (s, 3H). ¹⁹F NMR (376 MHz, Methanol-*d*₄) δ -111.74, -139.28 - -139.52. MS-ESI [M+H]⁺: 616.2.

### Preparation of Payload Molecule P3: 4-(4-((1R,5S)-3,8-diazabicyclo[3.2.1]octan-3-yl)-2-(((3S,4S)-4-(difluoromethyl)-1,3-dimethylpiperidin-3-yl)methoxy)-8-fluoro-5-methoxypyrido[4,3-d]pyrimidin-7-yl)-5-ethynyl-6-fluoronaphthalen-2-ol

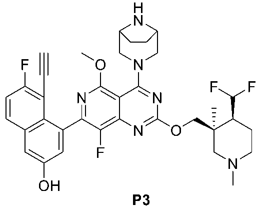

Payload molecule **P3** was prepared following the synthetic route for payload molecule **P2,** using intermediate **A8** (tert-butyl (1*R*,5*S*)-3-(8-fluoro-7-(7-fluoro-8-((triisopropylsilyl)ethynyl)-3-((triisopropylsilyl)oxy)naphthalen-1-yl)-5-methoxy-2-(methylsulfonyl)pyrido[4,3-*d*]pyrimidin-4-yl)-3,8-diazabicyclo[3.2.1]octane-8-carboxylate) instead of intermediate **A7** (tert-butyl (1*R*,5*S*)-3-(8-fluoro-7-(7-fluoro-8-((triisopropylsilyl)ethynyl)-3-((triisopropylsilyl)oxy)naphthalen-1-yl)-2-(methylsulfonyl)pyrido[4,3-*d*]pyrimidin-4-yl)-3,8-diazabicyclo[3.2.1]octane-8-carboxylate), and using intermediate **A2** ((3*S*,4*S*)-4-(difluoromethyl)-1,3-dimethylpiperidin-3-yl)methanol instead of intermediate **A1** (*S*,*E*)-(4-(fluoromethylene)-1,3-dimethylpiperidin-3-yl)methanol. ¹H NMR (400 MHz, DMSO-*d*₆) δ 10.17 (s, 1H), 7.97 (dd, *J* = 9.2, 5.9 Hz, 1H), 7.52-7.43 (m, 1H), 7.38 (d, *J* = 2.6 Hz, 1H), 7.22 (d, *J* = 2.5 Hz, 1H), 6.46-6.12 (m, 1H), 4.49-4.35 (m, 2H), 4.17-4.02 (m, 1H), 4.01-3.77 (m, 5H), 3.52-3.45 (m, 2H), 3.45-3.38 (m, 2H), 2.91-2.75 (m, 2H), 2.11 (s, 3H), 1.90-1.49 (m, 9H), 1.11 (s, 3H). ¹⁹F NMR (376 MHz, DMSO-*d*₆) δ -110.49, -115.86, -119.02, -149.80. MS-ESI [M+H]⁺: 665.3.

### Preparation of Payload Molecule P4: 4-(4-((1R,5S)-3,8-diazabicyclo[3.2.1]octan-3-yl)-8-fluoro-2-(((S,E)-4-(fluoromethylene)-1,3-dimethylpiperidin-3-yl)methoxy)-5-methoxypyrido[4,3-d]pyrimidin-7-yl)-5-ethynyl-6-fluoronaphthalen-2-ol

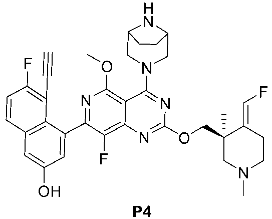

Payload molecule **P4** was prepared following the synthetic route for payload molecule **P2,** using intermediate **A8** (tert-butyl (1*R*,5*S*)-3-(8-fluoro-7-(7-fluoro-8-((triisopropylsilyl)ethynyl)-3-((triisopropylsilyl)oxy)naphthalen-1-yl)-5-methoxy-2-(methylsulfonyl)pyrido[4,3-*d*]pyrimidin-4-yl)-3,8-diazabicyclo[3.2.1]octane-8-carboxylate) instead of intermediate **A7** (tert-butyl (1*R*,5*S*)-3-(8-fluoro-7-(7-fluoro-8-((triisopropylsilyl)ethynyl)-3-((triisopropylsilyl)oxy)naphthalen-1-yl)-2-(methylsulfonyl)pyrido[4,3-*d*]pyrimidin-4-yl)-3,8-diazabicyclo[3.2.1]octane-8-carboxylate). ¹H NMR (400 MHz, Methanol-*d*₄) δ 7.82 (dd, *J* = 9.2, 5.7 Hz, 1H), 7.32-7.25 (m, 2H), 7.24-7.19 (m, 1H), 6.78-6.46 (m, 1H), 4.61 (dd, *J* = 10.5, 2.7 Hz, 1H), 4.44-4.06 (m, 3H), 3.99 (s, 3H), 3.64-3.42 (m, 4H), 3.28-3.26 (m, 1H), 2.91-2.84 (m, 1H), 2.83-2.75 (m, 1H), 2.71-2.61 (m, 1H), 2.33-2.20 (m, 4H), 2.05-1.91 (m, 2H), 1.88-1.61 (m, 4H), 1.19 (s, 3H). ¹⁹F NMR (376 MHz, Methanol-*d*₄) δ -111.90, -139.31, -150.93. MS-ESI [M+H]⁺: 645.3.

### Preparation of Payload Molecule P5: 4-(4-((1R,5S)-3,8-diazabicyclo[3.2.1]octan-3-yl)-5-ethynyl-8-fluoro-2-(((S,E)-4-(fluoromethylene)-1,3-dimethylpiperidin-3-yl)methoxy)pyrido[4,3-d]pyrimidin-7-yl)-5-ethynyl-6-fluoronaphthalen-2-ol

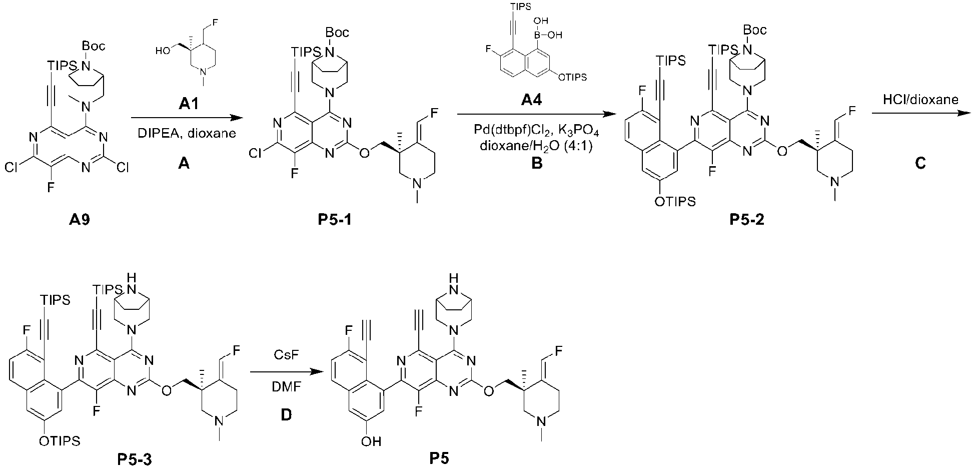

### Step A: tert-butyl (1R,5S)-3-(7-chloro-8-fluoro-2-(((S,E)-4-(fluoromethylene)-1,3-dimethylpiperidin-3-yl)methoxy)-5-((triisopropylsilyl)ethynyl)pyrido[4,3-d]pyrimidin-4-yl)-3,8-diazabicyclo[3.2.1]octane-8-carboxylate

At room temperature, tert-butyl (1*R*,5*S*)-3-(2,7-dichloro-8-fluoro-5-((triisopropylsilyl)ethynyl)pyrido[4,3-*d*]pyrimidin-4-yl)-3,8-diazabicyclo[3.2.1]octane-8-carboxylate (Intermediate **A9,** 960 mg, 1.58 mmol) was dissolved in anhydrous dioxane (10 mL), and (*S*,*E*)-(4-(fluoromethylene)-1,3-dimethylpiperidin-3-yl)methanol (Intermediate **A1**, 546 mg, 3.15 mmol) and DIPEA (612 mg, 4.73 mmol) were added. The resulting mixture was heated to 100 °C and stirred overnight. The reaction was quenched by adding 30 mL of saturated ammonium chloride solution, and extracted with ethyl acetate (3 × 30 mL). The organic phases were combined, washed with saturated brine, dried over anhydrous sodium sulfate, filtered, and concentrated. The crude product was purified by FCC (SiO₂, EA/PE = 0-100%) to give tert-butyl (1*R*,5*S*)-3-(7-chloro-8-fluoro-2-(((*S*,*E*)-4-(fluoromethylene)-1,3-dimethylpiperidin-3-yl)methoxy)-5-((triisopropylsilyl)ethynyl)pyrido[4,3-*d*]pyrimidin-4-yl)-3,8-diazabicyclo[3.2.1]octane-8-carboxylate (270 mg, 23% yield) as a white solid. MS-ESI [M+H]⁺: 745.4.

### Step B: tert-butyl (1R,5S)-3-(8-fluoro-7-(7-fluoro-8-((triisopropylsilyl)ethynyl)-3-((triisopropylsilyl)oxy)naphthalen-1-yl)-2-(((S,E)-4-(fluoromethylene)-1,3-dimethylpiperidin-3-yl)methoxy)-5-((triisopropylsilyl)ethynyl)pyrido[4,3-d]pyrimidin-4-yl)-3,8-diazabicyclo[3.2.1]octane-8-carboxylate

At room temperature, tert-butyl (1*R*,5*S*)-3-(7-chloro-8-fluoro-2-(((*S*,*E*)-4-(fluoromethylene)-1,3-dimethylpiperidin-3-yl)methoxy)-5-((triisopropylsilyl)ethynyl)pyrido[4,3-*d*]pyrimidin-4-yl)-3,8-diazabicyclo[3.2.1]octane-8-carboxylate (120 mg, 0.161 mmol), (7-fluoro-8-((triisopropylsilyl)ethynyl)-3-((triisopropylsilyl)oxy)naphthalen-1-yl)boronic acid (Intermediate **A4,** 131 mg, 0.241 mmol), potassium phosphate (103 mg, 0.483 mmol), and Pd(dtbpf)Cl₂ (15.6 mg, 0.024 mmol) were added to a reaction flask, and the atmosphere was replaced with nitrogen three times. 1,4-Dioxane (4 mL) and water (1 mL) were added, and the resulting mixture was heated to 100 °C and stirred overnight. The system was cooled to room temperature, quenched with 30 mL of saturated ammonium chloride solution, and extracted with ethyl acetate (3 × 30 mL). The organic phases were combined, washed with saturated brine, dried over anhydrous sodium sulfate, filtered, and concentrated. The crude product was purified by FCC (SiO₂, EA/PE = 0-100%) to give tert-butyl (1*R*,5*S*)-3-(8-fluoro-7-(7-fluoro-8-((triisopropylsilyl)ethynyl)-3-((triisopropylsilyl)oxy)naphthalen-1-yl)-2-(((*S*,*E*)-4-(fluoromethylene)-1,3-dimethylpiperidin-3-yl)methoxy)-5-((triisopropylsilyl)ethynyl)pyrido[4,3-*d*]pyrimidin-4-yl)-3,8-diazabicyclo[3.2.1]octane-8-carboxylate (90 mg, 46% yield). MS-ESI [M/2+H]⁺: 604.4.

### Step C: 4-((1R,5S)-3,8-diazabicyclo[3.2.1]octan-3-yl)-8-fluoro-7-(7-fluoro-8-((triisopropylsilyl)ethynyl)-3-((triisopropylsilyl)oxy)naphthalen-1-yl)-2-(((S,E)-4-(fluoromethylene)-1,3-dimethylpiperidin-3-yl)methoxy)-5-((triisopropylsilyl)ethynyl)pyrido[4,3-d]pyrimidine

At room temperature, tert-butyl (1*R*,5*S*)-3-(8-fluoro-7-(7-fluoro-8-((triisopropylsilyl)ethynyl)-3-((triisopropylsilyl)oxy)naphthalen-1-yl)-2-(((*S*,*E*)-4-(fluoromethylene)-1,3-dimethylpiperidin-3-yl)methoxy)-5-((triisopropylsilyl)ethynyl)pyrido[4,3-*d*]pyrimidin-4-yl)-3,8-diazabicyclo[3.2.1]octane-8-carboxylate (90 mg, 0.075 mmol) was dissolved in hydrochloric acid in 1,4-dioxane (4M, 20 mL, 80 mmol), and the resulting reaction solution was stirred at room temperature for 1 hour. After the reaction was complete as monitored by LCMS, the mixture was concentrated to dryness, and the residue was neutralized with saturated sodium bicarbonate solution (20 mL) and extracted with ethyl acetate (3 × 30 mL). The organic phase was dried over anhydrous sodium sulfate, filtered, and concentrated to give crude 4-((1*R*,5*S*)-3,8-diazabicyclo[3.2.1]octan-3-yl)-8-fluoro-7-(7-fluoro-8-((triisopropylsilyl)ethynyl)-3-((triisopropylsilyl)oxy)naphthalen-1-yl)-2-(((*S*,*E*)-4-(fluoromethylene)-1,3-dimethylpiperidin-3-yl)methoxy)-5-((triisopropylsilyl)ethynyl)pyrido[4,3-*d*]pyrimidine (80 mg, 97% yield) as a yellow solid. MS-ESI [M/2+H]⁺: 554.3.

### Step D: 4-(4-((1R,5S)-3,8-diazabicyclo[3.2.1]octan-3-yl)-5-ethynyl-8-fluoro-2-(((S,E)-4-(fluoromethylene)-1,3-dimethylpiperidin-3-yl)methoxy)pyrido[4,3-d]pyrimidin-7-yl)-5-ethynyl-6-fluoronaphthalen-2-ol

A solution of 4-((1*R*,5*S*)-3,8-diazabicyclo[3.2.1]octan-3-yl)-8-fluoro-7-(7-fluoro-8-((triisopropylsilyl)ethynyl)-3-((triisopropylsilyl)oxy)naphthalen-1-yl)-2-(((*S*,*E*)-4-(fluoromethylene)-1,3-dimethylpiperidin-3-yl)methoxy)-5-((triisopropylsilyl)ethynyl)pyrido[4,3-*d*]pyrimidine (80 mg, 0.072 mmol) and cesium fluoride (55 mg, 0.361 mmol) in DMF (3 mL) was heated to 60 °C and stirred for 1 hour. After the reaction was complete as monitored by LCMS, the insoluble material was filtered off, and the filtrate was collected and separated by pre-HPLC (C18, ACN/(0.1% NH₄HCO₃/H₂O) = 40-60%) to give 4-(4-((1*R*,5*S*)-3,8-diazabicyclo[3.2.1]octan-3-yl)-5-ethynyl-8-fluoro-2-(((*S*,*E*)-4-(fluoromethylene)-1,3-dimethylpiperidin-3-yl)methoxy)pyrido[4,3-*d*]pyrimidin-7-yl)-5-ethynyl-6-fluoronaphthalen-2-ol **(P5,** 30 mg, 65% yield) as a white solid. ¹H NMR (400 MHz, DMSO-*d*₆) δ 10.19 (s, 1H), 8.00 (dd, *J* = 9.2, 5.9 Hz, 1H), 7.5-7.45 (m, 1H), 7.41 (d, *J* = 2.6 Hz, 1H), 7.2-7.18 (m, 1H), 6.81 (d, *J* = 5.6 Hz, 0.5H), 6.60 (d, *J* = 4.8 Hz, 0.5H), 4.77 (s, 1H), 4.61 (dd, *J* = 13.6, 10.6 Hz, 1H), 4.30 (d, *J* = 10.6 Hz, 1H), 3.9-3.70 (m, 2H), 3.60-3.48 (m, 2H), 3.4-3.33 (m, 2H), 3.29 (s, 1H), 2.7-2.63 (m, 2H), 2.56-2.52 (m, 1H), 2.28-2.17 (m, 1H), 2.14 (s, 3H), 1.94-1.78 (m, 2H), 1.69-1.28 (m, 4H), 1.10 (d, *J* = 2.6 Hz, 3H). ¹⁹F NMR (376 MHz, DMSO-*d*₆) δ -110.44, -138.72, -138.74. MS-ESI [M+H]⁺: 639.3.

### Preparation of Payload Molecule P6: (S)-4-(4-((1R,5S)-3,8-diazabicyclo[3.2.1]octan-3-yl)-2-(((3S,4S)-4-(difluoromethyl)-3-methyl-1-(methyl-d₃)piperidin-3-yl)methoxy-d₂)-8-fluoro-6-(trifluoromethyl)quinazolin-7-yl)-2-aminobenzo[b]thiophene-3-carbonitrile or (R)-4-(4-((1R,5S)-3,8-diazabicyclo[3.2.1]octan-3-yl)-2-(((3S,4S)-4-(difluoromethyl)-3-methyl-1-(methyl-d₃)piperidin-3-yl)methoxy-d₂)-8-fluoro-6-(trifluoromethyl)quinazolin-7-yl)-2-aminobenzo[b]thiophene-3-carbonitrile

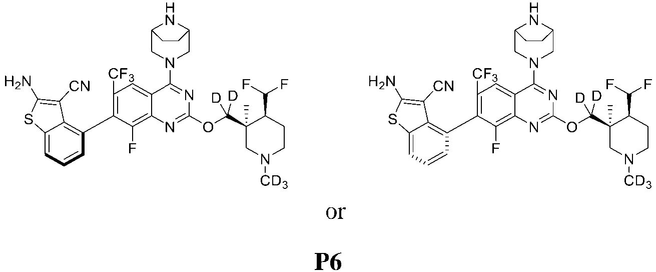

Payload molecule **P6** was prepared following the synthetic route for payload molecule **P2,** using intermediate **A10** (tert-butyl (1*R*,5*S*)-3-(7-(2-((tert-butoxycarbonyl)amino)-3-cyanobenzo[*b*]thiophen-4-yl)-6-chloro-2,8-difluoroquinazolin-4-yl)-3,8-diazabicyclo[3.2.1]octane-8-carboxylate) instead of intermediate **A7** (tert-butyl (1*R*,5*S*)-3-(8-fluoro-7-(7-fluoro-8-((triisopropylsilyl)ethynyl)-3-((triisopropylsilyl)oxy)naphthalen-1-yl)-2-(methylsulfonyl)pyrido[4,3-*d*]pyrimidin-4-yl)-3,8-diazabicyclo[3.2.1]octane-8-carboxylate), and using intermediate **A3** (*S,E*)-(4-(fluoromethylene)-3-methyl-1-(methyl-*d₃*)piperidin-3-yl)methylene-d₂-ol instead of intermediate **A1** (*S*,*E*)-(4-(fluoromethylene)-1,3-dimethylpiperidin-3-yl)methanol. ¹H NMR (400 MHz, DMSO-*d*₆) δ 8.06 (s, 1H), 7.83-7.72 (m, 3H), 7.27-7.14 (m, 2H), 6.46-6.13 (m, 1H), 4.34-4.26 (m, 2H), 3.63-3.50 (m, 4H), 2.88-2.75 (m, 2H), 1.89-1.50 (m, 9H), 1.11 (s, 3H). ¹⁹F NMR (376 MHz, DMSO-*d*₆) δ -57.27 (3F), -115.88, -119.02, -123.88. MS-ESI [M+H]⁺: 695.3.

### Preparation of Payload Molecule P7: 4-(4-((1R,5S)-3,8-diazabicyclo[3.2.1]octan-3-yl)-2-(((3S,4S)-4-(difluoromethyl)-1,3-dimethylpiperidin-3-yl)methoxy)-5-ethynyl-8-fluoropyrido[4,3-d]pyrimidin-7-yl)-5-ethynyl-6-fluoronaphthalen-2-ol

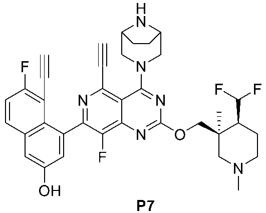

Payload molecule **P7** was prepared following the synthetic route for payload molecule **P5,** using intermediate **A2** ((3*S*,4*S*)-4-(difluoromethyl)-1,3-dimethylpiperidin-3-yl)methanol instead of intermediate **A1** (*S*,*E*)-(4-(fluoromethylene)-1,3-dimethylpiperidin-3-yl)methanol. MS-ESI [M+H]⁺: 659.3.

### Preparation of Payload Molecule P8: N,N-dimethyl-(S)-5-(2-{[(S)-(E)-4-fluoromethylene-1-ethyl-3-methylpiperidin-3-yl]methoxy}-7-(2-amino-3-cyano-5-fluoro-1-benzothiophen-4-yl)-8-fluoro-6-(trifluoromethyl)quinazolin-4-yl)-3-(methoxymethyl)-6-methyl-5,6,7,8-tetrahydro-4H-1,5,8a-triazazolin-2-carboxamide

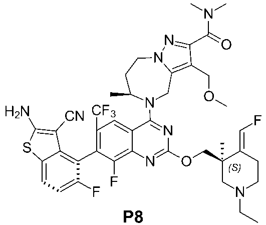

Payload **P8** was prepared using intermediate **A18** instead of intermediate **A19** in the synthesis method for **P9.** ¹H NMR (400 MHz, DMSO-*d*₆) δ 8.04 (s, 1H), 7.95 (s, 2H), 7.84 (dd, *J* = 8.7, 4.9 Hz, 1H), 7.12 (t, *J* = 9.1 Hz, 1H), 6.80 (s, 0.5H), 6.58 (s, 0.5H), 5.14 (d, *J* = 16.6 Hz, 1H), 4.92-4.67 (m, 2H), 4.62-4.45 (m, 2H), 4.44-4.32 (m, 2H), 4.31-4.23 (m, 1H), 4.20-4.06 (m, 1H), 3.24 (s, 3H), 3.06 (s, 3H), 2.96 (s, 3H), 2.85-2.70 (m, 2H), 2.59-2.53 (m, 1H), 2.44-2.24 (m, 4H), 2.17-2.02 (m, 1H), 1.99-1.77 (m, 2H), 1.64 (d, *J* = 6.2 Hz, 3H), 1.08 (s, 3H), 0.95 (t, *J* = 7.1 Hz, 3H). ¹⁹F NMR (376 MHz, DMSO-*d*₆) δ -59.39, -117.50, -122.29, -139.05. MS-ESI [M+H]⁺: 856.3.

### Preparation of Payload Molecule P9: N,N-dimethyl-(S)-5-(2-{[(S)-(E)-4-fluoromethylene-1-ethyl-3-methylpiperidin-3-yl]methoxy}-7-(2-amino-3-cyano-5-fluoro-1-benzothiophen-4-yl)-6-chloro-8-fluoroquinazolin-4-yl)-3-(methoxymethyl)-6-methyl-5,6,7,8-tetrahydro-4H-1,5,8a-triazazolin-2-carboxamide

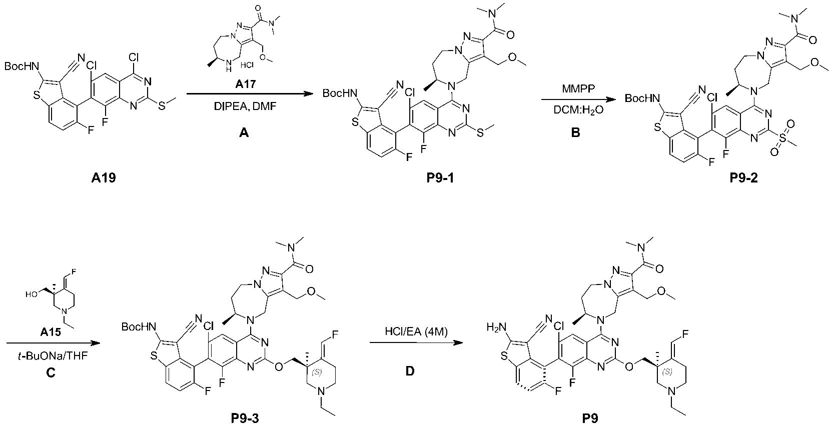

**Step A:** At room temperature, **A17** (163 mg, 0.54 mmol), **A19** (150 mg, 0.27 mmol), and DIEA (350 mg, 2.7 mmol) were sequentially added to DMF (4 mL). After replacing with N₂ three times, the mixture was warmed to 50 °C and stirred for 16 hours. The reaction was complete as monitored by LCMS and TLC. The reaction solution was returned to room temperature, water (30 mL) was added, and the mixture was extracted with EA (3 × 30 mL). The organic phase was collected, washed with saturated brine, dried over anhydrous Na₂SO₄, filtered, concentrated, and purified by FCC (SiO₂, EA/PE = 0-100%) to give **P9-1** (100 mg) as a pale yellow solid. MS-ESI [M+H]⁺: 783.2.

**Step B:** At room temperature, magnesium monoperoxyphthalate hexahydrate (316 mg, 0.64 mmol) was added to a solution of **P9-1** (100 mg, 0.13 mmol) in DCM/H₂O (5 mL, V/V = 1/1), and the mixture was stirred at room temperature for 16 hours. After the reaction was complete as monitored by LCMS, the reaction solution was poured into NaHCO₃ solution (30 mL) to quench, and extracted with DCM (3 × 30 mL). The organic phase was collected, washed with saturated brine, dried over anhydrous Na₂SO₄, and concentrated to give **P9-2** (110 mg, crude), which was used directly in the next step. MS-ESI [M+H]⁺: 815.2.

**Step C:** Under a dry ice-ethanol bath, t-BuONa (0.33 mL, 2M in THF, 0.65 mmol) was added to a mixed solution of compound **P9-2** (110 mg), intermediate **A15** (50 mg, 0.26 mmol), and THF (4 mL). The temperature was maintained for 10 minutes, then the mixture was warmed to room temperature and stirred for 30 minutes. After the reaction was complete as monitored by LCMS, the reaction solution was poured into half-saturated aqueous NH₄Cl (20 mL) and extracted with EA (3 × 30 mL). The organic phase was collected, washed with saturated brine, dried over anhydrous Na₂SO₄, filtered, and concentrated to give **P9-3** (120 mg, crude). MS-ESI [M+H]⁺: 922.3.

**Step D:** At room temperature, 4M HCl-ethyl acetate (5 mL, 20 mmol) was added to **P9-3** (120 mg) and stirred at room temperature for 1 hour. After the reaction was complete as monitored by LCMS, the mixture was concentrated, saturated sodium bicarbonate aqueous solution (20 mL) was added, and the mixture was extracted with EA (2 × 30 mL). The extract was collected, washed with brine (40 mL), and dried over anhydrous Na₂SO₄, filtered, and concentrated. and purification by pre-HPLC (C18, ACN/(10 mmol FA/H₂O) = 30-38%) gave the first eluted white solid isomer 1 as **P9** (21.10 mg, Rt = 8.75 min), chiral analysis Rt = 2.202 min. Further purification by SFC (Waters SFC 150, column: DAICEL CHIRALPAK^{®}IH, 250 * 30 mm, 10 µm, mobile phase: CO₂/MeOH (0.2% 7.0M NH₃-MeOH) = 75/25; flow rate: 140 mL/min) removed minor non-isomeric impurities to give **P9** (21.10 mg) as a white solid. ¹H NMR (400 MHz, DMSO-*d*₆) δ 7.99 (s, 2H), 7.85 (dd, *J* = 8.8,4.8 Hz, 1H),7.74 (s, 1H), 7.14 (t, *J* =9.2Hz, 1H), 6.80 (s, 0.5H), 6.58 (s, 0.5H), 5.12 (d, *J* =16.8 Hz, 1H), 4.86-4.80 (m,2H), 4.72 (d, *J* =16.0Hz, 1H), 4.62-4.52 (m, 2H), 4.40-4.35 (m, 1H), 4.72 (d, *J* =10.4Hz, 1H), 4.16-4.08 (m,1H), 3.23 (s, 3H), 3.07 (s, 3H), 2.96 (s, 3H), 2.79-2.73 (m, 2H), 2.54-2.48 (m, 1H), 2.35-2.27 (m, 4H), 2.15-2.07 (m, 1H),1.95-1.87 (m, 1H), 1.82 (d, *J* =11.2 Hz, 1H), 1.56(d, *J* =6.0 Hz, 3H), 1.08 (s, 3H), 0.95 (t, *J* =7.2 Hz, 3H). ¹⁹F NMR (376 MHz, DMSO-*d*₆) δ - 118.67, -120.61, -139.16. MS-ESI [M+H]⁺: 822.3.

Chiral analysis method: Waters UPCC (CA-352), analytical column: DAICEL CHIRALPAK^{®}IH, 100 * 3 mm, 3 µm; Mobile phase A: CO₂, Mobile phase B: MeOH (0.1% DEA); Flow rate: 1.5 mL/min; Column temperature: 35 °C; Back pressure: 1800 psi; Gradient: 0-8.0 min, A/B = 75/25.

### Preparation of Payload Molecule P10: 4-[(2R,4R,7S,8S,9S)-17-{[(2R,7aS)-2-fluorohexahydro-1H-pyrrolizin-7a-yl]methoxy}-14-fluoro-9-methyl-10-oxa-2,12,16,18,20-pentaazapentacyclo[9.7.1.^{14,7}.0^{2,8}.0^{15,19}]icosa-1(18),11(19),12,14,16-pentaen-13-yl]-5-ethynyl-6-fluoronaphthalen-2-ol

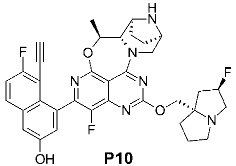

Payload **P10** was prepared following the synthetic method for compound **Z552** in WO2023030385. ¹H NMR (400 MHz, DMSO-*d*₆) δ 7.94 (m, 1H), 7.45 (q, *J* = 8.8 Hz, 1H), 7.37 (d, *J* = 2.6 Hz, 1H), 7.13 (dd, *J* = 51.8, 2.6 Hz, 1H), 5.28 (d, *J* = 54.2 Hz, 1H), 5.12 (d, *J* = 12.9 Hz, 1H), 4.51-4.42 (m, 1H), 4.19-3.89 (m, 4H), 3.58 (brs, 1H), 3.45 (brs, 1H), 3.08-3.00 (m, 4H), 2.87-2.76 (m, 1H), 2.12-1.94 (m, 3H), 1.88-1.61 (m, 7H), 1.43 (t, *J* = 6.6 Hz, 3H); ¹⁹F NMR (376 MHz, DMSO-*d*₆) δ -110.45 (d, *J* = 53.8 Hz, 1F), -145.47 (d, *J* = 54.1 Hz, 1F), -171.87 (d, *J* = 31.1 Hz, 1F). MS-ESI [M+H]⁺: 643.0.

### Preparation of Payload Molecule P11: 5-[(2R,4R,7S,8S,9S)-17-{[(7aS)-2-difluoromethylenehexahydro-1H-pyrrolizin-7a-yl]methoxy}-14-fluoro-9-methyl-10-oxa-2,12,16,18,20-pentaazapentacyclo[9.7.1.1^{4,7}.0^{2,8}.0^{15,19}]icosa-1(18),11(19),12,14,16-pentaen-13-yl]-3-chloro-4-(trifluoromethyl)aniline

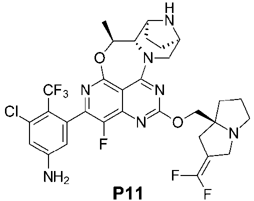

Payload **P11** was prepared following the synthetic method for compound **Z1084** in WO2024061370. ¹H NMR (400 MHz, DMSO-*d*₆) δ 6.88 (d, *J* = 2.4 Hz, 1H), 6.44 (d, *J* = 64.4 Hz, 1H), 5.08 (d, *J* = 12.8 Hz, 1H), 4.53 (brs, 1H), 4.20-4.05 (m, 2H), 3.99 (brs, 1H), 3.66 (brs, 1H), 3.60-3.59 (m, 1H), 3.48 (d, J = 5.4 Hz, 1H), 3.32 (d, *J* = 14.4 Hz, 1H), 3.10-2.93 (m, 2H), 2.69-2.56 (m, 2H), 2.43-2.35 (m, 1H), 1.97-1.59 (m, 8H), 1.44 (d, J = 6.2 Hz, 3H). ¹⁹F NMR (376 MHz, DMSO-*d*₆) δ -53.35 (d, *J* = 106.4 Hz, 3F), -90.53 - -91.64 (m, 2F), -146.71 (d, *J* = 176.5 Hz, 1F). MS-ESI [M+H]⁺: 681.3.

### Preparation of Payload Molecule P12: 4-[(8S)-17-{[(2R,7aS)-2-fluorohexahydro-1H-pyrrolizin-7a-yl]methoxy}-14-fluoro-6,10-dioxa-2,12,16,18-tetraazatetracyclo[9.7.1.0^{2,8}.0^{15,19}]nonadeca-1(18),11(19),12,14,16-pentaen-13-yl]-5-ethynyl-6-fluoronaphthalen-2-ol

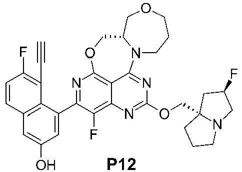

Payload **P12** was prepared following the synthetic method for compound **33** in WO2024009191. ¹H NMR (400 MHz, DMSO-*d*₆) δ 8.27 (s, 1H), 7.96 (dd, *J* = 9.2, 5.8 Hz, 1H), 7.47 (t, *J* = 9.0 Hz, 1H), 7.39 (d, *J* = 2.4 Hz, 1H), 7.18 (dd, *J* = 27.8, 2.6 Hz, 1H), 5.30 (d, *J* = 54.2 Hz, 1H), 5.01 (brs, 1H), 4.73-4.60 (m, 1H), 4.49 (d, *J* = 13.2 Hz, 1H), 4.33-4.28 (m, 1H), 4.18-4.10 (m, 2H), 4.09-3.86 (m, 3H), 3.59-3.53 (m, 1H), 3.49-4.34 (m, 2H), 3.14-3.05 (m, 3H), 2.88-2.82 (m, 1H), 2.16 (d, *J* = 4.8 Hz, 1H), 2.09-2.01 (m, 3H), 1.95-1.72 (m, 4H). ¹⁹F NMR (376 MHz, DMSO-*d₆*) δ - 110.50 (d, *J* = 51.5 Hz, 1F), -145.78 (d, *J* = 234.2 Hz, 1F), -172.00 (d, *J* = 35.5 Hz, 1F). MS-ESI [M+H]⁺: 618.0.

### Preparation of Payload Molecule P13: 4-(5-{(7S,13S)-7-[(1S,2S)-2-methylcyclobutamido]-21-ethyl-17,17-dimethyl-8,14-dioxo-15-oxa-4-thia-9,21,27,28-tetraazapentacyclo[17.5.2.1^{2,5}.1^{9,13}.0^{22,26}]octacosa-1(25),2,5(28),19,22(26),23-hexaen-20-yl}-6-[(S)-1-methoxyethyl]pyridin-3-yl)-1-methylpiperazine

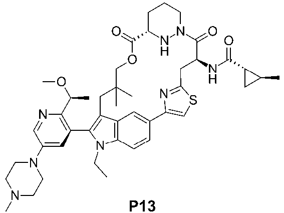

Payload **P13** is compound **A122** (RMC6236) from WO2022060836.

### Preparation of Payload Molecule P14: 3-[(4R,7S,8S)-17-{[(S)-(E)-4-fluoromethylene-1-ethyl-3-methylpiperidin-3-yl]methoxy}-14-fluoro-9-methyl-10-oxa-2,12,16,18,20-pentaazapentacyclo[9.7.1.1^{4,7}.0^{2,8}.0^{15,19}]icosa-1(18),11,13,15(19),16-pentaen-13-yl]-5-chloro-4-(trifluoromethyl)aniline

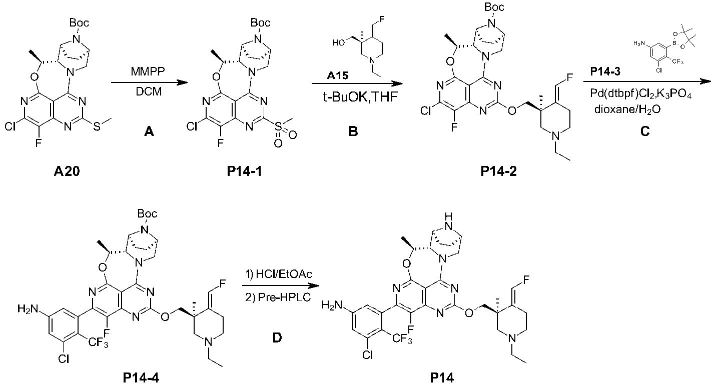

**Step A:** At room temperature, magnesium monoperoxyphthalate hexahydrate (MMPP, 1.67 mg, 85% purity, 2.87 mmol) was added to a solution of compound **A20** (277 mg, 0.575 mmol) in toluene and water (5 mL, 1:1). The temperature was maintained and stirred for 1 hour. After the reaction was complete as monitored by LCMS, 10 mL of water was added to the reaction solution, and extracted three times with ethyl acetate. The organic phases were combined, washed with saturated brine, dried over anhydrous sodium sulfate, filtered, and concentrated to dryness to give crude **P14-1** (270 mg) as a white solid. MS-ESI [M+H]⁺: 514.1.

**Step B:** Under a dry ice-ethanol bath (-70 °C), *t*-BuOK (1.05 mL, 1M in THF, 1.05 mmol) was added dropwise to a mixture of compound **P14-1** (135 mg), intermediate **A15** (98.4 mg, 0.525 mmol), and anhydrous THF (5 mL). The mixture was then allowed to warm to room temperature and stirred for 2 hours. After the reaction was complete as monitored by LCMS, the reaction was quenched with saturated NH₄Cl solution (20 mL) and extracted with EA (3 × 30 mL). The organic phase was collected, washed with saturated brine, dried over anhydrous Na₂SO₄, and concentrated to dryness to give crude **P14-2** (163 mg) as a white solid. MS-ESI [M+H]⁺: 621.3.

**Step C:** At room temperature, compound **P14-2** (163 mg), compound **P14-3** (175 mg, 0.545 mmol), Pd(dtbpf)Cl₂ (23.4 mg, 0.036 mmol), and K₃PO₄ (231 mg, 1.09 mmol) were sequentially added to a mixed solution of 1,4-dioxane and water (5 mL, 4:1). After replacing with N₂ three times, the mixture was warmed to 85 °C and stirred for 4 hours. After the reaction was complete as monitored by LCMS, the reaction was cooled to room temperature and concentrated directly to dryness. The crude product was purified by FCC (SiO₂, THF/PE = 0-100%) to give **P14-4** (70 mg) as a white solid. MS-ESI [M+H]⁺: 780.3.

**Step D:** At room temperature, 4M HCl-ethyl acetate (20 mL, 80 mmol) was added to compound **P14-4** (70 mg) and stirred at room temperature for 1 hour. After the reaction was complete as monitored by LCMS, the acid solution was removed by concentration, and the crude product was purified by pre-HPLC (C18, ACN/(0.1%FA/H₂O) = 15-40%) and lyophilized to give **P14** (4.2 mg) as a white solid. ¹H NMR (400 MHz, DMSO-*d*₆) δ 8.14 (s, 0.4H), 6.91-6.23 (m, 5H), 5.23 (dd, *J* = 14.3, 2.8 Hz, 1H), 4.78-4.66 (m, 1H), 4.66-4.54 (m, 1H), 4.37-4.26 (m, 1H), 4.26-4.17 (m, 1H), 4.17-4.09 (m, 1H), 4.04-3.92 (m, 1H), 2.92-2.73 (m, 2H), 2.64-2.52 (m, 4H), 2.43-2.16 (m, 2H), 2.09-1.72 (m, 5H), 1.46 (d, *J* = 6.3 Hz, 3H), 1.12 (s, 3H), 1.00 (s, 3H). MS-ESI [M+H]⁺: 680.2.

### Preparation of Payload Molecule P15: 3-[(2R,4R,7S,8S,9S)-17-{[(3S,4S)-4-(difluoromethyl)-1-ethyl-3-methylpiperidin-3-yl]methoxy}-14-fluoro-9-methyl-10-oxa-2,12,16,18,20-pentaazapentacyclo[9.7.1.1^{4,7}.0^{2,8}.0^{15,19}]icosa-1(18),11(19),12,14,16-pentaen-13-yl]-5-chloro-4-(trifluoromethyl) aniline

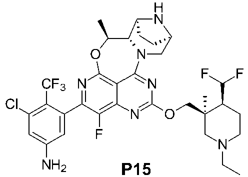

Payload **P15** was prepared using intermediate **A16** instead of intermediate **A15** in the synthesis method for **P14.** ¹H NMR (400 MHz, DMSO-*d*₆) δ 8.19 (s, 1H, HCOOH), 6.86 (s, 1H), 6.56-6.10 (m, 4H), 5.16-5.05 (m, 1H), 4.60-4.43 (m, 2H), 4.43-4.32 (m, 1H), 4.04-3.92 (m, 1H), 3.62-3.55 (m, 1H), 3.51-3.45 (m, 1H), 3.09-3.01 (m, 1H), 2.97-2.87 (m, 2H), 2.56-2.52 (m, 1H), 2.36-2.19 (m, 2H), 1.92-1.53 (m, 8H), 1.44 (d, *J* = 6.3 Hz, 3H), 1.12 (s, 3H), 0.91 (t, *J* = 7.1 Hz, 3H). ¹⁹F NMR (376 MHz, DMSO-*d*₆) δ -53.29 (s, 3F), -117.67 (d, *J* = 282.6 Hz), -117.67 (d, *J* = 282.6 Hz), -146.47 (s, 1F). MS-ESI [M+H]⁺: 699.9.

### Preparation of Payload Molecule P16: 4-((8S)-17-(((S)-(E)-4-fluoromethylene-1-ethyl-3-methylpiperidin-3-yl)methoxy)-14-fluoro-6,10-dioxa-2,12,16,18-tetraazatetracyclo(9.7.1.0^{2,8}.0^{15,19})nonadeca-1(18),11(19),12,14,16-pentaen-13-yl)-5-ethynyl-6-fluoronaphthalen-2-ol

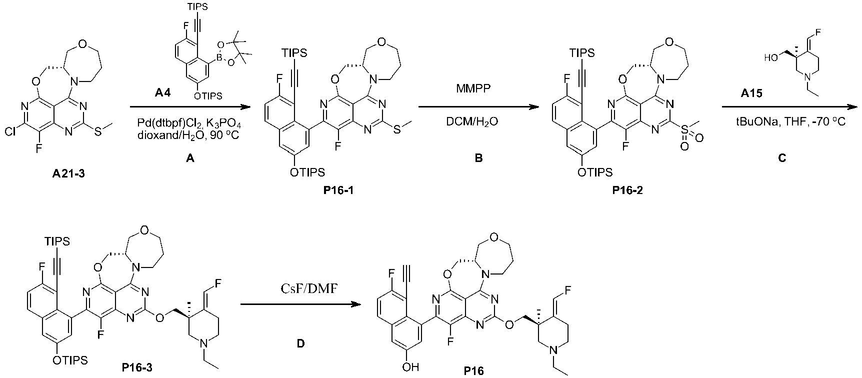

**Step A:** At room temperature under nitrogen, **A4** (1.89 g, 3.49 mmol), Pd(dtbpf)Cl₂ (0.15 g, 0.25 mmol), and potassium phosphate (1.59 g, 7.50 mmol) were added to a solution of compound **A21-3** (0.89 g, 2.50 mmol) in 1,4-dioxane (20 mL) and water (4 mL). The reaction solution was stirred at 100 °C for 6 hours. The reaction solution was filtered through Celite, and the filtrate was concentrated to dryness. The resulting crude product was purified by FCC (SiO₂, DCM = 100%) to give **P16-1** (1.70 g, 85% yield) as a pale yellow solid. MS-ESI [M+H]⁺: 819.3.

**Step B:** At room temperature, magnesium monoperoxyphthalate hexahydrate (4.20 g, 87% purity, 7.38 mmol) was added to a solution of intermediate **P16-1** (1.70 g, 2.08 mmol) in dichloromethane (20 mL) and water (20 mL). The reaction solution was stirred at room temperature for 16 hours. Washed once with water, dried over Na₂SO₄, and concentrated under reduced pressure to remove solvent, to give **P16-2** (1.76 g, 99% yield) as a white solid. MS-ESI [M+Na]⁺: 851.0.

**Step C:** Under nitrogen at -70 °C, 1M sodium tert-butoxide in THF (1.7 mL, 1.7 mmol) was added to a solution of compound **P16-2** (460 mg, 0.54 mmol) and **A15** (121 mg, 0.65 mmol) in anhydrous tetrahydrofuran (15 mL). The resulting reaction solution was stirred at -70 °C for 1 hour. After the reaction was complete as monitored by LCMS, the reaction solution was poured into saturated ammonium chloride solution (30 mL), extracted with ethyl acetate. The organic layers were combined, washed with saturated brine, dried over anhydrous sodium sulfate, filtered, and concentrated to dryness to give a crude product which was purified by FCC (SiO2, THF/PE = 0-50%) to give **P16-3** (450 mg, 87% yield) as a yellow solid. MS-ESI [M/2+H]⁺: 479.8.

**Step D:** At room temperature, cesium fluoride (228 mg, 1.50 mmol) was added to a solution of compound **P16-3** (70 mg, 0.15 mmol) in N,N-dimethylformamide (2 mL). The reaction solution was stirred at 50 °C for 1 hour. Purification by pre-HPLC (C18, ACN/(10 mmol NH₄HCO₃) = 50-55%) to give **A16** (28 mg, 59% yield) as a white solid. ¹H NMR (400 MHz, DMSO-*d*₆) δ 10.15 (s, 1H), 7.96 (dd, *J* = 9.2, 5.9 Hz, 1H), 7.51-7.42 (m, 1H), 7.41-7.32 (m, 1H), 7.23-7.09 (m, 1H), 6.71 (d, *J* = 86.8 Hz, 1H), 5.07-4.91 (m, 1H), 4.71-4.56 (m, 2H), 4.55-4.44 (m, 1H), 4.34-4.23 (m, 2H), 4.19-3.84 (m, 3H), 3.72-3.40 (m, 3H), 2.84-2.71 (m, 2H), 2.57-2.52 (m, 1H), 2.30 (q, *J* = 7.2 Hz, 2H), 2.23-2.00 (m, 2H), 1.98-1.80 (m, 3H), 1.11 (s, 3H), 0.97 (t, *J* = 7.2 Hz, 3H). ¹⁹F NMR (376 MHz, DMSO-*d*₆) δ -108.07 - -115.60 (m, 1F), -136.44 - -142.09 (m, 1F), -143.22 - -150.25 (m, 1F). MS-ESI [M+H]⁺: 646.0.

### Preparation of Payload Molecule P17: 4-[(8S)-17-{[(3S,4S)-4-(difluoromethyl)-1-ethyl-3-methylpiperidin-3-yl]methoxy}-14-fluoro-6,10-dioxo-2,12,16,18-tetraazatetracyclo[9.7.1.0^{2,8}.0^{15,19}]nonadeca-1(18),11(19),12,14,16-pentaen-13-yl]-5-ethynyl-6-fluoronaphthalen-2-ol

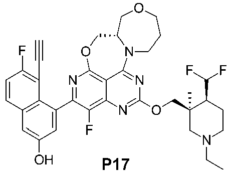

Payload **P17** was prepared using intermediate **A16** instead of intermediate **A15** in the synthesis method for **P16.** ¹H NMR (400 MHz, DMSO-*d*₆) δ 10.15 (s, 1H), 7.96 (dd, *J* = 9.2, 5.9 Hz, 1H), 7.50-7.41 (m, 1H), 7.39-7.31 (m, 1H), 7.22-7.10 (m, 1H), 6.29 (t, *J* = 55.6 Hz, 1H), 5.07-4.93 (m, 1H), 4.72-4.61 (m, 1H), 4.56-4.45 (m, 2H), 4.44-4.33 (m, 1H), 4.33-4.24 (m, 1H), 4.18-3.86 (m, 3H), 3.71-3.43 (m, 3H), 2.93 (d, *J* = 11.1 Hz, 2H), 2.33-2.21 (m, 2H), 2.14-2.00 (m, 1H), 1.96-1.55 (m, 6H), 1.13 (s, 3H), 0.98-0.84 (m, 3H). ¹⁹F NMR (376 MHz, DMSO-*d*₆) δ -108.82 - -112.59 (m), -113.72 - -121.50 (m), -142.47 - -148.37 (m). MS-ESI [M+H]^{+:} 666.0.

### Preparation of Payload Molecule P18: 3-[(8S)-17-{[(S)-(E)-4-fluoromethylene-1-ethyl-3-methylpiperidin-3-yl]methoxy}-14-fluoro-6,10-dioxa-2,12,16,18-tetraazatetracyclo[9.7.1.0^{2,8}.0^{15,19}]nonadeca-1(18),11(19),12,14,16-pentaen-13-yl]-5-chloro-4-(trifluoromethyl) aniline

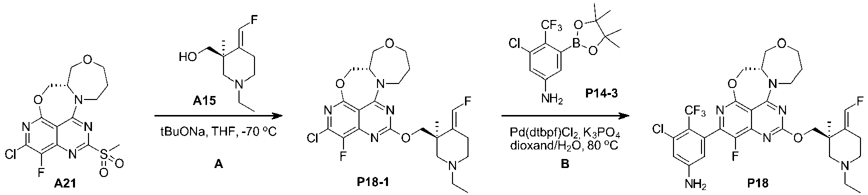

**Step A:** Under nitrogen at -70 °C, sodium tert-butoxide in THF (5.1 mL, 1M, 5.10 mmol) was added to a solution of compound **A21** (500 mg, 1.28 mmol) and **A15** (289 mg, 1.54 mmol) in anhydrous tetrahydrofuran (20 mL). The reaction solution was stirred at -70 °C for another 1 hour. After the reaction was complete as monitored by LCMS, the reaction solution was poured into saturated ammonium chloride solution (30 mL), extracted with ethyl acetate. The organic layers were combined, washed with saturated brine, dried over anhydrous sodium sulfate, filtered, and concentrated to dryness to give a crude product which was purified by FCC (SiO₂, THF/PE = 0-35%) to give **P18-1** (560 mg, 88% yield) as a yellow solid. MS-ESI [M+H]⁺: 496.2.

**Step B:** Under nitrogen at room temperature, **P14-3** (726 mg, 2.26 mmol), Pd(dtbpf)Cl₂ (71 mg, 0.11 mmol), and potassium phosphate (719 mg, 3.39 mmol) were added to a solution of compound **P18-1** (560 mg, 1.13 mmol) in 1,4-dioxane (15 mL) and water (3 mL). The reaction solution was stirred at 80 °C for 40 minutes. The reaction solution was filtered through Celite, and the filtrate was concentrated to dryness. The crude product was purified by FCC (SiO₂, THF/PE = 0-55%) to give 550 mg of a yellow solid. This yellow solid was further purified by pre-HPLC (C18, ACN/(10mM NH₄HCO₃) = 45-60%) to give **P18** (223 mg, 30% yield) as a white solid. ¹H NMR (400 MHz, DMSO-*d*₆) δ 6.85 (d, *J* = 2.3 Hz, 1H), 6.70 (d, *J* = 86.8 Hz, 1H), 6.56 -6.39 (m, 1H), 6.30 (s, 2H), 5.06-4.92 (m, 1H), 4.70-4.44 (m, 3H), 4.40-4.23 (m, 2H), 4.15-4.03 (m, 1H), 3.94-3.80 (m, 1H), 3.65-3.51 (m, 1H), 3.49-3.34 (m, 2H), 2.84-2.70 (m, 2H), 2.55-2.51 (m, 1H), 2.30 (q, *J* = 7.0 Hz, 2H), 2.24-2.09 (m, 1H), 2.08-1.80 (m, 4H), 1.11 (s, 3H), 0.97 (t, *J* = 7.1 Hz, 3H). ¹⁹F NMR (376 MHz, DMSO-*d*₆) δ -51.45- -55.22 (m, 1F), -139.04 (s, 1F), -146.62 (s, 1F). MS-ESI [M+H]⁺: 654.9.

### Preparation of Payload Molecule P19: 3-[(8S)-17-{[(3S,4S)-4-(difluoromethyl)-1-ethyl-3-methylpiperidin-3-yl]methoxy}-14-fluoro-6,10-dioxo-2,12,16,18-tetraazatetracyclo[9.7.1.0^{2,8}.0^{15,19}]nonadeca-1(18),11(19),12,14,16-pentaen-13-yl]-5-chloro-4-(trifluoromethyl)aniline

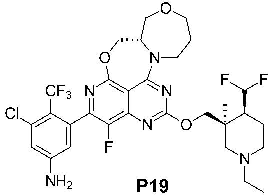

Payload **P19** was prepared using intermediate **A16** instead of intermediate **A15** and intermediate **P14-3** instead of intermediate **A4** in the synthesis method for **P16.** ¹H NMR (400 MHz, DMSO-*d*₆) δ 8.24 (s, 1H, HCOOH), 6.86 (d, *J* = 2.2 Hz, 1H), 6.55-6.11 (m, 4H), 5.05-4.94 (m, 1H), 4.71-4.59 (m, 1H), 4.56-4.44 (m, 2H), 4.44-4.35 (m, 1H), 4.35-4.22 (m, 1H), 4.16-4.03 (m, 1H), 3.94-3.82 (m, 1H), 3.63-3.53 (m, 1H), 3.48-3.32 (m, 2H), 2.98-2.85 (m, 2H), 2.35-2.19 (m, 2H), 2.09-1.95 (m, 1H), 1.95-1.58 (m, 6H), 1.12 (s, 3H), 0.92 (t, *J* = 7.1 Hz, 3H). ¹⁹F NMR (376 MHz, DMSO-*d*₆) δ -53.36 (s, 3F), -116.28 (d, *J* = 281.7 Hz), -119.11 (d, *J* = 281.6 Hz), -146.51 (s, 1F). MS-ESI [M+H]⁺: 674.9.

### 6.2 Preparation of Linker Units

### Preparation of Intermediate B1:

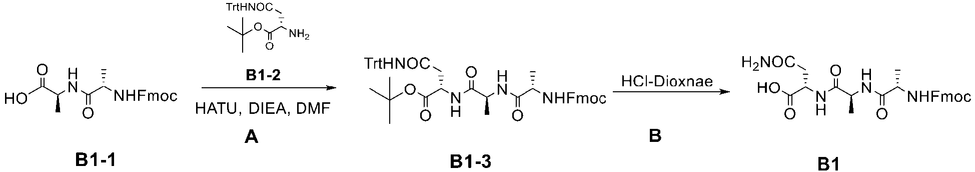

**Step A:** At 0 °C, HATU (3.87 g, 30.00 mmol) was slowly added to a solution of compound **B1-1** (3.82 g, 10.0 mmol) and compound **B1-2** (4.30 g, 10.0 mmol) in DMF (60 mL), followed by dropwise addition of N,N-diisopropylethylamine (20 mL). The resulting mixture was stirred at 25 °C for 3 hours. After the reaction was complete, the reaction solution was slowly added dropwise to ice water (600 mL), filtered, and the collected solid filter cake was dispersed in methyl tert-butyl ether (200 mL), stirred at room temperature for 10 minutes, filtered, and the filter cake was collected and dried under vacuum to give **B1-3** (7.1 g, 89.4% yield) as a white solid. MS-ESI [M+Na]⁺: 817.3.

**Step B:** At room temperature, compound **B1-3** (7.1 g, 8.93 mmol) was added to HCl-Dioxane (6M, 200 mL), and the resulting mixture was stirred at room temperature for 16 hours. After the reaction was complete, the reaction solution was concentrated. The residue was added to methyl tert-butyl ether (200 mL), stirred at room temperature for 10 minutes, filtered, and the filter cake was collected and dried under vacuum to give **B1** (4.0 g, 90.2% yield) as a white solid. MS-ESI [M+H]⁺: 497.2.

### Preparation of Intermediate B2:

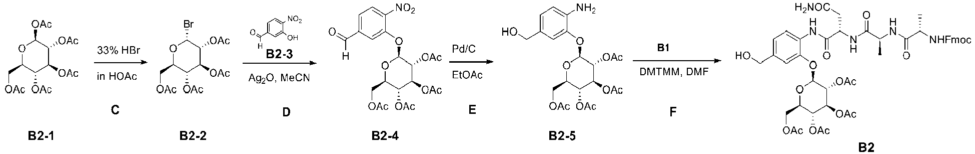

**Step** C: At 0 °C under nitrogen, HBr/HOAc solution (33%, 20 mL) was added to compound **B2-1** (5.0 g, 12.82 mmol) and stirred at room temperature for 14 hours. The mixture was concentrated to remove solvent and purified by flash column chromatography (ethyl acetate/petroleum ether = 0-30%) to give **B2-2** (5.0 g, 95.1% yield) as a white solid. ¹H NMR (400 MHz, Chloroform-*d*) δ 6.61 (d, *J* = 4.0 Hz, 1H), 5.56 (t, *J* = 9.7 Hz, 1H), 5.22-5.10 (m, 1H), 4.84 (dd, *J* = 10.0, 4.1 Hz, 1H), 4.39-4.26 (m, 2H), 4.18-4.13 (m, 1H), 2.11 (s, 3H), 2.10 (s, 3H), 2.06 (s, 3H), 2.04 (s, 3H). MS-ESI [M+Na]⁺: 433.0.

**Step D:** To a solution of compound **B2-2** (2.0 g, 4.88 mmol) in acetonitrile (80 mL) was added compound **B2-3** (1.63 g, 9.76 mmol), followed by silver oxide (4.52 g, 19.51 mmol) at room temperature. The mixture was stirred under nitrogen protection in the dark at room temperature for 16 hours. After the reaction was complete, the mixture was filtered, and the filtrate was concentrated to remove solvent. The crude product was purified by FCC (EA/PE = 0-50%) to give **B2-4** (2.1 g, 86.8% yield) as a white solid. ¹H NMR (400 MHz, DMSO-*d*₆) δ 10.08 (s, 1H), 8.13 (dd, *J* = 7.2, 1.2 Hz, 1H), 7.86 (dd, *J* = 7.6, 1.2 Hz, 2H), 5.79 (d, *J* = 7.6 Hz, 1H), 5.41 (t, *J* = 9.2 Hz, 1H), 5.11 (dd, *J* = 10.0, 8.0 Hz, 1H), 5.04 (t, *J* = 9.2 Hz, 1H), 4.40-4.31 (m, 1H), 4.23-4.10 (m, 2H), 2.06 (s, 3H), 2.03 (s, 3H), 2.02 (s, 3H), 1.98 (s, 3H). MS-ESI [M+Na]⁺: 520.0.

**Step E:** To a solution of compound **B2-4** (2.1 g, 4.22 mmol) in ethyl acetate (30 mL) was added 10% Pd/C (210 mg), and the mixture was stirred under hydrogen protection in the dark at room temperature for 16 hours. After the reaction was complete, the mixture was filtered, and the filtrate was concentrated to remove solvent to give **B2-5** (1.8 g, 90.9% yield) as an off-white solid. MS-ESI [M+H]⁺: 470.1.

**Step F:** To a solution of compound **B2-5** (2.0 g, 4.26 mmol) in DMF (30 mL) was added compound **B1** (2.12 g, 4.26 mmol), followed by DMTMM (1.42 g, 5.12 mmol) at room temperature. The mixture was stirred under nitrogen at room temperature for 16 hours. After the reaction was complete, the mixture was slowly added dropwise to ice water (400 mL), and a white solid precipitated, which was filtered and dried to give **B2** (2.0 g, 49.5% yield) as a white solid. ¹H NMR (400 MHz, Methanol-*d*₄) δ 7.98 (d, *J* = 8.0 Hz, 1H), 7.78 (d, *J* = 7.6 Hz, 2H), 7.68-7.65 (m, 2H), 7.39-7.36 (m, 2H), 7.31-7.29 (m, 2H), 7.14-7.12 (m, 1H), 7.04-6.98 (m, 1H), 5.42-5.35 (m, 3H), 5.18-5.12 (m, 1H), 4.92-4.88 (m, 1H), 4.55 (s, 2H), 4.38-4.35 (m, 2H), 4.34-4.28 (m, 2H), 4.25-4.19 (m, 2H), 4.18-4.13 (m, 2H), 2.80 (t, *J* = 6.4 Hz, 2H), 2.08 (s, 3H), 2.02 (s, 3H), 2.00 (s, 3H), 1.98 (s, 3H), 1.41 (d, *J* = 6.8 Hz, 3H), 1.36 (d, *J* = 7.2 Hz, 3H). MS-ESI [M+H]⁺: 948.4.

Linkers **B3-B11** were prepared following the synthetic route for linker **B2,** using the starting materials in the table below instead of intermediate **B2-1.**

| **No.** | **Structural Formula** | **¹H NMR / MS-ESI [M+H]⁺** |
|---|---|---|
| **B3** | 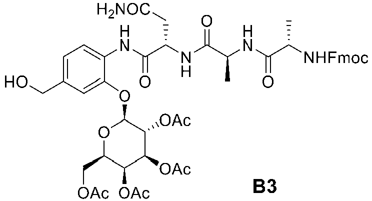 | **MS-ESI [M+H]⁺:** 948.2 |
| **B4** | 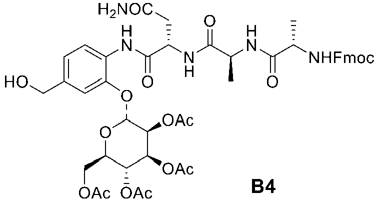 | **MS-ESI [M+H]⁺:** 948.3 |
| **B5** | 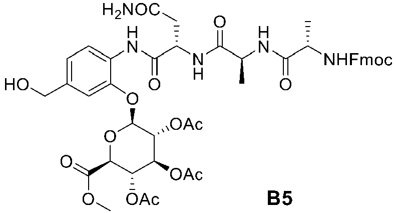 | ¹H NMR (400 MHz, Methanol-*d*₄) δ 7.96 (d, *J* = 8.2 Hz, 1H), 7.80-7.77 (m, 2H), 7.67 (t, *J* = 7.9 Hz, 2H), 7.39 (d, *J* = 7.5 Hz, 2H), 7.32-7.28 (m, 2H), 7.14 (d, *J* = 2.0 Hz, 1H), 7.05-6.99 (m, 1H), 5.50-5.40 (m, 3H), 5.27 (t, *J* = 9.4 Hz, 1H), 4.88 (t, *J* = 6.3 Hz, 1H), 4.54 (s, 2H), 4.45 (dd, *J* = 10.9, 8.5 Hz, 2H), 4.41-4.35 (m, 2H), 4.24-4.16 (m, 2H), 3.68 (s, 3H), 2.82 (qd, *J* = 15.8, 6.4 Hz, 2H), 2.07 (s, 3H), 2.01 (s, 3H), 1.97 (s, 3H), 1.41 (d, *J* = 7.2 Hz, 3H), 1.36 (d, *J* = 7.2 Hz, 3H). |
| | | **MS-ESI [M+H]⁺:** 934.2 |
| **B6** | 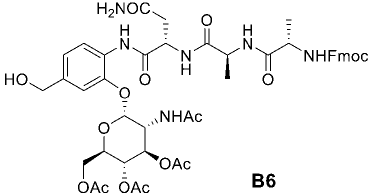 | **MS-ESI [M+Na]⁺:** 969.3 |
| **B7** | 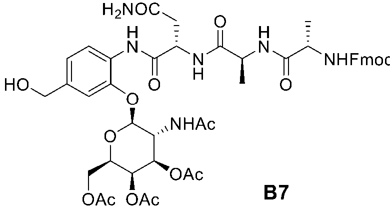 | **MS-ESI [M+Na]⁺:** 969.3 |
| **B8** | 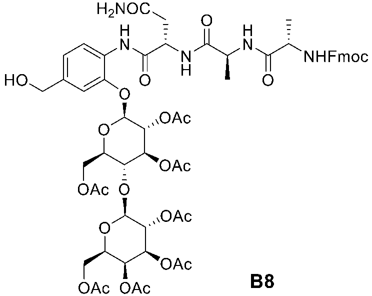 | **MS-ESI [M+H]⁺:** 1236.4 |
| **B9** | 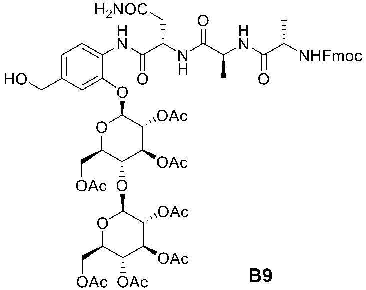 | **MS-ESI [M+H]⁺:** 1236.4 |
| **B10** | 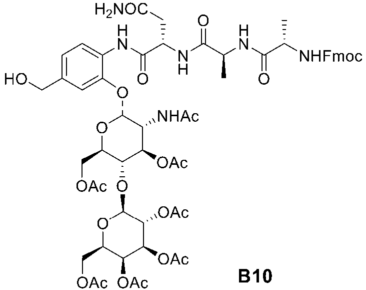 | **MS-ESI [M+Na]⁺:** 1257.4 |
| **B11** | 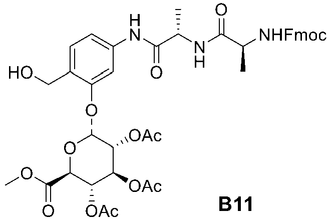 | **MS-ESI [M-17]⁺:** 802.3 |

### Preparation of Intermediate B12: methyl (2S,3S,4S,5R)-3,4,5-triacetoxy-6-(2-bromoethoxy)tetrahydro-2H-pyran-2-carboxylate

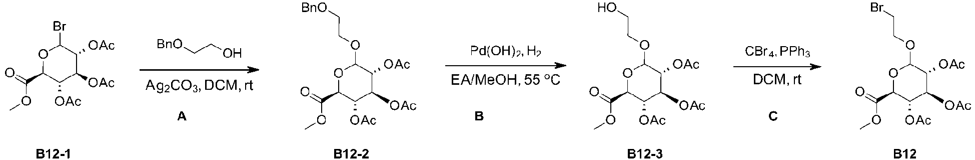

### Step A: methyl (2S,3S,4S,5R)-3,4,5-triacetoxy-6-[2-(benzyloxy)ethoxy]tetrahydro-2H-pyran-2-carboxylate

To a solution of methyl (2*S*,3*S*,4*S*,5*R*)-3,4,5-triacetoxy-6-bromotetrahydro-2*H*-pyran-2-carboxylate (2.00 g, 5.04 mmol) and 2-(benzyloxy)ethanol (1.60 g, 10.53 mmol) in anhydrous dichloromethane (30 mL) was added 4 Å molecular sieves (2.00 g). The reaction solution was protected with nitrogen, cooled to -10 °C, and silver carbonate (1.60 g, 5.98 mmol) was added. The reaction solution was slowly warmed to room temperature and stirred for 24 hours. Diluted with acetonitrile (30 mL), filtered, concentrated, and the crude product was purified by FCC (SiO₂, EA/PE = 0-40%) to give methyl (2*S*,3*S*,4*S*,5*R*)-3,4,5-triacetoxy-6-[2-(benzyloxy)ethoxy]tetrahydro-2H-pyran-2-carboxylate (1.05 g, 44% yield) as a colorless oil. ¹H NMR (400 MHz, CDCl₃) δ 7.38-7.27 (m, 5H), 5.31-5.18 (m, 2H), 5.08-4.99 (m, 1H), 4.68 (d, *J* = 8.0 Hz, 1H), 4.53 (d, *J* = 4.0 Hz, 2H), 4.06-3.96 (m, 2H), 3.82-3.72 (m, 4H), 3.63-3.65 (m, 2H), 2.02 (s, 6H), 1.99 (s, 3H). MS-ESI [M+Na]⁺: 491.1.

### Step B: methyl (2S,3S,4S,5R)-3,4,5-triacetoxy-6-(2-hydroxyethoxy)tetrahydro-2H-pyran-2-carboxylate

To a solution of methyl (2*S*,3*S*,4*S*,5*R*)-3,4,5-triacetoxy-6-[2-(benzyloxy)ethoxy]tetrahydro-2*H-*pyran-2-carboxylate (1.05 g, 2.24 mmol) in ethyl acetate (50 mL) and methanol (50 mL) was added 20% Pd(OH)₂/C (0.21 g, containing 50% H₂O). The atmosphere was replaced with hydrogen three times, and the mixture was stirred under hydrogen at 55 °C overnight, filtered and concentrated, to give methyl (2*S*,3*S*,4*S*,5*R*)-3,4,5-triacetoxy-6-(2-hydroxyethoxy)tetrahydro-2*H*-pyran-2-carboxylate (0.84 g, 99% yield) as a colorless oil. ¹H NMR (400 MHz, CDCl₃) δ 5.32-5.20 (m, 2H), 5.07-4.99 (m, 1H), 4.62 (d, *J* = 8.0 Hz, 1H), 4.08 (d, *J* = 9.4 Hz, 1H), 3.84-3.86 (m, 2H), 3.77 (s, 3H), 3.72-3.76 (m, 2H), 2.06 (s, 3H), 2.03 (d, *J* = 2.2 Hz, 6H). MS-ESI [M+Na]⁺: 401.1.

### Step C: methyl (2S,3S,4S,5R)-3,4,5-triacetoxy-6-(2-bromoethoxy)tetrahydro-2H-pyran-2-carboxylate

To a solution of methyl (2*S*,3*S*,4*S*,5*R*)-3,4,5-triacetoxy-6-(2-hydroxyethoxy)tetrahydro-2*H-*pyran-2-carboxylate (7.10 g, 18.78 mmol) in anhydrous dichloromethane (150 mL), cooled to 0 °C with ice water, were sequentially added triphenylphosphine (9.85 g, 37.60 mmol) and carbon tetrabromide (13.70 g, 41.27 mmol). The reaction was stirred at 0 °C for 15 minutes, warmed to room temperature and reacted for 2 hours. Water (100 mL) was added for washing, and the organic phase was concentrated and purified by FCC (SiO₂, ethyl acetate/petroleum ether = 0-40%) to give methyl (2*S*,3*S*,4*S*,5*R*)-3,4,5-triacetoxy-6-(2-bromoethoxy)tetrahydro-2*H*-pyran-2-carboxylate (7.41 g, 89.5% yield). ¹H NMR (400 MHz, Chloroform-*d*) δ 5.31-5.18 (m, 2H), 5.08-4.99 (m, 1H), 4.63 (d, *J* = 8.0 Hz, 1H), 4.26-4.16 (m, 1H), 4.05 (d, *J* = 9.4 Hz, 1H), 3.78-3.84 (m, 1H), 3.76 (s, 3H), 3.51-3.41 (m, 2H), 2.07 (s, 3H), 2.03 (s, 6H). MS-ESI [M+Na]⁺: 463.0.

### Preparation of Intermediate B13: (S)-3-[(tert-butyl)(oxycarbonylamino)]-2-(2,5-dioxo-3-pyrrolin-1-yl)propanoic acid

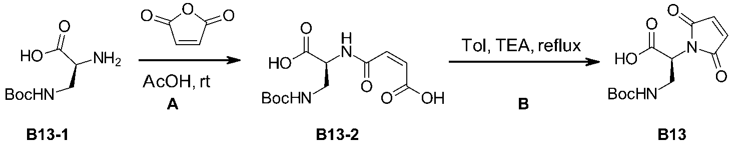

### Step A: (Z)-3-{N-(S)-2-[(tert-butyl)(oxycarbonylamino)]-1-carboxyethylcarbamoyl}acrylic acid

At room temperature, (*S*)-2-amino-3-[(tert-butyl)(oxycarbonylamino)]propanoic acid (25.0 g, 122.4 mmol) and maleic anhydride (12.0 g, 122.4 mmol) were added to anhydrous acetic acid (250 mL). After addition, the mixture was stirred at room temperature overnight. The reaction was complete as monitored by TLC. The organic solution was concentrated, and the crude product was slurried with dichloromethane (125 mL) twice to give (*Z*)-3-{*N*-(*S*)-2-[(tert-butyl)(oxycarbonylamino)]-1-carboxyethylcarbamoyl}acrylic acid (30.8 g, 81.0% yield) as a white solid. ¹H NMR (400 MHz, DMSO-*d*₆) δ 9.09 (d, *J* = 8.2 Hz, 1H), 6.89 (t, *J* = 6.2 Hz, 1H), 6.43 (d, *J* = 12.4 Hz, 1H), 6.29 (d, *J* = 12.4 Hz, 1H), 4.43-4.38 (m, 1H), 3.41-3.35 (m, 1H), 3.29-3.22 (m, 1H), 1.37 (s, 10H). MS-ESI [M+H-56]⁺: 247.0.

### Step B: (S)-3-[(tert-butyl)(oxycarbonylamino)]-2-(2,5-dioxo-3-pyrrolin-1-yl)propanoic acid

(*Z*)-3-{*N*-(*S*)-2-[(tert-butyl)(oxycarbonylamino)]-1-carboxyethylcarbamoyl}acrylic acid (5.0 g, 16.5 mmol) and triethylamine (8.4 g, 82.7 mmol) were added to anhydrous toluene (150 mL). After addition, the reaction solution was refluxed overnight. The reaction was complete as monitored by TLC. The organic solution was concentrated, and the crude product was purified by FCC (SiO₂, MeOH/DCM = 0-10%) to give (*S*)-3-[(tert-butyl)(oxycarbonylamino)]-2-(2,5-dioxo-3-pyrrolin-1-yl)propanoic acid (2.5 g, 53% yield) as a pale yellow solid. ¹H NMR (400 MHz, DMSO-*d*₆) δ 6.99 (s, 1H), 6.85 (t, *J* = 6.2 Hz, 1H), 4.34 (dd, *J* = 10.6, 4.2 Hz, 1H), 3.65-3.53 (m, 1H), 3.49-3.36 (m, 1H), 1.32 (s, 9H). MS-ESI [M+Na]⁺: 307.1.

### Preparation of Intermediate B14: tert-butyl (S)-3-amino-2-(2,5-dioxo-3-pyrrolin-1-yl)propanoate hydrochloride

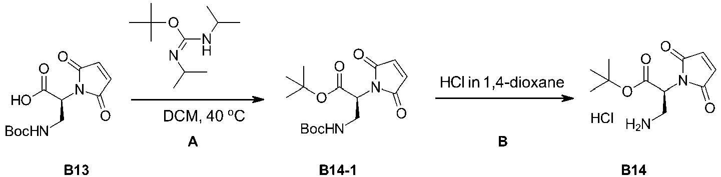

### Step A: tert-butyl (S)-3-[(tert-butyl)(oxycarbonylamino)]-2-(2,5-dioxo-3-pyrrolin-1-yl)propanoate

(*S*)-3-[(tert-butyl)(oxycarbonylamino)]-2-(2,5-dioxo-3-pyrrolin-1-yl)propanoic acid (8.9 g, 31.3 mmol) and (*E*)-2-(tert-butyl)-1,3-bis(propan-2-yl)isourea (45 mL) were added to anhydrous dichloromethane. The atmosphere was replaced with nitrogen three times, and the mixture was refluxed overnight. The reaction was complete as monitored by TLC. The reaction solution was filtered, and the filtrate was concentrated to give a crude product. The crude product was purified by FCC (SiO₂, EA/PE = 0-25%) to give tert-butyl (*S*)-3-[(tert-butyl)(oxycarbonylamino)]-2-(2,5-dioxo-3-pyrrolin-1-yl)propanoate (6.9 g, 65% yield) as a yellow oil. ¹H NMR (400 MHz, DMSO-*d₆*) δ 7.11 (s, 2H), 6.98 (t, *J* = 6.2 Hz, 1H), 4.58 (dd, *J* = 10.4, 4.0 Hz, 1H), 3.58-3.52 (m, 1H), 3.41-3.33 (m, 1H), 1.36 (s, 9H), 1.32 (s, 9H). MS-ESI [M+Na-56]⁺: 363.1.

### Step B: tert-butyl (S)-3-amino-2-(2,5-dioxo-3-pyrrolin-1-yl)propanoate hydrochloride

Under ice-water bath, hydrochloric acid in dioxane (4M, 6.25 mL) was added dropwise to a solution of tert-butyl (*S*)-3-[(tert-butyl)(oxycarbonylamino)]-2-(2,5-dioxo-3-pyrrolin-1-yl)propanoate (2.2 g, 6.4 mmol) in acetonitrile (30 mL). The mixture was stirred under ice-water bath for 3 hours. The acid solution was removed by concentration to give tert-butyl (*S*)-3-amino-2-(2,5-dioxo-3-pyrrolin-1-yl)propanoate hydrochloride (1.8 g, 100% yield) as a white solid. ¹H NMR (400 MHz, DMSO-*d₆*) δ 8.47 (s, 3H), 7.19 (s, 2H), 4.97 (dd, *J* = 10.4, 4.4 Hz, 1H), 3.45-3.25 (m, 2H), 1.37 (s, 9H). MS-ESI [M+H]⁺: 241.

### Preparation of Intermediate B15: 2-(trimethylsilyl)ethyl (S)-3-amino-2-(2,5-dioxo-3-pyrrolin-1-yl)propanoate

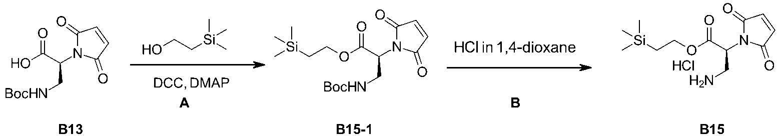

### Step A: 2-(trimethylsilyl)ethyl (S)-3-[(tert-butyl)(oxycarbonylamino)]-2-(2,5-dioxo-3-pyrrolin-1-yl)propanoate

Under ice-water bath, *N*,*N'*-dicyclohexylcarbodiimide (686.0 mg, 3.3 mmol) was added to a solution of (*S*)-3-[(tert-butyl)(oxycarbonylamino)]-2-(2,5-dioxo-3-pyrrolin-1-yl)propanoic acid (900.0 mg, 3.2 mmol), 2-(trimethylsilyl)ethanol (451.0 mg, 3.2 mmol), and 4-dimethylaminopyridine (39.0 mg, 0.3 mmol) in dichloromethane (10 mL). The reaction solution was stirred under ice-water bath for 3 hours. After the reaction was complete as monitored by LCMS, filtered, and the filtrate was concentrated to give a crude product. The crude product was purified by FCC (SiO₂, EA/PE = 0-20%) to give 2-(trimethylsilyl)ethyl (*S*)-3-[(tert-butyl)(oxycarbonylamino)]-2-(2,5-dioxo-3-pyrrolin-1-yl)propanoate (575.0 mg, 47% yield) as a yellow oil. ¹H NMR (400 MHz, DMSO-*d*₆) δ 7.12 (s, 2H), 7.01 (t, *J* = 6.2 Hz, 1H), 4.68 (dd, *J* = 10.4, 3.8 Hz, 1H), 4.24-4.10 (m, 1H), 3.61-3.55 (m, 1H), 3.45-3.37 (m, 1H), 1.33 (s, 9H), 0.93-0.89(m, 1H), 0.00 (s, 9H). MS-ESI [M+Na]⁺: 407.1.

### Step B: 2-(trimethylsilyl)ethyl (S)-3-amino-2-(2,5-dioxo-3-pyrrolin-1-yl)propanoate

Under ice-water bath, hydrochloric acid in dioxane (4M, 1 mL) was added dropwise to a solution of 2-(trimethylsilyl)ethyl (*S*)-3-[(tert-butyl)(oxycarbonylamino)]-2-(2,5-dioxo-3-pyrrolin-1-yl)propanoate (100.0 mg, 0.3 mmol) in acetonitrile (3 mL). The mixture was stirred under ice-water bath for 3 hours. The acid solution was removed by concentration to give 2-(trimethylsilyl)ethyl (*S*)-3-amino-2-(2,5-dioxo-3-pyrrolin-1-yl)propanoate hydrochloride (83.4 mg, 100% yield) as a pale yellow solid. ¹H NMR (400 MHz, DMSO-*d*₆) δ 8.48 (s, 3H), 7.20 (s, 2H), 5.07 (dd, *J =* 10.2, 4.2 Hz, 1H), 4.24-4.16 (m, 2H), 3.42-3.22 (m, 2H), 0.94-0.88 (m, 1H), 0.00 (s, 9H). MS-ESI [M+H]⁺: 285.

### Preparation of Intermediate B16: (N-methyl{[N-methyl({N-methyl[(N-methyl{[N-methyl({N-methyl({N-methyl({N-methyl[(N-methylacetamido]methyl]carbonylamino}methyl(carbonylamino]methyl}carbonylamino)methyl]ca rbonylamino]methyl}carbonylamino)methyl]carbonylamino}methyl)carbonylamino]methyl}carbon ylamino)acetic acid

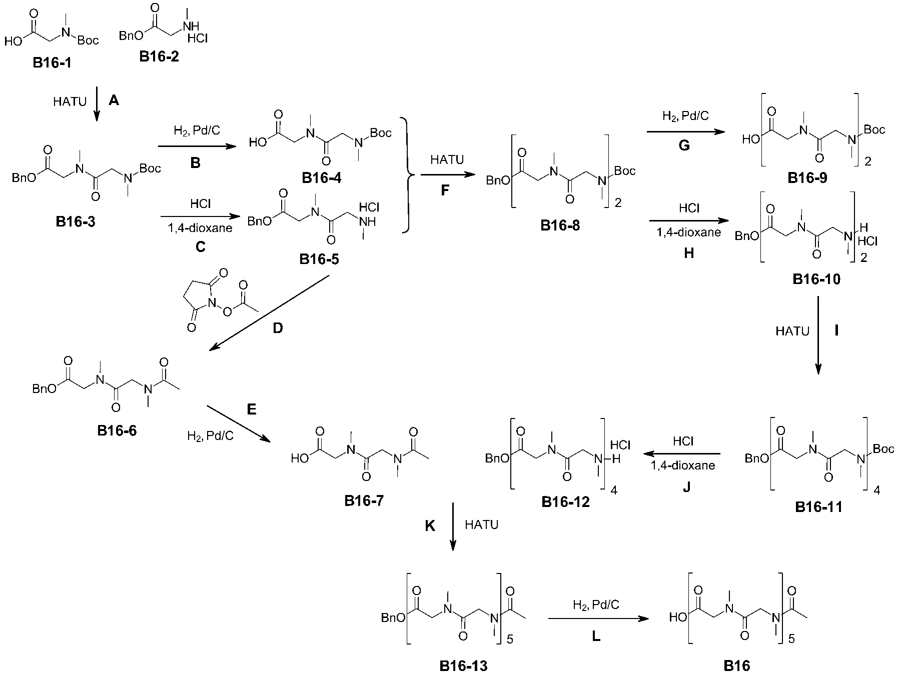

### Step A: benzyl (N-methyl{[(tert-butyl)(methyl)(oxycarbonylamino)]methyl}carbonylamino)acetate

At 0 °C, HATU (17.6 g, 46.4 mmol) was added to a solution of compound **B16-1** (8.8 g, 46.4 mmol) and compound **B16-2** (10 g, 46.4 mmol) in acetonitrile (100 mL), followed by DIPEA (18.0 g, 139.1 mmol). The resulting reaction solution was stirred at room temperature for 1 hour. The solvent was removed by concentration under reduced pressure, the residual liquid was diluted with EA (100 mL), washed with 1M HCl (3 × 100 mL), the organic phase was dried over Na₂SO₄, filtered, and purified by column chromatography (PE/EA = 0-100%) to give **B16-3** (16 g, 98.5% yield) as a colorless oil. ¹H NMR (400 MHz, DMSO-*d*₆) δ 7.64-7.09 (m, 5H), 5.23-5.09 (m, 2H), 4.31-3.89 (m, 4H), 3.00-2.65 (m, 6H), 1.42-1.30 (m, 9H). MS-ESI [M+Na]⁺: 373.3.

### Step B: (N-methyl{[(tert-butyl)(methyl)(oxycarbonylamino)]methyl}carbonylamino)acetic acid

At room temperature, Pd/C (900 mg, 10% wt) was added to a solution of intermediate **B16-3** (9 g, 25.7 mmol) in THF (90 mL). The reaction solution was stirred under a hydrogen atmosphere for 5 hours. The reaction solution was filtered and concentrated to give **B16-4** (6.7 g, 100% yield) as a colorless oil. MS-ESI [M+Na]⁺: 283.3.

### Step C: benzyl {N-methyl[(methylamino)methyl]carbonylamino}acetate hydrochloride

At room temperature, HCl-Dioxane (4M, 40 mL) was added to a solution of intermediate **B16-3** (8 g, 22.8 mmol) in DCM (80 mL). The reaction solution was stirred at room temperature for 3 hours. The solvent was removed by concentration under reduced pressure, to give **B16-5** (6.6 g, 100% yield) as a white solid. MS-ESI [M+H]⁺: 251.3.

### Step D: benzyl {N-methyl[(N-methylacetamido)methyl]carbonylamino}acetate

At 0 °C, 1-(acetoxy)-2,5-pyrrolidinedione (12.6 g, 80 mmol) was added to a solution of intermediate **B16-5** (10 g, 40 mmol) in acetonitrile (100 mL). The reaction solution was stirred at room temperature for 1 hour. The solvent was removed by concentration under reduced pressure, the residual liquid was diluted with EA (100 mL), washed with 0.5M HCl (3 × 100 mL), the organic phase was dried over anhydrous Na₂SO₄, filtered, and purified by column chromatography (DCM/MeOH = 0-10%) to give **B16-6** (4 g, 34.3% yield) as a colorless oil. MS-ESI [M+H]⁺: 293.4.

### Step E: {N-methyl[(N-methylacetamido)methyl]carbonylamino}acetic acid

At room temperature, Pd/C (400 mg, 10% wt) was added to a solution of intermediate **B16-6** (4 g, 13.7 mmol) in THF (40 mL). The reaction solution was stirred under a hydrogen atmosphere for 1 hour. The reaction solution was filtered and concentrated to give **B16-7** (2.8 g, 100% yield) as a colorless oil. MS-ESI [M+Na]⁺: 225.3.

### Step F: benzyl [N-methyl({ N-methyl[(N-methyl { [(tert-butyl)(methyl)(oxycarbonylamino)]methyl}carbonylamino)methyl]carbonylamino}methyl)carbonyl amino]acetate

At 0 °C, HATU (8.8 g, 23 mmol) was added to a solution of compound **B16-4** (6.0 g, 23 mmol) and compound **B16-5** (6.6 g, 23 mmol) in acetonitrile (60 mL), followed by DIPEA (8.9 g, 69 mmol). The resulting reaction solution was stirred at room temperature for 1 hour. The solvent was removed by concentration under reduced pressure, purified by reverse phase (acetonitrile/water = 5-95%), to give **B16-8** (9.8 g, 86.4% yield) as a pale yellow oil. ¹H NMR (400 MHz, DMSO-*d*₆) δ 7.63-7.10 (m, 5H), 5.27-5.08 (m, 2H), 4.50-3.66 (m, 8H), 3.12-2.62 (m, 12H), 1.46-1.26 (m, 9H). MS-ESI [M+H]⁺: 493.5.

### Step G: [N-methyl({N-methyl[(N-methyl{[(tert-butyl)(methyl)(oxycarbonylamino)]methyl}carbonylamino)methyl]carbonylamino}methyl)carbonyl amino]acetic acid

At room temperature, Pd/C (500 mg, 10% wt) was added to a solution of intermediate **B16-8** (4.9 g, 10 mmol) in THF (50 mL). The reaction solution was stirred under a hydrogen atmosphere for 5 hours. The reaction solution was filtered and concentrated to give **B16-9** (4 g, 100% yield) as a colorless oil. MS-ESI [M+H]⁺: 403.4.

### Step H: benzyl (N-methyl{[N-methyl({N-methyl[(methylamino)methyl]carbonylamino}methyl)carbonylamino]methyl}carbonylamino) hydrochloride

At room temperature, HCl-Dioxane (4M, 20 mL) was added to a solution of intermediate **B16-8** (4.9 g, 10 mmol) in DCM (50 mL). The reaction solution was stirred at room temperature for 3 hours. The solvent was removed by concentration under reduced pressure, to give **B16-10** (4.3 g, 100% yield) as a white solid. MS-ESI [M+H]⁺: 393.3.

### Step I: benzyl (N-methyl{[N-methyl({N-methyl[(N-methyl{[N-methyl({N-methyl[(N-methyl{[(tert-butyl)(methyl)(oxycarbonylamino)]methyl}carbonylamino)methyl]carbonylamino}methyl)carbonyl amino]methyl}carbonylamino)methyl]carbonylamino}methyl)carbonylamino]methyl}carbonylami no)acetate

At 0 °C, HATU (3.8 g, 9.9 mmol) was added to a solution of compound **B16-9** (4 g, 9.9 mmol) and compound **B16-10** (4.3 g, 9.9 mmol) in acetonitrile (60 mL), followed by DIPEA (3.9 g, 29.8 mmol). The resulting reaction solution was stirred at room temperature for 1 hour. The solvent was removed by concentration under reduced pressure, purified by reverse phase (acetonitrile/water = 5-95%), to give **B16-11** (7 g, 90.7% yield) as a pale yellow oil. ¹H NMR (400 MHz, DMSO-*d*₆) δ 7.45-7.28 (m, 5H), 5.24-5.09 (m, 2H), 4.38-3.83 (m, 16H), 3.05-2.70 (m, 24H), 1.39-1.30 (m, 9H). MS-ESI [M+H]⁺: 777.5.

### Step J: benzyl {N-methyl[(N-methyl{[N-methyl({N-methyl[(N-methyl{[N-methyl({N-methyl[(methylamino)methyl]carbonylamino}methyl)carbonylamino]methyl}carbonylamino)meth yl]carbonylamino } methyl)carbonylamino] methyl } carbonylamino)methyl]carbonylamino } acetate hydrochloride

At room temperature, HCl-Dioxane (4M, 30 mL) was added to a solution of intermediate **B16-11** (7 g, 9 mmol) in DCM (70 mL). The reaction solution was stirred at room temperature for 3 hours. The solvent was removed by concentration under reduced pressure, to give **B16-12** (6.4 g, 100% yield) as a white solid. MS-ESI [M+H]⁺: 677.5.

### Step K: benzyl (N-methyl{[N-methyl({N-methyl[(N-methyl{[N-methyl({N-methyl[(N-methyl{[N-methyl({N-methyl[(N-methylacetamido)methyl]carbonylamino}methyl)carbonylamino]methyl}carbonylamino)methyl]ca rbonylamino}methyl)carbonylamino]methyl}carbonylaminocarbonylamino}methyl)carbon ylamino]methyl}carbonylamino)acetate

At 0 °C, HATU (1.1 g, 2.8 mmol) was added to a solution of compound **B16-12** (2 g, 2.8 mmol) and compound **B16-7** (567 mg, 2.8 mmol) in acetonitrile (20 mL), followed by DIPEA (1.1 g, 8.4 mmol). The resulting reaction solution was stirred at room temperature for 1 hour. The solvent was removed by concentration under reduced pressure, purified by reverse phase (acetonitrile/water = 5-95%), to give **B16-13** (1.3 g, 53.9% yield) as a white solid. ¹H NMR (400 MHz, DMSO-*d*₆) δ 7.46-7.28 (m, 5H), 5.24-5.08 (m, 2H), 4.46-3.80 (m, 20H), 3.07-2.70 (m, 30H), 2.03-1.72 (m, 3H). MS-ESI [M+H]⁺: 861.5.

### Step L: (N-methyl{[N-methyl({N-methyl[(N-methyl{[N-methyl({N-methyl[(N-methyl{[N-methyl({N-methyl[(N-methylacetamido)methyl]carbonylamino}methyl)carbonylamino]methyl}carbonylamino)methyl]ca rbonylamino}methyl)carbonylamino]methyl} carbonylamino)methyl]carbonylamino } methyl)carbon ylamino]methyl}carbonylamino)acetic acid

At room temperature, Pd/C (80 mg, 10% wt) was added to a solution of intermediate **B16-13** (800 mg, 0.929 mmol) in THF (10 mL). The reaction solution was stirred under a hydrogen atmosphere for 2 hours. The reaction solution was filtered and concentrated to give **B16** (500 mg, 69.8% yield) as a white solid. ¹H NMR (400 MHz, DMSO-*d*₆) δ 4.47-3.80 (m, 20H), 3.05-2.67 (m, 30H), 2.02-1.73 (m, 3H). MS-ESI [M+H]⁺: 771.4.

### Preparation of Intermediate B17: 4-(hydroxymethyl)phenyl 4-nitrobenzoate

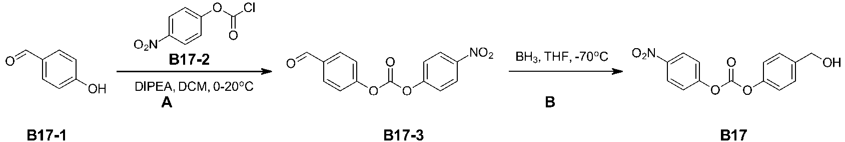

**Step A:** Under ice-water bath, **B17-2** (1.7 g, 8.43 mmol) and *N,N*-diisopropylethylamine (1.1 g, 8.53 mmol) were added to a solution of **B17-1** (1.0 g, 8.20 mmol) in dichloromethane (10 mL). The reaction system was warmed to room temperature and stirred overnight. After the reaction was complete, the reaction solution was concentrated, and the crude product was purified by FCC (SiO₂, EA/PE = 0-70%) to give **B17-3** (1.3 g, 55% yield) as a white solid. MS-ESI [M+H]⁺: 288.0.

**Step B:** At -70 °C, **B17-3** (200 mg, 0.70 mmol) was dissolved in tetrahydrofuran (10 mL), and a solution of borane in tetrahydrofuran (1M, 1.4 mL, 1.40 mmol) was added. The mixture was stirred at -70 °C for 3 hours. After the reaction was complete, the reaction was quenched by adding a small amount of methanol, and the mixture was warmed to room temperature. The reaction solution was concentrated, and the crude product was purified by FCC (SiO₂, EA/PE = 0-70%) to give **B17** (125 mg, 62% yield) as a white solid. MS-ESI [M+Na]⁺: 312.0.

### Preparation of Intermediate B18: (S)-3-[(1R,2S,3R,4R)-1,2,3,4,5-pentahydroxypentylcarbonylamino]-2-(2,5-dioxo-3-pyrrolin-1-yl)propanoic acid

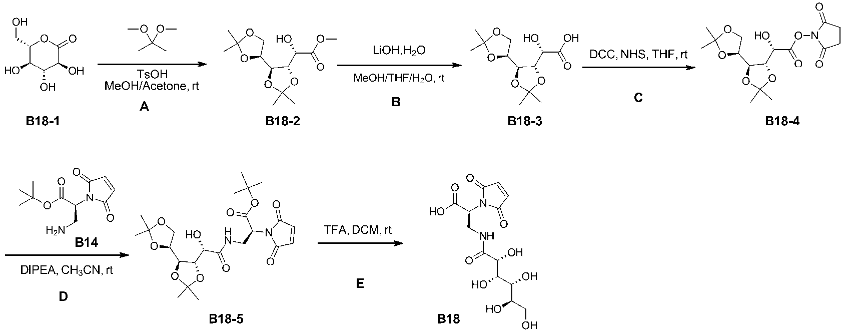

**Step A:** At room temperature, 2,2-dimethoxypropane (22.50 mL) and p-toluenesulfonic acid (150 mg, 0.87 mmol) were added to a solution of **B18-1** (13.4 g, 75.28 mmol) in methanol (2.3 mL) and acetone (7.5 mL). The reaction was stirred at room temperature for 48 hours. TLC indicated complete consumption of starting material. Sodium bicarbonate (23.9 g, 225.66 mmol) was added, and the mixture was stirred at room temperature for 10 minutes. The reaction solution was filtered, and the filtrate was concentrated. Water (100 mL) was added, and the mixture was extracted with dichloromethane (3 × 50 mL). The organic phases were combined, washed with saturated sodium chloride solution (50 mL), concentrated, and the resulting crude product was purified by FCC (SiO₂, EA/PE = 0-20%) to give **B18-2** (15.5 g, 71% yield) as a colorless oil. MS-ESI [M+Na]⁺: 313.1.

**Step B:** At room temperature, tetrahydrofuran (40 mL) and water (16 mL) were added to a solution of **B18-2** (6.1 g, 21.03 mmol) in methanol (40 mL). Lithium hydroxide monohydrate (1.3 g, 31.55 mmol) was dissolved in water (10 mL), and this aqueous solution was added to the reaction solution and stirred at room temperature for 1 hour. After the reaction was complete, tetrahydrofuran and methanol were removed by a water pump. Under ice-water bath, HCl (1M) was slowly added to adjust pH to 1-2, and extracted with dichloromethane (5 × 50 mL). The organic phases were combined, dried over anhydrous sodium sulfate and filtered. The filtrate was concentrated to give **B18-3** (4.7 g, 81% yield) as a white solid. MS-ESI [M+Na]⁺: 299.1.

**Step C:** At room temperature, DCC (6.0 g, 29.08 mmol) was added to a solution of **B18-3** (4.7 g, 17.03 mmol) in DMF (10 mL). The mixture was stirred at room temperature overnight. After the reaction was complete, the mixture was filtered, and the filtrate was concentrated. The crude product was purified by FCC (SiO₂, EA/PE = 0-40%) to give **B18-4** (2.0 g, 32% yield) as a white solid. MS-ESI [M+Na]⁺: 396.1.

**Step D:** At room temperature, **B14** (300 mg, 1.09 mmol) and N,N-diisopropylethylamine (350 mg, 2.71 mmol) were added to a solution of **B18-4** (490 mg, 1.31 mmol) in acetonitrile (10 mL). The mixture was stirred at room temperature for 5 hours. After the reaction was complete, the reaction solution was concentrated, and the crude product was purified by FCC (SiO₂, EA/PE = 0-70%) to give **B18-5** (470 mg, 87% yield) as a white solid. MS-ESI [M+H]⁺: 499.3.

**Step E:** Under ice-water bath, trifluoroacetic acid (5 mL) was added to a solution of **B18-5** (300 mg, 0.60 mmol) in dichloromethane (20 mL). The mixture was stirred at room temperature for 2 hours. After the reaction was complete, the reaction solution was concentrated. The crude product obtained after concentration was purified by reverse phase C18 column (ACN/H₂O = 0-50%) to give **B18** (120 mg, 54% yield) as a white solid. MS-ESI [M+H]⁺: 363.1.

### Preparation of Intermediate B19: N-[(S)-3-(2,5-dioxo-1-pyrrolidinyloxy)-2-(2,5-dibromo-3-pyrrolin-1-yl)-3-oxopropyl]-(2S,3S,4S,5R)-3,4,5,6-tetrahydroxytetrahydro-2H-pyran-2-carboxamide

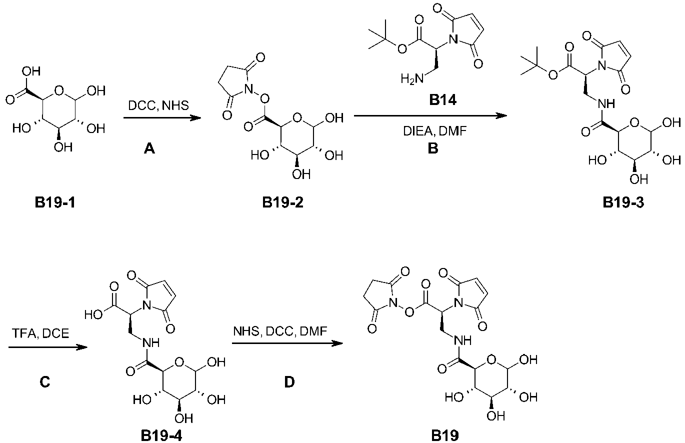

### Step A: 1-{[(2S,3S,4S,5R)-3,4,5,6-tetrahydroxytetrahydro-2H-pyran-2-yl]carbonyloxy}-2,5-pyrrolidinedione

Under ice bath, *N,N*'-dicyclohexylcarbodiimide (2.1 g, 10.9 mmol) was added to a reaction solution of *D*-glucuronic acid (1.9 g, 10.9 mmol) and *N*-hydroxysuccinimide (1.2 g, 10.9 mmol) in anhydrous *N,N*-dimethylformamide (10 mL), stirred at room temperature overnight. The reaction solution was filtered, and the filtrate was used directly in the next step. MS-ESI [M+Na]⁺: 314.0.

### Step B: tert-butyl (S)-3-[(2S,3S,4S,5R)-3,4,5,6-tetrahydroxytetrahydro-2H-pyran-2-ylcarbonylamino]-2-(2,5-dioxo-3-pyrrolin-1-yl)propanoate

At room temperature, tert-butyl (*S*)-3-amino-2-(2,5-dioxo-3-pyrrolin-1-yl)propanoate (2.6 g, 10.9 mmol) and *N,N*-diisopropylethylamine (4.2 g, 32.7 mmol) were added to the filtrate obtained in the previous step. The reaction solution was stirred at room temperature for 2 hours. After the reaction was complete as monitored by LCMS, the reaction solution was purified by pre-HPLC (C18, 0.1% FA(aq)/ACN = 5-25%) to give tert-butyl (*S*)-3-[(2*S*,3*S*,4*S*,5*R*)-3,4,5,6-tetrahydroxytetrahydro-2*H-*pyran-2-ylcarbonylamino]-2-(2,5-dioxo-3-pyrrolin-1-yl)propanoate (1.0 g, 22% yield) as a brown solid. MS-ESI [M+H]⁺: 417.1.

### Step C: (S)-3-[(2S,3S,4S,5R)-3,4,5,6-tetrahydroxytetrahydro-2H-pyran-2-ylcarbonylamino]-2-(2,5-dioxo-3-pyrrolin-1-yl)propanoic acid

At room temperature, trifluoroacetic acid (5 mL) was added to a solution of tert-butyl (*S*)-3-[(2*S*,3*S*,4*S*,5*R*)-3,4,5,6-tetrahydroxytetrahydro-2*H*-pyran-2-ylcarbonylamino]-2-(2,5-dioxo-3-pyrrolin-1-yl)propanoate (1.0 g, 2.4 mmol) in 1,2-dichloroethane (20 mL). The resulting reaction solution was stirred at room temperature for 4 hours. After the reaction was complete as monitored by LCMS, the reaction solution was purified by pre-HPLC (C18, (0.1% FA(aq)/ACN)/ACN = 5%) to give (*S*)-3-[(2*S*,3*S*,4*S*,5*R*)-3,4,5,6-tetrahydroxytetrahydro-2*H*-pyran-2-ylcarbonylamino]-2-(2,5-dioxo-3-pyrrolin-1-yl)propanoic acid (0.3 g, 30% yield) as a white solid. MS-ESI [M+H]⁺: 361.1.

### Step D: N-[(S)-3-(2,5-dioxo-1-pyrrolidinyloxy)-2-(2,5-dibromo-3-pyrrolin-1-yl)-3-oxopropyl]-(2S,3S,4S,5R)-3,4,5,6-tetrahydroxytetrahydro-2H-pyran-2-carboxamide

Under ice bath, *N,N*'-dicyclohexylcarbodiimide (69.2 mg, 0.34 mmol) was added to a reaction solution of (*S*)-3-[(2*S*,3*S*,4*S*,5*R*)-3,4,5,6-tetrahydroxytetrahydro-2*H*-pyran-2-ylcarbonylamino]-2-(2,5-dioxo-3-pyrrolin-1-yl)propanoic acid (100.0 mg, 0.28 mmol) and *N*-hydroxysuccinimide (32.2 mg, 0.28 mmol) in anhydrous *N,N*-dimethylformamide (1 mL). The mixture was stirred at room temperature overnight. The reaction solution was filtered, and the filtrate was used directly in the next step. MS-ESI [M+H]⁺: 458.1.

### Preparation of Intermediate B20: (S)-3-[N-(2S,3R,4R,5R)-2,3,4-5,6-pentahydroxyhexylamino]-2-(2,5-dioxo-3-pyrrolin-1-yl)propanoic acid

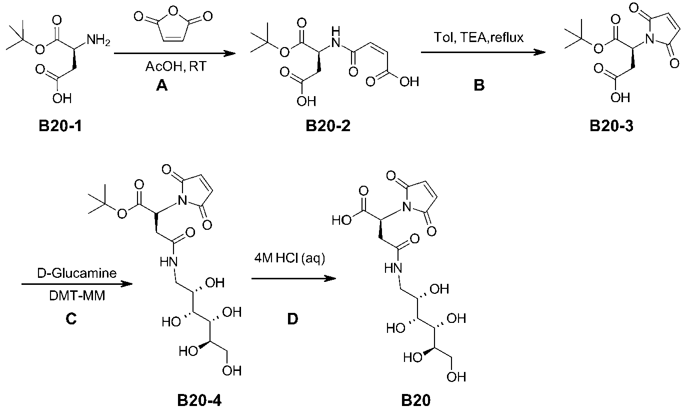

### Step A: (Z)-3-[N-(S)-1-(tert-butoxycarbonyl)-2-carboxyethylcarbamoyl]acrylic acid

At room temperature, (*S*)-3-amino-3-(tert-butoxycarbonyl)propanoic acid (10.0 g, 52.8 mmol) and maleic anhydride (5.2 g, 52.8 mmol) were added to acetic acid (100 mL). The resulting mixture was stirred at room temperature overnight. After the reaction was complete, the reaction solution was concentrated. The resulting residue was added to dichloromethane (250 mL), stirred at room temperature for 30 minutes, filtered, and the filter cake was collected and dried under vacuum to give (*Z*)-3-[*N*-(*S*)-1-(tert-butoxycarbonyl)-2-carboxyethylcarbamoy]acrylic acid (10.6 g, 70% yield) as a white solid. ¹H NMR (400 MHz, DMSO-*d*₆) δ 9.15 (d, *J =* 7.8 Hz, 1H), 6.45-6.24 (m, 2H), 4.59-4.50 (m, 1H), 2.75-2.59 (m, 2H), 1.40 (s, 9H). MS-ESI [M+Na]⁺: 310.1.

### Step B: (S)-3-(tert-butoxycarbonyl)-3-(2,5-dioxo-3-pyrrolin-1-yl)propanoic acid

(*Z*)-3-[*N*-(*S*)-1-(tert-butoxycarbonyl)-2-carboxyethylcarbamoyl]acrylic acid (5.0 g, 17.4 mmol) and triethylamine (8.8 g, 87 mmol) were added to anhydrous toluene (50 mL). After addition, the mixture was refluxed overnight. The reaction was complete as monitored by TLC. filtered, and the filtrate was purified by pre-HPLC (C18, (0.01% HCl(aq)/ACN)/ACN = 35-40%) to give the product (*S*)-3-(tert-butoxycarbonyl)-3-(2,5-dioxo-3-pyrrolin-1-yl)propanoic acid (1.0 g, 22% yield) as a white solid. ¹H NMR (400 MHz, DMSO-*d*₆) δ 12.50 (s, 1H), 7.13 (s, 2H), 4.91 (t, *J =* 7.2 Hz, 1H), 3.01 (dd, *J =* 16.6, 7.6 Hz, 1H), 2.78 (dd, *J =* 16.6, 7.6 Hz, 1H), 1.34 (s, 9H). MS-ESI [M+Na]⁺: 292.0.

### Step C: tert-butyl (R)-3-[N-(2S,3R,4R,5R)-2,3,4-5,6-pentahydroxyhexylamino]-2-(2,5-dioxo-3-pyrrolin-1-yl)propanoate

(*S*)-3-(Tert-butoxycarbonyl)-3-(2,5-dioxo-3-pyrrolin-1-yl)propanoic acid (400.0 mg, 1.5 mmol), 4-(4,6-dimethoxy-1,3,5-triazin-2-yl)-4-methylmorpholinium chloride (455.0 mg, 1.6 mmol) and *D*-glucosamine (271.0 mg, 1.5 mmol) were added to anhydrous N,N-dimethylformamide (1 mL). After addition, the mixture was stirred at room temperature for 3 hours. After the reaction was complete, the reaction solution was purified by pre-HPLC (C18, H₂O/ACN = 30-45%) to give the product tert-butyl (*R*)-3-[*N*-(2*S*,3*R*,4*R*,5*R*)-2,3,4-5,6-pentahydroxyhexylamino]-2-(2,5-dioxo-3-pyrrolin-1-yl)propanoate (416 mg, 64.8% yield) as a white solid. ¹H NMR (400 MHz, DMSO-*d*₆) δ 7.96-7.92 (m, 1H), 7.08-7.07 (m, 2H), 5.04-4.99 (m, 1H), 4.13 (s, 4H), 3.60 -3.50 (m, 3H), 3.49-3.43 (m, 1H), 3.41-3.32 (m, 2H), 3.32-3.12 (m, 1H), 3.11-2.87 (m, 2H), 2.75-2.59 (m, 1H), 1.60 (s, 1H), 1.34 (s, 9H). MS-ESI [M+H]⁺: 433.2.

### Step D: (S)-3-[N(2S,3R,4R,SR)-2,3,4-5,6-pentahydroxyhexylamino]-2-(2,5-dioxo-3-pyrrolin-1-yl)propanoic acid

At room temperature, tert-butyl (*R*)-3-[*N*-(2*S*,3*R*,4*R*,5*R*)-2,3,4-5,6-pentahydroxyhexylamino]-2-(2,5-dioxo-3-pyrrolin-1-yl)propanoate (500.0 mg, 1.2 mmol) was added to aqueous hydrochloric acid (4M, 5 mL) and stirred at room temperature for 3 hours. After the reaction was complete, the reaction solution was purified by pre-HPLC (C18, (0.01% HCl(aq)/ACN)/ACN = 5-30%) to give (*S*)-3-[*N-*(2*S*,3*R*,4*R*,5*R*)-2,3,4-5,6-pentahydroxyhexylamino]-2-(2,5-dioxo-3-pyrrolin-1-yl)propanoic acid (200 mg, 47.2% yield) as a white solid. MS-ESI [M+H]⁺: 377.1.

### Preparation of Intermediate B21: (S)-3-{[(N-methyl{[N-methyl({N-methyl[(N-methyl{[N-methyl({N-methyl[(N-methyl{[N-methyl({N-methyl[(N-methylacetamido)methyl]carbonylamino}methyl)carbonylamino]methyl}carbonylamino)methyl]ca rbonylamino}methyl)carbonylamino]methyl}carbonylamino)methyl]carbonylamino}methyl)carbon ylamino]methyl}carbonylamino)methyl]carbonylamino}-2-(2,5-dioxo-3-pyrrolidin-1-yl)propanoic acid

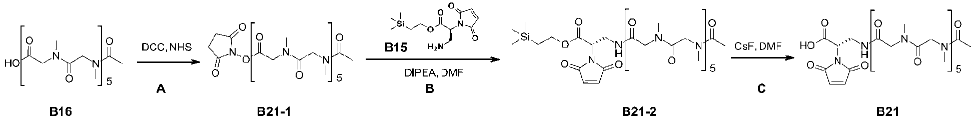

### Step A: N-[({[(2-(2,5-dioxo-1-pyrrolidinyloxy)-2-oxoethyl]-N-methylcarbamoyl}methyl)-N-methylcarbamoyl]methyl}-N-methylcarbamoyl)methyl]-N-methyl[N-methyl({N-methyl[(N-methyl{[N-methyl({N-methyl[(N-methylacetamido)methyl]carbonylamino}methyl)carbonylamino]methyl}carbonylamino)methyl]ca rbonylamino } methyl)carbonylamino] acetamide

At room temperature, *N,N'*-dicyclohexylcarbodiimide (29.5 mg, 0.14 mmol) was added to a reaction solution of (*N*-methyl{[*N*-methyl({*N*-methyl[(*N*-methyl{[*N*-methyl({*N*-methyl[(*N-*methyl{[*N*-methyl({*N*-methyl[(*N-*methylacetamido)methyl]carbonylamino}methyl)carbonylamino]methyl}carbonylamino)methyl]ca rbonylamino}methyl)carbonylamino]methyl}carbonylamino)methyl]carbonylamino}methyl)carbon ylamino]methyl}carbonylamino)acetic acid (100 mg, 0.13 mmol) and *N*-hydroxysuccinimide (18.4 mg, 0.16 mmol) in anhydrous *N,N*-dimethylformamide (1 mL). The mixture was stirred at room temperature overnight. The reaction solution was filtered, and the filtrate was used directly in the next step. MS-ESI [M+H]⁺: 868.4.

### Step B: 2-(trimethylsilyl)ethyl (S)-3-{[(N-methyl{[N-methyl({N-methyl[(N-methyl{[N-methyl({N-methyl[(N-methyl{[N-methyl({N-methyl[(N-methylacetamido)methyl]carbonylamino}methyl)carbonylamino]methyl}carbonylamino)methyl]ca rbonylamino}methyl)carbonylamino]methyl}carbonylamino)methyl]carbonylamino}methyl)carbon ylamino]methyl}carbonylamino)methyl]carbonylamino}-2-(2,5-dioxo-3-pyrrolidin-1-yl)propanoate

At room temperature, 2-(trimethylsilyl)ethyl (*S*)-3-amino-2-(2,5-dioxo-3-pyrrolidin-1-yl)propanoate (37.0 mg, 0.13 mmol) and *N,N*-diisopropylethylamine (42.0 mg, 0.33 mmol) were added to the filtrate obtained in the previous step. The reaction solution was stirred at room temperature for 2 hours. After the reaction was complete as monitored by LCMS, the reaction solution was purified by pre-HPLC (C18, H₂O/ACN = 25-30%) to give 2-(trimethylsilyl)ethyl *(S)-3-{[(N-*methyl{[*N*-methyl({*N*-methyl[(*N*-methyl{[*N*-methyl({*N*-methyl[(*N*-methyl{[*N*-methyl({*N-*methyl[(*N-*methylacetamido)methyl]carbonylamino}methyl)carbonylamino]methyl}carbonylamino)methyl]ca rbonylamino } methyl)carbonylamino]methyl }carbonylamino)methyl]carbonylamino } methyl)carbon ylamino]methyl}carbonylamino)methyl]carbonylamino}-2-(2,5-dioxo-3-pyrrolidin-1-yl)propanoate (40 mg, 35.4% yield) as a white solid. ¹H NMR (400 MHz, DMSO-*d*₆) δ 8.46-8.00 (m, 1H), 7.22-7.11 (m, 2H), 4.82-4.71 (m, 1H), 4.48-3.68 (m, 31H), 3.58-3.45 (m, 1H), 3.04-2.65 (m, 24H), 2.09-1.64 (m, 3H), 0.00 (s, 9H). MS-ESI [M+H]⁺: 1037.0.

### Step C: (S)-3-{[(N-methyl{[N-methyl({N-methyl[(N-methyl{[N-methyl({N-methyl[(N-methyl{[N-methyl({N-methyl[(N-methylacetamido)methyl]carbonylamino}methyl)carbonylamino]methyl}carbonylamino)methyl]ca rbonylamino}methyl)carbonylamino]methyl}carbonylamino)methyl]carbonylamino}methyl)carbon ylamino]methyl}carbonylamino)methyl]carbonylamino}-2-(2,5-dioxo-3-pyrrolidin-1-yl)propanoic acid

At room temperature, cesium fluoride (61.0 mg, 0.4 mmol) was added to a solution of 2-(trimethylsilyl)ethyl (*S*)-3-{[(*N*-methyl{[*N*-methyl({*N*-methyl[(*N*-methyl{[*N*-methyl({*N*-methyl[(*N-*methyl{[*N*-methyl({*N*-methyl[(*N-*methylacetamido)methyl]carbonylamino}methyl)carbonylamino]methyl}carbonylamino)methyl]ca rbonylamino}methyl)carbonylamino]methyl}carbonylamino)methyl]carbonylamino}methyl)carbon ylamino]methyl}carbonylamino)methyl]carbonylamino}-2-(2,5-dioxo-3-pyrrolidin-1-yl)propanoate (100.0 mg, 0.1 mmol) in *N,N*-dimethylformamide (1 mL). The resulting reaction solution was stirred at room temperature for 2 hours. After the reaction was complete as monitored by LCMS, the reaction solution was purified by pre-HPLC (C18, (0.01% HCl(aq)/ACN)/ACN = 5%) to give (*S*)-3-{[(*N-*methyl{[*N*-methyl({*N*-methyl[(*N*-methyl{[*N*-methyl({*N*-methyl[(*N*-methyl{[*N*-methyl({*N-*methyl[(*N-*methylacetamido)methyl]carbonylamino}methyl)carbonylamino]methyl}carbonylamino)methyl]ca rbonylamino}methyl)carbonylamino]methyl}carbonylamino)methyl]carbonylamino}methyl)carbon ylamino]methyl}carbonylamino)methyl]carbonylamino}-2-(2,5-dioxo-3-pyrrolidin-1-yl)propanoic acid (50 mg, 53.4% yield) as a white solid. ¹H NMR (400 MHz, DMSO-*d*₆) δ 8.49-7.93 (m, 1H), 7.15-7.07 (m, 2H), 4.82-4.71 (m, 1H), 4.48-3.68 (m, 31H), 3.58-3.45(m, 1H), 3.04-2.65 (m, 20H), 2.09-1.64 (m, 3H). MS-ESI [M+H]⁺: 937.3.

### Preparation of Intermediate B22: (S)-2-(2,5-dioxo-3-pyrrolidin-1-yl)-3-(2,5,8,11,14,17,20,23,26-nonaoxaheptacosan-27-ylcarbonylamino)propanoic acid

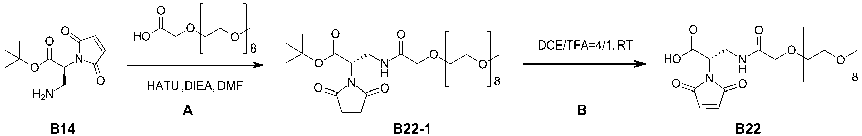

### Step A: tert-butyl (S)-2-(2,5-dioxo-3-pyrrolidin-1-yl)-3-(2,5,8,11,14,17,20,23,26-nonaoxaheptacosan-27-ylcarbonylamino)propanoate

Under ice-water bath, 2-(7-azabenzotriazol-1-yl)-*N*,*N*,*N'*,*N'*-tetramethyluronium hexafluorophosphate (456.2 mg, 1.2 mmol) and *N,N*-diisopropylethylamine (258 mg, 2.0 mmol) were sequentially added to a reaction solution of tert-butyl (*S*)-3-amino-2-(2,5-dioxo-3-pyrrolidin-1-yl)propanoate (276.5 mg, 1.0 mmol) and 3,6,9,12,15,18,21,24,27-nonaoxaoctacosanoic acid (442.5 mg, 1.0 mmol) in *N,N-*dimethylformamide (3 mL), stirred under ice-water bath for 2 hours. The reaction solution was purified by pre-HPLC (C18, (0.1% TFA(aq)/ACN)/ACN = 35-45%) to give the product tert-butyl (*S*)-2-(2,5-dioxo-3-pyrrolidin-1-yl)-3-(2,5,8,11,14,17,20,23,26-nonaoxaheptacosan-27-ylcarbonylamino)propanoate (335 mg, 50.4% yield) as a yellow oil. ¹H NMR (400 MHz, DMSO-*d*₆) δ 7.91 (t, *J =* 6.2 Hz, 1H), 7.11 (s, 2H), 4.66 (dd, *J =* 10.0, 4.2 Hz, 1H), 3.79 (s, 2H), 3.77-3.68 (m, 1H), 3.62-3.40 (m, 33H), 3.24 (s, 3H), 1.37 (s, 9H). MS-ESI [M+H]⁺: 665.4.

### Step B: (S)-2-(2,5-dioxo-3-pyrrolidin-1-yl)-3-(2,5,8,11,14,17,20,23,26-nonaoxaheptacosan-27-ylcarbonylamino)propanoic acid

Under ice-water bath, trifluoroacetic acid (1 mL) was added dropwise to a solution of tert-butyl (*S*)-2-(2,5-dioxo-3-pyrrolidin-1-yl)-3-(2,5,8,11,14,17,20,23,26-nonaoxaheptacosan-27-ylcarbonylamino)propanoate (100.0 mg, 0.15 mmol) in 1,2-dichloroethane (4 mL). After addition, the mixture was stirred at room temperature for 4 hours, the solvent was removed by concentration to obtain a crude product, which was purified by pre-HPLC (C18, (H₂O/ACN)/ACN = 15-20%) to give (*S*)-2-(2,5-dioxo-3-pyrrolidin-1-yl)-3-(2,5,8,11,14,17,20,23,26-nonaoxaheptacosan-27-ylcarbonylamino)propanoic acid (63.9 mg, 70% yield) as a yellow oil. ¹H NMR (400 MHz, DMSO-*d*₆) δ 7.92 (t, *J =* 6.3 Hz, 1H), 7.08 (s, 2H), 4.65 (dd, *J =* 10.2, 4.2 Hz, 1H), 3.78 (s, 2H), 3.76-3.72 (m, 1H), 3.65-3.59 (m, 1H), 3.58-3.40 (m, 33H), 3.24 (s, 3H). MS-ESI [M+H]⁺: 609.3.

### Preparation of Intermediate B23: methyl (2S,3S,4S,5R)-6-(2-{2-[(S)-2-[(S)-2-(S)2-{(9H-fluoren-9-yl)methyl}propanamido]propanamido]-3-formylpropanamido]-5-(hydroxymethyl)phenoxy}ethoxy)-3,4,5-triacetoxytetrahydro-2H-pyran-2-carboxylate

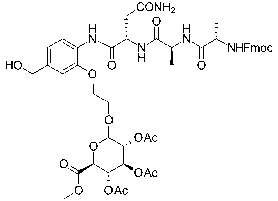

Intermediate **B23** was prepared following the synthetic method for intermediate **B24** below, using 3-hydroxy-4-nitrobenzaldehyde instead of 2-hydroxy-4-nitrobenzaldehyde in **Step A.** MS-ESI [M+NH]⁺: 978.3.

### Preparation of Intermediate B24: methyl (2S,3S,4S,5R)-6-(2-{5-[(S)-2-[(S)-2-(S)-2-{(9H-fluoren-9-yl)methyl}propanamido]propanamido]-4-(aminooxy)butanamido]-2-(hydroxymethyl)phenoxy}ethoxy)-3,4,5-triacetoxytetrahydro-2H-pyran-2-carboxylate

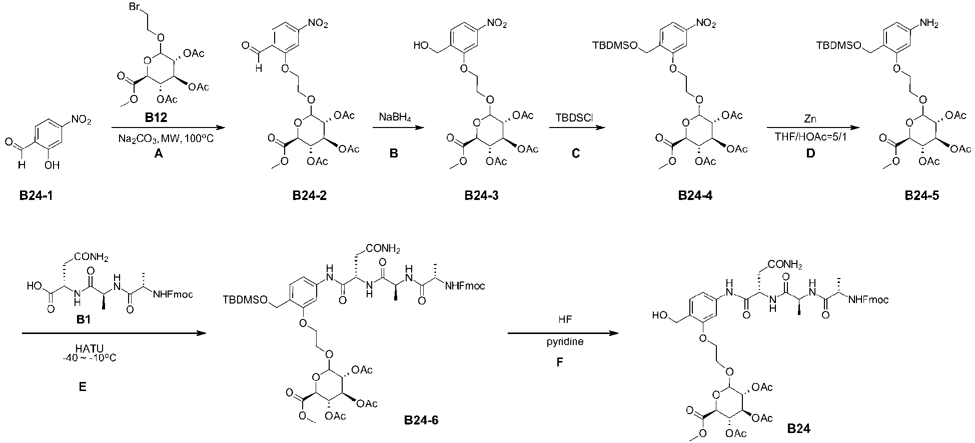

### Step A: methyl (2S,3S,4S,5R)-3,4,5-triacetoxy-6-[2-(2-formyl-5-nitrophenoxy)ethoxy]tetrahydro-2H-pyran-2-carboxylate

At room temperature, methyl (2S,3S,4S,5R)-3,4,5-triacetoxy-6-(2-bromoethoxy)tetrahydro-2*H-*pyran-2-carboxylate (3.60 g, 8.18 mmol) and 2-hydroxy-4-nitrobenzaldehyde (1.80 g, 10.78 mmol) were added to anhydrous N,N-dimethylformamide (21 mL). Sodium carbonate (1.5 g, 14.71 mmol) and potassium iodide (0.21 g, 1.27 mmol) were added. The reaction solution was purged with nitrogen for 1 minute, sealed, and microwaved at 100 °C for 1.5 hours. The reaction solution was cooled to room temperature, ethyl acetate (100 mL) and saturated aqueous ammonium chloride (50 mL) were added. The organic phase was washed with water (3 × 30 mL), dried over anhydrous sodium sulfate, concentrated, and purified by FCC (SiO₂, ACN/DCM = 0-10%) to give a crude product, which was then hot slurried with petroleum ether/ethyl acetate = 10/1 (20 mL) at 55 °C, cooled to room temperature, and filtered to give methyl (2S,3S,4S,5R)-3,4,5-triacetoxy-6-[2-(2-formyl-5-nitrophenoxy)ethoxy]tetrahydro-2*H*-pyran-2-carboxylate (2.01 g, 46.7% yield). MS-ESI [M+Na]⁺: 550.2.

### Step B: methyl (2S,3S,4S,5R)-3,4,5-triacetoxy-6-12-[2-(hydroxymethyl)-5-nitrophenoxy]ethoxy}tetrahydro-2H-pyran-2-carboxylate

Methyl (2*S*,3*S*,4*S*,5*R*)-3,4,5-triacetoxy-6-[2-(2-formyl-5-nitrophenoxy)ethoxy]tetrahydro-2*H-*pyran-2-carboxylate (2.00 g, 3.80 mmol) was dissolved in chloroform (35 mL) and n-propanol (7 mL). At 0 °C, 100-200 mesh silica gel (1.2 g) and sodium borohydride (0.20 g, 5.26 mmol) were added. The mixture was stirred at 0 °C for 1 hour. Dichloromethane (50 mL) and water (20 mL) were added, stirred for 15 minutes, and the organic phase was dried over anhydrous sodium sulfate, filtered, and concentrated to give methyl (2*S*,3*S*,4*S*,5*R*)-3,4,5-triacetoxy-6-{2-[2-(hydroxymethyl)-5-nitrophenoxy]ethoxy}tetrahydro-2*H*-pyran-2-carboxylate (2.00 g, 99.9% yield). ¹H NMR (400 MHz, Chloroform-d) δ 7.88 (dd, *J* = 8.2, 2.0 Hz, 1H), 7.69 (d, *J* = 2.2 Hz, 1H), 7.52 (d, *J* = 8.2 Hz, 1H), 5.30-5.22 (m, 2H), 5.02 (dd, *J* = 9.0, 7.6 Hz, 1H), 4.78-4.70 (m, 3H), 4.30-4.26 (m, 2H), 4.24-4.20 (m, 1H), 4.10-4.07 (m, 1H), 4.02-3.99 (m, 1H), 3.75 (s, 3H), 2.03 (s, 3H), 2.02 (s, 3H), 1.98 (s, 3H). MS-ESI [M+Na]⁺: 552.2.

### Step C: methyl (2S,3S,4S,5R)-3,4,5-triacetoxy-6-[2-(2-{ [(tert-butyl)dimethylsilyloxy]methyl}-5-nitrophenoxy)ethoxy]tetrahydro-2H-pyran-2-carboxylate

Methyl (2*S*,3*S*,4*S*,5*R*)-3,4,5-triacetoxy-6-{2-[2-(hydroxymethyl)-5-nitrophenoxy]ethoxy }tetrahydro-2*H*-pyran-2-carboxylate (2.00 g, 3.80 mmol) was dissolved in dichloromethane (30 mL). tert-Butyldimethylsilyl chloride (1.20 g, 7.95 mmol) and imidazole (0.54 g, 7.94 mmol) were added, and stirred at room temperature overnight. The reaction was quenched by adding saturated aqueous sodium bicarbonate (30 mL). The organic phase was washed with water and saturated brine, concentrated, and purified by FCC (SiO₂, ethyl acetate/petroleum ether = 0-50%) to give methyl (2*S*,3*S*,4*S*,5*R*)-3,4,5-triacetoxy-6-[2-(2-{[(tert-butyl)dimethylsilyloxy]methyl}-5-nitrophenoxy)ethoxy]tetrahydro-2*H*-pyran-2-carboxylate (2.07 g, 84.8% yield). MS-ESI [M+Na]⁺: 666.2.

### Step D: methyl (2S,3S,4S,5R)-3,4,5-triacetoxy-6-[2-(5-amino-2-{[(tert-butyl)dimethylsilyloxy]methyl}phenoxy)ethoxy]tetrahydro-2H-pyran-2-carboxylate

Methyl (2*S*,3*S*,4*S*,5*R*)-3,4,5-triacetoxy-6-[2-(2-{[(tert-butyl)dimethylsilyloxy]methyl}-5-nitrophenoxy)ethoxy]tetrahydro-2*H*-pyran-2-carboxylate (1.00 g, 1.55 mmol) was dissolved in tetrahydrofuran (30 mL) and acetic acid (1.5 mL). Zinc powder (1.60 g, 30.85 mmol) was added, and the mixture was stirred at room temperature for 3 hours, filtered, and the filtrate was quenched with a mixture of saturated brine (30 mL) and saturated aqueous sodium bicarbonate (30 mL), washed with saturated brine, dried over anhydrous sodium sulfate, filtered, and concentrated to give methyl (2*S*,3*S*,4*S*,5*R*)-3,4,5-triacetoxy-6-[2-(5-amino-2-{[(tert-butyl)dimethylsilyloxy]methyl}phenoxy)ethoxy]tetrahydro-2*H*-pyran-2-carboxylate (0.95 g, 99% yield). MS-ESI [M+H]⁺: 614.3.

### Step E: methyl (2S,3S,4S,5R)-6-(2-{5-[(S)-2-[(S)-2-(S)-2-{(9H-fluoren-9-yl)methyl}propanamido]propanamido]-4-(aminooxy)butanamido]-2-{[(tert-butyl)dimethylsilyloxy]methyl}phenoxy}ethoxy)-3,4,5-triacetoxytetrahydro-2H-pyran-2-carboxylate

Methyl (2*S*,3*S*,4*S*,5*R*)-3,4,5-triacetoxy-6-[2-(5-amino-2-{[(tert-butyl)dimethylsilyloxy]methyl}phenoxy)ethoxy]tetrahydro-2H-pyran-2-carboxylate (0.90 g, 1.47 mmol) and (*S*)-2-[(*S*)-2-[(*S*)-2- {(9*H*-fluoren-9-yl)methyl}propanamido]propanamido]-4-(aminooxy)butanoic acid (0.67 g, 1.35 mmol) were dissolved in *N,N*-dimethylformamide (30 mL) and cooled to -40 °C. HATU (0.54 g, 1.42 mmol) dissolved in *N,N-*dimethylformamide (3 mL) was added, followed by N,N-diisopropylethylamine (0.21 g, 1.63 mmol). The temperature was warmed to -10 °C, stirred for 0.5 hours, then slowly warmed to room temperature and stirred for another 0.5 hours. The reaction solution was poured into ice water, and a solid precipitated, filtered, and washed with water. The resulting solid was dried with an oil pump to give methyl (2*S*,3*S*,4*S*,5*R*)-6-(2-{5-[(*S*)-2-[(*S*)-2-(*S*)-2-{(9*H*-fluoren-9-yl)methyl}propanamido]propanamido]-4-(aminooxy)butanamido]-2-{[(tert-butyl)dimethylsilyloxy]methyl}phenoxy}ethoxy)-3,4,5-triacetoxytetrahydro-2*H*-pyran-2-carboxylate (1.18 g, 80.6% yield). ¹H NMR (400 MHz, DMSO-*d*₆) δ 7.87 (d, *J* = 7.4 Hz, 2H), 7.69 (t, *J* = 8.0 Hz, 2H), 7.40 (t, *J =* 7.4 Hz, 2H), 7.37-7.29 (m, 3H), 7.25-7.18 (m, 2H), 5.30 (t, *J =* 9.6 Hz, 1H), 4.98-4.90 (m, 2H), 4.82-4.77 (m, 1H), 4.61-4.59 (m, 3H), 4.40 (d, *J =* 10.0 Hz, 1H), 4.29-4.15 (m, 4H), 4.08-3.96 (m, 4H), 3.92-3.83 (m, 1H), 3.60 (s, 3H), 2.60-2.55 (m, 2H), 1.96 (s, 3H), 1.93 (s, 3H), 1.80 (s, 3H), 1.23 (d, *J =* 7.0 Hz, 6H), 0.88 (s, 9H), 0.05 (d, *J =* 1.2 Hz, 6H). MS-ESI [M+Na]⁺: 1114.3.

### Step F: methyl (2S,3S,4S,5R)-6-(2-{5-[(S)-2-[(S)-2-(S)-2-{(9H-fluoren-9-yl)methyl}propanamido]propanamido]-4-(aminooxy)butanamido]-2-(hydroxymethyl)phenoxy}ethoxy)-3,4,5-triacetoxytetrahydro-2H-pyran-2-carboxylate

Methyl (2*S*,3*S*,4*S*,5*R*)-6-(2-{5-[(*S*)-2-[(*S*)-2-(*S*)-2-{(9*H*-fluoren-9-yl)methyl}propanamido]propanamido]-4-(aminooxy)butanamido]-2-{[(tert-butyl)dimethylsilyloxy]methyl}phenoxy}ethoxy)-3,4,5-triacetoxytetrahydro-2*H*-pyran-2-carboxylate (1.18 g, 1.08 mmol) was dissolved in N,N-dimethylformamide (30 mL) under nitrogen and cooled to 0 °C. Hydrogen fluoride pyridine solution (6 mL, 70% pyridine solution) was added, and the mixture was slowly warmed to room temperature and stirred for 2 hours. The reaction solution was poured into ice water, and a solid precipitated, filtered, and washed with water. The resulting solid was dried with an oil pump to give methyl (2*S*,3*S*,4*S*,5*R*)-6-(2-{5-[(*S*)-2-[(*S*)-2-(*S*)-2-{(9*H-*fluoren-9-yl)methyl}propanamido]propanamido]-4-(aminooxy)butanamido]-2-(hydroxymethyl)phenoxy}ethoxy)-3,4,5-triacetoxytetrahydro-2*H*-pyran-2-carboxylate (0.74 g, 70.1% yield). ¹H NMR (400 MHz, DMSO-*d*₆) δ 8.21 (dd, *J =* 50.0, 7.0 Hz, 1H), 7.89 (d, *J =* 7.6 Hz, 2H), 7.70 (t, *J =* 8.0 Hz, 2H), 7.43 (t, *J =* 7.6 Hz, 2H), 7.38-7.30 (m, 3H), 7.27-7.23 (m, 2H), 5.32 (t, *J* = 8.0 Hz, 1H), 4.98-4.93 (m, 2H), 4.81 (t, *J =* 8.0 Hz, 1H), 4.62-4.60 (m, 1H), 4.44-4.41 (m, 3H), 4.34-4.14 (m, 4H), 4.05-4.02 (m, 4H), 3.89 (brs, 1H), 3.62 (s, 3H), 2.61-2.60 (m, 2H), 1.99 (s, 3H), 1.95 (s, 3H), 1.84 (s, 3H), 1.26 (d, *J =* 6.8 Hz, 6H). MS-ESI [M+Na]⁺: 1000.3.

### Preparation of Intermediate B25: (S)-1-[N-(S)-1-{N-(S)-1,2-bis[N-p-(hydroxymethyl)phenylcarbamoyl]ethylcarbamoyl}ethylcarbamoyl]ethyl (9H-fluoren-9-yl)methyl carbamate

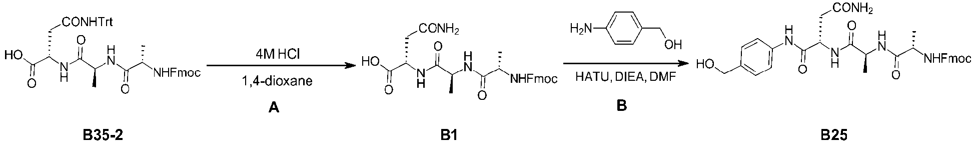

### Step A: (S)-2-[(S)-2-[(S)-2-{(9H-fluoren-9-yl)methyl}propanamido]propanamido]-3-formylpropanoic acid

((*S*)-3-(N-trimethylcarbamoyl)-2-[(*S*)-2-[(*S*)-2-{(9*H*-fluoren-9-yl)methyl}propanamido]propanamido]propanoic acid (9.50 g, 12.86 mmol) was added to hydrochloric acid in dioxane solution (4M, 60 mL) and stirred at 50 °C for 24 hours. The reaction solution was concentrated to dryness. The crude product was slurried with a mixture of methyl tert-butyl ether (50 mL) and dioxane (10 mL), filtered to give (*S*)-2-[(*S*)-2-[(*S*)-2-{(9*H*-fluoren-9-yl)methyl}propanamido]propanamido]-3-formylpropanoic acid (3.50 g, 54% yield). ¹H NMR (400 MHz, DMSO-*d*₆) δ 8.02 (t, *J =* 8.6 Hz, 2H), 7.89 (d, *J* = 7.6 Hz, 2H), 7.73 (t, *J* = 7.0 Hz, 2H), 7.52 (d, *J = 7.8* Hz, 1H), 7.43-7.38 (m, 3H), 7.35-7.33 (m, 2H), 6.92 (s, 1H), 4.52-4.47 (m, 1H), 4.32-4.21 (m, 4H), 4.10-4.03 (m, 1H), 2.51 (s, 2H), 1.21 (t, *J =* 7.6 Hz, 6H). MS-ESI [M+H]⁺: 497.2.

### Step B: (S)-1-[N(S)-1-{N(S)-1,2-bis[N-p-(hydroxymethyl)phenylcarbamoyl]ethylcarbamoyl}ethylcarbamoyl]ethyl (9H-fluoren-9-yl)methyl carbamate

(*S*)-2-[(*S*)-2-[(*S*)-2-{(9*H*-fluoren-9-yl)methyl}propanamido]propanamido]-3-formylpropanoic acid (3.70 g, 7.43 mmol) and (p-aminophenyl)methanol (2.01 g, 16.34 mmol) were dissolved in *N,N-*dimethylformamide (30 mL) and cooled to 0 °C. HATU (4.20 g, 11.05 mmol) was added, stirred for 5 minutes, then *N,N*-diisopropylethylamine (2.80 g, 21.71 mmol) was added. The mixture was warmed to room temperature and stirred overnight. The reaction solution was poured into ice water, stirred for 5 minutes, filtered, and the filter cake was washed with water and methyl tert-butyl ether. The resulting solid was dried with an oil pump to give (*S*)-1-[*N*-(*S*)-1-{*N*-(*S*)-1,2-bis[*N-p-*(hydroxymethyl)phenylcarbamoyl]ethylcarbamoyl}ethylcarbamoyl]ethyl (9H-fluoren-9-yl)methyl carbamate (3.20 g, 71.8% yield). MS-ESI [M+H]⁺: 602.3.

### Preparation of Intermediate B26: 4-(hydroxymethyl)phenyl 2-({N-methyl[(S)-1-[(S)-2-{(S)-2-[5-(2,5-dioxo-3-pyrrolin-1-yl)pentylcarbonylamino]propanamido}propanamido]-2-aminoethyl]carbonylamino}methyl)piperidine-1-carboxylate

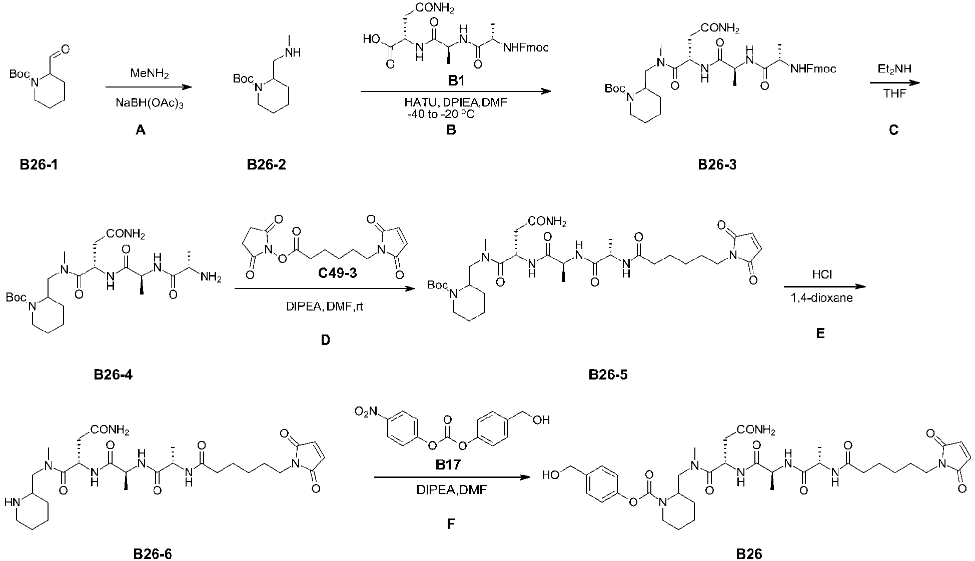

**Step A:** At room temperature, **B26-1** (3.0 g, 14.08 mmol) was dissolved in dichloromethane (80 mL). Methylamine hydrochloride (9.5 g, 140.85 mmol) and *N,N*-diisopropylethylamine (18.2 g, 141.09 mmol) were added, followed by slow addition of sodium triacetoxyborohydride (9.0 g, 42.45 mmol). The mixture was stirred at room temperature overnight. After the reaction was complete, water (200 mL) was added, and the aqueous phase was extracted with dichloromethane (3 × 100 mL). The organic phases were combined, dried over anhydrous sodium sulfate and filtered. The filtrate was concentrated, and the resulting crude product was purified by reverse phase C18 column (ACN/H₂O(0.5% NH₄HCO₃) = 0-50%) to give **B26-2** (1.1 g, 87% yield) as a colorless oil. MS-ESI [M+H]⁺: 229.1.

**Step B:** At -40 °C, **B1** (1.0 g, 2.01 mmol) was dissolved in DMF (10 mL), and **B26-2** (504 mg, 2.21 mmol), HATU (765 mg, 2.01 mmol), and *N,N*-diisopropylethylamine (520 mg, 4.03 mmol) were added. The reaction solution was warmed to -10 °C and stirred for 1 hour. The reaction system was then warmed to room temperature and stirred for another 2 hours. After the reaction was complete, the reaction solution was concentrated under vacuum. The crude product was purified by FCC (SiO₂, MeOH/DCM = 0-10%) to give **B26-3** (740 mg, 52% yield) as a white solid. MS-ESI [M+H]⁺: 707.4.

**Step** C: At room temperature, **B26-3** (740 mg, 1.05 mmol) was dissolved in tetrahydrofuran (16 mL), and diethylamine (4 mL) was added. The mixture was stirred at room temperature overnight. After the reaction was complete, the reaction solution was concentrated. The crude product was purified by reverse phase C18 column (ACN/H₂O(0.1% HCO₂H) = 0-50%) to give **B26-4** (460 mg, 91% yield) as a white solid. MS-ESI [M+H]⁺: 485.3.

**Step D:** At room temperature, **B26-4** (460 mg, 0.95 mmol) was dissolved in DMF (5 mL), and **C49-3** (325 mg, 1.06 mmol) and *N,N*-diisopropylethylamine (245 mg, 1.90 mmol) were added. The mixture was stirred at room temperature overnight. After the reaction was complete, the reaction solution was concentrated under vacuum. The crude product was purified by reverse phase C18 column (ACN/H₂O(0.1% HCO₂H) = 0-60%) to give **B26-5** (310 mg, 48% yield) as a white solid. MS-ESI [M+H]⁺: 678.4.

**Step E:** At room temperature, **B26-5** (310 mg, 0.46 mmol) was dissolved in dichloromethane (10 mL), and hydrogen chloride in 1,4-dioxane solution (4M, 10 mL) was added. The mixture was stirred at room temperature for 5 hours. After the reaction was complete, the solvent was evaporated to give **B26-6** (250 mg, 89% yield) as a white solid. MS-ESI [M+H]⁺: 578.3.

**Step F:** At room temperature, **B26-6** (250 mg, 0.41 mmol) was dissolved in DMF (5 mL), and **B17** (141 mg, 0.49 mmol) and N,N-diisopropylethylamine (105 mg, 0.81 mmol) were added. The mixture was stirred at room temperature for 5 hours. After the reaction was complete, the reaction solution was purified by reverse phase C18 column (ACN/H₂O(0.1% HCO₂H) = 0-60%) to give **B26** (220 mg, 70% yield) as a white solid. MS-ESI [M+H]⁺: 728.4.

### Preparation of Intermediate B27: (S)-1-[N-(S)-1-[N-(S)-1,2-bis[(2-{p-(hydroxymethyl)phenyl(oxycarbonylamino)}ethyl)-N-methylcarbamoyl]ethylcarbamoyl]ethylcarbamoyl]ethyl (9H-fluoren-9-yl)methyl carbamate

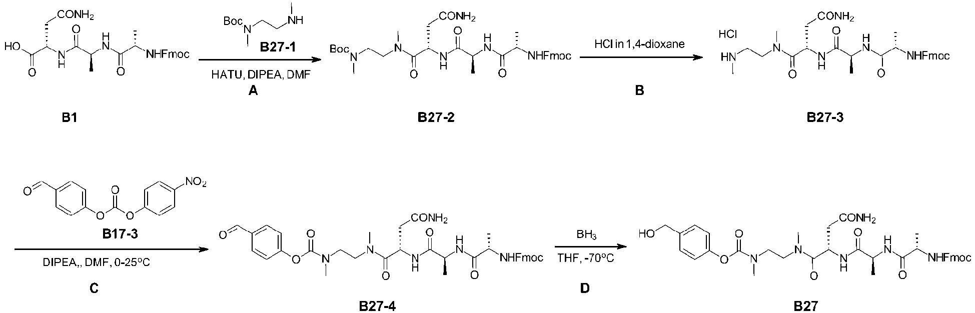

### Step A: 2-2-{N-methyl[(S)-1-[(S)-2-[(S)-2-{(9H-fluoren-9-yl)methyl}propanamido]propanamido]-2-carbamoylethyl]carbonylamino}ethyl2-methyl2-methyl-2-propane carbamate

(*S*)-2-[(*S*)-2-[(*S*)-2-{(9*H*-fluoren-9-yl)methyl}propanamido]propanamido]-3-carbamoylpropanoic acid (2.00 g, 4.02 mmol) and tert-butyl methyl[2-(methylamino)ethyl]carbamate (0.76 g, 4.02 mmol) were dissolved in dichloromethane (20 mL) and cooled to 0 °C. HATU (1.67 g, 4.42 mmol) and *N,N*-diisopropylethylamine (1.00 g, 8.04 mmol) were added sequentially. The mixture was stirred at 0 °C for 1 hour, then at room temperature for 1 hour. The reaction solution was washed with water, and the organic phase was concentrated and purified by FCC (SiO₂, methanol/dichloromethane = 0-15%) to give 2-2-{*N*-methyl[(*S*)-1-[(*S*)-2-[(*S*)-2-{(9*H*-fluoren-9-yl)methyl}propanamido]propanamido]-2-carbamoylethyl]carbonylamino}ethyl 2-methyl2-methyl-2-propane carbamate (1.72 g, 64.2% yield). MS-ESI [M+H-100]⁺: 667.4.

### Step B: (S)-1-[N-(S)-1-[N-(S)-1,2-bis{N-methyl[2-(methylamino)ethyl]carbamoyl}ethylcarbamoyl]ethylcarbamoyl]ethyl (9H-fluoren-9-yl)methyl carbamate hydrochloride

2-2-{*N*-methyl[(*S*)-1-[(*S*)-2-[(*S*)-2-{(9*H*-fluoren-9-yl)methyl}propanamido]propanamido]-2-carbamoylethyl]carbonylamino}ethyl 2-methyl 2-methyl-2-propane carbamate (1.72 g, 2.57 mmol) was dissolved in 1,4-dioxane (5 mL), and hydrochloric acid in dioxane solution (4M, 10 mL) was added. The mixture was stirred under nitrogen at room temperature for 2 hours. The reaction solution was concentrated to dryness, to give the crude product, and the crude product was slurried with a mixture of methyl tert-butyl ether (30 mL) and dioxane (5 mL), filtered to give (*S*)-1-[*N*-(*S*)-1-[*N-*(*S*)-1,2-bis{*N*-methyl[2-(methylamino)ethyl]carbamoyl}ethylcarbamoyl]ethylcarbamoyl]ethyl (9*H-*fluoren-9-yl)methyl carbamate hydrochloride (0.91 g, 62% yield). MS-ESI [M+H]⁺: 567.3.

### Step C: (S)-1-[N-(S)-1-[N-(S)-1,2-bis({2-[(p-formylphenyl)(methyl)(oxycarbonylamino)]ethyl}-N-methylcarbamoyl)ethylcarbamoyl]ethylcarbamoyll ethyl (9H-fluoren-9-yl)methyl carbamate

(*S*)-1-[*N*-(*S*)-1-[*N*(*S*)-1,2-bis{*N*-methyl[2-(methylamino)ethyl]carbamoyl}ethylcarbamoyl]ethylcarbamoyl]ethyl (9*H*-fluoren-9-yl)methyl carbamate hydrochloride (0.89 g, 1.49 mmol) was dissolved in *N,N*-dimethylformamide (80 mL) and cooled to 0 °C. *N,N*-Diisopropylethylamine (0.38 g, 2.98 mmol) was added and stirred for 5 minutes, then p-formylphenyl p-nitrophenyl carbonate (0.52 g, 1.81 mmol) was added. The mixture was stirred under nitrogen at room temperature for 2 hours. The reaction solution was concentrated under vacuum to dryness, and the crude product was slurried with a mixture of methyl tert-butyl ether (10 mL) and petroleum ether (10 mL), filtered to give (*S*)-1-[*N*-(*S*)-1-[*N-*(*S*)-1,2-bis({2-[(p-formylphenyl)(methyl)(oxycarbonylamino)]ethyl}*-N-*methylcarbamoyl)ethylcarbamoyl]ethylcarbamoyl]ethyl (9H-fluoren-9-yl)methyl carbamate (0.48 g, 45.3% yield). MS-ESI [M+H]⁺: 715.3.

### Step D: (S)-1-[N-(S)-1-[N-(S)-1,2-bis[(2-{p-(hydroxymethyl)phenyl(oxycarbonylamino)}ethyl)-N-methylcarbamoyl]ethylcarbamoyl]ethylcarbamoyl]ethyl (9H-fluoren-9-yl)methyl carbamate

(*S*)-1-[*N*-(*S*)-1-[*N*-(*S*)-1,2-bis({2-[(p-formylphenyl)(methyl)(oxycarbonylamino)]ethyl}-*N-*methylcarbamoyl)ethylcarbamoyl]ethylcarbamoyl]ethyl (9*H*-fluoren-9-yl)methyl carbamate (0.12 g, 0.17 mmol) was dissolved in anhydrous tetrahydrofuran (12 mL), cooled to -70 °C under nitrogen, and a solution of borane in tetrahydrofuran (1M, 1 mL, 1.00 mmol) was added. The mixture was stirred at -70 °C for 6 hours, then methanol (10 mL) was added and stirred at -10 °C for 0.5 hours to quench. The product (*S*)-1-[*N*-(*S*)-1-[*N*-(*S*)-1,2-bis({2-[(p-formylphenyl)(methyl)(oxycarbonylamino)]ethyl}*-N-*methylcarbamoyl)ethylcarbamoyl]ethylcarbamoyl]ethyl (9*H*-fluoren-9-yl)methyl carbamate was used directly in the next step in the reaction solution. MS-ESI [M+H]⁺: 717.3.

### Preparation of Intermediate B28: 2-({N-methyl[(S)-1-[(S)-2-[(S)-2-{(9H-fluoren-9-yl)methyl}propanamido]propanamido]-2-aminoethyl]carbonylamino}methyl)benzoic acid

### Preparation of Intermediate B29: (S)-1-[N-(S)-1-[N-(S)-1,2-bis[({2-[N-4-(hydroxymethyl)phenylcarbamoyl]phenyl}methyl)-N-methylcarbamoyl]ethylcarbamoyl]ethyl (9H-fluoren-9-yl)methyl carbamate

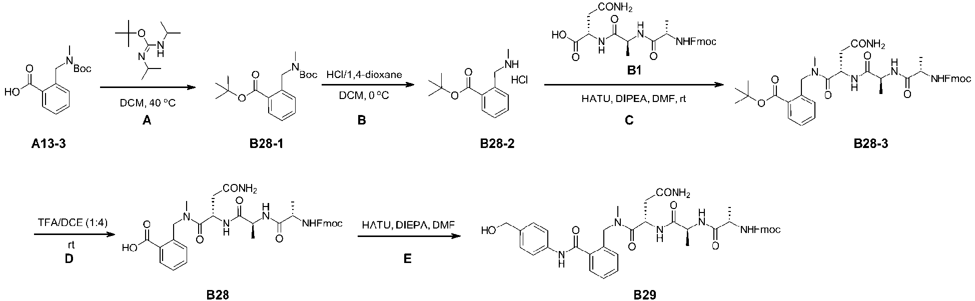

**Step A:** At room temperature, **A13-3** (2.0 g, 7.55 mmol) was dissolved in dichloromethane (10 mL), and *O*-tert-butyl-*N*,*N'*-diisopropylisourea (7.6 g, 38.00 mmol) was added. The mixture was heated to 40 °C and stirred for 8 hours. After the reaction was complete, the solvent was evaporated. The crude product was purified by FCC (SiO₂, EA/PE = 0-70%) to give **B28-1** (1.8 g, 74% yield) as a white solid. ¹H NMR (400 MHz, DMSO-*d*₆) δ 7.78 (d, *J* = 7.6, Hz, 1H), 7.57 (t, *J* = 7.6, Hz, 1H), 7.37 (t, *J* = 7.6, Hz, 1H), 7.17 (d, *J* = 7.6 Hz, 1H), 4.67 (s, 2H), 2.81 (s, 3H), 1.55 (s, 9H), 1.44 (s, 4.5H), 1.29 (s, 4.5H).

**Step B:** Under ice-water bath, **B28-1** (1.8 g, 5.61 mmol) was dissolved in dichloromethane (30 mL), and hydrogen chloride in 1,4-dioxane solution (4M, 10 mL) was added. The mixture was stirred under ice-water bath for 4 hours. After the reaction was complete, the solvent was evaporated to give **B28-2** (1.3 g, 93% yield) as a white solid. MS-ESI [M+H]⁺: 222.1.

**Step C:** Under ice-water bath, **B1** (2.0 g, 4.03 mmol) was dissolved in DMF (20 mL), and **B28-2** (1.3 g, 5.06 mmol), HATU (1.53 g, 4.03 mmol), and N,N-diisopropylethylamine (1.3 g, 10.08 mmol) were added. The reaction system was warmed to room temperature and stirred for 2 hours. After the reaction was complete, water (300 mL) was added, and the mixture was extracted with dichloromethane (3 × 50 mL). The organic phases were combined, dried over anhydrous sodium sulfate and filtered. The filtrate was concentrated, and the crude product was purified by FCC (SiO₂, MeOH/DCM = 0-5%) to give **B28-3** (1.8 g, 64% yield) as a white solid. MS-ESI [M+H]⁺: 700.3.

**Step D:** At room temperature, **B28-3** (1.8 g, 2.57 mmol) was dissolved in 1,2-dichloroethane (20 mL), and trifluoroacetic acid (4 mL) was added. The reaction solution was warmed to room temperature and stirred for 30 minutes. After the reaction was complete, the solvent was evaporated. Water (300 mL) was added, and the mixture was extracted with dichloromethane (3 × 50 mL). The organic phases were combined, dried over anhydrous sodium sulfate, filtered, and concentrated to give **B28** (1.0 g, 60% yield) as a white solid. MS-ESI [M+H]⁺: 644.3.

**Step E:** At room temperature, **B28** (350 mg, 0.54 mmol) was dissolved in DMF (5 mL), and HATU (210 mg, 0.55 mmol) and *N,N-*diisopropylethylamine (175 mg, 1.36 mmol) were added. The mixture was stirred at room temperature for 2 hours. After the reaction was complete, water (200 mL) was added, and the mixture was extracted with dichloromethane (3 × 50 mL). The organic phases were combined, dried over anhydrous sodium sulfate and filtered. The filtrate was concentrated, and the resulting crude product was purified by FCC (SiO₂, MeOH/DCM = 0-10%) to give **B29** (330 mg, 81% yield) as a white solid. MS-ESI [M+H]⁺: 749.3.

### Preparation of Intermediate B30: (1R,2S,3R,4R)-1-[(2-{5-[(S)-2-[(S)-2-(S)-2-{(9H-fluoren-9-yl)methyl}propanamido]propanamido]-3-carbamoylpropanamido]-2-(hydroxymethyl)phenoxy}ethyl)-N-methylcarbamoyl]-2,3,4,5-tetraacetoxypentyl acetate

### Preparation of Intermediate B31: (S)-1-[N-(S)-1-[N-(S)-1,2-bis[N-3-(2-{N-methyl[(N-methyl{[N-methyl({N-methyl[(N-methyl{[N-methyl({N-methyl[(N-methyl{[N-methyl({N-methyl[(N-methylacetamido)methyl]carbonylamino}methyl)carbonylamino]methyl}carbonylamino)methyl]ca rbonylamino}methyl)carbonylamino]methyl}carbonylamino)methyl]carbonylamino}methyl)carbon ylamino]methyl}carbonylamino)methyl]carbonylamino}ethoxy)-4-(hydroxymethyl)phenylcarbamoyl]ethylcarbamoyl]ethylcarbamoyl]ethyl (H-fluoren-9-yl)methyl carbamate

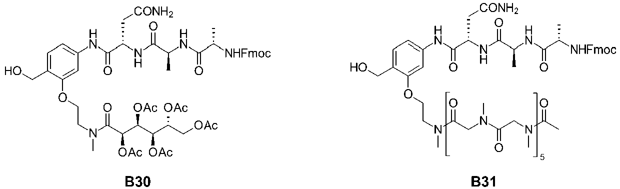

Intermediates **B30** and **B31** were prepared following the synthetic method for intermediate **B32** below, using 2,3,4,5,6-pentaacetylgluconic acid and intermediate **B16,** respectively, instead of 3,6,9,12,15,18,21,24,27-nonaoxaoctacosanoic acid in **Step I. B30** MS-ESI [M+NH]⁺: 1085.3; **B31** MS-ESI [M+NH]⁺: 1449.6.

### Preparation of Intermediate B32: (S)-1-[N-(S)-1-[N-(S)-1,2-bis{N-3-[2-(N-methyl-2,5,8,11,14,17,20,23,26-nonaoxacyclooctacosan-27-ylcarbonylamino)ethoxy]-4-(hydroxymethyl)phenylcarbamoyl}ethylcarbamoyl]ethylcarbamoyl]ethyl (9H-fluoren-9-yl)methyl carbamate

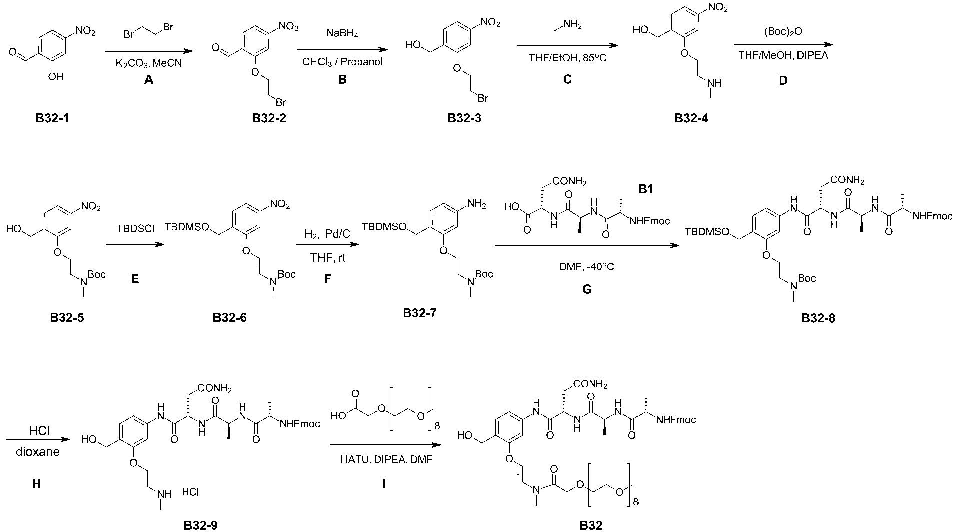

### Step A: 2-(2-bromoethoxy)-4-nitrobenzaldehyde

(2-Hydroxy-4-nitrobenzaldehyde (15.00 g, 89.82 mmol) and 1,2-dibromoethane (50.60 g, 269.15 mmol) were added to anhydrous acetonitrile (150 mL). Potassium carbonate (31.20 g, 224.46 mmol) was added, and the mixture was stirred under nitrogen at 85 °C overnight. Cooled to room temperature, ethyl acetate (100 mL) was added, filtered, and the filtrate was concentrated and purified by FCC (SiO₂, ethyl acetate/petroleum ether = 0-30%) to give 2-(2-bromoethoxy)-4-nitrobenzaldehyde (12.6 g, 51.2% yield). ¹H NMR (400 MHz, Chloroform-d) δ 10.59 (d, *J =* 0.8 Hz, 1H), 8.03 (d, *J =* 12 Hz, 1H), 7.94-7.92 (m, 1H), 7.84 (d, *J* = 2.0 Hz, 1H), 4.55 (t, *J =* 5.6 Hz, 2H), 3.77 (t, *J =* 5.6 Hz, 2H). MS-ESI [M+H]⁺: 550.2.

### Step B: [2-(2-bromoethoxy)-4-nitrophenyl]methanol

Compound 2-(2-bromoethoxy)-4-nitrobenzaldehyde (12.60 g, 45.99 mmol) was dissolved in a mixture of chloroform (100 mL) and n-propanol (20 mL). At 0 °C, 100-200 mesh silica gel (4.00 g) and sodium borohydride (3.00 g, 78.95 mmol) were added. The mixture was stirred at 0 °C for 1 hour. Dichloromethane (50 mL), water (50 mL), and dilute hydrochloric acid (1M, 10 mL) were added, stirred for 5 minutes, filtered, and the organic phase was washed with 20% brine, dried over anhydrous sodium sulfate, filtered, and concentrated to give [2-(2-bromoethoxy)-4-nitrophenyl]methanol (12.69 g, 100% yield). MS-ESI [M-18]⁺: 258.0.

### Step C: {2-[2-(methylamino)ethoxy]-4-nitrophenyl}methanol

Compound [2-(2-bromoethoxy)-4-nitrophenyl]methanol (12.69 g, 45.99 mmol) was dissolved in tetrahydrofuran (20 mL), and methylamine in ethanol solution (100 mL, 20% ethanol solution) was added. The tube was sealed and stirred at 85 °C overnight. The reaction solution was concentrated to give a solid, which was slurried with ice water and filtered, then dried with an oil pump to give {2-[2-(methylamino)ethoxy]-4-nitrophenyl } methanol (10.39 g, 100% yield). MS-ESI [M-18]⁺: 227.1.

### Step D: 2-2-[2-(hydroxymethyl)-5-nitrophenoxy]ethyl 2-methyl-2-methyl-2-propyl carbamate

To a mixed solution of {2-[2-(methylamino)ethoxy]-4-nitrophenyl}methanol (10.39 g, 45.99 mmol) in tetrahydrofuran (80 mL) and methanol (80 mL) was added di-tert-butyl dicarbonate (20.10 g, 91.98 mmol) in portions. The mixture was cooled with an ice-water bath, and *N,N-*diisopropylethylamine (8.89 g, 68.98 mmol) was added. After addition, the mixture was stirred at room temperature for 2 hours. The solvent was removed by concentration, ethyl acetate (100 mL) was added, the organic phase was washed with water and saturated brine, dried over anhydrous sodium sulfate, filtered, concentrated, and purified by FCC (SiO₂, ethyl acetate/petroleum ether = 0-40%) to give 2-2-[2-(hydroxymethyl)-5-nitrophenoxy]ethyl 2-methyl-2-methyl-2-propyl carbamate (10.90 g, 72.7% yield). ¹H NMR (400 MHz, Chloroform-d) δ 7.83 (d, *J* = 6.6 Hz, 1H), 7.67 (d, *J =* 2.0 Hz, 1H), 7.42 (d, *J =* 6.6 Hz, 1H), 4.69 (brs, 2H), 4.19 (t, *J =* 5.0 Hz, 2H), 3.73 (brs, 2H), 2.98 (s, 3H), 1.48 (s, 9H). MS-ESI [M+Na]⁺: 349.1.

### Step E: 2-2-(2-{[(tert-butyl)dimethylsilyloxy]methyl}-5-nitrophenoxy)ethyl 2-methyl-2-methyl-2-propyl carbamate

2-2-[2-(hydroxymethyl)-5-nitrophenoxy]ethyl 2-methyl-2-methyl-2-propyl carbamate (10.90 g, 33.44 mmol) was dissolved in dichloromethane (100 mL). tert-Butyldimethylsilyl chloride (10.10 g, 66.88 mmol) and imidazole (4.78 g, 70.22 mmol) were added, and the mixture was stirred at room temperature for 2 hours. The reaction was quenched by adding saturated aqueous sodium bicarbonate (100 mL). The organic phase was washed with water and saturated ammonium chloride, dried over anhydrous sodium sulfate, concentrated, and purified by FCC (SiO₂, ethyl acetate/petroleum ether = 0-40%) to give 2-2-(2-{[(tert-butyl)dimethylsilyloxy]methyl}-5-nitrophenoxy)ethyl 2-methyl-2-methyl-2-propyl carbamate (14.40 g, 97.9% yield). ¹H NMR (400 MHz, DMSO-*d*₆) δ 7.91 (d, *J =* 8.4 Hz, 1H), 7.77 (d, *J =* 8.0 Hz, 1H), 7.61 (brs, 1H), 4.74 (s, 2H), 4.29-4.25 (m, 2H), 3.59 (t, *J =* 5.2 Hz, 2H), 2.87 (d, *J =* 7.6 Hz, 3H), 1.35 (d, *J =* 27.6 Hz, 9H), 0.92 (s, 9H), 0.10 (s, 6H). MS-ESI [M+H-100]⁺: 341.2.

### Step F: tert-butyl 2-(5-amino-2-{[(tert-butyl)dimethylsilyloxy]methyl}phenoxy)ethylcarbamate

2-2-(2-{[(tert-butyl)dimethylsilyloxy]methyl}-5-nitrophenoxy)ethyl 2-methyl-2-methyl-2-propyl carbamate (3.00 g, 6.80 mmol) was dissolved in tetrahydrofuran (100 mL). 10% Pd/C (0.30 g, 50% H₂O) was added, the atmosphere was replaced with hydrogen three times, and the mixture was stirred at room temperature overnight, filtered and concentrated to give tert-butyl 2-(5-amino-2-{[(tert-butyl)dimethylsilyloxy]methyl}phenoxy)ethylcarbamate (2.78 g, 99.7% yield). ¹H NMR (400 MHz, DMSO-*d*₆) δ 6.92 (d, *J =* 8.0 Hz, 1H), 6.18 (s, 1H), 6.13 (dd, *J =* 8.0, 2.0 Hz, 1H), 4.99 (s, 2H), 4.51 (s, 2H), 3.94 (d, *J =* 5.6 Hz, 2H), 3.51 (t, *J =* 5.4 Hz, 2H), 2.89 (d, *J =* 14.4 Hz, 3H), 1.38 (d, *J* = 8.4 Hz, 9H), 0.86 (s, 9H), 0.01 (s, 6H). MS-ESI [M-OTBDMS]⁺: 279.1.

### Step G: N-(S)-1-(N-4-{[(tert-butyl)dimethylsilyloxylmethyl}-3-{2-[(tert-butyl)(methyl)(oxycarbonylamino)]ethoxy}phenylcarbamoyl)-3-(aminooxy)propylcarbamoyl]ethylcarbamoyl]ethyl (9H-fluoren-9-yl)methyl carbamate

tert-Butyl 2-(5-amino-2-{[(tert-butyl)dimethylsilyloxy]methyl}phenoxy)ethylcarbamate (2.36 g, 5.76 mmol) and (*S*)-2-[(*S*)-2-[(*S*)-2-{(9*H*-fluoren-9-yl)methyl}propanamido]propanamido]-4-(aminooxy)butanoic acid (1.60 g, 3.22 mmol) were dissolved in *N,N*-dimethylformamide (20 mL). N,N-Diisopropylethylamine (0.62 g, 4.80 mmol) was added, and the mixture was cooled to -40 °C. HATU (1.70 g, 4.48 mmol) dissolved in *N,N*-dimethylformamide (3 mL) was slowly added to the reaction solution, and the mixture was stirred at -40 °C for 1 hour. The reaction solution was poured into ice water, stirred for 15 minutes, and the solid was obtained by filtration and dried with an oil pump. The solid was slurried with methyl tert-butyl ether/tetrahydrofuran = 4/1 (20 mL) at room temperature and filtered to give *N*-(*S*)-1-(*N*-4-{[(tert-butyl)dimethylsilyloxy]methyl}-3-{2-[(tert-butyl)(methyl)(oxycarbonylamino)]ethoxy}phenylcarbamoyl)-3-(aminooxy)propylcarbamoyl]ethylcarbamoyl]ethyl (9*H*-fluoren-9-yl)methyl carbamate (1.70 g, 59.7% yield). MS-ESI [M-Boc-OTBDMS]⁺: 657.3.

### Step H: (S)-1-[N(S)-1-[N-,2-bis{N-4-(hydroxymethyl)-3-[2-(methylamino)ethoxy]phenylcarbamoyl}ethylcarbamoyl]ethyl (9H-fluoren-9-yl)methyl carbamate hydrochloride

*N*-(*S*)-1-(*N*-4-{[(tert-butyl)dimethylsilyloxy]methyl}-3-{2-[(tert-butyl)(methyl)(oxycarbonylamino)]ethoxy}phenylcarbamoyl)-3-(aminooxy)propylcarbamoyl]ethylcarbamoyl]ethyl (9*H*-fluoren-9-yl)methyl carbamate (0.70 g, 0.79 mmol) was dissolved in 1,4-dioxane (30 mL), and hydrochloric acid in dioxane solution (4M, 10 mL) was added. The mixture was stirred at room temperature overnight. The reaction solution was concentrated to dryness to give (*S*)-1-[*N*-(*S*)-1-[*N*-,2-bis{*N*-4-(hydroxymethyl)-3-[2-(methylamino)ethoxy]phenylcarbamoyl}ethylcarbamoyl]ethyl (9*H*-fluoren-9-yl)methyl carbamate hydrochloride (0.70 g, crude). MS-ESI [M+H]⁺: 675.3.

### Step I: (S)-1-[N-(S)-1-[N-(S)-1,2-bis{N-3-[2-(N-methyl-2,5,8,11,14,17,20,23,26-nonaoxacyclooctacosan-27-ylcarbonylamino)ethoxy]-4-(hydroxymethyl)phenylcarbamoyl}ethylcarbamoyl]ethylcarbamoyl]ethyl (9H-fluoren-9-yl)methyl carbamate

(*S*)-1-[*N*-(*S*)-1-[*N*-,2-bis{*N*-4-(hydroxymethyl)-3-[2-(methylamino)ethoxy]phenylcarbamoyl}ethylcarbamoyl]ethyl (9*H*-fluoren-9-yl)methyl carbamate hydrochloride (0.70 g, crude) and *N,N*-diisopropylethylamine (0.20 g, 1.57 mmol) were dissolved in *N,N*-dimethylformamide (15 mL), stirred at 0 °C for 15 minutes, then cooled to -10 °C for later use. 3,6,9,12,15,18,21,24,27-Nonaoxaoctacosanoic acid (0.35 g, 0.79 mmol) and HATU (0.30 g, 0.79 mmol) were dissolved in *N,N*-dimethylformamide (8 mL), *N,N*-diisopropylethylamine (0.20 g, 1.57 mmol) was added, and the mixture was stirred at room temperature for 10 minutes. This reaction solution was added to the above reserved reaction solution, warmed to 0 °C, and stirred for 1 hour. The reaction was quenched by adding water, and directly purified by reverse phase column (ACN/H₂O = 5-40%) to give (*S*)-1-[*N*-(*S*)-1-[*N*-(*S*)-1,2-bis{*N*-3-[2-(*N*-methyl-2,5,8,11,14,17,20,23,26-nonaoxacyclooctacosan-27-ylcarbonylamino)ethoxy]-4-(hydroxymethyl)phenylcarbamoyl }ethylcarbamoyl]ethylcarbamoyl]ethyl (9*H*-fluoren-9-yl)methyl carbamate (0.10 g, 11.6% yield over two steps). MS-ESI [M-OH]⁺: 1081.5.

### Preparation of Intermediate B33: 2-({N-methyl[(S)-1-[(S)-2-[(S)-2-{(9H-fluoren-9-yl)methyl}propanamido]propanamido]-2-aminoethyl]carbonylamino}methyl)nicotinic acid

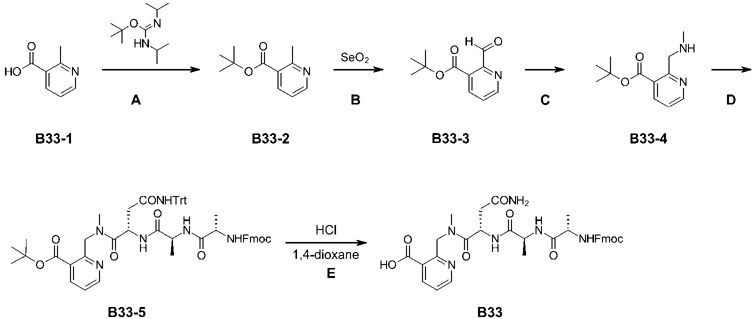

### Step A: tert-butyl 2-methylnicotinate

At room temperature, 2-methylnicotinic acid (10.0 g, 73.0 mmol) and *O*-tert-butyl-*N,N'-*diisopropylisourea (36.5 g, 182.5 mmol) were added to tetrahydrofuran (100 mL). After addition, the reaction solution was stirred at 50 °C for 2 hours. After the reaction was complete, the mixture was cooled to room temperature, filtered, and the filtrate was purified by FCC (SiO₂, EA/PE = 0-25%) to give tert-butyl 2-methylnicotinate (7.0 g, 50% yield) as a pale yellow oil. ¹H NMR (400 MHz, DMSO-*d*₆) δ 8.60 (dd, *J =* 4.8, 1.8 Hz, 1H), 8.09 (dd, *J =* 7.8, 1.8 Hz, 1H), 7.35 (dd, *J* = 7.8, 4.8 Hz, 1H), 2.69 (s, 3H), 1.56 (s, 9H). MS-ESI [M-OH]⁺: 194.1.

### Step B: tert-butyl 2-formylnicotinate

At room temperature, tert-butyl 2-methylnicotinate (3.2 g, 16.6 mmol) and selenium dioxide (25.0 g, 166.0 mmol) were added to 1,4-dioxane (30 mL). After addition, the reaction solution was stirred at 100 °C under nitrogen overnight. After the reaction was complete, the mixture was cooled to room temperature, filtered, and the filtrate was purified by FCC (SiO₂, EA/PE = 0-25%) to give tert-butyl 2-formylnicotinate (1.7 g, 49% yield) as a yellow oil. ¹H NMR (400 MHz, DMSO-*d*₆) δ 10.16 (s, 1H), 8.89 (dd, *J =* 4.8, 1.6 Hz, 1H), 8.15 (dd, *J =* 7.8, 1.6 Hz, 1H), 7.76 (dd, *J =* 7.8, 4.8 Hz, 1H), 1.56 (s, 9H). MS-ESI [M-OH]⁺: 208.1.

### Step C: tert-butyl 2-[(methylamino)methyl]nicotinate

At room temperature, tert-butyl 2-formylnicotinate (3.3 g, 16.1 mmol) and methylamine hydrochloride (3.3 g, 48.3 mmol) were added to methanol (35 mL), and *N*,*N*-diisopropylethylamine was used to adjust the pH to slightly basic. The reaction solution was stirred at room temperature for 30 minutes, then sodium cyanoborohydride (1.0 g, 16.1 mmol) was added. After addition, the mixture was stirred at room temperature for 2 hours, filtered, and the filtrate was purified by FCC (SiO₂, MeOH/DCM = 0-5%) to give tert-butyl 2-[(methylamino)methyl]nicotinate (2.1 g, 60% yield) as a red solid. ¹H NMR (400 MHz, DMSO-*d*₆) δ 9.19 (s, 1H), 8.82 (dd, *J =* 4.8, 1.6 Hz, 1H), 8.34 (dd, *J =* 7.8 1.6 Hz, 1H), 7.61 (dd, *J* = 7.8, 4.8 Hz, 1H), 4.63 (s, 2H), 2.69 (s, 3H), 1.58 (s, 9H). MS-ESI [M-OH]⁺: 223.1.

### Step D: tert-butyl 2-({N-methyl[(S)-2-(N-tritylcarbamoyl)-1-[(S)-2-[(S)-2-{(9H-fluoren-9-yl)methyl}propanamido]propanamido]ethyl]carbonylamino}methyl)nicotinate

Under ice-water bath, HATU (1107.8 mg, 2.9 mmol) and N,N-diisopropylethylamine (619.2 mg, 4.8 mmol) were sequentially added to a reaction solution of tert-butyl 2-[(methylamino)methyl]nicotinate (522.0 mg, 2.4 mmol) and (S)-3-(N-trimethylcarbamoyl)-2-[(S)-2-[(*S*)-2-{(9*H*-fluoren-9-yl)methyl}propanamido]propanamido]propanoic acid (2142.3 mg, 2.9 mmol) in N,N-dimethylformamide (10 mL). The mixture was stirred under ice-water bath for 2 hours. After the reaction was complete, the reaction solution was purified by pre-HPLC (C18, (0.1% TFA(aq)/ACN)/ACN = 35-45%) to give tert-butyl 2-({*N*-methyl[(*S*)-2-(*N*-tritylcarbamoyl)-1-[(*S*)-2-[(*S*)-2-{(9*H*-fluoren-9-yl)methyl}propanamido]propanamido]ethyl]carbonylamino}methyl)nicotinate (1357.9 mg, 60.0% yield) as a yellow solid. MS-ESI [M-OH]⁺: 943.4.

### Step E: 2-({N-methyl[(S)-1-[(S)-2-[(S)-2-{(9H-fluoren-9-yl)methyl}propanamido]propanamido]-2-aminoethyl]carbonylamino}methyl)nicotinic acid

Under ice-water bath, tert-butyl 2-({*N*-methyl[(*S*)-2-(*N*-tritylcarbamoyl)-1-[(*S*)-2-[(*S*)-2-{(9*H-*fluoren-9-yl)methyl}propanamido]propanamido]ethyl]carbonylamino } methyl)nicotinate (800.0 mg, 0.85 mmol) was added to hydrochloric acid in dioxane (4M, 8 mL). After addition, the mixture was stirred at 35 °C overnight. After the reaction was complete, the reaction solution was purified by pre-HPLC (C18, (0.1% TFA(aq)/ACN)/ACN = 25-45%) to give 2-({*N*-methyl[(*S*)-1-[(*S*)-2-[(*S*)-2-{(*9*H-fluoren-9-yl)methyl}propanamido]propanamido]-2-aminoethyl]carbonylamino}methyl)nicotinic acid (383.2 mg, 70.0% yield) as a pale yellow solid. MS-ESI [M-OH]⁺: 645.3.

### Preparation of Intermediate B34: 3-({N-methyl[(S)-1-[(S)-2-[(S)-2-{(9H-fluoren-9-yl)methyl}propanamido]propanamido]-2-formylethyl]carbonylamino}methyl)pyridine-2-carboxylic acid

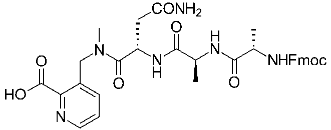

**Intermediate B34** was prepared following the synthetic method for intermediate **B33,** using 3-methyl-2-pyridinecarboxylic acid instead of 2-methylnicotinic acid in **Step A.** ¹H NMR (400 MHz, DMSO-*d*₆) δ 8.68-8.50 (m, 1H), 8.37 (d, *J =* 7.4 Hz, 1H), 7.99 (d, *J =* 7.2 Hz, 1H), 7.89 (d, *J =* 7.6 Hz, 2H), 7.82-7.48 (m, 4H), 7.47-7.11 (m, 5H), 6.93 (s, 1H), 5.11 (dd, *J =* 8.8, 5.4 Hz, 1H), 4.83 (d, *J* = 4.8 Hz, 1H), 4.29-4.04 (m, 4H), 3.08 (d, *J =* 4.4 Hz, 4H), 1.23-1.19 (m, 4H), 1.11 (s, 6H). MS-ESI [M+H]+: 645.3.

### Preparation of Intermediate B35: 6-(hydroxymethyl)pyridin-3-yl2-({N-methyl[(S)-1-[(S)-2-[(S)-2-{2-(trimethylsilyl)ethyl}propanamido]propanamido]-2-formylethyl]carbonylamino}methyl)piperidine-1-carboxylate

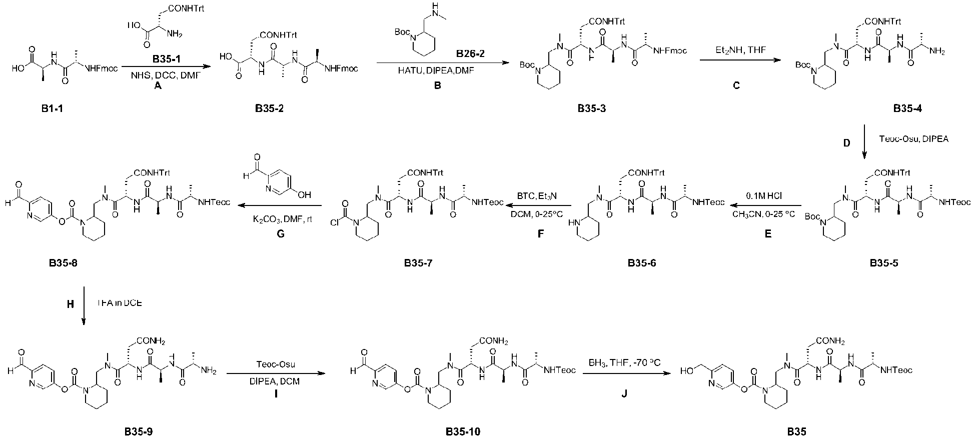

**Step A:** Under ice-water bath, **B1-1** (25.0 g, 65.37 mmol) was dissolved in DMF (435 mL). *N-*Hydroxysuccinimide (7.9 g, 68.64 mmol) and *N,N'*-dicyclohexylcarbodiimide (14.8 g, 71.74 mmol) were added. The mixture was warmed to room temperature and stirred overnight. After the reaction was complete, the mixture was filtered. Under ice-water bath, a solution of **B35-1** (24.5 g, 65.44 mmol) in DMF (100 mL) and *N,N*-diisopropylethylamine (16.9 g, 131.00 mmol) were added to the filtrate. The mixture was warmed to room temperature and stirred overnight. After the reaction was complete, the reaction solution was concentrated to about 50 mL. The reaction solution was added dropwise to excess methyl tert-butyl ether, and a large amount of solid precipitated, filtered, washed, collected the solid, and dried under vacuum to give **B35-2** (46.4 g, 96% yield) as a white solid. MS-ESI [M+Na]⁺: 761.3.

**Step B:** Under ice-water bath, **B35-2** (4.1 g, 5.56 mmol) was dissolved in DMF (25 mL). HATU (2.2 g, 5.79 mmol) and N,N-diisopropylethylamine (2.1 g, 16.27 mmol) were added. The reaction solution was stirred for 10 minutes, then **B26-2** (1.4 g, 6.14 mmol) was added. The mixture was warmed to room temperature and stirred for 2 hours. After the reaction was complete, water (300 mL) was added, and the mixture was extracted with dichloromethane (3 × 50 mL). The organic phases were combined, dried over anhydrous sodium sulfate and filtered. The filtrate was concentrated. The crude product obtained after concentration was purified by FCC (SiO₂, MeOH/DCM = 0-5%) to give **B35-3** (4.3 g, 89% yield) as a white solid. MS-ESI [M+H]⁺: 949.4.

**Step C:** At room temperature, **B35-3** (4.3 g, 4.53 mmol) was dissolved in tetrahydrofuran (30 mL), and diethylamine (5 mL) was added. The mixture was stirred at room temperature for 2 hours. After the reaction was complete, the reaction solution was concentrated. The crude product was purified by reverse phase C18 column (ACN/H₂O(0.1% NH₄HCO₃) = 0-70%) to give **B35-4** (2.0 g, 60% yield) as a white solid. MS-ESI [M+H]⁺: 727.4.

**Step D:** At room temperature, **B35-4** (2.0 g, 2.75 mmol) was dissolved in dichloromethane (15 mL). Teoc-Osu (1.1 g, 4.25 mmol) and triethylamine (835 mg, 8.27 mmol) were added. The mixture was stirred at room temperature for 2 hours. After the reaction was complete, the reaction solution was concentrated. The crude product was purified by reverse phase C18 column (ACN/H₂O(0.1% NH₄HCO₃) = 0-50%) to give **B35-5** (1.6 g, 68% yield) as a white solid. MS-ESI [M+H]⁺: 871.5.

**Step E:** Under ice-water bath, **B35-5** (1.6 g, 1.84 mmol) was dissolved in acetonitrile (50 mL), and concentrated hydrochloric acid (12M, 0.4 mL) was added. The mixture was stirred under ice-water bath for 12 hours. After the reaction was complete, water (100 mL) was added, and the reaction solution was adjusted to basic with sodium bicarbonate aqueous solution, then extracted with dichloromethane (3 × 100 mL). The organic phases were combined, dried over anhydrous sodium sulfate and filtered. The filtrate was concentrated. The crude product obtained after concentration was purified by reverse phase C18 column (ACN/H₂O(0.1% NH₄HCO₃) = 0-80%) to give **B35-6** (950 mg, 67% yield) as a white solid. MS-ESI [M+H]⁺: 771.4.

**Step F:** Under nitrogen protection and ice-water bath, **B35-6** (950 mg, 1.23 mmol) was dissolved in tetrahydrofuran (15 mL). Triphosgene (365 mg, 1.23 mmol) and triethylamine (500 mg, 4.95 mmol) were added. The mixture was warmed to room temperature and stirred for 2 hours. After the reaction was complete, dilute hydrochloric acid (0.1M, 100 mL) was added, and the mixture was extracted with dichloromethane (3 × 100 mL). The organic phases were combined, dried over anhydrous sodium sulfate, filtered, and concentrated to give **B35-7** (1.0 g, 98% yield) as a yellow solid. MS-ESI [M+H]⁺: 833.4.

**Step G:** At room temperature, **B35-7** (1.0 g, 1.20 mmol) was dissolved in DMF (5 mL). 2-Formyl-5-hydroxypyridine (740 mg, 6.02 mmol) and potassium carbonate (830 mg, 6.01 mmol) were added. The mixture was stirred at room temperature overnight. After the reaction was complete, water (400 mL) was added, and the mixture was extracted with dichloromethane (4 × 50 mL). The organic phases were combined, dried over anhydrous sodium sulfate and filtered. The crude product obtained after concentration was purified by FCC (SiO₂, MeOH/DCM = 0-5%) to give **B35-8** (490 mg, 45% yield) as a white solid. MS-ESI [M+H]⁺: 920.4.

**Step H:** At room temperature, **B35-8** (490 mg, 0.53 mmol) was dissolved in 1,2-dichloroethane (20 mL), and trifluoroacetic acid (5 mL) was added. The mixture was stirred at room temperature for 4 hours. After the reaction was complete, the reaction solution was concentrated to give crude **B35-9** (500 mg), which was used directly in the next step without further purification. MS-ESI [M+H]⁺: 534.3.

**Step I:** At room temperature, crude **B35-9** (500 mg) was dissolved in dichloromethane (20 mL). Teoc-Osu (210 mg, 0.81 mmol) and triethylamine (220 mg, 2.18 mmol) were added. The mixture was stirred at room temperature for 2 hours. The reaction solution was concentrated. The crude product was purified by reverse phase C18 column (ACN/H₂O(0.1% HCO₂H) = 0-40%) to give **B35-10** (205 mg, 57% yield over two steps) as a white solid. MS-ESI [M+H]⁺: 678.3.

**Step J:** At -70 °C, **B35-10** (205 mg, 0.30 mmol) was dissolved in tetrahydrofuran (5 mL), and a solution of borane in tetrahydrofuran (1M, 0.6 mL, 0.60 mmol) was added. The mixture was stirred at -70 °C for 1 hour. After the reaction was complete, the reaction was quenched by adding a small amount of methanol, and the mixture was warmed to room temperature. The reaction solution was concentrated, and the crude product was purified by reverse phase C18 column (ACN/H₂O(0.1% HCO₂H) = 0-40%) to give **B35** (110 mg, 53% yield) as a white solid. MS-ESI [M+H]⁺: 680.4.

### Preparation of Intermediate B36: 6-(hydroxymethyl)pyridin-3-yl 2-({N-methyl[(S)-1-[(S)-2-[(S)-2-{2-(trimethylsilyl)ethyl}propanamido]propanamido]-2-formylethyl]carbonylamino}methyl)-4-methylpiperazine-1-carboxylate

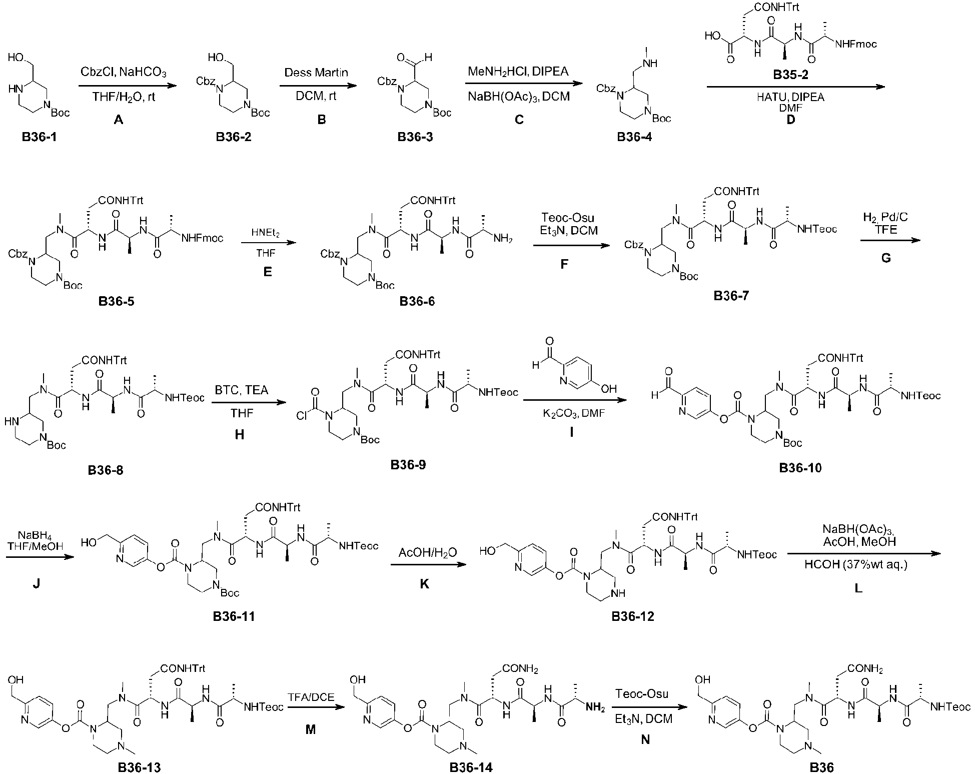

**Step A:** At room temperature, **B36-1** (20.0 g, 92.59 mmol) was dissolved in tetrahydrofuran (150 mL) and water (150 mL). CbzCl (16.5 g, 97.06 mmol) and NaHCO₃ (31.1 g, 370.23 mmol) were added. The mixture was stirred at room temperature overnight. After the reaction was complete, the aqueous phase was extracted with dichloromethane (3 × 100 mL). The organic phases were combined, dried over anhydrous sodium sulfate and filtered to give **B36-2** (30.0 g, 94% yield). ¹H NMR (400 MHz, DMSO-*d*₆) δ 7.40-7.27 (m, 5H), 5.11 (d, *J =* 12.0 Hz, 1H), 5.08 (d, *J =* 12.0 Hz, 1H), 4.83 (t, *J* = 5.2 Hz, 1H), 3.99-3.74 (m, 3H), 3.45-3.35 (m, 2H), 3.04-2.70 (m, 3H), 1.40 (s, 9H). MS-ESI [M+Na]⁺: 373.2.

**Step B:** At room temperature, **B36-2** (30.0 g, 85.71 mmol) was dissolved in dichloromethane (200 mL), and Dess-Martin periodinane (54.5 g, 128.54 mmol) was slowly added. The mixture was stirred at room temperature overnight. After the reaction was complete, the mixture was filtered. The filtrate was concentrated, and the resulting crude product was purified by FCC (SiO₂, EA/PE = 0-40%) to give **B36-3** (24.0 g, 80% yield) as a white solid. ¹H NMR (400 MHz, DMSO-*d*₆) δ 9.56 (s, 1H), 7.43-7.25 (m, 5H), 5.14-5.06 (m, 2H), 4.80-4.70 (m, 1H), 4.80-4.70 (m, 1H), 3.89-3.61 (m, 2H), 3.26-3.11 (m, 1H), 3.07-2.77 (m, 2H), 1.37 (s, 9H). MS-ESI [M+Na]⁺: 371.1.

**Step C:** At room temperature, **B36-3** (20.0 g, 57.47 mmol) was dissolved in dichloromethane (300 mL). Methylamine hydrochloride (39.1 g, 575.00 mmol) and N,N-diisopropylethylamine (74.2 g, 575.19 mmol) were added, followed by slow addition of sodium triacetoxyborohydride (24.4 g, 115.09 mmol). The mixture was stirred at room temperature for 4 hours. After the reaction was complete, water (500 mL) was added, and extracted with dichloromethane (3 × 200 mL). The organic phases were combined, dried over anhydrous sodium sulfate and filtered. The filtrate was concentrated, and the resulting crude product was purified by FCC (SiO₂, MeOH/DCM = 0-10%) to give **B36-4** (16.0 g, 77% yield) as a colorless oil. ¹H NMR (400 MHz, CDCl₃) δ 7.40-7.28 (m, 5H), 5.18 (d, *J =* 12.2 Hz, 1H), 5.13 (d, *J =* 12.2 Hz, 1H), 4.50-4.02 (m, 3H), 3.20-2.61 (m, 7H), 2.52-2.28 (m, 3H), 1.46 (s, 9H). MS-ESI [M+H]⁺: 364.2.

**Step D:** Under ice-water bath, **B35-2** (6.4 g, 8.66 mmol) was dissolved in DMF (40 mL). HATU (3.3 g, 8.68 mmol) and N,N-diisopropylethylamine (2.7 g, 20.93 mmol) were added. The reaction solution was stirred for 10 minutes, then **B36-4** (3.0 g, 8.26 mmol) was added. The system was warmed to room temperature and stirred for 2 hours. After the reaction was complete, water (300 mL) was added, and extracted with dichloromethane (3 × 100 mL). The organic phases were combined, dried over anhydrous sodium sulfate and filtered. The filtrate was concentrated, and the crude product was purified by FCC (SiO₂, MeOH/DCM = 0-5%) to give **B36-5** (6.5 g, 72% yield) as a white solid. MS-ESI [M+H]⁺: 1084.1.

**Step E:** At room temperature, **B36-5** (6.5 g, 6.00 mmol) was dissolved in tetrahydrofuran (30 mL), and diethylamine (5 mL) was added. The mixture was stirred at room temperature for 3 hours. After the reaction was complete, the reaction solution was concentrated. The crude product was purified by FCC (SiO₂, MeOH/DCM = 0-5%) to give **B36-6** (4.0 g, 76% yield) as a white solid. MS-ESI [M+H]⁺: 862.4

**Step F:** At room temperature, **B36-6** (4.0 g, 4.64 mmol) was dissolved in dichloromethane (20 mL). Teoc-Osu (1.4 g, 5.40 mmol) and triethylamine (1.4 g, 13.86 mmol) were added. The mixture was stirred at room temperature for 2 hours. After the reaction was complete, the reaction solution was concentrated. The resulting crude product was purified by FCC (SiO₂, MeOH/DCM = 0-5%) to give **B36-7** (4.0 g, 86% yield) as a white solid. MS-ESI [M+H]⁺: 1006.5.

**Step G:** At room temperature, **B36-7** (4.0 g, 3.98 mmol) was dissolved in trifluoroethanol (100 mL), and wet palladium on carbon (10% wt, 400 mg) was added. The atmosphere was replaced with hydrogen three times. The mixture was stirred at room temperature overnight. After the reaction was complete, the reaction solution was filtered through Celite. The filtrate was concentrated, and the resulting crude product was purified by FCC (SiO₂, MeOH/DCM = 0-5%) to give **B36-8** (3.2 g, 92% yield) as a white solid. MS-ESI [M+H]⁺: 872.5.

**Step H:** Under nitrogen protection and ice-water bath, **B36-8** (3.2 g, 3.67 mmol) was dissolved in tetrahydrofuran (40 mL). Triphosgene (1.1 g, 3.70 mmol) and triethylamine (1.5 g, 14.85 mmol) were added. The system was warmed to room temperature and stirred for 2 hours. After the reaction was complete, dilute hydrochloric acid (0.1M, 100 mL) was added, and the mixture was extracted with dichloromethane (3 × 100 mL). The organic phases were combined, dried over anhydrous sodium sulfate, filtered, and concentrated to give **B36-9** (3.3 g, 96% yield) as a yellow solid. MS-ESI [M+H]⁺: 934.4.

**Step I:** At room temperature, **B36-9** (3.3 g, 3.53 mmol) was dissolved in DMF (15 mL). 2-Formyl-5-hydroxypyridine (1.3 g, 10.57 mmol) and potassium carbonate (1.5 g, 10.87 mmol) were added. The mixture was stirred at room temperature for 4 hours. After the reaction was complete, water (500 mL) was added, and the mixture was extracted with dichloromethane (4 × 50 mL). The organic phases were combined, dried over anhydrous sodium sulfate and filtered. The crude product obtained after concentration was purified by FCC (SiO₂, MeOH/DCM = 0-5%) to give **B36-10** (4.0 g, 86% yield) as a white solid. MS-ESI [M+H]⁺: 1021.4.

**Step J:** Under ice-water bath, **B36-10** (2.0 g, 1.96 mmol) was dissolved in tetrahydrofuran (5 mL) and methanol (5 mL). Sodium borohydride (150 mg, 3.95 mmol) was added. The system was warmed to room temperature and stirred for 3 hours. After the reaction was complete, the reaction solution was concentrated. The crude product obtained after concentration was purified by FCC (SiO₂, MeOH/DCM = 0-5%) to give **B36-11** (1.5 g, 75% yield) as a white solid. MS-ESI [M+H]⁺: 1023.5.

**Step K:** At room temperature, **B36-11** (1.5 g, 1.47 mmol) was dissolved in water (30 mL) and acetic acid (30 mL). The mixture was stirred at 60 °C for 50 hours. The reaction system was cooled to room temperature. The reaction solution was concentrated and purified by reverse phase C18 column (ACN/H₂O (0.1% HCO₂H) = 0-40%) to give **B36-12** (950 mg, 70% yield) as a white solid. MS-ESI [M+H]⁺: 923.4.

**Step L:** At room temperature, **B36-12** (950 mg, 1.03 mmol) was dissolved in methanol (20 mL). Formaldehyde aqueous solution (37% wt, 835 mg, 10.30 mmol) and acetic acid (60 mg, 1.00 mmol) were added. The mixture was stirred at room temperature for 30 minutes, then sodium triacetoxyborohydride (1.09 g, 5.14 mmol) was added. The mixture was stirred at room temperature for 2 hours. The reaction solution was concentrated. The crude product obtained after concentration was purified by reverse phase C18 column (ACN/H₂O(0.1% NH₄HCO₃) = 0-40%) to give **B36-13** (450 mg, 47% yield) as a white solid. MS-ESI [M+H]⁺: 937.5.

**Step M:** At room temperature, **B36-13** (450 mg, 0.48 mmol) was dissolved in 1,2-dichloroethane (20 mL), and trifluoroacetic acid (5 mL) was added. The mixture was stirred at room temperature for 4 hours. After the reaction was complete, the reaction solution was concentrated to give crude **B36-14** (500 mg), which was used directly in the next step without further purification. MS-ESI [M+H]⁺: 551.3.

**Step N:** At room temperature, crude **B36-14** (500 mg) was dissolved in dichloromethane (20 mL). Teoc-Osu (187 mg, 0.72 mmol) and triethylamine (242 mg, 2.40 mmol) were added. The mixture was stirred at room temperature for 2 hours. After the reaction was complete, the reaction solution was concentrated. The crude product obtained after concentration was purified by reverse phase C18 column (ACN/H₂O(0.1% NH₄HCO₃) = 0-40%) to give **B36** (150 mg, 45% yield over two steps) as a white solid. MS-ESI [M+H]⁺: 695.4.

### Preparation of Intermediate B37: 6-(hydroxymethyl)pyridin-3-yl 4-[2-(N-methyl{[N-methyl({N-methyl[(N-methyl[N-methyl{N-methyl][(N-methylacetamido)methyl]carbonylamino}-methyl)carbonylamino]methyl}carbonylamino}methyl)carbonylamino]methyl}carbonylamino)met hyl]-methyl}carbonylamino)methyl]carbonylamino}methyl)](oxycarbonylamino)}propanamido]propana mido]-2-formylethyl]carbonylamino}methyl)piperazine-1-carboxylate

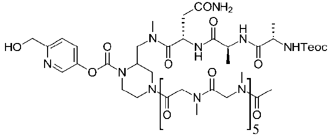

Intermediate **B37** was prepared following the synthetic method for intermediate **B36,** using amide condensation with intermediate **B16** instead of reductive amination with formaldehyde aqueous solution in **Step L.** MS-ESI [M+H]⁺: 1433.7.

### Preparation of Intermediate B38: 6-(hydroxymethyl)pyridin-3-yl 2-({N-methyl[(S)-1-[(S)-2-{(S)-2-(3,3-dimethyl-3-silabutyl)(oxycarbonylamino)]propanamido}propanamido]-2-formylethyl]carbonylamino}methyl)-4-(3,6,9,12,15,18,21,24,27-nonaoxacyclooctanosyl)piperazine-1-carboxylate

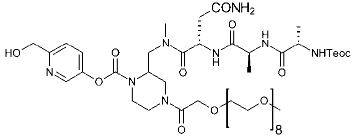

Intermediate **B38** was prepared following the synthetic method for intermediate **B36,** using amide condensation with 3,6,9,12,15,18,21,24,27-nonaoxaoctacosanoic acid instead of reductive amination with formaldehyde aqueous solution in **Step L.** ¹H NMR (400 MHz, DMSO-*d*₆) δ 8.43-7.97 (m, 2H), 7.93-6.56 (m, 6H), 5.45-5.38 (m, 1H), 5.05-4.93 (m, 1H), 4.63-3.71 (m, 22H), 3.60-3.52 (m, 8H), 3.16-2.60 (m, 25H), 2.32-1.66 (m, 2H), 1.26-0.78 (m, 8H), 0.02-0.01 (m, 9H). MS-ESI [M+H]⁺: 1105.5.

### Preparation of Intermediate B39: 6-(hydroxymethyl)pyridin-3-yl 4-[2-(N-methyl{[N-methyl({N-methyl[(N-methyl{[N-methyl({N-methyl[(N-methyl{[N-methyl({N-methyl[(N-methylacetamido)methyl]carbonylamino}methyl)carbonylamino]methyl}carbonylamino)methyl]ca rbonylamino}methyl)carbonylamino]methyl}carbonylamino)methyl]carbonylamino}methyl)carbon ylamino]methyl}carbonylamino)acetyl]-2-({N-methyl[(S)-1-[(S)-2-{[2-((trimethylsilyl)ethyl](oxycarbonylamino)}propanamido]ethyl]carbonylamino}methyl)piperazine-1-carboxylate

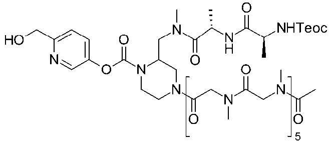

Intermediate **B39** was prepared following the synthetic method for intermediate B37, using intermediate **B1-1** instead of intermediate **B35-2** in **Step D.** MS-ESI [M+H]⁺: 1319.1.

### Preparation of Intermediate B40: 4-(hydroxymethyl)phenyl 4-[2-(N-methyl{[N-methyl({N-methyl[(N-methyl{[N-methyl({N-methyl[(N-methyl{[N-methyl({N-methyl[(N-methylacetamido)methyl]carbonylamino}methyl)carbonylamino]methyl}carbonylamino)methyl]ca rbonylamino}methyl)carbonylamino]methyl}carbonylaminocarbonylamino}methyl)carbon ylamino]methyl}carbonylamino)acetyl]-2-({N-methyl[(S)-1-[(S)-2-{[2-(trimethylsilyl)ethyl](oxycarbonylamino)}propanamido]ethyl]carbonylamino}methyl)piperazine-1-carboxylate

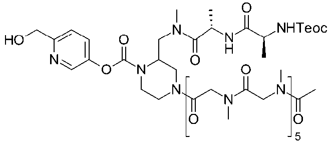

Intermediate **B40** was prepared following the synthetic method for intermediate **B39,** using intermediate **B17-1** (4-hydroxybenzaldehyde) instead of intermediate 2-formyl-5-hydroxypyridine in **Step I.** MS-ESI [M+H]⁺: 1318.1.

### Preparation of Intermediate B41: 4-(chloromethyl)phenyl 4-[2-(N-methyl{[N-methyl({N-methyl[(N-methyl{[N-methyl({N-methyl[(N-methyl{[N-methyl({N-methyl[(N-methylacetamido)methyl]carbonylamino}methyl)carbonylamino]methyl}carbonylamino)methyl]ca rbonylamino}methyl)carbonylamino]methyl}carbonylamino)methyl]carbonylamino}methyl)carbon ylamino]methyl}carbonylamino)acetyl]-2-({N-methyl[(S)-1-[(S)-2-{[2-(trimethylsilyl)ethyl](oxycarbonylamino)}propanamido]ethyl]carbonylamino}methyl)piperazine-1-carboxylate

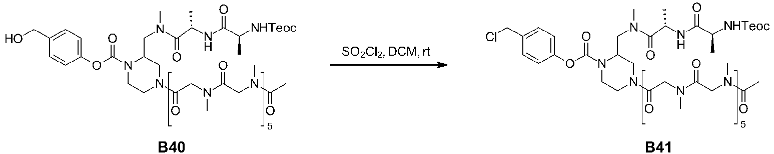

At room temperature, **B40** (500 mg, 0.38 mmol) was dissolved in dichloromethane (5 mL), and a solution of thionyl chloride in dichloromethane (4 mL, 1M, 4.00 mmol) was added. The mixture was stirred at room temperature for 1 hour. After the reaction was complete, the reaction solution was concentrated. The crude product obtained after concentration was purified by reverse phase C18 column (ACN/H₂O (0.1% HCO₂H) = 0-45%) to give **B41** (400 mg, 79% yield) as a white solid. MS-ESI [M+H]⁺: 1336.0.

### Preparation of Intermediate B42: 6-(hydroxymethyl)pyridin-3-yl-3-({N-methyl[(S)-1-[(S)-2-{[2-(trimethylsilyl)ethyl](oxycarbonylamino)}propanamido]ethyl]carbonylamino}methyl)morpholine-4-carboxylate

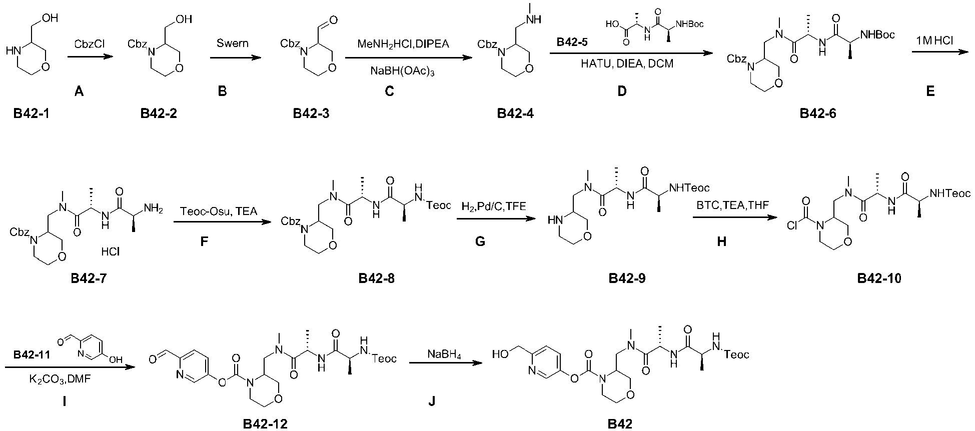

**Step A:** Under ice-water bath, sodium bicarbonate (28.7 g, 341.67 mmol) and benzyl chloroformate (14.6 g, 85.59 mmol) were added to a solution of **B42-1** (10.0 g, 85.4 mmol) in tetrahydrofuran/water (v/v = 1/1, 200 mL), stirred at room temperature overnight. After the reaction was complete, the aqueous phase was extracted with ethyl acetate (3 × 100 mL). The organic phases were combined, dried over anhydrous sodium sulfate, filtered, concentrated, and the crude product obtained after concentration was purified by FCC (SiO₂, EA/PE = 0-75%) to give **B42-2** (21.0 g, 98% yield) as a colorless oil. ¹H NMR (400 MHz, DMSO-*d*₆) δ 7.43-7.26 (m, 5H), 5.14-5.05 (m, 2H), 4.90 (t, *J* = 5.6 Hz, 1H), 3.90 (d, *J =* 11.6 Hz, 1H), 3.85-3.80 (m, 1H), 3.77 (d, *J =* 9.8 Hz, 1H), 3.70-3.60 (m, 2H), 3.47-3.29 (m, 3H), 3.07 (t, *J =* 13.0 Hz, 1H). MS-ESI [M+H]⁺: 252.1.

**Step B:** At -78 °C under nitrogen, dimethyl sulfoxide (467 mg, 6.0 mmol) was added to a solution of oxalyl chloride (379.3 mg, 3.0 mmol) in dichloromethane (3 mL). After addition, the mixture was stirred at -78 °C for 30 minutes. Then **B42-2** (500 mg, 2.0 mmol) was added to the reaction solution. The reaction solution was stirred at -78 °C for 1 hour. Finally, *N,N-*diisopropylethylamine (1540 mg, 11.94 mmol) was added. The mixture was stirred at -78 °C for 30 minutes, then allowed to warm to room temperature and stirred overnight. After the reaction was complete, the reaction solution was extracted with dichloromethane (3 × 10 mL), and the organic phases were combined, dried over anhydrous sodium sulfate, the solvent was removed by concentration under reduced pressure, and the crude product was purified by FCC (SiO₂, EA/PE = 0-35%) to give **B42-3** (450 mg, 90% yield) as a yellow oil. ¹H NMR (400 MHz, DMSO-*d*₆) δ 9.63 (d, *J* = 3.2 Hz, 1H), 7.45-7.27 (m, 5H), 5.11 (d, *J =* 23.4 Hz, 2H), 4.61 (dd, *J =* 23.6, 4.2 Hz, 1H), 4.44 (dd, *J* = 12.4, 4.2 Hz, 1H), 3.83-3.60 (m, 3H), 3.39 (tdd, *J* = 12.2, 5.6, 3.2 Hz, 1H), 3.02 (dtd, *J =* 58.4, 12.8, 3.8 Hz, 1H). MS-ESI [M+H]⁺: 250.1.

**Step C:** At room temperature, **B42-3** (440 mg, 1.77 mmol) was dissolved in dichloromethane (4 mL). Methylamine hydrochloride (1823.0 mg, 27.01 mmol) and N,N-diisopropylethylamine (3483 mg, 27.00 mmol) were added, followed by slow addition of sodium triacetoxyborohydride (763 mg, 3.60 mmol), stirred at room temperature for 4 hours. After the reaction was complete, water (15 mL) was added, and the mixture was extracted with dichloromethane (3 × 20 mL). The organic phases were combined, dried over anhydrous sodium sulfate and filtered. The filtrate was concentrated, and the resulting crude product was purified by FCC (SiO₂, MeOH/DCM = 0-10%) to give **B42-4** (238.0 mg, 50% yield) as a yellow oil. ¹H NMR (400 MHz, DMSO-*d*₆) δ 7.44-7.25 (m, 5H), 5.15-5.04 (m, 2H), 3.95-3.80 (m, 2H), 3.76 (d, *J* = 9.6 Hz, 1H), 3.66 (dd, *J* = 13.6, 2.8 Hz, 1H), 3.40 (dd, *J* = 11.6, 3.2 Hz, 1H), 3.33 (td, *J =* 11.8, 3.0 Hz, 1H), 3.12-2.98 (m, 1H), 2.77 (dd, *J* = 11.8, 8.4 Hz, 1H), 2.57 (dd, *J =* 11.8, 6.6 Hz, 1H), 2.24 (s, 3H). MS-ESI [M+H]⁺: 265.1.

**Step D:** Under ice-water bath, **B42-4** (2.7 g, 10.2 mmol) and **B42-5** (2.9 g, 11.2 mmol) were dissolved in N,N-dimethylformamide (30 mL), and then *N*,*N*,*N'*,*N'*-tetramethyl-*O*-(7-azabenzotriazol-1-yl)uronium hexafluorophosphate (HATU, 5.8 g, 15.3 mmol) and N,N-diisopropylethylamine (DIPEA, 3.9 g, 30.6 mmol) were added sequentially. The system was warmed to room temperature and stirred for 1 hour. After the reaction was complete, water (100 mL) was added, and the mixture was extracted with dichloromethane (3 × 50 mL). The organic phases were combined, dried over anhydrous sodium sulfate and filtered. The filtrate was concentrated, and the crude product was purified by FCC (SiO₂, MeOH/DCM = 0-5%) to give **B42-6** (2.7 g, 51% yield) as a white solid. MS-ESI [M+H]⁺: 507.3.

**Step E:** Under ice-water bath, **B42-6** (3.8 g, 7.5 mmol) was dissolved in acetonitrile (33 mL), and hydrogen chloride in 1,4-dioxane solution (11 mL, 4M) was added. The system was warmed to room temperature and stirred for 1 hour. After the reaction was complete, the reaction solution was concentrated to give **B42-7** (3.3 g, quantitative) as a yellow solid. MS-ESI [M+H]⁺: 407.2.

**Step F:** At room temperature, **B42-7** (3.3 g, 7.5 mmol) was dissolved in dichloromethane (33 mL). N-[2-(trimethylsilyl)ethoxycarbonyloxy]succinimide (2.5 g, 9.8 mmol) and triethylamine (2.3 g, 22.5 mmol) were added. The mixture was stirred at room temperature for 2 hours. After the reaction was complete, the reaction solution was concentrated. The crude product was purified by FCC (SiO₂, MeOH/DCM = 0-5%) to give **B42-8** (3.4 g, 82% yield) as a pale yellow oil. MS-ESI [M+H]⁺: 551.3.

**Step G:** At room temperature, **B42-8** (1.7 g, 3.1 mmol) was dissolved in trifluoroethanol (30 mL), and wet palladium on carbon (10% wt, 340 mg) was added. The atmosphere was replaced with hydrogen three times. The mixture was stirred at room temperature overnight. After the reaction was complete, the reaction solution was filtered through Celite. The filtrate was concentrated to give **B42-9** (1.3 g, 99% yield) as a yellow liquid. MS-ESI [M+H]⁺: 417.3.

**Step H:** Under nitrogen protection and ice-water bath, **B42-9** (1.8 g, 4.3 mmol) was dissolved in tetrahydrofuran (20 mL). Triphosgene (1.3 g, 4.3 mmol) and triethylamine (1.5 g, 15.05 mmol) were added. The system was warmed to room temperature and stirred for 2 hours. After the reaction was complete, dilute hydrochloric acid (0.1M, 100 mL) was added, and the mixture was extracted with dichloromethane (3 × 100 mL). The organic phases were combined, dried over anhydrous sodium sulfate, filtered, and concentrated to give **B42-10** (1.7 g, 81% yield) as a yellow solid. MS-ESI [M+H]⁺: 479.2.

**Step I:** At room temperature, **B42-10** (1.7 g, 3.5 mmol) was dissolved in *N,N-*dimethylformamide (15 mL). **B42-11** (862 mg, 7.01 mmol) and potassium carbonate (1.5 g, 10.85 mmol) were added. The mixture was stirred at room temperature for 4 hours. After the reaction was complete, the reaction solution was purified by C18 (ACN/H₂O = 0-80%) to give **B42-12** (1.3 g, 65% yield) as a white solid. MS-ESI [M+H]⁺: 566.3.

**Step J:** Under ice-water bath, **B42-12** (2.0 g, 3.5 mmol) was dissolved in tetrahydrofuran (20 mL) and methanol (20 mL). Sodium borohydride (134 mg, 3.5 mmol) was added. The system was warmed to room temperature and stirred for 1 hour. After the reaction was complete, the reaction solution was concentrated. The resulting crude product was purified by C18 (ACN/H₂O = 0-50%) to give **B42** (1.8 g, 90% yield) as a white solid. ¹H NMR (400 MHz, DMSO-*d*₆)) δ 8.34-8.17 (m, 1H), 8.12-7.82 (m, 1H), 7.64-7.37 (m, 2H), 7.20-7.01 (m, 1H), 5.42 (s, 1H), 4.72-4.57 (m, 1H), 4.53 (s, 2H), 4.39-4.14 (m, 1H), 4.07-3.79 (m, 5H), 3.78-3.32 (m, 5H), 3.15-2.76 (m, 4H), 1.26-0.95 (m, 6H), 0.94-0.79 (m, 2H), 0.04 - -0.06 (m, 9H). MS-ESI [M+H]⁺: 568.1.

### 6.3 Preparation of KRas Mutant Inhibitor-Linker Unit Conjugation Fragments

### Preparation of Intermediate C1:

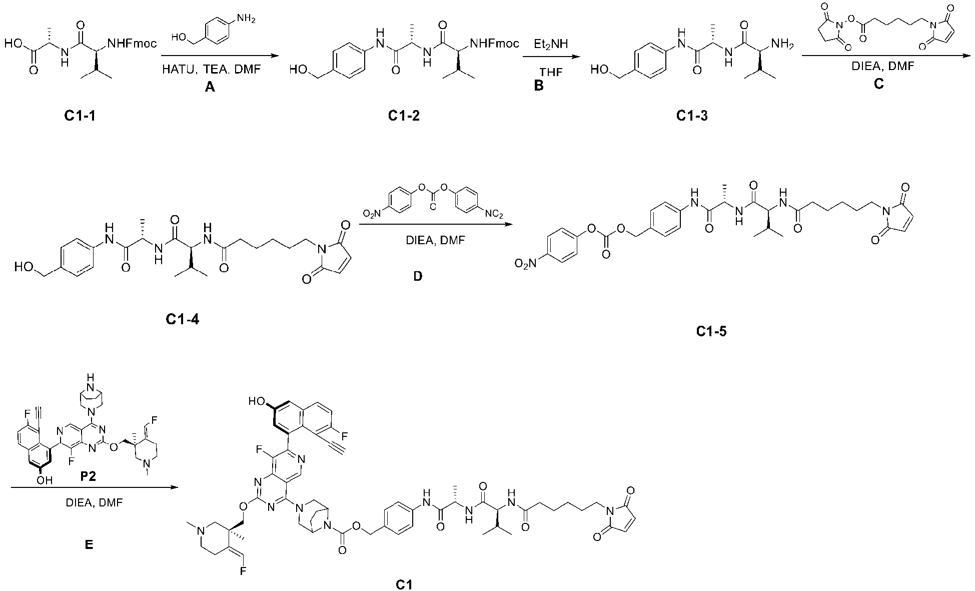

**Step A:** At 0 °C, HATU (5.6 g, 14.76 mmol) was slowly added to a solution of compound **C1-1** (5.0 g, 12.23 mmol) and p-hydroxy methyl aniline (2.3 g, 18.32 mmol) in DMF (60 mL), followed by dropwise addition of triethylamine (4.7 g, 36.69 mmol). The resulting mixture was stirred at 25 °C for 1 hour. After the reaction was complete, the reaction solution was slowly added dropwise to ice water (600 mL), filtered, and the collected solid filter cake was dispersed in methyl tert-butyl ether (200 mL), stirred at room temperature for 10 minutes, filtered, and the filter cake was collected and dried under vacuum to give **C1-2** (5.0 g, 79.6% yield) as a white solid. MS-ESI [M-H₂O]⁺: 498.2.

**Step B:** At room temperature, diethylamine (50 mL) was added to a solution of compound **C1-2** (5.0 g, 9.74 mmol) in tetrahydrofuran (50 mL). The resulting reaction solution was stirred at 25 °C for 5 hours. The solvent was removed by concentration under reduced pressure, the solid was slurried with methyl tert-butyl ether (100 mL), the mixture was filtered, and the filter cake was collected and dried under vacuum to give **C1-3** (1.7 g, 60.7% yield) as a white solid. MS-ESI [M+H]⁺: 294.2.

**Step C:** At 0 °C, *N,N-*diisopropylethylamine (1.7 g, 13.32 mmol) was slowly added dropwise to a solution of compound **C1-3** (1.3 g, 4.44 mmol) and 6-(maleimido)hexanoic acid succinimidyl ester (1.5 g, 4.88 mmol) in DMF (30 mL). The reaction solution was stirred at room temperature for 2 hours. After the reaction was complete, the reaction solution was slowly added dropwise to methyl tert-butyl ether (100 mL), stirred for 10 minutes, filtered, and the filter cake was collected and dried under vacuum to give **C1-4** (800 mg, 38.1% yield) as a white solid. MS-ESI [M-H₂O]⁺: 469.2.

**Step D:** At 0 °C, N,N-diisopropylethylamine (318 mg, 2.46 mmol) was slowly added dropwise to a solution of compound **C1-4** (400 mg, 0.82 mmol) and bis(p-nitrophenyl) carbonate (750 mg, 2.46 mmol) in DMF (10 mL). The reaction solution was stirred at room temperature for 2 hours. After the reaction was complete, the reaction solution was concentrated under reduced pressure and purified by column chromatography (MeOH/DCM = 0-5%) to give **C1-5** (500 mg, 93.4% yield) as a yellow solid. ¹H NMR (400 MHz, DMSO-*d*₆) δ10.00 (s, 1H), 8.31 (d, *J =* 9.2 Hz, 2H), 8.17 (d, *J =* 6.9 Hz, 1H), 7.81 (d, *J =* 8.6 Hz, 1H), 7.67-7.61 (m, 2H), 7.58-7.53 (m, 2H), 7.43-7.38 (m, 2H), 7.00 (s, 2H), 4.39 (t, *J =* 7.0 Hz, 1H), 4.17 (dd, *J =* 8.6, 6.8 Hz, 1H), 3.36 (t, *J =* 7.1 Hz, 2H), 2.89 (s, 1H), 2.73 (d, *J* = 0.7 Hz, 1H), 2.15 (ddq, *J =* 21.4, 14.3, 7.4 Hz, 2H), 1.95 (p, *J =* 6.9 Hz, 1H), 1.48 (t, *J =* 7.8 Hz, 4H), 1.31 (d, *J* = 7.1 Hz, 3H), 1.18 (t, *J =* 7.6 Hz, 2H), 0.87 (d, *J =* 6.7 Hz, 3H), 0.83 (d, *J =* 6.8 Hz, 3H). MS-ESI [M+H]⁺: 652.2.

**Step E:** At 0 °C, *N,N*-diisopropylethylamine (13.9 mg, 0.099 mmol) was slowly added dropwise to a solution of compound **C1-5** (20 mg, 0.033 mmol) and **Intermediate P2** (22.3 mg, 0.033 mmol) in DMF (1 mL). The reaction solution was stirred at room temperature for 2 hours. (Waters 2767 preparative HPLC, Waters XBridge C18 10 µm, 19 mm × 250 mm, preparative HPLC, mobile phase A: 0.1% TFA, B: acetonitrile, flow rate 20 mL/min, UV absorbance monitoring at dual wavelengths of 214 and 254 nm, gradient elution: 0-12 min, 30% B; 12-12.2 min, 30-95% B; 12.2-14.2 min, 95% B; 14.2-14.5 min, 95-20% B; 14.5-16 min, 30% B; The retention time of this compound: 8.7 min), to give **C1** (12.16 mg, 32.5% yield) as a white solid. ¹H NMR (400 MHz, DMSO-*d*₆) δ 9.11-9.01 (m, 1H), 7.96 (dd, J = 9.2, 5.9 Hz, 1H), 7.59 (d, J = 8.5 Hz, 2H), 7.50-7.31 (m, 4H), 7.18 (t, J = 2.4 Hz, 1H), 6.92 (s, 2H), 5.09 (s, 2H), 4.67-4.25 (m, 7H), 4.10 (d, J = 6.9 Hz, 1H), 3.86 (s, 1H), 3.79 (s, 3H), 3.46 (s, 1H), 3.34 (t, J = 7.0 Hz, 2H), 2.83 (d, J = 32.9 Hz, 6H), 2.22-2.04 (m, 2H), 1.99-1.66 (m, 6H), 1.54-1.05 (m, 13H), 0.82 (dd, J = 14.2, 6.8 Hz, 6H). MS-ESI [M+H]⁺: 1127.5.

Intermediates **C2-C17** were prepared following the synthetic route for linker **C1,** using the intermediates in the table below.

| **No.** | **Structural formula/payload/peptide linkage** | **¹H NMR / MS-ESI [M+H]⁺** |
|---|---|---|
| **C2** | 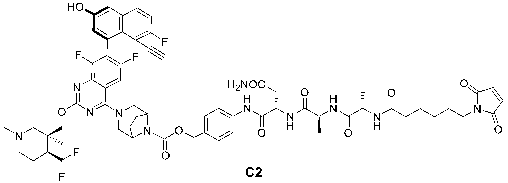 | ¹H NMR (400 MHz, DMSO-*d*₆+D₂O) δ 8.00 (dd, *J* = 9.2, 5.9 Hz, 1H), 7.65 (d, 3H), 7.54-7.44 (m, 2H), 7.39 (d, *J* = 8.2 Hz, 2H), 7.19 (d, *J* = 2.6 Hz, 1H), 6.89 (d, *J* = 2.8 Hz, 2H), 6.39 (t, *J* = 54.8 Hz, 1H), 5.13 (s, 2H), 4.70-4.51 (m, 2H), 4.50-4.28 (m, 5H), 4.26-4.10 (m, 2H), 3.85 (d, *J =* 3.4 Hz, 1H), 3.59 (dd, *J =* 32.7, 12.3 Hz, 4H), 3.37 (t, *J =* 7.0 Hz, 2H), 3.06-2.89 (m, 2H), 2.81 (s, 3H), 2.70-2.63 (m, 2H), 2.36-2.20 (m, 1H), 2.12 (t, *J =* 7.4 Hz, 2H), 2.03-1.82 (m, 4H), 1.77 (d, *J =* 8.0 Hz, 2H), 1.54-1.39 (m, 4H), 1.31-1.13 (m, 11H). |
| | **Payload: P1** | |
| | **Peptide Linkage: AAN**-PABC | |
| | | **MS-ESI [M+H]⁺:** 1250.4 |
| **C3** | 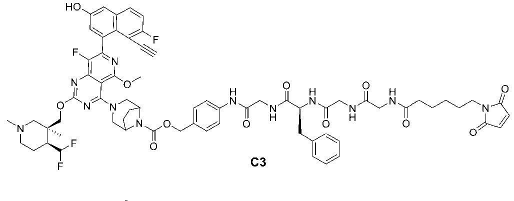 | ¹H NMR (400 MHz, DMSO-*d*₆+D₂O) δ 7.98 (dd, *J* = 9.2, 6.1 Hz, 1H), 7.62 (d, *J* = 8.1 Hz, 2H), 7.48 (t, *J =* 9.0 Hz, 1H), 7.44-7.36 (m, 3H), 7.32-7.14 (m, 6H), 6.90 (d, *J* = 1.7 Hz, 2H), 6.40 (t, *J* = 54.7 Hz, 1H), 5.12 (s, 2H), 4.62-4.42 (m, 2H), 4.42-4.30 (m, 3H), 3.88-3.73 (m, 5H), 3.71-3.57 (m, 5H), 3.57-3.45 (m, 2H), 3.42 (d, *J* = 12.5 Hz, 1H), 3.35 (t, *J* = 7.0 Hz, 2H), 3.22-3.06 (m, 1H), 3.05-2.75 (m, 7H), 2.37-2.18 (m, 2H), 2.13 (t, *J =* 7.6 Hz, 2H), 2.03-1.81 (m, 4H), 1.80-1.55 (m, 2H), 1.47 (tt, *J* = 14.5, 7.1 Hz, 5H), 1.29-1.07 (m, 5H). |
| | **Payload: P3** | |
| | **Peptide Linkage: GGFG**-PABC | |
| | | **MS-ESI [M+H]⁺:** 1325.4 |
| **C4** | 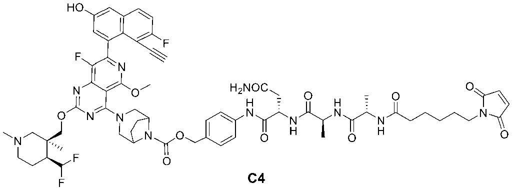 | ¹H NMR (400 MHz, DMSO-*d*₆+D₂O) δ 7.98 (dd, *J* = 9.2, 5.8 Hz, 1H), 7.66 (d, *J =* 8.3 Hz, 2H), 7.48 (t, *J* = 9.0 Hz, 1H), 7.44-7.36 (m, 3H), 7.25 (t, *J =* 3.3 Hz, 1H), 6.90 (s, 2H), 6.40 (t, *J =* 54.7 Hz, 1H), 5.12 (s, 2H), 4.60 (t, *J* = 6.5 Hz, 1H), 4.49 (dd, *J =* 16.9, 11.6 Hz, 1H), 4.45-4.26 (m, 4H), 4.17 (dq, *J* = 23.9, 7.0 Hz, 3H), 3.91 (s, 3H), 3.84 (s, 1H), 3.67-3.39 (m, 4H), 3.36 (t, *J =* 7.0 Hz, 2H), 3.09-2.88 (m, 2H), 2.81 (s, 3H), 2.63 (d, *J =* 6.9 Hz, 2H), 2.34-2.18 (m, 1H), 2.11 (t, *J =* 7.4 Hz, 2H), 2.03-1.92 (m, 2H), 1.91-1.77 (m, 2H), 1.66 (d, *J* = 60.7 Hz, 2H), 1.55-1.40 (m, 4H), 1.32-1.07 (m, 11H). |
| | **Payload: P3** | |
| | **Peptide Linkage: AAN**-PABC | |
| | | **MS-ESI [M+H]⁺:** 1263.4 |
| **C5** | 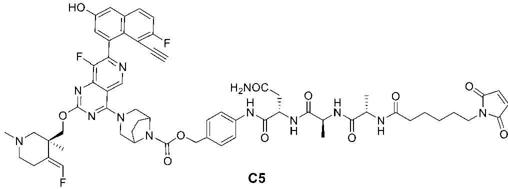 | ¹H NMR (400 MHz, DMSO-*d*₆+D₂O) δ 9.07 (d, 1H), 7.98 (dd, *J* = 9.2, 5.9 Hz, 1H), 7.73-7.60 (m, 2H), 7.48 (t, *J* = 9.0 Hz, 1H), 7.43 (d, *J* = 2.6 Hz, 1H), 7.39 (d, *J* = 8.5 Hz, 2H), 7.20 (t, *J* = 2.3 Hz, 1H), 7.04-6.94 (m, 1H), 6.92 (s, 2H), 5.12 (s, 2H), 4.68-4.53 (m, 3H), 4.50-4.40 (m, 3H), 4.39-4.26 (m, 1H), 4.18 (dq, *J* = 24.0, 7.1 Hz, 2H), 3.88-3.82 (m, 2H), 3.82-3.63 (m, 3H), 3.63-3.40 (m, 2H), 3.36 (t, *J =* 7.0 Hz, 2H), 2.99-2.76 (m, 6H), 2.65-2.60 (m, 2H), 2.11 (t, *J =* 7.4 Hz, 2H), 1.99-1.78 (m, 2H), 1.73 (d, *J =* 8.9 Hz, 2H), 1.47 (h, *J* = 7.6 Hz, 4H), 1.34-1.10 (m, 10H). |
| | **Payload: P2** | |
| | **Peptide Linkage: AAN**-PABC | |
| | | **MS-ESI [M+H]⁺:** 1213.3 |
| **C6** | 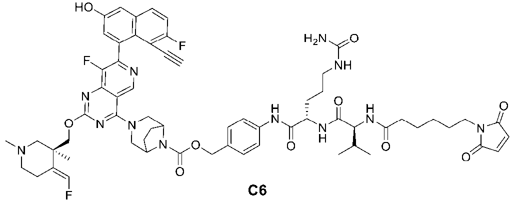 | ¹H NMR (400 MHz, DMSO-*d*₆+D₂O) δ 9.08 (s, 1H), 7.99 (dd, J = 9.3, 5.9 Hz, 1H), 7.68 (d, *J =* 8.1 Hz, 1H), 7.61 (d, *J=* 8.1 Hz, 1H), 7.48 (t, *J* = 9.0 Hz, 1H), 7.43 (d, *J =* 2.5 Hz, 1H), 7.38 (d, *J* = 8.1 Hz, 2H), 7.21 (s, 1H), 7.01-6.80 (d, *J =* 84.0 Hz 1H), 6.92 (d, *J* = 8.0 Hz, 2H), 5.12 (s, 2H), 4.70-4.21 (m, 8H), 4.17-3.96 (m, 2H), 3.81-3.62 (m, 4H), 3.63-3.42 (m, 3H), 3.42-3.26 (m, 3H), 3.14-2.73 (m, 9H), 2.45-2.28 (m, 1H), 2.29-2.07 (m, 2H), 2.02-1.83 (m, 3H), 1.81-1.66 (m, 3H), 1.67-1.58 (m, 1H), 1.55-1.33 (m, 7H), 1.29-1.08 (m, 5H), 0.98-0.74 (m, 6H). |
| | **Payload: P2** | |
| | **Peptide Linkage: VCit**-PABC | |
| | | **MS-ESI [M+H]⁺:** 1213.5 |
| **C7** | 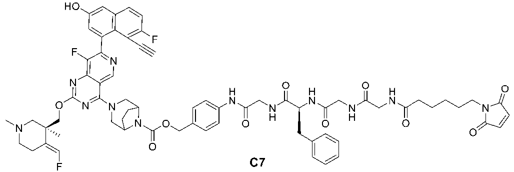 | ¹H NMR (DMSO-*d*₆+D₂O) δ 9.08 (s, 1H), 7.99 (dd, *J* = 9.3, 5.9 Hz, 1H), 7.62 (d, *J =* 8.6 Hz, 2H), 7.48 (t, *J* = 9.0 Hz, 1H), 7.44-7.35 (m, 3H), 7.32-7.23 (m, 4H), 7.23-7.18 (m, 2H), 7.01-6.80 (d, *J =* 84.0 Hz lH), 6.92 (s, 2H), 5.12 (s, 2H), 4.72-4.54 (m, 2H), 4.51-4.39 (m, 4H), 4.33 (d, *J* = 10.5 Hz, 1H), 3.98-3.81 (m, 4H), 3.77-3.57 (m, 5H), 3.54-3.43 (m, 2H), 3.36 (t, *J* = 7.0 Hz, 2H), 3.20-3.03 (m, 2H), 3.01-2.75 (m, 7H), 2.14 (dt, *J =* 14.9, 7.8 Hz, 2H), 1.94-1.68 (m, 4H), 1.47 (m, 4H), 1.37-1.03 (m, 5H). |
| | **Payload: P2** | |
| | **Peptide Linkage: GGFG**-PABC | |
| | | **MS-ESI [M+H]⁺:** 1275.5 |
| **C8** | 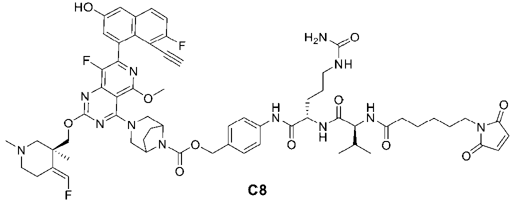 | ¹H NMR (400 MHz, DMSO-*d*₆+D₂O) δ 7.98 (dd, *J* = 9.3, 6.0 Hz, 1H), 7.69 (d, *J* = 8.3 Hz, 1H), 7.61 (d, *J* = 8.5 Hz, 1H), 7.48 (t, *J* = 9.0 Hz, 1H), 7.41 (d, *J = 2.6* Hz, 1H), 7.36 (dd, *J =* 8.7, 2.5 Hz, 2H), 7.24 (t, *J =* 2.8 Hz, 1H), 7.00 (s, 0.5H), 6.93 (d, *J* = 7.9 Hz, 2H), 6.79 (s, 0.5H), 5.10 (s, 2H), 4.58 |
| | **Payload: P4** | |
| | **Peptide Linkage: VCit**-PABC | (d, *J* = 11.1 Hz, 1H), 4.41-4.26 (m, 4H), 4.12 (d, *J =* 6.9 Hz, 1H), 4.04 (d, *J* = 7.7 Hz, 1H), 3.90 (d, *J* = 2.3 Hz, 4H), 3.53-3.29 (m, 6H), 3.07-2.76 (m, 8H), 2.15 (ddp, *J* = 20.3, 13.4, 6.9, 6.3 Hz, 3H), 1.95 (dp, *J* = 14.3, 7.0 Hz, 2H), 1.85 (s, 2H), 1.78-1.55 (m, 4H), 1.55-1.30 (m, 7H), 1.19 (d, *J* = 9.7 Hz, 5H), 0.85 (ddd, *J =* 14.6, 10.4, 6.7 Hz, 6H). |
| | | **MS-ESI [M+H]⁺:** 1243.6 |
| **C9** | 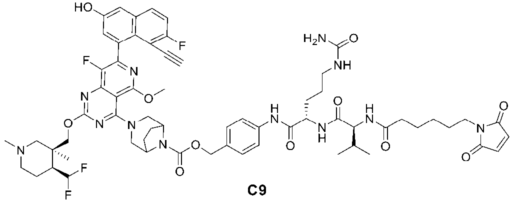 | ¹H NMR (400 MHz, DMSO-*d*₆+D₂O) δ 7.98 (dd, *J* = 9.3, 5.9 Hz, 1H), 7.68 (d, *J* = 8.4 Hz, 1H), 7.61 (d, *J* = 8.5 Hz, 1H), 7.48 (t, *J =* 9.0 Hz, 1H), 7.43 (d, *J* = 2.6 Hz, 1H), 7.38 (dd, *J =* 8.6, 3.8 Hz, 2H), 7.26 (dd, *J =* 4.5, 2.6 Hz, 1H), 6.89 (d, *J* = 8.9 Hz, 2H), 6.46 (dd, *J* = 54.8 Hz, 1H), 5.12 (s, 2H), 4.50 (dd, *J =* 16.2, 11.6 Hz, 1H), 4.35 (d, *J* = 10.4 Hz, 4H), 3.91 (d, *J* = 1.4 Hz, 3H), 3.82 (s, 1H), 3.58 (dd, *J* = 27.7, 12.8 Hz, 3H), 3.45-3.27 (m, 3H), 3.10-2.91 (m, 4H), 2.81 (d, *J =* 1.4 Hz, 3H), 2.16 (hept, *J* = 6.9 Hz, 2H), 1.98 (p, *J =* 7.0 Hz, 2H), 1.85 (d, *J* = 14.4 Hz, 2H), 1.73 (s, 1H), 1.66-1.55 (m, 2H), 1.54-1.33 (m, 6H), 1.27-1.07 (m, 5H), 0.96-0.73 (m, 6H). |
| | **Payload: P3** | |
| | **Peptide Linkage: VCit-**PABC | |
| | | **MS-ESI [M+H]⁺:** 1263.5 |
| **C10** | 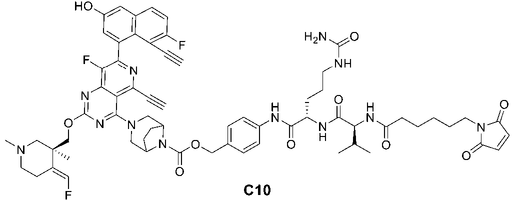 | ¹H NMR (400 MHz, DMSO-*d*₆+D₂O) δ 8.00 (dd, *J* = 9.3, 5.9 Hz, 1H), 7.68 (d, *J* = 8.3 Hz, 1H), 7.61 (d, *J* = 8.5 Hz, 1H), 7.49 (t, *J* = 9.0 Hz, 1H), 7.44 (d, *J = 2.6* Hz, 1H), 7.37 (dd, *J =* 8.8, 2.3 Hz, 2H), 7.23 (s, 1H), 7.00 (d, *J =* 6.8 Hz, 0.5H), 6.93 (d, *J* = 7.4 Hz, 2H), 6.79 (d, *J* = 6.8 Hz, 0.5H), 5.11 (s, 2H), 4.65 (s, 2H), 4.48-4.25 (m, 5H), 4.12 (d, *J =* 6.9 Hz, 1H), 3.35 (dt, *J* = 16.7, 7.0 Hz, 2H), 3.06-2.74 (m, 8H), 2.23-2.09 (m, 2H), 2.02-1.89 (m, 2H), 1.85-1.68 (m, 3H), 1.61 (d, *J =* 8.8 Hz, 1H), 1.54-1.31 (m, 7H), 1.19 (q, *J* = 10.3, 7.8 Hz, 4H), 0.85 (ddd, *J* = 13.6, 10.6, 6.7 Hz, 6H). **MS-ESI [M+H]⁺:** 1237.5 |
| | | |
| | **Payload: P5** | |
| | **Peptide Linkage: VCit**-PABC | |
| **C11** | 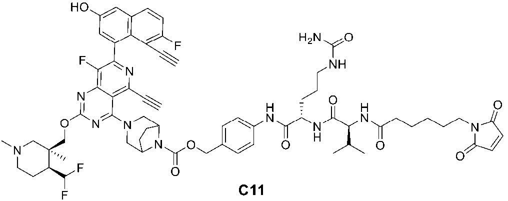 | ¹H NMR (400 MHz, DMSO-*d*₆+D₂O) δ 8.00 (dd, *J* = 9.2, 5.9 Hz, 1H), 7.67 (d, *J* = 8.0 Hz, 1H), 7.60 (d, *J* = 8.1 Hz, 1H), 7.50 (t, *J =* 9.0 Hz, 1H), 7.44 (d, *J* = 2.6 Hz, 1H), 7.41-7.33 (m, 2H), 7.25 (d, *J =* 3.3 Hz, 1H), 6.90 (d, *J* = 8.3 Hz, 2H), 6.40 (t, *J* = 54.8 Hz, 1H), 5.12 (s, 2H), 4.62 (s, 1H), 4.54-4.24 (m, 5H), 4.10 (d, *J =* 6.9 Hz, 1H), 3.57 (dd, *J =* 26.6, 12.5 Hz, 3H), 3.34 (dt, *J* = 20.3, 6.9 Hz, 3H), 3.19 (s, 1H), 3.08-2.89 (m, 4H), 2.81 (s, 3H), 2.33-2.07 (m, 4H), 1.95 (d, *J =* 8.6 Hz, 3H), 1.82 (s, 2H), 1.75-1.56 (m, 3H), 1.44 (dt, *J* = 21.2, 7.3 Hz, 7H), 1.20 (d, *J* = 20.5 Hz, 6H), 0.86 (ddd, *J* = 13.6, 10.8, 6.7 Hz, 6H). |
| | **Payload: P7** | |
| | **Peptide Linkage: VCit**-PABC | |
| | | **MS-ESI [M+H]⁺:** 1257.5 |
| **C12** | 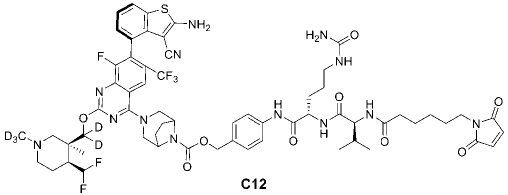 | ¹H NMR (400 MHz, DMSO-*d*₆+D₂O) δ 8.13 (s, 1H), 7.80 (dd, *J* = 7.8, 1.2 Hz, 1H), 7.69 (d, *J =* 8.4 Hz, 1H), 7.61 (d, *J* = 8.3 Hz, 1H), 7.38 (dd, *J* = 8.8, 2.5 Hz, 2H), 7.26 (t, *J* = 7.7 Hz, 1H), 7.20 (d, *J* = 7.5 Hz, 1H), 6.92 (d, *J* = 7.9 Hz, 2H), 6.42 (dd, *J* = 56.0 Hz, 1H), 5.12 (s, 2H), 4.53-4.27 (m, 5H), 4.13 (d, *J* = 6.9 Hz, 1H), 3.63 (d, *J* = 13.0 Hz, 1H), 3.53 (d, *J* = 12.0 Hz, 1H), 3.35 (dt, *J* = 17.6, 7.1 Hz, 2H), 3.01 (s, 1H), 3.00 (dt, *J =* 20.3, 7.0 Hz, 2H), 2.92 (d, *J* = 13.3 Hz, 1H), 2.30-2.09 (m, 2H), 1.96 (tt, *J* = 14.0, 6.7 Hz, 1H), 1.95 (s, 2H), 1.87 (s, 2H), 1.65 (d, *J* = 9.3 Hz, 2H), 1.48 (d, *J =* 7.6 Hz, 2H), 1.48-1.33 (m, 1H), 1.23 (s, 3H), 1.19 (d, *J* = 7.4 Hz, 1H), 1.16 (s, 2H), 0.86 (ddd, *J* = 12.3, 10.5, 6.7 Hz, 6H). **MS-ESI [M+H]⁺:** 1293.5 |
| | **Payload: P6** | |
| | **Peptide Linkage: VCit**-PABC | |
| **C13** | 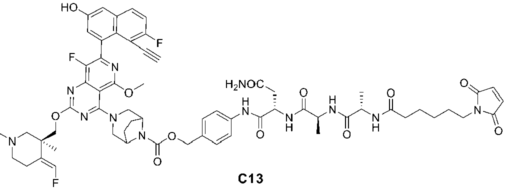 | ¹H NMR (400 MHz, DMSO-*d*₆+D₂O) δ 7.94 (dd, *J* = 9.2, 5.9 Hz, 1H), 7.69-7.58 (m, 2H), 7.45 (t, *J =* 9.0 Hz, 1H), 7.41-7.29 (m, 2H), 7.21 (t, *J =* 2.6 Hz, 1H), 6.97 (s, 1H), 6.89 (s, 2H), 6.76 (s, 1H), 5.08 (s, 2H), 4.57 (t, *J* = 6.4 Hz, 2H), 4.33 (s, 3H), 4.15 (dq, *J* = 25.3, 6.9 Hz, 4H), 3.87(s, 3H), 3.33 (t, *J* = 7.0 Hz, 3H), 2.83 (d, *J =* 35.5 Hz, 4H), 2.65-2.57 (m, 2H), 2.39-2.26 (m, 1H), 2.12-2.00 (m, 2H), 1.82 (s, 2H), 1.64 (d, *J =* 41.9 Hz, 2H), 1.44 (h, *J =* 7.6 Hz, 3H), 1.20 (q, *J* = 6.7, 6.2 Hz, 8H). |
| | **Payload: P4** | |
| | **Peptide Linkage: AAN**-PABC | |
| | | **MS-ESI [M+H]⁺:** 1243.5 |
| **C14** | 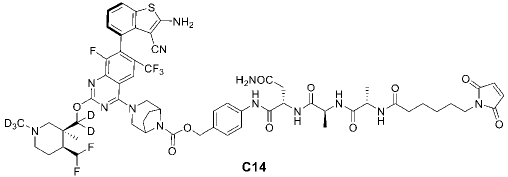 | ¹H NMR (400 MHz, DMSO-*d*₆+D₂O) δ 8.14 (s, 1H), 7.80 (d, *J* = 7.9 Hz, 1H), 7.66 (d, *J =* 8.2 Hz, 2H), 7.38 (d, *J* = 8.2 Hz, 2H), 7.29-7.13 (m, 2H), 6.93 (d, *J* = 6.1 Hz, 2H), 6.42 (t, *J =* 54.9 Hz, 1H), 5.12 (s, 2H), 4.60 (t, *J* = 6.5 Hz, 1H), 4.43 (d, *J* = 37.0 Hz, 4H), 4.18 (dt, *J* = 26.1, 7.2 Hz, 2H), 3.51 (s, 3H), 3.37 (t, *J* = 7.0 Hz, 3H), 3.07-2.83 (m, 3H), 2.61 (d, *J =* 6.7 Hz, 2H), 2.25 (s, 2H), 2.15-2.02 (m, 3H), 1.90 (d, *J =* 31.5 Hz, 4H), 1.65 (d, *J =* 8.8 Hz, 2H), 1.46 (p, *J =* 7.4 Hz, 4H), 1.23 (q, *J =* 12.0, 9.7 Hz, 10H). |
| | **Payload: P6** | |
| | **Peptide Linkage: AAN-**PABC | |
| | | **MS-ESI [M+H]⁺:** 1293.4 |
| **C15** | 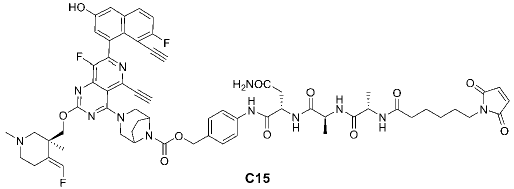 | ¹H NMR (400 MHz, DMSO-*d*₆+D₂O) δ 8.00 (dd, *J* = 9.2, 6.0 Hz, 1H), 7.65 (d, *J* = 8.2 Hz, 2H), 7.57-7.33 (m, 4H), 7.24 (s, 1H), 7.00 (s, 0.5H), 6.91 (s, 2H), 6.79 (s, 0.5H), 5.13 (s, 2H), 4.61 (d, *J =* 6.6 Hz, 3H), 4.44-4.28 (m, 4H), 4.18 (dd, *J* = 24.7, 7.2 Hz, 4H), 3.49 (s, 2H), 3.36 (t, *J* = 7.0 Hz, 2H), 2.82 (s, 6H), 2.63 (d, *J* = 6.8 Hz, 2H), 2.17-2.01 (m, 3H), 1.81 (s, 2H), 1.46 (p, *J* = 8.0, 7.5 Hz, 4H), 1.23 (p, *J* = 14.8, 11.0 Hz, 10H). |
| | **Payload: P5** | |
| | **Peptide Linkage: AAN**-PABC | |
| | | **MS-ESI [M+H]⁺:** 1237.4 |
| **C16** | 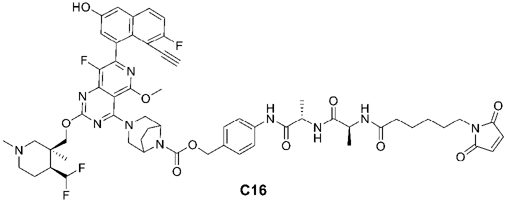 | ¹H NMR (400 MHz, DMSO-*d*₆+D₂O) δ 7.98 (dd, J = 9.2, 5.9 Hz, 1H), 7.63 (d, *J* = 8.2 Hz, 2H), 7.49 (t, *J* = 9.0 Hz, 1H), 7.45-7.35 (m, 3H), 7.26 (t, *J =* 3.3 Hz, 1H), 6.90 (s, 2H), 6.40 (dd, *J* = 54.7 Hz, 1H), 5.22-4.97 (m, 2H), 4.49 (dd, *J* = 16.8, 11.6 Hz, 1H), 4.42-4.26 (m, 4H), 4.19 (q, *J =* 7.2 Hz, 1H), 4.00-3.94 (m, 2H), 3.91 (s, 3H), 3.84 (s, 1H), 3.68-3.39 (m, 4H), 3.36 (t, *J* = 7.0 Hz, 2H), 3.07-2.89 (m, 2H), 2.81 (s, 3H), 2.36-2.18 (m, 1H), 2.13 (t, *J* = 7.3 Hz, 2H), 1.92 (d, *J* = 44.9 Hz, 4H), 1.78-1.55 (m, 2H), 1.48 (dp, *J =* 14.5, 7.3 Hz, 4H), 1.33 (d, *J* = 7.2 Hz, 3H), 1.29-1.12 (m, 8H). |
| | **Payload: P3** | |
| | **Peptide Linkage: AA-**PABC | |
| | | **MS-ESI [M+H]⁺:** 1149.4 |
| **C17** | 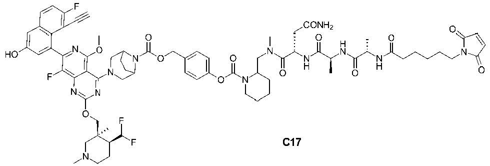 | ¹H NMR (400 MHz, CD₃CN/D₂O) δ 8.37 (s, 1H), 7.94-7.87 (m, 1H), 7.49-7.34 (m, 4H), 7.31-7.27 (m, 1H), 7.15-7.11 (m, 2H), 6.77-6.74 (m, 2H), 6.19 (t, *J* = 55.4 Hz, 1H), 5.24-4.94 (m, 3H), 4.84-3.76 (m, 12H), 3.69-3.36 (m, 4H), 3.32-3.08 (m, 6H), 2.91 (s, 1H), 2.65-2.09 (m, 9H), 1.92-1.43 (m, 17H), 1.33-1.15 (m, 14H). ¹⁹F NMR (376 MHz, CD₃CN/D₂O) δ -111.07 (d, *J* = 6.6 Hz, 1F), -118.16 (d, *J* = 284.9 Hz), -121.38 (d, *J* = 284.9 Hz), -151.21 (d, *J =* 29.8 Hz). **MS-ESI [M+H]⁺:** 1418.6 |
| | **Payload: P3** | |
| | **Linker: B26** | |

### Preparation of Intermediate C18:

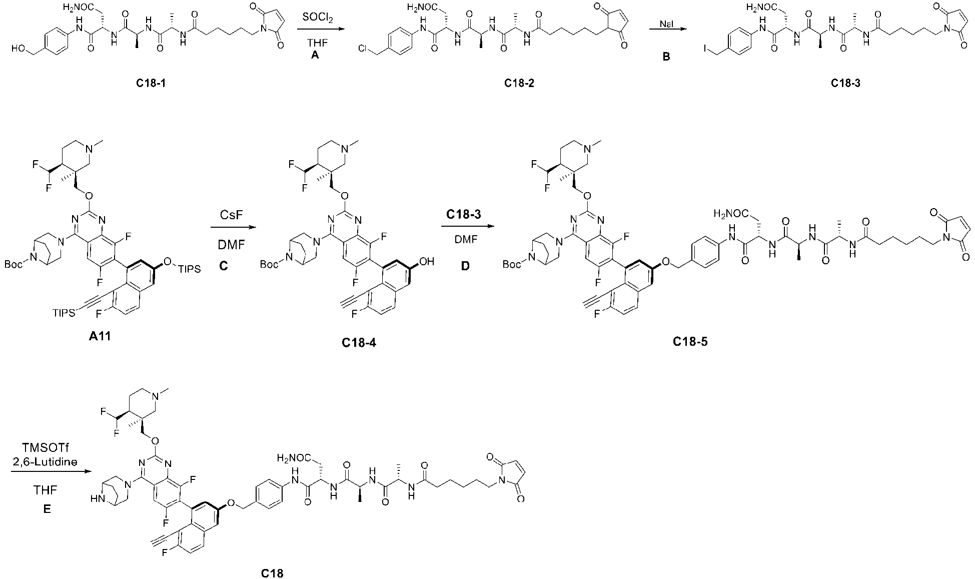

**Step** A: At 0 °C, a solution of thionyl chloride in dichloromethane (1.0M, 7 mL, 7 mmol) was slowly added dropwise to a stirred mixture of compound **C18-1** (2.0 g, 3.49 mmol) in anhydrous tetrahydrofuran (40 mL). After 5 minutes, the addition was complete, and the resulting mixture was stirred at 0 °C to room temperature for 3 hours. After the reaction was complete, the reaction solution was concentrated under reduced pressure, methyl tert-butyl ether (20 mL) was added, stirred for 10 minutes, filtered, and the filter cake was collected and dried under vacuum to give **C18-2** (2.0 g, 87.0% yield) as a yellow solid. MS-ESI [M+H]⁺: 591.3.

**Step B:** At 0 °C, sodium iodide (9.7 g, 64.4 mmol) was added in portions to a stirred mixture of compound **C18-2** (1.9 g, 3.22 mmol) in anhydrous acetone (30 mL). The resulting mixture was stirred at 0 °C to room temperature in the dark for 16 hours. After the reaction was complete, the resulting mixture was concentrated under reduced pressure, to remove the solvent, ice water (20 mL) was added, stirred at room temperature for 10 minutes, filtered, and the filter cake was collected and dried under vacuum to give **C18-3** (1.5 g). MS-ESI [M+H]⁺: 683.2.

**Step C:** A solution of **Intermediate A11** (500 mg, 0.47 mmol) in anhydrous DMF (10 mL) was cooled to 0 °C, cesium fluoride (214.2 mg, 1.41 mmol) was added, and stirred at room temperature for 2 hours. After the reaction was complete, the mixture was poured into ice water, extracted with ethyl acetate, and washed with saturated brine. The organic phase was dried over anhydrous sodium sulfate, filtered, and concentrated to give **C18-4** (300 mg, 85.1% yield) as a yellow solid. MS-ESI [M+H]⁺: 752.3.

**Step D:** At 0 °C, compound **C18-4** (226.9 mg, 0.332 mmol) was added to a solution of **A2-5** (100 mg, 0.133 mmol) and *N*,*N*-diisopropylethylamine (34.3 mg, 0.266 mmol) in DMF (5 mL). The resulting mixture was stirred at room temperature for 4 hours. After the reaction was complete, the mixture was poured into ice water, extracted with ethyl acetate, and washed with saturated brine (3 × 50 mL). The organic phase was dried over anhydrous sodium sulfate, filtered, and concentrated to give **C18-5** (158 mg, 90.5% yield) as a brown solid. MS-ESI [M+H]⁺: 1306.4.

**Step E:** Compound **C18-5** (150 mg, 0.114 mmol) was dissolved in anhydrous tetrahydrofuran (10 mL). 2,6-dimethylpyridine (12.2 mg, 0.114 mmol) and TMSOTf (118.3 mg, 0.574 mmol) were sequentially added, and the reaction solution was stirred at room temperature for 2 hours. After the reaction was complete, the reaction solution was concentrated, and the residue was purified by reverse phase preparative HPLC to give **A2** (10 mg, 7.2% yield) as a yellow solid. ¹H NMR (400 MHz, DMSO-*d*₆) δ 10.30 (s, 1H), 9.94 (s, 1H), 9.47 (d, *J =* 10.3 Hz, 1H), 9.18 (d, *J =* 7.8 Hz, 1H), 8.17 (dd, *J =* 12.3, 7.0 Hz, 2H), 8.12-7.93 (m, 2H), 7.82 (d, *J* = 8.1 Hz, 2H), 7.63 (d, *J* = 9.9 Hz, 1H), 7.56-7.45 (m, 3H), 7.43 (d, *J =* 2.6 Hz, 1H), 7.14 (t, *J =* 52.2, 49.9 Hz, 1H), 6.99 (s, 2H), 6.46 (t, *J =* 54.8 Hz, 1H), 4.73-4.50 (m, 4H), 4.39 (t, *J* = 11.1 Hz, 2H), 4.32-4.14 (m, 5H), 3.97 (s, 1H), 3.76 (dd, *J* = 13.7, 6.6 Hz, 2H), 3.66-3.43 (m, 6H), 2.97 (s, 3H), 2.60 (t, *J =* 6.1 Hz, 2H), 2.39-2.21 (m, 3H), 2.10 (t, *J =* 7.4 Hz, 2H), 1.94 (q, *J* = 11.2 Hz, 4H), 1.47 (h, *J* =7.4 Hz, 4H), 1.33-1.10 (m, 11H). MS-ESI [M+H]⁺: 1206.4.

### Preparation of Intermediate C19:

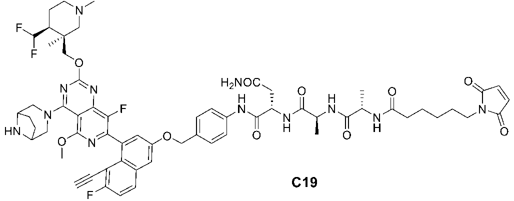

Intermediate **C19** was prepared following the synthetic route for linker **C18,** using **Intermediate A12** instead of **Intermediate A11**. ¹H NMR (400 MHz, DMSO-*d*₆) δ 10.24 (s, 1H), 9.89 (s, 1H), 9.13 (m, 2H), 8.19 (d, *J =* 6.4 Hz, 1H), 8.13 (m, 1H), 8.05 (d, *J =* 6.8 Hz, 1H), 7.98 (dd, *J* = 9.3, 6.0 Hz, 1H), 7.84-7.79 (m, 2H), 7.52 (d, *J =* 8.1 Hz, 2H), 7.48-7.44 (m, 1H), 7.40 (d, *J* = 2.6 Hz, 1H), 7.21 (dd, *J =* 8.6, 2.6 Hz, 1H), 6.99 (d, *J =* 1.7 Hz, 2H), 6.46 (m, 1H), 4.60 (m, 4H), 4.40 (t, *J =* 11.5 Hz, 1H), 4.34-4.10 (m, 5H), 3.96 (s, 1H), 3.92 (s, 3H), 3.64 (m, 3H), 3.43 (m, 2H), 2.96 (s, 3H), 2.60 (m, 2H), 2.36-2.18 (m, 2H), 2.14-2.03 (m, 3H), 2.00-1.74 (m, 5H), 1.47 (m, 5H), 1.23 (m, 13H). MS-ESI [M+H]⁺: 1219.3.

### Preparation of Intermediate C20:

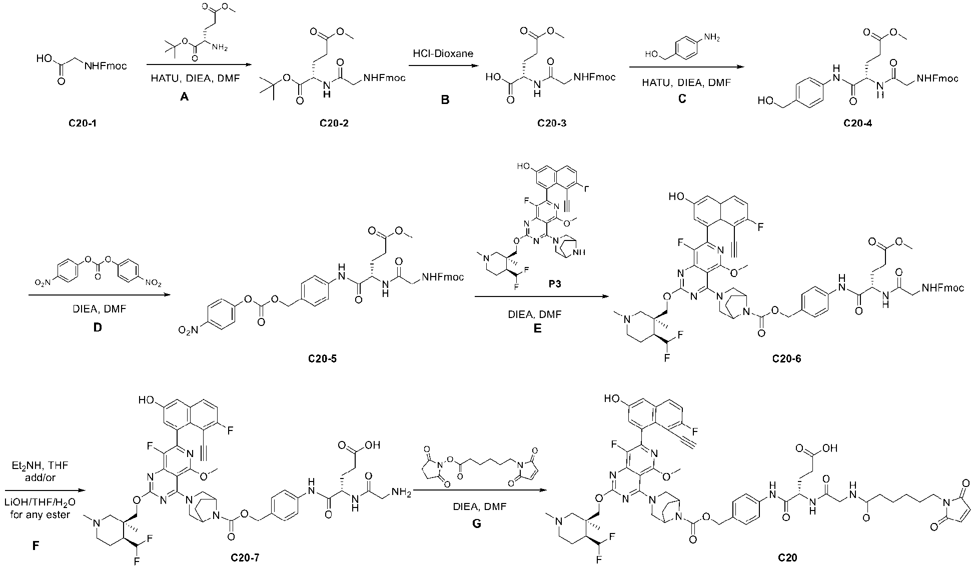

**Step A:** To a solution of compound **C20-1** (4.90 g, 16.50 mmol) in DMF (50 mL) was added compound L-glutamic acid-α-tert-butyl-γ-methyl ester (4.19 g, 16.50 mmol), followed by *N,N-*diisopropylethylamine (6.39 g, 49.50 mmol) and HATU (7.52 g, 19.80 mmol) at room temperature. The mixture was stirred at room temperature for 1 hour. After the reaction was complete, the mixture was extracted with ethyl acetate (200 mL) and water (200 mL). The organic phase was washed with water (2 × 200 mL) and saturated brine (200 mL), dried over anhydrous sodium sulfate, and concentrated to remove solvent. The crude product was purified by FCC (EA/PE = 0-50%) to give **C20-2** (7.0 g, 97.8% yield) as a yellow oil. ¹H NMR (400 MHz, Methanol-*d*₄) δ 7.79 (dt, *J =* 7.6, 0.8 Hz, 2H), 7.66 (d, *J =* 7.6 Hz, 2H), 7.38 (tt, *J* = 7.6, 0.8 Hz, 2H), 7.30 (td, *J* = 7.6, 1.2 Hz, 2H), 4.39-4.31 (m, 3H), 4.23 (t, *J =* 7.2 Hz, 1H), 3.80 (d, *J =* 1.6 Hz, 2H), 3.62 (s, 3H), 2.40 (t, *J =* 7.2 Hz, 2H), 2.19-2.07 (m, 1H), 1.97-1.85 (m, 1H), 1.45 (s, 9H). MS-ESI [M+23]⁺: 519.2.

**Step B:** To compound **C20-2** (3.0 g, 6.05 mmol) was added HCl/dioxane (6M, 20 mL) and stirred at room temperature for 4 hours. The mixture was concentrated to remove solvent to give **C20-3** (2.5 g, 94.0% yield) as a yellow oil. ¹H NMR (400 MHz, Methanol-*d*₄) δ 7.82-7.76 (m, 2H), 7.66 (d, *J* = 7.2 Hz, 2H), 7.41-7.34 (m, 2H), 7.30 (td, *J* = 7.2, 1.2 Hz, 2H), 4.53-4.43 (m, 1H), 4.40-4.30 (m, 2H), 4.23 (t, *J* = 6.8 Hz, 1H), 3.81 (s, 2H), 3.65 (s, 3H), 2.41 (t, *J* = 7.2 Hz, 2H), 2.26-2.09 (m, 1H), 1.99-1.88 (m, 1H). MS-ESI [M+H]⁺: 441.2.

**Step C:** To a solution of compound **C20-3** (440 mg, 1.00 mmol) in DMF (10 mL) was added p-hydroxy methyl aniline (123 mg, 1.00 mmol), followed by HATU (456 mg, 1.20 mmol) and *N,N-*diisopropylethylamine (387 mg, 3.00 mmol) at room temperature. The mixture was stirred at room temperature for 1 hour. After the reaction was complete, the mixture was extracted with ethyl acetate (50 mL) and water (50 mL). The organic phase was washed with water (2 × 50 mL) and saturated brine (50 mL), dried over anhydrous sodium sulfate, and concentrated to remove solvent. The crude product was purified by FCC (EA/PE = 0-50%) to give **C20-4** (350 mg, 64.2% yield) as a white solid. ¹H NMR (400 MHz, Methanol-*d*₄) δ 7.78 (d, *J =* 7.6 Hz, 2H), 7.64 (d, *J =* 7.6 Hz, 2H), 7.59-7.51 (m, 2H), 7.41-7.22 (m, 6H), 4.59-4.47 (m, 3H), 4.42-4.28 (m, 2H), 4.22 (t, *J =* 7.2 Hz, 1H), 3.82 (s, 2H), 3.61 (s, 3H), 2.45 (t, *J =* 6.8 Hz, 2H), 2.28-2.15 (m, 1H), 2.06-1.93 (m, 1H). MS-ESI [M+H]⁺: 546.2.

**Step D:** At 0 °C, bis(p-nitrophenyl) carbonate (390 mg, 1.28 mmol) was added to a solution of compound **C20-4** (350 mg, 0.64 mmol) in DMF (10 mL), followed by *N*,*N*-diisopropylethylamine (249 mg, 1.93 mmol). The mixture was stirred at room temperature for 16 hours. After the reaction was complete, the mixture was concentrated to remove solvent and purified by FCC (EA/PE = 0-60%) to give **C20-5** (230 mg, 50.4% yield) as an off-white solid. ¹H NMR (400 MHz, Chloroform-*d*) δ 8.33-8.22 (m, 2H), 7.83-7.71 (m, 2H), 7.69-7.54 (m, 4H), 7.44-7.29 (m, 9H), 5.25 (s, 2H), 4.63-4.50 (m, 1H), 4.48-4.36 (m, 2H), 4.14-4.06 (m, 1H), 3.95-3.78 (m, 2H), 3.65 (s, 3H), 2.56-2.37 (m, 2H), 2.33-2.16 (m, 1H), 2.04-1.99 (m, 1H). MS-ESI [M+H]⁺: 528.2.

**Step E:** To a solution of compound **C20-5** (38 mg, 0.05 mmol) in DMF (3 mL) was added **Intermediate P3** (30 mg, 0.05 mmol), followed by *N*,*N*-diisopropylethylamine (17 mg, 0.14 mmol). The mixture was stirred at room temperature for 16 hours. After the reaction was complete, the mixture was concentrated to remove solvent to give crude **C20-6** (55 mg, 98.5% yield), which was used directly in the next step without purification. MS-ESI [M+H]⁺: 1236.4.

**Step F:** At 0 °C, lithium hydroxide (4 mg, 0.09 mmol) was added to a solution of compound **C20-6** (55 mg, 0.04 mmol) in tetrahydrofuran-water (3 mL/1 mL), followed by *N,N-*diisopropylethylamine (17 mg, 0.14 mmol). The mixture was stirred at 0 °C for 2 hours. After the reaction was complete, the mixture was concentrated to remove solvent and purified by reverse phase column chromatography (H₂O/MeOH = 95-0%) and lyophilized to give **C20-7** (20 mg, 45.5% yield) as a yellow solid. MS-ESI [M+H]⁺: 1000.4.

**Step G:** To a solution of compound **C20-7** (20 mg, 0.02 mmol) in DMF (3 mL) was added 6-(maleimido)hexanoic acid succinimidyl ester compound (8 mg, 0.03 mmol), followed by *N,N-*diisopropylethylamine (8 mg, 0.06 mmol). The mixture was stirred at room temperature for 2 hours. After the reaction was complete, the mixture was purified by preparative HPLC and lyophilized to give **C20** (4 mg, 16.7% yield) as a yellow solid. ¹H NMR (400 MHz, Methanol-*d*₄) δ 7.89-7.80 (m, 1H), 7.71-7.58 (m, 2H), 7.43-7.35 (m, 2H), 7.35-7.27 (m, 2H), 7.26-7.19 (m, 1H), 6.74 (s, 2H), 6.26 (t, *J* = 53.6 Hz, 1H), 5.22-5.11 (m, 2H), 4.81-4.71 (m, 3H), 4.55-4.37 (m, 3H), 4.27-4.21 (m, 1H), 4.00 (d, *J =* 2.4 Hz, 3H), 3.85 (d, *J =* 3.2 Hz, 2H), 3.67-3.61 (m, 2H), 3.60-3.47 (m, 2H), 3.43 (t, *J =* 7.2 Hz, 2H), 3.36-3.31 (m, 1H), 3.25-3.22 (m, 2H), 3.13-2.98 (m, 2H), 2.91 (d, *J =* 2.8 Hz, 3H), 2.45-2.42 (m, 1H), 2.28-2.17 (m, 4H), 2.09-1.88 (m, 4H), 1.82-1.70 (m, 1H), 1.65-1.49 (m, 4H), 1.29-1.26 (m, 6H). MS-ESI [M+H]⁺: 1193.4.

Intermediates **C21-C38** were prepared following the synthetic route for linker **C20,** using the intermediates in the table below.

| **No.** | **Structural Formula/Payload/Linker** | **¹H NMR / MS-ESI [M+H]⁺** |
|---|---|---|
| **C21** | | ¹H NMR (400 MHz, DMSO-*d*₆+D₂O) δ 9.05 (s, 1H), 8.10 (dd, *J* = 8.4, 3.2 Hz, 1H), 7.98 (dd, *J =* 9.6, 6.0 Hz, 1H), 7.52-7.39 (m, 2H), 7.28-7.16 (m, 2H), 7.11-6.95 (m, 4H), 5.09 (s, 2H), 4.85-4.76 (m, 1H), 4.73-4.53 (m, 3H), 4.53-4.41 (m, 3H), 4.39-4.19 (m, 3H), 3.92-3.84 (m, 1H), 3.77-3.64 (m, 3H), 3.63-3.55 (m, 3H), 3.43-3.26 (m, 6H), 3.26-3.17 (m, 1H), 2.98-2.78 (m, 5H), 2.70-2.62 (m, 1H), 2.37-2.23 (m, 2H), 2.14-1.99 (m, 2H), 1.98-1.82 (m, 2H), 1.81-1.68 (m, 2H), 1.56-1.33 (m, 6H), 1.28-1.12 (m, 9H). |
| | 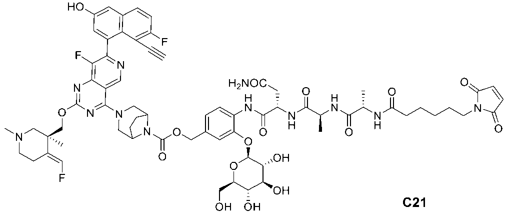 | |
| | **Payload: P2** | |
| | **Linker: B2** | |
| | | **MS-ESI [M+H]⁺:** 1391.5 |
| **C22** | | ¹H NMR (400 MHz, DMSO-*d*₆+D₂O) δ 9.03 (s, 1H), 8.00 (d, *J =* 8.3 Hz, 1H), 7.94 (dd, *J* = 9.2, 5.9 Hz, 1H), 7.44 (t, *J* = 9.0 Hz, 1H), 7.39 (d, *J* = 2.6 Hz, 1H), 7.24-7.15 (m, 2H), 7.05 (d, *J* = 8.5 Hz, 1H), 6.96 (m, 0.5H), 6.85 (s, 2H), 6.75 (m, 0.5H), 5.07 (s, 2H), 4.76 (d, *J = 7.7* Hz, 1H), 4.69-4.33 (m, 7H), 4.31-4.19 (m, 1H), 4.15 (d, *J =* 7.2 Hz, 1H), 4.01 (m, 8H), 3.80-3.66 (m, 3H), 3.58 (m, 2H), 3.47 (m, 1H), 3.33 (t, *J* = 7.0 Hz, 2H), 2.81 (m, 5H), 2.71-2.60 (m, 1H), 2.56 (d, *J =* 6.6 Hz, 1H), 2.33 (m, 1H), 2.06 (t, *J =* 7.5 Hz, 2H), 1.78 (m, 4H), 1.43 (m, 4H), 1.26-1.11 (m, 11H). |
| | 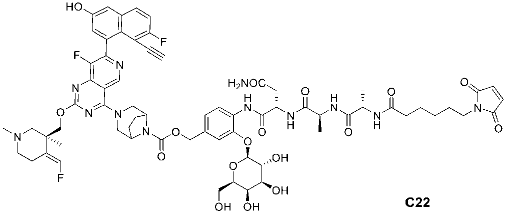 | |
| | **Payload: P2** | |
| | **Linker: B3** | |
| | | **MS-ESI [M+H]⁺:** 1391.5 |
| **C23** | 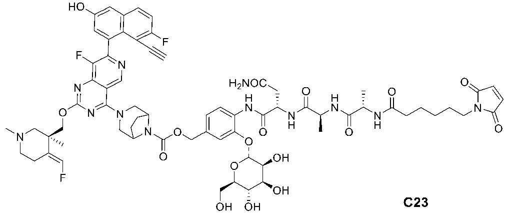 | ¹H NMR (400 MHz, DMSO-*d*₆+D₂O) δ 9.08 (s, 1H), 7.98 (dt, *J* = 9.4, 4.8 Hz, 2H), 7.48 (t, *J* = 9.0 Hz, 1H), 7.41 (d, *J* = 12.3 Hz, 2H), 7.20 (t, *J* = 2.3 Hz, 1H), 7.05 (d, *J* = 8.4 Hz, 1H), 7.01 (s, 0.5H), 6.93 (d, *J* = 4.2 Hz, 2H), 6.80 (s, 0.5H), 5.10 (s, 2H), 4.74 (t, *J* = 6.4 |
| | **Payload: P2** | Hz, 1H), 4.62 (d, *J* = 13.6 Hz, 2H), 4.44 (s, 3H), 4.23 (dt, *J =* 13.7, 6.9 Hz, 1H), 4.02 (d, *J* = 15.5 Hz, 2H), 3.57-3.43 (m, 4H), 3.42-3.29 (m, 3H), 2.87 (d, *J* = 46.0 Hz, 6H), 2.72 (d, *J* = 23.6 Hz, 2H), 2.09 (q, *J =* 7.9 Hz, 2H), 1.90 (s, 2H), 1.73 (d, *J* = 8.0 Hz, 2H), 1.47 (d, *J* = 7.9 Hz, 4H), 1.30-1.08 (m, 9H). |
| | **Linker: B4** | |
| | | **MS-ESI [M+H]⁺:** 1391.5 |
| **C24** | | ¹H NMR (400 MHz, DMSO-*d*₆+D₂O) δ 9.09 (s, 1H), 8.08 (dq, *J* = 7.4, 4.0 Hz, 1H), 7.98 (dd, *J =* 9.2, 5.9 Hz, 1H), 7.48 (t, *J* = 9.0 Hz, 1H), 7.42 (d, *J* = 2.6 Hz, 1H), 7.28-7.15 (m, 2H), 7.16-7.06 (m, 1H), 6.90 (d, *J* = 88.1 Hz, 1H), 6.93 (d, *J* = 3.6 Hz, 2H), 5.11 (s, 2H), 5.05-4.96 (m, 1H), 4.91 (d, *J* = 7.4 Hz, 1H), 4.75-4.56 (m, 3H), 4.52-4.38 (m, 3H), 4.33-4.18 (m, 3H), 4.02-3.90 (m, 3H), 3.89-3.74 (m, 4H), 3.57-3.27 (m, 6H), 3.00-2.77 (m, 6H), 2.73-2.63 (m, 1H), 2.62-2.55 (m, 1H), 2.15-2.03 (m, 2H), 1.90 (d, *J =* 9.2 Hz, 2H), 1.74 (d, *J =* 7.7 Hz, 2H), 1.52-1.41 (m, 4H), 1.31-1.11 (m, 10H). |
| | 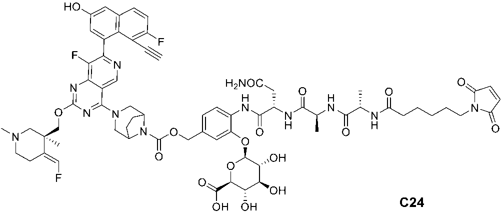 | |
| | **Payload: P2** | |
| | **Linker: B5** | |
| | | **MS-ESI [M+H]⁺:** 1405.3 |
| **C25** | 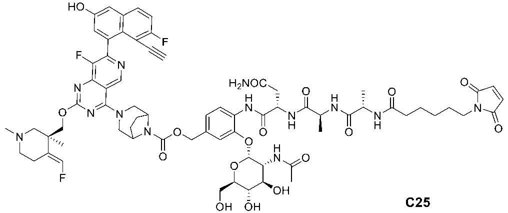 | ¹H NMR (400 MHz, DMSO-*d*₆+D₂O) δ 9.08 (s, 1H), 8.18 (d, *J =* 8.3 Hz, 1H), 7.99 (t, *J =* 7.7 Hz, 1H), 7.57-7.39 (m, 2H), 7.19 (d, *J =* 19.4 Hz, 2H), 7.07 (d, *J* = 8.5 Hz, 1H), 7.01-6.80 (d, *J =* 84.0 1H), 6.91 (d, *J* = 4.9 Hz, 2H), 5.10 (s, 2H), 5.03-4.95 (m, 1H), 4.84-4.74 (m, 1H), 4.70-4.54 (m, 2H), 4.51-4.39 (m, 3H), 4.39-4.33 (m, 1H), 4.27-4.17 (m, 1H), 3.87-3.66 (m, 5H), 3.62-3.43 (m, 5H), 3.37 (t, *J =* 7.3 Hz, 2H), 3.33-3.22 (m, 2H), 3.04-2.75 (m, 7H), 2.68-2.60 (m, 2H), 2.18-2.03 (m, 3H), 1.93 (d, *J* = 17.0 Hz, 4H), 1.81-1.66 (m, 2H), 1.59-1.41 (m, 4H), 1.35-1.14 (m, 11H). **MS-ESI [M+H]⁺:** 1432.5 |
| | **Payload: P2** | |
| | **Linker: B6** | |
| **C26** | 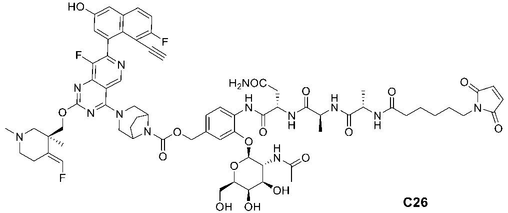 | **MS-ESI [M+H]⁺:** 1432.5 |
| | **Payload: P2** | |
| | **Linker: B7** | |
| **C27** | 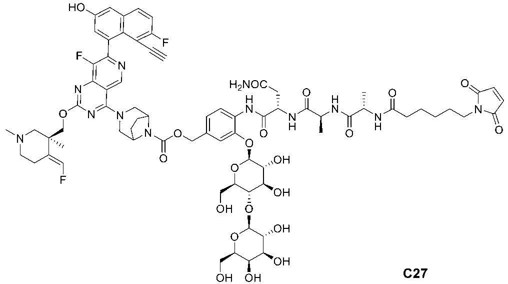 | ¹H NMR (400 MHz, DMSO-*d*₆+D₂O) δ 9.08 (s, 1H), 8.08 (dd, *J* = 8.2, 3.5 Hz, 1H), 7.98 (dd, *J =* 9.2, 5.9 Hz, 1H), 7.48 (t, *J* = 9.0 Hz, 1H), 7.43 (d, *J* = 2.5 Hz, 1H), 7.28-7.23 (m, 1H), 7.21 (t, *J =* 2.5 Hz, 1H), 7.11 (d, *J* = 8.5 Hz, 1H), 7.01 (m, 0.5H), 6.92 (d, *J* = 4.1 Hz, 2H), 6.80 (m, 0.5H), 5.11 (s, 2H), 4.92 (d, *J* = 7.1 Hz, 1H), 4.66 (m, 3H), 4.45 (m, 3H), 4.38-4.14 (m, 4H), 3.69 (m, 5H), 3.53 (m, 10H), 3.42-3.30 (m, 5H), 3.00-2.77 (m, 7H), 2.74-2.58 (m, 2H), 2.45-2.25 (m, 1H), 2.10 (t, *J =* 7.4 Hz, 2H), 1.96-1.68 (m, 4H), 1.47 (m, 4H), 1.38-1.09 (m, 11H). |
| | **Payload: P2** | |
| | **Linker: B8** | |
| | | **MS-ESI [M+H]⁺:** 1553.6 |
| **C28** | 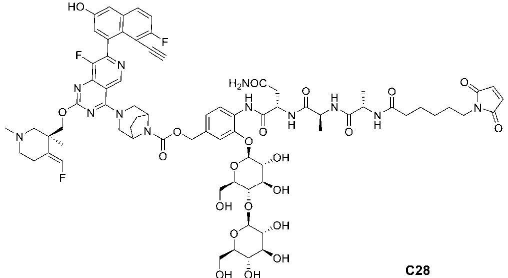 | ¹H NMR (400 MHz, DMSO-*d*₆+D₂O) δ 9.08 (s, 1H), 8.09 (dd, *J* = 8.1, 3.6 Hz, 1H), 7.98 (dd, *J* = 9.2, 5.9 Hz, 1H), 7.48 (t, *J* = 9.0 Hz, 1H), 7.42 (d, *J* = 2.6 Hz, 1H), 7.30-7.17 (m, 2H), 7.11 (d, *J* = 8.5 Hz, 1H), 7.01 (s, 0.5H), 6.93 (d, *J* = 3.7 Hz, 2H), 6.80 (s, 0.5H), 5.11 (s, 2H), 4.90 (d, *J* = 7.0 Hz, 1H), 4.73-4.54 (m, 3H), 4.44 (s, 3H), 4.39-4.15 (m, 4H), 3.63-3.30 (m, 9H), 3.21 (dd, *J* = 15.5, 6.9 Hz, 2H), 3.06 (dt, *J* = 17.1, 8.9 Hz, 2H), 2.87 (d, *J* = 40.6 Hz, 6H), 2.67 (t, *J* = 7.8 Hz, 2H), 2.14-2.03 (m, 2H), 1.90 (s, 2H), 1.74 (d, *J* = 7.8 Hz, 2H), 1.46 (q, *J* = 7.3 Hz, 4H), 1.39-1.06 (m, 10H). |
| | **Payload: P2** | **MS-ESI [M+H]⁺:** 1553.5 |
| | **Linker: B9** | |
| | | |
| **C29** | 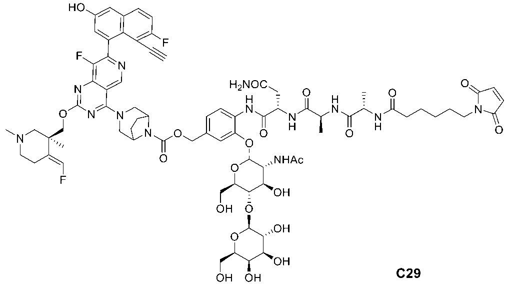 | ¹H NMR (400 MHz, DMSO-*d*₆+D₂O) δ 9.08 (s, 1H), 8.20 (d, *J =* 8.1 Hz, 1H), 7.98 (d, *J =* 9.3 Hz, 1H), 7.60-7.33 (m, 2H), 7.19 (d, *J =* 14.3 Hz, 2H), 7.08 (d, *J* = 8.2 Hz, 1H), 7.00 (s, 0.5H), 6.97 (s, 2H), 6.80 (s, 0.5H), 5.08 (d, *J* = 22.9 Hz, 2H), 4.79 (s, 1H), 4.61 (s, 1H), 4.44 (s, 2H), 4.29 (s, 2H), 3.53 (d, *J =* 12.2 Hz, 4H), 3.38 (d, *J =* 13.0 Hz, 3H), 2.85 (d, *J =* 27.6 Hz, 5H), 2.08 (d, *J* = 8.4 Hz, 2H), 1.93 (d, *J =* 19.0 Hz, 3H), 1.74 (s, 2H), 1.47 (s, 3H), 1.38-1.08 (m, 7H). |
| | **Payload: P2** | |
| | **Linker: B10** | |
| | | **MS-ESI [M+H]⁺:** 1594.6 |
| **C30** | | ¹H NMR (400 MHz, DMSO-*d*₆+D₂O) δ 7.98 (dd, *J* = 9.2, 6.0 Hz, 1H), 7.49 (t, *J =* 9.0 Hz, 1H), 7.42 (d, *J =* 2.6 Hz, 1H), 7.25 (s, 1H), 6.93 (q, *J =* 3.7, 3.1 Hz, 2H), 6.42 (dd, *J* = 54.7 Hz, 1H), 5.05-4.89 (m, 1H), 4.68-4.61 (m, 1H), 4.58-4.29 (m, 3H), 4.30-4.11 (m, 3H), 3.93 (d, *J =* 2.9 Hz, 3H), 3.91-3.83 (m, 2H), 3.58 (dd, *J =* 28.8, 12.5 Hz, 3H), 3.37 (p, *J =* 7.2 Hz, 3H), 3.10-2.89 (m, 2H), 2.82 (s, 3H), 2.76-2.60 (m, 2H), 2.41-2.18 (m, 3H), 2.09 (t, *J =* 7.3 Hz, 2H), 2.06-1.90 (m, 2H), 1.89-1.61 (m, 3H), 1.57-1.40 (m, 4H), 1.30-1.07 (m, 10H). |
| | 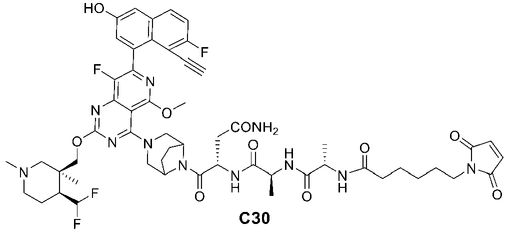 | |
| | **Payload: P3** | |
| | **Linker: AAN** | |
| | | **MS-ESI [M+H]⁺:** 1114.4 |
| **C31** | 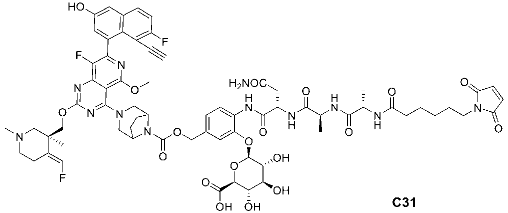 | ¹H NMR (400 MHz, DMSO-*d*₆+D₂O) δ 8.06 (d, *J* = 8.1 Hz, 1H), 7.98 (dd, *J* = 9.4, 5.9 Hz, 1H), 7.48 (t, *J* = 9.0 Hz, 1H), 7.42 (d, *J* = 2.6 Hz, 1H), 7.25 (s, 1H), 7.18 (s, 1H), 7.09 (d, *J* = 8.5 Hz, 1H), 6.99 (s, 0.5H), 6.92 (d, *J* = 4.3 Hz, 2H), 6.78 (s, 0.5H), 5.06 (d, *J =* 33.3 Hz, 4H), 4.77-4.53 (m, 3H), 4.46-4.15 (m, 8H), 3.65-3.31 (m, 9H), 2.82 (s, 6H), 2.67 (d, *J* = 6.1 Hz, 2H), 2.09 (d, *J* = 7.9 Hz, 3H), 1.85 (s, 2H), 1.67 (m, 2H), 1.47 (m, 4H), 1.22 (m, 10H). |
| | **Payload: P4** | |
| | **Linker: B5** | |
| | | **MS-ESI [M+H]⁺:** 1435.5 |
| **C32** | | ¹H NMR (400 MHz, DMSO-*d*₆+D₂O) δ 8.07 (d, *J* = 8.2 Hz, 1H), 7.98 (dd, *J* = 9.2, 5.9 Hz, 1H), 7.48 (t, *J* = 9.0 Hz, 1H), 7.42 (d, *J* = 2.6 Hz, 1H), 7.27-7.23 (m, 1H), 7.18 (s, 1H), 7.09 (d, *J =* 8.4 Hz, 1H), 6.92 (d, *J =* 4.1 Hz, 2H), 6.41 (t, *J =* 54.6 Hz, 1H), 5.10 (s, 2H), 5.02 (d, *J =* 7.4 Hz, 1H), 4.73-4.59 (m, 1H), 4.55-4.32 (m, 5H), 4.24 (dd, *J =* 25.8, 7.1 Hz, 3H), 4.01-3.88 (m, 6H), 3.66-3.30 (m, 9H), 3.08-2.88 (m, 3H), 2.81 (d, *J* = 1.8 Hz, 3H), 2.67 (d, *J =* 6.5 Hz, 1H), 2.59 (d, *J =* 6.6 Hz, 1H), 2.25 (s, 1H), 2.09 (q, *J =* 7.7 Hz, 2H), 1.96 (s, 2H), 1.86 (s, 2H), 1.67 (m, 2H), 1.53-1.40 (m, 4H), 1.31-1.13 (m, 10H). |
| | 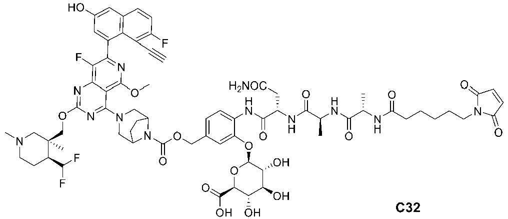 | |
| | **Payload: P3** | |
| | **Linker: B5** | |
| | | **MS-ESI [M+H]⁺:** 1455.5 |
| **C33** | 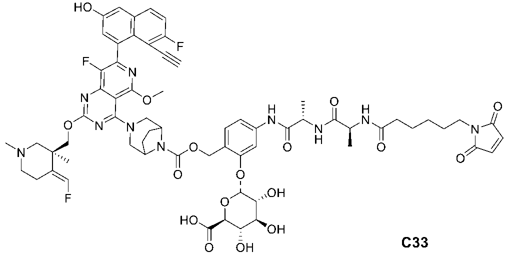 | ¹H NMR (400 MHz, Methanol-*d*₄) δ 7.84 (ddd, *J* = 9.1, 5.7, 2.1 Hz, 1H), 7.52 (d, *J =* 1.6 Hz, 1H), 7.41-7.25 (m, 4H), 7.22 (dd, *J* = 6.4, 2.6 Hz, 1H), 7.01 (s, 0.5H), 6.79 (s, 0.5H), 6.74 (d, *J* = 5.1 Hz, 2H), 4.59-4.33 (m, 7H), 4.31-4.12 (m, 3H), 4.00 (dd, *J* = 8.9, 5.2 Hz, 5H), 3.58-3.46 (m, 5H), 3.44-3.32 (m, 4H), 3.11-2.82 (m, 7H), 2.64 (dd, *J* = 29.7, 7.4 Hz, 2H), 2.31-2.13 (m, 3H), 1.95 (s, 3H), 1.79 (d, *J =* 27.4 Hz, 2H), 1.70-1.49 (m, 5H), 1.45-1.29 (m, 10H). **MS-ESI** 1321.4 |
| | **Payload: P4** | **[M+H]⁺:** |
| | **Linker: B11** | |
| **C34** | 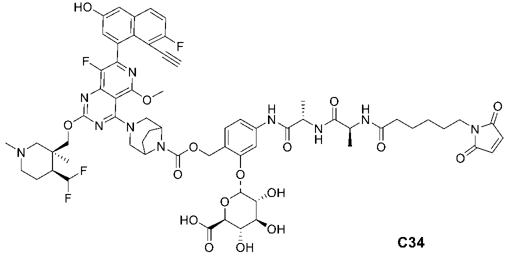 | ¹H NMR (400 MHz, Methanol-*d*₄) δ 7.88-7.81 (m, 1H), 7.37-7.28 (m, 2H), 7.27-7.18 (m, 1H), 6.93 (d, *J* = 83.5 Hz, 1H), 6.80-6.72 (m, 2H), 4.80-4.64 (m, 2H), 4.63-4.50 (m, 2H), 4.46-4.25 (m, 3H), 4.03 (d, *J* = 2.8 Hz, 3H), 3.93-3.77 (m, 1H), 3.77-3.40 (m, 4H), 3.36 (d, *J* = 5.0 Hz, 1H), 3.16-2.87 (m, 6H), 2.66-2.51 (m, 1H), 2.22 (td, *J* = 7.5, 2.9 Hz, 2H), 2.14-1.98 (m, 1H), 1.95-1.75 (m, 2H), 1.68-1.49 (m, 4H), 1.43 (d, *J* = 6.9 Hz, 1H), 1.40-1.22 (m, 10H). |
| | **Payload: P3** | |
| | **Linker: B11** | |
| | | **MS-ESI [M +** 2**H**]²⁺/2: 671.4 |
| **C35** | 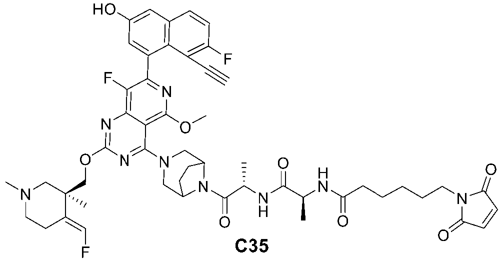 | ¹H NMR (400 MHz, Methanol-*d*₄) δ 7.92-7.78 (m, 1H), 7.38-7.27 (m, 2H), 7.26-7.22 (m, 1H), 6.82-6.71 (m, 2H), 6.27 (t, *J* = 54.8 Hz, 1H), 4.81-4.73 (m, 2H), 4.74-4.56 (m, 2H), 4.40-4.14 (m, 3H), 4.03 (s, 3H), 4.00-3.76 (m, 1H), 3.73-3.53 (m, 4H), 3.53-3.40 (m, 3H), 3.34 (d, *J* = 15.0 Hz, 1H), 3.15-2.96 (m, 2H), 2.92 (d, *J =* 3.8 Hz, 3H), 2.38-2.13 (m, 4H), 2.06 (d, *J* = 10.6 Hz, 2H), 1.96-1.73 (m, 2H), 1.76-1.49 (m, 4H), 1.44 (d, *J =* 6.6 Hz, 1H), 1.42-1.20 (m, 10H). |
| | **Payload: P4** | |
| | **Linker: AA** | |
| | | **MS-ESI [M+H]⁺:** 980.3 |
| **C36** | 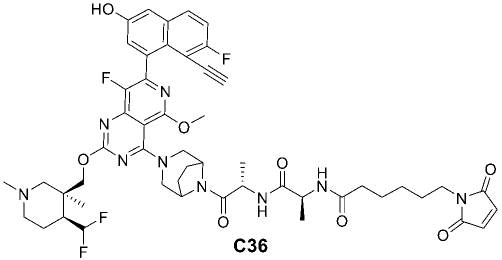 | **MS-ESI [M+H]⁺:** 1000.4 |
| | **Payload: P3** | |
| | **Linker: AA** | |
| **C37** | | ¹H NMR (400 MHz, Methanol-*d*₄) δ 8.07 (t, *J* = 7.4 Hz, 1H), 7.90-7.78 (m, 1H), 7.60 (d, *J* = 8.1 Hz, 2H), 7.46 (t, *J* = 8.7 Hz, 2H), 7.38 (d, *J* = 8.1 Hz, 2H), 6.76 (d, *J* = 1.5 Hz, 2H), 6.26 (t, *J* = 54.8 Hz, 1H), 5.17 (d, *J* = 24.6 Hz, 2H), 4.58-4.36 (m, 4H), 4.31-4.09 (m, 4H), 3.99 (s, 3H), 3.66 (m, 2H), 3.59-3.41 (m, 5H), 3.22-2.97 (m, 5H), 2.91 (s, 3H), 2.65 (t, *J* = 7.4 Hz, 2H), 2.51-2.17 (m, 6H), 2.06 (m, 3H), 1.90 (m, 3H), 1.77 (m, 4H), 1.61 (m, 4H), 1.42 (m, 2H), 1.27 (d, *J* = 5.3 Hz, 3H), 0.96 (m, 6H). |
| | 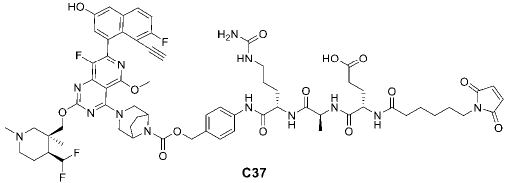 | |
| | **Payload: P3** | |
| | **Linker: EVCit-**PABC | |
| | | **MS-ESI [M+H]⁺:** 1392.5 |
| **C38** | 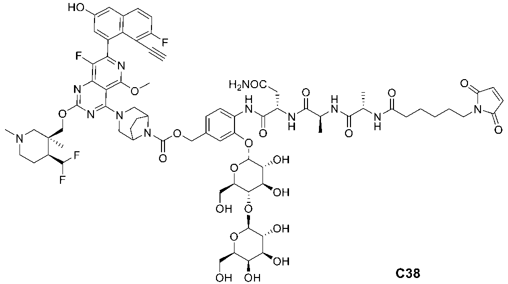 | ¹H NMR (400 MHz, Methanol-*d*₄) δ 8.16-8.07 (m, 1H), 7.88-7.81 (m, 1H), 7.37-7.26 (m, 3H), 7.26-7.20 (m, 1H), 7.15-7.07 (m, 1H), 6.81-6.70 (m, 2H), 6.27 (t, *J* = 54.8 Hz, 1H), 5.25-5.07 (m, 3H), 5.01-4.93 (m, 2H), 4.81-4.72 (m, 2H), 4.52-4.33 (m, 5H), 4.31-4.16 (m, 4H), 4.01 (d, *J* = 4.8 Hz, 2H), 3.94-3.89 (m, 1H), 3.84-3.75 (m, 2H), 3.74-3.52 (m, 10H), 3.51-3.41 (m, 3H), 3.11-2.97 (m, 3H), 2.94-2.90 (m, 2H), 2.31-2.14 (m, 4H), 2.09-2.02 (m, 1H), 1.99-1.89 (m, 2H), 1.84-1.70 (m, 2H), 1.63-1.53 (m, 3H), 1.51-1.45 (m, 3H), 1.39-1.33 (m, 6H), 1.32-1.26 (m, 6H). **MS-ESI [M + 2H**]²⁺/**2**: 802.9 |
| | **Payload: P3** | |
| | **Linker: B8** | |
| **C39** | 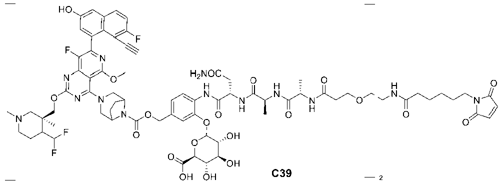 | **MS-ESI [M +2H]²⁺/2:** 1459.0 |
| | **Payload: P3** | |
| | **Linker: B5** | |

Intermediates **C40-C48** were prepared following the synthetic route for linker **C1,** using the intermediates in the table below.

| **No.** | **Structural formula/Payload/peptide linkage** | **¹H NMR / MS-ESI [M+H]⁺** |
|---|---|---|
| **C40** | | ¹H NMR (400 MHz, CD₃CN) δ 7.91 (m, 1H), 7.45 (d, *J* = 8.2 Hz, 2H), 7.42-7.40 (m, 1H), 7.37 (d, *J* = 9.0 Hz, 1H), 7.30-7.28 (m, 1H), 7.17-7.13 (m, 2H), 6.76 (s, 2H), 6.19 (t, *J =* 54.8 Hz, 1H), 5.18 (s, 2H), 5.12-5.01 (m, 1H), 4.74 (d, *J* = 11.8 Hz, 1H), 4.41 (brs, 2H), 4.29-4.14 (m, 3H), 3.97 (d, *J =* 2.8 Hz, 3H), 3.74-3.48 (m, 7H), 3.43 (t, *J =* 7.0 Hz, 3H), 3.27 (d, *J =* 5.2 Hz, 1H), 3.14-3.06 (m, 4H), 3.02-2.91 (m, 5H), 2.81 (s, 3H), 2.63-2.61 (m, 1H), 2.46-2.43 (m, 1H), 2.29-2.12 (m, 4H), 2.04-1.99 (m, 1H), 1.91-1.87 (m, 2H), 1.80-1.76 (m, 1H), 1.59-1.51 (m, 5H), 1.28-1.21 (m, 12H). |
| | 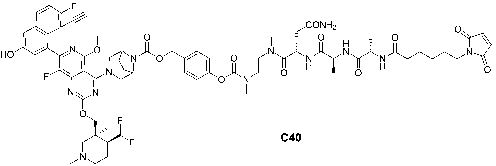 | |
| | **Payload: P3** | |
| | **Linker: B27** | |
| | | ¹⁹F NMR (376 MHz, CD₃CN) δ - 110.91 (d, *J* = 10.0 Hz, 1F), -120.02 (dd, *J* = 287.2, 25.2 Hz, 1F), -122.31 - -123.29 (m, 1F), -151.66 (d, *J =* 58.4 Hz, 1F). |
| | | **MS-ESI [M+H]⁺:** 1378.4 |
| **C41** | | ¹H NMR (400 MHz, CD₃CN) δ 8.11-8.04 (m, 1H), 7.95-7.90 (m, 1H), 7.43-7.35 (m, 2H), 7.31-7.29 (m, 1H), 7.09 (s, 1H), 7.03 (d, *J* = 8.0 Hz, 1H), 6.84 (d, *J* = 18.2 Hz, 2H), 6.34-6.005(m, 1H), 5.14 (brs, 2H), 4.88-4.81 (m, 1H), 4.74 (d, *J* = 12.0 Hz, 1H), 4.56-4.50 (m, 1H), 4.40 (brs, 2H), 4.34-4.17 (m, 8H), 4.09-3.95 (m, 5H), 3.86-3.83 (m, 1H), 3.63 (t, *J =* 11.6 Hz, 2H), 3.55-3.42 (m, 4H), 3.29-3.24 (m, 2H), 2.96-2.92 (m, 2H), 2.82-2.72 (m, 4H), 2.67-2.63 (m, 1H), 2.27-2.13 (m, 2H), 2.05-1.99 (m, 2H), 1.92-1.86 (m, 2H), 1.82-1.73 (m, 1H), 1.63-1.60 (m, 1H), 1.37-1.30 (m, 6H), 1.22 (d, *J* = 5.6 Hz, 3H). |
| | 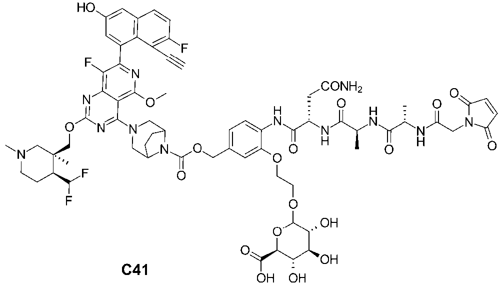 | |
| | **Payload: P3** | |
| | **Linker: B23** | ¹⁹F NMR (376 MHz, CD₃CN) δ - 110.91 (d, *J* = 12.6 Hz, 1F), -120.00 (dd, *J* = 287.4, 21.8 Hz, 1F), -122.78 (dd, *J* = 287.2, 27.2 Hz, 1F), -151.65 (d, *J* = 68.2 Hz, 1F). |
| | | **MS-ESI [M+H]⁺:** 1443.4 |
| **C42** | | ¹H NMR (400 MHz, CD₃CN) δ 7.92-7.88 (m, 1H), 7.46 (s, 1H), 7.41-7.39 (m, 1H), 7.36-7.25 (d, *J* = 46.4 Hz, 4H), 6.85 (s, 2H), 6.20 (t, *J =* 54.2 Hz, 1H), 5.17 (brs, 2H), 4.73-4.67 (m, 2H), 4.44-4.36 (m, 4H), 4.27-4.14 (m, 9H), 3.97-3.95 (m, 5H), 3.72-3.51 (m, 7H), 2.99-2.89 (m, 2H), 2.81-2.74 (m, 4H), 2.68-2.62 (m, 1H), 2.26-2.18 (m, 2H), 2.002-1.99(m, 2H), 1.89-1.76 (m, 3H), 1.60 (brs, 1H), 1.36-1.33 (m, 6H), 1.23 (d, *J* = 3.4 Hz, 3H). |
| | 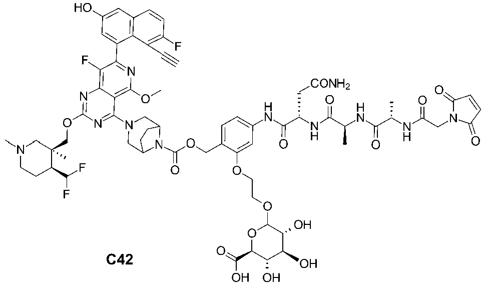 | |
| | **Payload: P3** | |
| | **Linker: B24** | ¹⁹F NMR (376 MHz, CD₃CN) δ - 110.93 (s, 1F), -119.65 (d, *J* = 289.6 Hz, 1F), -122.63 (d, *J* = 287.2 Hz, 1F), -151.48 (d, *J* = 70.6Hz, 1F). **MS-ESI [M+H]⁺:** 1443.4 |
| **C43** | | ¹H NMR (400 MHz, DMSO-*d*₆) δ 10.18 (s, 1H), 9.71 (d, *J* = 22.2 Hz, 1H), 9.40 (s, 1H), 8.37-8.24 (m, 1H), 8.17-7.92 (m, 3H), 7.65 (dd, *J* = 8.4, 5.8 Hz, 2H), 7.47 (t, *J* = 9.0 Hz, 1H), 7.42-7.32 (m, 4H), 7.22 (t, *J =* 3.4 Hz, 1H), 7.07 (d, *J* = 16.4 Hz, 1H), 6.96 (d, *J =* 21.6 Hz, 2H), 6.43 (t, *J =* 54.6 Hz, 1H), 5.10 (s, 2H), 4.68-4.55 (m, 2H), 4.47-4.14 (m, 9H), 3.90 (s, 6H), 3.58-3.35 (m, 8H), 3.25-3.07 (m, 4H), 2.80 (d, *J* = 3.6 Hz, 4H), 2.61-2.52 (m, 2H), 2.31-2.13 (m, 2H), 1.94-1.57 (m, 7H), 1.27-1.18 (m, 8H). |
| | 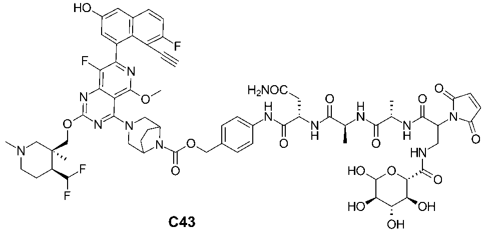 | |
| | **Payload: P3** | |
| | **Linker: B25+B19** | ¹⁹F NMR (376 MHz, DMSO-*d*₆) δ - 110.38 (s, 1F) , -117.57 (d,*J =* 285.6 Hz, 1F), -120.18 (d, *J =* 285.6 Hz, 1F), -149.46 (d, *J* = 51.2 Hz, 1F). |
| | | **MS-ESI [M+H]⁺:** 1412.3 |
| **C44** | | ¹H NMR (400 MHz, DMSO-*d*₆) δ 10.50 (s, 1H), 8.36-8.17 (m, 2H), 8.03-7.81 (m, 3H), 7.75 (d, *J* = 8.2 Hz, 2H), 7.60-7.28 (m, 8H), 7.25-7.15 (m, 2H), 6.99 (s, 2H), 6.90-6.80 (m, 1H), 6.30 (t, *J* = 55.4 Hz, 1H), 5.17-5.02 (m, 3H), 4.79-4.54 (m, 2H), 4.51-4.01 (m, 9H), 3.96 (d, *J* = 5.5 Hz, 1H), 3.89 (s, 3H), 3.55-3.30 (m, 4H), 3.01 (s, 3H), 2.93-2.62 (m, 4H), 2.36-2.00 (m, 6H), 1.92-1.40 (m, 12H), 1.26-1.02 (m, 10H). |
| | 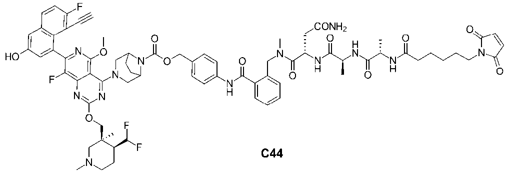 | |
| | **Payload: P3** | |
| | **Linker: B29** | ¹⁹F NMR (376 MHz, DMSO-*d*₆) δ - 110.49 (d, *J* = 6.8 Hz, 1F), -115.80 (dd, *J* = 281.8, 18.0 Hz, 1F), -118.95 (dd, *J* = 281.8, 23.8 Hz, 1F), -149.45 (d, *J* = 7.0 Hz, 1F). |
| | | **MS-ESI [M+Na]⁺:** 1432.5 |
| **C45** | | ¹H NMR (400 MHz, CD₃CN/D₂O) δ 10.20 (s, 1H), 9.59-9.22 (m, 1H), 8.27-7.93 (m, 4H), 7.72-7.19 (m, 12H), 7.03-6.96 (m, 2H), 6.42 (t, *J =* 55.2 Hz, 1H), 5.14-4.94 (m, 2H), 4.88-4.77 (m, 1H), 4.64-4.11 (m, 10H), 3.91-3.86 (m, 5H), 3.59-3.48 (m, 4H), 3.40-3.32 (m, 3H), 2.96-2.83 (m, 4H), 2.82-2.75 (m, 3H), 2.60-2.52 (m, 2H), 2.29-2.16 (m, 1H), 2.11-2.06 (m, 2H), 1.98-1.62 (m, 6H), 1.52-1.40 (m, 4H), 1.26-1.13 (m, 11H). |
| | 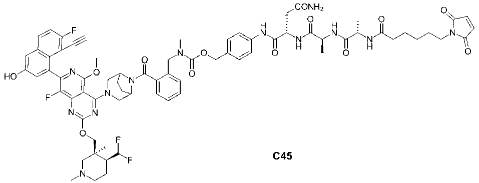 | |
| | **Payload: P3** | |
| | **Linker: A13** | ¹⁹F NMR (376 MHz, DMSO-*d*₆) δ - 110.42 (d, *J* = 22.7 Hz, 1F), -117.47 (d, *J =* 285.7 Hz, 1F), -120.16 (dd,*J* = 284.0, 30.6 Hz, 1F), -149.42 (t, *J* = 53.8 Hz, 1F). |
| | | **MS-ESI [M+H]⁺:** 1410.4 |
| **C46** | | ¹H NMR (400 MHz, CD₃CN) δ 7.93-7.87 (m, 1H), 7.51-7.20 (m, 6H), 6.84 (d, *J* = 7.8 Hz, 2H), 6.19 (t, *J* = 56.0 Hz, 1H), 5.16 (d, *J* = 8.0 Hz, 2H), 4.75-4.60 (m, 3H), 4.37 (brs, 3H), 4.28-4.19 (m, 7H), 4.08-3.93 (m, 6H), 3.73-3.37 (m, 10H), 3.20-3.12 (m, 4H), 3.05-3.02 (m, 1H), 2.78-2.57 (m, 8H), 2.17-2.08 (m, 2H), 1.99 (s, 1H), 1.89-1.75 (m, 5H), 1.65-1.59 (m, 1H), 1.37-1.33 (m, 6H), 1.21 (d, *J =* 4.0 Hz, 3H). |
| | 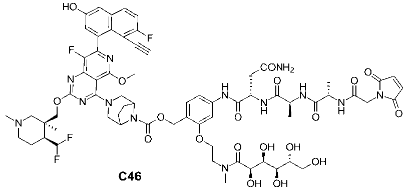 | |
| | **Payload: P3** | |
| | **Linker: B30** | ¹⁹F NMR (376 MHz, CD₃CN) δ - 110.96 (d, *J* = 10.3 Hz, 1F), - 118.85(d, *J* = 285.76 Hz, 1F), - 121.89(d, *J =* 323.36 Hz, 1F), -151.36 (dd, *J* = 72.0, 39.4 Hz, 1F). |
| | | **MS-ESI [M+H]⁺:** 1458.6 |
| **C47** | | ¹H NMR (400 MHz, DMSO-*d*₆) δ 7.97 (dd, *J* = 9.2, 5.8 Hz, 1H), 7.50-7.40 (m, 3H), 7.31-7.20 (m, 3H), 7.02 (s, 1H), 6.28 (t, *J* = 56.0 Hz,1H), 5.11 (s, 2H), 4.62-4.57 (m, 1H), 4.49-3.89 (m, 38H), 3.43-3.38 (m, 2H), 3.13-3.06 (m, 2H), 2.97-2.71 (m, 35H), 2.11 (s, 3H), 2.01 (d, *J* = 8.0 Hz, 2H), 1.86-1.63 (m, 10H), 1.42 (d, *J* = 8.0 Hz, 1H), 1.29-1.24 (m, 5H), 1.11 (s, 3H). |
| | 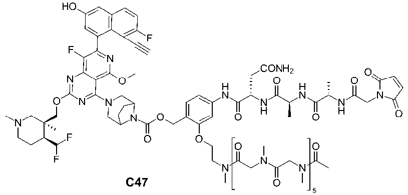 | |
| | **Payload: P3** | |
| | **Linker: B31** | ¹⁹F NMR (376 MHz, DMSO-*d*₆) δ - 110.19(s, 1F), -115.97 (d, *J* = 280.8 Hz, 1F), -119.20 (d, *J* = 282.1 Hz, 1F), -149.50(s, 1F). |
| | | **MS-ESI [M+2H]²⁺/2:** 1017.5 |
| **C48** | | ¹H NMR (400 MHz, CD₃CN) δ 7.90 (dd, *J* = 9.2, 5.8 Hz, 1H), 7.52-7.20 (m, 6H), 6.85 (s, 2H), 6.19 (t, *J =* 56.0 Hz, 1H), 5.14 (d, *J* = 12.0Hz, 2H), 4.73-4.69 (m, 1H), 4.59 (d, *J* = 8.0 Hz, 1H), 4.36-4.33 (m, 4H), 4.26-4.23 (m, 1H), 4.21-4.16 (m, 6H), 3.94 (d, *J =* 4.0 Hz, 3H), 3.77-3.70 (m, 2H), 3.67-3.54 (m, 33H), 3.49-3.47 (m, 3H), 3.29-3.26 (m, 6H), 3.07-2.97 (m, 3H), 2.78-2.67 (m, 1H), 2.67-2.60 (m, 1H), 2.53-2.41 (m, 4H), 2.08-1.99 (m, 3H), 1.89-1.77 (m, 4H), 1.65-1.57 (m, 1H), 1.37-1.37 (m, 6H), 1.19-1.18 (m, 3H). |
| | 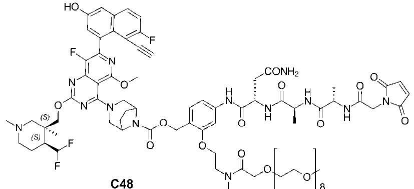 | |
| | **Payload: P3** | |
| | **Linker: B32** | |
| | | ¹⁹F NMR (376 MHz, CD₃CN) δ - 110.99 (d, *J* = 7.6 Hz, 1F), -118.63 (d, *J* = 283.8 Hz, 1F), -121.77 (d, *J =* 286.4 Hz, 1F), -151.28 (dd, *J* = 34.2, 7.2 Hz, 1F). |
| | | **MS-ESI [M+2H]²⁺/2:** 853.4 |

### Preparation of Intermediate C49: N-[(2-{[3-(7'-{[(3S,4S)-4-(difluoromethyl)-1-methyl-3-methylpiperidin-3-yl]methoxy}-8-ethynyl-1',7-difluoro-3-hydroxy-4'-methoxy-3',6',8'-triaza-1,2'-binaphthalen-5'-yl)-3,8-diazabicyclo[3.2.1]octan-8-yl]carbonyl}phenyl)methyl]-N-methyl-(S)-2-[(S)-2-{(S)2-[5-(2,5-dioxo-3-pyrrolin-1-yl)pentylcarbonylamino]propanamido}propanamido]succinamide

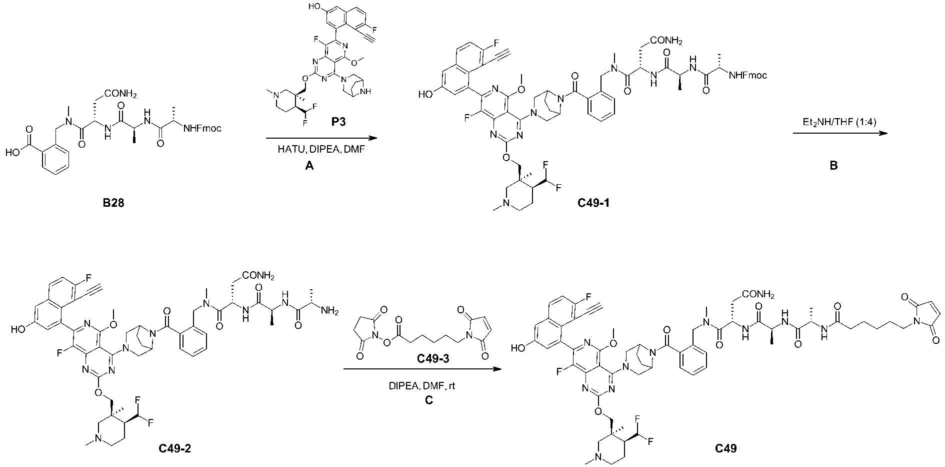

**Step A:** At room temperature, **B28** (110 mg, 0.17 mmol) was dissolved in DMF (2 mL), and **P3** (115 mg, 0.17 mmol), HATU (66 mg, 0.17 mmol), and *N*,*N*-diisopropylethylamine (45 mg, 0.35 mmol) were added. The mixture was stirred at room temperature for 2 hours. After the reaction was complete, water (100 mL) was added, and the mixture was extracted with dichloromethane (3 × 50 mL). The organic phases were combined, dried over anhydrous sodium sulfate and filtered. The filtrate was concentrated, and the resulting crude product was purified by FCC (SiO₂, MeOH/DCM = 0-10%) to give **C49-1** (140 mg, 64% yield) as a white solid. MS-ESI [M+H]⁺: 1290.5.

**Step B:** At room temperature, **C49-1** (140 mg, 0.11 mmol) was dissolved in tetrahydrofuran (4 mL), and diethylamine (1 mL) was added. The mixture was stirred at room temperature for 3 hours. After the reaction was complete, the reaction solution was concentrated. The resulting crude product was purified by FCC (SiO₂, MeOH/DCM = 0-10%) to give **C49-2** (50 mg, 43% yield) as a yellow solid. MS-ESI [M+H]⁺: 1068.4.

**Step C:** At room temperature, **C49-2** (50 mg, 0.05 mmol) was dissolved in DMF (2 mL), and **C49-3** (16 mg, 0.05 mmol) and *N,N*-diisopropylethylamine (13 mg, 0.10 mmol) were added. The mixture was stirred at room temperature overnight. After the reaction was complete, the reaction solution was purified by reverse phase C18 column (ACN/H₂O(0.1% HCO₂H) = 0-60%) to give **C49** (20 mg, 34% yield) as a white solid. ¹H NMR (400 MHz, DMSO-*d*₆) δ 10.30 (s, 1H), 8.42-8.11 (m, 2H), 8.02-7.79 (m, 3H), 7.50-7.30 (m, 6H), 7.25-7.17 (m, 2H), 6.99 (s, 2H), 6.91-6.78 (m, 1H), 6.29 (t, *J =* 55.3 Hz, 1H), 5.22-4.98 (m, 1H), 4.90-3.79 (m, 15H), 3.67-3.39 (m, 4H), 3.03 (s, 3H), 2.93-2.64 (m, 4H), 2.37-1.39 (m, 18H), 1.28-0.99 (m, 10H). ¹⁹F NMR (376 MHz, DMSO-*d*₆) δ -110.53 (s, 1F), -115.82 (dd, *J* = 281.2, 27.63 Hz, 1F), -118.87 (dd, *J* = 281.2, 38.38 Hz, 1F), -149.47 (s, 1F). MS-ESI [M+H]⁺: 1261.5.

Intermediates **C50-C51** were prepared following the synthetic route for linker **C49,** using the intermediates in the table below.

| **No.** | **Structural Formula/Payload/Linker** | **¹H NMR/MS-ESI [M+H]⁺** |
|---|---|---|
| **C50** | | ¹H NMR (400 MHz, DMSO-*d*₆) δ 10.23 (s, 1H), 9.57 (s, 1H), 8.58 (s, 1H), 8.36 (d, *J =* 7.6 Hz, 1H), 8.16-7.81 (m, 3H), 7.50-7.37 (m, 3H), 7.34-7.20 (m, 2H), 7.08 (d, *J* = 1.6 Hz, 2H), 6.79 (d, *J =* 29.1 Hz, 1H), 6.43 (t, *J =* 54.8 Hz, 1H), 5.18-4.73 (m, 3H), 4.72-4.37 (m, 4H), 4.35-4.08 (m, 4H), 4.08-3.85 (m, 8H), 3.17-2.88 (m, 3H), 2.82-2.59 (m, 4H), 2.48-1.36 (m, 10H), 1.37-1.04 (m, 11H). |
| | 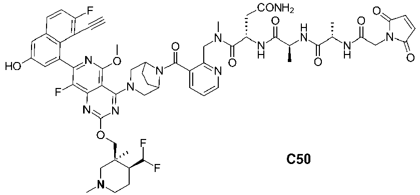 | |
| | **Payload: P3** | ¹⁹F NMR (376 MHz, DMSO-*d*₆) δ -110.37 (s, 1F) , -117.46 (d, *J* = 285.6 Hz, 1F), -120.09 (d, *J* = 285.6 Hz, 1F), -149.41 (d, *J* = 56.2 Hz, 1F). |
| | **Linker: B33** | |
| | | **MS-ESI [M+H]⁺:** 1206.5 |
| **C51** | | ¹H NMR (400 MHz, DMSO-*d₆*) δ 9.49 (s, 1H), 8.53 (d, *J* = 13.6 Hz, 1H), 8.43-8.21 (m, 2H), 8.07-7.94 (m, 2H), 7.65 (t, *J* = 7.6 Hz, 1H), 7.47 (td, *J* = 9.0, 1.8 Hz, 2H), 7.42-7.31 (m, 2H), 7.26-7.21 (m, 1H), 7.08 (d, *J* = 1.4 Hz, 2H), 6.86 (d, *J =* 22.2 Hz, 1H), 6.43 (t, *J =* 54.8 Hz, 1H), 5.17-4.96 (m, 1H), 4.88 (s, 1H), 4.79-4.21 (m, 5H), 4.19-4.05 (m, 4H), 3.95-3.65( m, 8H ), 3.55 (d, *J =* 12.8 Hz, 4H), 3.13-2.85 (m, 4H), 2.84-2.57 (m, 5H), 2.33-2.16 (m, 1H), 1.92 (s, 5H), 1.28-1.13 (m, 9H). |
| | 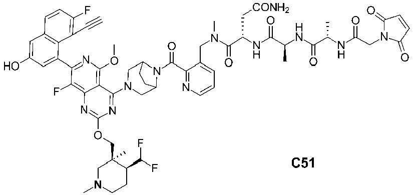 | |
| | **Payload: P3** | |
| | **Linker: B34** | ¹⁹F NMR (376 MHz, DMSO-*d*₆) δ -110.44 (d, *J* = 27.4 Hz, 1F), -117.93 (d, *J =* 9.8 Hz, 1F), - 119.75 (d, *J* = 16.2 Hz, 1F), -149.49 (d, *J =* 67.4 Hz, 1F). |
| | | **MS-ESI [M+H]⁺:** 1206.4 |

### Preparation of Intermediate C52: {5-[2-({N-methyl[(S)-1-[(S)-2-[(S)-2-)-2-{[(2,5-dioxo-3-pyrrolin-1-yl)methyl]carbonylamino}propanamido]propanamido]-2-formylethyl]carbonylamino}-methyl)piperidin-1-ylcarbonyloxy]pyridin-2-yl}methyl3-(7'-{[(3S,4S)-4-(difluoromethyl)-1-methyl-3-methylpiperidin-3-yl]methoxy}-8-ethynyl-1',7-difluoro-3-hydroxy-4'-methoxy-3',6',8'-triaza-1',2'-binaphthalen-5'-yl)-3,8-diazabicyclo[3.2.1]octane-8-carboxylate

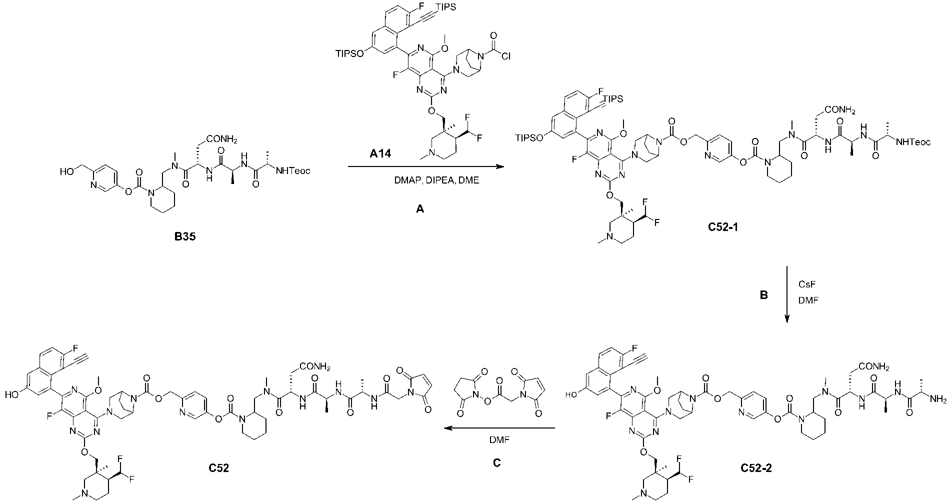

**Step A:** At room temperature, **B35** (110 mg, 0.16 mmol) was dissolved in ethylene glycol dimethyl ether (5 mL). 4-Dimethylaminopyridine (30 mg, 0.25 mmol), **A14** (255 mg, 0.25 mmol), and *N*,*N*-diisopropylethylamine (42 mg, 0.33 mmol) were added. The tube was sealed and stirred at 100 °C for 50 hours. After the reaction was complete, the reaction solution was concentrated. The resulting crude product was purified by reverse phase C18 column (ACN/H₂O(0.1% HCO₂H) = 0-60%) to give **C52-1** (60 mg, 22% yield) as a white solid. MS-ESI [M+2H]⁺/2: 842.1.

**Step B:** At room temperature, **C52-1** (60 mg, 0.04 mmol) was dissolved in DMF (2 mL), and cesium fluoride (270 mg, 1.78 mmol) was added. The mixture was stirred at room temperature overnight. After the reaction was complete, the reaction solution was purified by reverse phase C18 column (ACN/H₂O(0.1% NH₄HCO₃) = 0-60%) to give **C52-2** (20 mg, 45% yield) as a white solid. MS-ESI [M+Na]⁺: 1248.5.

**Step C:** At room temperature, **C52-2** (20 mg, 0.02 mmol) was dissolved in DMF (2 mL), and maleimidoacetic acid succinimidyl ester (5 mg, 0.02 mmol) was added. The mixture was stirred at room temperature overnight. After the reaction was complete, the reaction solution was purified by pre-HPLC (C18, ACN/H₂O(0.1% HCO₂H) = 0-70%) to give **C52** (5 mg, 23% yield) as a white solid. ¹H NMR (400 MHz, DMSO-*d*₆) δ 8.58-7.91 (m, 6H), 7.74-7.15 (m, 6H), 7.10-7.03 (m, 2H), 6.88-6.60 (m, 1H), 6.30 (t, *J* = 55.4 Hz, 1H), 5.21 (s, 1H), 5.15-4.90 (m, 1H), 4.55-3.80 (m, 12H), 3.78-3.17 (m, 14H), 3.13-2.54 (m, 5H), 2.30-2.16 (m, 1H), 2.10 (s, 3H), 1.94-1.05 (m, 22H). ¹⁹F NMR (376 MHz, DMSO-*d*₆) δ -110.64 (s, 1F), -115.75 (dd, *J =* 281.6, 18.0 Hz, 1F), -118.93 (dd, *J =* 281.6, 23.2 Hz, 1F), -149.45 (s, 1F). MS-ESI [M+H]⁺: 1363.5.

Intermediates **C53-C55** were prepared following the synthetic route for linker **C52,** using the intermediates in the table below.

| **No.** | **Structural Formula/Payload/Linker** | **¹H NMR / MS-ESI [M+H]⁺** |
|---|---|---|
| **C53** | 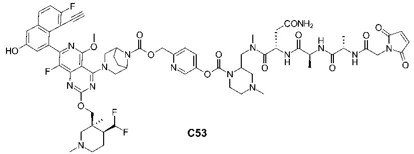 | ¹H NMR (400 MHz, CD₃CN/D₂O) δ 8.52-8.24 (m, 2H), 7.93-7.86 (m, 1H), 7.71-7.46 (m, 2H), 7.42-7.32 (m, 2H), 7.31-7.25 (m, 1H), 6.88-6.81 (m, 2H), 6.19 (t, *J =* 55.2 Hz, 1H), 5.24 (s, 2H), 5.20-4.95 (m, 1H), 4.75-3.44 (m, 15H), 3.20-2.01 (m, 25H), 1.91-1.59 (m, 5H), 1.38-1.10 (m, 11H). |
| | **Payload:P3** | ¹⁹F NMR (376 MHz, CD₃CN/D₂O) δ -111.03 (s, 1F), -118.01 (d, *J* = 284.0 Hz, 1F), -121.31 (d, *J =* 284.0 Hz, 1F), -151.12 (s, 1F). |
| | **Linker: B36** | |
| | | **MS-ESI [M+H]⁺:** 1378.5 |
| **C54** | 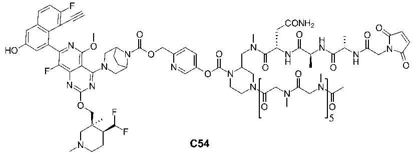 | **MS-ESI [M+2H]²⁺/2:** 1059.1 |
| | **Payload:P3** | |
| | **Linker: B37** | |
| **C55** | 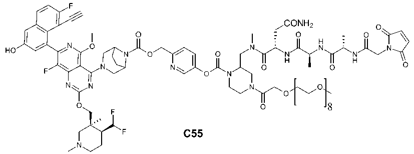 | ¹H NMR (400 MHz, CD₃CN/D₂O) δ 8.41 (s, 1H), 7.91 (dd, *J* = 9.2, 5.8 Hz, 1H), 7.75-7.64 (m, 1H), 7.54 (d, *J =* 8.4 Hz, 1H), 7.45-7.33 (m, 2H), 7.29 (s, 1H), 6.89-6.83 (m, 2H), 6.16 (t, *J* = 55.2 Hz, 1H), 5.26 (s, 2H), 5.17-4.94 (m, 1H), 4.77-3.72 (m, 18H), 3.71-3.46 (m, 37H), 3.34-2.83 (m, 11H), 2.74-2.14 (m, 7H), 1.95-1.60 (m, 8H), 1.37-1.05 (m, 9H). |
| | **Payload:P3** | |
| | | ¹⁹F NMR (376 MHz, CD₃CN/D₂O) δ -111.98 (s, 1F), -117.33 (d, *J* = 250.0 Hz, 1F), -120.81 (d, *J =* 250.0 Hz, 1F), -150.88 (s, 1F). |
| | **Linker: B38** | |
| | | **MS-ESI [M+H]⁺:** 1788.8 |

### Preparation of Compound C56: {p-[(S)-2-[(S)-2-[(S)-2-{3-[(1R,2S,3R,4R)-1,2,3,4,5-pentahydroxypentylcarbonylamino]-2-(2,5-dioxo-3-pyrrolidin-1-yl)propanamido}propanamido]propanamido]-3-carbamoylpropanamido]phenyl} methyl 3-(7'-{[(3S,4S)-4-(difluoromethyl)-1-methyl-3-methyl-3-piperidinyl]methoxy}-8-ethynyl-1',7-difluoro-3-hydroxy-4'-methoxy-3',6',8'-triaza-1,2'-binaphthalen-5'-yl)-3,8-diazabicyclo[3.2.1]octane-8-carboxylate

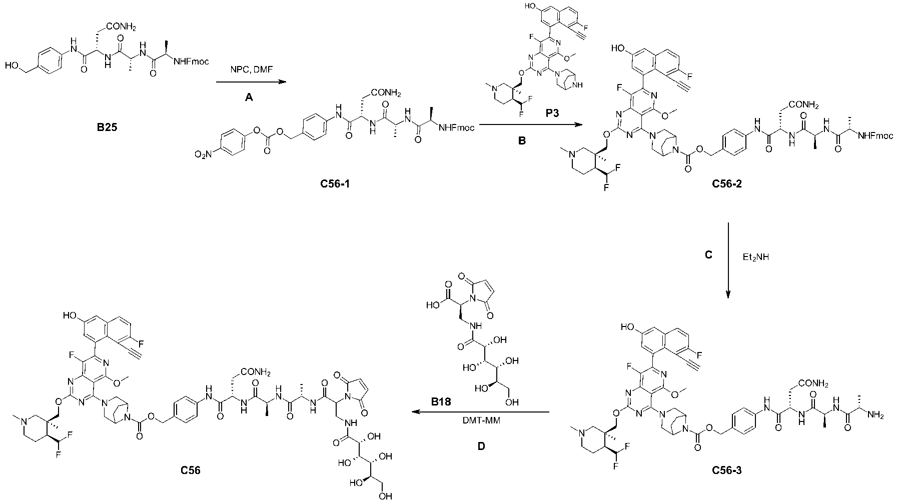

### Step A: {p-[(S)-2-[(R)-2-[(R)-2-{(9H-fluoren-9-yl)methyl}propanamido]propanamido]-3-carbamoylpropanamido]phenyl} p-nitrophenyl methyl carbonate

Under ice-water bath, (*R*)-1-[*N*-(*R*)-1-{*N*-(*S*)-1,2-bis[*N*-*p-*(hydroxymethyl)phenylcarbamoyl]ethylcarbamoyl}ethylcarbamoyl]ethyl (9*H*-fluoren-9-yl)methyl carbamate (500.0 mg, 0.83 mmol) was dissolved in *N,N*-dimethylformamide (6 mL). Bis(p-nitrophenyl) carbonate (555.5 mg, 1.83 mmol) and *N,N*-diisopropylethylamine (321.2 mg, 2.49 mmol) were added sequentially. After addition, the reaction solution was stirred at room temperature for 3 hours. After the reaction was complete, the reaction solution was poured into methyl tert-butyl ether (70 mL) to precipitate a solid, stirred at room temperature for 30 minutes, filtered, and the filter cake was dried to give {*p*-[(*S*)-2-[(*R*)-2-[(*R*)-2-{(9*H*-fluoren-9-yl)methyl}propanamido]propanamido]-3-carbamoylpropanamido]phenyl }p-nitrophenyl methyl carbonate (450.8 mg, 70% yield) as a brown-yellow solid. MS-ESI [M+H]⁺: 767.2.

### Step B: {p-[(S)-2-[(S)-2-[(S)-2-{[(9H-fluoren-9-yl)methyl](oxycarbonylamino)}propanamido]propanamido]-3-carbamoylpropanamido]phenyl}methyl 3-(7'-{[(3S,4S)-4-(difluoromethyl)-1-methyl-3-methyl-3-piperidinyl]methoxy}-8-ethynyl-1',7-difluoro-3-hydroxy-4'-methoxy-3',6',8'-triaza-1,2'-binaphthalen-5'-yl)-3,8-diazabicyclo[3.2.1]octane-8-carboxylate

Under ice-water bath, {*p*-[(*S*)-2-[(*R*)-2-[(*R*)-2-{(9*H*-fluoren-9-yl)methyl}propanamido]propanamido]-3-carbamoylpropanamido]phenyl} p-nitrophenyl methyl carbonate (200 mg, 0.26 mmol) and 7'-{[(3*S*,4*S*)-4-(difluoromethyl)-1-methyl-3-methyl-3-piperidinyl]methoxy}-5'-(3,8-diazabicyclo[3.2.1]octan-3-yl)-8-ethynyl-1',7-difluoro-4'-methoxy-3',6',8'-triaza-[1,2'-binaphthalen]-3-ol (172.0 mg, 0.26 mmol) were dissolved in *N,N-*dimethylformamide (2 mL), and *N,N*-diisopropylethylamine (51.0 mg, 0.39 mmol) was added. After addition, the reaction solution was stirred under ice-water bath for 3 hours. After the reaction was complete, the reaction solution was poured into methyl tert-butyl ether (25 mL) to precipitate a solid, stirred at room temperature for 30 minutes, filtered, and the filter cake was the product {*p*-[(*S*)-2-[(*S*)-2-[(*S*)-2-{(9*H*-fluoren-9-yl)methyl}propanamido]propanamido]-3-carbamoylpropanamido]phenyl}methyl 3-(7'-{[(3*S*,4*S*)-4-(difluoromethyl)-1-methyl-3-methyl-3-piperidinyl]methoxy }-8-ethynyl-1',7-difluoro-3-hydroxy-4'-methoxy-3',6',8'-triaza-1,2'-binaphthalen-5'-yl)-3,8-diazabicyclo[3.2.1]octane-8-carboxylate (268.7 mg, 80% yield) as a brown solid. MS-ESI [M+H]⁺: 1292.4.

### Step C: {p-[(S)-2-{(S)-2-[(S)-2-aminopropanamido]propanamido}-3-carbamoylpropanamido]phenyl}methyl 3-(7'-{[(3S,4S)-4-(difluoromethyl)-1-methyl-3-methyl-3-piperidinyl]methoxy}-8-ethynyl-1',7-difluoro-3-hydroxy-4'-methoxy-3',6',8'-triaza-1,2'-binaphthalen-5'-yl)-3,8-diazabicyclo[3.2.1]octane-8-carboxylate

Under ice-water bath, {*p*-[(*S*)-2-[(*S*)-2-[(*S*)-2-{(9*H*-fluoren-9-yl)methyl}propanamido]propanamido]-3-carbamoylpropanamido]phenyl}methyl 3-(7'-{[(3*S*,4*S*)-4-(difluoromethyl)-1-methyl-3-methyl-3-piperidinyl] methoxy } -8-ethynyl-1',7-difluoro-3-hydroxy-4'-methoxy-3',6',8'-triaza-1,2'-binaphthalen-5'-yl)-3,8-diazabicyclo[3.2.1]octane-8-carboxylate (100.0 mg, 0.08 mmol) was added to a prepared solution of diethylamine/*N*,*N*-dimethylformamide (V/V = 1/19, 1 mL). The reaction solution was stirred under ice-water bath for 3 hours. After the reaction was complete, the reaction solution was purified by pre-HPLC (C18, (H₂O/MeOH)/MeOH = 45-55%) to give the product {*p*-[(*S*)-2-{(*S*)-2-[(*S*)-2-aminopropanamido]propanamido}-3-carbamoylpropanamido]phenyl}methyl 3-(7'-{[(3*S*,4*S*)-4-(difluoromethyl)-1-methyl-3-methyl-3-piperidinyl]methoxy}-8-ethynyl-1',7-difluoro-3-hydroxy-4'-methoxy-3',6',8'-triaza-1,2'-binaphthalen-5'-yl)-3,8-diazabicyclo[3.2.1]octane-8-carboxylate (51.4 mg, 60.0% yield) as a pale yellow solid. MS-ESI [M+H]⁺: 1070.4.

### Step D: {p-[(S)-2-[(S)-2-[(S)-2-{3-[(1R,2S,3R,4R)-1,2,3,4,5-pentahydroxypentylcarbonylamino]-2-(2,5-dioxo-3-pyrrolidin-1-yl)propanamido}propanamido]propanamido] -3-carbamoylpropanamidolphenyl}methyl 3-(7'-{[(3S,4S)-4-(difluoromethyl)-1-methyl-3-methyl-3-piperidinyl]methoxy}-8-ethynyl-1',7-difluoro-3-hydroxy-4'-methoxy-3',6',8'-triaza-1,2'-binaphthalen-5'-yl)-3,8-diazabicyclo[3.2.1]octane-8-carboxylate

Under ice-water bath, (*S*)-3-[(1*R*,2*S*,3*R*,4*R*)-1,2,3,4,5-pentahydroxypentylcarbonylamino]-2-(2,5-dioxo-3-pyrrolin-1-yl)propanoic acid (90.0 mg, 0.25 mmol) and 4-(4,6-dimethoxy-1,3,5-triazin-2-yl)-4-methylmorpholinium chloride (63.0 mg, 0.23 mmol) were stirred in anhydrous *N,N-*dimethylformamide (1 mL) for 30 minutes. Then {*p*-[(*S*)-2-{(*S*)-2-[(*S*)-2-aminopropanamido]propanamido}-3-carbamoylpropanamido]phenyl}methyl 3-(7'-{[(3*S*,4*S*)-4-(difluoromethyl)-1-methyl-3-methyl-3-piperidinyl]methoxy}-8-ethynyl-1',7-difluoro-3-hydroxy-4'-methoxy-3',6',8'-triaza-1,2'-binaphthalen-5'-yl)-3,8-diazabicyclo[3.2.1]octane-8-carboxylate (276.5 mg, 0.25 mmol) was added to the above reaction solution and stirring continued for 1 hour. After the reaction was complete, the reaction solution was purified by pre-HPLC (C18, (0.1% TFA(aq)/ACN)/ACN *=* 45-55%) to give {*p*-[(*S*)-2-[(*S*)-2-[(*S*)-2-{3-[(1*R*,2*S*,3*R*,4*R*)-1,2,3,4,5-pentahydroxypentylcarbonylamino]-2-(2,5-dioxo-3-pyrrolidin-1-yl)propanamido}propanamido]propanamido]-3-carbamoylpropanamido]phenyl}methyl 3-(7'-{[(3*S*,4*S*)-4-(difluoromethyl)-1-methyl-3-methyl-3-piperidinyl]methoxy}-8-ethynyl-1',7-difluoro-3-hydroxy-4'-methoxy-3',6',8'-triaza-1,2'-binaphthalen-5'-yl)-3,8-diazabicyclo[3.2.1]octane-8-carboxylate (70.7 mg, 20% yield) as a yellow solid. ¹H NMR (400 MHz, DMSO-*d*₆) δ 9.74 (s, 1H), 9.50 (s, 1H), 8.32 (d, *J =* 7.2 Hz, 1H), 8.14 (d, *J =* 6.6 Hz, 1H), 8.09 (d, *J =* 7.6 Hz, 1H), 7.98 (dd, *J* = 9.2, 5.6 Hz, 1H), 7.75 (t, *J =* 6.2 Hz, 1H), 7.65 (d, *J =* 8.0 Hz, 2H), 7.47 (t, *J =* 8.8 Hz, 1H), 7.42-7.33 (m, 4H), 7.23 (t, *J =* 3.2Hz, 1H), 6.99 (s, 2H), 6.93 (s, 1H), 6.43 (t, *J =* 54.7 Hz, 1H), 5.10 (s, 2H), 4.75-3.95 (m, 10H), 3.94-3.46 (m, 12H), 3.43-3.26 (m, 3H), 3.13-2.82 (m, 3H), 2.82-2.72 (m, 3H), 2.55 (t, *J =* 6.8 Hz, 2H), 2.65-1.46 (m, 11H), 1.30-1.05 (m, 11H). ¹⁹F NMR (376 MHz, DMSO-*d₆*) δ -110.39(s, 1F), -117.55 (d, *J* = 284.6 Hz, 1F), -120.17 (d, *J* = 284.4 Hz, 1F), -149.45 (d, *J =* 51.2 Hz , 1F). MS-ESI [M+H]⁺: 1414.5.

Intermediates **C57-C71** were prepared following the synthetic route for linker **C56,** using the intermediates in the table below.

| **No.** | **Structural Formula/Payload/Linker** | **¹H NMR / MS-ESI [M+H]⁺** |
|---|---|---|
| **C57** | 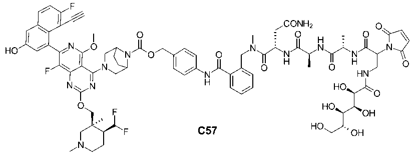 | ¹H NMR (400 MHz, DMSO-*d*₆) δ 8.82-8.73 (m, 4H), 8.36-8.27 (m, 1H), 8.24-8.18 (m, 2H), 8.03-7.91 (m, 2H), 7.76-7.73 (m, 4H), 7.56-7.51 (m, 1H), 7.48 (d, *J =* 9.0 Hz, 1H), 7.44 (d, *J* = 5.6 Hz, 1H), 7.40 (d, *J* = 7.8 Hz, 2H), 7.24-7.19 (m, 1H), 7.00-6.98 (m, 2H), 6.85 (d, *J =* 22.6 Hz, 1H), 6.44 (t, *J =* 54.6 Hz, 1H), 5.13 (s, 2H), 4.73-4.54 (m, 10H), 4.48-4.34 (m,9H), 4.32-4.15 (m, 7H), 3.95-3.90 (m, 4H), 3.70-3.30 (m, 11H), 3.01 (s, 2H), 2.80 (s, 2H), 1.96-1.67 (m, 5H), 1.27-1.16 m, 6H), 1.15-1.06 (m, 3H). |
| | **Payload: P3** | |
| | **Linker: B29+B18** | ¹⁹F NMR (376 MHz, DMSO-*d*₆) δ -110.39(s, 1F), -117.55 (d, *J* = 285.6 Hz, 1F), -120.16 (d, *J* = 285.0 Hz , 1F), -149.44 (d, *J* = 53.6 Hz , 1F). |
| | | **MS-ESI [M+H]⁺:** 1562.5 |
| **C58** | 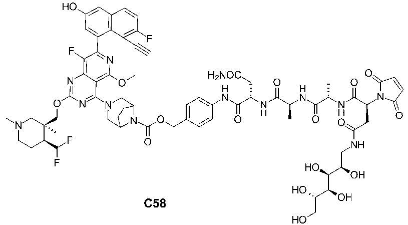 | ¹H NMR (400 MHz, CD₃CN/D₂O) δ 7.96-7.82 (m, 1H), 7.62 (d, *J =* 7.8 Hz, 2H), 7.44-7.21 (m, 5H), 6.99-6.67 (m, 2H), 6.15 (t, *J* = 54.6 Hz, 1H), 5.08 (d, *J =* 28.8 Hz, 2H), 4.70 (d, *J =* 12.6 Hz, 2H), 4.35 (s, 3H), 4.14 (s,4H), 3.92 (s, 4H), 3.80-3.40 (m, 12H), 3.00-2.50 (m, 11H), 2.15 (d, *J =* 34.9 Hz, 3H), 1.42-1.06 (m, 12H). |
| | **Payload: P3** | ¹⁹F NMR (400 MHz, CD₃CN/D₂O) δ - 108.17(s, 1F), -117.24 (dd, *J =* 287.2, 27.2 Hz, 1F), -120.07 (dd, *J* = 287.2, 19.2 Hz, 1F), - 148.97 (d, *J* = 58.2 Hz). |
| | **Linker: B25+B20** | |
| | | **MS-ESI [M+H]**⁺:1429.4 |
| **C59** | 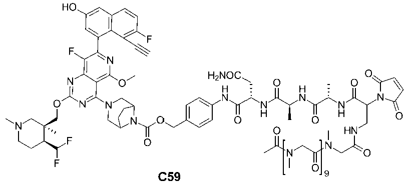 | ¹H NMR (400 MHz, CD₃CN/D₂O) δ 7.91 (ddd, *J* = 8.6, 5.8, 2.0 Hz, 1H), 7.77-7.66 (m, 2H), 7.447.35 (m, 4H), 7.30 (dd, *J* = 8.6, 2.6 Hz, 1H), 6.86 (t, *J* = 10.4 Hz, 2H), 6.19 (td, *J =* 55.4, 6.1 Hz, 1H), 5.14 (s, 2H), 4.74 (d, *J =* 12.2 Hz, 2H), 4.45-3.72 (m, 34H), 3.70-3.31 (m, 6H), 3.06-2.88 (m, 24H), 2.82-2.73 (m, 8H), 2.23 (s, 2H), 2.06 (d, *J* = 8.2 Hz, 3H), 1.92-1.51 (m, 10H), 1.45-1.01 (m, 7H). |
| | **Payload: P3** | ¹⁹F NMR (400 MHz, CD₃CN/D₂O) δ -110.96 (s, 1F), -119.97 (dd, *J =* 287.2, 27.8 Hz, 1F), - 123.10 (d, *J* = 20.6 Hz, 1F), -151.75 (d, *J* = 78.0 Hz, 1F). |
| | **Linker: B25+B21** | |
| | | **MS-ESI [M+2H]²⁺/2:** 995.1 |
| **C60** | 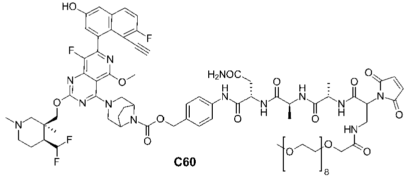 | ¹H NMR (400 MHz, DMSO-*d*₆) δ 9.73 (s, 1H), 9.41 (s, 1H), 8.32 (d, *J =* 7.2 Hz, 1H), 8.15 (d, *J* = 6.8 Hz, 1H), 8.09 (d, *J* = 7.6 Hz, 1H), 7.98 (dd, *J* = 9.2, 5.8 Hz, 1H), 7.82 (t, *J* = 6.2 Hz, 1H), 7.69-7.62 (m, 2H), 7.47 (t, *J* = 9.0 Hz, 1H), 7.40-7.34 (m, 4H), 7.22 (t, *J* = 3.3 Hz, 1H), 7.04 (d, *J* = 23.1 Hz, 2H), 6.94 (s, 1H), 6.43 (t, *J =* 54.5 Hz, 1H), 5.09 (s, 2H), 4.61 (dt, *J* = 7.6, 4.2 Hz, 2H), 4.47-4.03 (m, 9H), 3.91 (d, *J* = 6.2 Hz, 4H), 3.76 (d, *J* = 4.0 Hz, 3H), 3.50 (s, 34H), 3.23 (s, 4H), 3.05-2.76 (m, 6H), 2.55 (t, *J* = 6.8 Hz, 2H), 2.24 (d, *J* = 14.4 Hz, 1H), 1.98-1.61 (m, 6H), 1.24-1.10 (m, 9H). |
| | **Payload: P3** | |
| | **Linker: B25+B22** | |
| | | ¹⁹F NMR (376 MHz, DMSO-*d*₆) δ -110.39(s, 1F), -117.58 (d, *J* = 284.8 Hz, 1F), -120.19 (d, *J =* 285.0 Hz, 1F), -149.47 (d, *J =* 51.2 Hz, 1F). |
| | | **MS-ESI [M+H]⁺:** 1660.6 |
| **C61** | 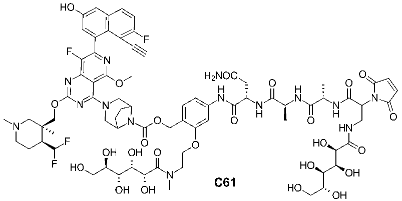 | ¹H NMR (400 MHz, CD₃CN) δ 7.96-7.87 (m, 1H), 7.52-7.17 (m, 6H), 6.86 (d, *J* = 11.8 Hz, 2H), 6.20 (t, *J =* 56.0 Hz, 1H), 5.17 (d, *J =* 8.0 Hz, 2H), 4.84-4.67 (d, *J* = 69.9 Hz, 4H), 4.39 (brs, 3H), 4.25-4.14 (m, 6H), 3.99-3.95 (m, 6H), 3.83-3.60 (m, 13H), 3.24 (d, *J =* 24.0 Hz, 3H), 3.03 (s, 1H), 2.93-2.72 (m, 8H), 2.22-2.15 (m, 2H), 1.90-1.58 (m, 5H), 1.40-1.27 (m, 8H), 1.21 (d, *J =* 4.0 Hz, 3H). |
| | **Payload: P3** | ¹⁹F NMR (376 MHz, CD₃CN) δ -110.88(s, 1F), -119.75 (d, *J* = 245.8 Hz, 1F), -122.19(d, *J =* 334.6 Hz, 1F), -151.55(s, 1F). |
| | **Linker: B30+B18** | |
| | | **MS-ESI [M+H]⁺:** 1665.6 |
| **C62** | 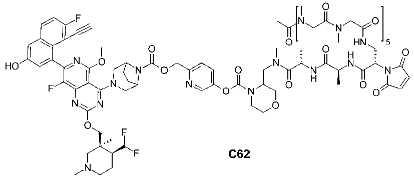 | ¹H NMR (400 MHz, CD₃CN) δ 8.42-8.29 (m, 1H), 7.93-7.86 (m, 1H), 7.72-7.58 (m, 1H), 7.56-7.50 (m, 1H), 7.40 (d, *J* = 2.6 Hz, 1H), 7.35 (t, *J* = 9.1 Hz, 1H), 7.27 (dd, *J* = 6.5, 2.6 Hz, 1H), 6.85-6.76 (m, 2H), 6.35-5.96 (m, 1H), 5.27-5.21 (m, 2H), 4.76-4.67 (m, 2H), 4.42 (s, 3H), 4.32-4.18 (m, 16H), 4.12-4.02(m, 6H), 3.95 (d, *J* = 2.8 Hz, 4H), 3.85-3.48 (m, 10H), 3.12-2.77 (m, 42H), 2.25-2.04 (m, 5H), 1.79-1.73 (m, 1H), 1.37-1.07 (m, 16H). |
| | **Payload: P3** | |
| | **Linker: B42** | ¹⁹F NMR (376 MHz, CD₃CN) δ -110.84 (d, *J* = 11.4 Hz, 1F), -120.01 (dd, *J* = 287.0, 22.4 Hz, 1F), -122.75 (dd, *J* = 287.0, 22.8 Hz, 1F), - 151.50 (d, *J =* 52.6 Hz, 1F). |
| | | **MS-ESI [M+2H]²⁺/2:** 1016.7 |
| **C63** | 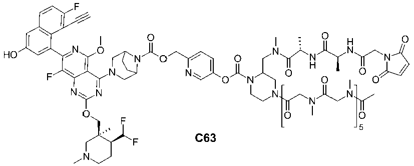 | ¹H NMR (400 MHz, CD₃CN) δ 8.36 (s, 1H), 7.87 (s, 1H), 7.74-7.50 (m, 2H), 7.42-7.20 (m, 3H), 6.98-6.52 (m, 2H), 6.16 (t, *J* = 54.8 Hz, 1H), 5.22 (s, 2H), 4.63 (s, 2H), 4.52-4.02 (m, 31H), 3.71-3.15 (m, 11H), 3.14-2.67 (m, 44H), 2.16 (s, 3H), 1.64-1.44 (m, 3H), 1.31-1.01 (m, 10H). |
| | **Payload: P3** | ¹⁹F NMR (376 MHz, CD₃CN) δ -107.82(s, 1F), -117.32 (d, *J* = 287.8 Hz, 1F), -119.66 (d, *J =* 287.8 Hz, 1F), -148.64(s, 1F). |
| | **Linker: B39** | |
| | | **MS-ESI [M+2H]²⁺/2:** 1001.7 |
| **C64** | 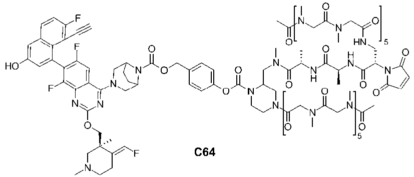 | ¹H NMR (400 MHz, CD₃CN) δ 7.96-7.92 (m, 1H), 7.63-7.61 (m, 1H), 7.48-7.45 (m, 3H), 7.42-7.37 (m, 1H), 7.23-7.20 (m, 1H), 7.18-7.00 (m, 2H), 6.86-6.80 (m, 2H), 6.36-6.08 (m, 1H), 5.20 (s, 2H), 4.78-4.47 (m, 9H), 4.40-4.20 (m, 30H), 4.15-3.95 (m, 14H), 3.85-3.56 (m, 11H), 3.03-2.82 (m, 70H), 2.29-2.05 (m, 8H), 1.81-1.77 (m, 3H), 1.26-1.10 (m, 9H). |
| | **Payload: P1** | ¹⁹F NMR (376 MHz, CD₃CN) δ -110.35 (s, 1F), -117.15 (s, 1F), -120.01 (d, *J* = 289.5 Hz, 1F), -122.43 (d, *J =* 289.5 Hz, 1F), -126.78 (s, 1F). |
| | **Linker: B40** | |
| | | **MS-ESI [M+2H]²⁺/2:** 1385.6 |
| **C65** | 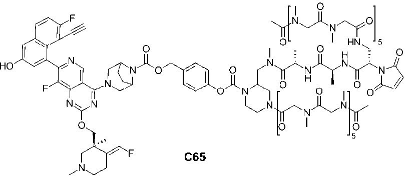 | ¹H NMR (400 MHz, CD₃CN) δ 9.06 (s, 1H), 7.94-7.90 (m, 1H), 7.48-7.43 (m, 3H), 7.40-7.35 (m, 1H), 7.28-7.26 (m, 1H), 7.15-7.00 (m, 2H), 6.97-6.76 (m, 3H), 5.19 (s, 2H), 4.75 (s, 3H), 4.60-4.40 (m, 6H), 4.37-4.20 (m, 27H), 4.15-3.96 (m, 14H), 3.90-3.78 (m, 4H), 3.70-3.25 (m, 8H), 3.07-2.77 (m, 72H), 2.08-2.05 (m, 4H), 1.92-1.75 (m, 7H), 1.30-1.10 (m, 9H). |
| | **Payload: P2** | ¹⁹F NMR (376 MHz, CD₃CN) δ -110.86 (s, 1F), -134.65 (d, *J =* 97.1 Hz, 1F), -140.72 (d, *J* = 106.8 Hz, 1F). |
| | **Linker: B40** | |
| | | **MS-ESI [M+2H]²⁺/2:** 1367.1 |
| **C66** | 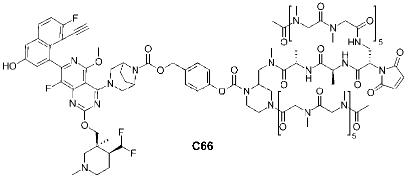 | ¹H NMR (400 MHz, CD₃CN) δ 7.95-7.91 (m, 1H), 7.47-7.44 (m, 3H), 7.42-7.37 (m, 1H), 7.32-7.29 (m, 1H), 7.18-7.00 (m, 2H), 6.86-6.80 (m, 2H), 6.36-6.08 (m, 1H), 5.19 (s, 2H), 4.75-4.70 (m, 3H), 4.43-4.23 (m, 33H), 4.15-3.95 (m, 17H), 3.93-3.56 (m, 11H), 3.20-2.80 (m, 73H), 2.24-2.05 (m, 8H), 1.92-1.75 (m, 4H), 1.25-1.10 (m, 9H). |
| | **Payload: P3** | ¹⁹F NMR (376 MHz, CD₃CN) δ -110.69 (d, *J* = 13.7 Hz), -119.98 (d, *J* = 289.5 Hz, 1F), - 122.54 (d, *J* = 289.5 Hz, 1F), -151.14 (d, J *=* 46.5 Hz). |
| | **Linker: B40** | |
| | | **MS-ESI [M+2H]²⁺/2:** 1392.1 |
| **C67** | 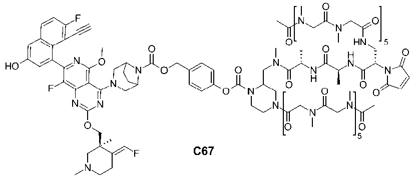 | ¹H NMR (400 MHz, CD₃CN) δ 7.94 (dd, *J =* 9.2, 5.8 Hz, 1H), 7.49-7.36 (m, 4H), 7.36-7.25 (m, 1H), 7.21-7.03 (m, 2H), 7.01-6.66 (m, 3H), 5.19 (s, 2H), 4.75 (s, 2H), 4.58-4.40 (m, 6H), 4.39-4.15 (m, 29H), 4.15-3.91 (m, 19H), 3.90-3.31 (m, 14H), 3.04-2.78 (m, 70H), 2.10-2.03 (m, 4H), 1.93-1.84 (m, 4H), 1.29-1.15 (m, 9H). |
| | **Payload: P4** | ¹⁹F NMR (376 MHz, CD₃CN) δ -107.85(s, 1F), -131.03- -131.09(m, 1F), -147.69- -149.45 (m, 1F). |
| | **Linker: B40** | |
| | | **MS-ESI [M+2H]²⁺/2:** 1382.1 |
| **C68** | 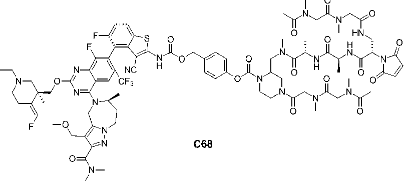 | **MS-ESI [M+2H]²⁺/2:** 1487.6 |
| | **Payload: P8** | |
| | **Linker: B40** | |
| **C69** | 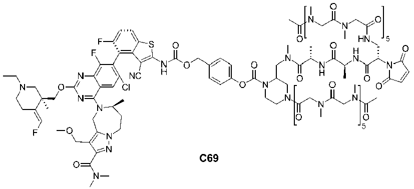 | ¹H NMR (400 MHz, CD₃CN) δ 8.11-7.98 (m, 1H), 7.87 (s, 1H), 7.53-7.42 (m, 2H), 7.41-7.29 (m, 1H), 7.24-7.06 (m, 2H), 7.05-6.52 (m, 3H), 5.28 (s, 2H), 5.23-5.14 (m, 1H), 4.98-4.85 (m, 1H), 4.81-4.64 (m, 3H), 4.59-4.50 (m, 2H), 4.48-4.38 (m, 3H), 4.35-4.19 (m, 27H), 4.16-3.95 (m, 15H), 3.94-3.72 (m, 5H), 3.68-3.49 (m, 4H), 3.48-3.37 (m, 1H), 3.35-3.23 (m, 4H), 3.20-3.07 (m, 10H), 3.03-2.94 (m, 40H), 2.92-2.74 (m, 23H), 2.60-2.39 (m, 2H), 2.38-2.22 (m, 1H), 2.19-2.02 (m, 5H), 1.95-1.76 (m, 2H), 1.61 (d, J = 6.3 Hz, 3H), 1.35-1.10 (m, 14H). |
| | **Payload: P9** | |
| | **Linker: B40** | ¹⁹F NMR (376 MHz, CD₃CN) δ -76.38 (s, 1F) , -118.25 (s, 1F), -122.25 (s, 1F). |
| | | **MS-ESI [M+2H]²⁺/2:** 1470.7 |
| **C70** | 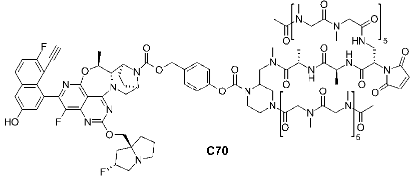 | **MS-ESI [M+2H]²⁺/2:** 1381.1 |
| | **Payload: P10** | |
| | **Linker: B40** | |
| **C71** | 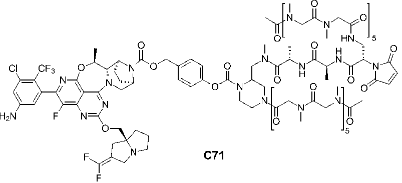 | **MS-ESI [M+H]²⁺/2:** 1400.6 |
| | **Payload: P11** | |
| | **Linker: B40** | |

### Preparation of Compound C72: (2R,7aS)-4-[(4-{4-[2-(N-methyl{[N-methyl({N-methyl[(N-methyl{[N-methyl({N-methyl[(N-methyl{[N-methyl({N-methyl[(N-methylacetamido)methyl]carbonylamino}methyl)carbonylamino]methyl}carbonylamino)methyl]ca rbonylamino } methyl)carbonylamino]methyl} carbonylamino)methyl]carbonylamino } methyl)carbon ylamino]methyl}carbonylamino)acetyl]-2-({N-methyl[(S)-1-[(S)-2-[(S)-3-{[(N-methyl{[N-methyl({N-methyl[(N-methyl{[N-methyl({N-methyl[(N-methyl{[N-methyl({N-methyl[(N-methylacetamido)methyl] carbonylamino }methyl)carbonylamino]methyl}carbonylamino)methyl]ca rbonylamino}methyl)carbonylamino]methyl}carbonylamino}methyl)carbon ylamino]methyl}carbonylamino)methyl]carbonylamino}-2-(2,5-dioxo-3-pyrrolin-1-yl)propanamido]propanamido]ethyl]carbonylamino}methyl)piperazin-1-ylcarbonyloxy}phenyl)methyl]-7a-({(8S)-13-(8-ethynyl-7-fluoro-3-hydroxynaphthalen-1-yl)-14-fluoro-6,10-dioxa-2,12,16,18-tetraazatetracyclo[9.7.1.0^{2,8}.0^{15,19}]nonadeca-1(18),11(19),12,14,16-pentaen-17-yloxy}methyl)-2-fluorohexahydro-1H-pyrrolizin-4-ium formate

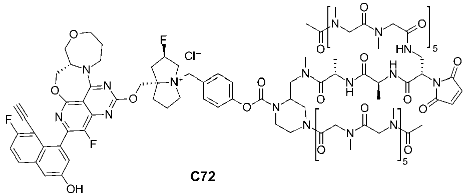

Compound **C72** was prepared following the synthetic method for compound **C73,** using intermediate **A23** instead of **P13** in **Step A.** MS-ESI [M+H]²⁺/2: 1346.6

### Preparation of Compound C73: 1-[(4-{4-[2-(N-methyl{[N-methyl({N-methyl[(N-methyl{[N-methyl({N-methyl[(N-methyl{[N-methyl({N-methyl[(N-methylacetamido)methyl]carbonylamino}methyl)carbonylamino]methyl}carbonylamino)methyl]ca rbonylamino}methyl)carbonylamino]methyl}carbonylamino)methyl]carbonylamino}methyl)carbon ylamino]methyl}carbonylamino)acetyl]-2-({N-methyl[(S)-1-[(S)-2-[(S)-3-{[(N-methyl{[N-methyl({N-methyl[(N-methyl{[N-methyl({N-methyl[(N-methyl{[N-methyl({N-methy][(N-methylacetamido)methyl]carbonylamino}methyl)carbonylamino]methyl}carbonylamino)methyl]ca rbonylamino}methyl)carbonylamino]methyl}carbonylamino}methyl)carbon ylamino]methyl}carbonylamino)methyl]carbonylamino}-2-(2,5-dioxo-3-pyrrolin-1-yl)propanamido]propanamido]ethyl]carbonylamino}methyl)piperazin-1-ylcarbonyloxy}phenyl)methyl]-4-(5-{(7S,13S)-7-[(1S,2S)-2-methylcyclobutamido]-21-ethyl-17,17-dimethyl-8,14-dioxo-15-oxa-4-thia-9,21,27,28-tetraazapentacyclo[17.5.2.1^{2,5}.1^{9,13}.0^{22,26}]octacosa-1(25),2,5(28),19,22(26),23-hexaen-20-yl}-6-[(S)-1-methoxyethyl]pyridin-3-yl)-1-methylpiperazin-1-ium formate

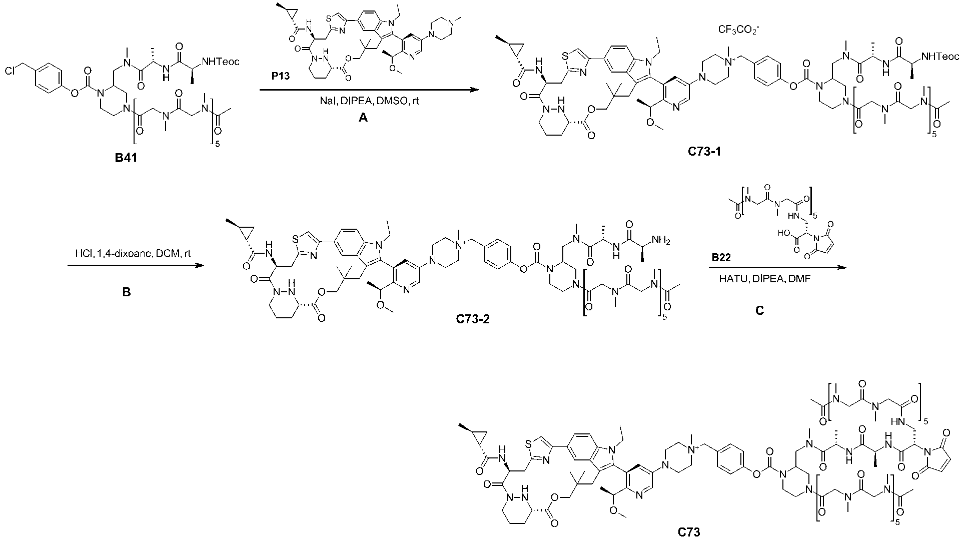

**Step A:** At room temperature, **B41** (350 mg, 0.26 mmol) was dissolved in dimethyl sulfoxide (4 mL). **P13** (212 mg, 0.26 mmol), sodium iodide (39 mg, 0.26 mmol), and *N,N*-diisopropylethylamine (100 mg, 0.78 mmol) were added. The mixture was stirred at room temperature for 18 hours. After the reaction was complete, the reaction solution was purified by reverse phase C18 column (ACN/H₂O(0.1% CF₃O₂H) = 0-45%) to give **C73-1** (560 mg, 75% yield) as a white solid. MS-ESI [M+H]²⁺/2: 1055.8.

**Step B:** At room temperature, **C73-1** (100 mg, 45 µmol) was dissolved in dichloromethane (10 mL), and hydrogen chloride in ethyl acetate solution (4M, 5 mL, 20 mmol) was added. The mixture was stirred at room temperature for 30 minutes. After the reaction was complete, the reaction solution was removed under vacuum. Water was added, and the mixture was lyophilized to give **C73-2** (90 mg, 100% yield) as a white solid. MS-ESI [M+H]²⁺/2: 984.2.

**Step C:** At room temperature, **C73-2** (90 mg, 45 µmol) was dissolved in *N,N-*dimethylformamide (2 mL). **B22** (63 mg, 67 µmol), *N,N,N',N'*-tetramethyl-O-(7-azabenzotriazol-1-yl)uronium hexafluorophosphate (26 mg, 68 µmol), and *N*,*N*-diisopropylethylamine (30 mg, 233 µmol) were added. The mixture was stirred at room temperature for 1 hour. After the reaction was complete, the reaction solution was purified by pre-HPLC (C18, ACN/H₂O (0.1% HCO₂H) = 0-70%) to give **C73** (25 mg, 19% yield) as a white solid. ¹H NMR (400 MHz, DMSO-*d*₆) δ 8.59-8.30 (m, 4H), 7.90-7.50 (m, 5H), 7.40-7.10 (m, 3H), 7.04-6.72 (m, 2H), 5.58-5.48 (m, 1H), 4.82-4.18 (m, 40H), 3.98-2.64 (m, 106H), 2.11-0.27 (m, 34H). MS-ESI [M+H]²⁺/2: 1443.2.

Intermediates **C74-C86** are shown in the table below.

| **No.** | **Structural Formula/Payload/Linker** | **¹H NMR / MS-ESI [M+H]⁺** |
|---|---|---|
| **C74** | 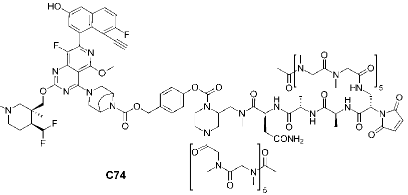 | **MS-ESI [M+2H]²⁺/2:** 1450.2 |
| | **Payload: P3** | |
| **C75** | 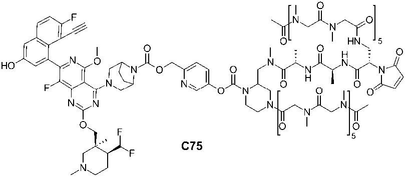 | **MS-ESI [M+2H]²⁺/2:** 1392.6 |
| | **Payload: P3** | |
| **C76** | 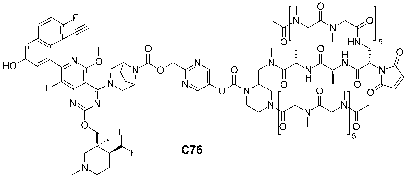 | **MS-ESI [M+2H]²⁺/2:** 1393.1 |
| | **Payload: P3** | |
| **C77** | 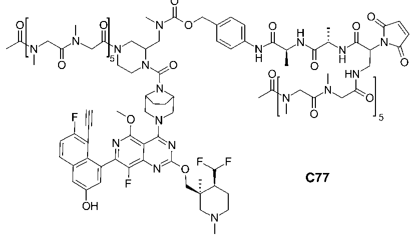 | **MS-ESI [M+2H]²⁺/2:** 1391.7 |
| | **Payload: P3** | |
| **C78** | t 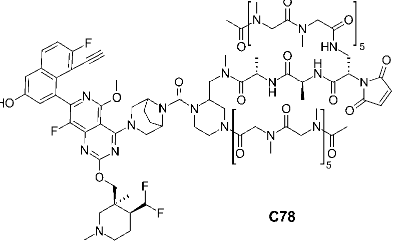 | **MS-ESI [M+2H]²⁺/2:** 1317.1 |
| | **Payload: P3** | |
| **C79** | 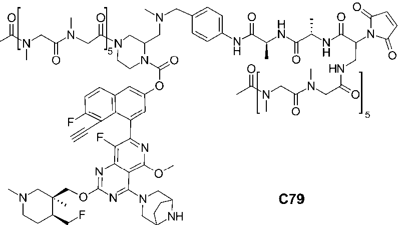 | **MS-ESI [M+2H]²⁺/2:** 1369.7 |
| | **Payload: P3** | |
| **C80** | 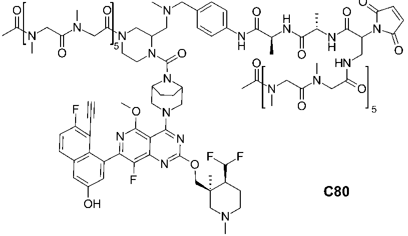 | **MS-ESI [M+2H]²⁺/2:** 1369.7 |
| | **Payload: P3** | |
| **C81** | 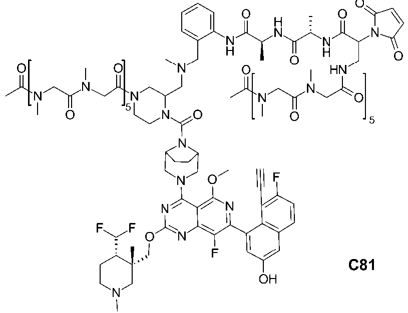 | **MS-ESI [M+2H]²⁺/2:** 1369.7 |
| | **Payload: P3** | |
| **C82** | 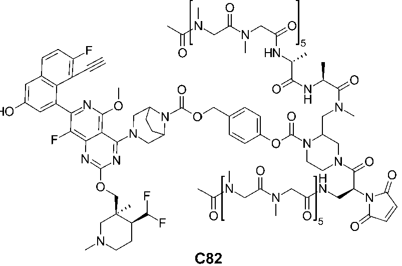 | **MS-ESI [M+2H]²⁺/2:** 1392.1 |
| | **Payload: P3** | |
| **C83** | 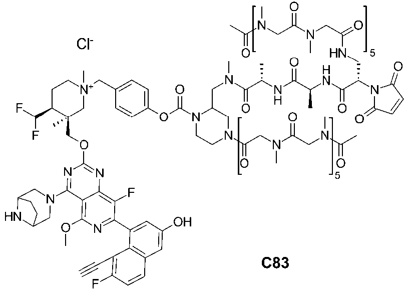 | **MS-ESI [M+H]²⁺/2:** 1370.1 |
| | **Payload: P3** | |
| **C84** | 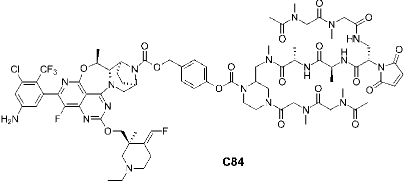 | **MS-ESI [M+2H]²⁺/2:** 1399.7 |
| | **Payload: P14** | |
| **C85** | 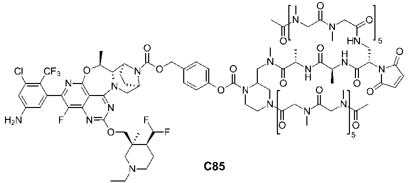 | **MS-ESI [M+2H]²⁺/2:** 1409.6 |
| | **Payload: P15** | |
| **C86** | 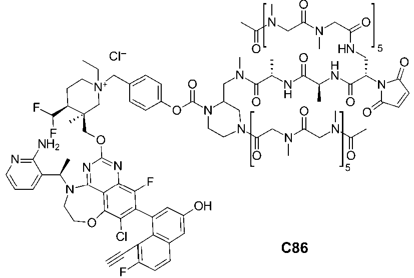 | **MS-ESI [M+H]²⁺/2:** 1412.1 |
| | **Payload: P20** | |

### 6.4 Synthesis of ADCs

### Example 1: Preparation and Characterization of ADC-1

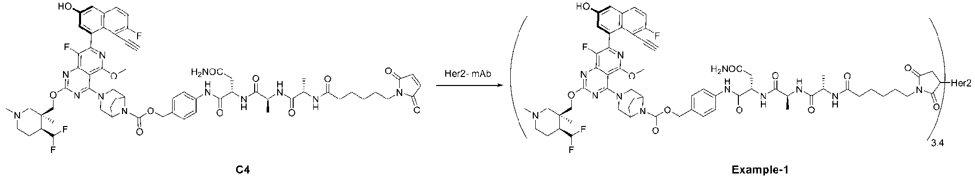

### Step A: Antibody Reduction

At 25 °C, an aqueous solution of tris(2-carboxyethyl)phosphine (Sigma) (1.5 mM, 0.111 mL, 0.167 µmol, 2.5 eq) was added to an anti-HER2 antibody Trastuzumab (CAS No. 180288-69-1, purchased from Shanghai Henlius Biopharmaceutical Co., Ltd.) in PBS buffer solution (pH 7.2, 1.0 mL, 10 mg/mL, 0.067 µmol, 1.0 eq). The mixture was placed at 37 °C in a water bath shaker for 3 hours.

### Step B: Conjugation of KRas Mutant Inhibitor

Compound **C4** (1.01 mg, 0.80 µmol, 12 eq) was dissolved in DMSO (0.05 mL) and added dropwise to the solution from the previous step. The reaction proceeded for 2.5 hours. Desalting and purification were performed using a Thermo Zeba desalting spin column (40 K MWCO) (elution phase: PBS buffer, pH 7.2) to give the antibody conjugate **ADC-1** (8.33 mg/mL, 1.10 mL), which was stored frozen at -78 °C.

### Step C: Concentration Determination of ADC-1

The concentration of the ADC was monitored by UV spectrophotometry (Thermo nanodrop one UV-Vis spectrophotometer). The total absorbance of the ADC solution at a specific wavelength is equal to the sum of the absorbance contributions of the toxin molecule and the monoclonal antibody at that wavelength.

After subtracting the solvent blank, the absorbances of the test sample solution at 280 nm and 258 nm were measured using the Thermo nanodrop one UV-Vis spectrophotometer.

Calculation of results:
(1) ${A}_{280 nm} = \left({C}_{drug}\times {E}_{drug - 280}+{C}_{mab}\times {E}_{mab - 280}\right) \times I$
(2) ${A}_{258 nm} = \left({C}_{drug}\times {E}_{drug - 258}+{C}_{mab}\times {E}_{mab - 258}\right) \times I$
(3) $R = \left({E}_{drug - 258}\right) / \left({E}_{drug - 280}\right)$
In the formula,
A₂₈₀ₙₘ: Average absorbance of the test sample solution at 280 nm wavelength with a path length of 1 cm;
A₂₅₈ₙₘ: Average absorbance of the test sample solution at 258 nm wavelength with a path length of 1 cm;
E_{mab-280}: Mass extinction coefficient of the antibody protein at 280 nm, 1.48 L/(g × cm);
E_{mab-258}: Mass extinction coefficient of the antibody protein at 258 nm, 0.653 L/(g × cm);
R: Ratio of toxin extinction coefficients at 258 nm and 280 nm;
C_{mab}: Protein concentration, mg/mL;
C_{drug}: Toxin concentration, mg/mL;
I: Path length, cm.

Combining equations (1), (2), and (3), the concentration of the antibody conjugate can be calculated from the extinction coefficients and absorbances of the monoclonal antibody and the drug at the two detection wavelengths. ${C}_{mab} = \left({A}_{280 nm}\times R-{A}_{258 nm}\right) / \left({E}_{mab - 280}\times R-{E}_{mab - 258}\right)$

The concentration of antibody conjugate **ADC-1** determined by the above method was 8.33 mg/mL.

### Step D: DAR Value (Drug-to-Antibody Ratio) Determination for ADC-1

**ADC-1** (0.1 mg, 12.0 µL) was dissolved in PBS7.2 buffer (100 µL), DTT (2 µL, 0.25M) was added, and the mixture was placed at 37 °C in a water bath shaker for 1 hour. Then, RP-HPLC chromatography (reverse-phase high-performance liquid chromatography, column: WELCH Ultimate LP-C18, 4.6 * 150 mm * 3.5 um, mobile phase A: 0.05% TFA in water, B: 0.05% TFA in acetonitrile, column temperature: 60 °C, flow rate 1.0 mL/min, UV absorbance monitoring at 280 nm, gradient elution: 0-5.0 min, 20-32% B; 5.0-14.0 min, 32-37% B; 14.0-27.0 min, 37-50% B; 27.0-27.1 min, 50-20% B; 27.1-33.0 min, 20% B) was used. The area ratios of various reduced heavy and light chains of the antibody conjugate at 280 nm were measured, and the DAR of the antibody conjugate **ADC-1** was calculated to be 3.4.

### Step E: Protein Monomer Content Determination for ADC-1

SEC chromatography (Size Exclusion Chromatography, SHIMADZU SHIMSEN Ankylo SEC-300 3 um 7.8 mm × 300 mm) was used, with mobile phase 300 mM NaCl in 50 mM PBS (pH 6.8)/ACN = 90/10, flow rate 0.5 mL/min, column temperature 20 °C, injection volume 20 µL, and run time 30 minutes. The protein monomer content of antibody conjugate **ADC-1** was determined to be 86%.

Examples **ADC-2** to **ADC-94** were prepared following the method of Example **ADC-1,** using the **Intermediate C** listed in the table below instead of intermediate **C4** and the corresponding antibody.

| **Example** | **Structural Formula/Antibody/Intermediate C** | **Concentration (mg/mL)** | **Protein amount (%)** | **DAR** |
|---|---|---|---|---|
| **1** | 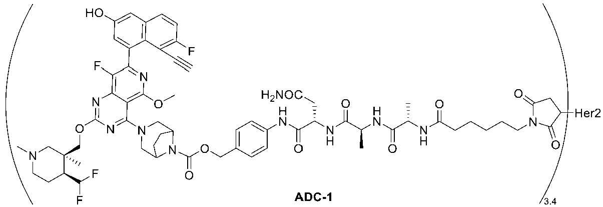 | 8.33 | 86 | 3.4 |
| | **Antibody:Her2** | | | |
| | **Intermediate C: C4 (P3)** | | | |
| **2** | 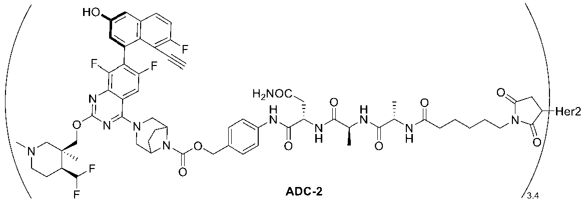 | 5.80 | 83 | 3.4 |
| | **Antibody:Her2** | | | |
| | **Intermediate C: C2 (P1)** | | | |
| **3** | 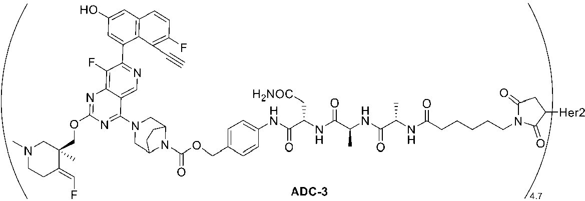 | 5.00 | 86 | 4.7 |
| | **Antibody:Her2** | | | |
| | **Intermediate C: C5 (P2)** | | | |
| **4** | 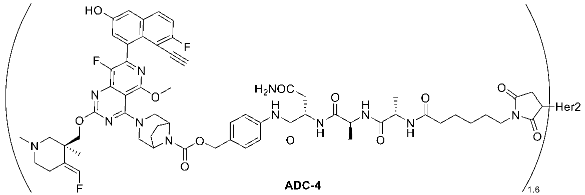 | 4.88 | 92 | 1.6 |
| | **Antibody:Her2** | | | |
| | **Intermediate C: C13 (P4)** | | | |
| **5** | 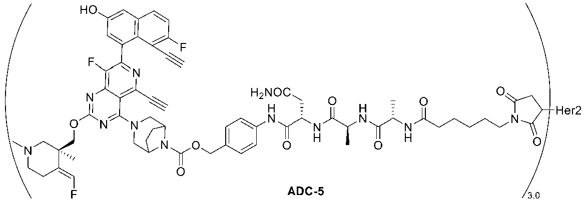 | 4.42 | 96 | 3.0 |
| | **Antibody:Her2** | | | |
| | **Intermediate C: C15 (P5)** | | | |
| **6** | 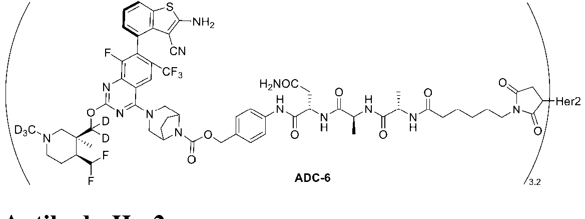 | 5.35 | 96 | 3.2 |
| | **Antibody:Her2** | | | |
| | **Intermediate C: C14 (P6)** | | | |
| **7** | 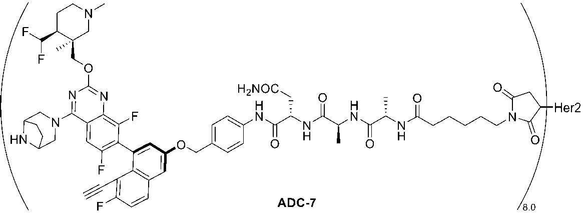 | 6.83 | 96 | 8.0 |
| | **Antibody:Her2** | | | |
| | **Intermediate C: C18 (P1)** | | | |
| **8** | 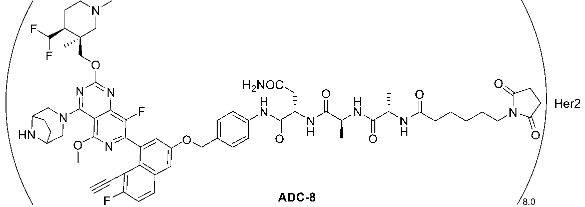 | 7.50 | 94 | 8.0 |
| | **Antibody:Her2** | | | |
| | **Intermediate C: C19 (P3)** | | | |
| **9** | 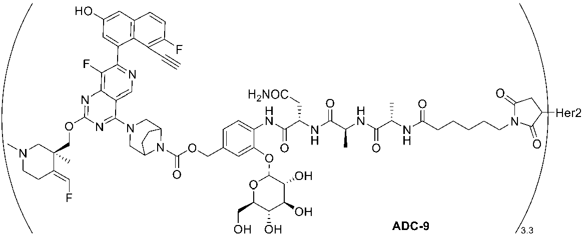 | 5.18 | 85 | 3.3 |
| | **Antibody:Her2** | | | |
| | **Intermediate C: C21 (P2)** | | | |
| **10** | 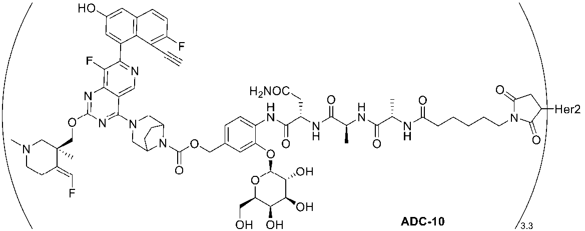 | 5.37 | 77 | 3.3 |
| | **Antibody:Her2** | | | |
| | **Intermediate C: C22 (P2)** | | | |
| **11** | 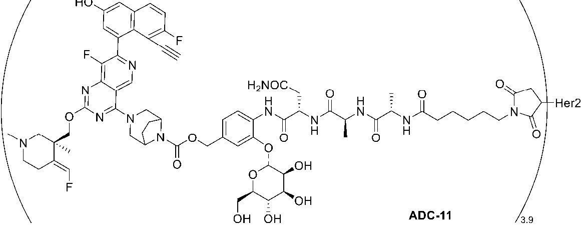 | 4.63 | 95 | 3.9 |
| | **Antibody:Her2** | | | |
| | **Intermediate C: C23 (P2)** | | | |
| **12** | 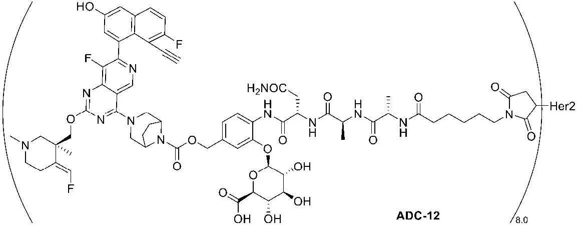 | 5.00 | 98 | 8.0 |
| | **Antibody:Her2** | | | |
| | **Intermediate C: C24 (P2)** | | | |
| **13** | 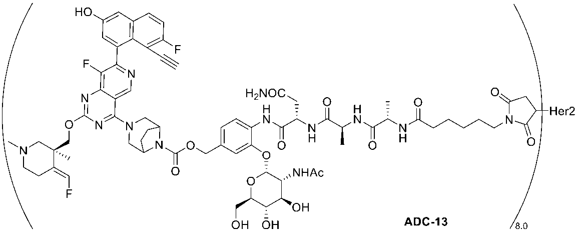 | 5.00 | 85 | 8.0 |
| | **Antibody:Her2** | | | |
| | **Intermediate C: C25 (P2)** | | | |
| **14** | 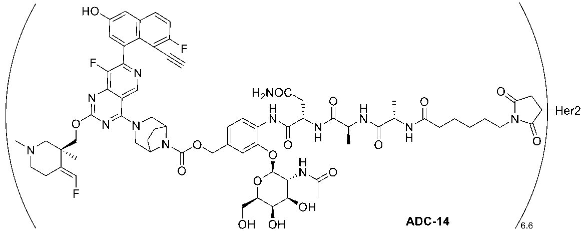 | 6.32 | 92 | 6.6 |
| | **Antibody:Her2** | | | |
| | **Intermediate C: C26 (P2)** | | | |
| **15** | 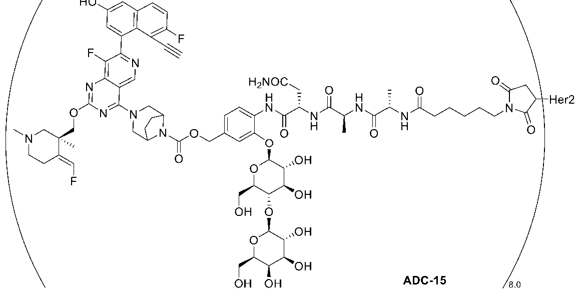 | 4.69 | 84 | 8.0 |
| | **Antibody:Her2** | | | |
| | **Intermediate C: C27 (P2)** | | | |
| **16** | 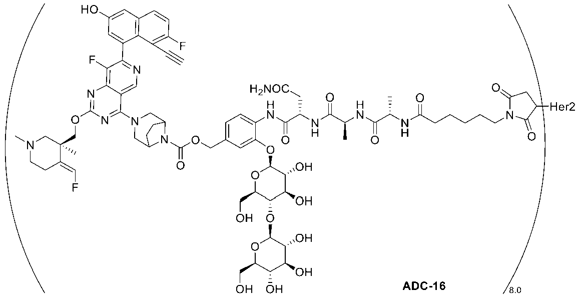 | 5.25 | 94 | 8.0 |
| | **Antibody:Her2** | | | |
| | **Intermediate C: C28 (P2)** | | | |
| **17** | 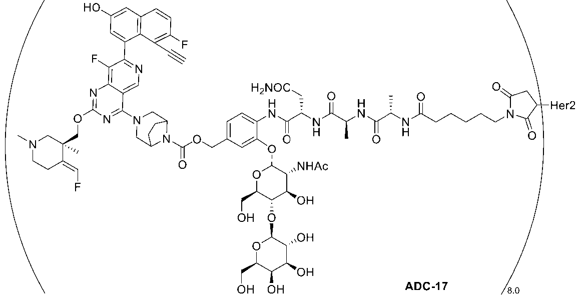 | 7.05 | 95 | 8.0 |
| | **Antibody:Her2** | | | |
| | **Intermediate C: C29 (P2)** | | | |
| **18** | 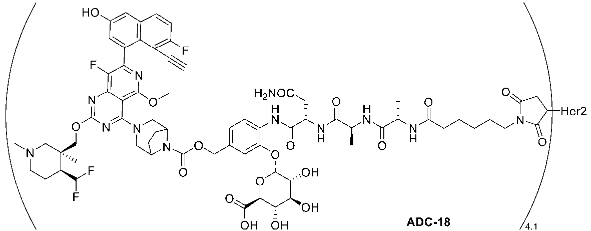 | 12.65 | 98 | 4.1 |
| | **Antibody:Her2** | | | |
| | **Intermediate C: C32 (P3)** | | | |
| **19** | 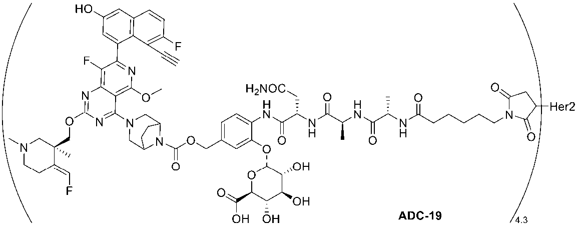 | 9.90 | 96 | 4.3 |
| | **Antibody:Her2** | | | |
| | **Intermediate C: C31 (P4)** | | | |
| **20** | 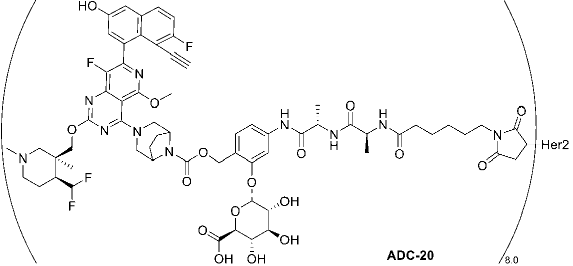 | 7.47 | 97 | 8.0 |
| | **Antibody:Her2** | | | |
| | **Intermediate C: C34 (P3)** | | | |
| **21** | 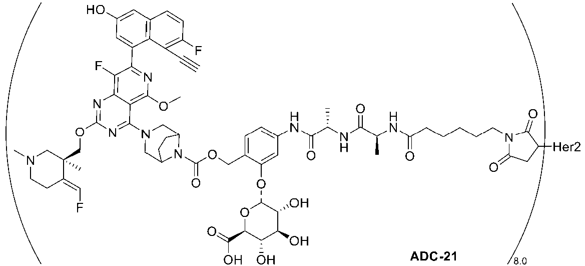 | 7.92 | 95 | 8.0 |
| | **Antibody:Her2** | | | |
| | **Intermediate C: C33 (P4)** | | | |
| **22** | 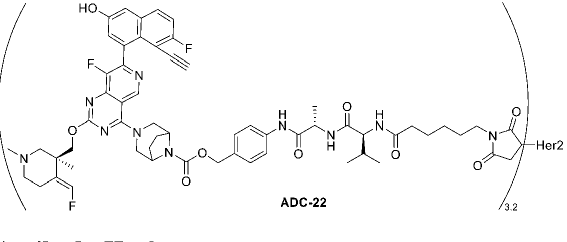 | 5.16 | 60 | 3.2 |
| | **Antibody:Her2** | | | |
| | **Intermediate C: C1 (P2)** | | | |
| **23** | 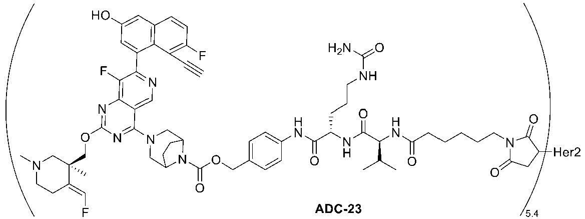 | 4.97 | 92 | 5.4 |
| | **Antibody:Her2** | | | |
| | **Intermediate C: C6 (P2)** | | | |
| **24** | 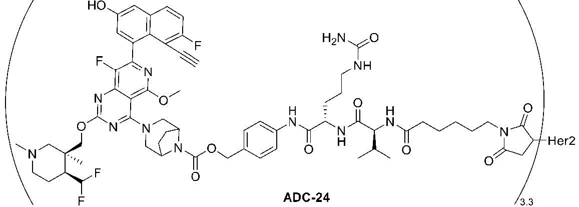 | 7.08 | 60 | 3.3 |
| | **Antibody:Her2** | | | |
| | **Intermediate C: C9 (P3)** | | | |
| **25** | 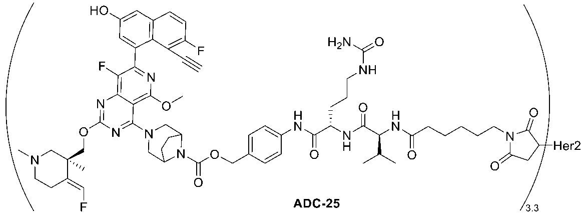 | 5.98 | 60 | 3.3 |
| | **Antibody:Her2** | | | |
| | **Intermediate C: C8 (P4)** | | | |
| **26** | 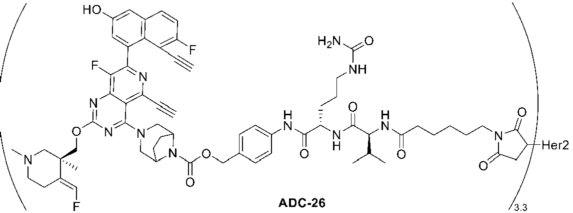 | 5.91 | 73 | 3.3 |
| | **Antibody:Her2** | | | |
| | **Intermediate C: C10 (P5)** | | | |
| **27** | 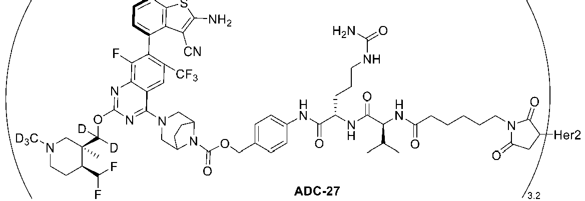 | 6.05 | 84 | 3.2 |
| | **Antibody:Her2** | | | |
| | **Intermediate C: C12 (P6)** | | | |
| **28** | 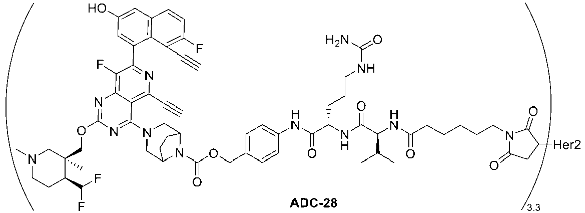 | 6.41 | 66 | 3.3 |
| | **Antibody:Her2** | | | |
| | **Intermediate C: C11 (P7)** | | | |
| **29** | 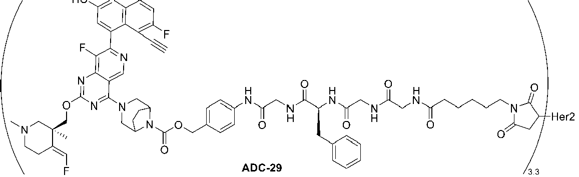 | 5.94 | 81 | 3.3 |
| | **Antibody:Her2** | | | |
| | **Intermediate C: C7 (P2)** | | | |
| **30** | 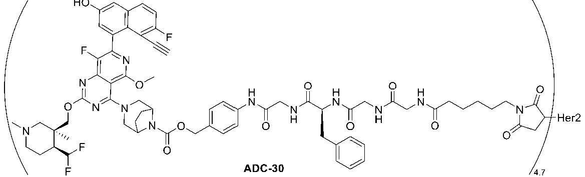 | 5.42 | 84 | 4.7 |
| | **Antibody:Her2** | | | |
| | **Intermediate C: C3 (P3)** | | | |
| **31** | 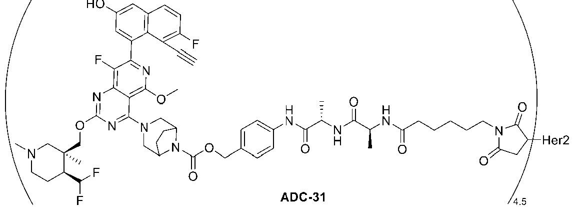 | 7.79 | 97 | 4.5 |
| | **Antibody:Her2** | | | |
| | **Intermediate C: C16 (P3)** | | | |
| **32** | 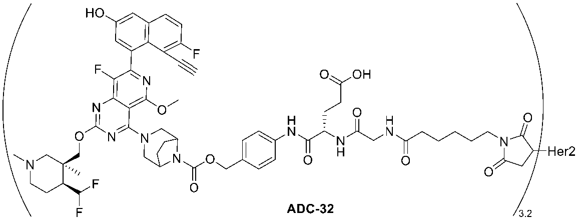 | 7.95 | 98 | 3.2 |
| | **Antibody:Her2** | | | |
| | **Intermediate C: C20 (P3)** | | | |
| **33** | 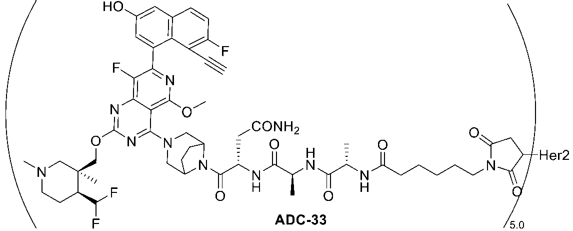 | 8.02 | 97 | 5.0 |
| | **Antibody:Her2** | | | |
| | **Intermediate C: C30 (P3)** | | | |
| **34** | 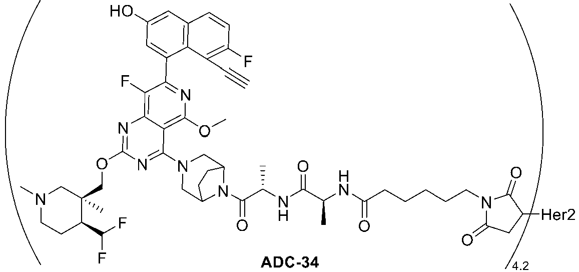 | 5.46 | 96 | 4.2 |
| | **Antibody:Her2** | | | |
| | **Intermediate C: C36 (P3)** | | | |
| **35** | 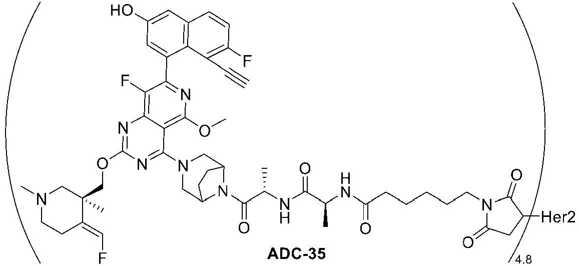 | 7.37 | 96 | 4.8 |
| | **Antibody:Her2** | | | |
| | **Intermediate C: C35 (P4)** | | | |
| **36** | 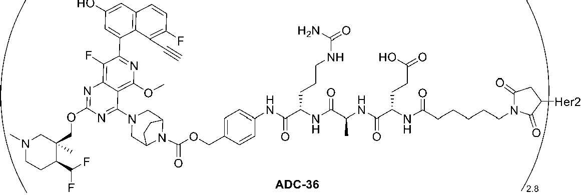 | 5.00 | 80 | 2.8 |
| | **Antibody:Her2** | | | |
| | **Intermediate C: C37 (P3)** | | | |
| **37** | 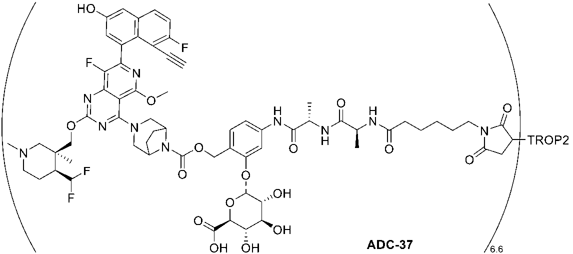 | 6.85 | 95 | 6.6 |
| | **Antibody:TROP2** | | | |
| | **Intermediate C: C34 (P3)** | | | |
| **38** | 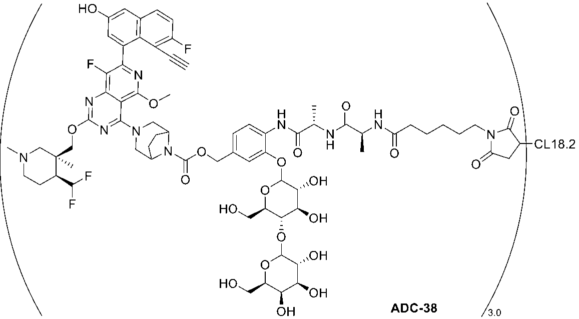 | 1.00 | 99 | 3.0 |
| | **Antibody:CL18.2** | | | |
| | **Intermediate C: C38 (P3)** | | | |
| **39** | 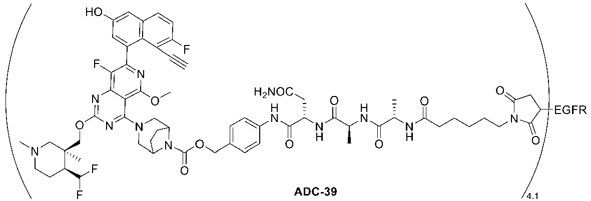 | 4.70 | 98 | 4.1 |
| | **Antibody:EGFR** | | | |
| | **Intermediate C: C4 (P3)** | | | |
| **40** | 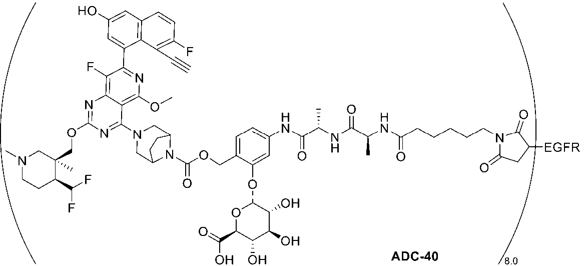 | 4.88 | 99 | 8.0 |
| | **Antibody:EGFR** | | | |
| | **Intermediate C: C34 (P3)** | | | |
| **41** | 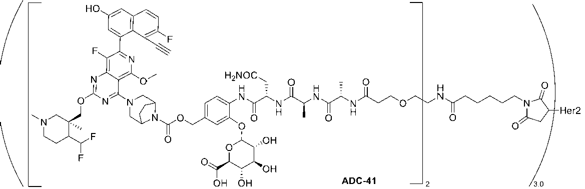 | 8.21 | 69 | 3.0 × 2 |
| | **Antibody:Her2** | | | |
| | **Intermediate C: C39 (P3)** | | | |
| **42** | 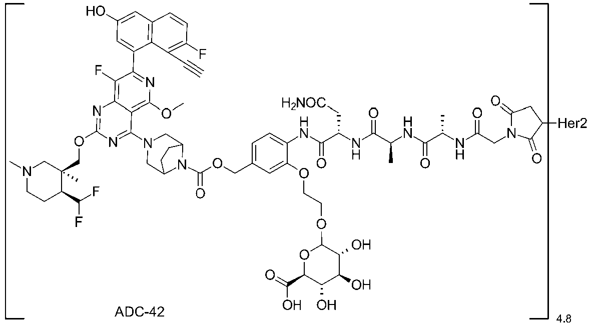 | 5.82 | 96 | 4.8 |
| | **Antibody:Her2** | | | |
| | **Intermediate C: C41 (P3)** | | | |
| **43** | 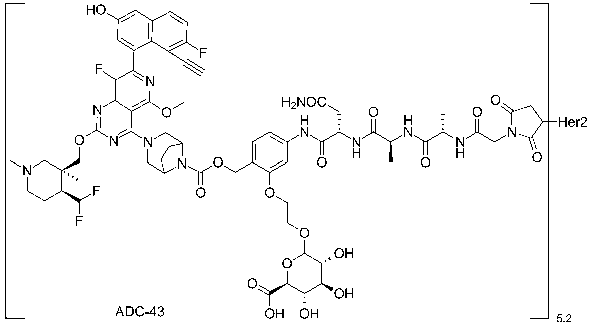 | 5.63 | 98 | 5.2 |
| | **Antibody:Her2** | | | |
| | **Intermediate C: C42 (P3)** | | | |
| **44** | 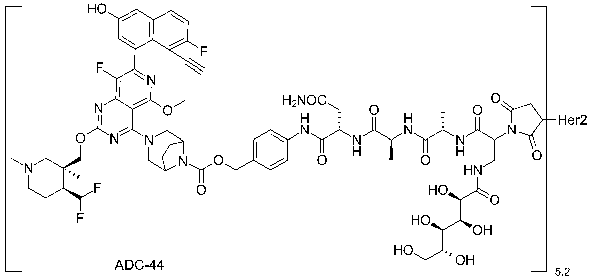 | 5.85 | 71 | 5.2 |
| | **Antibody:Her2** | | | |
| | **Intermediate C: C56 (P3)** | | | |
| **45** | 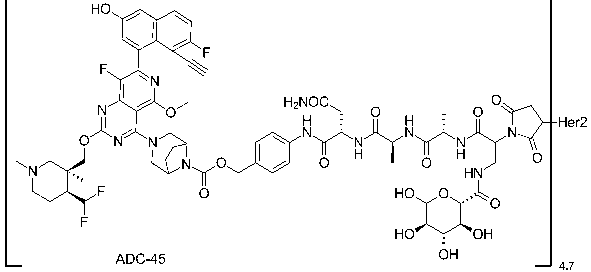 | 5.72 | 89 | 4.7 |
| | **Antibody:Her2** | | | |
| | **Intermediate C: C43 (P3)** | | | |
| **46** | 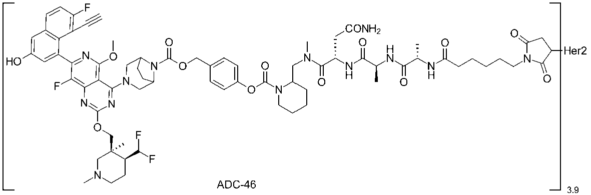 | 5.81 | 98 | 3.9 |
| | **Antibody:Her2** | | | |
| | **Intermediate C: C17 (P3)** | | | |
| **47** | 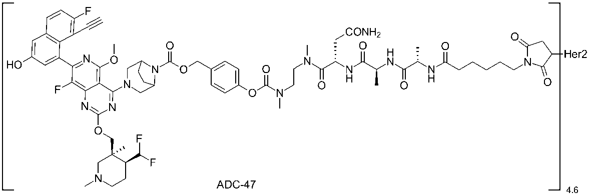 | 5.81 | 95 | 4.6 |
| | **Antibody:Her2** | | | |
| | **Intermediate C: C40 (P3)** | | | |
| **48** | 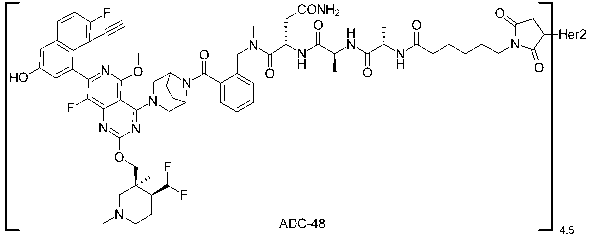 | 5.58 | 99 | 4.5 |
| | **Antibody:Her2** | | | |
| | **Intermediate C: C49 (P3)** | | | |
| **49** | 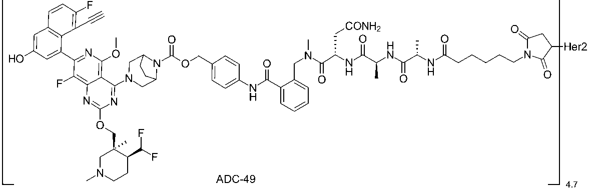 | 5.84 | 95 | 4.7 |
| | **Antibody:Her2** | | | |
| | **Intermediate C: C44 (P3)** | | | |
| **50** | 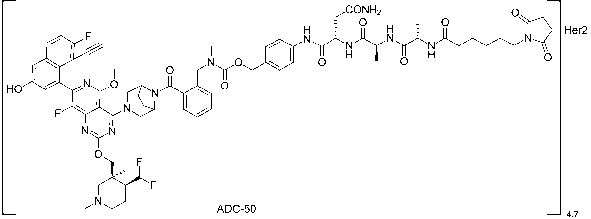 | 5.90 | 99 | 4.7 |
| | **Antibody:Her2** | | | |
| | **Intermediate C: C45 (P3)** | | | |
| **51** | 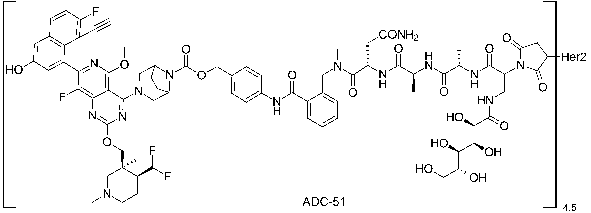 | 5.90 | 74 | 4.5 |
| | **Antibody:Her2** | | | |
| | **Intermediate C: C57 (P3)** | | | |
| **52** | 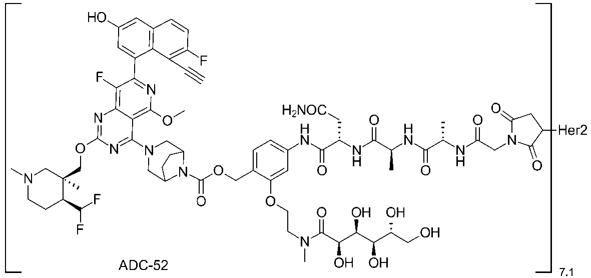 | 4.78 | 96 | 7.1 |
| | **Antibody:Her2** | | | |
| | **Intermediate C: C46 (P3)** | | | |
| **53** | 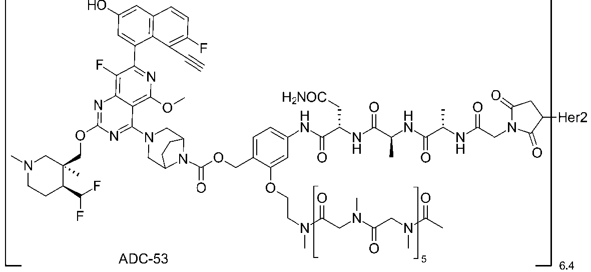 | 5.26 | 92 | 6.4 |
| | **Antibody:Her2** | | | |
| | **Intermediate C: C47 (P3)** | | | |
| **54** | 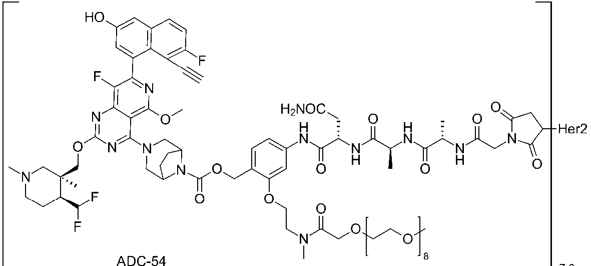 | 5.13 | 90 | 7.6 |
| | **Antibody:Her2** | | | |
| | **Intermediate C: C48 (P3)** | | | |
| **55** | 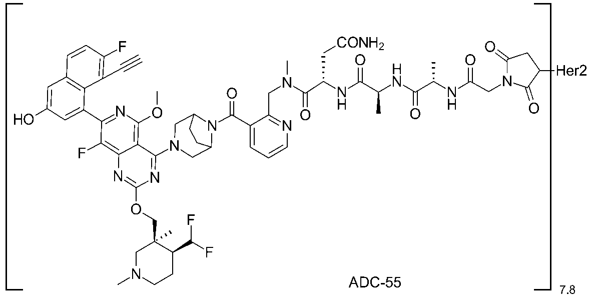 | 4.47 | 90 | 7.8 |
| | **Antibody:Her2** | | | |
| | **Intermediate C: C50 (P3)** | | | |
| **56** | 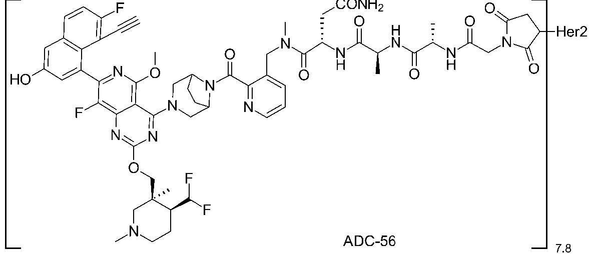 | 4.83 | 95 | 7.8 |
| | **Antibody:Her2** | | | |
| | **Intermediate C: C51 (P3)** | | | |
| **57** | 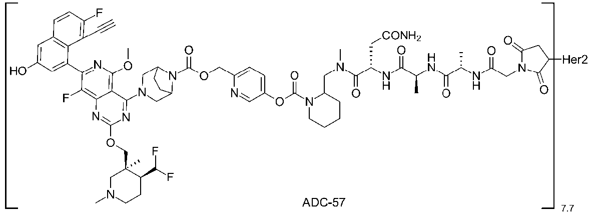 | 4.12 | 96 | 7.7 |
| | **Antibody:Her2** | | | |
| | **Intermediate C: C52 (P3)** | | | |
| **58** | 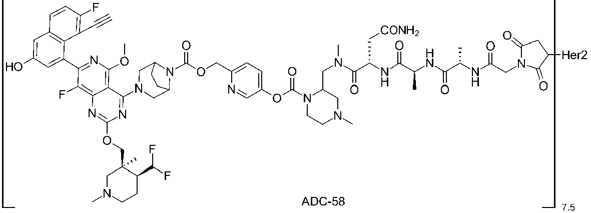 | 4.82 | 96 | 7.5 |
| | **Antibody:Her2** | | | |
| | **Intermediate C: C53 (P3)** | | | |
| **59** | 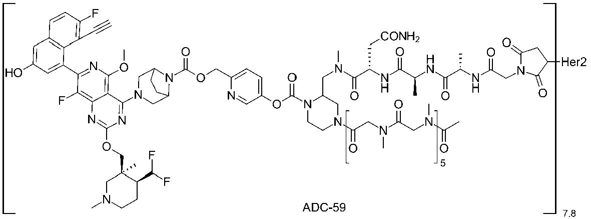 | 4.74 | 90 | 7.8 |
| | **Antibody:Her2** | | | |
| | **Intermediate C: C54 (P3)** | | | |
| **60** | 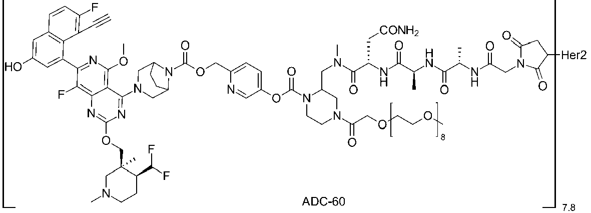 | 4.99 | 90 | 7.8 |
| | **Antibody:Her2** | | | |
| | **Intermediate C: C55 (P3)** | | | |
| **61** | 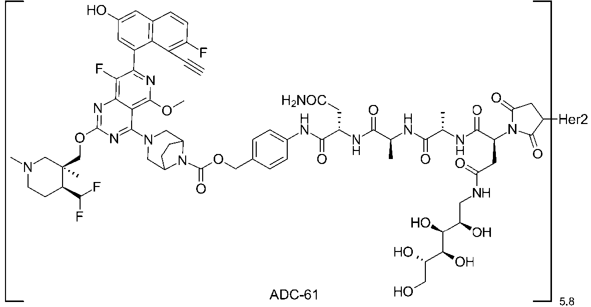 | 4.90 | 87 | 5.8 |
| | **Antibody:Her2** | | | |
| | **Intermediate C: C58 (P3)** | | | |
| **62** | 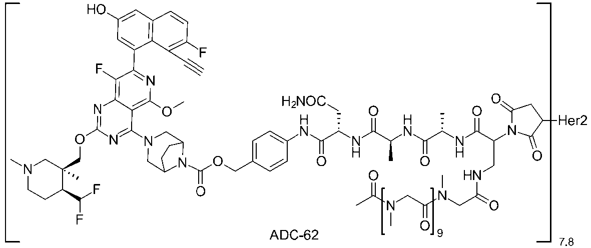 | 5.18 | 92 | 7.8 |
| | **Antibody:Her2** | | | |
| | **Intermediate C: C59 (P3)** | | | |
| **63** | 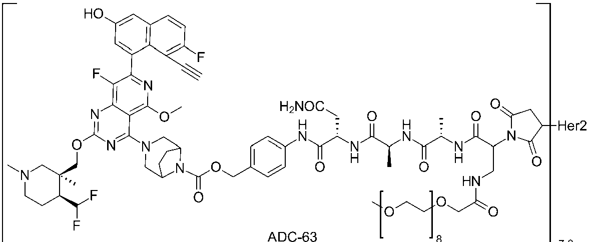 | 4.82 | 98 | 7.8 |
| | **Antibody:Her2** | | | |
| | **Intermediate C: C60 (P3)** | | | |
| **64** | 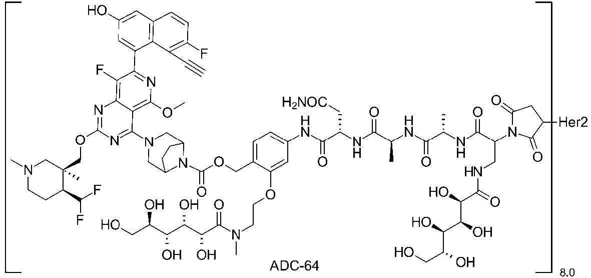 | 5.26 | 79 | 8.0 |
| | **Antibody:Her2** | | | |
| | **Intermediate C: C61 (P3)** | | | |
| **65** | 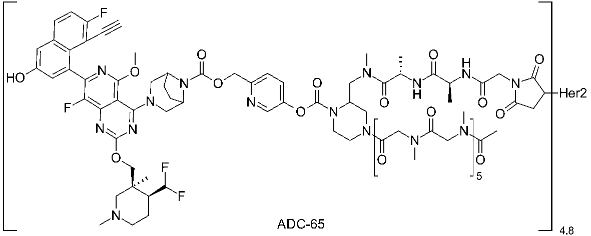 | 6.44 | 98 | 4.8 |
| | **Antibody:Her2** | | | |
| | **Intermediate C: C63 (P3)** | | | |
| **66** | 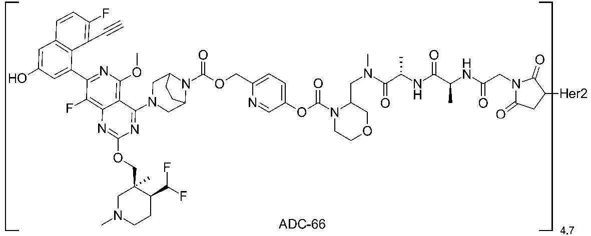 | 6.13 | 98 | 4.7 |
| | **Antibody:Her2** | | | |
| | **Intermediate C: C62 (P3)** | | | |
| **67** | | | | |
| | 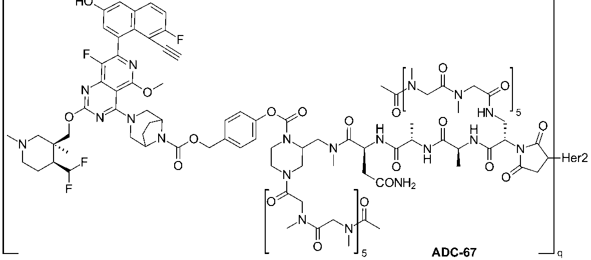 | | | |
| | **Antibody:Her2** | | | |
| | **Intermediate C: C74 (P3)** | | | |
| **68** | 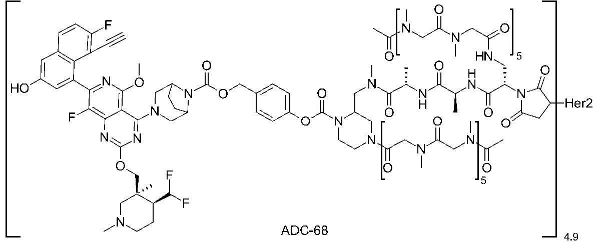 | 7.11 | 99 | 4.9 |
| | **Antibody:Her2** | | | |
| | **Intermediate C: C66 (P3)** | | | |
| **69** | 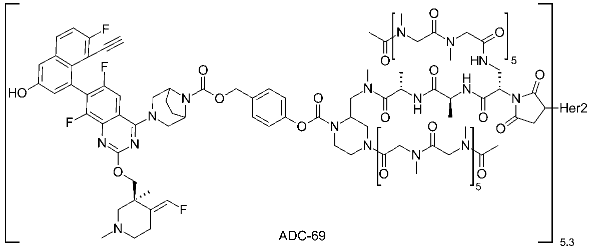 | 6.54 | 99 | 5.3 |
| | **Antibody:Her2** | | | |
| | **Intermediate C: C64 (P1)** | | | |
| **70** | 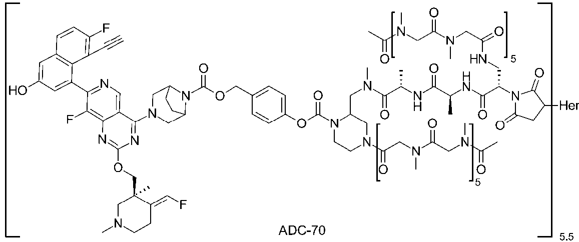 | 6.01 | 99 | 5.5 |
| | **Antibody:Her2** | | | |
| | **Intermediate C: C65 (P2)** | | | |
| **71** | 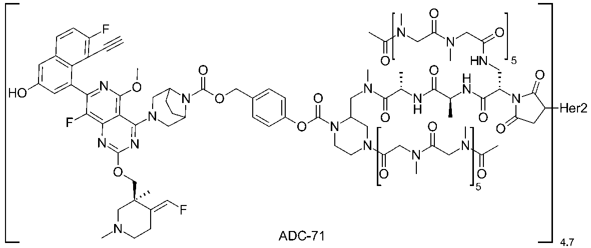 | 6.63 | 99 | 4.7 |
| | **Antibody:Her2** | | | |
| | **Intermediate C: C67 (P4)** | | | |
| **72** | 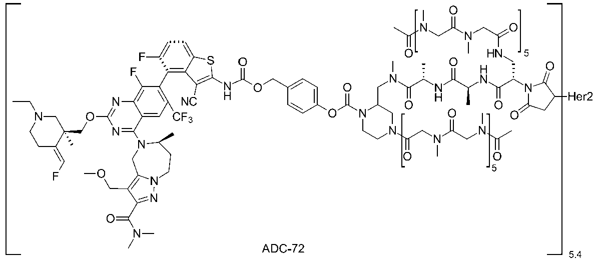 | 6.18 | 99 | 5.4 |
| | **Antibody:Her2** | | | |
| | **Intermediate C: C68 (P8)** | | | |
| **73** | 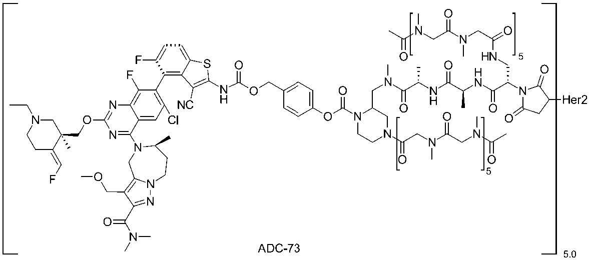 | 6.45 | 99 | 5.0 |
| | **Antibody:Her2** | | | |
| | **Intermediate C: C69 (P9)** | | | |
| **74** | 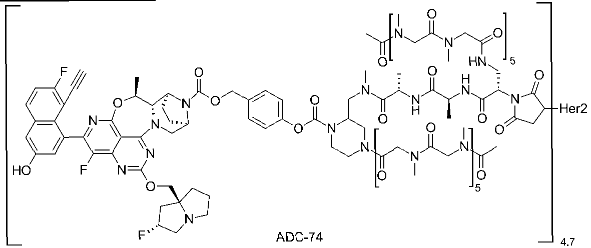 | 5.66 | 99 | 4.7 |
| | **Antibody:Her2** | | | |
| | **Intermediate C: C70 (P10)** | | | |
| **75** | 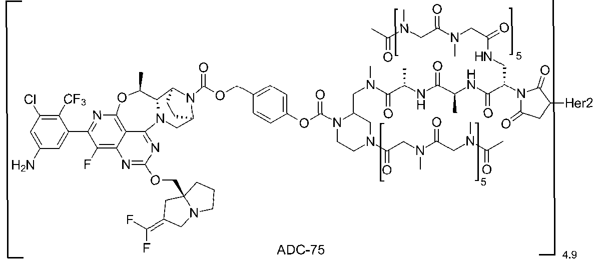 | 5.77 | 99 | 4.9 |
| | **Antibody:Her2** | | | |
| | **Intermediate C: C71 (P11)** | | | |
| **76** | 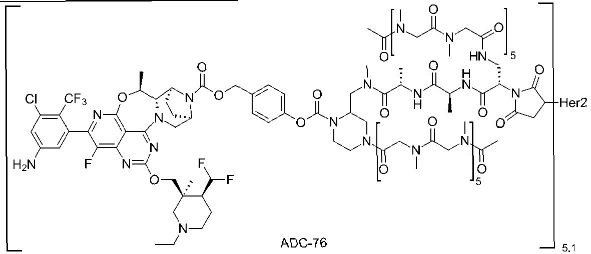 | 6.11 | 99 | 5.1 |
| | **Antibody:Her2** | | | |
| | **Intermediate C: C85 (P15)** | | | |
| **77** | 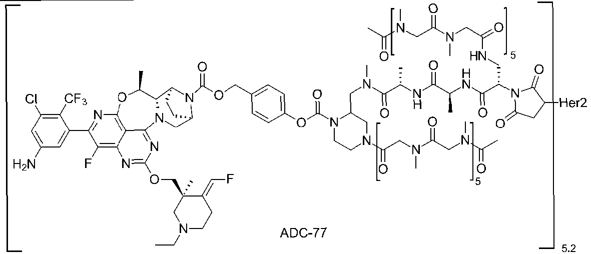 | 5.88 | 99 | 5.2 |
| | **Antibody:Her2** | | | |
| | **Intermediate C: C84 (P14)** | | | |
| **78** | 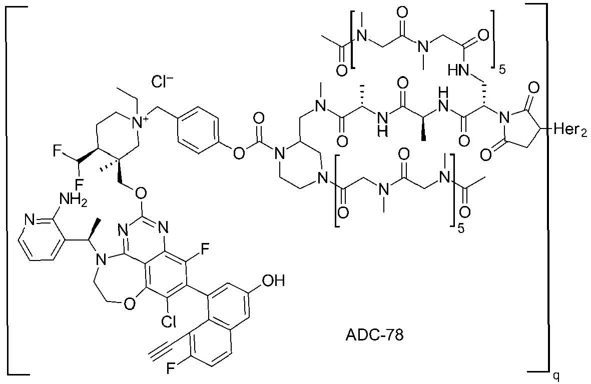 | | | |
| | **Antibody:Her2** | | | |
| | **Intermediate C: C86 (P20)** | | | |
| **79** | 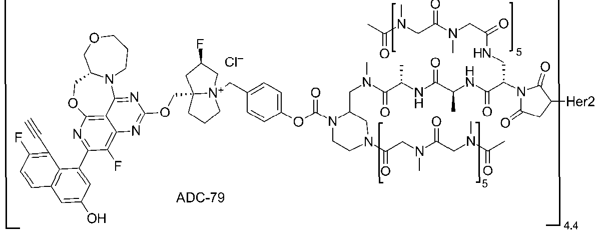 | 5.40 | 99 | 4.4 |
| | **Antibody:Her2** | | | |
| | **Intermediate C: C72 (P12)** | | | |
| **80** | 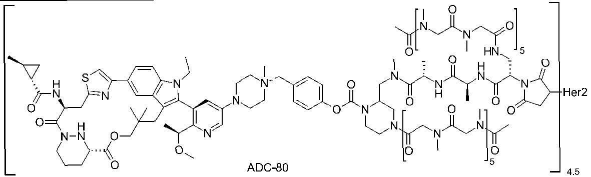 | 6.89 | 99 | 4.5 |
| | **Antibody:Her2** | | | |
| | **Intermediate C: C73 (P13)** | | | |
| **81** | 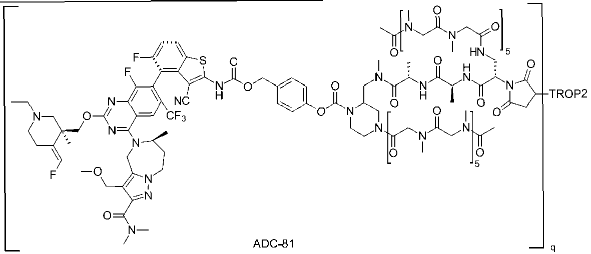 | 7.15 | 97 | |
| | **Antibody:TROP2** | | | |
| | **Intermediate C: C68 (P8)** | | | |
| **82** | 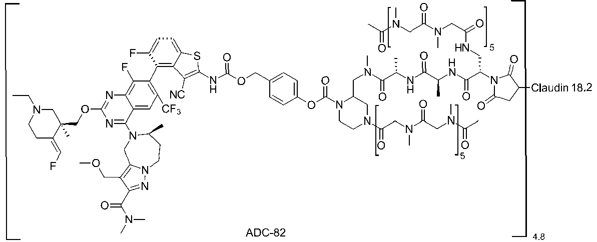 | 2.39 | 99 | 4.8 |
| | **Antibody: Claudin18.2** | | | |
| | **Intermediate C: C68 (P8)** | | | |
| **83** | 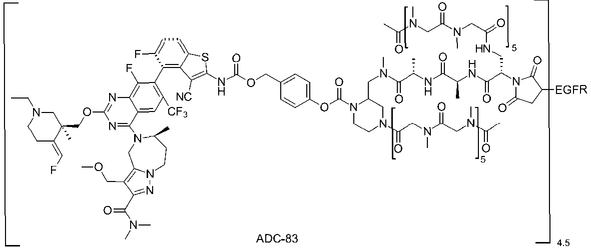 | 4.99 | 98 | 4.5 |
| | **Antibody:EGFR** | | | |
| | **Intermediate C: C68 (P8)** | | | |
| **84** | 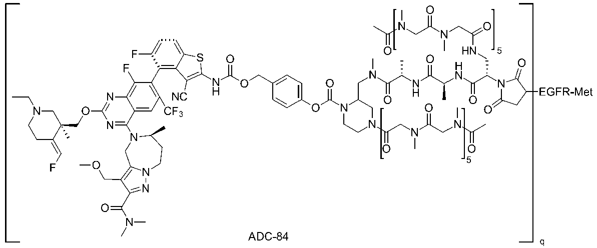 | | | |
| | **Antibody:EGFR-Met** | | | |
| | **Intermediate C: C68 (P8)** | | | |
| **85** | 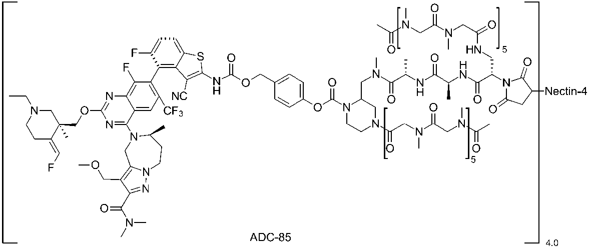 | 6.58 | 99 | 4.0 |
| | **Antibody:Nectin-4** | | | |
| | **Intermediate C: C68 (P8)** | | | |
| **86** | 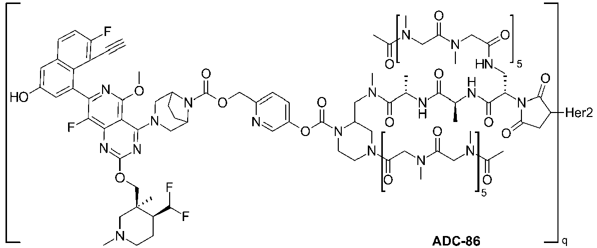 | | | |
| | **Antibody:Her2** | | | |
| | **Intermediate C: C75 (P3)** | | | |
| **87** | 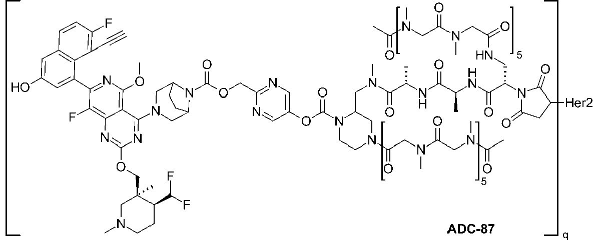 | | | |
| | **Antibody:Her2** | | | |
| | **Intermediate C: C76 (P3)** | | | |
| **88** | 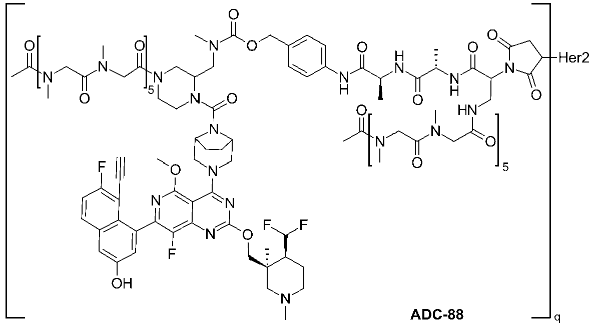 | | | |
| | **Antibody:Her2** | | | |
| | **Intermediate C: C77 (P3)** | | | |
| **89** | 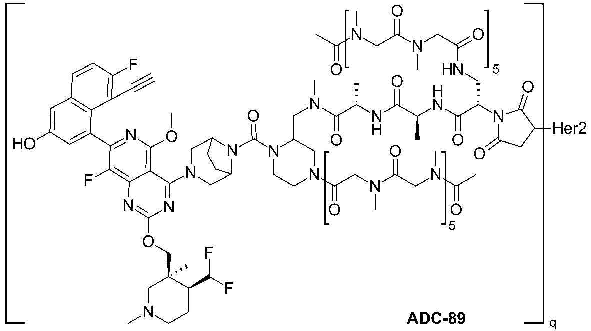 | | | |
| | **Antibody:Her2** | | | |
| | **Intermediate C: C78 (P3)** | | | |
| **90** | 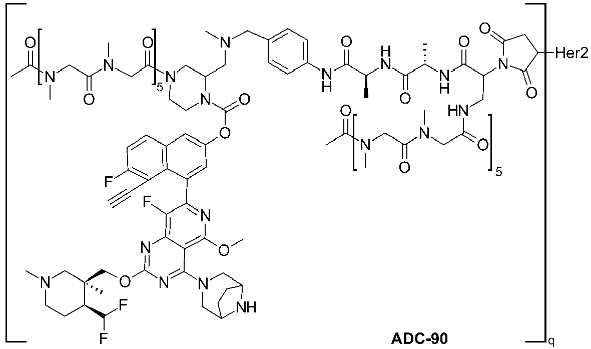 | | | |
| | **Antibody:Her2** | | | |
| | **Intermediate C: C79 (P3)** | | | |
| **91** | 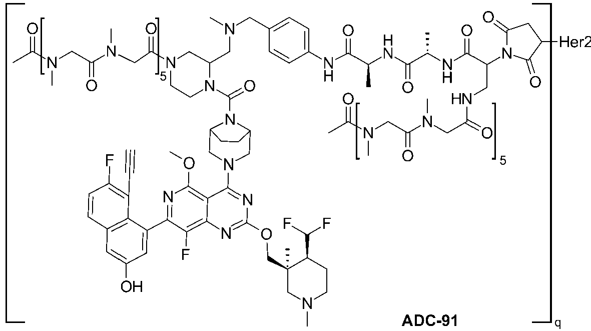 | | | |
| | **Antibody:Her2** | | | |
| | **Intermediate C: C80 (P3)** | | | |
| **92** | 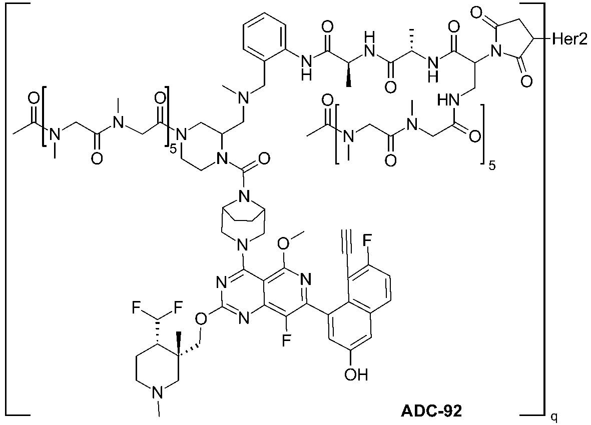 | | | |
| | **Antibody:Her2** | | | |
| | **Intermediate C: C81 (P3)** | | | |
| **93** | 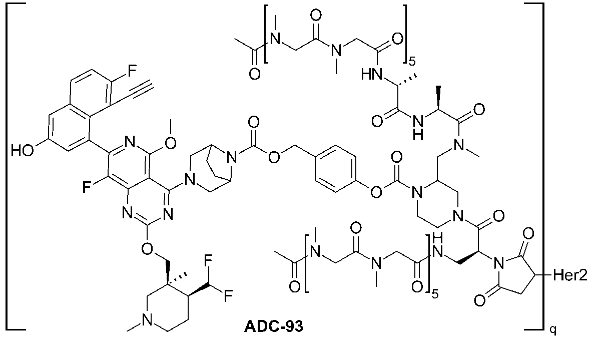 | | | |
| | **Antibody:Her2** | | | |
| | **Intermediate C: C82 (P3)** | | | |
| **94** | 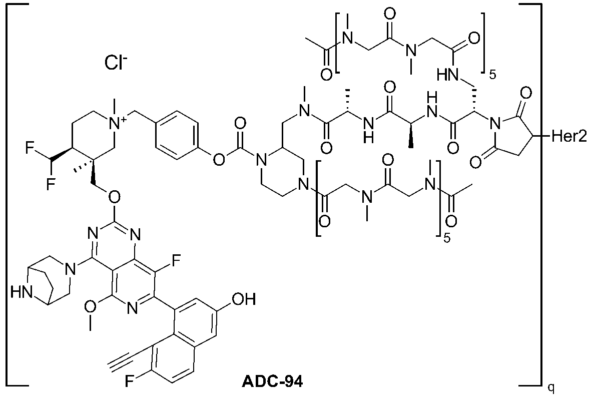 | | | |
| | **Antibody:Her2** | | | |
| | **Intermediate C: C83 (P3)** | | | |
| Wherein, | | | | |
| the antibody denoted as Her2 is specifically Trastuzumab; | | | | |
| the antibody denoted as CL18.2 is specifically Zolbetuximab; | | | | |
| the antibody denoted as TROP2 is specifically Sacituzumab; | | | | |
| the antibody denoted as EGFR is specifically Cetuximab; | | | | |
| the antibody denoted as EGFR-Met is specifically Amivantamab; | | | | |
| the antibody denoted as Nectin-4 is specifically Enfortumab. | | | | |

The data in the above table indicates:
1) Ras mutant inhibitors can be used to synthesize ADCs via the amine or hydroxyl groups in the molecule and are compatible with various antibodies, peptides, self-immolative linkers, and solubilizing sugar units.
2) The amine group (Examples 7 and 8) and sugar units (Examples 12, 13, 15, 17, 20, 21, etc.) in the drug payload can significantly improve the solubility of intermediates and ADCs, increasing the DAR value to the maximum of 8.0.
3) Solubilizing sugar units can improve conjugation efficiency. For example, for antibodies with weaker hydrophilicity such as TROP2 or CL18.2, the conjugation reaction occurs more readily when solubilizing sugar units are introduced, improving conjugation efficiency.
4) Sugar units can also promote the conjugation reaction where one conjugation site links two payloads (Example 44), multiplying the DAR value.
5) Solubilizing units in the drug payload can significantly improve the solubility of the ADC, increase the DAR value, and enhance biological activity.

### VII: Activity Examples

### Activity Example 1: Inhibitory Effect of Representative Drug P Unit Compounds of ADC of the Present Disclosure on Proliferation of KRAS G12D Mutant AGS Cells

AGS cells (adherent) (Nanjing Kebai Biotechnology Co., Ltd., Cat# CBP60476) were cultured at 37 °C, 5% CO₂, 95% humidity (3000/well, RPMI-1640 + 10% FBS (GIBCO, Cat#10099-141)).

Cells in the logarithmic growth phase were harvested and counted using a platelet counter. Cell viability was assessed by trypan blue exclusion, ensuring >90% viability. The cell concentration was adjusted; 90 µL of cell suspension was added to each well of a 96-well plate (Corning^{®}, Cat# 3603). The cells in the 96-well plate were cultured at 37 °C, 5% CO₂ (Thermo Scientific, Model 3100 Series).

Drug dilution and addition: a) Single-point inhibition rate assay: 10x drug solutions were prepared, and 10 µL drug solution was added each well of 96-well plate inoculated with cells to achieve a working concentration of 1 µM, with three replicate wells per drug concentration; b) Preparation of drug for IC₅₀ assay: 10x drug solutions were prepared, and 10 µL drug solution was added each well of 96-well plate inoculated with cells to achieve a top working concentration of 10 µM with 3.16-fold serial dilution (9 concentrations), three replicate wells per drug concentration. The cells in the drug-treated 96-well plate were cultured at 37 °C under 5% CO₂ for 3 days, followed by CTG assay (CellTiter-Glo^{®} Luminescent Cell Viability Assay, Promega, Cat# G7572).

CTG reagent was thawed, and cell plates were equilibrated to room temperature for 30 minutes. An equal volume of CTG solution was added to each well, and the plates were shaken on an orbital shaker for 5 minutes to lyse the cells. The plates were placed at room temperature for 20 minutes to stabilize the luminescent signal, and luminescence was read (SpectraMax Multi-Mode Microplate Reader, MD, 2104-0010A). Data were analyzed using GraphPad Prism 7.0 software, and dose-response curves were fitted by nonlinear S-curve regression to calculate IC50 values. 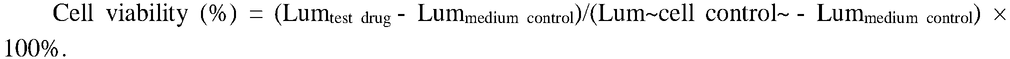 Cell viability (%) = (Lumtest drug - Lummedium control)/(Lum~cell control~ - Lummedium control) × 100%.

Representative drug P unit compounds of the present disclosure showed satisfactory anti-proliferative activity against KRAS G12D mutant AGS human gastric adenocarcinoma cells. Experimental data are shown in the table below.

| Drug P Unit Compound | IC₅₀ (µM) |
|---|---|
| P1 | 0.007 |
| P2 | 0.004 |
| P3 | 0.002 |
| P4 | 0.002 |
| P5 | 0.001 |

### Activity Example 2: Mouse Pharmacokinetic Properties of Representative Drug P Unit Compounds of ADC of the Present Disclosure

Male CD-1 mice, age: 6-8 weeks, were purchased from Zhejiang Vital River Laboratory Animal Technology Co., Ltd.

IV administration group: Compounds were formulated in 20% Captisol (sulfobutyl ether β-cyclodextrin, Wuhan Fengyao Tonghui Chemical Products Co., Ltd., Cas#182410-00-0) in sodium acetate buffer (10 mM sodium acetate solution, adjusted to pH 4.0 with acetic acid) (sodium acetate (Aladdin), MC (Sigma, 9004-67-5)) to a final concentration of 0.6 mg/mL. The drug formulation was injected into CD-1 mice by tail vein injection at an injection volume of 5 mL/kg. Blood samples were collected from the submandibular vein or other suitable sites at 5 minutes, 15 minutes, 30 minutes, 1 hour, 2 hours, 4 hours, 6 hours, 8 hours, and 24 hours post-dose. Blood samples were centrifuged at low temperature for 6 minutes, and the plasma was collected and stored at -80 °C until analysis. For the PO administration group, compounds were formulated in 0.5% Tween 80 + 99.5% (0.5% MC (400 cp)) or 5% DMSO + 95% (0.5% MC (400 cp)) to achieve a final concentration of 1 mg/mL for each compound. The drug formulations were administered to CD-1 mice by oral gavage at a dosing volume of 10 mL/kg. Blood samples were collected from the submandibular vein or other suitable routes at 15 min, 30 min, 1 h, 2 h, 4 h, 6 h, 8 h, and 24 h post-dose. The blood samples were centrifuged at low temperature for 6 minutes, and the plasma was collected and stored at -80 °C until analysis.

10 µL of plasma sample was taken, 200 µL of methanol containing internal standard was added as precipitant to precipitate plasma proteins, and test compounds in plasma were extracted, centrifuged at low temperature, and 10 µL of the supernatant was injected to analyze the blood drug concentration of the drug using LC-MS/MS. Pharmacokinetic parameters were calculated from blood drug concentration -time data using Winnonlin.

Experimental results showed that representative drug P unit compounds of the present disclosure exhibited favorable pharmacokinetic properties in mouse pharmacokinetic evaluation.

| Example | Route of administration | T_{1/2} (hr) | AUC₀₋ₜ (ng.hr/mL) | AUC_{0-∞} (ng.hr/mL) | Formula |
|---|---|---|---|---|---|
| P1 | PO | 10.75 | 384.10 | 413.82 | 5%DMSO + 95% (0.5%MC(400cp)) |
| P2 | PO | 8.15 | 652.58 | 703.82 | 5%DMSO + 95% (0.5%MC(400cp)) |

### Activity Example 3: In Vivo Efficacy Study of Representative Drug P Unit Compounds of ADC of the Present Disclosure in a Human Pancreatic Cancer HPAC Subcutaneous Xenograft NOD/SCID Mouse Model

6-8 week old female NOD/SCID mice (Jiangsu Jicui Yaokang Biotechnology Co., Ltd.) were subcutaneously inoculated on the right posterior back with 1×10⁷ HPAC cells (carrying KRas G12D mutant protein, ATCC) in 0.1 mL volume. When tumors grew to an average volume of 150 mm³, mice were randomized into groups based on tumor size and mouse body weight. The day of dosing was considered Day 0. Test compounds were administered once daily via tail vein injection or oral gavage, 5 mice per group, at a dose of 50 mg/kg. The control group was administered the same volume of vehicle control via tail vein injection or oral gavage.

Body weight and tumor size were measured twice weekly during the experiment. The formula for tumor volume was calculated as: Tumor volume (mm³) = 0.5 × (tumor long diameter × tumor short diameter²). Mean tumor growth inhibition TGI% = [(mean tumor volume in control group - mean initial tumor volume in control group) - (mean tumor volume in treatment group - mean initial tumor volume in treatment group)] / (mean tumor volume in control group - mean initial tumor volume in control group).

Experimental results showed that representative drug P unit compounds of the present disclosure demonstrated target-related excellent tumor inhibitory activity in the human pancreatic cancer HPAC model. Meanwhile, there were no significant changes in the body weights of mice in each group.

| Group | Administered dose | Mode and frequency of administration | Tumor Volume (mm³) (Day 20) | TGI (%) |
|---|---|---|---|---|
| Vehicle Control * | / | PO, BID × 21 | 771 | / |
| P1 | 50 mpk | PO, BID × 21 | 135 | 102 |
| P2 | 50 mpk | PO, BID × 21 | 77 | 112 |
| P3 | 50 mpk | PO, BID × 21 | 117 | 105 |
| P4 | 50 mpk | PO, BID × 21 | 113 | 106 |

| | | | | |
|---|---|---|---|---|
| * The vehicle administered by gavage was 5% DMSO + 95% (0.5% MC (400 cp)) (sodium acetate (aladdin) MC sigma, 9004-67-5). | | | | |

### Activity Example 4: Anti-tumor Effect of Representative Drug P Unit Compounds of ADC of the Present Disclosure in a KRAS-G12D Mutant Human Colon Cancer GP2D Subcutaneous Xenograft BALB/c Nude Mouse Model

GP2D cells (Nanjing Kebai Biotechnology Co., Ltd.) were cultured in culture medium containing DMEM and 10% FBS, and cells in logarithmic growth phase were collected and resuspended in PBS to an appropriate concentration for subcutaneous tumor inoculation in BALB/c female nude mice (nude, purchased from Jiangsu Ji Yao Kang Biotechnology Co., Ltd.). Experimental mice were subcutaneously inoculated on the right posterior back with GP2D cells at a density of 1 × 10⁷ cells per mouse, with the cells resuspended in a 1:1 PBS and Matrigel (inoculation volume: 0.1 mL). When average tumor volume reached ~150 mm³, mice were randomized based on tumor size and body weight. Dosing began immediately after grouping; the day of dosing was considered Day 0. Compounds were administered twice daily by oral gavage at a dose of 10 mg/kg or vehicle control (0.5% Tween 80 + 99.5% (0.5% MC (400cp))). Body weight and tumor size were measured twice weekly during the experiment. 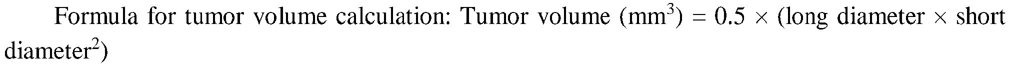 Formula for tumor volume calculation: Tumor volume (mm3) = 0.5 × (long diameter × short diameter2) 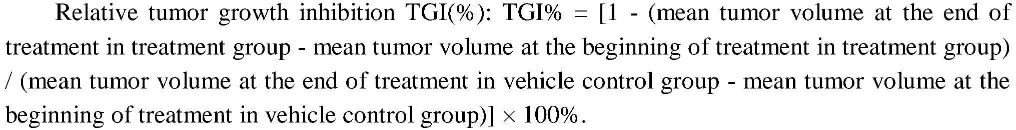 Relative tumor growth inhibition TGI(%): TGI% = [1 - (mean tumor volume at the end of treatment in treatment group - mean tumor volume at the beginning of treatment in treatment group) / (mean tumor volume at the end of treatment in vehicle control group - mean tumor volume at the beginning of treatment in vehicle control group)] × 100%.

| Group | Tumor Volume (mm³) (Day 21) | TGI (%) |
|---|---|---|
| Vehicle Control * | 1026 | / |
| P6 | 70 | 109 |
| P5 | 419 | 70 |

Experimental results showed that representative drug P unit compounds of the present disclosure demonstrated target-related excellent tumor inhibitory activity in the human colon cancer GP2D model. Meanwhile, body weights of mice in all groups showed no significant changes.

### Activity Example 5: Inhibitory Effect of Representative Drug P Unit Compounds of ADC of the Present Disclosure on Proliferation of 5 KRAS G12D Mutant Tumor Cell Lines

The following 5 cell lines were purchased from Nanjing Kebai Biotechnology Co., Ltd.:

| **KRAS** | **Cell lines** | **Item No.** | **Cell type** | **Complete medium** |
|---|---|---|---|---|
| G12D | GP2D | CBP60010 | Adherent | DMEM (Gibco, 11965092) + 10% FBS (Gibco, 10091-148) |
| G12D | HPAC | CBP60539 | Adherent | HPAC Complete Medium (Cobioer, CBP60539M) |
| G12D | AsPC-1 | CBP60546 | Adherent | RPMI-1640 (Gibco, SH30809.01) + 10% FBS |
| G12D | KP-4 | CBP60541 | Adherent | RPMI-1640 + 10% FBS |
| G12D | Panc 04.03 | CBP60683 | Adherent | RPMI-1640 + 15% FBS + 10 ug/ml Insulin (Gibco, 41400045) |

A sterile 1% methylcellulose 3D culture medium was prepared in advance. Cells in the logarithmic growth phase were harvested, and cell counting and viability assessment were performed using a Countstar automatic cell counter based on the classical trypan blue staining principle, ensuring that cell viability was above 90%. Cell concentrations for GP2D, HPAC, AsPC-1, KP-4, and Panc 04.03 were adjusted to a final methylcellulose (Sigma, 9004-67-5) concentration of 0.65%, mixed, and allowed to stand until no visible air bubbles. 80 µL of cell suspension (1000-2000 cells) was added to a 96-well plate (Corning, 3603). The cells in the 96-well plate were cultured overnight at 37 °C, 5% CO₂, and 95% humidity (Thermo Scientific, Model 371 Series).

Preparation of drug for IC₅₀ assay: 10 mM DMSO stock solutions of each compound were prepared. First dilution in DMSO to 5 mM, then 3-fold serial dilution to 9 concentrations to prepare DMSO solutions of different concentrations for each compound. Second 1:100 dilution (phosphate-buffered saline DPBS, Corning, 21-031-CVC) was used to prepare a 5x dilution of each compound, using complete medium as solvent. Finally, 20 µL of each compound dilution was added per well to the cell-seeded plates, with a final top concentration of 10 µM, 9 concentrations, 3-fold dilution, two replicate wells per drug concentration.

The treated plates were incubated at 37 °C, 5% CO₂ for 120 hours, followed by CTG analysis (CellTiter-Glo^{®} Luminescent Cell Viability Assay, Promega, Cat# G7573). CTG reagent was thawed, and cell plates were equilibrated to room temperature for 30 minutes. 75 µL of CTG solution was added per well. Plates were shaken on an orbital shaker for 5 minutes to lyse cells, then placed at room temperature for 20 minutes to stabilize the luminescent signal. Luminescence was read (EnVision Multimode Plate Reader, PerkinElmer #2105). Data were analyzed using GraphPad Prism 9.0 software, and dose-response curves were fitted by nonlinear S-curve regression to calculate IC50 values (nM). $Cell viability \left(%\right) = \left({Lum}_{test drug}-{Lum}_{medium control}\right) / \left({Lum}_{vehicle control}-{Lum}_{medium control}\right) \times 100 %$

| Example | IC50 (nM) | | | | |
|---|---|---|---|---|---|
| | GP2D | HPAC | AsPC-1 | KP-4 | Panc 04.03 |
| P2 | 0.42 | 1.44 | 2.09 | 6.92 | 2.12 |
| P6 | 0.43 | 5.38 | 1.38 | 4.74 | 1.36 |

Representative drug P unit compounds of the present disclosure showed satisfactory anti-proliferative activity against the 5 KRAS G12D mutant cell lines.

### Activity Example 6: Inhibitory Effect of Representative Drug P Unit Compounds of ADC of the Present Disclosure on Proliferation of KRAS Mutant Cells

Following essentially the same experimental method and procedure as Activity Example 5, the inhibitory effect of representative drug P unit compounds of ADC of the present disclosure on **proliferation of** KRAS mutant cells was tested in cell lines H358, H727, A549, GP2D, MKN1 (purchased from Nanjing Kebai Biotechnology Co., Ltd.) and cell lines A375 and HCT116 (purchased from Cell Bank of the Chinese Academy of Sciences):

| **KRAS** | **Cell Line** | **Catalog No.** | **Cell Type** | **Complete Medium** |
|---|---|---|---|---|
| G12C | H358 | CBP60136 | Adherent | RPMI1640 + 10% FBS |
| G12S | A549 | CBP60084 | Adherent | F12K Nutrient Mixture + 10% FBS |
| G12D | GP2D | CBP60010 | Adherent | DMEM(Gibco, 11965092) + 10% FBS |
| G12V | H727 | CBP60182 | Adherent | RPMI1640 + 10% FBS |
| G13D | HCT116 | TCHu99 | Adherent | McCOY's 5A + 10% FBS |
| WT | A375 | SCSP-533 | Adherent | DMEM(Gibco, 11965092) + 10% FBS |
| WT, amp | MKN1 | CBP60486 | Adherent | RPMI1640 + 10% FBS |

The anti-proliferative activity of representative drug P compounds against various KRAS mutant cells is shown in the table below:

| Drug P | IC50 (nM) | | | | | | |
|---|---|---|---|---|---|---|---|
| | H358 (G12C) | GP2D (G12D) | A549 (G12S) | H727 (G12V) | HCT116 (G13D) | A375 (WT) | MKN1 (WT, amp) |
| P8 | +++ | +++ | + | +++ | ++ | + | +++ |
| P9 | +++ | +++ | + | +++ | + | + | +++ |
| P10 | | +++ | | | | + | + |
| P11 | | +++ | | | | + | + |
| P12 | ++ | +++ | + | ++ | + | + | ++ |
| P13 | +++ | +++ | + | +++ | ++ | + | + |
| P14 | | +++ | | | | + | + |
| P15 | | +++ | | | | + | + |
| P16 | ++ | +++ | | +++ | | | |
| P17 | ++ | +++ | | +++ | | | |

Representative drug P compounds of ADC of the present disclosure showed good anti-proliferative activity against various KRAS mutant cells. IC50 < 5 nM +++, 5-100 nM ++, >100 nM +

### Activity Example 7: Inhibitory Effect of ADC Compounds of the Present Disclosure on Proliferation of 3 KRAS G12D Mutant Tumor Cell Lines

The following 3 cell lines were purchased from Nanjing Kebai Biotechnology Co., Ltd.:

| **KRAS** | **Cell Line** | **Catalog No.** | **Cell Type** | **Complete Medium** |
|---|---|---|---|---|
| G12D | AGS | CBP60476 | Adherent | F12K Nutrient Mixture(Gibco , 21127-022) + 10%FBS(Gibco, 10091-148) |
| G12D | GP2D | CBP60010 | Adherent | DMEM(Gibco, 11965092) + 10% FBS |
| G12D | HPAC | CBP60539 | Adherent | HPAC Complete Medium (Cobioer, CBP60539M) |

Cells in the logarithmic growth phase were harvested, and cell counting and viability assessment were performed using a Countstar automatic cell counter based on the classical trypan blue staining principle, ensuring that cell viability was above 90%. After adjusting cell concentration, 135 µL of cell suspension was added to a 96-well clear-bottom black-walled plate (Greiner, Cat# 655090), 800-1000 cells/well. The cells in the 96-well plate were cultured overnight at 37 °C, 5% CO₂ (Thermo Scientific, Model 371 Series).

Preparation of drug for IC₅₀ assay: 10x drug solutions were prepared in culture medium. 15 µL of drug solution was added per well to the cell-seeded plates, with a final top concentration of 600 nM, 9 concentrations, 3-fold dilution (DPBS, Corning, 21-031-CVC), two replicate wells per concentration.

The cells in the drug-treated 96-well plate were further cultured at 37 °C, 5% CO₂ for 120 hours, followed by CTG analysis (CellTiter-Glo^{®} Luminescent Cell Viability Assay, Promega, Cat# G7573). CTG reagent was thawed, and cell plates were equilibrated to room temperature for 30 minutes. 50 µL of CTG solution was added per well. Plates were shaken on an orbital shaker for 2 minutes to lyse cells, then placed at room temperature for 10 minutes to stabilize the luminescent signal. Luminescence was read (EnVision Multimode Plate Reader, PerkinElmer #2105) and the data was collected. Data were analyzed using GraphPad Prism 9.0 software, and dose-response curves were fitted by nonlinear S-curve regression to calculate IC₅₀ values. $Cell viability \left(%\right) = \left({Lum}_{test drug}-{Lum}_{medium control}\right) / \left({Lum}_{vehicle control}-{Lum}_{medium control}\right) \times 100 %$

The ADC compounds of the present disclosure showed good anti-proliferative activity against the 3 KRAS G12D mutant cell lines in this example.

| Example | IC50(nM) | | | | Example | IC50(nM) | | |
|---|---|---|---|---|---|---|---|---|
| | AGS | GP2D | HPAC | | | AGS | GP2D | HPAC |
| **1** | 11.9 | 2.3 | 48.1 | | **23** | 5.9 | 114.6 | 737.3 |
| **2** | 21.4 | 2.3 | 198.8 | | **24** | 38.6 | 175.9 | >600 |
| **3** | 3.2 | 8.2 | 198.5 | | **25** | 47.1 | 76.2 | 528 |
| **4** | 8.6 | 3.9 | 424 | | **26** | 16.2 | 49.5 | 342 |
| **5** | 2.4 | 3.2 | 283.3 | | **27** | 173.5 | 195.6 | >600 |
| **6** | 8.3 | 3.2 | 251.4 | | **28** | 31.3 | 27.4 | 395 |
| **7** | 2.8 | 0.4 | 72.3 | | **29** | 68.8 | 22.2 | 1353.1 |
| **8** | 1.8 | 0.2 | 39 | | **30** | 11.1 | 0.6 | 572.1 |
| **9** | 3.2 | 1.8 | 107.6 | | **31** | 6.1 | 11 | 391.3 |
| **10** | 7.9 | 4.6 | 159.3 | | **32** | 17.2 | 40.6 | >600 |
| **11** | 7.7 | 2.5 | 160.7 | | **33** | 2.1 | 1.9 | 158.5 |
| **12** | 98.6 | 4.4 | 206.8 | | **34** | 149.0 | 102.4 | >600 |
| **13** | 7.7 | 24.7 | 869.5 | | **36** | 4.7 | 10.8 | 319.6 |
| **14** | 7.9 | 3.1 | 114.6 | | **37** | 3.6 | 19.9 | 48.6 |
| **15** | 33.3 | 2.7 | 81.8 | | **38** | NT | NT | NT |
| **16** | 33.3 | 1.5 | 31 | | **39** | 14.7 | 1.3 | 481.9 |
| **17** | 2.3 | 2.3 | 256.8 | | **40** | 6.9 | 0.1 | 19.7 |
| **18** | 8.3 | 9.1 | 113.3 | | **41** | 3.2 | 4.2 | 16.6 |
| **19** | 6.1 | 3.0 | 66.8 | | | | | |
| **20** | 3.0 | 2.8 | 19.9 | | | | | |
| **21** | 1.5 | 5.2 | 30.6 | | | | | |
| **22** | 85.3 | 32.4 | 801.7 | | | | | |

The ADC compounds **42** to **64** of the present disclosure exhibited good anti-proliferative activity against two KRAS G12D mutant cell lines. IC₅₀ < 5 nM +++, 5-100 nM ++, >100 nM +

| Example | IC50(nM) | | | Example | IC50(nM) | |
|---|---|---|---|---|---|---|
| | AGS | GP2D | | | AGS | GP2D |
| **42** | + | +++ | | **54** | +++ | +++ |
| **43** | +++ | +++ | | **55** | + | + |
| **44** | + | +++ | | **56** | + | + |
| **45** | + | +++ | | **57** | + | + |
| **46** | + | + | | **58** | +++ | + |
| **47** | + | + | | **59** | + | +++ |
| **48** | + | + | | **60** | +++ | +++ |
| **49** | + | + | | **61** | + | +++ |
| **50** | + | + | | **62** | +++ | +++ |
| **51** | + | + | | **63** | + | + |
| **52** | +++ | +++ | | **64** | +++ | +++ |
| **53** | +++ | +++ | | | | |

The ADC compounds of the present disclosure exhibited good anti-proliferative activity against various KRAS mutant cell lines in this example, such as NCI-H358 (G12C), GP2D (G12D), A549 (G12S), NCI-H727 (G12V), HCT116 (G13D), A375 (WT), and MKN1 (WT, amp). IC₅₀ < 5 nM +++, 5-100 nM ++, >100 nM +

| Example | GP2D (G12D) |
|---|---|
| **65** | ++ |
| **66** | + |
| **68** | + |
| **69** | + |
| **71** | ++ |
| **72** | ++ |
| **73** | ++ |

### Activity Example 8: Anti-tumor Effect of ADC Compounds of the Present Disclosure in a KRAS-G12D Mutant Human Colon Cancer GP2D Subcutaneous Xenograft BALB/c Nude Mouse Model

GP2D cells (Nanjing Kebai Biotechnology Co., Ltd.) were cultured in culture medium containing DMEM and 10% FBS, and cells in logarithmic growth phase were collected and resuspended in PBS to an appropriate concentration mixed 1:1 with matrigel for subcutaneous tumor inoculation on the right posterior back of BALB/c female mice (purchased from Jiangsu Jicui Yaokang Biotechnology Co., Ltd.). Mice were inoculated subcutaneously on the right posterior back with 1×10⁷ GP2D cells per mouse in 0.1 mL volume. When average tumor volume reached ~150 mm³, mice were randomized into groups based on tumor size and body weight. The day of dosing was considered D0. Compounds were administered via tail vein injection at 20 mg/kg or 10 mg/kg. The vehicle control group received PBS. Mouse body weights and tumor volumes were measured twice weekly. 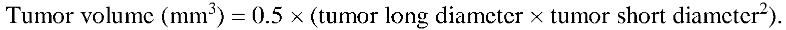 Tumor volume (mm3) = 0.5 × (tumor long diameter × tumor short diameter2). 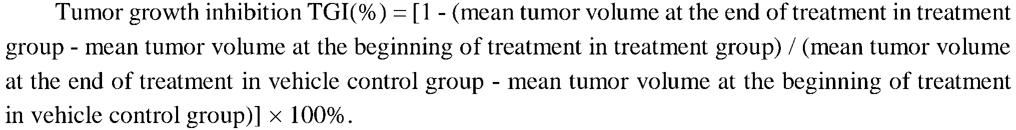 Tumor growth inhibition TGI(%) = [1 - (mean tumor volume at the end of treatment in treatment group - mean tumor volume at the beginning of treatment in treatment group) / (mean tumor volume at the end of treatment in vehicle control group - mean tumor volume at the beginning of treatment in vehicle control group)] × 100%.

The tumor inhibitory effects of representative ADC compounds of the present disclosure are shown in the tables below.

| Group | Tumor volume (mm³, Day 21) | Dose (mg/kg) | TGI (%) | Dosing frequency |
|---|---|---|---|---|
| Vehicle control* | 1143 | - | / | D0,7 |
| Example **1** | 110 | 20 | 103 | D0,7 |
| Example **2** | 222 | 20 | 92 | D0,7 |

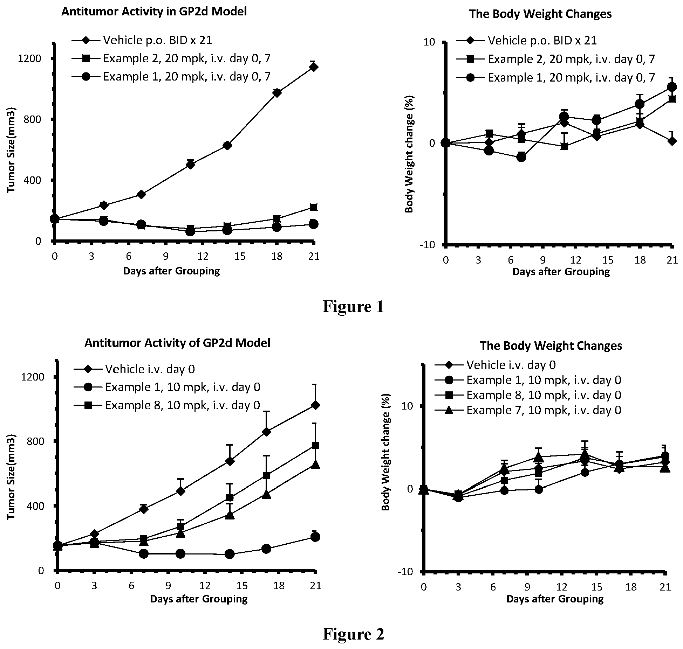

See Figure 1 for details.

| Group | Tumor volume (mm³, Day 21) | Dose(mg/kg) | TGI (%) | Dosing frequency |
|---|---|---|---|---|
| Vehicle control* | 1026 | - | / | D0 |
| Example **1** | 208 | 10 | 94 | D0 |
| Example **7** | 658 | 10 | 42 | D0 |
| Example **8** | 774 | 10 | 29 | D0 |

See Figure 2 for details.

| Group | Tumor volume (mm³, Day 21) | Dose(mg/kg) | TGI (%) | Dosing frequency |
|---|---|---|---|---|
| Vehicle control* | 952 | - | / | D0 |
| Example **1** | 193 | 10 | 95 | D0 |
| Example **4** | 258 | 10 | 86 | D0 |
| Example **5** | 621 | 10 | 41 | D0 |
| Example **6** | 397 | 10 | 69 | D0 |
| Example **30** | 230 | 10 | 90 | D0 |
| Example **31** | 166 | 10 | 98 | D0 |
| Example **32** | 218 | 10 | 91 | D0 |
| Example **33** | 197 | 10 | 94 | D0 |

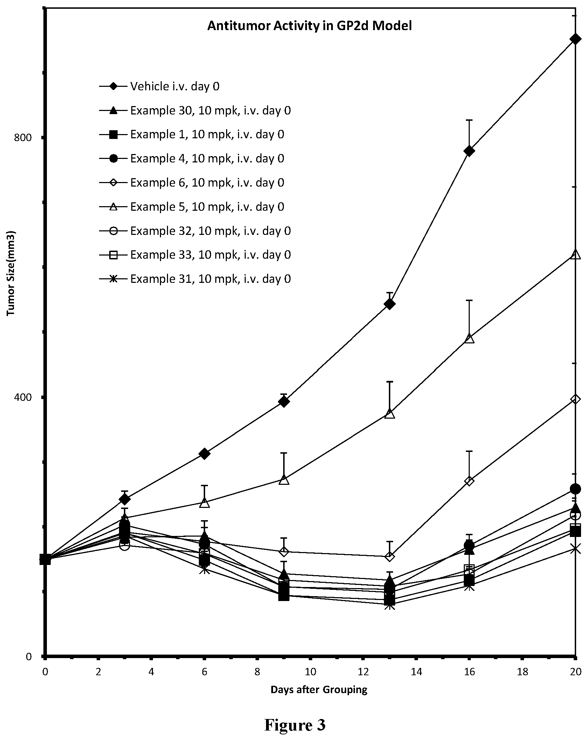

See Figure 3 for details.

| Group | Tumor volume (mm³, Day 21) | Dose(mg/kg) | TGI (%) | Dosing frequency |
|---|---|---|---|---|
| Vehicle control* | 1054 | - | / | D0 |
| Example **1** | 164 | 10 | 99 | D0 |
| Example **3** | 350 | 10 | 78 | D0 |
| Example **9** | 728 | 10 | 36 | D0 |
| Example **10** | 558 | 10 | 55 | D0 |
| Example **12** | 429 | 10 | 69 | D0 |
| Example **13** | 629 | 10 | 47 | D0 |
| Example **15** | 715 | 10 | 38 | D0 |
| Example **17** | 664 | 10 | 43 | D0 |
| Example **22** | 488 | 10 | 63 | D0 |
| Example **23** | 609 | 10 | 49 | D0 |
| Example **24** | 213 | 10 | 93 | D0 |
| Example **25** | 345 | 10 | 79 | D0 |
| Example **26** | 583 | 10 | 52 | D0 |
| Example **27** | 499 | 10 | 62 | D0 |
| Example **28** | 860 | 10 | 21 | D0 |
| Example **29** | 624 | 10 | 48 | D0 |

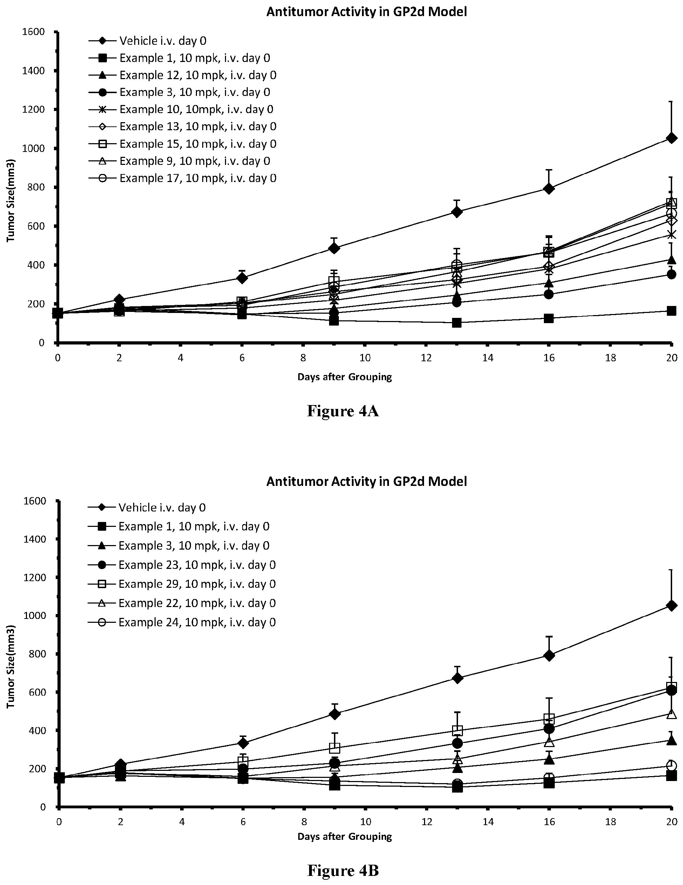

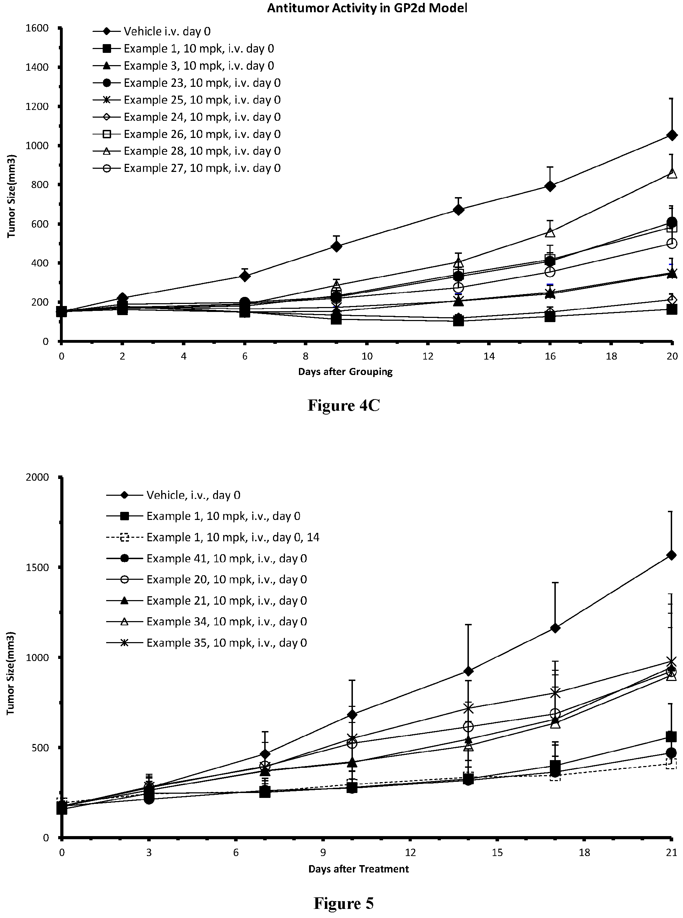

See Figures 4A, 4B, 4C for details.

| Group | Tumor volume (mm³, Day 21) | Dose(mg/kg) | TGI (%) | Dosing frequency |
|---|---|---|---|---|
| Vehicle control* | 1568 | - | / | D0 |
| Example **1** | 559 | 10 | 71 | D0 |
| Example **1** × 2 | 410 | 10 | 84 | D0, 14 |
| Example **20** | 920 | 10 | 46 | D0 |
| Example **21** | 945 | 10 | 44 | D0 |
| Example **34** | 900 | 10 | 48 | D0 |
| Example **35** | 978 | 10 | 42 | D0 |
| Example 41 | 470 | 10 | 79 | D0 |

See Figure 5 for details.

| Group | Tumor volume (mm³, Day 21) | Dose(mg/kg) | TGI (%) | Dosing frequency |
|---|---|---|---|---|
| Vehicle control* | 1042 | - | / | D0,7,14 |
| Example **1** | 312 | 10 | 81 | D0,7,14 |
| Example **1** × 3 | 322 | 10 | 85 | D0,7,14 |
| Drug payloadP3 | 1050 | 0.15 (payload **P3** equivalent in **1)** | -1 | D0,7,14 |
| Antibody Trastuzumab | 702 | 10 | 39 | D0,7,14 |
| Example **36** | 818 | 10 | 25 | D0 |
| Example **37** | 725 | 10 | 36 | D0 |
| Example **39** | 316 | 10 | 83 | D0 |
| Example **40** | 263 | 10 | 89 | D0 |

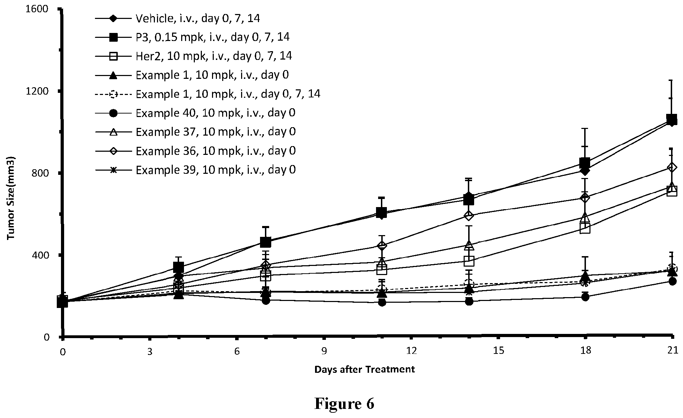

See Figure 6 for details.

The experimental results show that although the drug payload, linker unit, antibody, etc., affect the in vivo anti-tumor proliferation efficacy to varying degrees, the ADC compounds of the present disclosure all demonstrate target-related excellent tumor inhibitory activity in the human colon cancer GP2D model. Meanwhile, there were no significant changes in body weight in each group of mice receiving all tested ADC compounds.

The specific embodiments provided herein are merely illustrative of the present disclosure and are not intended to limit the scope defined by the claims. Based on the content disclosed in this application, equivalent variations of the technical solutions of the present application will be readily apparent to those skilled in the art, and these variations are also intended to be encompassed within the scope of the present application.

## Claims

1. An antibody-drug conjugate of formula (X) or a pharmaceutically acceptable salt or solvate thereof:
[P-L]_{q}-Ab (X)
wherein,
Ab represents an antibody or antigen-binding fragment that binds to a target antigen;
q represents the number of [P-L] units connected to Ab, which is an integer or non-integer of at least 1;
L represents a linker unit connecting P to Ab;
P represents a Ras mutant inhibitor compound.

2. The antibody-drug conjugate according to claim 1, wherein the Ras mutant inhibitor compound is a compound of formula (I): 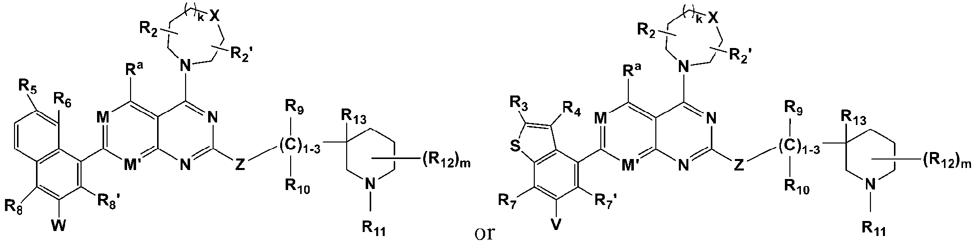
or a stereoisomer, tautomer, stable isotopic variant, pharmaceutically acceptable salt, prodrug, or solvate thereof,
wherein:
M is selected from N or C-R₁;
M' is selected from N or C-R₁';
R₁ and R₁' are each independently selected from H, halogen, CN, -C₁₋₆ alkyl optionally substituted by halogen, and -OC₁₋₆ alkyl optionally substituted by halogen;
R^{a} is selected from H, halogen, CN, -C₁₋₆ alkyl optionally substituted by halogen, -C₂₋₆ alkynyl optionally substituted by halogen, and -OC₁₋₆ alkyl optionally substituted by halogen or deuterium;
X is selected from CH₂, N, and O;
R₂ and R₂' are each independently selected from H, OH, or -C₁₋₆ alkyl optionally substituted by halogen; or R₂ and R₂' attached to non-adjacent ring carbon atoms together form an intra-ring bridge -(CH₂)₁₋₂- or -CH₂=CH₂-; or R₂ and R₂' attached to the same ring carbon atom together with the ring carbon atom to which they are attached form a 4-6 membered spirocycloalkyl or a 4-6 membered spiroheterocycloalkyl containing 1 or 2 heteroatoms selected from N and O;
R₃ is selected from H, -NH₂, -NHC₁₋₆ alkyl, -N(C₁₋₆ alkyl)₂, and -OC₁₋₆ alkyl;
R₄ is selected from -CN, halogen, -NO₂, -C₂₋₆ alkynyl optionally substituted by halogen, and - C₁₋₆ alkyl optionally substituted by halogen;
R₅ is selected from H, -CN, halogen, -NO₂, and halogen-substituted -C₁₋₆ alkyl;
R₆ is selected from H, halogen, CN, -C₁₋₆ alkyl, and -C₂₋₆ alkynyl, wherein the -C₁₋₆ alkyl and - C₂₋₆ alkynyl are each independently optionally substituted by halogen;
R₇, R₇', R₈, and R₈' are each independently selected from H, halogen, CN, -NO₂, and -C₁₋₆ alkyl optionally substituted by halogen;
V and W are each independently selected from H, halogen, -C₁₋₆ alkyl, OH, and NH₂;
Z is selected from O, N, and CH₂;
R₉ and R₁₀ are each independently selected from H, deuterium, -C₁₋₆ alkyl, and -(CH₂)ₙ-C₃₋₆ cycloalkyl, wherein the -C₁₋₆ alkyl and -C₃₋₆ cycloalkyl are each independently optionally substituted by deuterium, halogen, or -O-C₁₋₆ alkyl, or R₉ and R₁₀ attached to the same carbon atom together with the carbon atom to which they are attached form a C₃₋₄ cycloalkyl;
R₁₁ is selected from H, -C₁₋₆ alkyl, -C₂₋₆ alkenyl, -C₂₋₆ alkynyl, and -(CH₂)ₙ-C₃₋₆ cycloalkyl, wherein the C₁₋₆ alkyl, -C₂₋₆ alkenyl, -C₂₋₆ alkynyl, or C₃₋₆ cycloalkyl are each independently optionally substituted by deuterium, halogen, CN, or -O-C₁₋₆ alkyl;
R₁₂ is selected from H, halogen, -CN, -OH, -NH₂, -NHC₁₋₆ alkyl, -N(C₁₋₆ alkyl)₂, -O-C₁₋₆ alkyl, -O-C₃₋₆ cycloalkyl, -C₁₋₆ alkyl, -C₂₋₆ alkenyl, -C₂₋₆ alkynyl, -(CH₂)ₙ-C₃₋₆ cycloalkyl, and =C(R^{d})₂, wherein R^{d} is each independently selected from H, halogen, and -C₁₋₆ alkyl optionally substituted by halogen, wherein each occurrence of C₁₋₆ alkyl, -C₂₋₆ alkenyl, -C₂₋₆ alkynyl, or C₃₋₆ cycloalkyl is each independently optionally substituted by halogen, CN, or -OC₁₋₆ alkyl;
R₁₃ is selected from H, -C₁₋₆ alkyl, and -(CH₂)ₙ-C₃₋₆ cycloalkyl, wherein the -C₁₋₆ alkyl and -C₃₋₆ cycloalkyl are each independently optionally substituted by halogen or -O-C₁₋₆ alkyl;
k is selected from 0 or 1;
m is selected from 0 to 6;
n is selected from 0 to 2;
wherein the compound of formula (I) is bonded to the linker unit L via the ring NH present in the 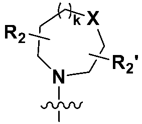 fragment thereof, and/or via the OH present on the side-chain naphthalene or benzothiophene ring.

3. The antibody-drug conjugate according to claim 2, wherein the structural fragment 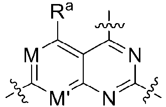 in formula (I) is 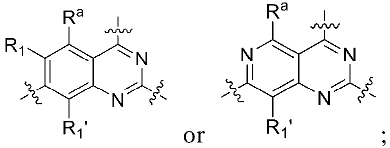 Preferably,
M is C-R₁ and M' is C-R₁', wherein R₁ is selected from halogen or -C₁₋₆ alkyl optionally substituted by halogen, R^{a} is H, and R₁' is halogen; or M is N and M' is C-R₁', wherein R^{a} is selected from H, -C₂₋₆ alkynyl, and -OC₁₋₆ alkyl, and R₁' is halogen,
More preferably,
M is C-R₁ and M' is C-R₁', wherein R₁ is selected from F or Cl, or -C₁₋₃ alkyl substituted by halogen, R^{a} is H, and R₁' is F; or M is N and M' is C-R₁', wherein R^{a} is selected from H, -C₂₋₄ alkynyl, and -OC₁₋₃ alkyl optionally substituted by deuterium, and R₁' is F;
**and/or**
wherein in the structural fragment 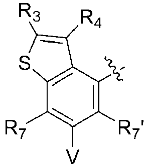 in formula (I), R₃ is selected from -NH₂, - NHC₁₋₆ alkyl, and N(C₁₋₆ alkyl)₂; R₄ is selected from -CN, halogen, -NO₂, and -C₂₋₆ alkynyl substituted by halogen; V is selected from H, -C₁₋₆ alkyl, and halogen; R₇ and R₇' are each H, or each are halogen, or one of them is H and the other is halogen or halogen-substituted C₁₋₆ alkyl, or one is halogen and the other is halogen-substituted C₁₋₆ alkyl,
Preferably, the structural fragment 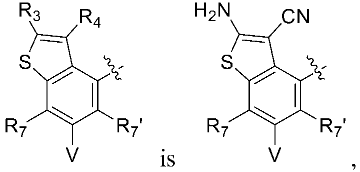 V is H, R₇ and R₇' are each H, or one of them is H and the other is halogen, or
wherein in the structural fragment 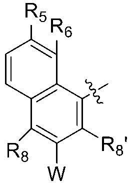 in formula (I), W is OH; R₅ is H or halogen; R₆ is selected from halogen, -C₂₋₆ alkynyl, and -C₁₋₆ alkyl; R₈ and R₈' are each H; or R₈ and R₈' are each halogen; or one of R₈ and R₈' is H and the other is selected from halogen, CN, and NO₂; or one of R₈ and R₈' is H and the other is selected from -C₁₋₆ alkyl optionally substituted by halogen; or one of R₈ and R₈' is selected from H, halogen, -NO₂, CN, and -C₁₋₆ alkyl optionally substituted by halogen, and the other is selected from -C₁₋₆ alkyl optionally substituted by halogen,
Preferably, the structural fragment 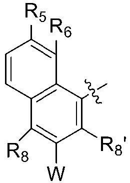 is 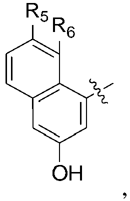 wherein R₅ is halogen, R₆ is selected from -C₂₋₆ alkynyl;
**and/or**
wherein the structural fragment 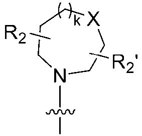 in formula (I) is 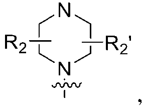 wherein R₂ and R₂ attached to non-adjacent ring carbon atoms together form an intra-ring bridge -CH₂-, -CH₂CH₂-, or -CH₂=CH₂-, preferably 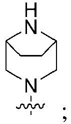 **and/or**
wherein Z is O, and the structural fragment connected to Z is 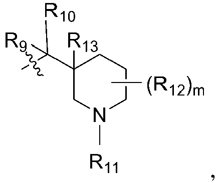 preferably 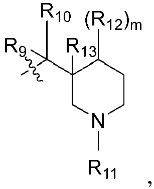 more preferably

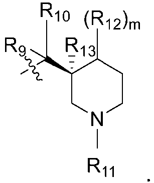

4. The antibody-drug conjugate according to claim 2 or 3, wherein
R₉ and R₁₀ are both H, or one or both are deuterium; **and/or**
wherein R₁₁ is -C₁₋₆ alkyl, wherein the hydrogen atom is optionally replaced by one or more isotope deuterium; **and/or**
wherein R₁₂ is selected from -C₁₋₆ alkyl optionally substituted by halogen and =C(R^{d})₂, wherein R^{d} is each independently selected from H and halogen, and m is 1 or 2; **and/or**
wherein R₁₃ is -C₁₋₆ alkyl, preferably -C₁₋₃ alkyl, more preferably -CH₃.

5. The antibody-drug conjugate according to any one of claims 1 to 4, wherein the structural fragment 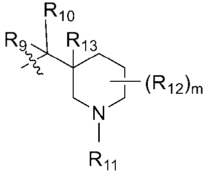 is selected from

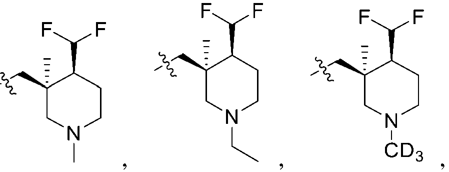

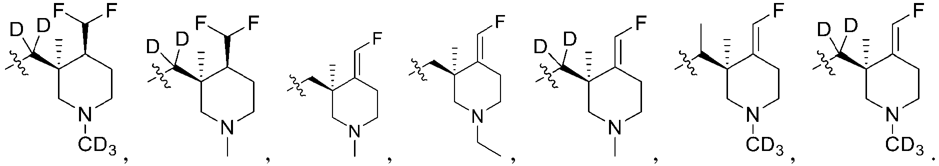

6. The antibody-drug conjugate according to any one of claims 1 to 5, wherein the compound of formula (I) has any one of the following sub-general formulas: 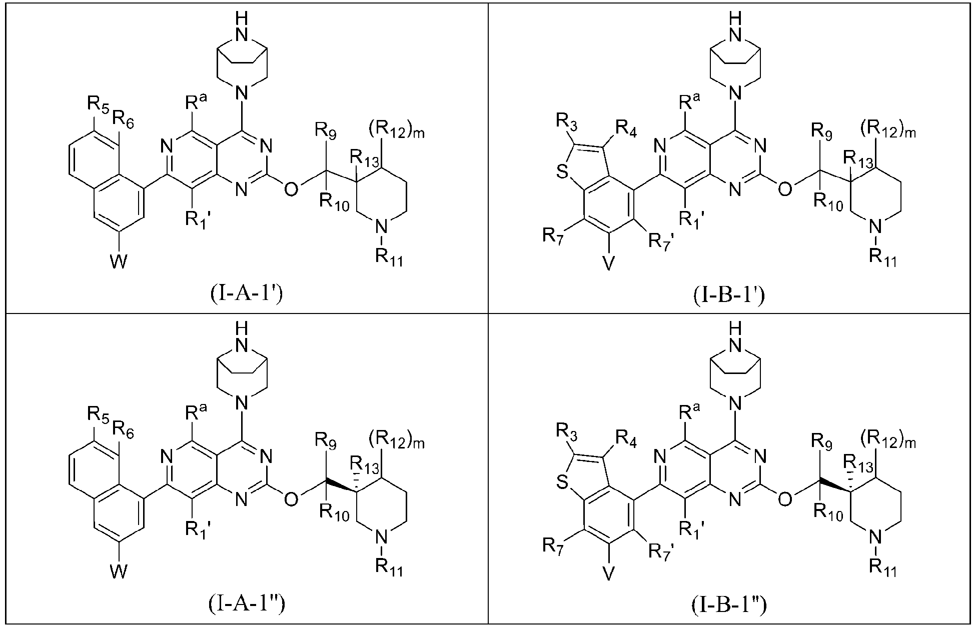 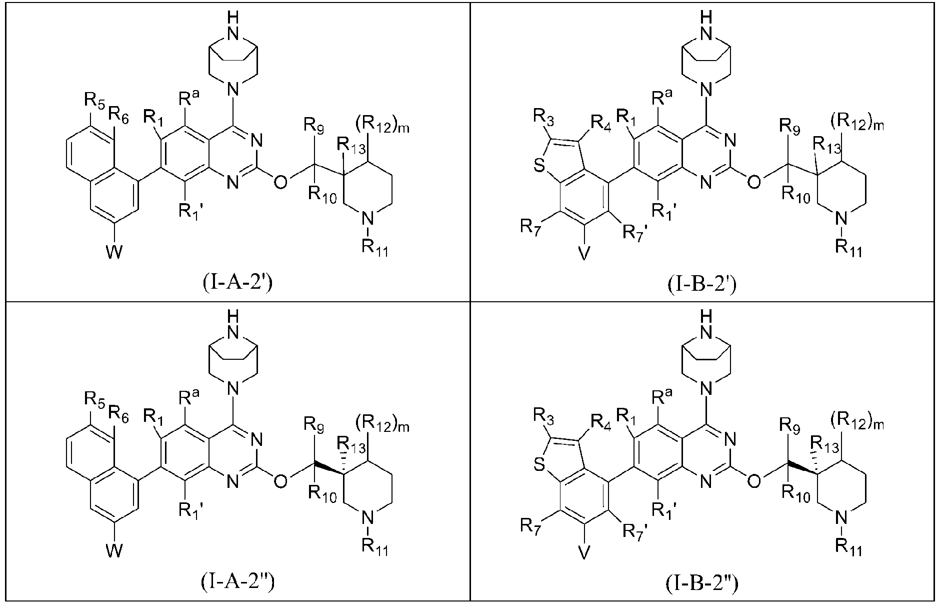 wherein R₁, R₁', R^{a}, R₃, R₄, R₅, R₆, R₇, R₇', W, V, R₉, R₁₀, R₁₁, R₁₂, R₁₃, m are each as defined in the corresponding claims 1 to 11; preferably
when M is C-R₁ and M' is C-R₁', R₁ is selected from F or Cl, or -C₁₋₃ alkyl substituted by halogen, R^{a} is H, and R₁' is F; or when M is N and M' is C-R₁', R^{a} is selected from H, -C₂₋₄ alkynyl, and -OC₁₋₃ alkyl optionally substituted by deuterium, and R₁' is F;
R₃ is -NH₂, R₄ is -CN, V is H, R₇ and R₇' are each H, or one of them is H and the other is halogen;
R₅ is halogen, R₆ is selected from -C₂₋₆ alkynyl, R₈ and R₈' are each H, W is OH;
R₉ and R₁₀ are both H, or one or both are deuterium;
R₁₁ is -C₁₋₃ alkyl, wherein the hydrogen atom is optionally replaced by one or more isotope deuterium;
R₁₂ is selected from -C₁₋₃ alkyl optionally substituted by halogen and =C(R^{d})₂, wherein R^{d} is each independently selected from H and halogen;
m is 1 or 2;
R₁₃ is -C₁₋₃ alkyl.

7. The antibody-drug conjugate according to any one of claims 1 to 6, wherein the Ras inhibitor compound is selected from: 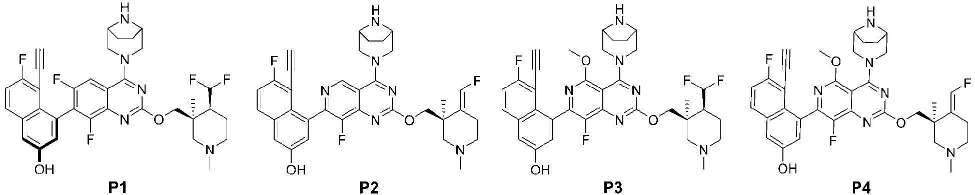 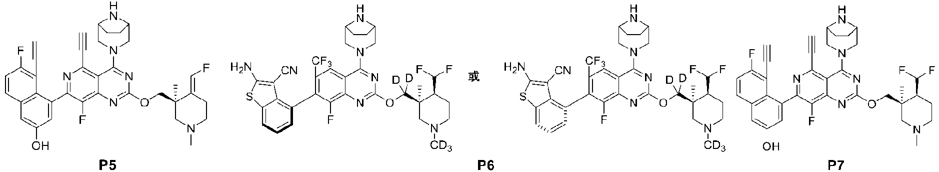 stereoisomers, tautomers, stable isotopic variants, pharmaceutically acceptable salts, or solvates thereof.

8. The antibody-drug conjugate according to claim 1, wherein the Ras inhibitor compound has any one of the following sub-general formulas: 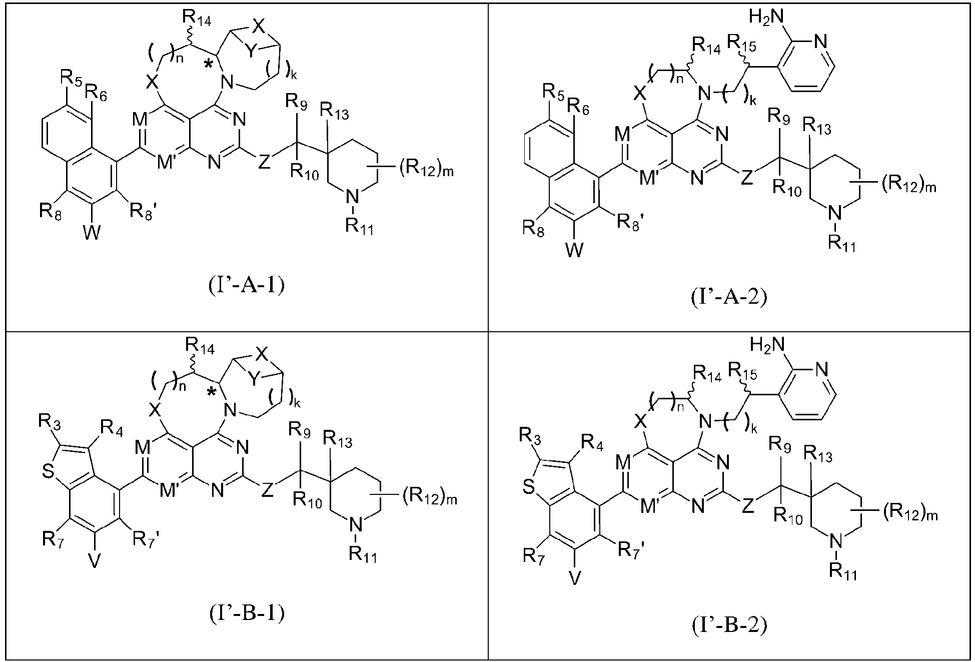 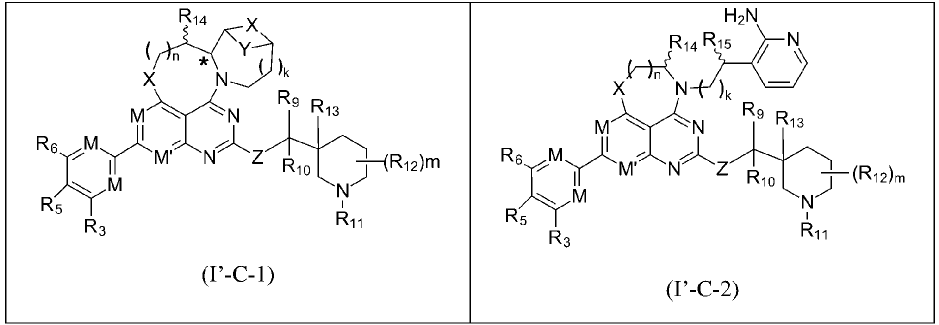 or a stereoisomer, tautomer, stable isotopic variant, pharmaceutically acceptable salt, or solvate thereof,
wherein M, M', X, Z, V, W, R₁, R₁', R₃, R₄, R₅, R₆, R₇, R₇', R₅, R₈', R₉, R₁₀, R₁₁, R₁₂, R₁₃, k, m, n, the structural fragments 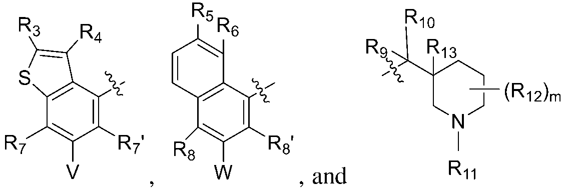 are respectively as defined in any one of claims 2 to 6;
Y is selected from -CH₂-, -CH₂CH₂-, -CH₂OCH₂-, or is absent;
R₁₄ and R₁₅ are each independently selected from H and -C₁₋₆ alkyl;
**Preferably,**
in the 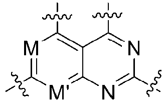 fragment, when M is C-R₁, R₁ is selected from halogen (preferably F or Cl) or -C₁₋₆ alkyl optionally substituted by halogen (preferably -C₁₋₃ alkyl substituted by halogen, more preferably -CF₃), and M' is C-R₁' and R₁' is halogen (preferably F); or when M is N, M' is C-R₁' and R₁' is halogen (preferably F);
in the 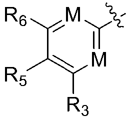 fragment, both M are C-R₁, or one M is N and the other M is C-R₁, R₁ are each independently selected from H, halogen (preferably F), and halogen-substituted -C₁₋₆ alkyl (preferably -C₁₋₃ alkyl substituted by one or more F, more preferably -CF₃), preferably the M ortho to R₃ is selected from N and C-R₁ (preferably C-halogen, more preferably C-F), and the M ortho to R₆ is C-R₁ (preferably C-halogen-substituted -C₁₋₃ alkyl, more preferably -C-CF₃); R₃ is selected from -NH₂, -NHC₁₋₆ alkyl, and N(C₁₋₆ alkyl)₂ (preferably selected from -NH₂, -NHC₁₋₃ alkyl, and N(C₁₋₃ alkyl)₂, more preferably -NH₂); R₅ is H or halogen (preferably F); R₆ is selected from halogen (preferably Cl) and -C₁₋₆ alkyl (preferably -C₁₋₃ alkyl);
in the 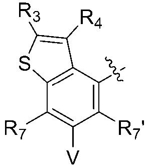 fragment, R₃ is selected from -NH₂, -NHC₁₋₆ alkyl, and N(C₁₋₆ alkyl)₂ (preferably selected from -NH₂, -NHC₁₋₃ alkyl, and N(C₁₋₃ alkyl)₂, more preferably -NH₂); R₄ is selected from -CN, halogen, -NO₂, and -C₂₋₆ alkynyl substituted by halogen; V is selected from H, - C₁₋₆ alkyl (preferably -C₁₋₃ alkyl), and halogen; R₇ and R₇' are each H, or each are halogen, or one is H and the other is halogen or halogen-substituted C₁₋₆ alkyl (preferably halogen-substituted -C₁₋₃ alkyl), or one is halogen and the other is halogen-substituted C₁₋₆ alkyl (preferably halogen-substituted -C₁₋₃ alkyl), wherein the halogen is preferably F;
Preferably, R₃ is -NH₂, R₄ is -CN, V is H, R₇ and R₇' are each H, or one is H and the other is halogen (preferably F);
in the 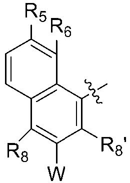 fragment, R₅ is H or halogen (preferably F); R₆ is selected from halogen, - C₂₋₆ alkynyl (preferably -C₂₋₄ alkynyl), and -C₁₋₆ alkyl (preferably -C₁₋₃ alkyl); R₈ and R₈' are each H, or R₈ and R₈' are each halogen, or one of R₈ and R₈' is H and the other is selected from halogen, CN, and NO₂, or one of R₈ and R₈' is H and the other is selected from -C₁₋₆ alkyl optionally substituted by halogen, or one of R₈ and R₈' is selected from H, halogen, -NO₂, CN, and -C₁₋₆ alkyl optionally substituted by halogen, and the other is selected from -C₁₋₆ alkyl optionally substituted by halogen; W is OH;
Preferably, R₅ is halogen (preferably F), R₆ is selected from -C₂₋₆ alkynyl (preferably -C₂₋₄ alkynyl), R₈ and R₈' are each H, W is OH;
X is selected from O and NH;
Y is selected from -CH₂CH₂- and is absent;
Z is selected from O;
R₉ and R₁₀ are both H; or one or both are deuterium;
R₁₁ is -C₁₋₆ alkyl (preferably -C₁₋₃ alkyl), wherein the hydrogen atom is optionally replaced by one or more deuterium;
R₁₂ is selected from -C₁₋₆ alkyl optionally substituted by halogen (preferably -C₁₋₃ alkyl) and =C(R^{d})₂, wherein R^{d} is each independently selected from H and halogen (preferably F);
m is 0, 1, or 2 (preferably 1 or 2);
n and k are 0 or 1 (preferably 0);
R₁₃ is -C₁₋₆ alkyl (preferably -C₁₋₃ alkyl, more preferably -CH₃);
R₁₄ and R₁₅ are H or -C₁₋₃ alkyl (preferably -CH₃).

9. The antibody-drug conjugate according to claim 1, wherein the Ras inhibitor compound is selected from: 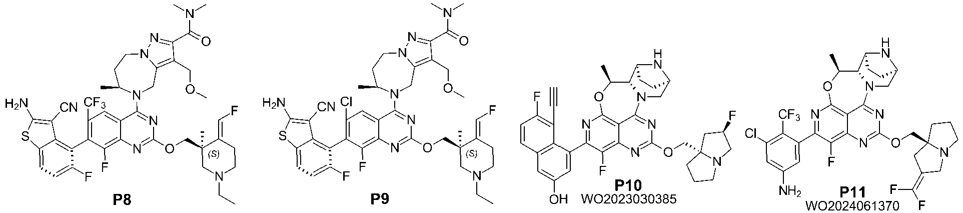 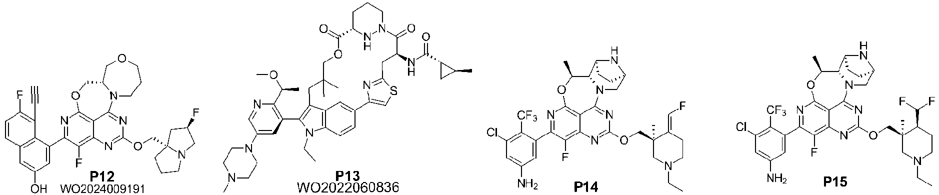 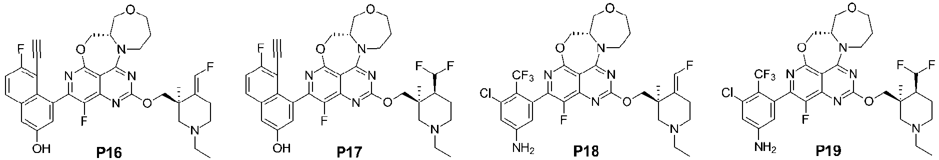 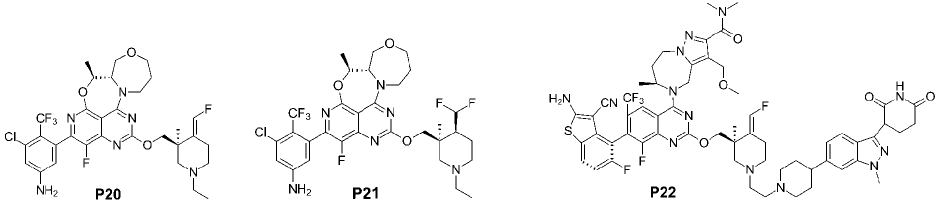 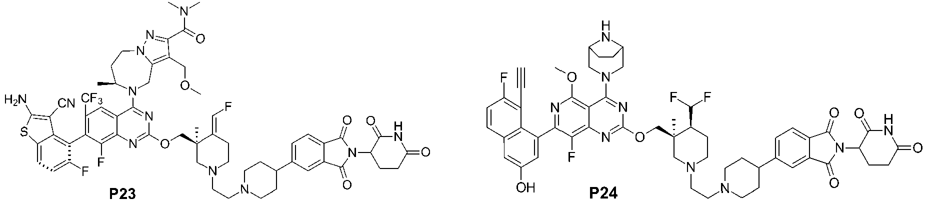 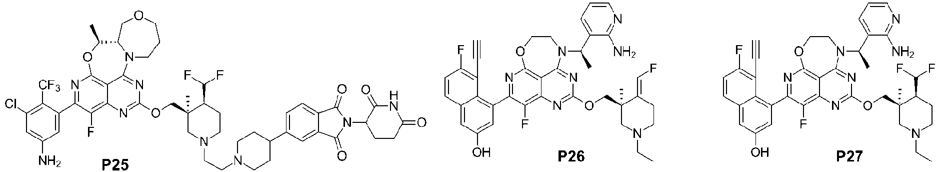 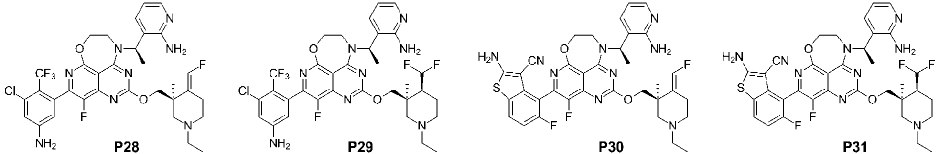 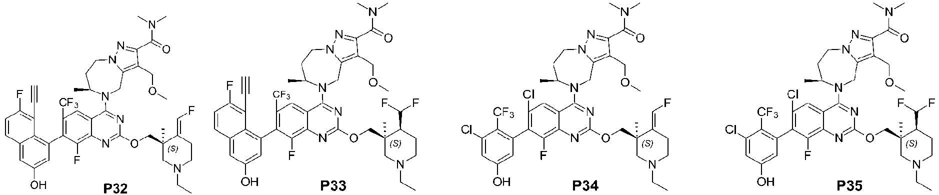 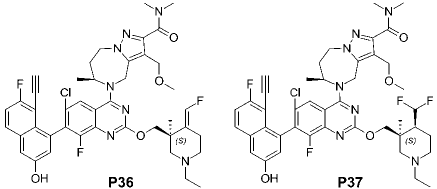 or stereoisomers, tautomers, stable isotopic variants, pharmaceutically acceptable salts, or solvates thereof.

10. The antibody-drug conjugate according to any one of claims 1 to 9, wherein the linker unit L has the structure:
-A(S)-B-G- (II-3)
or
-B-G- (II-5)
wherein:
**A** is selected from 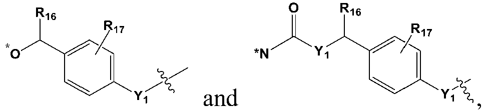 preferably 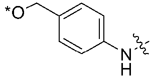 and 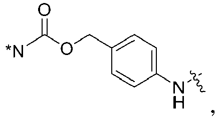 wherein the end marked with * is connected to the drug P, and the N or O marked with * is an atom from the drug P; Y₁ is each independently NH or O; R₁₆ is H; R₁₇ is H;
**S** is selected from optionally present glucose, galactose, mannose, glucosamine, galactosamine, mannosamine, galacturonic acid, glucuronic acid, mannuronic acid, acetylgalactosamine, acetylglucosamine, acetylmannosamine, acetylgalactosaminuronic acid, acetylglucosaminuronic acid, acetylmannosaminuronic acid, glucosaminuronic acid, galactosaminuronic acid, mannosaminuronic acid; lactose, maltose, or their respective amino derivatives, acetamido derivatives, uronic acid derivatives, amino uronic acid derivatives, acetamido uronic acid derivatives; preferably S is selected from D-glucose, D-galactose, D-mannose, D-galacturonic acid, D-glucuronic acid, D-mannuronic acid, 2-acetamido-2-deoxy-D-galactose, 2-acetamido-2-deoxy-D-glucose, 2-acetamido-2-deoxy-D-mannose, 2-acetamido-2-deoxy-D-galacturonic acid, 2-acetamido-2-deoxy-D-glucuronic acid, 2-acetamido-2-deoxy-D-mannuronic acid; D-lactose, D-maltose, D-sucrose, or their respective 2-acetamido and/or 6-carboxy derivatives;
**B** is of formula (B-2): -(AA)_{d}-, wherein
d is an integer from 2 to 12, preferably an integer from 2 to 4;
AA is an amino acid selected from: alanine, glycine, asparagine, valine, phenylalanine, citrulline, glutamic acid; preferably -(AA)_{d}- is the following polypeptide from C-terminus to N-terminus: Ala-Ala, Cit-Val, Ala-Val, Glu-Gly, Asn-Ala-Ala, Cit-Ala-Glu, Gly-Phe-Gly-Gly; wherein the N-terminus of the peptide is connected to the G part, and the C-terminus of the peptide is connected to the self-immolative linker A;
**G** is selected from 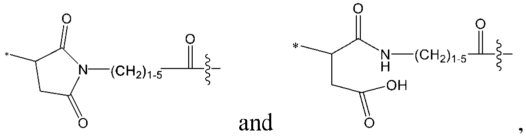 wherein the * end indicates the point of attachment to the antibody Ab, and the 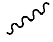 end indicates the point of attachment to B;
Specifically, the linker unit L has the formula (II-3-1): 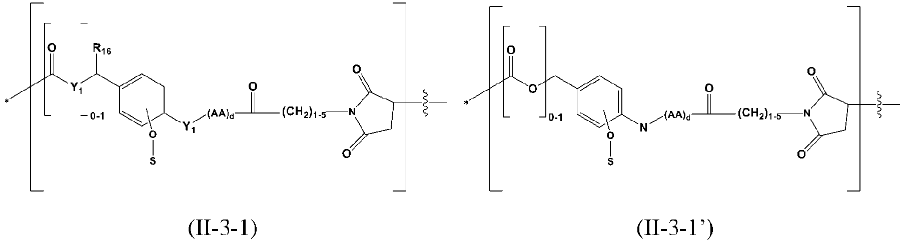
and the corresponding general formula fragments where the maleimide moiety is 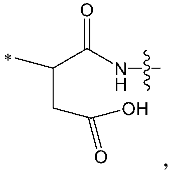 wherein -O- in -O-S is O forming the glycosidic bond with the phenyl ring in the solubilizing sugar unit, or
Specifically, when the solubilizing sugar unit S is absent, the linker unit L has the formula: 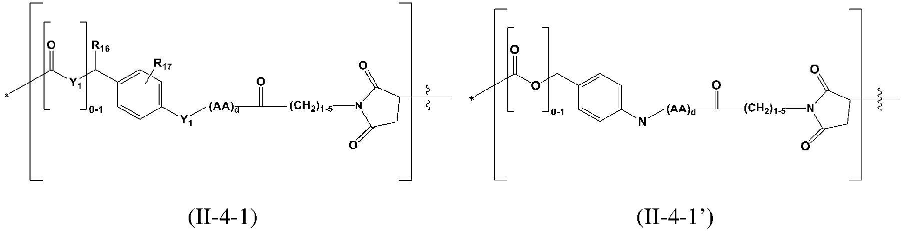
and the corresponding general formula fragments where the maleimide moiety is 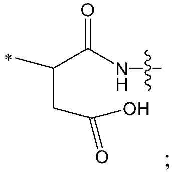 or
the linker unit L has the formula: 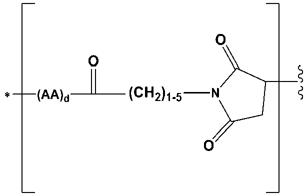 (11-5-1), and the corresponding general formula fragments where the maleimide moiety is

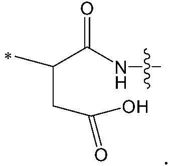

11. The antibody-drug conjugate according to claim 10, wherein the solubilizing sugar unit in the linker unit is selected from:
| | | | |
|---|---|---|---|
| 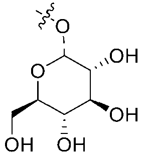 Glucose | 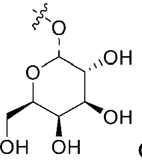 Galactose | 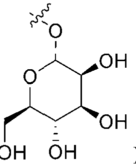 Mannose | 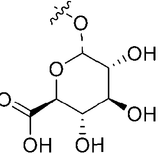 |
| | | | D-glucuronic acid |
| 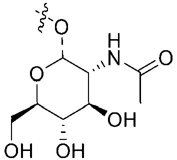 | 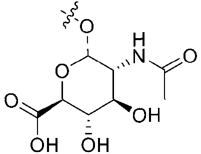 | 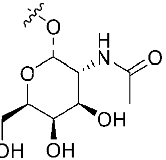 | 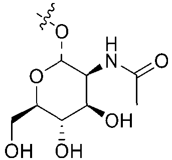 |
| 2-Acetamido-2-deoxy-D-glucose | 2-Acetamido-2-deoxy-D-glucuronic acid | 2-Acetamido-2-deoxy-D-galactose | 2-Acetamido-2-deoxy-D-mannose |
| 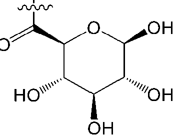 | 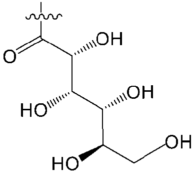 | 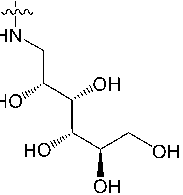 | 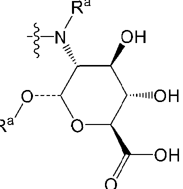 |
| 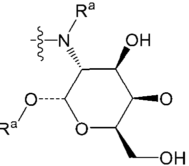 | 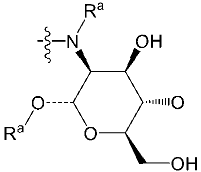 | 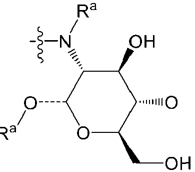 | Where R^{a} is H or C₁₋₆ alkyl, e.g. methyl |
| 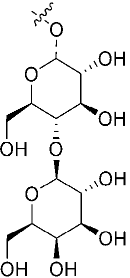 | 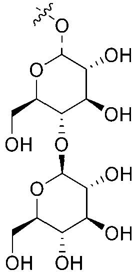 | 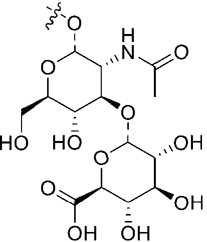 | 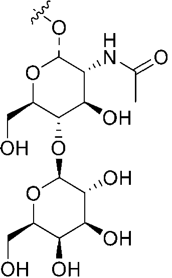 |
| Lactose | Maltose | Maltose derivative-acetamido and carboxylic acid derivative | Lactose derivative-acetamido derivative |
| 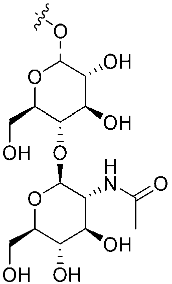 | 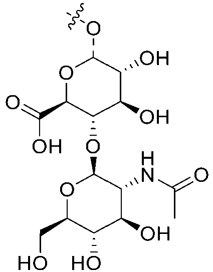 | | |
| Maltose derivative-acetamido derivative | Maltose derivative-acetamido and carboxylic acid derivative | | |
wherein the wavy bond indicates the connection bond of the solubilizing sugar unit to the self-immolative linker A, the O attached is the O of the solubilizing sugar unit forming the glycosidic bond with the phenyl ring, and the dashed line at position 1 of the solubilizing sugar unit indicates that the configuration of the glycosidic bond can be α, β, or a mixture thereof.

12. The antibody-drug conjugate according to any one of claims 10-11, wherein B in the linker unit is selected from the following peptides: 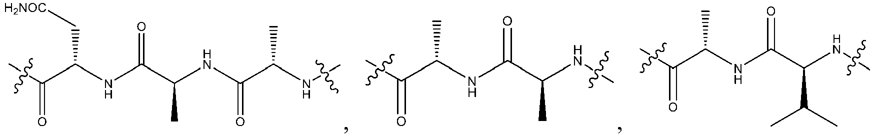 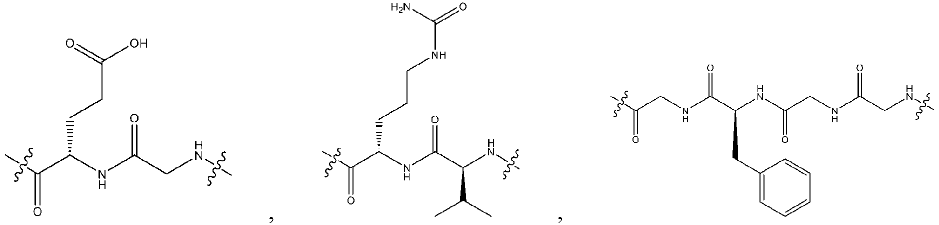 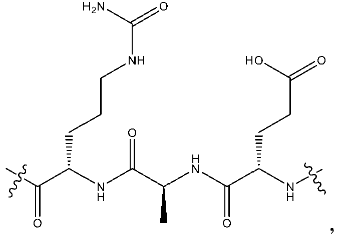 wherein the left carbonyl group is connected to the self-immolative linker A or the drug P, and the right N-terminus is connected to the G part.

13. The antibody-drug conjugate according to claim 1, wherein the [P-L] conjugation fragment has any one of the following general formulas: 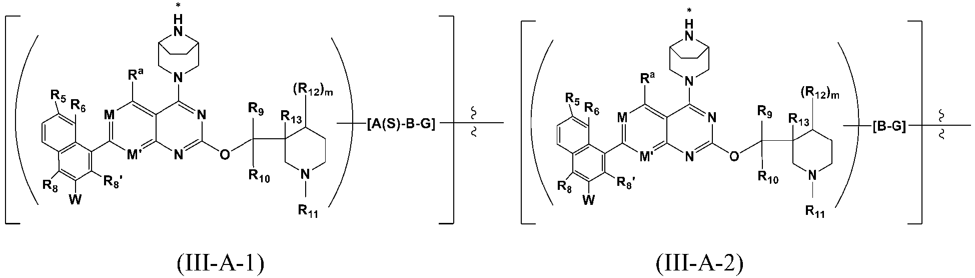 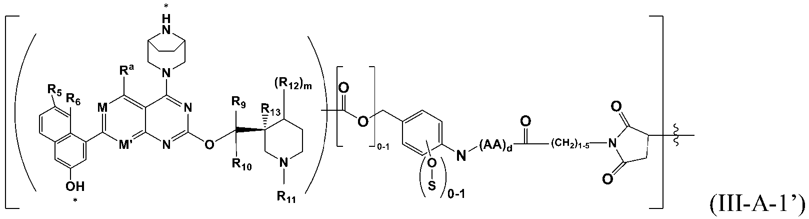 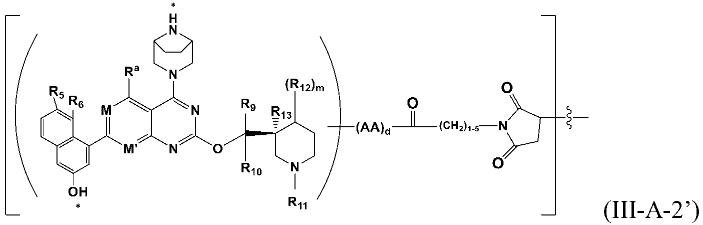 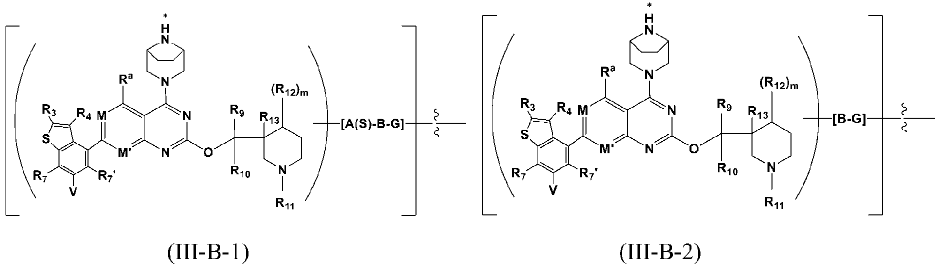 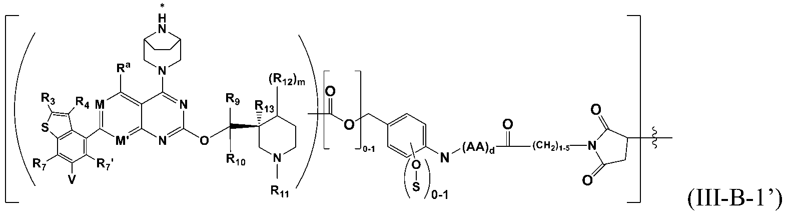 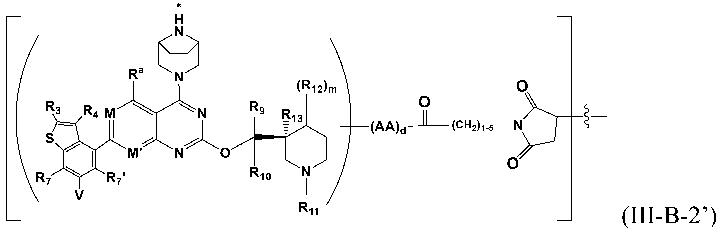 and the corresponding general formula fragments where the maleimide moiety is 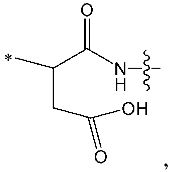 or a stereoisomer, tautomer, stable isotopic variant, pharmaceutically acceptable salt, or solvate thereof, wherein
M, M', R^{a}, R₃, R₄, R₅, R₆, R₇, R₇', R₅, R₈', R₉, R₁₀, R₁₁, R₁₂, R₁₃, W, V, m, and the respective structural fragments in the drug P unit have the meanings defined in the corresponding claims 2-9, and A, S, B, G, AA, d in the linker unit L have the meanings defined in the corresponding claims 10-12;
the wavy line indicates the point of attachment to Ab; the atom marked with * is the attachment site in the drug P unit for connection to the linker unit.

14. The antibody-drug conjugate according to claim 1, having any one of the following general formulas: 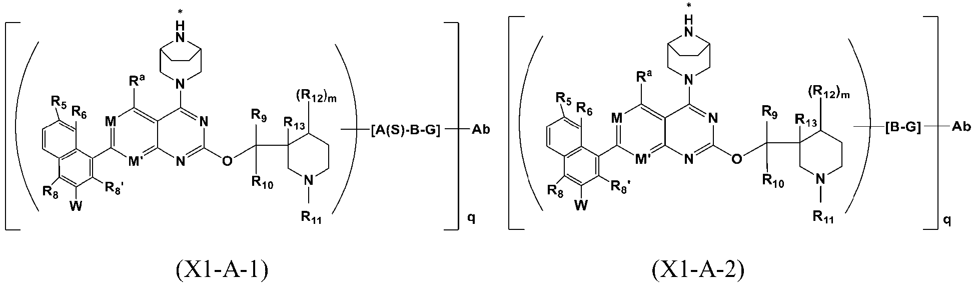 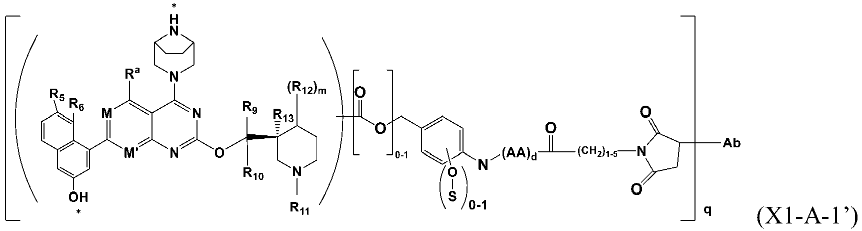 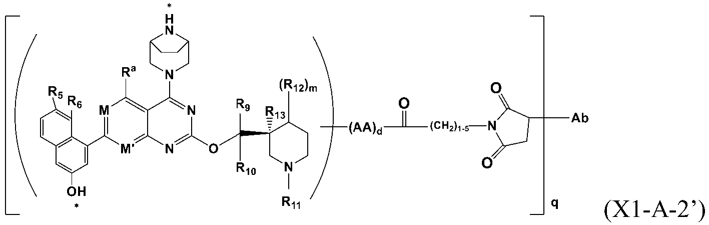 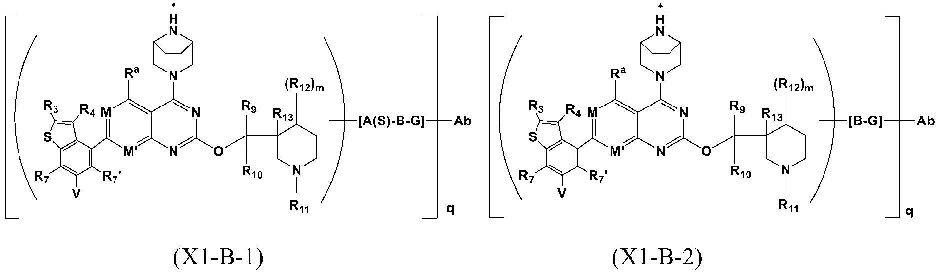 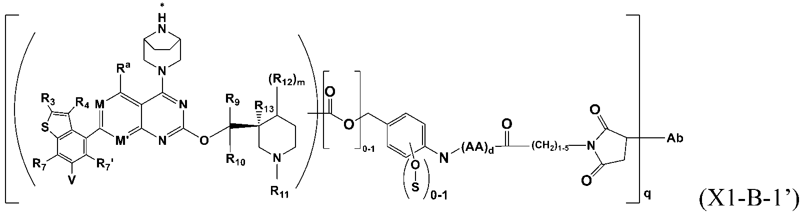 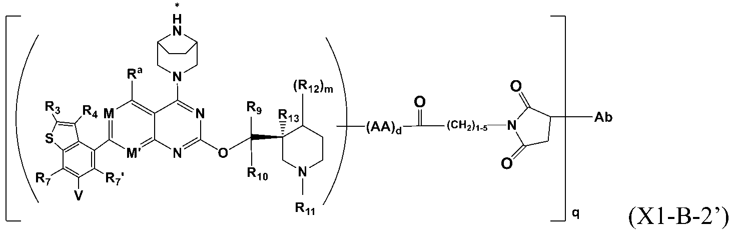 and the corresponding conjugates where the maleimide moiety is 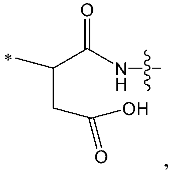 or a stereoisomer, tautomer, stable isotopic variant, pharmaceutically acceptable salt, or solvate thereof, wherein:
M, M', R^{a}, R₃, R₄, R₅, R₆, R₇, R₇', R₅, R₈', R₉, R₁₀, R₁₁, R₁₂, R₁₃, W, V, m, and the respective structural fragments in the drug P unit have the meanings defined in the corresponding claims 2-9, and A, S, B, G, AA, d in the linker unit L have the meanings defined in the corresponding claims 10-12;
the atom marked with * is the attachment site in the drug P unit for connection to the linker unit;
q is an integer or non-integer from 1 to 10, or a range formed by any two values between 1 and 10; preferably q is from 1 to 8, or an average DAR value in a range formed by any two values between 1 and 8.

15. The antibody-drug conjugate according to any one of claims 1 to 9, wherein the linker unit L has the structure of formula (II'-3):
-J-B-G- (II'-3)
wherein,
**J** is selected from -J₁-, -J₂-, -J₁-J₂-, -J₂-J₁-, wherein
-J₁- is selected from 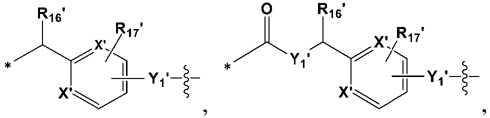 wherein the * indicates the point of attachment to drug P or to -J₂-, and the wavy line indicates the point of attachment to the cleavable linker B;
-J₂- is selected from 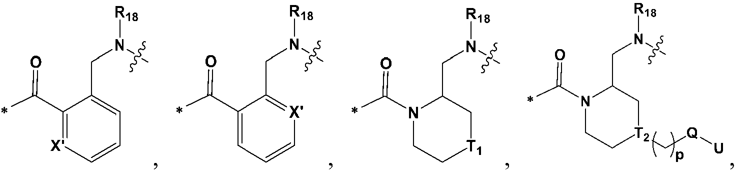 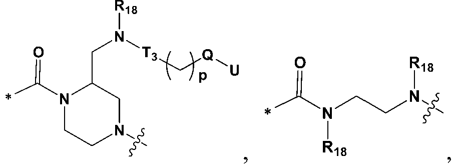 wherein the * indicates the point of attachment to drug P or to -J₁-, and the wavy line indicates the point of attachment to the cleavable linker B;
each occurrence of X' is independently selected from CH or N;
each occurrence of Y₁' is independently selected from -NH-, -NC₁₋₆ alkyl-, or -O-;
each occurrence of R₁₆' is independently selected from H and -C₁₋₆ alkyl, preferably H or C₁₋₃ alkyl, more preferably H;
each occurrence of R₁₇' is independently selected from H, -NO₂, -NH₂, -CF₃, or a moiety of formula (A1): 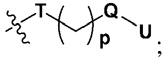
each occurrence of R₁₈ is independently selected from -C₁₋₆ alkyl, preferably C₁₋₃ alkyl, more preferably methyl;
T is selected from -C(R^{a})₂-, -O-, -NR^{a}-, or is absent;
T₁ is selected from -C(R^{a})₂-, -O-, -NR^{a}-;
T₂ is selected from -CR^{a}- and -N-;
T₃ is a peptide of 2~6 amino acids linked via the carboxyl terminus to N;
Q is selected from -CO-, -O-, -NR^{a}-, or is absent;
U is selected from glycosyl or a derivative thereof, 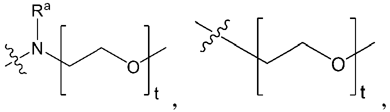 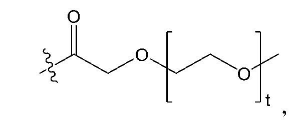 and a hydrophilic peptide comprising 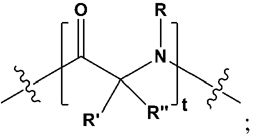
the subunit 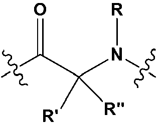 is selected from natural amino acid residues and unnatural amino acid residues;
R^{a} is each independently selected from H or -C₁₋₆ alkyl, preferably H or -CH₃;
p is an integer from 0 to 4, for example 0-2, 1-4, 1-2, 2-4;
t is an integer from 0 to 10, for example 0-8, 0-6, 0-4, 0-2, 1~10, 2~10, 2~8, 2~6, 2~4, 6~10, 8~10;
**B** is of formula (B-2): -(AA)_{d}-, wherein d is an integer from 2 to 12, preferably an integer from 2 to 4, preferably -(AA)_{d}- is the following polypeptide from C-terminus to N-terminus: Ala-Ala, Cit-Val, Ala-Val, Glu-Gly, Asn-Ala-Ala, Cit-Ala-Glu, Gly-Phe-Gly-Gly, wherein the N-terminus of the peptide is connected to the G part of the linker unit, and the C-terminus of the peptide is connected to J;
**G** is 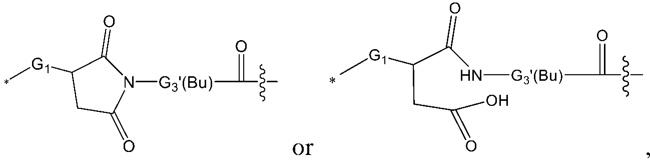 wherein G₁ is the atom in Ab responsible for attachment to L, G₃' is -C₁-₁₀ alkylene-, preferably -C₁-₅ alkylene-, Bu is absent or is a structural fragment of formula (A) 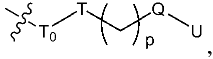 wherein T₀ is -C₁₋₆ alkylene-, preferably -C₁₋₂ alkylene-, more preferably methylene, and p, Q, U are each as defined above.

16. The antibody-drug conjugate according to claim 15, wherein J is selected from: 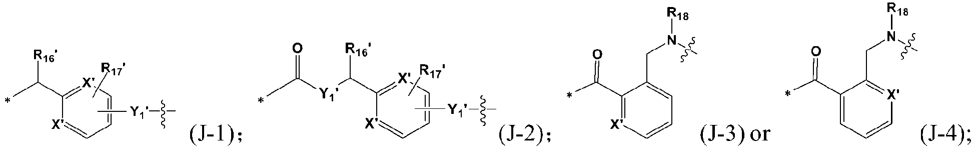 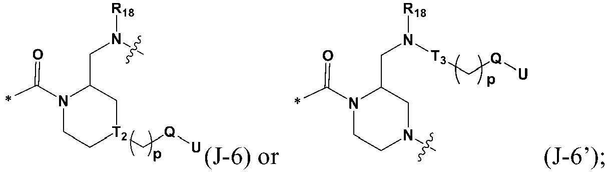 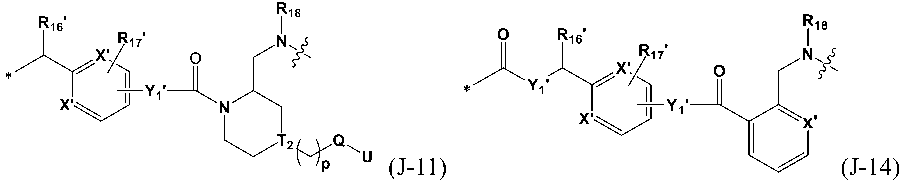 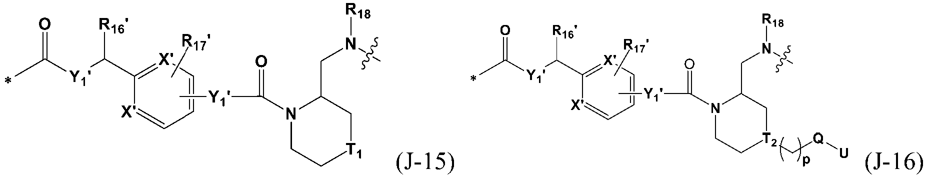 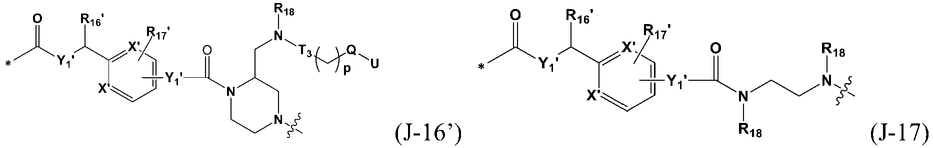 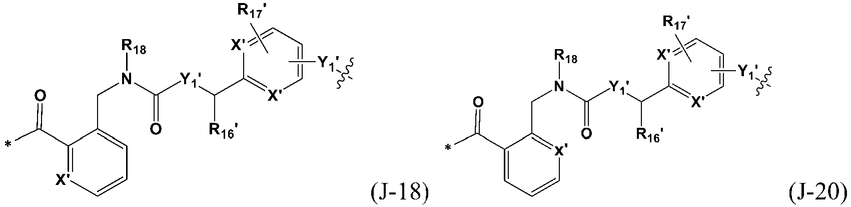 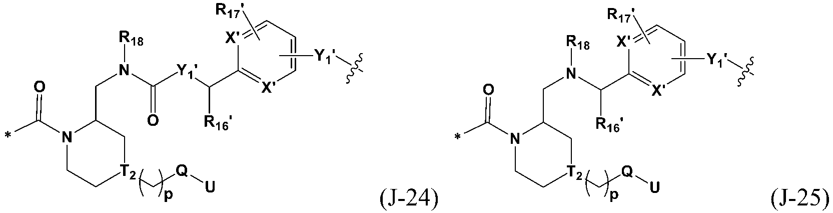
wherein the * indicates the point of attachment to drug P, and the wavy line indicates the point of attachment to the remainder of the linker unit;
each occurrence of X' is independently selected from -N- or -CH-;
each occurrence of Y₁' is independently selected from -NH- or -O-; wherein the Y₁' attached to the six-membered aromatic ring is attached para or ortho to the other attachment point of the ring;
each occurrence of R₁₆' is independently selected from H or C₁₋₃ alkyl, preferably H;
each occurrence of R₁₇' is independently selected from H or a fragment of formula (A1) 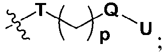
each occurrence of R₁₈ is independently selected from -C₁₋₃ alkyl, preferably methyl;
each occurrence of T is selected from -O-, -NR^{a}-, or is absent;
each occurrence of T₁ is selected from -CH₂-, -O-, -NCH₃-;
each occurrence of T₂ is selected as -N-;
each occurrence of T₃ is a 2-4 peptide linked via the carboxyl terminus to N, selected from Ala-Ala, Cit-Val, Ala-Val, Glu-Gly, Asn-Ala-Ala, Cit-Ala-Glu, Gly-Phe-Gly-Gly;
each occurrence of Q is absent or is -NR^{a}- or -O-;
each occurrence of U is selected from 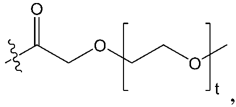 a hydrophilic peptide such as 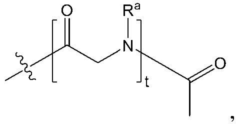 or a glycosyl (preferably a monosaccharide or disaccharide or uronic acid thereof) or derivative thereof, preferably a hydrophilic peptide, wherein t is 2-14, such as 4-14, 6-12, preferably 10;
each occurrence of p is 0-4, preferably 0-2;
For example, the self-immolative linker J is selected from:

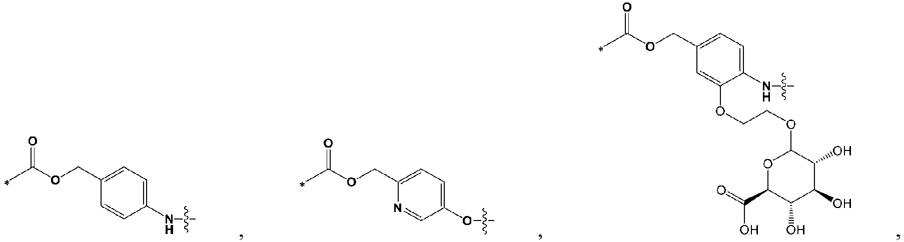

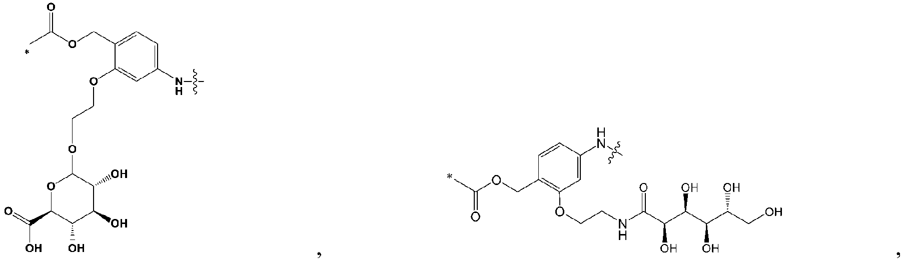

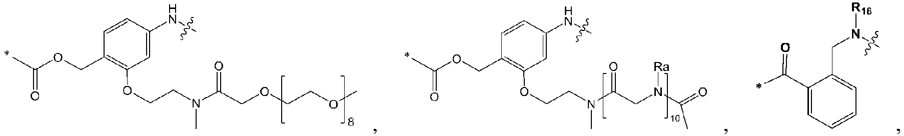

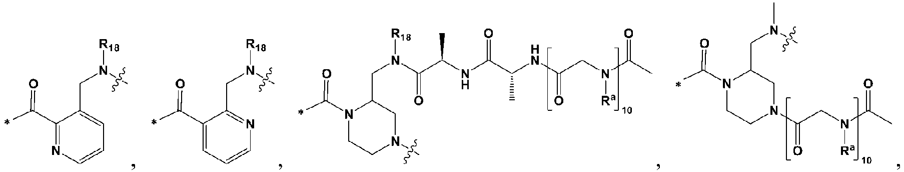

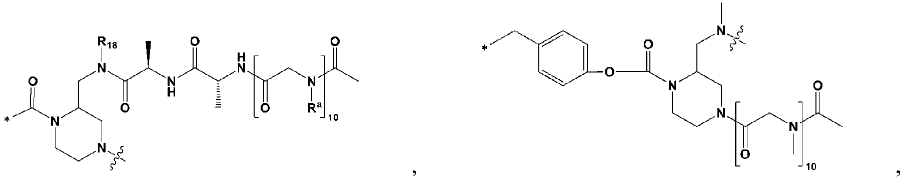

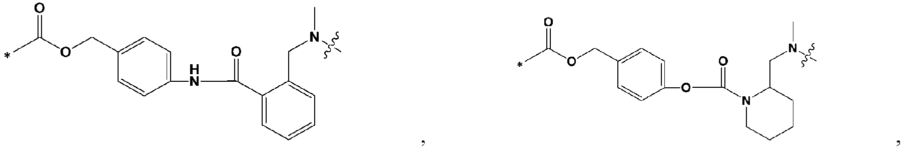

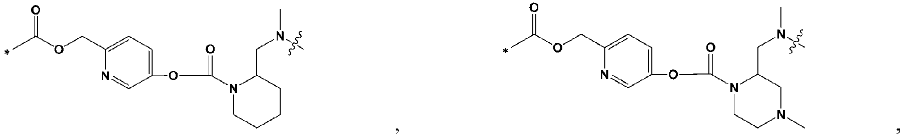

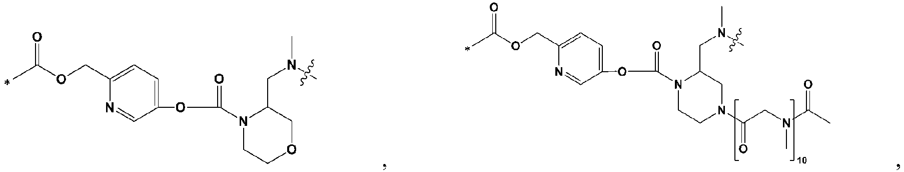

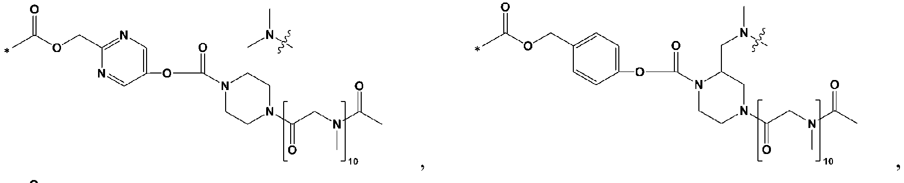

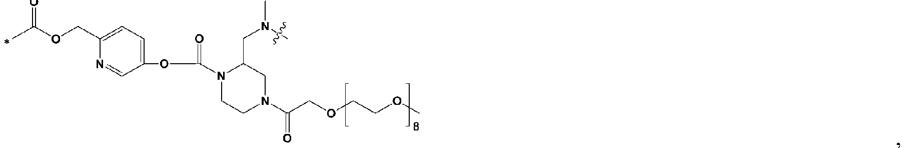

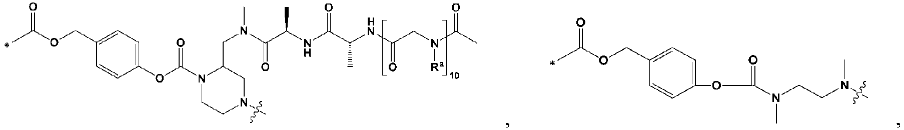

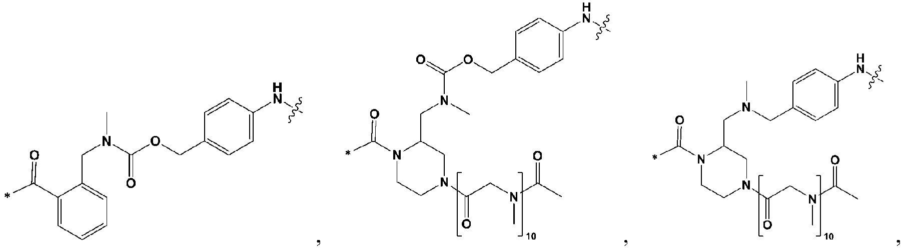

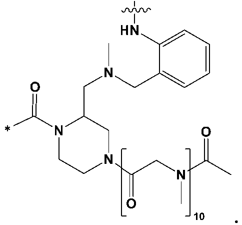

17. The antibody-drug conjugate according to claim 15 or 16, wherein G is 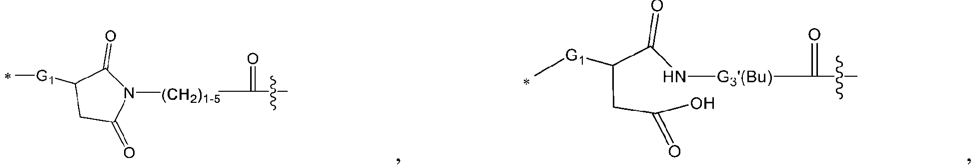 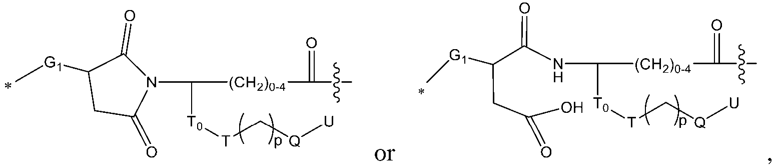 wherein T₀ is -C₁₋₄ alkylene- (e.g., -C₁₋₂ alkylene-, such as methylene), T is absent or is -C(R^{a})₂-, p is 0-4 (e.g., 0-2) and Q is selected from -O- or -NR^{a}-, and U is selected from a uronic acid (preferably a uronic acid of a monosaccharide or disaccharide) or derivative thereof linked via a carbonyl group, 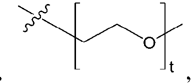 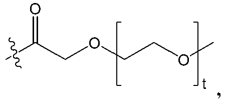 a hydrophilic peptide such as 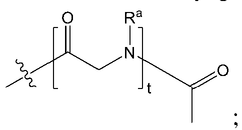 or wherein T₀ is -C₁₋₄ alkylene- (e.g., -C₁₋₂ alkylene-, such as methylene), T is absent or is -C(R^{a})₂-, p is 0-4 (e.g., 0-2) and Q is -CO-, U is selected from an amino sugar (preferably an amino monosaccharide or amino disaccharide) or derivative thereof linked via an amino group, 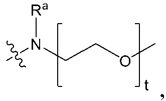 or a hydrophilic peptide such as 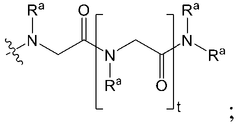 or wherein T₀ is -C₁₋₄ alkylene- (e.g., -C₁₋₂ alkylene-, such as methylene), T is selected from -C(R^{a})₂-, -O-, -NR^{a}-, or is absent, Q is absent, p is 0-4 (e.g., 0-2, 1-2), and U is selected from a glycosyl (preferably a monosaccharide or disaccharide) or derivative thereof linked via a glycosidic bond, or an amino sugar (preferably an amino monosaccharide or amino disaccharide) or derivative thereof linked via an amino group; wherein t is an integer from 1~20, e.g., 2-10, 2-8, 2-6, 2-4, 4-10, 4-14, 6-10, 6-12, 8-10, 8-14.

18. The antibody-drug conjugate according to any one of claims 15-17, wherein B is as defined in claim 12, and/or
the subunit 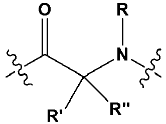 of the hydrophilic peptide corresponds to a residue of arginine, serine, threonine, tyrosine, cysteine, aspartic acid, asparagine, glutamic acid, glutamine, lysine, histidine, glycine, tryptophan, ornithine, citrulline, sarcosine, or R, R', R" are each independently additionally a group containing carboxyl, sulfonic acid, phosphate, amino, amido, quaternary ammonium, mercapto, and/or hydroxyl;
preferably the hydrophilic peptide comprises 4-14 units of polysarcosine, polyarginine, or polyglycine, preferably 6-12 units of polysarcosine.

19. The antibody-drug conjugate according to any one of claims 1-9 and 15-18, wherein the linker unit has the formula:
| |
|---|
| 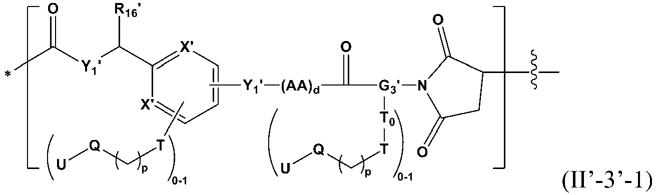 |
| 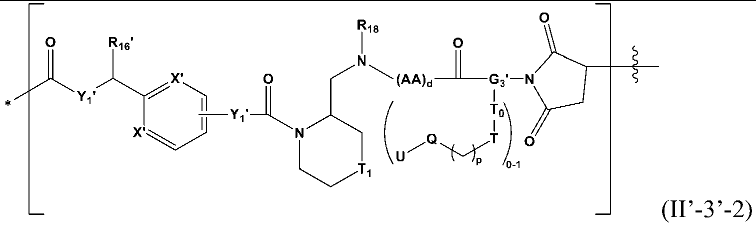 |
| 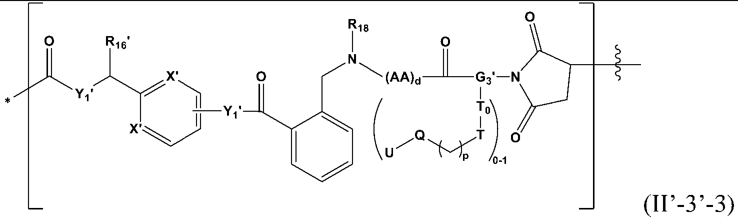 |
| 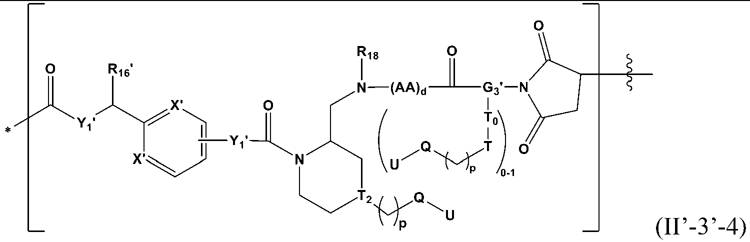 |
| 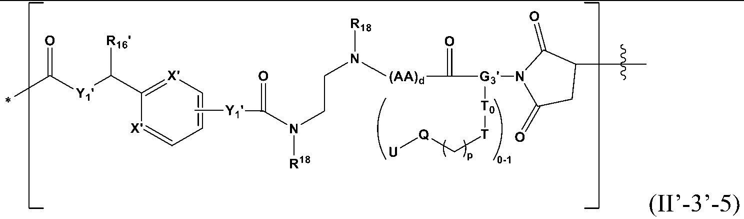 |
| 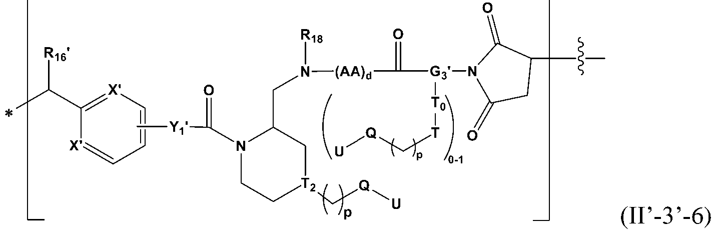 |
| 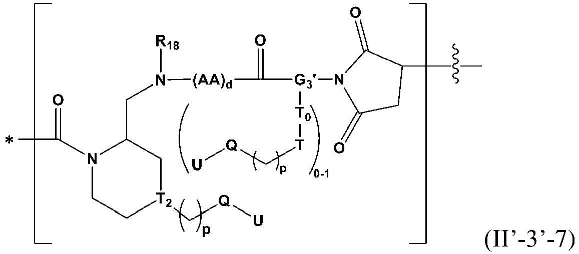 |
| 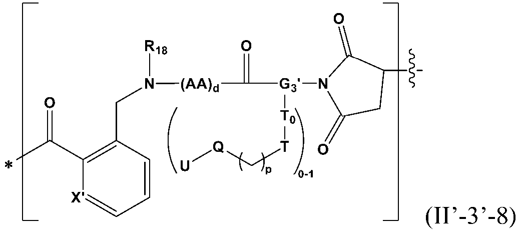 |
| 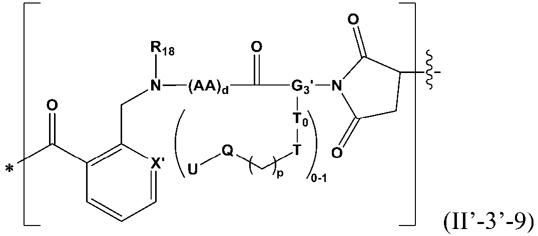 |
| 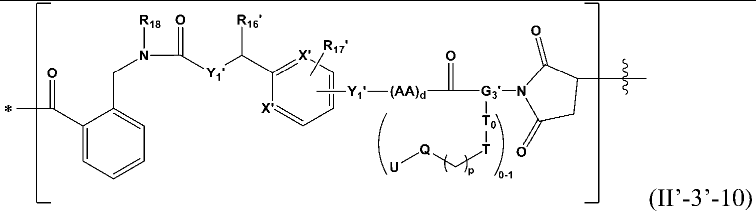 |
| 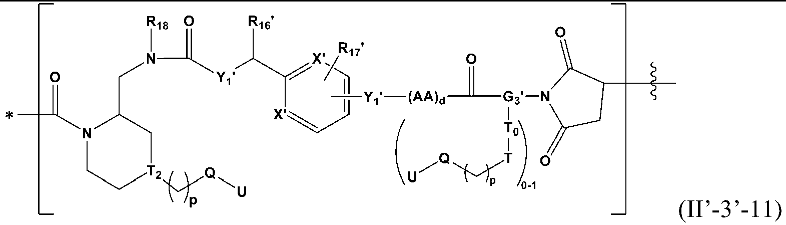 |
| 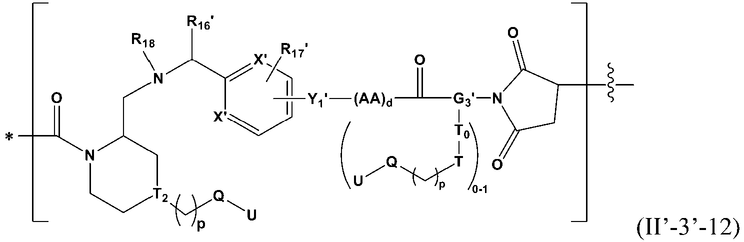 |
| 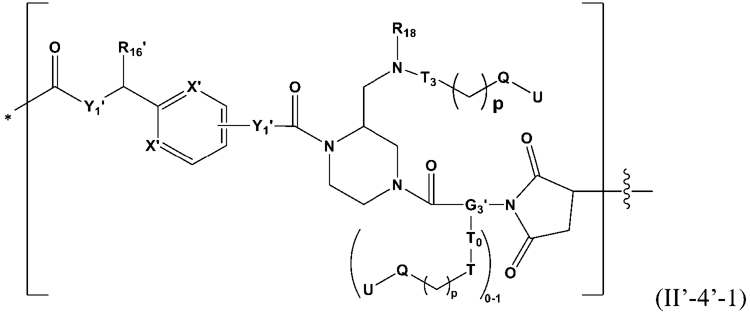 |
| and the corresponding general formula fragments where the maleimide moiety is 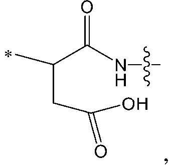 |
wherein each variable is as defined in any one of claims 15-18;
For example, the linker unit L is selected from the structural fragments shown in **Table 7** of the specification.

20. The antibody-drug conjugate according to claim 1, wherein the [P-L] conjugation fragment has any one of the following general formulas: 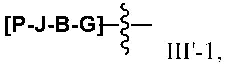 wherein the -J-B-G- fragment is as defined in any one of claims 15-19, preferably as defined in claim 19, and the drug P unit is a Ras inhibitor compound as defined in any one of claims 1 to 9, preferably a Ras inhibitor compound as defined in claim 7 or 9, or a stereoisomer, tautomer, stable isotopic variant, pharmaceutically acceptable salt, or solvate thereof;
Preferably, the [P-L] conjugation fragment is a conjugation fragment as shown in **Table 8-1** of the specification.

21. The antibody-drug conjugate according to claim 1, having a formula selected from: 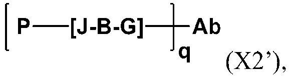
wherein the -J-B-G- fragment is as defined in any one of claims 15-19, preferably as defined in claim 19;
the drug P unit is a Ras inhibitor compound as defined in any one of claims 1 to 9, preferably a Ras inhibitor compound as defined in claim 7 or 9, or a stereoisomer, tautomer, stable isotopic variant, pharmaceutically acceptable salt, or solvate thereof;
q is from 1 to 10, or an average DAR value in a range formed by any two values between 1 and 10, for example about 1-10, 1-8, 2-8, 3-10, 3-8, 4-10, 4-8, 6-8, or 6-10.

22. The antibody-drug conjugate according to any one of claims 1 to 21, wherein Ab is an antibody or antigen-binding fragment that binds to a tumor-specific antigen or tumor-associated antigen, wherein the tumor-specific antigen or tumor-associated antigen is selected from: HER2, Her3, HER1 (ErbB1), HER4 (ErbB4), TROP2, Nectin-4, tissue factor, PD-L1, PD-1, PD-L1/PD-L2, MET, CLDN18.2, KIT, CTLA-4, RPR1, ephrin A2 receptor (EphA2), folate receptor (FRa), mesothelin, endothelin receptor, GCPII, IL-13Ra, BCMA, GD2, CLL-1, CA-IX, MUC1, 5T4, AOC3, ALK, AXL, C242, CA-125, CCL11, CCR5, CD2, CD3, CD4, CDS, CD15, CA15-3, CD16, CD18, CD19, CD20, CD21, CD22, CD25, CD30, CD32, CD33, CD37, CD38, CD44, CD52, CD56, CD64, CD66e, CD70, CD72, CD74, CD79a, CD79b, CD123, CD138, CD142, CD174, CD276, CDH3, CCD79b, CLDN9/CLDN6, CA19-9, DPEP3, AGS-16, IGF1R, IGF2R, VEGFR1, VEGFR2, VEGFR3, PDGFR-α, PDGFR-β, EGFR, EGFRvIII, ENPP3, FcRH5, FRα, KAAGl, LIV-1, Mesothelin, cMet, ROR1, SLTRK6, TF, BMPR1B, E16, TOP1, STEAP1, 0772P, MUC16, Napi3b, Sema 5b, PSCA hIg, ETBR, RNF124, prostate cancer-associated gene 1, TrpM4, teratocarcinoma-derived growth factor 1, C3DR, FcRH2, NCA, MDP, IL20R-α, Brevican, EphB2R, ASLG659, prostate stem cell antigen precursor, GEDA, BAFF-R, CXCR5, HLA-DOB, P2X5, LY64, FcRH1, IRTA2, TENB2, integrin α5β6, integrin α4β7, FGF2, FGFR1, FGFR2, FGFR3, FGFR4, PSMA, Somatostatin receptor, RANK, SLAMF7, ITGB6, CEACAM5, CA9, EGFRvlll, IL2RA, AXL receptor tyrosine kinase, TGF-βR, TNFRSF8, cancer/testis-associated antigens, CLEC14A, GRP78, stem cell-specific antigens, ASG-5, PRR4, GUCY2C, SLC39A6, TPBG, tumor-associated antigen CA242, FOLR1, GPNMB, HAVCR1, prostate tumor target Mindin, VTCN1, PTK7 protein tyrosine kinase 7, macrophage stimulating 1 receptor, TACSTD2, CA6, DLL3, DLL4, EpCAM, FAP, DKK-1, Endoglin, VCAM1, GPC3, DR5, ASCT2, B7H1, B7H3, B7H4, or any combination thereof;
Preferably, the tumor-specific antigen or tumor-associated antigen is selected from AXL, B7H1, B7H3, B7H4, BCMA, CD16, CD19, CD22, CD25, CD30, CD32, CD33, CD44, CD64, CD70, CD74, CD79, CD138, CD142, CD276, CDH3, CEACAM5, Claudin 18.2, CLDN9/CLDN6, DPEP3, EGFR, ENPP3, EphA, FcRH5, FOLR1, FRα, GCPII, HER2, HER3, KAAG1, KIT, LIV-1, Mesothelin, cMet, MUC1, Nectin-4, PD-L1, PD-L1/PD-L2, PSMA, ROR1, RPR1, TF, TOP1, TROP2, or any combination thereof;
More preferably, the tumor-specific antigen or tumor-associated antigen is selected from HER2, Claudin 18.2, EGFR, TROP2, Nectin-4, or a combination of EGFR and Met.

23. The antibody-drug conjugate according to any one of claims 1 to 22, wherein:
The Ab comprises 3 heavy chain complementarity determining regions (HCDR) and 3 light chain complementarity determining regions (LCDR) that specifically bind to HER2, wherein: according to Kabat definition,
HCDR1 comprises or consists of the amino acid sequence of SEQ ID NO: 6,
HCDR2 comprises or consists of the amino acid sequence of SEQ ID NO: 7,
HCDR3 comprises or consists of the amino acid sequence of SEQ ID NO: 8,
LCDR1 comprises or consists of the amino acid sequence of SEQ ID NO: 3,
LCDR2 comprises or consists of the amino acid sequence of SEQ ID NO: 4, and
LCDR3 comprises or consists of the amino acid sequence of SEQ ID NO: 5;
or the Ab comprises 3 heavy chain complementarity determining regions (HCDR) and 3 light chain complementarity determining regions (LCDR) that specifically bind to Trop2, wherein: according to Kabat definition,
HCDR1 comprises or consists of the amino acid sequence of SEQ ID NO: 18,
HCDR2 comprises or consists of the amino acid sequence of SEQ ID NO: 19,
HCDR3 comprises or consists of the amino acid sequence of SEQ ID NO: 20,
LCDR1 comprises or consists of the amino acid sequence of SEQ ID NO: 15,
LCDR2 comprises or consists of the amino acid sequence of SEQ ID NO: 16, and
LCDR3 comprises or consists of the amino acid sequence of SEQ ID NO: 17;
or the Ab comprises 3 heavy chain complementarity determining regions (HCDR) and 3 light chain complementarity determining regions (LCDR) that specifically bind to Claudin18.2, wherein: according to Kabat definition,
HCDR1 comprises or consists of the amino acid sequence of SEQ ID NO: 29,
HCDR2 comprises or consists of the amino acid sequence of SEQ ID NO: 30,
HCDR3 comprises or consists of the amino acid sequence of SEQ ID NO: 31,
LCDR1 comprises or consists of the amino acid sequence of SEQ ID NO: 26,
LCDR2 comprises or consists of the amino acid sequence of SEQ ID NO: 27, and
LCDR3 comprises or consists of the amino acid sequence of SEQ ID NO: 28;
or the Ab comprises 3 heavy chain complementarity determining regions (HCDR) and 3 light chain complementarity determining regions (LCDR) that specifically bind to EGFR, wherein: according to Kabat definition,
HCDR1 comprises or consists of the amino acid sequence of SEQ ID NO: 40,
HCDR2 comprises or consists of the amino acid sequence of SEQ ID NO: 41,
HCDR3 comprises or consists of the amino acid sequence of SEQ ID NO: 42,
LCDR1 comprises or consists of the amino acid sequence of SEQ ID NO: 37,
LCDR2 comprises or consists of the amino acid sequence of SEQ ID NO: 38, and
LCDR3 comprises or consists of the amino acid sequence of SEQ ID NO: 39;
or the Ab comprises 3 heavy chain complementarity determining regions (HCDR) and 3 light chain complementarity determining regions (LCDR) that specifically bind to Nectin4, wherein: according to Kabat definition,
HCDR1 comprises or consists of the amino acid sequence of SEQ ID NO: 72,
HCDR2 comprises or consists of the amino acid sequence of SEQ ID NO: 73,
HCDR3 comprises or consists of the amino acid sequence of SEQ ID NO: 74,
LCDR1 comprises or consists of the amino acid sequence of SEQ ID NO: 69,
LCDR2 comprises or consists of the amino acid sequence of SEQ ID NO: 70, and
LCDR3 comprises or consists of the amino acid sequence of SEQ ID NO: 71,
or the Ab comprises a first set of complementarity determining regions that specifically bind EGFR and a second set of complementarity determining regions that specifically bind MET, wherein,
the first set of complementarity determining regions comprises 3 heavy chain complementarity determining regions (HCDR) and 3 light chain complementarity determining regions (LCDR), wherein: according to Kabat definition,
HCDR1 comprises or consists of the amino acid sequence of SEQ ID NO: 49,
HCDR2 comprises or consists of the amino acid sequence of SEQ ID NO: 50,
HCDR3 comprises or consists of the amino acid sequence of SEQ ID NO: 51,
LCDR1 comprises or consists of the amino acid sequence of SEQ ID NO: 52,
LCDR2 comprises or consists of the amino acid sequence of SEQ ID NO: 53, and
LCDR3 comprises or consists of the amino acid sequence of SEQ ID NO: 54,
the second set of complementarity determining regions comprises 3 heavy chain complementarity determining regions (HCDR) and 3 light chain complementarity determining regions (LCDR), wherein: according to Kabat definition,
HCDR1 comprises or consists of the amino acid sequence of SEQ ID NO: 55,
HCDR2 comprises or consists of the amino acid sequence of SEQ ID NO: 56,
HCDR3 comprises or consists of the amino acid sequence of SEQ ID NO: 57,
LCDR1 comprises or consists of the amino acid sequence of SEQ ID NO: 58,
LCDR2 comprises or consists of the amino acid sequence of SEQ ID NO: 59, and
LCDR3 comprises or consists of the amino acid sequence of SEQ ID NO: 60.

24. The antibody-drug conjugate according to any one of claims 1 to 22, wherein the Ab comprises a heavy chain variable region and a light chain variable region, wherein the heavy chain variable region comprises the amino acid sequence described in SEQ ID NO: 10, and wherein the light chain variable region comprises the amino acid sequence shown in SEQ ID NO: 9; or
wherein the heavy chain variable region comprises the amino acid sequence described in SEQ ID NO: 22, and wherein the light chain variable region comprises the amino acid sequence shown in SEQ ID NO: 21; or
wherein the heavy chain variable region comprises the amino acid sequence described in SEQ ID NO: 33, and wherein the light chain variable region comprises the amino acid sequence shown in SEQ ID NO: 32; or
wherein the heavy chain variable region comprises the amino acid sequence described in SEQ ID NO: 44, and wherein the light chain variable region comprises the amino acid sequence shown in SEQ ID NO: 43; or
wherein the heavy chain variable region comprises the amino acid sequence described in SEQ ID NO: 76, and wherein the light chain variable region comprises the amino acid sequence shown in SEQ ID NO: 75; or
wherein the Ab comprises a first heavy chain variable region and a first light chain variable region, and a second heavy chain variable region and a second light chain variable region, wherein the first heavy chain variable region and first light chain variable region respectively comprise the amino acid sequences described in SEQ ID NO: 61 and SEQ ID NO: 62, and wherein the second heavy chain variable region and second light chain variable region respectively comprise the amino acid sequences described in SEQ ID NO: 63 and SEQ ID NO: 64.

25. The antibody-drug conjugate according to any one of claims 1 to 22, wherein the Ab comprises:
(a) a heavy chain comprising the amino acid sequence of SEQ ID NO: 2, and
(b) a light chain comprising the amino acid sequence of SEQ ID NO: 1;
or
(a) a heavy chain comprising the amino acid sequence of SEQ ID NO: 14, and
(b) a light chain comprising the amino acid sequence of SEQ ID NO: 13;
or
(a) a heavy chain comprising the amino acid sequence of SEQ ID NO: 25, and
(b) a light chain comprising the amino acid sequence of SEQ ID NO: 24;
or
(a) a heavy chain comprising the amino acid sequence of SEQ ID NO: 36, and
(b) a light chain comprising the amino acid sequence of SEQ ID NO: 35;
or
(a) a heavy chain comprising the amino acid sequence of SEQ ID NO: 76, and
(b) a light chain comprising the amino acid sequence of SEQ ID NO: 75,
or
(a) a first heavy chain comprising the amino acid sequence of SEQ ID NO: 45,
(b) a first light chain comprising the amino acid sequence of SEQ ID NO: 46,
(c) a second heavy chain comprising the amino acid sequence of SEQ ID NO: 47, and
(d) a second light chain comprising the amino acid sequence of SEQ ID NO: 48.

26. The antibody-drug conjugate according to any one of claims 1 to 22, wherein the Ab is selected from Trastuzumab or an antibody fragment thereof, or another anti-human HER2 antibody that recognizes the same epitope or competitively binds to human HER2, preferably Trastuzumab; or
wherein the Ab is selected from Sacituzumab or an antibody fragment thereof, or another anti-human TROP2 antibody that recognizes the same epitope or competitively binds to human TROP2, preferably Sacituzumab; or
wherein the Ab is selected from Zolbetuximab or an antibody fragment thereof, or another anti-human Claudin18.2 antibody that recognizes the same epitope or competitively binds to human Claudin18.2, preferably Zolbetuximab; or
wherein the Ab is selected from Cetuximab or an antibody fragment thereof, or another anti-human EGFR antibody that recognizes the same epitope or competitively binds to human EGFR, preferably Cetuximab; or
wherein the Ab is selected from Enfortumab or an antibody fragment thereof, or another anti-human Nectin4 antibody that recognizes the same epitope or competitively binds to human Nectin4, preferably Enfortumab; or
wherein the Ab is selected from Amivantamab or an antibody fragment thereof, or another anti-human EGFR and MET antibody that recognizes the same epitope or competitively binds to human EGFR and MET, preferably Amivantamab.

27. An antibody-drug conjugate selected from:
| | |
|---|---|
| ADC-1 | 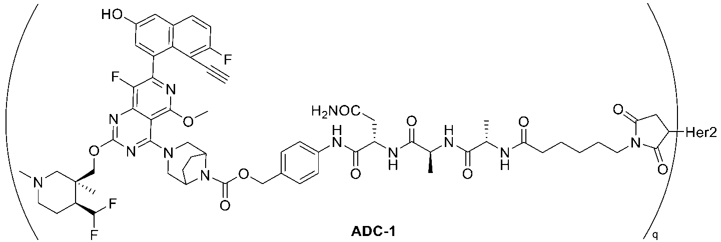 |
| ADC-2 | 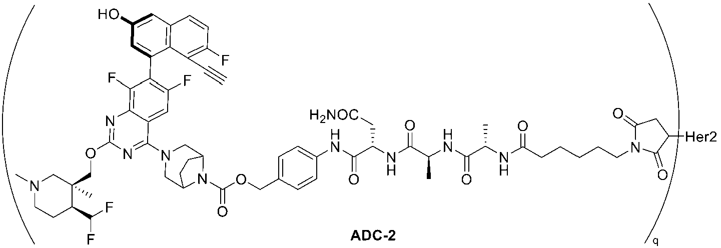 |
| ADC-3 | 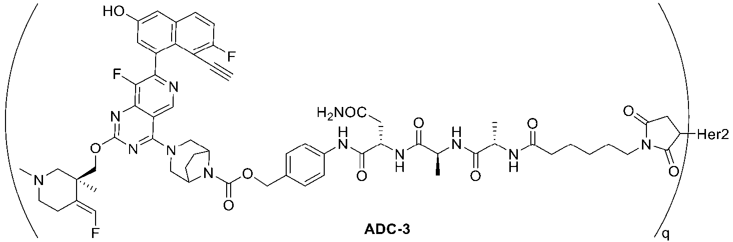 |
| ADC-4 | 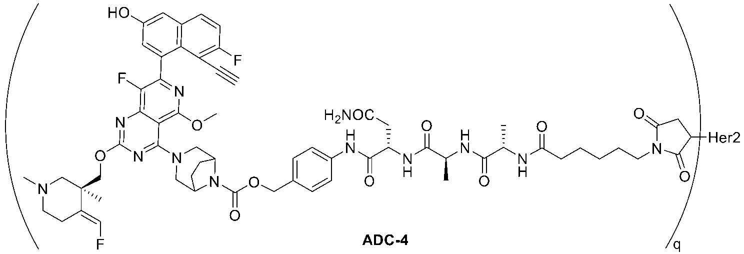 |
| ADC-5 | 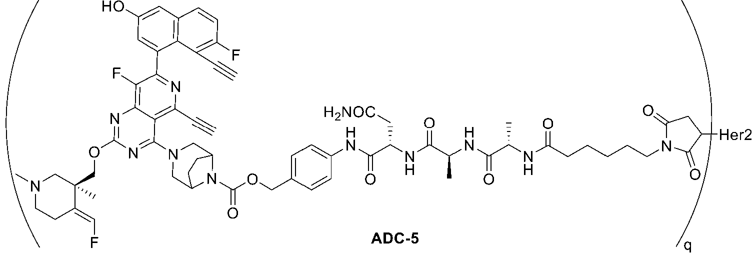 |
| ADC-6 | 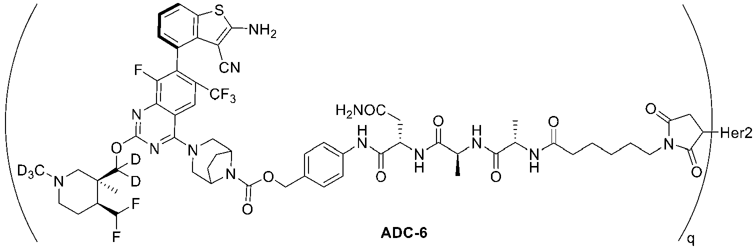 |
| ADC-7 | 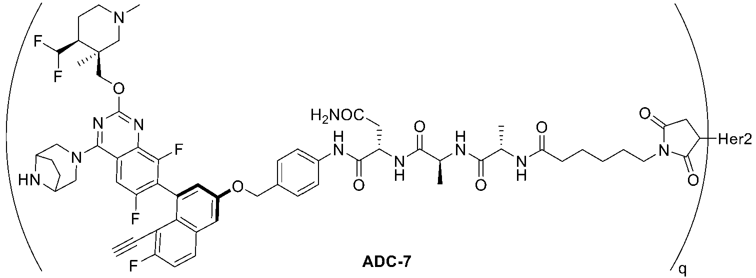 |
| ADC-8 | 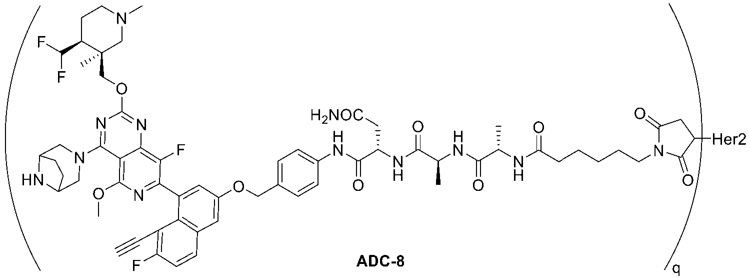 |
| ADC-9 | 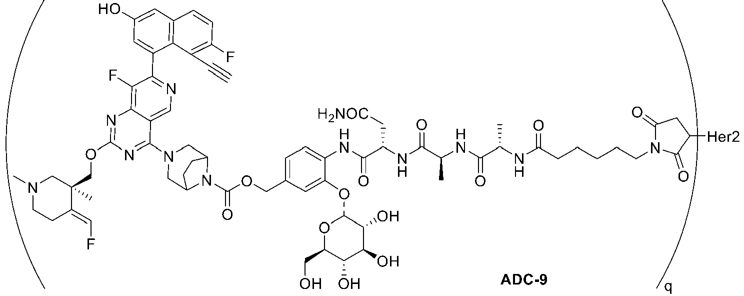 |
| ADC-10 | 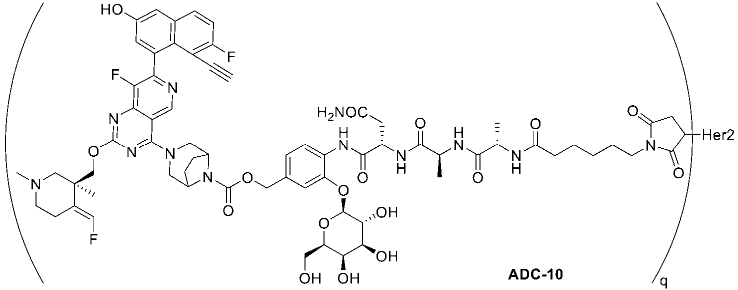 |
| ADC-11 | 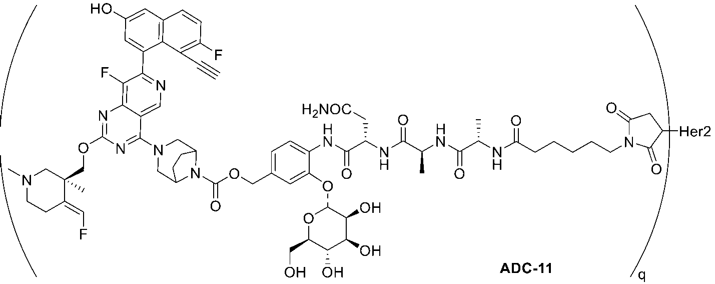 |
| ADC-12 | 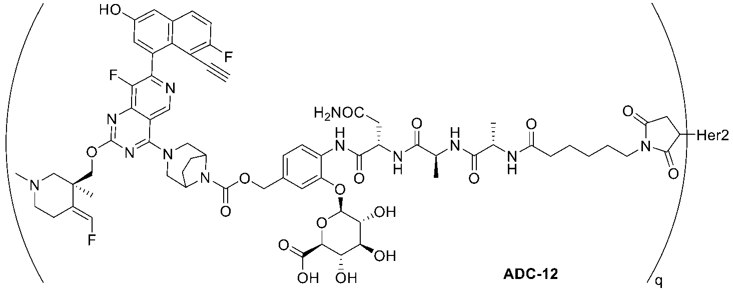 |
| ADC-13 | 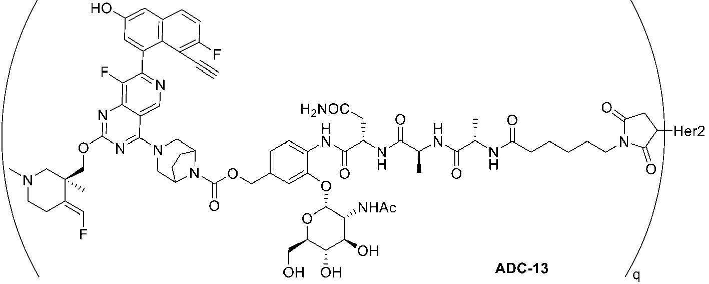 |
| ADC-14 | 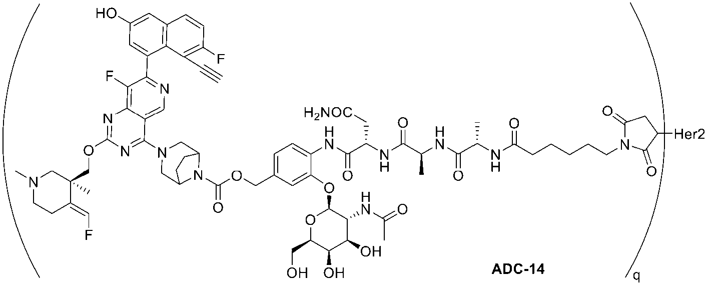 |
| ADC-15 | 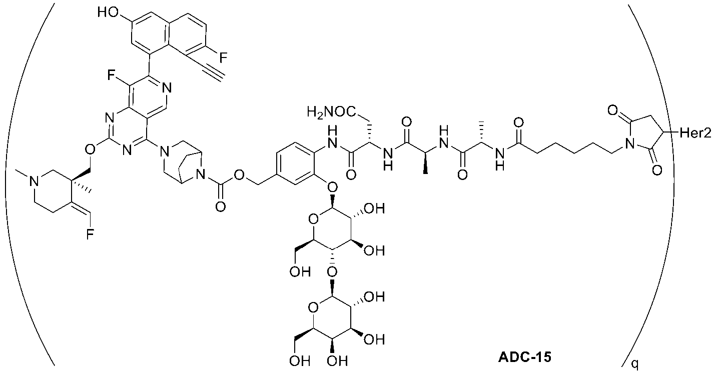 |
| ADC-16 | 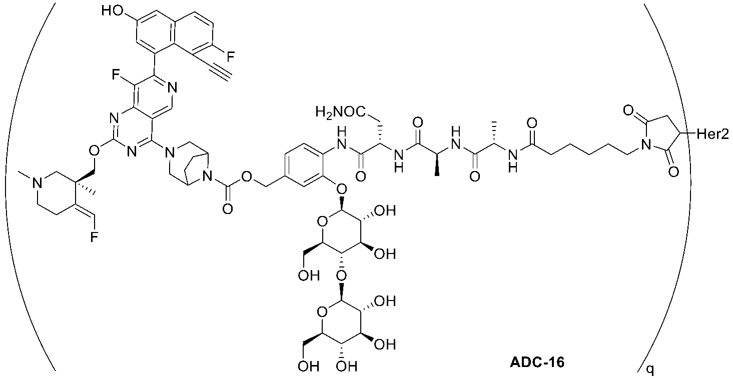 |
| ADC-17 | 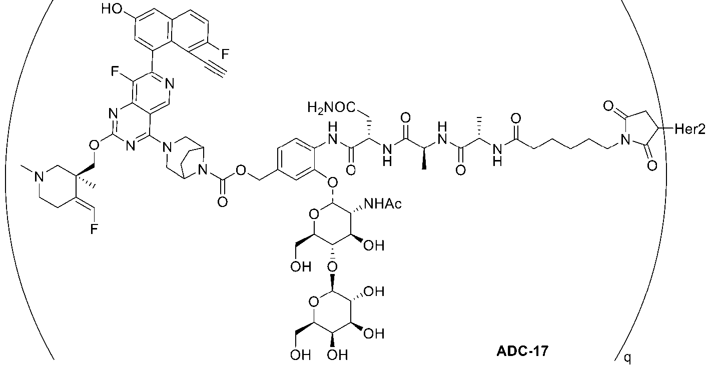 |
| ADC-18 | 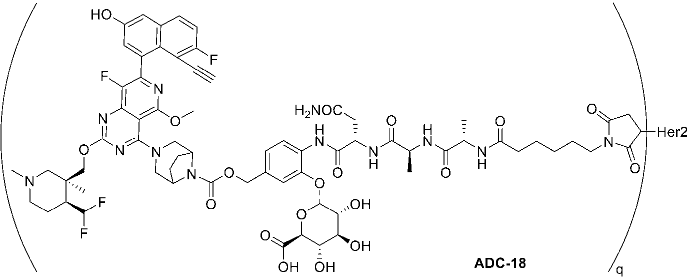 |
| ADC-19 | 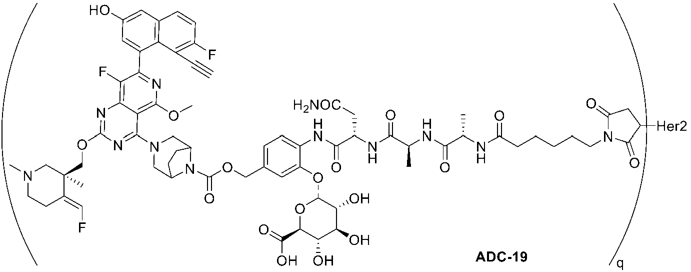 |
| ADC-20 | 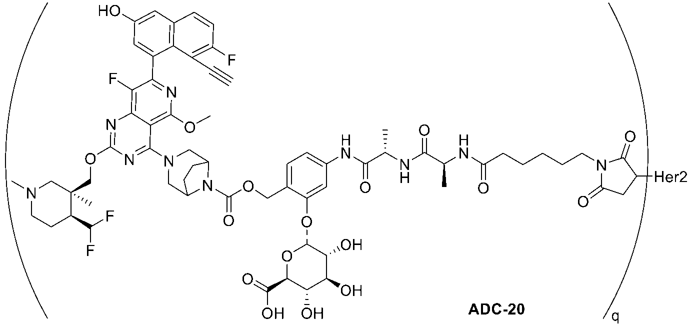 |
| ADC-21 | 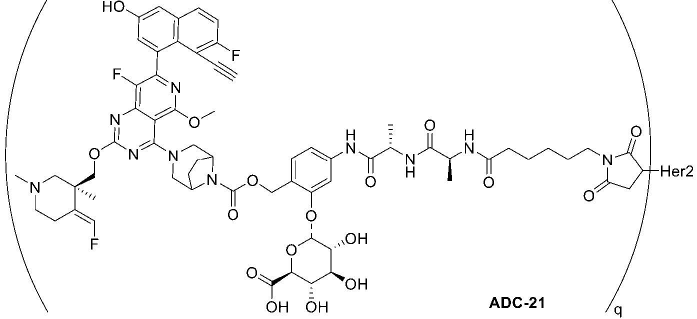 |
| ADC-22 | 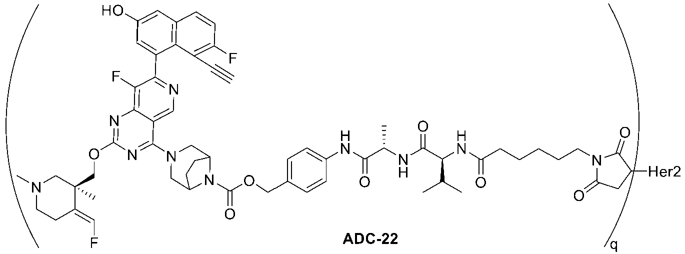 |
| ADC-23 | 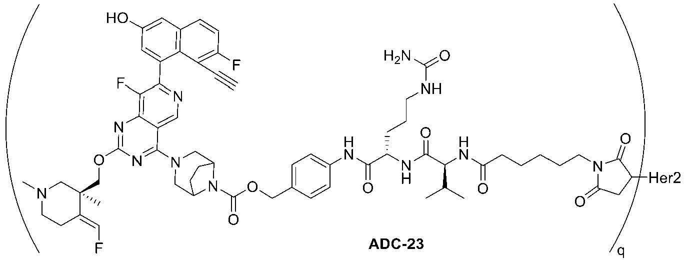 |
| ADC-24 | 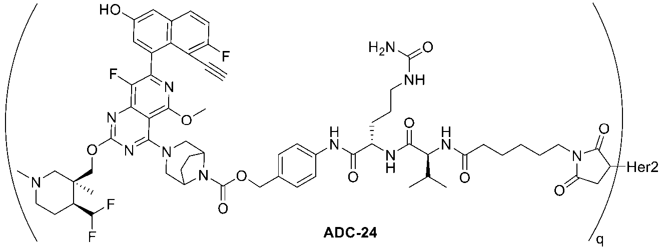 |
| ADC-25 | 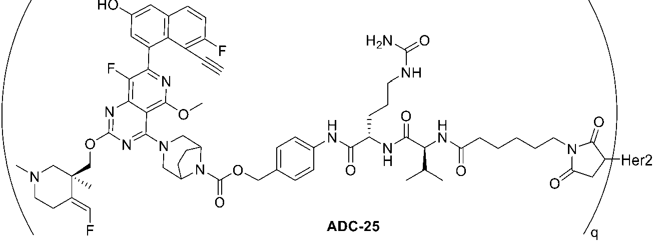 |
| ADC-26 | 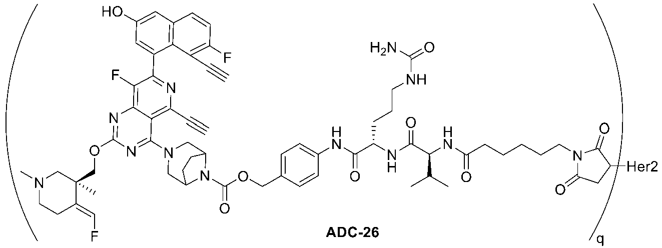 |
| ADC-27 | 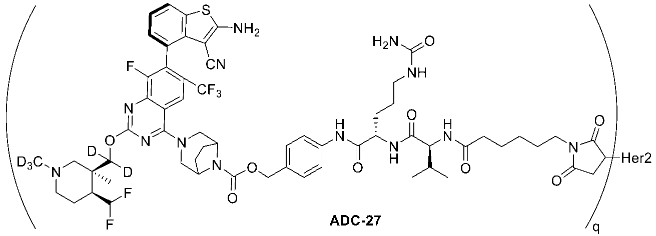 |
| ADC-28 | 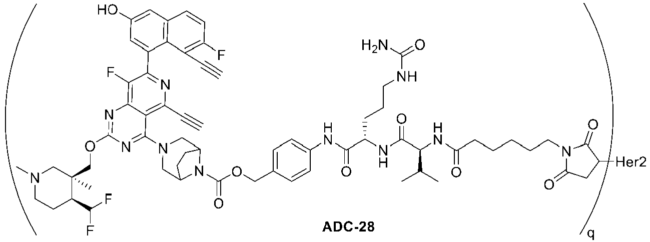 |
| ADC-29 | 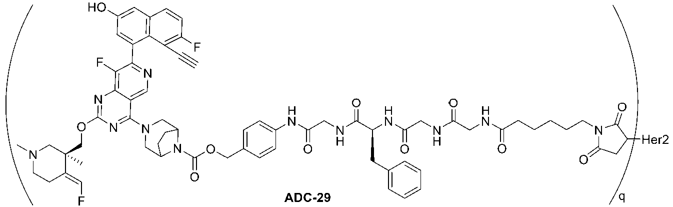 |
| ADC-30 | 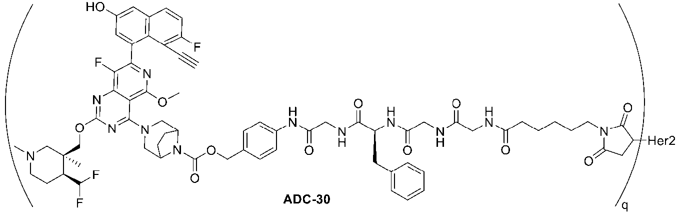 |
| ADC-31 | 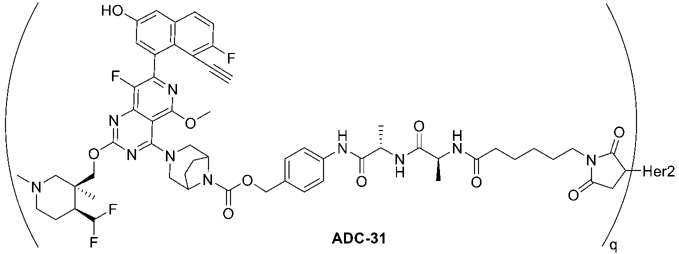 |
| ADC-32 | 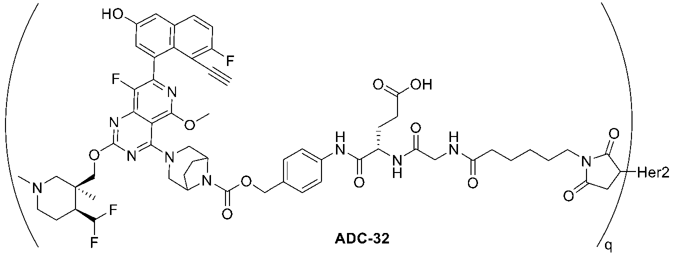 |
| ADC-33 | 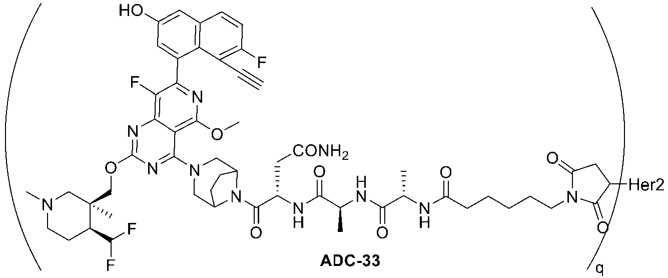 |
| ADC-34 | 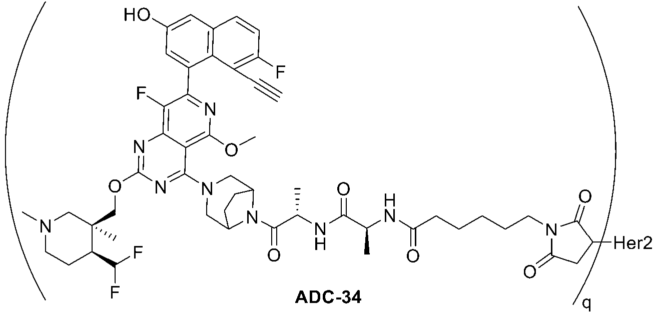 |
| ADC-35 | 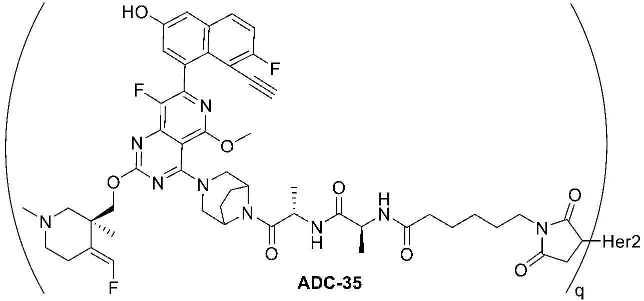 |
| ADC-36 | 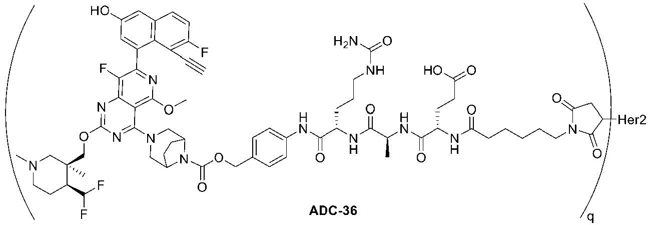 |
| ADC-37 | 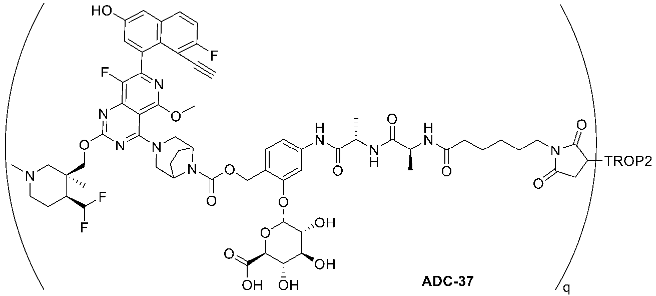 |
| ADC-38 | 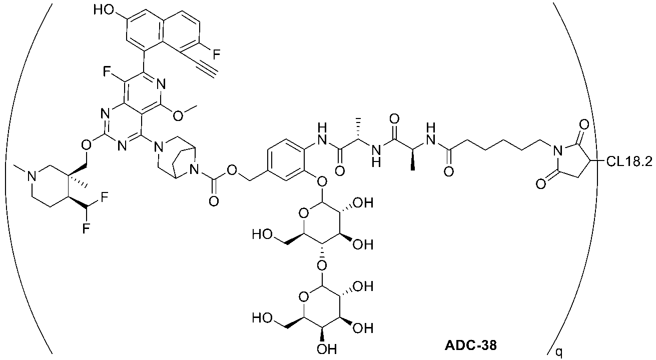 |
| ADC-39 | 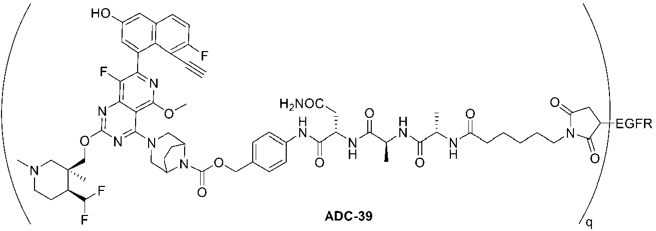 |
| ADC-40 | 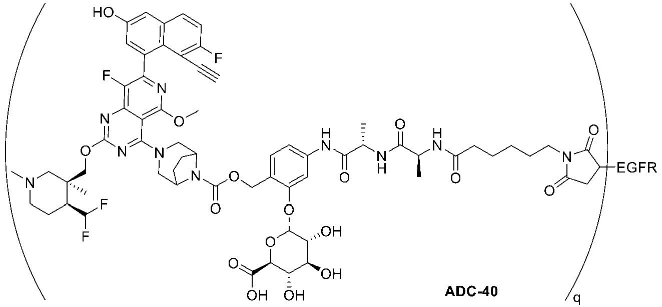 |
| ADC-41 | 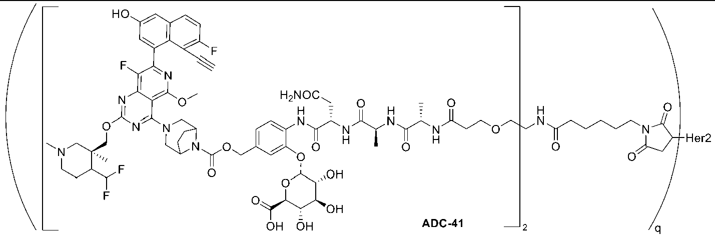 |
| ADC-42 | 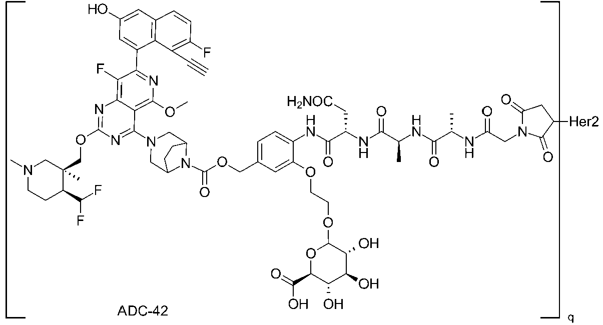 |
| ADC-43 | 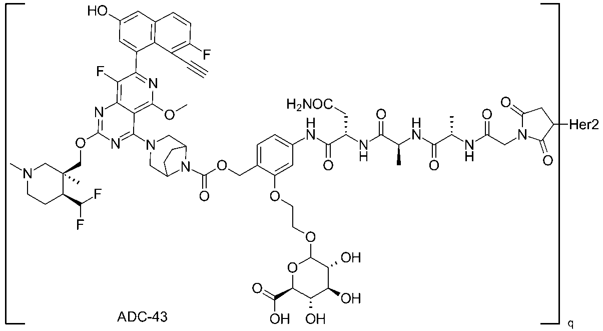 |
| ADC-44 | 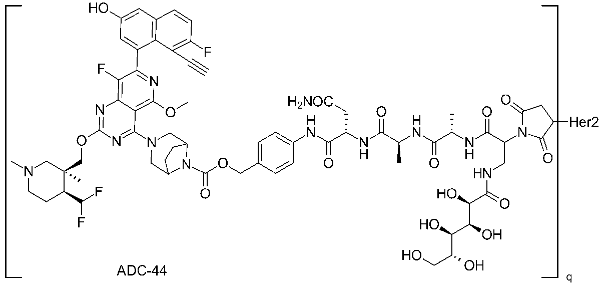 |
| ADC-45 | 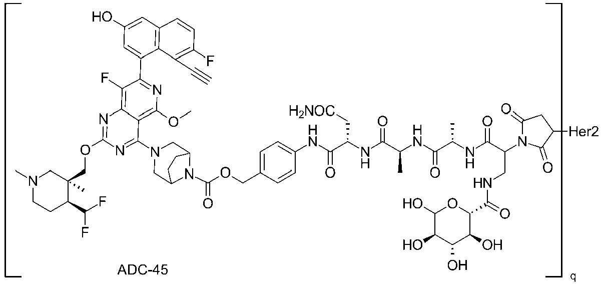 |
| ADC-46 | 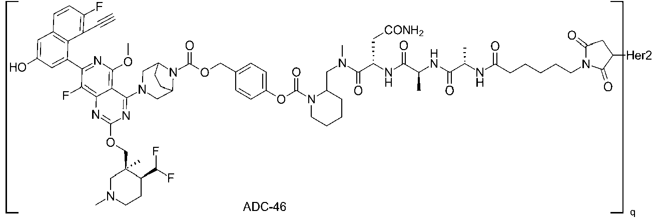 |
| ADC-47 | 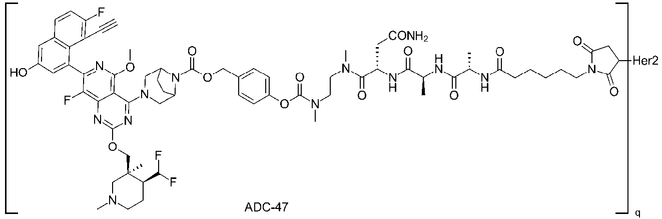 |
| ADC-48 | 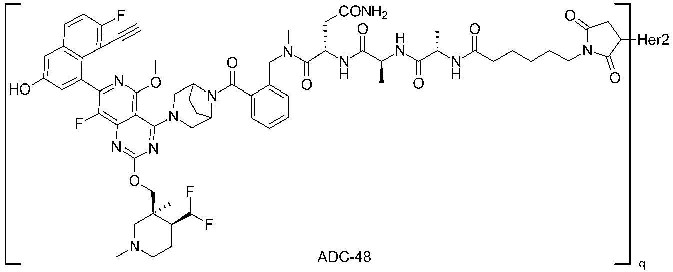 |
| ADC-49 | 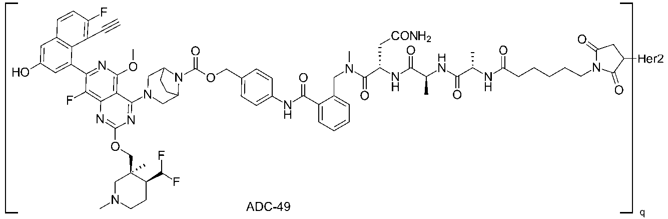 |
| ADC-50 | 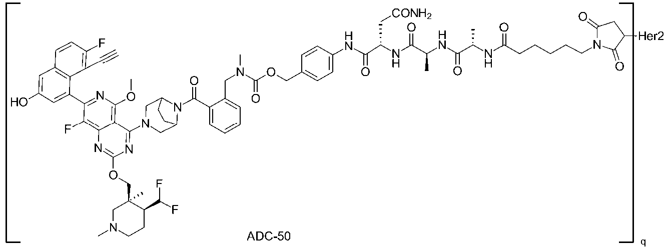 |
| ADC-51 | 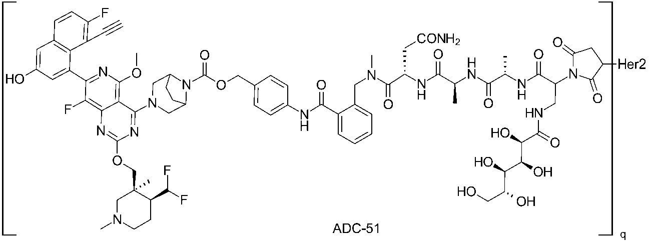 |
| ADC-52 | 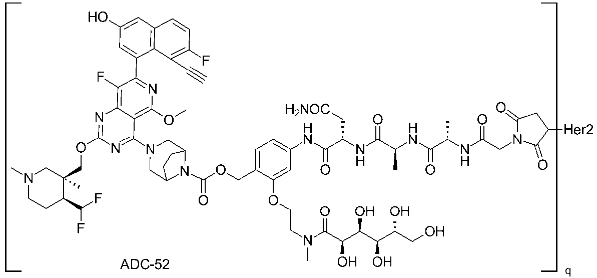 |
| ADC-53 | 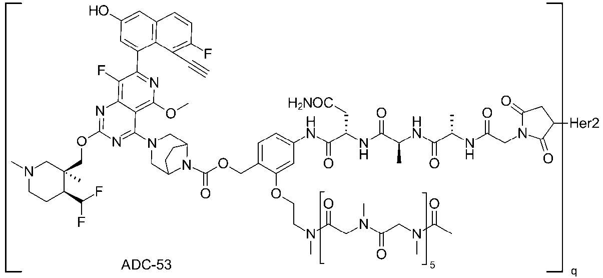 |
| ADC-54 | 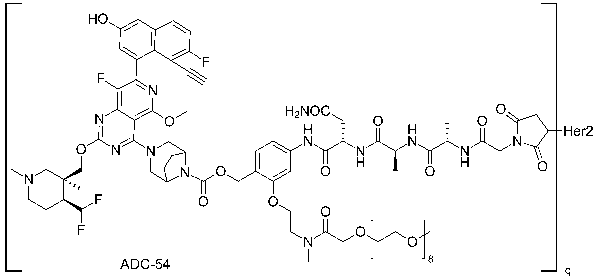 |
| ADC-55 | 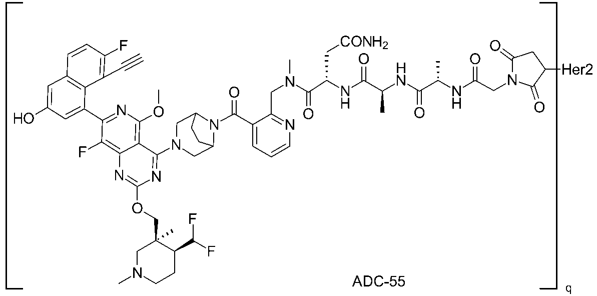 |
| ADC-56 | 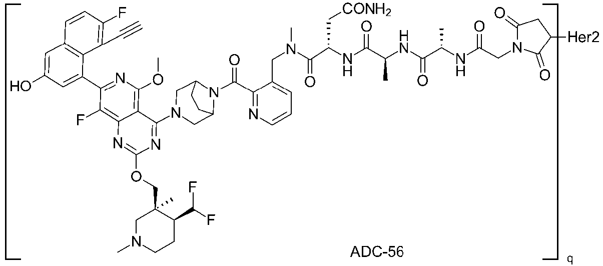 |
| ADC-57 | 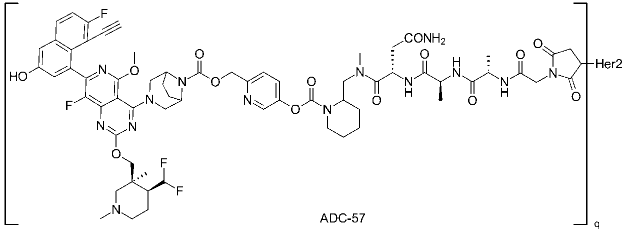 |
| ADC-58 | 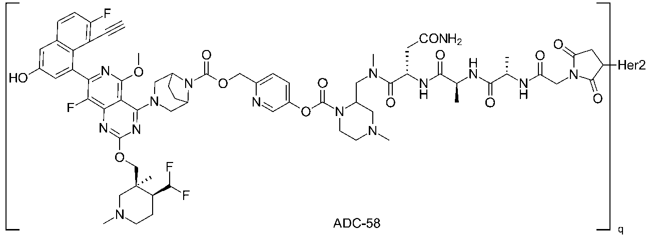 |
| ADC-59 | 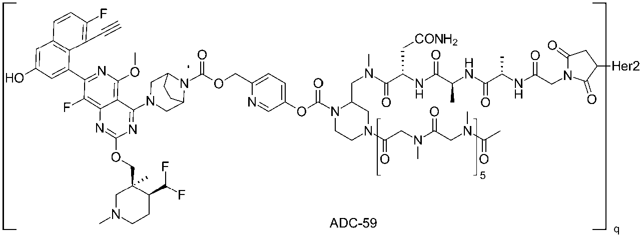 |
| ADC-60 | 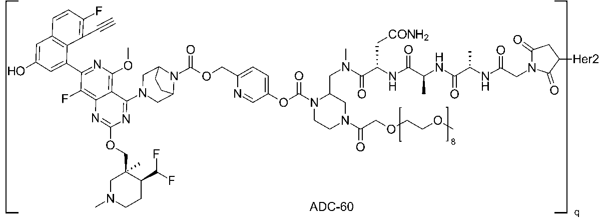 |
| ADC-61 | 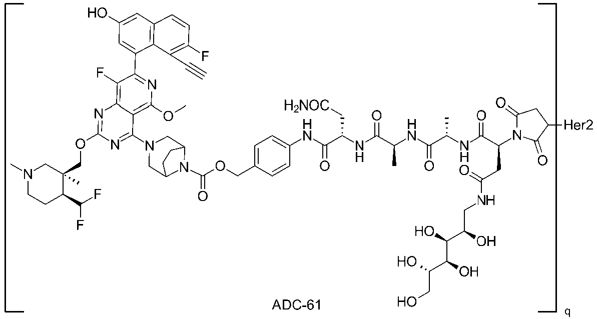 |
| ADC-62 | 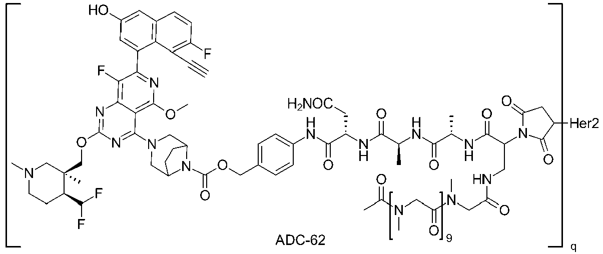 |
| ADC-63 | 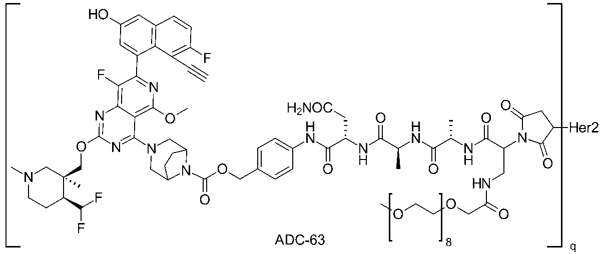 |
| ADC-64 | 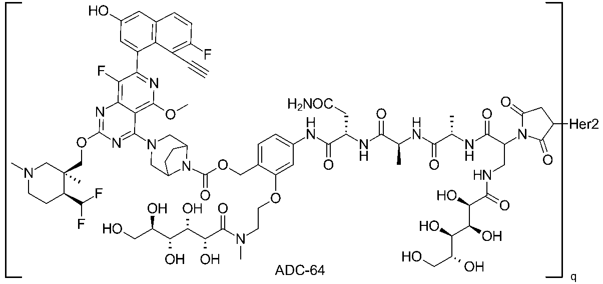 |
| ADC-65 | 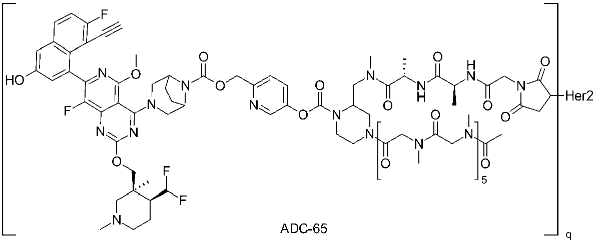 |
| ADC-66 | 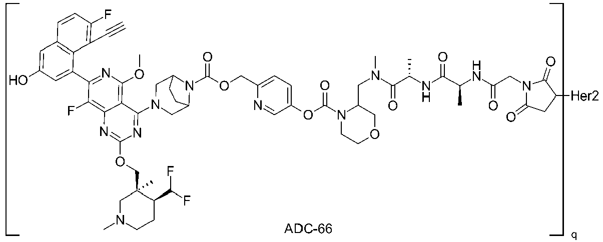 |
| ADC-67 | 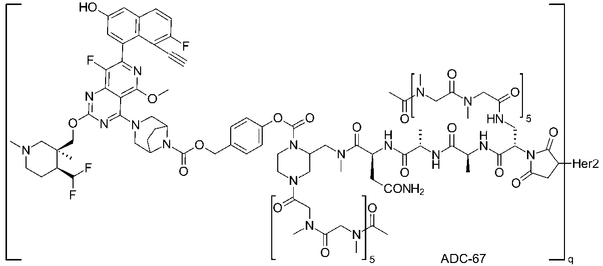 |
| ADC-68 | 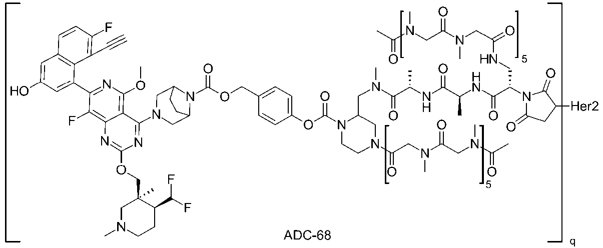 |
| ADC-69 | 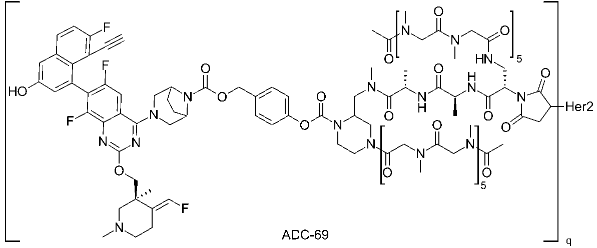 |
| ADC-70 | 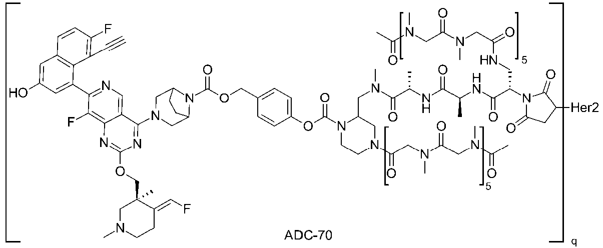 |
| ADC-71 | 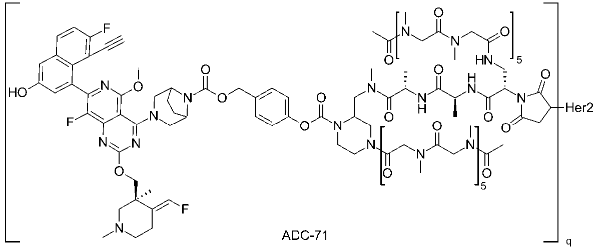 |
| ADC-72 | 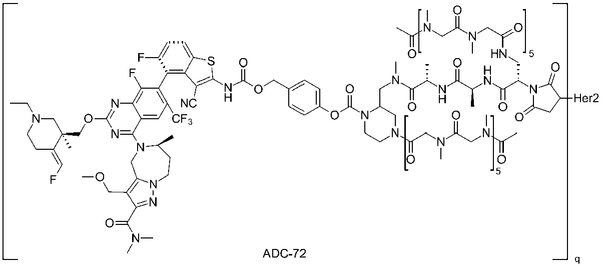 |
| ADC-73 | 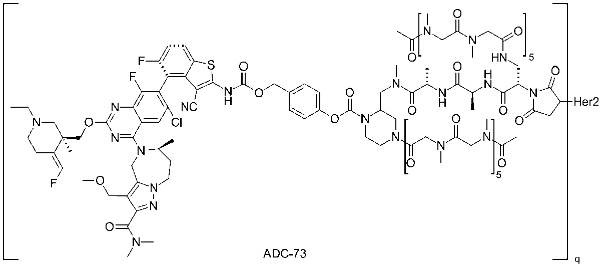 |
| ADC-74 | 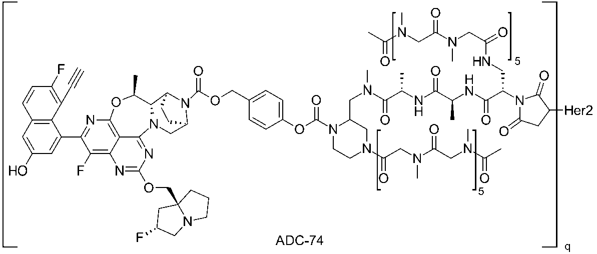 |
| ADC-75 | 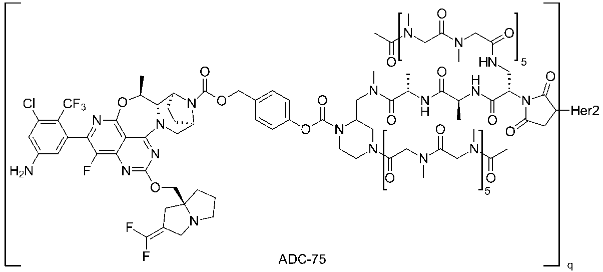 |
| ADC-76 | 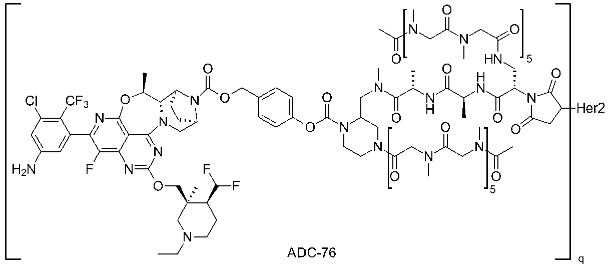 |
| ADC-77 | 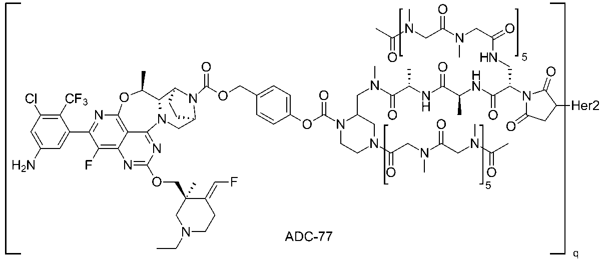 |
| ADC-78 | 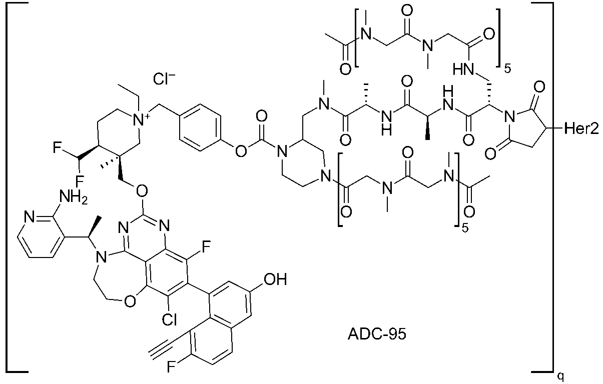 |
| ADC-79 | 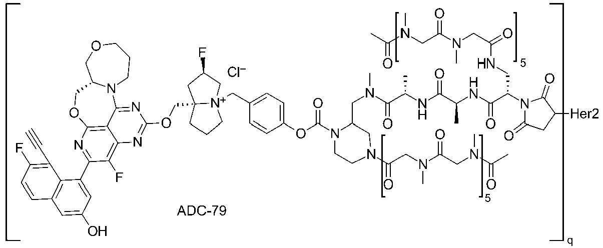 |
| ADC-80 | 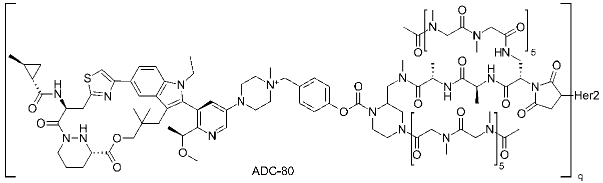 |
| ADC-81 | 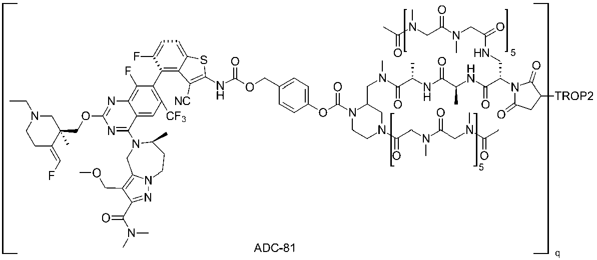 |
| ADC-82 | 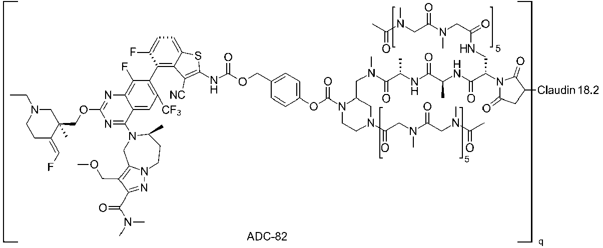 |
| ADC-83 | 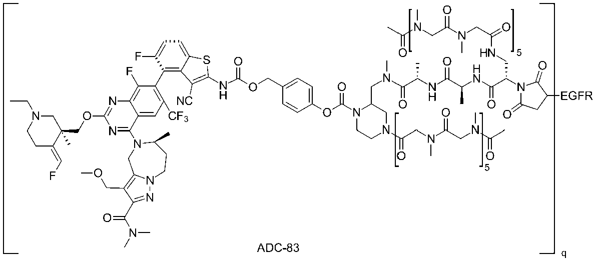 |
| ADC-84 | 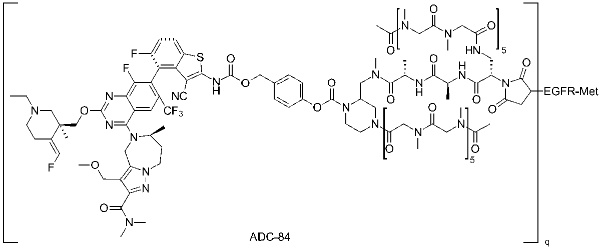 |
| ADC-85 | 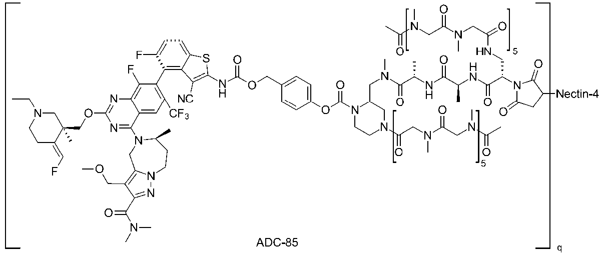 |
| ADC-86 | 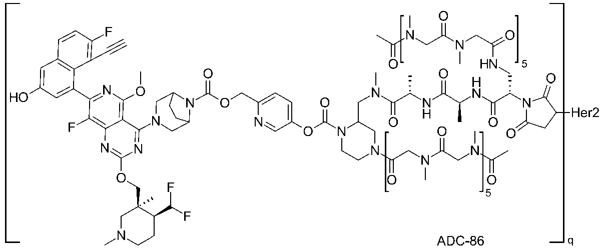 |
| ADC-87 | 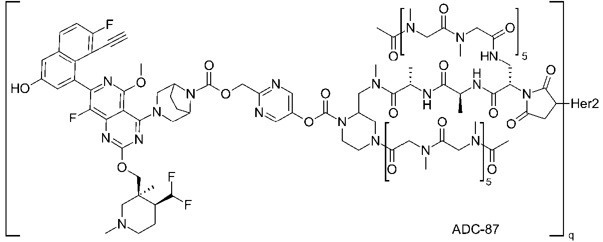 |
| ADC-88 | 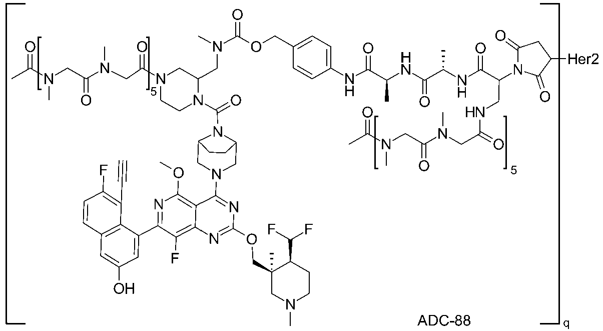 |
| ADC-89 | 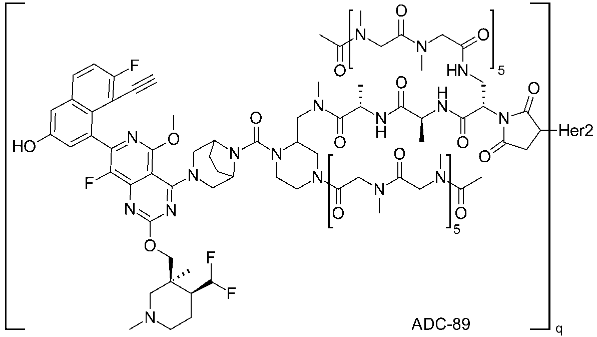 |
| ADC-90 | 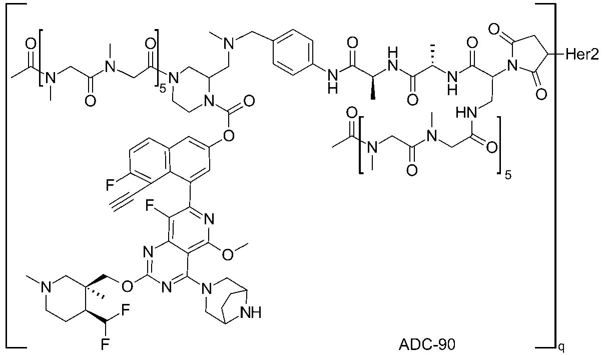 |
| ADC-91 | 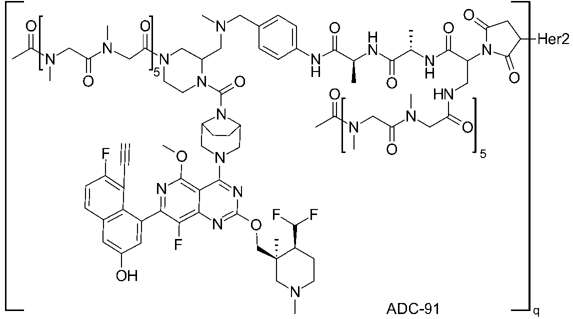 |
| ADC-92 | 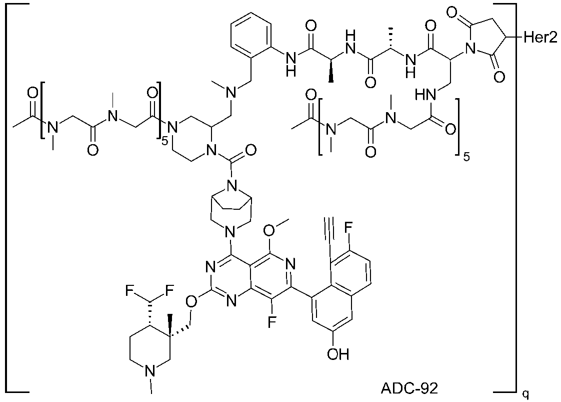 |
| ADC-93 | 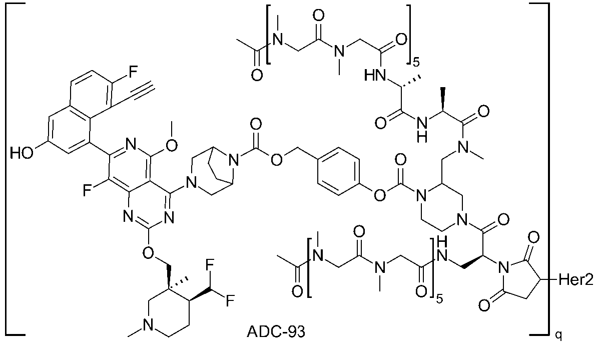 |
| ADC-94 | 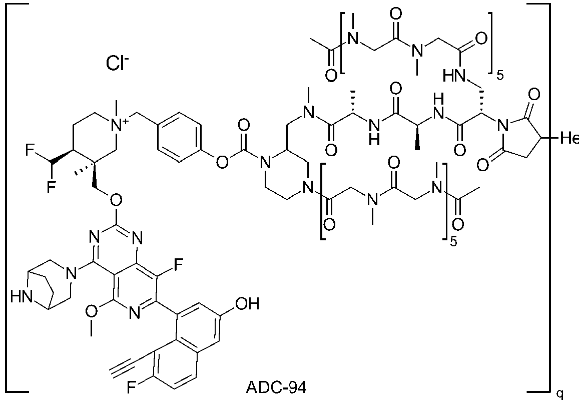 |
| and the corresponding ADCs where the maleimide moiety is 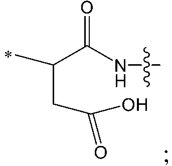 | |
| Preferably, | |
| the antibody indicated as Her2 is specifically Trastuzumab; | |
| the antibody indicated as CL18.2 is specifically Zolbetuximab; | |
| the antibody indicated as TROP2 is specifically Sacituzumab; | |
| the antibody indicated as EGFR is specifically Cetuximab; | |
| the antibody indicated as EGFR-Met is specifically Amivantamab; | |
| the antibody indicated as Nectin-4 is specifically Enfortumab; | |
| q represents 1 to 20, for example 1-10, 1-8, 2-8, 3-10, 3-8, 4-10, 4-8, 6-8, or 6-10; | |
or a stereoisomer, tautomer, stable isotopic variant, pharmaceutically acceptable salt, or solvate thereof;
preferably the antibody-drug conjugate of Examples 1-94, or a stereoisomer, tautomer, stable isotopic variant, pharmaceutically acceptable salt, or solvate thereof.

28. A pharmaceutical composition comprising the antibody-drug conjugate according to any one of claims 1-27 and one or more pharmaceutically acceptable excipients.

29. The pharmaceutical composition according to claim 28, for administration intravenously, intratumorally, subcutaneously, intramuscularly, orally, intranasally, intrathecally, transdermally, or topically, preferably for administration intravenously, intraperitoneally, subcutaneously, or intramuscularly.

30. The antibody-drug conjugate according to any one of claims 1-27 or the pharmaceutical composition according to claim 28 or 29, for use in treating or preventing a disease mediated by a Ras mutant protein, such as a KRas mutant protein, specifically a KRas G12D mutant protein, preferably for use in treating or preventing a hyperproliferative disease, more preferably for use in treating or preventing a tumor.

31. Use of the antibody-drug conjugate according to any one of claims 1-27 or the pharmaceutical composition according to claim 28 or 29 in treating or preventing a disease mediated by a Ras mutant protein, such as a KRas mutant protein, specifically a KRas G12D mutant protein, preferably a hyperproliferative disease, more preferably a tumor.

32. Use of the antibody-drug conjugate according to any one of claims 1-27 or the pharmaceutical composition according to claim 28 or 29 in the manufacture of a medicament for treating or preventing a disease mediated by a Ras mutant protein, such as a KRas mutant protein, specifically a KRas G12D mutant protein, preferably a hyperproliferative disease, more preferably a tumor.

33. A method for treating or preventing a disease mediated by a Ras mutant protein, such as a KRas mutant protein, specifically a KRas G12D mutant protein, preferably a hyperproliferative disease, more preferably a tumor, comprising administering to a human or animal the antibody-drug conjugate according to any one of claims 1-27 or the pharmaceutical composition according to claim 28 or 29.

34. The antibody-drug conjugate or pharmaceutical composition according to claim 30, the use according to claim 31 or 32, or the method according to claim 33, wherein the hyperproliferative disease or tumor includes solid tumors and hematological tumors, and all precancerous cells and cancer cells and tissues, selected from lung adenocarcinoma, lung cancer (including squamous cell carcinoma and non-small cell lung cancer, small cell lung cancer), bone cancer, pancreatic cancer, pancreatic ductal adenocarcinoma, skin cancer, head and neck cancer (including head and neck squamous cell carcinoma), melanoma (including cutaneous or intraocular melanoma), squamous cell carcinoma, anal region cancer, testicular cancer, urethral cancer, ureteral cancer, penile cancer, prostate cancer (including hormone-refractory prostate cancer), bladder cancer, uterine cancer, ovarian cancer, ovarian epithelial cancer, fallopian tube cancer, endometrial cancer, cervical cancer, vaginal cancer, vulvar cancer, gastric cancer, gastric adenocarcinoma, colon cancer, rectal cancer, colorectal cancer, liver cancer, breast cancer (including metastatic breast cancer, triple-negative breast cancer (TNBC)), esophageal cancer, small intestine cancer, lip cancer, laryngeal cancer, nasopharyngeal cancer, oral cancer, salivary gland cancer, peritoneal cancer, gastrointestinal stromal tumor, gastroesophageal junction (GEJ) cancer, mesothelioma, biliary tract cancer, hepatocellular carcinoma, seminoma, soft tissue sarcoma, osteosarcoma, urothelial carcinoma, sweat gland carcinoma, endocrine system cancer, thyroid cancer, medullary thyroid cancer, follicular thyroid cancer, papillary thyroid cancer, parathyroid cancer, kidney cancer, renal parenchymal cancer, renal cell carcinoma, renal pelvic cancer, adrenal cancer, brain cancers such as glioblastoma, astrocytoma, meningioma, medulloblastoma, peripheral neuroectodermal tumors, glioblastoma (including glioblastoma multiforme), neuroblastoma; chronic or acute leukemias, Hodgkin's disease, lymphomas (including lymphocytic lymphoma, Hodgkin lymphoma, non-Hodgkin lymphoma, Burkitt lymphoma, adult T-cell lymphoma, diffuse large B-cell lymphoma (DLBCL), primary CNS lymphoma), acute lymphoblastic leukemia (ALL), chronic lymphocytic leukemia (CLL), chronic myeloid leukemia (CLL) and lymphocytic carcinoma, acute myeloid leukemia (AML), myeloid leukemia (chronic myeloid leukemia (CML)), central nervous system (CNS) tumors, spinal tumors, brainstem glioma, or pituitary adenoma.

35. The antibody-drug conjugate or pharmaceutical composition, use, or method according to claim 34, wherein the tumor is selected from lung cancer, lung adenocarcinoma, colon cancer, rectal cancer, pancreatic cancer, endometrial cancer, cholangiocarcinoma, leukemia, and ovarian cancer.

36. A compound fragment having any of the general formulas (II'-3'-1)~(II'-3'-12) and (II'-4'-1) as defined in claim 19, preferably a structural fragment as shown in **Table 7** of the specification.
